# EUROPEAN PATENT APPLICATION

(11) **EP 3 744 727 A1**
(43) Date of publication of application: **02.12.2020**
(21) Application number: 20174779.7
(22) Date of filing: 14.03.2014
(51) Int. Cl.: C07H 21/02, C07H 21/04, C12N 5/04, C12N 5/10, C12N 15/00

(54) **COMPOSITIONS AND METHODS TO CONTROL INSECT PESTS**

(30) Priority: 14.03.2013 US 201361785680 P; 14.03.2013 US 201313831230
(62) Divisional of application: 14767960.9
(71) Applicant: Pioneer Hi-Bred International, Inc., Johnston, Iowa 50131-1014 (US); E. I. du Pont de Nemours and Company, Wilmington, DE 19805 (US)
(72) Inventor: HU, Xu, Johnston, IA Iowa 50131 (US); PRESNAIL, James, Saînt Louis, MO Missouri 63108 (US); RICHTMAN, Nina, Granger, IA Iowa 50109 (US); DIEHN, Scott, West Des Moines, IA Iowa 50265 (US); VAN ALLEN, Michelle, Urbandale, IA Iowa 50322 (US); PROCYK, Lisa, Ankeny, IA Iowa 50023 (US)
(74) Representative: J A Kemp LLP

(57) **Abstract**

Methods and compositions are provided which employ a silencing element that, when ingested by a pest, such as a Coleopteran plant pest or a *Diabrotica* plant pest, decrease the expression of a target sequence in the pest. The present invention provides various target polynucleotides set forth in any one of SEQ ID NOS: disclosed herein, (but not including the forward and reverse primers.) or active variants and fragments thereof, or complements thereof, wherein a decrease in expression of one or more of the sequences in the target pest controls the pest (i.e., has insecticidal activity). Plants, plant parts, bacteria and other host cells comprising the silencing elements or an active variant or fragment thereof of the invention are also provided.

## Description

### CROSS-REFERENCE TO RELATED APPLICATIONS

This Application claims the benefit of U.S. Provisional Application No. 61/785,680, filed on March 14, 2013, and U.S. Utility Application No. 13/831,230, filed on March 14, 2013, filed on both of which are incorporated herein by reference in their entirety.

### FIELD OF THE INVENTION

The present invention relates generally to methods of molecular biology and gene silencing to control pests.

### REFERENCE TO A SEQUENCE LISTING SUBMITTED AS A TEXT FILE VIA EFS-WEB

The Sequence Listing submitted March 14, 2014 as a text file named "36446_0007P1_ST25.txt," created on March 14, 2014, and having a size of 697,429 bytes is hereby incorporated by reference pursuant to 37 C.F.R. § 1.52(e)(5).

### BACKGROUND OF THE INVENTION

Insect pests are a serious problem in agriculture. They destroy millions of acres of staple crops such as corn, soybeans, peas, and cotton. Yearly, these pests cause over $100 billion dollars in crop damage in the U.S. alone. In an ongoing seasonal battle, farmers must apply billions of gallons of synthetic pesticides to combat these pests. Other methods employed in the past delivered insecticidal activity by microorganisms or genes derived from microorganisms expressed in transgenic plants. For example, certain species of microorganisms of the genus *Bacillus* are known to possess pesticidal activity against a broad range of insect pests including *Lepidoptera*, *Diptera*, *Coleoptera*, *Hemiptera*, and others. In fact, microbial pesticides, particularly those obtained from *Bacillus* strains, have played an important role in agriculture as alternatives to chemical pest control. Agricultural scientists have developed crop plants with enhanced insect resistance by genetically engineering crop plants to produce insecticidal proteins from *Bacillus.* For example, corn and cotton plants genetically engineered to produce Cry toxins (see, e.g., Aronson (2002) Cell Mol. Life Sci. 59(3):417-425; Schnepf et al. (1998) Microbiol. Mol. Biol. Rev. 62(3):775-806) are now widely used in American agriculture and have provided the farmer with an alternative to traditional insect-control methods. However, these *Bt* insecticidal proteins only protect plants from a relatively narrow range of pests. Moreover, these modes of insecticidal activity provided varying levels of specificity and, in some cases, caused significant environmental consequences. Thus, there is an immediate need for alternative methods to control pests.

### BRIEF SUMMARY OF THE INVENTION

Methods and compositions are provided which employ a silencing element that, when ingested by a pest, such as Coleopteran plant pest including a *Diabrotica* plant pest, is capable of decreasing the expression of a target sequence in the pest. In specific embodiments, the decrease in expression of the target sequence controls the pest and thereby the methods and compositions are capable of limiting damage to a plant. The present invention provides various target polynucleotides as set forth in SEQ ID NOS: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants or fragments thereof, or complements thereof, wherein a decrease in expression of one or more of the sequences in the target pest controls the pest (i.e., has insecticidal activity). Further provided are silencing elements, which when ingested by the pest, decrease the level of expression of one or more of the target polynucleotides. Plants, plant parts, plant cells, bacteria and other host cells comprising the silencing elements or an active variant or fragment thereof are also provided. Also provided are formulations of sprayable silencing agents for topical applications to pest insects or substrates where pest insects may be found.

In another embodiment, a method for controlling a pest, such as a Coleopteran plant pest or a *Diabrotica* plant pest, is provided. The method comprises feeding to a pest a composition comprising a silencing element, wherein the silencing element, when ingested by the pest, reduces the level of a target sequence in the pest and thereby controls the pest. Further provided are methods to protect a plant from a pest. Such methods comprise introducing into the plant or plant part a silencing element of the invention. When the plant expressing the silencing element is ingested by the pest, the level of the target sequence is decreased and the pest is controlled.

### BRIEF DESCRIPTION OF THE DRAWINGS

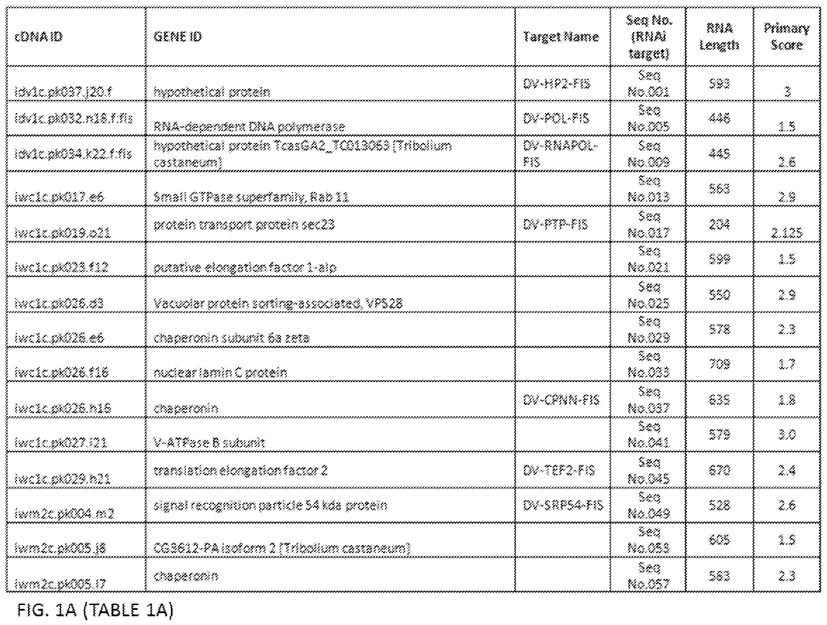

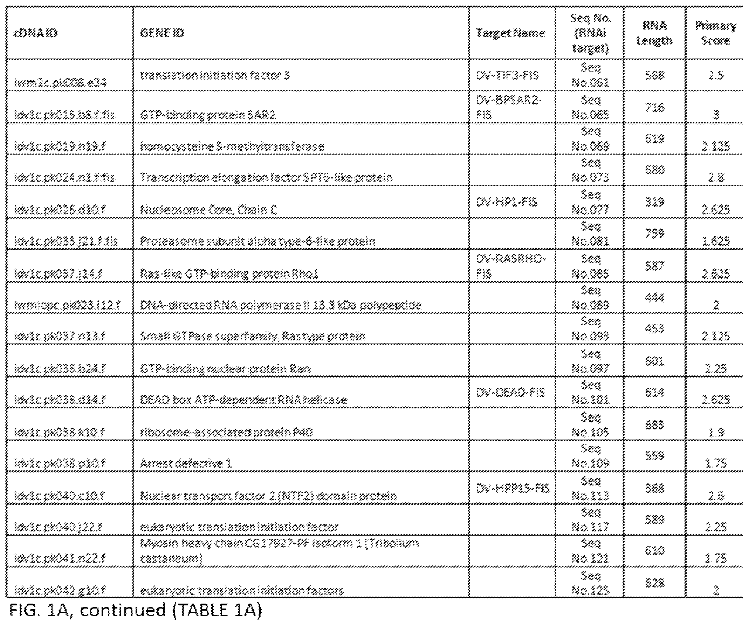

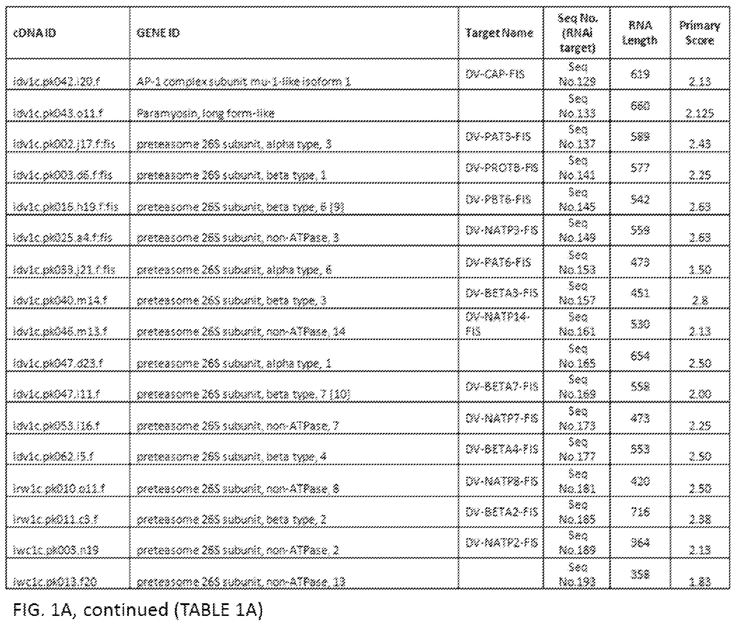

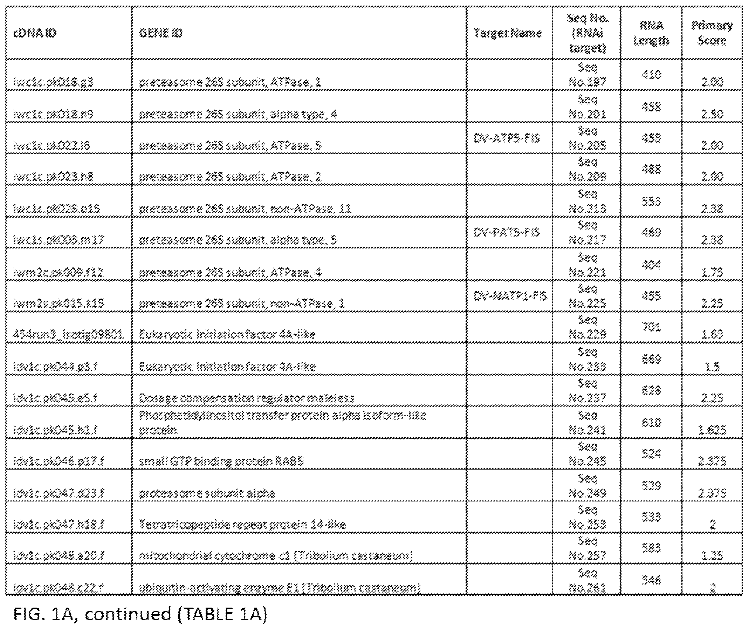

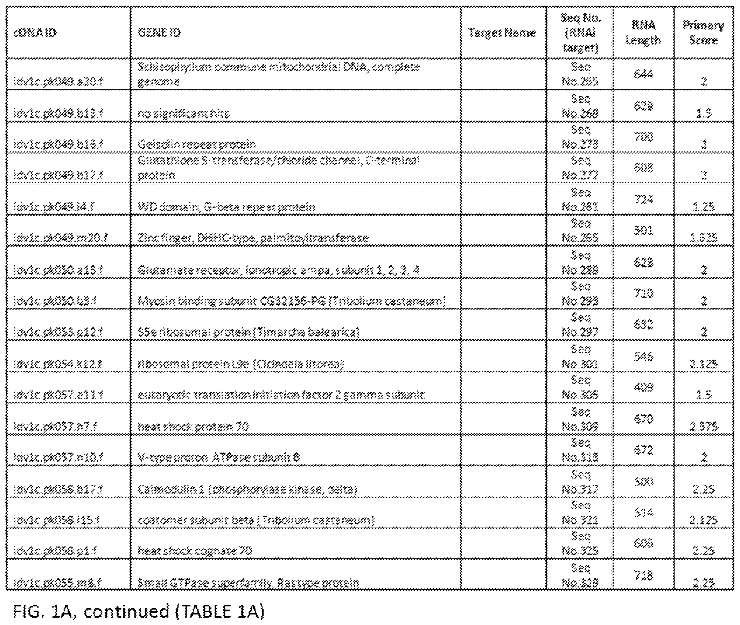

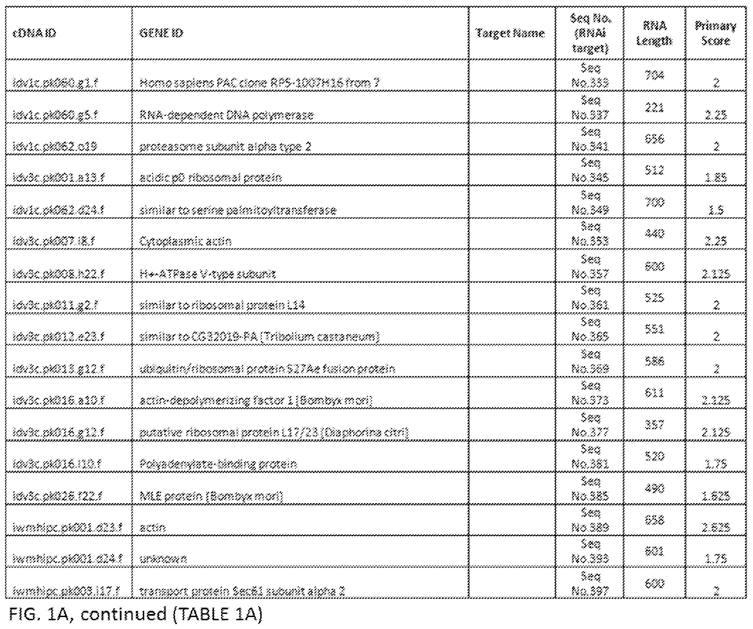

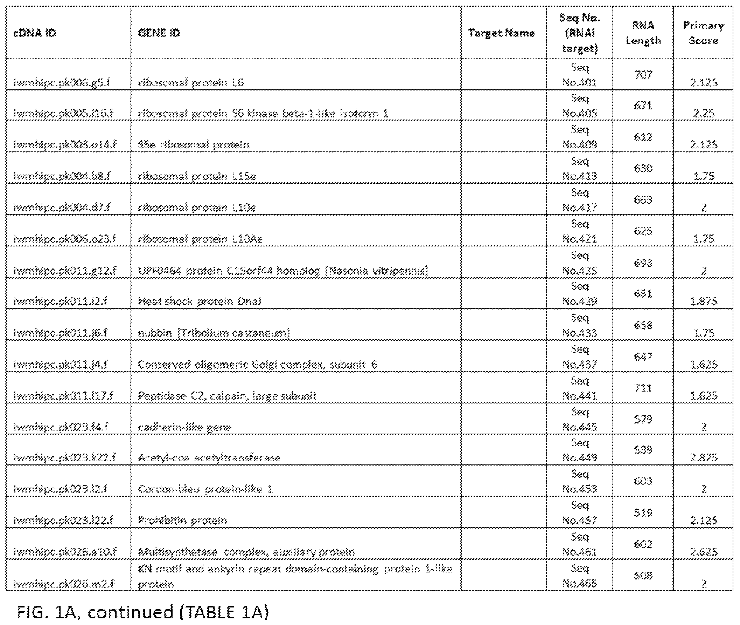

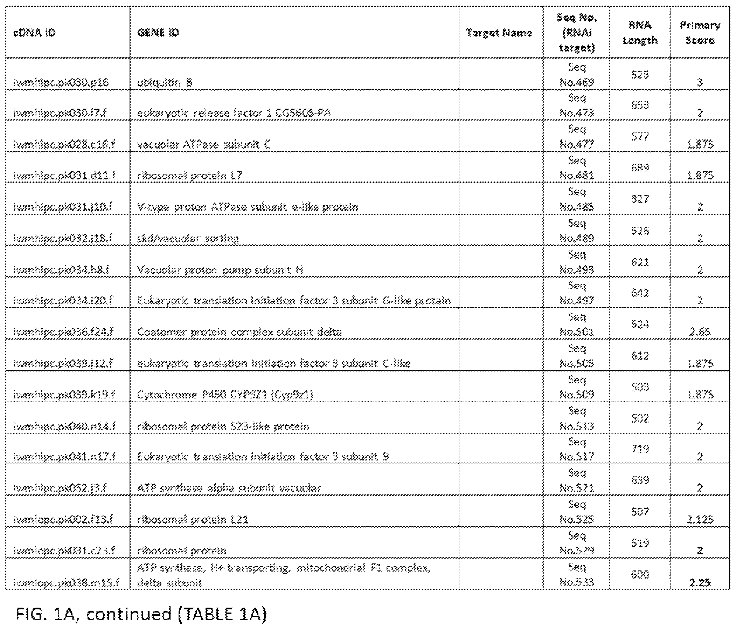

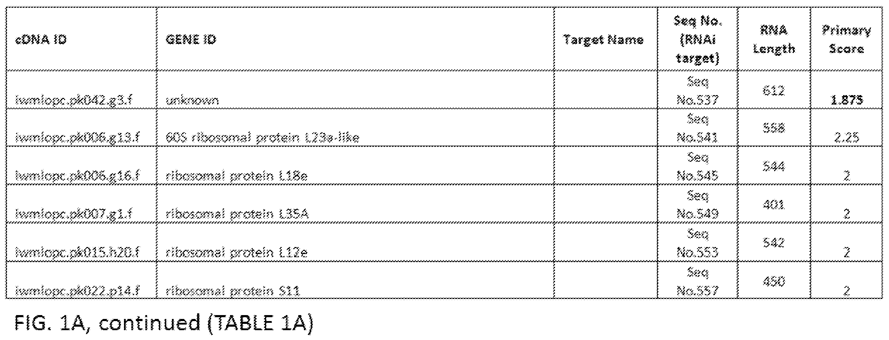

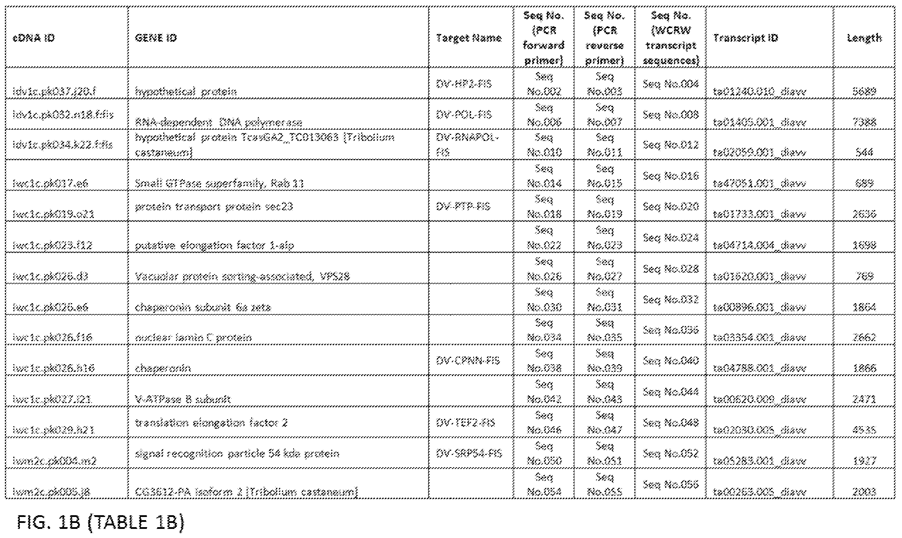

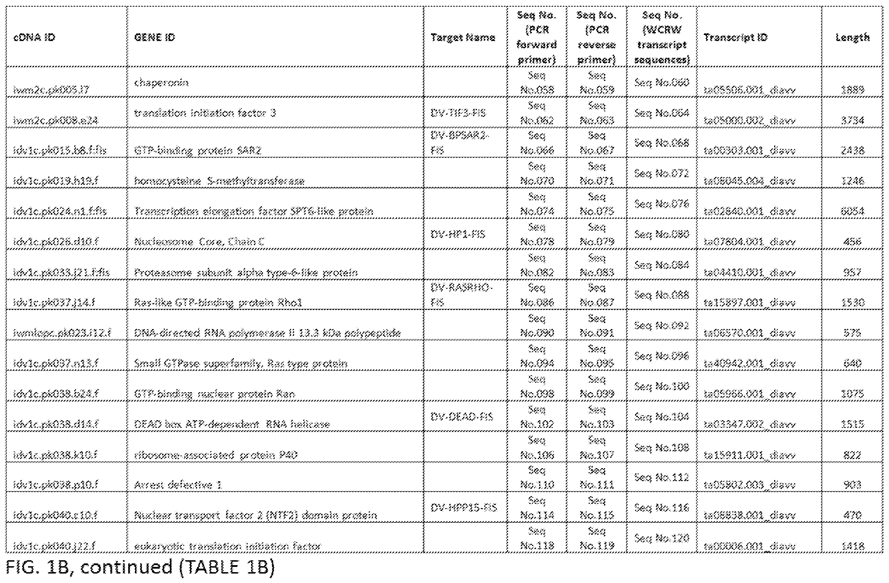

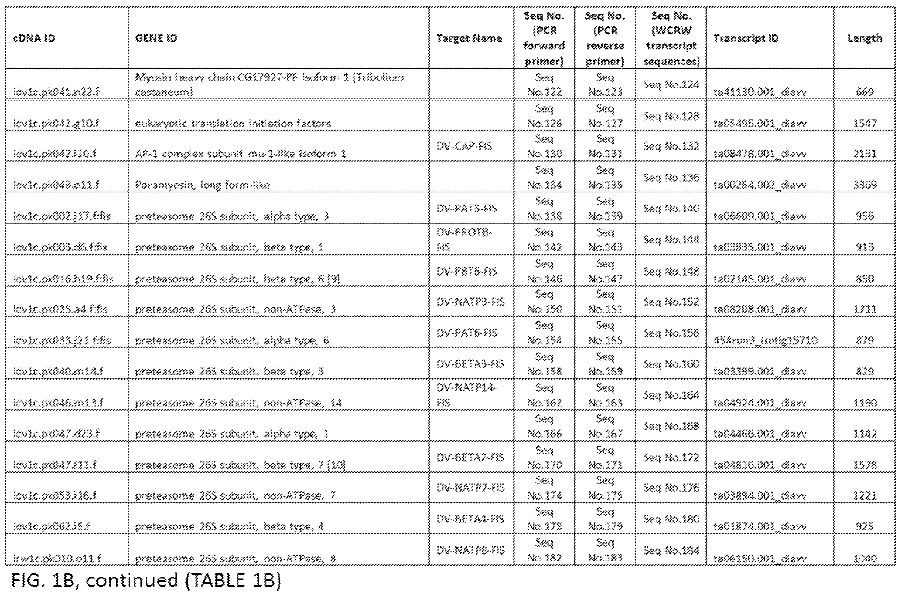

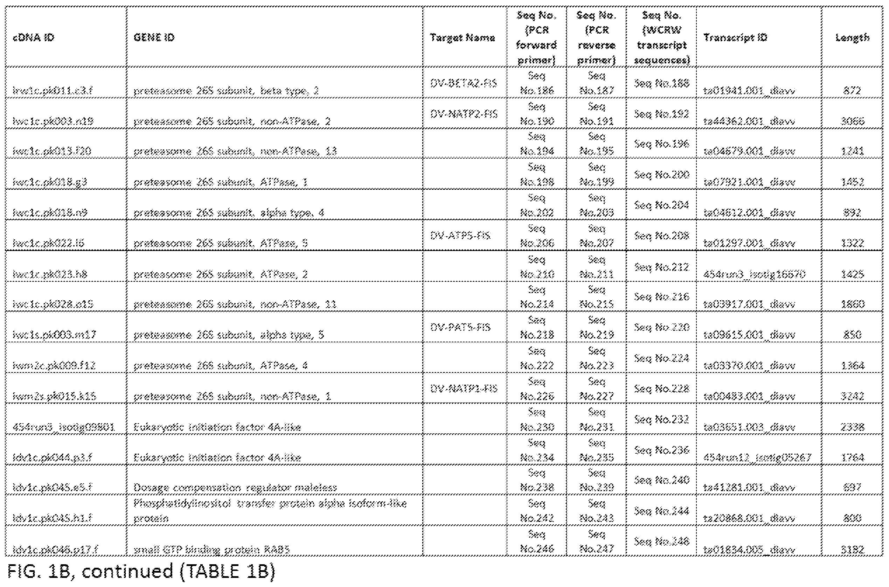

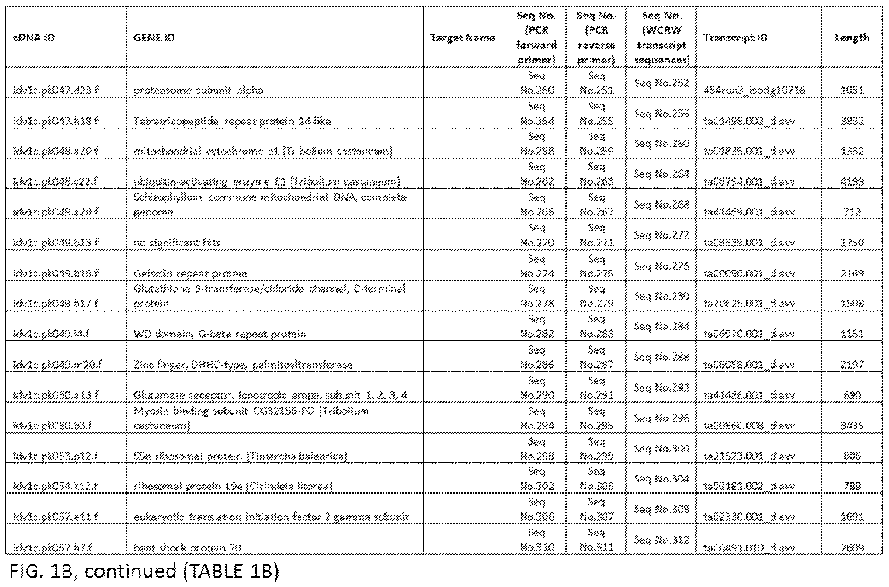

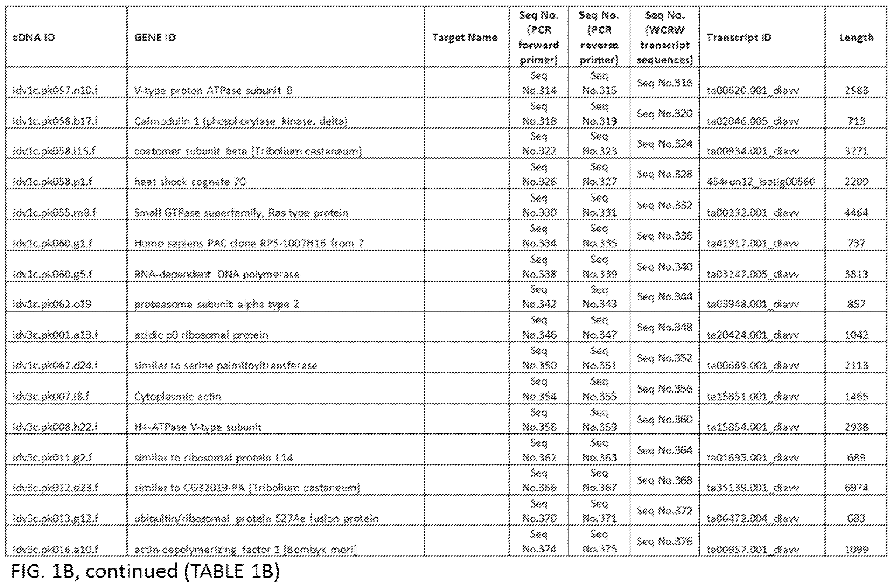

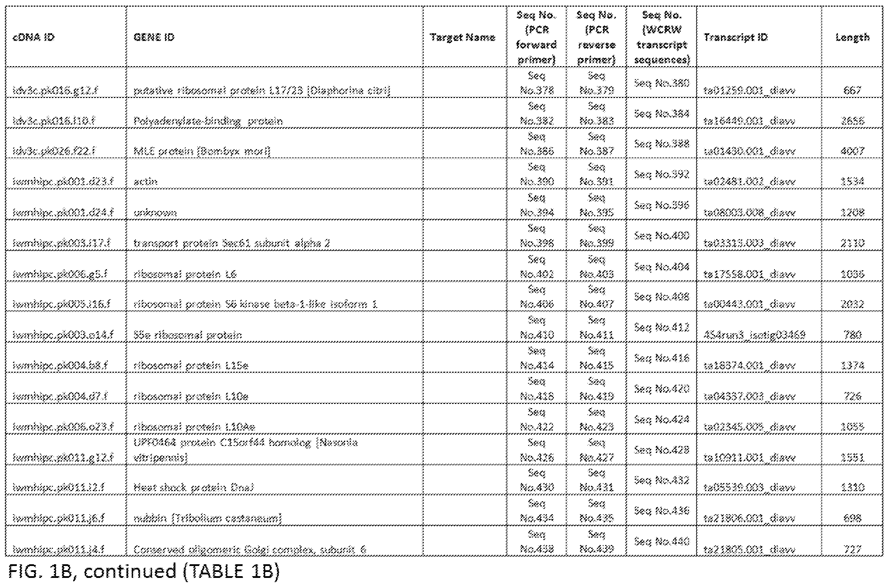

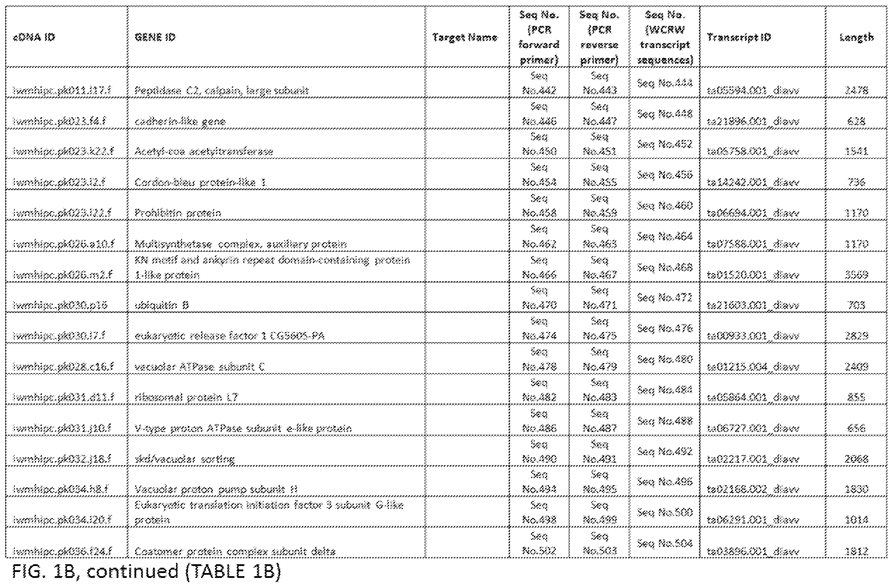

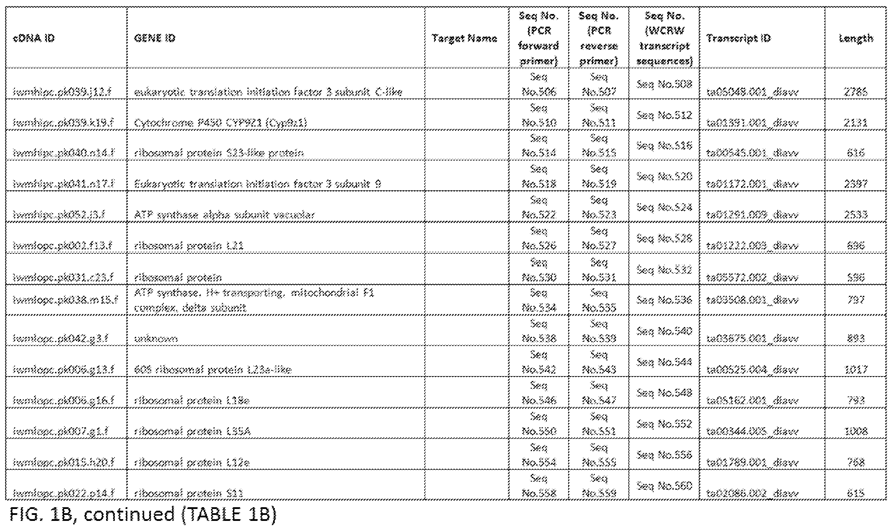

Figure 1 is a table, Tables 1A and 1B, which identifies RNAi active targets in diet assay using dsRNA produced by *in vitro* transcription (IVT).

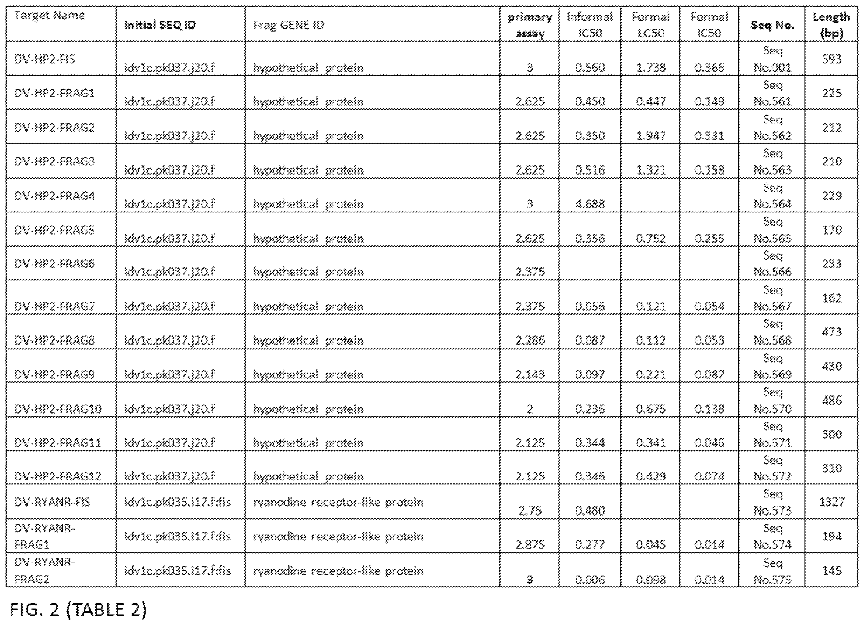

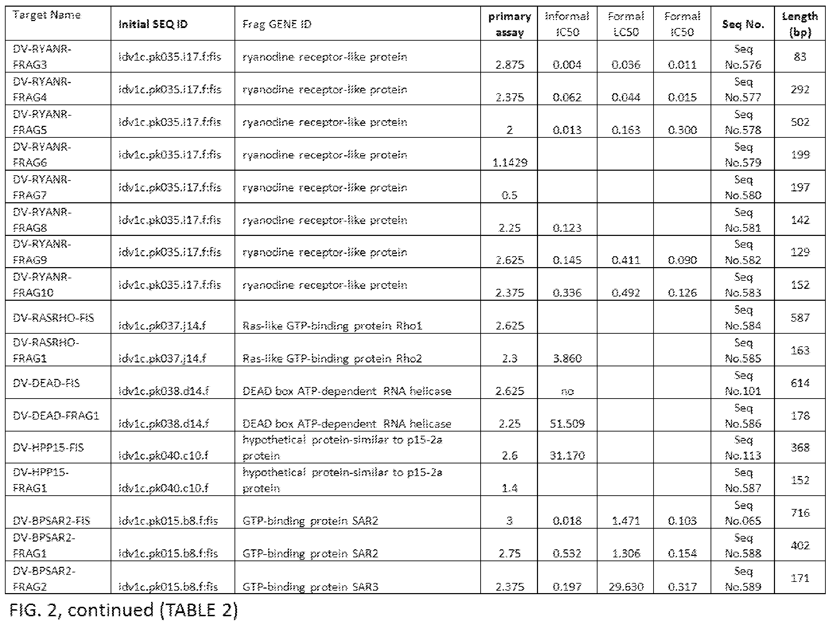

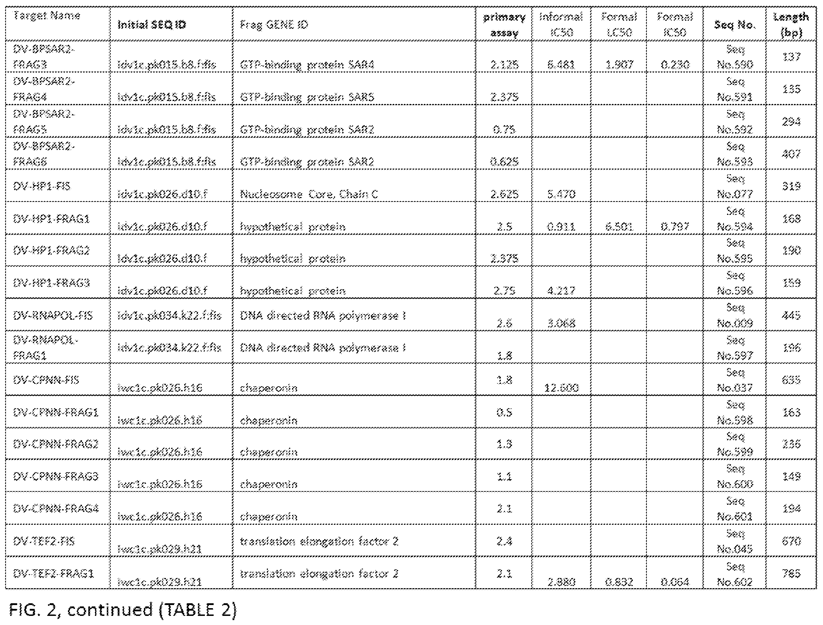

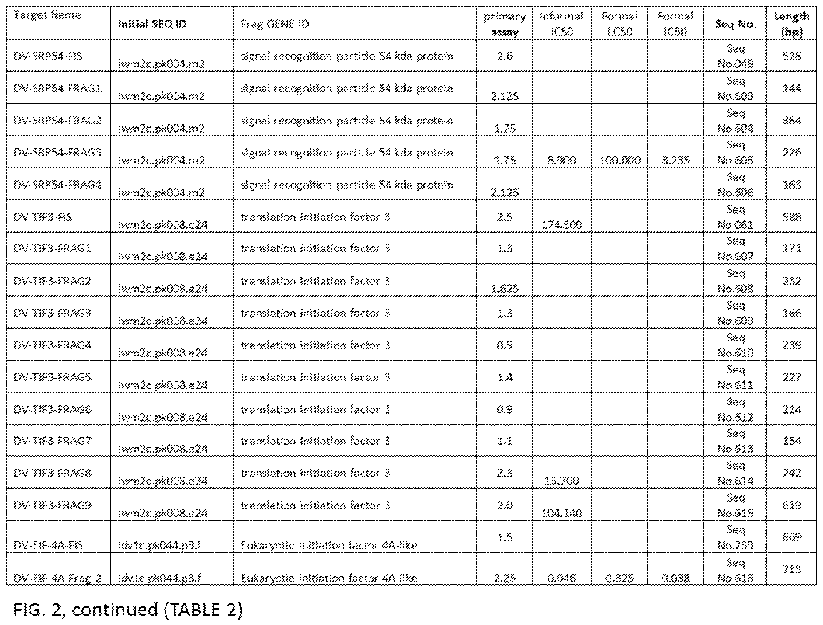

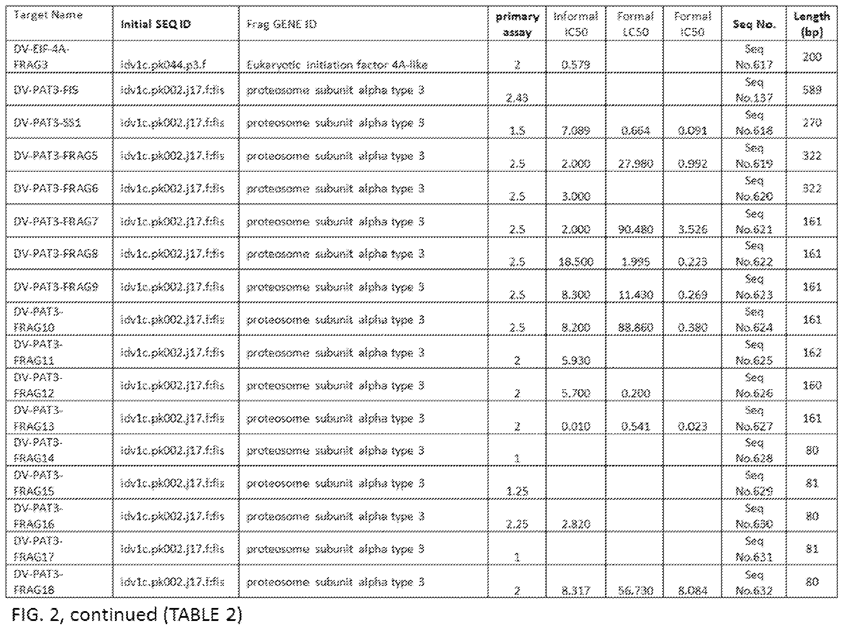

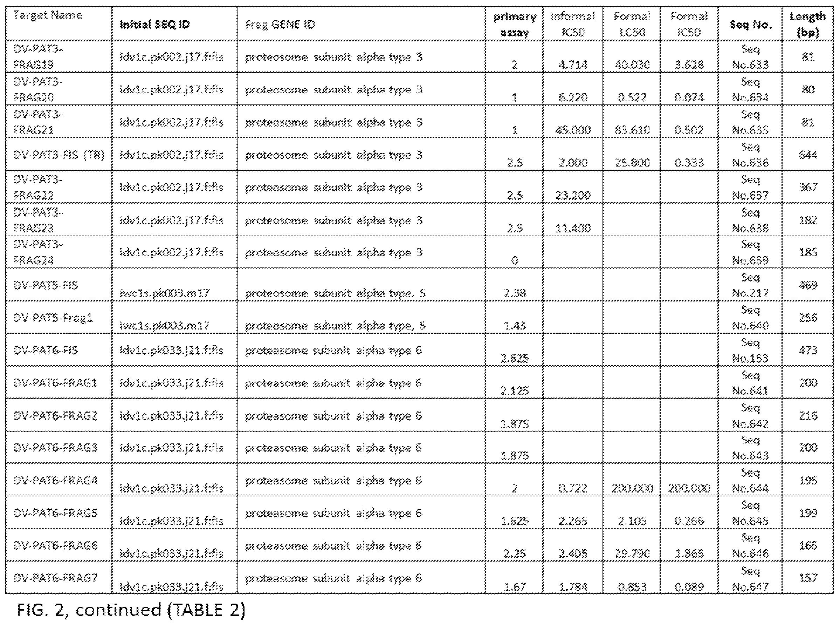

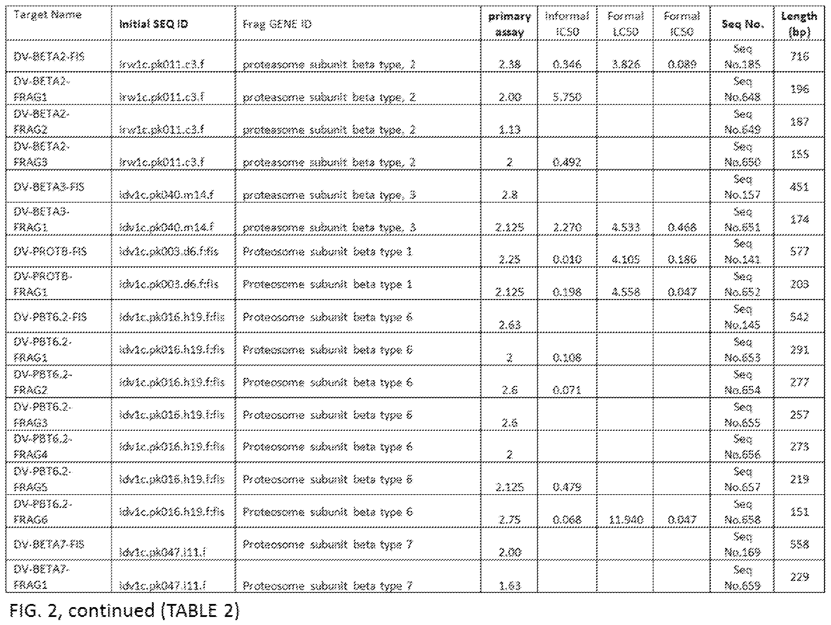

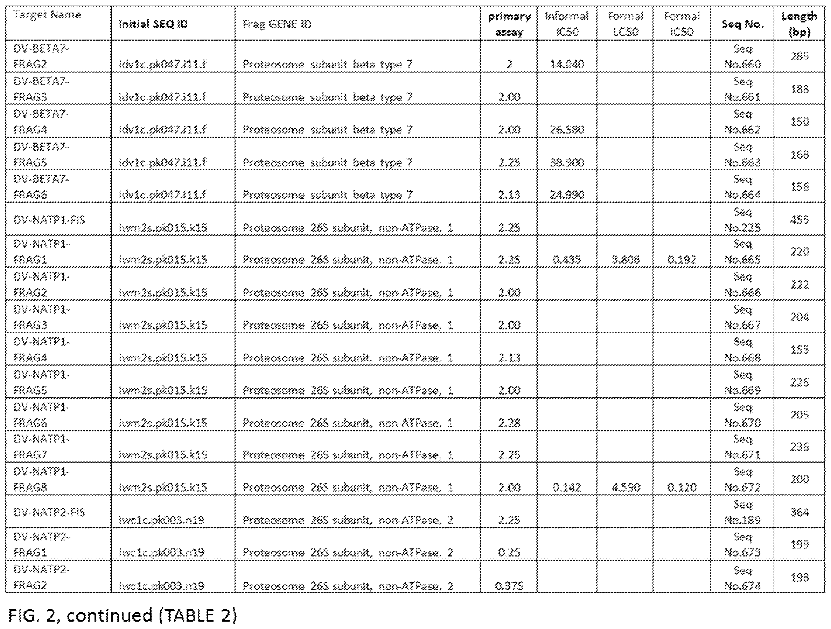

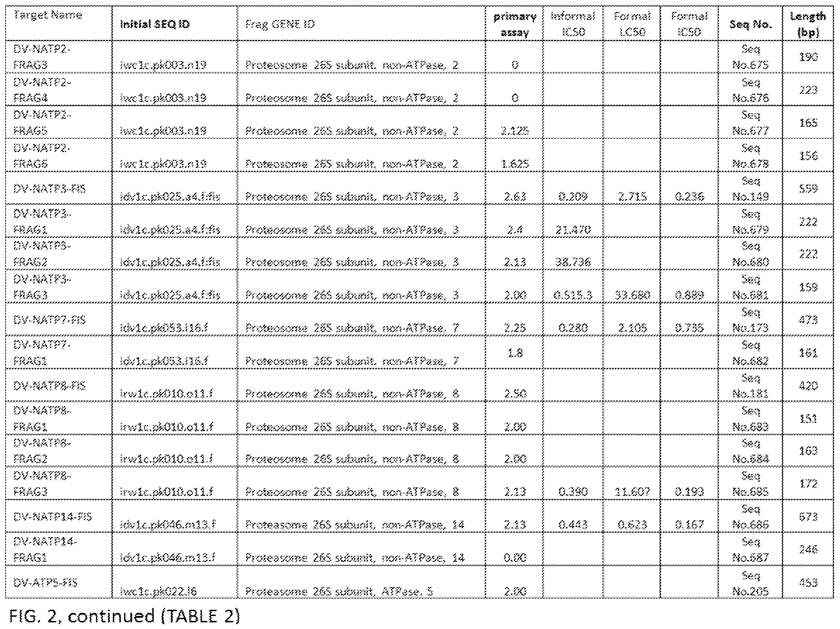

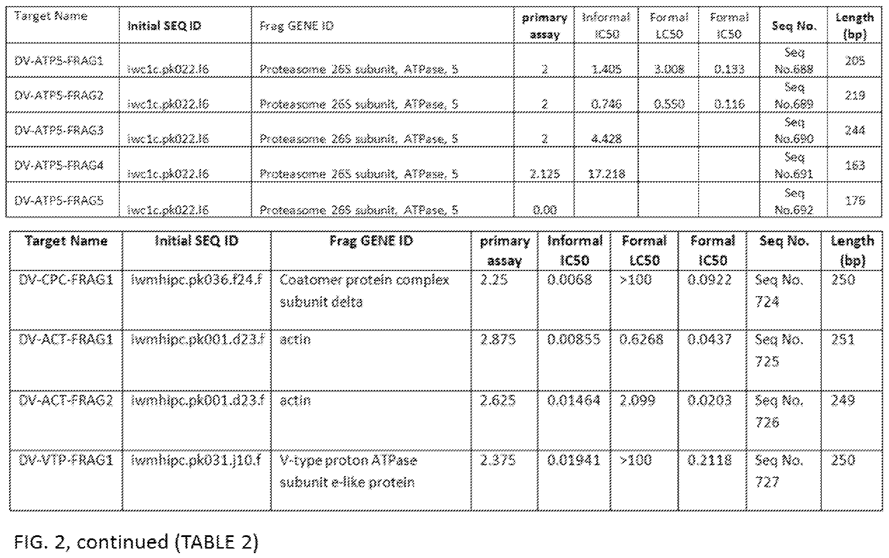

Figure 2 is a table, Table 2, which shows design and identification of RNAi active fragments.

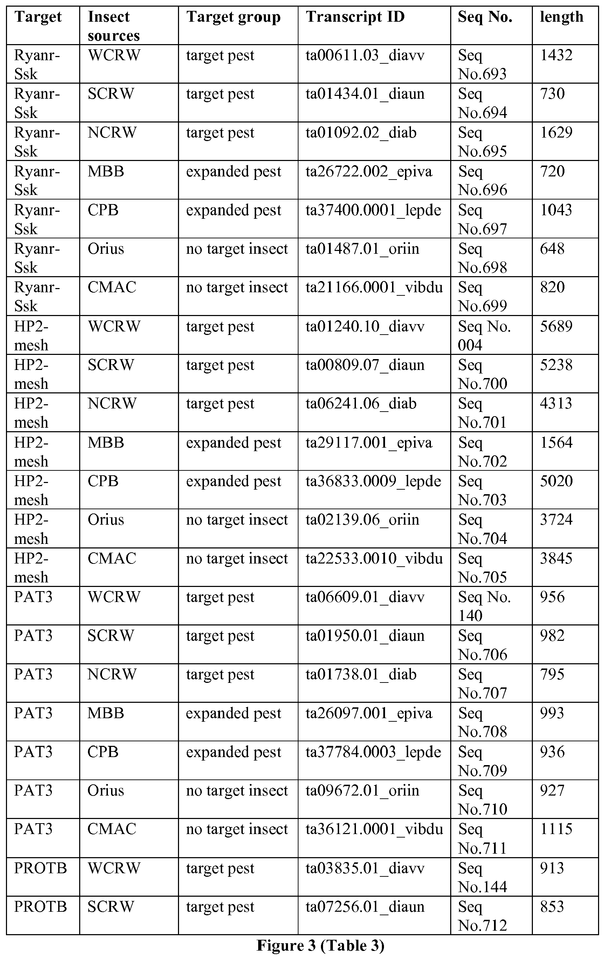

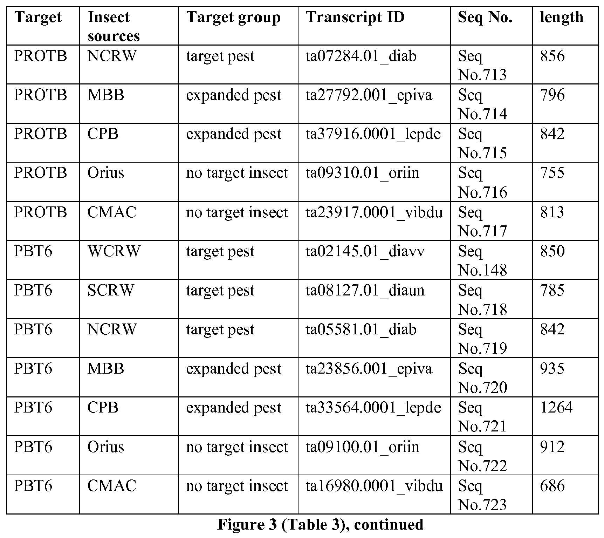

Figure 3 is a table, Table 3 which lists RNAi active targets from target pests, expanded pests and no target insects. Homologous sequences of selected RNAi actives were identified from transcriptome analyses of Western corn rootworm (WCRW, *Diabrotica virgifera*), Northern corn rootworm (NCRW, *Diabrotica barberi*), Southern corn rootworm (SCRW, *Diabrotica undecimpunctata*), Mexican Bean Beetle (MBB, *Epilachna varivestis*), Colorado potato beetle (CPB, *Leptinotarsa decemlineata*), insidious flower bug (Orius, *Orius insidiosus*) and Spotted Lady Beetle (CMAC, *Coleomegilla maculate*).
Figure 4 is a graphic showing a sequence alignment of the amino acid sequences of WCRW Ryanr (SEQ ID NO: 730) and Drosophila Ssk (SEQ ID NO: 731).
Figure 5 is a schematic of PAT3 fragments used in the gene and construct optimization experiment.
Figure 6 is a schematic showing the transgenic region of a representative disclosed construct, PHP58050 (SEQ ID NO: 729).
Figure 7 is a table, Table 4, which shows representative insecticidal activity against corn rootworms for maize plants comprising representative constructs of the present invention. The representative constructs used in the study to transform maize plants were as shown and described in the table.
Figure 8 shows representative Corn Rootworm Nodal Injury Score ("CRWNIS") data for maize with the constructs described in Figure 7.

### DETAILED DESCRIPTION OF THE INVENTION

The present inventions now will be described more fully hereinafter with reference to the accompanying drawings, in which some, but not all embodiments of the inventions are shown. Indeed, these inventions may be embodied in many different forms and should not be construed as limited to the embodiments set forth herein; rather, these embodiments are provided so that this disclosure will satisfy applicable legal requirements. Like numbers refer to like elements throughout.

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims. Although specific terms are employed herein, they are used in a generic and descriptive sense only and not for purposes of limitation.

### I. Overview

Frequently, RNAi discovery methods rely on evaluation of known classes of sensitive genes (transcription factors, housekeeping genes etc.). In contrast, the target polynucleotides set forth herein were identified based solely on high throughput screens of all singletons and representatives of all gene clusters from a cDNA library of neonate and/or 3^{rd} instar midgut western corn rootworms. This screen allowed for the discovery of many novel sequences, many of which have extremely low or no homology to known sequences. This method provided the advantage of having no built in bias to genes that are frequently highly conserved across taxa. As a result, many novel targets for RNAi as well as known genes not previously shown to be sensitive to RNAi have been identified.

As such, methods and compositions are provided which employ one or more silencing elements that, when ingested by a pest, such as a Coleopteran plant pest or a *Diabrotica* plant pest, is capable of decreasing the expression of a target sequence in the pest. In specific embodiments, the decrease in expression of the target sequence controls the pest and thereby the methods and compositions are capable of limiting damage to a plant or plant part. The present invention provides target polynucleotides as set forth in SEQ ID NOS: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof, including, for example, SEQ ID NOS: 1, 9, 37, 45, 49, 61, 65, 77, 101, 113, 137, 141, 145, 149, 153, 157, 169, 173, 181, 185, 189, 205, 217, 225,233, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, and active variants and fragments thereof, and complements thereof, and SEQ ID NOS: 4, 140, 144, 148, 693, 694, 695, 696, 697, 700, 701, 702, 703, 706, 707, 708, 709, 712, 713, 714, 715, 718, 719, 720, 721, 724, 725, 726, 727, 728, and active variants and fragments thereof, and complements thereof. Silencing elements comprising sequences, complementary sequences, active fragments or variants of these target polynucleotides are provided which, when ingested by or when contacting the pest, decrease the expression of one or more of the target sequences and thereby controls the pest (i.e., has insecticidal activity).

As used herein, by "controlling a pest" or "controls a pest" is intended any affect on a pest that results in limiting the damage that the pest causes. Controlling a pest includes, but is not limited to, killing the pest, inhibiting development of the pest, altering fertility or growth of the pest in such a manner that the pest provides less damage to the plant, decreasing the number of offspring produced, producing less fit pests, producing pests more susceptible to predator attack, or deterring the pests from eating the plant.

Reducing the level of expression of the target polynucleotide or the polypeptide encoded thereby, in the pest results in the suppression, control, and/or killing the invading pest. Reducing the level of expression of the target sequence of the pest will reduce the pest damage by at least about 2% to at least about 6%, at least about 5% to about 50%, at least about 10% to about 60%, at least about 30% to about 70%, at least about 40% to about 80%, or at least about 50% to about 90% or greater. Hence, the methods of the invention can be utilized to control pests, particularly, Coleopteran plant pests or a *Diabrotica* plant pest.

Assays measuring the control of a pest are commonly known in the art, as are methods to record nodal injury score. See, for example, Oleson et al. (2005) J. Econ. Entomol. 98:1-8. See, for example, the examples below.

The invention is drawn to compositions and methods for protecting plants from a plant pest, such as Coleopteran plant pests or *Diabrotica* plant pests or inducing resistance in a plant to a plant pest, such as Coleopteran plant pests or *Diabrotica* plant pests. As used herein "Coleopteran plant pest" is used to refer to any member of the Coleoptera order. Other plant pests that may be targeted by the methods and compositions of the present invention include, but are not limited to Mexican Bean Beetle (*Epilachna varivestis*), and Colorado potato beetle (*Leptinotarsa decemlineata*),

As used herein, the term "*Diabrotica* plant pest" is used to refer to any member of the *Diabrotica* genus. Accordingly, the compositions and methods are also useful in protecting plants against any *Diabrotica* plant pest including, for example, *Diabrotica adelpha; Diabrotica amecameca; Diabrotica balteata; Diabrotica barberi; Diabrotica biannularis; Diabrotica cristata; Diabrotica decempunctata; Diabrotica dissimilis; Diabrotica lemniscata; Diabrotica limitata* (including, for example, *Diabrotica limitata quindecimpuncata*); *Diabrotica longicornis; Diabrotica nummularis; Diabrotica porracea; Diabrotica scutellata; Diabrotica sexmaculata; Diabrotica speciosa* (including, for example, *Diabrotica speciosa speciosa); Diabrotica tibialis; Diabrotica undecimpunctata* (including, for example, Southern corn rootworm (*Diabrotica undecimpunctata*), *Diabrotica undecimpunctata duodecimnotata; Diabrotica undecimpunctata howardi* (spotted cucumber beetle); *Diabrotica undecimpunctata undecimpunctata* (western spotted cucumber beetle)); *Diabrotica virgifera* (including, for example, *Diabrotica virgifera virgifera* (western corn rootworm) and *Diabrotica virgifera zeae* (Mexican corn rootworm)); *Diabrotica viridula; Diabrotica wartensis; Diabrotica sp. JJG335; Diabrotica sp. JJG336; Diabrotica sp. JJG341; Diabrotica sp. JJG356; Diabrotica sp. JJG362*; and, *Diabrotica sp. JJG365.*

In specific embodiments, the *Diabrotica* plant pest comprises *D. virgifera virgifera*, *D. barberi*, *D. virgifera zeae, D. speciosa,* or *D. undecimpunctata howardi.*

### II. Target Sequences

As used herein, a "target sequence" or "target polynucleotide" comprises any sequence in the pest that one desires to reduce the level of expression thereof. In specific embodiments, decreasing the level of the target sequence in the pest controls the pest. For instance, the target sequence may be essential for growth and development. While the target sequence can be expressed in any tissue of the pest, in specific embodiments, the sequences targeted for suppression in the pest are expressed in cells of the gut tissue of the pest, cells in the midgut of the pest, and cells lining the gut lumen or the midgut. Such target sequences can be involved in, for example, gut cell metabolism, growth or differentiation. Non-limiting examples of target sequences of the invention include a polynucleotide set forth in SEQ ID NOS: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof, including, for example, SEQ ID NOS: 1, 9, 37, 45, 49, 61, 65, 77, 101, 113, 137, 141, 145, 149, 153, 157, 169, 173, 181, 185, 189, 205, 217, 225,233, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, and active variants and fragments thereof, and complements thereof, and SEQ ID NOS: 4, 140, 144, 148, 693, 694, 695, 696, 697, 700, 701, 702, 703, 706, 707, 708, 709, 712, 713, 714, 715, 718, 719, 720, 721, 724, 725, 726, 727, 728, and active variants and fragments thereof, and complements thereof. As exemplified elsewhere herein, decreasing the level of expression of one or more of these target sequences in a Coleopteran plant pest or a *Diabrotica* plant pest controls the pest.

### III. Silencing Elements

By "silencing element" is intended a polynucleotide which when contacted by or ingested by a pest, is capable of reducing or eliminating the level or expression of a target polynucleotide or the polypeptide encoded thereby. The silencing element employed can reduce or eliminate the expression level of the target sequence by influencing the level of the target RNA transcript or, alternatively, by influencing translation and thereby affecting the level of the encoded polypeptide. Methods to assay for functional silencing elements that are capable of reducing or eliminating the level of a sequence of interest are disclosed elsewhere herein. A single polynucleotide employed in the methods of the invention can comprise one or more silencing elements to the same or different target polynucleotides. The silencing element can be produced *in vivo* (i.e., in a host cell such as a plant or microorganism) or *in vitro.*

In specific embodiments, a silencing element may comprise a chimeric construction molecule comprising two or more sequences of the present invention. For example, the chimeric construction may be a hairpin or dsRNA as disclosed herein. A chimera may comprise two or more sequences of the present invention. In one embodiment, a chimera contemplates two complementary sequences set forth herein having some degree of mismatch between the complementary sequences such that the two sequences are not perfect complements of one another. Providing at least two different sequences in a single silencing element may allow for targeting multiple genes using one silencing element and/or for example, one expression cassette. Targeting multiple genes may allow for slowing or reducing the possibility of resistance by the pest, and providing the multiple targeting ability in one expressed molecule may reduce the expression burden of the transformed plant or plant product, or provide topical treatments that are capable of targeting multiple hosts with one application.

In specific embodiments, the target sequence is not endogenous to the plant. In other embodiments, while the silencing element controls pests, preferably the silencing element has no effect on the normal plant or plant part.

As discussed in further detail below, silencing elements can include, but are not limited to, a sense suppression element, an antisense suppression element, a double stranded RNA, a siRNA, a amiRNA, a miRNA, or a hairpin suppression element. Silencing elements of the present invention may comprise a chimera where two or more sequences of the present invention or active fragments or variants, or complements thereof, are found in the same RNA molecule. Further, a sequence of the present invention or active fragment or variant, or complement thereof, may be present as more than one copy in a DNA construct, silencing element, DNA molecule or RNA molecule. In a hairpin or dsRNA molecule, the location of a sense or antisense sequence in the molecule, for example, in which sequence is transcribed first or is located on a particular terminus of the RNA molecule, is not limiting to the invention, and the invention is not to be limited by disclosures herein of a particular location for such a sequence. Non-limiting examples of silencing elements that can be employed to decrease expression of these target Coleopteran plant pest sequences or *Diabrotica* plant pest sequences comprise fragments and variants of the sense or antisense sequence or consists of the sense or antisense sequence of the sequence set forth in SEQ ID NOS: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof, including, for example, SEQ ID NOS: 1, 9, 37, 45, 49, 61, 65, 77, 101, 113, 137, 141, 145, 149, 153, 157, 169, 173, 181, 185, 189, 205, 217, 225,233, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, and active variants and fragments thereof, and complements thereof, and SEQ ID NOS: 4, 140, 144, 148, 693, 694, 695, 696, 697, 700, 701, 702, 703, 706, 707, 708, 709, 712, 713, 714, 715, 718, 719, 720, 721, 724, 725, 726, 727, 728, and active variants and fragments thereof, and complements thereof. The silencing element can further comprise additional sequences that advantageously effect transcription and/or the stability of a resulting transcript. For example, the silencing elements can comprise at least one thymine residue at the 3' end. This can aid in stabilization. Thus, the silencing elements can have at least 1, 2, 3, 4, 5, 6, 7, 8, 9, 10 or more thymine residues at the 3' end. As discussed in further detail below, enhancer suppressor elements can also be employed in conjunction with the silencing elements disclosed herein.

By "reduces" or "reducing" the expression level of a polynucleotide or a polypeptide encoded thereby is intended to mean, the polynucleotide or polypeptide level of the target sequence is statistically lower than the polynucleotide level or polypeptide level of the same target sequence in an appropriate control pest which is not exposed to (i.e., has not ingested or come into contact with) the silencing element. In particular embodiments of the invention, reducing the polynucleotide level and/or the polypeptide level of the target sequence in a pest according to the invention results in less than 95%, less than 90%, less than 80%, less than 70%, less than 60%, less than 50%, less than 40%, less than 30%, less than 20%, less than 10%, or less than 5% of the polynucleotide level, or the level of the polypeptide encoded thereby, of the same target sequence in an appropriate control pest. Methods to assay for the level of the RNA transcript, the level of the encoded polypeptide, or the activity of the polynucleotide or polypeptide are discussed elsewhere herein.

### i. Sense Suppression Elements

As used herein, a "sense suppression element" comprises a polynucleotide designed to express an RNA molecule corresponding to at least a part of a target messenger RNA in the "sense" orientation. Expression of the RNA molecule comprising the sense suppression element reduces or eliminates the level of the target polynucleotide or the polypeptide encoded thereby. The polynucleotide comprising the sense suppression element may correspond to all or part of the sequence of the target polynucleotide, all or part of the 5' and/or 3' untranslated region of the target polynucleotide, all or part of the coding sequence of the target polynucleotide, or all or part of both the coding sequence and the untranslated regions of the target polynucleotide.

Typically, a sense suppression element has substantial sequence identity to the target polynucleotide, typically greater than about 65% sequence identity, greater than about 85% sequence identity, about 90%, 91%, 92%, 93%, 94%, 95%, 96%, 97%, 98% or 99% sequence identity. See, U.S. Patent Nos. 5,283,184 and 5,034,323; herein incorporated by reference. The sense suppression element can be any length so long as it allows for the suppression of the targeted sequence. The sense suppression element can be, for example, 15, 16, 17, 18, 19, 20, 22, 25, 30, 50, 100, 150, 200, 250, 300, 350, 400, 450, 500, 600, 700, 900, 1000, 1100, 1200, 1300 nucleotides or longer of the target polynucleotides set forth in any of SEQ ID NOS: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof, including, for example, SEQ ID NOS: 1, 9, 37, 45, 49, 61, 65, 77, 101, 113, 137, 141, 145, 149, 153, 157, 169, 173, 181, 185, 189, 205, 217, 225,233, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, and active variants and fragments thereof, and complements thereof, and SEQ ID NOS: 4, 140, 144, 148, 693, 694, 695, 696, 697, 700, 701, 702, 703, 706, 707, 708, 709, 712, 713, 714, 715, 718, 719, 720, 721, 724, 725, 726, 727, 728, and active variants and fragments thereof, and complements thereof. In other embodiments, the sense suppression element can be, for example, about 15-25, 19-35, 19-50, 25-100, 100-150, 150-200, 200-250, 250-300, 300-350, 350-400, 450-500, 500-550, 550-600, 600-650, 650-700, 700-750, 750-800, 800-850, 850-900, 900-950, 950-1000, 1000-1050, 1050-1100, 1100-1200, 1200-1300, 1300-1400, 1400-1500, 1500-1600, 1600-1700, 1700-1800 nucleotides or longer of the target polynucleotides set forth in any of SEQ ID NOS: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof, including, for example, SEQ ID NOS: 1, 9, 37, 45, 49, 61, 65, 77, 101, 113, 137, 141, 145, 149, 153, 157, 169, 173, 181, 185, 189, 205, 217, 225,233, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, and active variants and fragments thereof, and complements thereof, and SEQ ID NOS: 4, 140, 144, 148, 693, 694, 695, 696, 697, 700, 701, 702, 703, 706, 707, 708, 709, 712, 713, 714, 715, 718, 719, 720, 721, 724, 725, 726, 727, 728, and active variants and fragments thereof, and complements thereof.

### ii. Antisense Suppression Elements

As used herein, an "antisense suppression element" comprises a polynucleotide which is designed to express an RNA molecule complementary to all or part of a target messenger RNA. Expression of the antisense RNA suppression element reduces or eliminates the level of the target polynucleotide. The polynucleotide for use in antisense suppression may correspond to all or part of the complement of the sequence encoding the target polynucleotide, all or part of the complement of the 5' and/or 3' untranslated region of the target polynucleotide, all or part of the complement of the coding sequence of the target polynucleotide, or all or part of the complement of both the coding sequence and the untranslated regions of the target polynucleotide. In addition, the antisense suppression element may be fully complementary (i.e., 100% identical to the complement of the target sequence) or partially complementary (i.e., less than 100% identical to the complement of the target sequence) to the target polynucleotide. In specific embodiments, the antisense suppression element comprises at least 85%, 90%, 91%, 92%, 93%, 94%, 95%, 96%, 97%, 98%, or 99% sequence complementarity to the target polynucleotide. Antisense suppression may be used to inhibit the expression of multiple proteins in the same plant. See, for example, U.S. Patent No. 5,942,657. Furthermore, the antisense suppression element can be complementary to a portion of the target polynucleotide. Generally, sequences of at least 15, 16, 17, 18, 19, 20, 22, 25, 50, 100, 200, 300, 400, 450 nucleotides or greater of the sequence set forth in any of SEQ ID NOS: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof, including, for example, SEQ ID NOS: 1, 9, 37, 45, 49, 61, 65, 77, 101, 113, 137, 141, 145, 149, 153, 157, 169, 173, 181, 185, 189, 205, 217, 225,233, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, and active variants and fragments thereof, and complements thereof, and SEQ ID NOS: 4, 140, 144, 148, 693, 694, 695, 696, 697, 700, 701, 702, 703, 706, 707, 708, 709, 712, 713, 714, 715, 718, 719, 720, 721, 724, 725, 726, 727, 728, and active variants and fragments thereof, and complements thereof may be used. Methods for using antisense suppression to inhibit the expression of endogenous genes in plants are described, for example, in Liu et al (2002) Plant Physiol. 129:1732-1743 and U.S. Patent Nos. 5,759,829 and 5,942,657, each of which is herein incorporated by reference.

### iii. Double Stranded RNA Suppression Element

A "double stranded RNA silencing element" or "dsRNA" comprises at least one transcript that is capable of forming a dsRNA either before or after ingestion by a pest. Thus, a "dsRNA silencing element" includes a dsRNA, a transcript or polyribonucleotide capable of forming a dsRNA or more than one transcript or polyribonucleotide capable of forming a dsRNA. "Double stranded RNA" or "dsRNA" refers to a polyribonucleotide structure formed either by a single self-complementary RNA molecule or a polyribonucleotide structure formed by the expression of at least two distinct RNA strands. The dsRNA molecule(s) employed in the methods and compositions of the invention mediate the reduction of expression of a target sequence, for example, by mediating RNA interference "RNAi" or gene silencing in a sequence-specific manner. In the context of the present invention, the dsRNA is capable of reducing or eliminating the level or expression of a target polynucleotide or the polypeptide encoded thereby in a pest.

The dsRNA can reduce or eliminate the expression level of the target sequence by influencing the level of the target RNA transcript, by influencing translation and thereby affecting the level of the encoded polypeptide, or by influencing expression at the pre-transcriptional level (i.e., via the modulation of chromatin structure, methylation pattern, etc., to alter gene expression). See, for example, Verdel et al. (2004) Science 303:672-676; Pal-Bhadra et al. (2004) Science 303:669-672; Allshire (2002) Science 297:1818-1819; Volpe et al. (2002) Science 297:1833-1837; Jenuwein (2002) Science 297:2215-2218; and Hall et al. (2002) Science 297:2232-2237. Methods to assay for functional dsRNA that are capable of reducing or eliminating the level of a sequence of interest are disclosed elsewhere herein. Accordingly, as used herein, the term "dsRNA" is meant to encompass other terms used to describe nucleic acid molecules that are capable of mediating RNA interference or gene silencing, including, for example, short-interfering RNA (siRNA), double-stranded RNA (dsRNA), micro-RNA (miRNA), hairpin RNA, short hairpin RNA (shRNA), post-transcriptional gene silencing RNA (ptgsRNA), and others.

In specific embodiments, at least one strand of the duplex or double-stranded region of the dsRNA shares sufficient sequence identity or sequence complementarity to the target polynucleotide to allow for the dsRNA to reduce the level of expression of the target sequence. As used herein, the strand that is complementary to the target polynucleotide is the "antisense strand" and the strand homologous to the target polynucleotide is the "sense strand."

In another embodiment, the dsRNA comprises a hairpin RNA. A hairpin RNA comprises an RNA molecule that is capable of folding back onto itself to form a double stranded structure. Multiple structures can be employed as hairpin elements. In specific embodiments, the dsRNA suppression element comprises a hairpin element which comprises in the following order, a first segment, a second segment, and a third segment, where the first and the third segment share sufficient complementarity to allow the transcribed RNA to form a double-stranded stem-loop structure.

The "second segment" of the hairpin comprises a "loop" or a "loop region." These terms are used synonymously herein and are to be construed broadly to comprise any nucleotide sequence that confers enough flexibility to allow self-pairing to occur between complementary regions of a polynucleotide (i.e., segments 1 and 3 which form the stem of the hairpin). For example, in some embodiments, the loop region may be substantially single stranded and act as a spacer between the self-complementary regions of the hairpin stem-loop. In some embodiments, the loop region can comprise a random or nonsense nucleotide sequence and thus not share sequence identity to a target polynucleotide. In other embodiments, the loop region comprises a sense or an antisense RNA sequence or fragment thereof that shares identity to a target polynucleotide. See, for example, International Patent Publication No. WO 02/00904, herein incorporated by reference. In specific embodiments, the loop region can be optimized to be as short as possible while still providing enough intramolecular flexibility to allow the formation of the base-paired stem region. Accordingly, the loop sequence is generally less than 1000, 900, 800, 700, 600, 500, 400, 300, 200, 100, 50, 25, 20, 19, 18, 17, 16, 15, 10 nucleotides or less.

The "first" and the "third" segment of the hairpin RNA molecule comprise the base-paired stem of the hairpin structure. The first and the third segments are inverted repeats of one another and share sufficient complementarity to allow the formation of the base-paired stem region. In specific embodiments, the first and the third segments are fully complementary to one another. Alternatively, the first and the third segment may be partially complementary to each other so long as they are capable of hybridizing to one another to form a base-paired stem region. The amount of complementarity between the first and the third segment can be calculated as a percentage of the entire segment. Thus, the first and the third segment of the hairpin RNA generally share at least 50%, 60%, 70%, 80%, 85%, 90%, 91%, 92%, 93%, 94%, 95%, 96%, 97%, 98%, 99%, up to and including 100% complementarity.

The first and the third segment are at least about 1000, 500, 475, 450, 425, 400, 375, 350, 325, 300, 250, 225, 200, 175, 150, 125, 100, 75, 60, 50, 40, 30, 25, 22, 20, 19, 18, 17, 16, 15 or 10 nucleotides in length. In specific embodiments, the length of the first and/or the third segment is about 10-100 nucleotides, about 10 to about 75 nucleotides, about 10 to about 50 nucleotides, about 10 to about 40 nucleotides, about 10 to about 35 nucleotides, about 10 to about 30 nucleotides, about 10 to about 25 nucleotides, about 10 to about 19 nucleotides, about 10 to about 20 nucleotides, about 19 to about 50 nucleotides, about 50 nucleotides to about 100 nucleotides, about 100 nucleotides to about 150 nucleotides, about 100 nucleotides to about 300 nucleotides, about 150 nucleotides to about 200 nucleotides, about 200 nucleotides to about 250 nucleotides, about 250 nucleotides to about 300 nucleotides, about 300 nucleotides to about 350 nucleotides, about 350 nucleotides to about 400 nucleotides, about 400 nucleotide to about 500 nucleotides, about 600 nt, about 700 nt, about 800 nt, about 900 nt, about 1000 nt, about 1100 nt, about 1200 nt, 1300 nt, 1400 nt, 1500 nt, 1600 nt, 1700 nt, 1800 nt, 1900 nt, 2000 nt or longer. In other embodiments, the length of the first and/or the third segment comprises at least 10-19 nucleotides, 10-20 nucleotides; 19-35 nucleotides, 20-35 nucleotides; 30-45 nucleotides; 40-50 nucleotides; 50-100 nucleotides; 100-300 nucleotides; about 500 -700 nucleotides; about 700-900nucleotides; about 900-1100 nucleotides; about 1300 -1500 nucleotides; about 1500 - 1700 nucleotides; about 1700 - 1900 nucleotides; about 1900 - 2100 nucleotides; about 2100 - 2300 nucleotides; or about 2300 - 2500 nucleotides. See, for example, International Publication No. WO 0200904.

Hairpin molecules or double-stranded RNA molecules of the present invention may have more than one sequence of the present invention or active fragments or variants, or complements thereof, found in the same portion of the RNA molecule. For example, in a chimeric hairpin structure, the first segment of a hairpin molecule comprises two polynucleotide sections, each with a different sequence of the present invention. For example, reading from one terminus of the hairpin, the first segment is composed of sequences from two separate genes (A followed by B). This first segment is followed by the second segment, the loop portion of the hairpin. The loop segment is followed by the third segment, where the complementary strands of the sequences in the first segment are found (B* followed by A*) in forming the stem-loop, hairpin structure, the stem contains SeqA-A* at the distal end of the stem and SeqB-B* proximal to the loop region.

In specific embodiments, the first and the third segment comprise at least 20 nucleotides having at least 85% complementary to the first segment. In still other embodiments, the first and the third segments which form the stem-loop structure of the hairpin comprises 3' or 5' overhang regions having unpaired nucleotide residues.

In specific embodiments, the sequences used in the first, the second, and/or the third segments comprise domains that are designed to have sufficient sequence identity to a target polynucleotide of interest and thereby have the ability to decrease the level of expression of the target polynucleotide. The specificity of the inhibitory RNA transcripts is therefore generally conferred by these domains of the silencing element. Thus, in some embodiments of the invention, the first, second and/or third segment of the silencing element comprise a domain having at least 10, at least 15, at least 19, at least 20, at least 21, at least 22, at least 23, at least 24, at least 25, at least 30, at least 40, at least 50, at least 100, at least 200, at least 300, at least 500, at least 1000, or more than 1000 nucleotides that share sufficient sequence identity to the target polynucleotide to allow for a decrease in expression levels of the target polynucleotide when expressed in an appropriate cell. In other embodiments, the domain is between about 15 to 50 nucleotides, about 19-35 nucleotides, about 20-35 nucleotides, about 25-50 nucleotides, about 19 to 75 nucleotides, about 20 to 75 nucleotides, about 40-90 nucleotides about 15-100 nucleotides10-100 nucleotides, about 10 to about 75 nucleotides, about 10 to about 50 nucleotides, about 10 to about 40 nucleotides, about 10 to about 35 nucleotides, about 10 to about 30 nucleotides, about 10 to about 25 nucleotides, about 10 to about 20 nucleotides, about 10 to about 19 nucleotides, about 50 nucleotides to about 100 nucleotides, about 100 nucleotides to about 150 nucleotides, about 150 nucleotides to about 200 nucleotides, about 200 nucleotides to about 250 nucleotides, about 250 nucleotides to about 300 nucleotides, about 300 nucleotides to about 350 nucleotides, about 350 nucleotides to about 400 nucleotides, about 400 nucleotide to about 500 nucleotides or longer. In other embodiments, the length of the first and/or the third segment comprises at least 10-20 nucleotides, at least 10-19 nucleotides, 20-35 nucleotides, 30-45 nucleotides, 40-50 nucleotides, 50-100 nucleotides, or about 100-300 nucleotides.

In specific embodiments, the domain of the first, the second, and/or the third segment has 100% sequence identity to the target polynucleotide. In other embodiments, the domain of the first, the second and/or the third segment having homology to the target polypeptide have at least 50%, 60%, 70%, 80%, 85%, 90%, 91%, 92%, 93%, 94%, 95%, 96%, 97%, 98%, 99%, or greater sequence identity to a region of the target polynucleotide. The sequence identity of the domains of the first, the second and/or the third segments to the target polynucleotide need only be sufficient to decrease expression of the target polynucleotide of interest. See, for example, Chuang and Meyerowitz (2000) Proc. Natl. Acad. Sci. USA 97:4985-4990; Stoutjesdijk et al. (2002) Plant Physiol. 129:1723-1731; Waterhouse and Helliwell (2003) Nat. Rev. Genet. 4:29-38; Pandolfini et al. BMC Biotechnology 3:7, and U.S. Patent Publication No. 20030175965; each of which is herein incorporated by reference. A transient assay for the efficiency of hpRNA constructs to silence gene expression *in vivo* has been described by Panstruga et al. (2003) Mol. Biol. Rep. 30:135-140, herein incorporated by reference.

The amount of complementarity shared between the first, second, and/or third segment and the target polynucleotide or the amount of complementarity shared between the first segment and the third segment (i.e., the stem of the hairpin structure) may vary depending on the organism in which gene expression is to be controlled. Some organisms or cell types may require exact pairing or 100% identity, while other organisms or cell types may tolerate some mismatching. In some cells, for example, a single nucleotide mismatch in the targeting sequence abrogates the ability to suppress gene expression. In these cells, the suppression cassettes of the invention can be used to target the suppression of mutant genes, for example, oncogenes whose transcripts comprise point mutations and therefore they can be specifically targeted using the methods and compositions of the invention without altering the expression of the remaining wild-type allele. In other organisms, holistic sequence variability may be tolerated as long as some 22nt region of the sequence is represented in 100% homology between target polynucleitide and the suppression cassette.

Any region of the target polynucleotide can be used to design the domain of the silencing element that shares sufficient sequence identity to allow expression of the hairpin transcript to decrease the level of the target polynucleotide. For instance, the domain can be designed to share sequence identity to the 5' untranslated region of the target polynucleotide(s), the 3' untranslated region of the target polynucleotide(s), exonic regions of the target polynucleotide(s), intronic regions of the target polynucleotide(s), and any combination thereof. In specific embodiments, a domain of the silencing element shares sufficient homology to at least about 15, 16, 17, 18, 19, 20, 22, 25 or 30 consecutive nucleotides from about nucleotides 1-50, 25-75, 75-125, 50-100, 125-175, 175-225, 100-150, 150-200, 200-250, 225-275, 275-325, 250-300, 325-375, 375-425, 300-350, 350-400, 425-475, 400-450, 475-525, 450-500, 525-575, 575-625, 550-600, 625-675, 675-725, 600-650, 625-675, 675-725, 650-700, 725-825, 825-875, 750-800, 875-925, 925-975, 850-900, 925-975, 975-1025, 950-1000, 1000-1050, 1025-1075, 1075-1125, 1050-1100, 1125-1175, 1100-1200, 1175-1225, 1225-1275, 1200-1300, 1325-1375, 1375-1425, 1300-1400, 1425-1475, 1475-1525, 1400-1500, 1525-1575, 1575-1625, 1625-1675, 1675-1725, 1725-1775, 1775-1825, 1825-1875, 1875-1925, 1925-1975, 1975-2025, 2025-2075, 2075-2125, 2125-2175, 2175-2225, 1500-1600, 1600-1700, 1700-1800, 1800-1900, 1900-2000 of the target sequence. In some instances to optimize the siRNA sequences employed in the hairpin, the synthetic oligodeoxyribonucleotide/RNAse H method can be used to determine sites on the target mRNA that are in a conformation that is susceptible to RNA silencing. See, for example, Vickers et al. (2003) J. Biol. Chem 278:7108-7118 and Yang et al. (2002) Proc. Natl. Acad. Sci. USA 99:9442-9447, herein incorporated by reference. These studies indicate that there is a significant correlation between the RNase-H-sensitive sites and sites that promote efficient siRNA-directed mRNA degradation.

The hairpin silencing element may also be designed such that the sense sequence or the antisense sequence do not correspond to a target polynucleotide. In this embodiment, the sense and antisense sequence flank a loop sequence that comprises a nucleotide sequence corresponding to all or part of the target polynucleotide. Thus, it is the loop region that determines the specificity of the RNA interference. See, for example, WO 02/00904, herein incorporated by reference.

In addition, transcriptional gene silencing (TGS) may be accomplished through use of a hairpin suppression element where the inverted repeat of the hairpin shares sequence identity with the promoter region of a target polynucleotide to be silenced. See, for example, Aufsatz et al. (2002) PNAS 99 (Suppl. 4): 16499-16506 and Mette et al. (2000) EMBO J 19(19):5194-5201.

In other embodiments, the silencing element can comprise a small RNA (sRNA). sRNAs can comprise both micro RNA (miRNA) and short-interfering RNA (siRNA) (Meister and Tuschl (2004) Nature 431:343-349 and Bonetta et al. (2004) Nature Methods 1:79-86). miRNAs are regulatory agents comprising about 19 to about 24 ribonucleotides in length which are highly efficient at inhibiting the expression of target polynucleotides. See, for example Javier et al. (2003) Nature 425: 257-263, herein incorporated by reference. For miRNA interference, the silencing element can be designed to express a dsRNA molecule that forms a hairpin structure or partially base-paired structure containing 19, 20, 21, 22, 23, 24 or 25 -nucleotide sequence that is complementary to the target polynucleotide of interest. The miRNA can be synthetically made, or transcribed as a longer RNA which is subsequently cleaved to produce the active miRNA. Specifically, the miRNA can comprise 19 nucleotides of the sequence having homology to a target polynucleotide in sense orientation and 19 nucleotides of a corresponding antisense sequence that is complementary to the sense sequence. The miRNA can be an "artificial miRNA" or "amiRNA" which comprises a miRNA sequence that is synthetically designed to silence a target sequence.

When expressing an miRNA the final (mature) miRNA is present in a duplex in a precursor backbone structure, the two strands being referred to as the miRNA (the strand that will eventually basepair with the target) and miRNA*(star sequence). It has been demonstrated that miRNAs can be transgenically expressed and target genes of interest efficiently silenced (Highly specific gene silencing by artificial microRNAs in Arabidopsis Schwab R, Ossowski S, Riester M, Warthmann N, Weigel D. Plant Cell. 2006 May;18(5):1121-33. Epub 2006 Mar 10 & Expression of artificial microRNAs in transgenic Arabidopsis thaliana confers virus resistance. Niu QW, Lin SS, Reyes JL, Chen KC, Wu HW, Yeh SD, Chua NH. Nat Biotechnol. 2006 Nov;24(11):1420-8. Epub 2006 Oct 22. Erratum in: Nat Biotechnol. 2007 Feb;25(2):254.)

The silencing element for miRNA interference comprises a miRNA primary sequence. The miRNA primary sequence comprises a DNA sequence having the miRNA and star sequences separated by a loop as well as additional sequences flanking this region that are important for processing. When expressed as an RNA, the structure of the primary miRNA is such as to allow for the formation of a hairpin RNA structure that can be processed into a mature miRNA. In some embodiments, the miRNA backbone comprises a genomic or cDNA miRNA precursor sequence, wherein said sequence comprises a native primary in which a heterologous (artificial) mature miRNA and star sequence are inserted.

As used herein, a "star sequence" is the sequence within a miRNA precursor backbone that is complementary to the miRNA and forms a duplex with the miRNA to form the stem structure of a hairpin RNA. In some embodiments, the star sequence can comprise less than 100% complementarity to the miRNA sequence. Alternatively, the star sequence can comprise at least 99%, 98%, 97%, 96%, 95%, 90%, 85%, 80% or lower sequence complementarity to the miRNA sequence as long as the star sequence has sufficient complementarity to the miRNA sequence to form a double stranded structure. In still further embodiments, the star sequence comprises a sequence having 1, 2, 3, 4, 5 or more mismatches with the miRNA sequence and still has sufficient complementarity to form a double stranded structure with the miRNA sequence resulting in production of miRNA and suppression of the target sequence.

The miRNA precursor backbones can be from any plant. In some embodiments, the miRNA precursor backbone is from a monocot. In other embodiments, the miRNA precursor backbone is from a dicot. In further embodiments, the backbone is from maize or soybean. MicroRNA precursor backbones have been described previously. For example, US20090155910A1 (WO 2009/079532) discloses the following soybean miRNA precursor backbones: 156c, 159, 166b, 168c, 396b and 398b, and US20090155909A1 (WO 2009/079548) discloses the following maize miRNA precursor backbones: 159c, 164h, 168a, 169r, and 396h. Each of these references is incorporated by reference in their entirety.

Thus, the primary miRNA can be altered to allow for efficient insertion of heterologous miRNA and star sequences within the miRNA precursor backbone. In such instances, the miRNA segment and the star segment of the miRNA precursor backbone are replaced with the heterologous miRNA and the heterologous star sequences, designed to target any sequence of interest, using a PCR technique and cloned into an expression construct. It is recognized that there could be alterations to the position at which the artificial miRNA and star sequences are inserted into the backbone. Detailed methods for inserting the miRNA and star sequence into the miRNA precursor backbone are described in, for example, US Patent Applications 20090155909A1 and US20090155910A1, herein incorporated by reference in their entirety.

When designing a miRNA sequence and star sequence, various design choices can be made. See, for example, Schwab R, et al. (2005) Dev Cell 8: 517-27. In non-limiting embodiments, the miRNA sequences disclosed herein can have a "U" at the 5'-end, a "C" or "G" at the 19th nucleotide position, and an "A" or "U" at the 10th nucleotide position. In other embodiments, the miRNA design is such that the miRNA have a high free delta-G as calculated using the ZipFold algorithm (Markham, N. R. & Zuker, M. (2005) Nucleic Acids Res. 33: W577-W581.) Optionally, a one base pair change can be added within the 5' portion of the miRNA so that the sequence differs from the target sequence by one nucleotide.

The methods and compositions of the invention employ silencing elements that when transcribed "form" a dsRNA molecule. Accordingly, the heterologous polynucleotide being expressed need not form the dsRNA by itself, but can interact with other sequences in the plant cell or in the pest gut after ingestion to allow the formation of the dsRNA. For example, a chimeric polynucleotide that can selectively silence the target polynucleotide can be generated by expressing a chimeric construct comprising the target sequence for a miRNA or siRNA to a sequence corresponding to all or part of the gene or genes to be silenced. In this embodiment, the dsRNA is "formed" when the target for the miRNA or siRNA interacts with the miRNA present in the cell. The resulting dsRNA can then reduce the level of expression of the gene or genes to be silenced. See, for example, US Application Publication 2007-0130653, entitled "Methods and Compositions for Gene Silencing", herein incorporated by reference. The construct can be designed to have a target for an endogenous miRNA or alternatively, a target for a heterologous and/or synthetic miRNA can be employed in the construct. If a heterologous and/or synthetic miRNA is employed, it can be introduced into the cell on the same nucleotide construct as the chimeric polynucleotide or on a separate construct. As discussed elsewhere herein, any method can be used to introduce the construct comprising the heterologous miRNA.

### IV. Variants and Fragments

By "fragment" is intended a portion of the polynucleotide or a portion of the amino acid sequence and hence protein encoded thereby. Fragments of a polynucleotide may encode protein fragments that retain the biological activity of the native protein. Alternatively, fragments of a polynucleotide that are useful as a silencing element do not need to encode fragment proteins that retain biological activity. Thus, fragments of a nucleotide sequence may range from at least about 10, about 15, about 16, about 17, about 18, about 19, nucleotides, about 20 nucleotides, about 22 nucleotides, about 50 nucleotides, about 75 nucleotides, about 100 nucleotides, 200 nucleotides, 300 nucleotides, 400 nucleotides, 500 nucleotides, 600 nucleotides, 700 nucleotides and up to the full-length polynucleotide employed in the invention. Alternatively, fragments of a nucleotide sequence may range from 1-50, 25-75, 75-125, 50-100, 125-175, 175-225, 100-150, 100-300, 150-200, 200-250, 225-275, 275-325, 250-300, 325-375, 375-425, 300-350, 350-400, 425-475, 400-450, 475-525, 450-500, 525-575, 575-625, 550-600, 625-675, 675-725, 600-650, 625-675, 675-725, 650-700, 725-825, 825-875, 750-800, 875-925, 925-975, 850-900, 925-975, 975-1025, 950-1000, 1000-1050, 1025-1075, 1075-1125, 1050-1100, 1125-1175, 1100-1200, 1175-1225, 1225-1275, 1200-1300, 1325-1375, 1375-1425, 1300-1400, 1425-1475, 1475-1525, 1400-1500, 1525-1575, 1575-1625, 1625-1675, 1675-1725, 1725-1775, 1775-1825, 1825-1875, 1875-1925, 1925-1975, 1975-2025, 2025-2075, 2075-2125, 2125-2175, 2175-2225, 1500-1600, 1600-1700, 1700-1800, 1800-1900, 1900-2000 of any one of SEQ ID NOS: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof, including, for example, SEQ ID NOS: 1, 9, 37, 45, 49, 61, 65, 77, 101, 113, 137, 141, 145, 149, 153, 157, 169, 173, 181, 185, 189, 205, 217, 225,233, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, and active variants and fragments thereof, and complements thereof, and SEQ ID NOS: 4, 140, 144, 148, 693, 694, 695, 696, 697, 700, 701, 702, 703, 706, 707, 708, 709, 712, 713, 714, 715, 718, 719, 720, 721, 724, 725, 726, 727, 728, and active variants and fragments thereof, and complements thereof. Methods to assay for the activity of a desired silencing element are described elsewhere herein.

"Variants" is intended to mean substantially similar sequences. For polynucleotides, a variant comprises a deletion and/or addition of one or more nucleotides at one or more internal sites within the native polynucleotide and/or a substitution of one or more nucleotides at one or more sites in the native polynucleotide. A variant of a polynucleotide that is useful as a silencing element will retain the ability to reduce expression of the target polynucleotide and, in some embodiments, thereby control a pest of interest. As used herein, a "native" polynucleotide or polypeptide comprises a naturally occurring nucleotide sequence or amino acid sequence, respectively. For polynucleotides, conservative variants include those sequences that, because of the degeneracy of the genetic code, encode the amino acid sequence of one of the polypeptides employed in the invention. Variant polynucleotides also include synthetically derived polynucleotide, such as those generated, for example, by using site-directed mutagenesis, but continue to retain the desired activity. Generally, variants of a particular polynucleotide of the invention (i.e., a silencing element) will have at least about 40%, 45%, 50%, 55%, 60%, 65%, 70%, 75%, 80%, 85%, 90%, 91%, 92%, 93%, 94%, 95%, 96%, 97%, 98%, 99% or more sequence identity to that particular polynucleotide as determined by sequence alignment programs and parameters described elsewhere herein.

Variants of a particular polynucleotide of the invention (i.e., the reference polynucleotide) can also be evaluated by comparison of the percent sequence identity between the polypeptide encoded by a variant polynucleotide and the polypeptide encoded by the reference polynucleotide. Percent sequence identity between any two polypeptides can be calculated using sequence alignment programs and parameters described elsewhere herein. Where any given pair of polynucleotides employed in the invention is evaluated by comparison of the percent sequence identity shared by the two polypeptides they encode, the percent sequence identity between the two encoded polypeptides is at least about 40%, 45%, 50%, 55%, 60%, 65%, 70%, 75%, 80%, 85%, 90%, 91%, 92%, 93%, 94%, 95%, 96%, 97%, 98%, 99% or more sequence identity.

The following terms are used to describe the sequence relationships between two or more polynucleotides or polypeptides: (a) "reference sequence", (b) "comparison window", (c) "sequence identity", and, (d) "percentage of sequence identity."
(a) As used herein, "reference sequence" is a defined sequence used as a basis for sequence comparison. A reference sequence may be a subset or the entirety of a specified sequence; for example, as a segment of a full-length cDNA or gene sequence, or the complete cDNA or gene sequence.
(b) As used herein, "comparison window" makes reference to a contiguous and specified segment of a polynucleotide sequence, wherein the polynucleotide sequence in the comparison window may comprise additions or deletions (i.e., gaps) compared to the reference sequence (which does not comprise additions or deletions) for optimal alignment of the two polynucleotides. Generally, the comparison window is at least 20 contiguous nucleotides in length, and optionally can be 30, 40, 50, 100, or longer. Those of skill in the art understand that to avoid a high similarity to a reference sequence due to inclusion of gaps in the polynucleotide sequence a gap penalty is typically introduced and is subtracted from the number of matches.
   Unless otherwise stated, sequence identity/similarity values provided herein refer to the value obtained using GAP Version 10 using the following parameters: % identity and % similarity for a nucleotide sequence using GAP Weight of 50 and Length Weight of 3, and the nwsgapdna.cmp scoring matrix; % identity and % similarity for an amino acid sequence using GAP Weight of 8 and Length Weight of 2, and the BLOSUM62 scoring matrix; or any equivalent program thereof. By "equivalent program" is intended any sequence comparison program that, for any two sequences in question, generates an alignment having identical nucleotide or amino acid residue matches and an identical percent sequence identity when compared to the corresponding alignment generated by GAP Version 10.
(c) As used herein, "sequence identity" or "identity" in the context of two polynucleotides or polypeptide sequences makes reference to the residues in the two sequences that are the same when aligned for maximum correspondence over a specified comparison window. When percentage of sequence identity is used in reference to proteins it is recognized that residue positions which are not identical often differ by conservative amino acid substitutions, where amino acid residues are substituted for other amino acid residues with similar chemical properties (e.g., charge or hydrophobicity) and therefore do not change the functional properties of the molecule. When sequences differ in conservative substitutions, the percent sequence identity may be adjusted upwards to correct for the conservative nature of the substitution. Sequences that differ by such conservative substitutions are said to have "sequence similarity" or "similarity". Means for making this adjustment are well known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, e.g., as implemented in the program PC/GENE (Intelligenetics, Mountain View, California).
(d) As used herein, "percentage of sequence identity" means the value determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison, and multiplying the result by 100 to yield the percentage of sequence identity.

A method is further provided for identifying a silencing element from the target polynucleotides set forth in SEQ ID NOS: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof, including, for example, SEQ ID NOS: 1, 9, 37, 45, 49, 61, 65, 77, 101, 113, 137, 141, 145, 149, 153, 157, 169, 173, 181, 185, 189, 205, 217, 225,233, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, and active variants and fragments thereof, and complements thereof, and SEQ ID NOS: 4, 140, 144, 148, 693, 694, 695, 696, 697, 700, 701, 702, 703, 706, 707, 708, 709, 712, 713, 714, 715, 718, 719, 720, 721, 724, 725, 726, 727, 728, and active variants and fragments thereof, and complements thereof. Such methods comprise obtaining a candidate fragment of any one of SEQ ID NOS: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof, including, for example, SEQ ID NOS: 1, 9, 37, 45, 49, 61, 65, 77, 101, 113, 137, 141, 145, 149, 153, 157, 169, 173, 181, 185, 189, 205, 217, 225,233, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, and active variants and fragments thereof, and complements thereof, and SEQ ID NOS: 4, 140, 144, 148, 693, 694, 695, 696, 697, 700, 701, 702, 703, 706, 707, 708, 709, 712, 713, 714, 715, 718, 719, 720, 721, 724, 725, 726, 727, 728, and active variants and fragments thereof, and complements thereof, which is of sufficient length to act as a silencing element and thereby reduce the expression of the target polynucleotide and/or control a desired pest; expressing said candidate polynucleotide fragment in an appropriate expression cassette to produce a candidate silencing element and determining is said candidate polynucleotide fragment has the activity of a silencing element and thereby reduce the expression of the target polynucleotide and/or controls a desired pest. Methods of identifying such candidate fragments based on the desired pathway for suppression are known. For example, various bioinformatics programs can be employed to identify the region of the target polynucleotides that could be exploited to generate a silencing element. See, for example, Elbahir et al. (2001) Genes and Development 15:188-200, Schwartz et al. (2003) Cell 115:199-208, Khvorova et al. (2003) Cell 115:209-216. See also, siRNA at Whitehead (jura.wi.mit.edu/bioc/siRNAext/) which calculates the binding energies for both sense and antisense siRNAs. See, also genscript.com/ssl-bin/app/rnai?op=known; Block-iT™ RNAi designer from Invitrogen and GenScript siRNA Construct Builder.

### V. DNA constructs

The use of the term "polynucleotide" is not intended to limit the present invention to polynucleotides comprising DNA. Those of ordinary skill in the art will recognize that polynucleotides can comprise ribonucleotides and combinations of ribonucleotides and deoxyribonucleotides. Such deoxyribonucleotides and ribonucleotides include both naturally occurring molecules and synthetic analogues. The polynucleotides of the invention also encompass all forms of sequences including, but not limited to, single-stranded forms, double-stranded forms, hairpins, stem-and-loop structures, and the like.

The polynucleotide encoding the silencing element or in specific embodiments employed in the methods and compositions of the invention can be provided in expression cassettes for expression in a plant or organism of interest. It is recognized that multiple silencing elements including multiple identical silencing elements, multiple silencing elements targeting different regions of the target sequence, or multiple silencing elements from different target sequences can be used. In this embodiment, it is recognized that each silencing element can be contained in a single or separate cassette, DNA construct, or vector. As discussed, any means of providing the silencing element is contemplated. A plant or plant cell can be transformed with a single cassette comprising DNA encoding one or more silencing elements or separate cassettes comprising each silencing element can be used to transform a plant or plant cell or host cell. Likewise, a plant transformed with one component can be subsequently transformed with the second component. One or more silencing elements can also be brought together by sexual crossing. That is, a first plant comprising one component is crossed with a second plant comprising the second component. Progeny plants from the cross will comprise both components.

The expression cassette can include 5' and 3' regulatory sequences operably linked to the polynucleotide of the invention. "Operably linked" is intended to mean a functional linkage between two or more elements. For example, an operable linkage between a polynucleotide of the invention and a regulatory sequence (i.e., a promoter) is a functional link that allows for expression of the polynucleotide of the invention. Operably linked elements may be contiguous or non-contiguous. When used to refer to the joining of two protein coding regions, by operably linked is intended that the coding regions are in the same reading frame. The cassette may additionally contain at least one additional polynucleotide to be cotransformed into the organism. Alternatively, the additional polypeptide(s) can be provided on multiple expression cassettes. Expression cassettes can be provided with a plurality of restriction sites and/or recombination sites for insertion of the polynucleotide to be under the transcriptional regulation of the regulatory regions. The expression cassette may additionally contain selectable marker genes.

The expression cassette can include in the 5'-3' direction of transcription, a transcriptional and translational initiation region (i.e., a promoter), a polynucleotide comprising the silencing element employed in the methods and compositions of the invention, and a transcriptional and translational termination region (i.e., termination region) functional in plants. In other embodiment, the double stranded RNA is expressed from a suppression cassette. Such a cassette can comprise two convergent promoters that drive transcription of an operably linked silencing element. "Convergent promoters" refers to promoters that are oriented on either terminus of the operably linked silencing element such that each promoter drives transcription of the silencing element in opposite directions, yielding two transcripts. In such embodiments, the convergent promoters allow for the transcription of the sense and anti-sense strand and thus allow for the formation of a dsRNA. Such a cassette may also comprise two divergent promoters that drive transcription of one or more operably linked silencing elements. "Divergent promoters" refers to promoters that are oriented in opposite directions of each other, driving transcription of the one or more silencing elements in opposite directions. In such embodiments, the divergent promoters allow for the transcription of the sense and antisense strands and allow for the formation of a dsRNA. In such embodiments, the divergent promoters also allow for the transcription of at least two separate hairpin RNAs. In another embodiment, one cassette comprising two or more silencing elements under the control of two separate promoters in the same orientation is present in a construct. In another embodiment, two or more individual cassettes, each comprising at least one silencing element under the control of a promoter, are present in a construct in the same orientation.

The regulatory regions (i.e., promoters, transcriptional regulatory regions, and translational termination regions) and/or the polynucleotides employed in the invention may be native/analogous to the host cell or to each other. Alternatively, the regulatory regions and/or the polynucleotide employed in the invention may be heterologous to the host cell or to each other. As used herein, "heterologous" in reference to a sequence is a sequence that originates from a foreign species, or, if from the same species, is substantially modified from its native form in composition and/or genomic locus by deliberate human intervention. For example, a promoter operably linked to a heterologous polynucleotide is from a species different from the species from which the polynucleotide was derived, or, if from the same/analogous species, one or both are substantially modified from their original form and/or genomic locus, or the promoter is not the native promoter for the operably linked polynucleotide. As used herein, a chimeric gene comprises a coding sequence operably linked to a transcription initiation region that is heterologous to the coding sequence.

The termination region may be native with the transcriptional initiation region, may be native with the operably linked polynucleotide encoding the silencing element, may be native with the plant host, or may be derived from another source (i.e., foreign or heterologous) to the promoter, the polynucleotide comprising silencing element, the plant host, or any combination thereof. Convenient termination regions are available from the Ti-plasmid of *A. tumefaciens,* such as the octopine synthase and nopaline synthase termination regions. See also Guerineau et al. (1991) Mol. Gen. Genet. 262:141-144; Proudfoot (1991) Cell 64:671-674; Sanfacon et al. (1991) Genes Dev. 5:141-149; Mogen et al. (1990) Plant Cell 2:1261-1272; Munroe et al. (1990) Gene 91:151-158; Ballas et al. (1989) Nucleic Acids Res. 17:7891-7903; and Joshi et al. (1987) Nucleic Acids Res. 15:9627-9639.

Additional sequence modifications are known to enhance gene expression in a cellular host. These include elimination of sequences encoding spurious polyadenylation signals, exon-intron splice site signals, transposon-like repeats, and other such well-characterized sequences that may be deleterious to gene expression. The G-C content of the sequence may be adjusted to levels average for a given cellular host, as calculated by reference to known genes expressed in the host cell. When possible, the sequence is modified to avoid predicted hairpin secondary mRNA structures.

In preparing the expression cassette, the various DNA fragments may be manipulated, so as to provide for the DNA sequences in the proper orientation and, as appropriate, in the proper reading frame. Toward this end, adapters or linkers may be employed to join the DNA fragments or other manipulations may be involved to provide for convenient restriction sites, removal of superfluous DNA, removal of restriction sites, or the like. For this purpose, *in vitro* mutagenesis, primer repair, restriction, annealing, resubstitutions, e.g., transitions and transversions, may be involved.

A number of promoters can be used in the practice of the invention. The polynucleotide encoding the silencing element can be combined with constitutive, tissue-preferred, or other promoters for expression in plants.

Such constitutive promoters include, for example, the core promoter of the Rsyn7 promoter and other constitutive promoters disclosed in WO 99/43838 and U.S. Patent No. 6,072,050; the core CaMV 35S promoter (Odell et al. (1985) Nature 313:810-812); rice actin (McElroy et al. (1990) Plant Cell 2:163-171); ubiquitin (Christensen et al. (1989) Plant Mol. Biol. 12:619-632 and Christensen et al. (1992) Plant Mol. Biol. 18:675-689); pEMU (Last et al. (1991) Theor. Appl. Genet. 81:581-588); MAS (Velten et al. (1984) EMBO J. 3:2723-2730); ALS promoter (U.S. Patent No. 5,659,026), and the like. Other constitutive promoters include, for example, U.S. Patent Nos. 5,608,149; 5,608,144; 5,604,121; 5,569,597; 5,466,785; 5,399,680; 5,268,463; 5,608,142; and 6,177,611.

An inducible promoter, for instance, a pathogen-inducible promoter could also be employed. Such promoters include those from pathogenesis-related proteins (PR proteins), which are induced following infection by a pathogen; e.g., PR proteins, SAR proteins, beta-1,3-glucanase, chitinase, etc. See, for example, Redolfi et al. (1983) Neth. J. Plant Pathol. 89:245-254; Uknes et al. (1992) Plant Cell 4:645-656; and Van Loon (1985) Plant Mol. Virol. 4:111-116. See also WO 99/43819, herein incorporated by reference.

Additionally, as pathogens find entry into plants through wounds or insect damage, a wound-inducible promoter may be used in the constructions of the invention. Such wound-inducible promoters include potato proteinase inhibitor (pin II) gene (Ryan (1990) Ann. Rev. Phytopath. 28:425-449; Duan et al. (1996) Nature Biotechnology 14:494-498); wun1 and wun2, U.S. Patent No. 5,428,148; win1 and win2 (Stanford et al. (1989) Mol. Gen. Genet. 215:200-208); systemin (McGurl et al. (1992) Science 225:1570-1573); WIP1 (Rohmeier et al. (1993) Plant Mol. Biol. 22:783-792; Eckelkamp et al. (1993) FEBS Letters 323:73-76); MPI gene (Corderok et al. (1994) Plant J. 6(2):141-150); and the like, herein incorporated by reference.

Chemical-regulated promoters can be used to modulate the expression of a gene in a plant through the application of an exogenous chemical regulator. Depending upon the objective, the promoter may be a chemical-inducible promoter, where application of the chemical induces gene expression, or a chemical-repressible promoter, where application of the chemical represses gene expression. Chemical-inducible promoters are known in the art and include, but are not limited to, the maize In2-2 promoter, which is activated by benzenesulfonamide herbicide safeners, the maize GST promoter, which is activated by hydrophobic electrophilic compounds that are used as pre-emergent herbicides, and the tobacco PR-1a promoter, which is activated by salicylic acid. Other chemical-regulated promoters of interest include steroid-responsive promoters (see, for example, the glucocorticoid-inducible promoter in Schena et al. (1991) Proc. Natl. Acad. Sci. USA 88:10421-10425 and McNellis et al. (1998) Plant J. 14(2):247-257) and tetracycline-inducible and tetracycline-repressible promoters (see, for example, Gatz et al. (1991) Mol. Gen. Genet. 227:229-237, and U.S. Patent Nos. 5,814,618 and 5,789,156), herein incorporated by reference.

Tissue-preferred promoters can be utilized to target enhanced expression within a particular plant tissue. Tissue-preferred promoters include Yamamoto et al. (1997) Plant J. 12(2):255-265; Kawamata et al. (1997) Plant Cell Physiol. 38(7):792-803; Hansen et al. (1997) Mol. Gen Genet. 254(3):337-343; Russell et al. (1997) Transgenic Res. 6(2):157-168; Rinehart et al. (1996) Plant Physiol. 112(3):1331-1341; Van Camp et al. (1996) Plant Physiol. 112(2):525-535; Canevascini et al. (1996) Plant Physiol. 112(2):513-524; Yamamoto et al. (1994) Plant Cell Physiol. 35(5):773-778; Lam (1994) Results Probl. Cell Differ. 20:181-196; Orozco et al. (1993) Plant Mol Biol. 23(6):1129-1138; Matsuoka et al. (1993) Proc Natl. Acad. Sci. USA 90(20):9586-9590; and Guevara-Garcia et al. (1993) Plant J. 4(3):495-505. Such promoters can be modified, if necessary, for weak expression.

Leaf-preferred promoters are known in the art. See, for example, Yamamoto et al. (1997) Plant J. 12(2):255-265; Kwon et al. (1994) Plant Physiol. 105:357-67; Yamamoto et al. (1994) Plant Cell Physiol. 35(5):773-778; Gotor et al. (1993) Plant J. 3:509-18; Orozco et al. (1993) Plant Mol. Biol. 23(6):1129-1138; and Matsuoka et al. (1993) Proc. Natl. Acad. Sci. USA 90(20):9586-9590.

Root-preferred promoters are known and can be selected from the many available from the literature or isolated de novo from various compatible species. See, for example, Hire et al. (1992) Plant Mol. Biol. 20(2):207-218 (soybean root-specific glutamine synthetase gene); Keller and Baumgartner (1991) Plant Cell 3(10):1051-1061 (root-specific control element in the GRP 1.8 gene of French bean); Sanger et al. (1990) Plant Mol. Biol. 14(3):433-443 (root-specific promoter of the mannopine synthase (MAS) gene of *Agrobacterium tumefaciens*); and Miao et al. (1991) Plant Cell 3(1):11-22 (full-length cDNA clone encoding cytosolic glutamine synthetase (GS), which is expressed in roots and root nodules of soybean). See also Bogusz et al. (1990) Plant Cell 2(7):633-641, where two root-specific promoters isolated from hemoglobin genes from the nitrogen-fixing nonlegume *Parasponia andersonii* and the related non-nitrogen-fixing nonlegume *Trema tomentosa* are described. The promoters of these genes were linked to a β-glucuronidase reporter gene and introduced into both the nonlegume *Nicotiana tabacum* and the legume *Lotus corniculatus,* and in both instances root-specific promoter activity was preserved. Leach and Aoyagi (1991) describe their analysis of the promoters of the highly expressed rolC and rolD root-inducing genes of *Agrobacterium rhizogenes* (see Plant Science (Limerick) 79(1):69-76). They concluded that enhancer and tissue-preferred DNA determinants are dissociated in those promoters. Teeri *et al.* (1989) used gene fusion to lacZ to show that the *Agrobacterium* T-DNA gene encoding octopine synthase is especially active in the epidermis of the root tip and that the TR2' gene is root specific in the intact plant and stimulated by wounding in leaf tissue, an especially desirable combination of characteristics for use with an insecticidal or larvicidal gene (see EMBO J. 8(2):343-350). The TR1' gene, fused to *nptII* (neomycin phosphotransferase II) showed similar characteristics. Additional root-preferred promoters include the VfENOD-GRP3 gene promoter (Kuster et al. (1995) Plant Mol. Biol. 29(4):759-772); and rolB promoter (Capana et al. (1994) Plant Mol. Biol. 25(4):681-691. See also U.S. Patent Nos. 5,837,876; 5,750,386; 5,633,363; 5,459,252; 5,401,836; 5,110,732; and 5,023,179.

In one embodiment of this invention the plant-expressed promoter is a vascular-specific promoter such as a phloem-specific promoter. A "vascular-specific" promoter, as used herein, is a promoter which is at least expressed in vascular cells, or a promoter which is preferentially expressed in vascular cells. Expression of a vascular-specific promoter need not be exclusively in vascular cells, expression in other cell types or tissues is possible. A "phloem-specific promoter" as used herein, is a plant-expressible promoter which is at least expressed in phloem cells, or a promoter which is preferentially expressed in phloem cells.

Expression of a phloem-specific promoter need not be exclusively in phloem cells, expression in other cell types or tissues, e.g., xylem tissue, is possible. In one embodiment of this invention, a phloem-specific promoter is a plant-expressible promoter at least expressed in phloem cells, wherein the expression in non-phloem cells is more limited (or absent) compared to the expression in phloem cells. Examples of suitable vascular-specific or phloem-specific promoters in accordance with this invention include but are not limited to the promoters selected from the group consisting of: the SCSV3, SCSV4, SCSV5, and SCSV7 promoters (Schunmann et al. (2003) Plant Functional Biology 30:453-60; the rolC gene promoter of *Agrobacterium rhizogenes(*Kiyokawa et al. (1994) Plant Physiology 104:801-02; Pandolfini et al. (2003) BioMedCentral (BMC) Biotechnology 3:7, (www.biomedcentral.com/1472-6750/3/7); Graham et al. (1997) Plant Mol. Biol. 33:729-35; Guivarc'h *et al.* (1996); Almon et al. (1997) Plant Physiol. 115:1599-607; the rolA gene promoter of *Agrobacterium rhizogenes* (Dehio et al. (1993) Plant Mol. Biol. 23:1199-210); the promoter of the *Agrobacterium tumefaciens* T-DNA gene 5 (Korber et al. (1991) EMBO J. 10:3983-91); the rice sucrose synthase RSs1 gene promoter (Shi et al. (1994) J. Exp. Bot. 45:623-31); the CoYMV or Commelina yellow mottle badnavirus promoter (Medberry et al. (1992) Plant Cell 4:185-92; Zhou et al. (1998) Chin. J. Biotechnol. 14:9-16); the CFDV or coconut foliar decay virus promoter (Rohde et al. (1994) Plant Mol. Biol. 27:623-28; Hehn and Rhode (1998) J. Gen. Virol. 79:1495-99); the RTBV or rice tungro bacilliform virus promoter (Yin and Beachy (1995) Plant J. 7:969-80; Yin et al. (1997) Plant J. 12:1179-80); the pea glutamin synthase GS3A gene (Edwards et al. (1990) Proc. Natl. Acad. Sci. USA 87:3459-63; Brears et al. (1991) Plant J. 1:235-44); the inv CD111 and inv CD141 promoters of the potato invertase genes (Hedley et al. (2000) J. Exp. Botany 51:817-21); the promoter isolated from Arabidopsis shown to have phloem-specific expression in tobacco by Kertbundit et al. (1991) Proc. Natl. Acad. Sci. USA 88:5212-16); the VAHOX1 promoter region (Tornero et al. (1996) Plant J. 9:639-48); the pea cell wall invertase gene promoter (Zhang et al. (1996) Plant Physiol. 112:1111-17); the promoter of the endogenous cotton protein related to chitinase of US published patent application 20030106097, an acid invertase gene promoter from carrot (Ramloch-Lorenz et al. (1993) The Plant J. 4:545-54); the promoter of the sulfate transporter geneSultr1; 3 (Yoshimoto et al. (2003) Plant Physiol. 131:1511-17); a promoter of a sucrose synthase gene (Nolte and Koch (1993) Plant Physiol. 101:899-905); and the promoter of a tobacco sucrose transporter gene (Kuhn et al. (1997) Science 275-1298-1300).

Possible promoters also include the Black Cherry promoter for Prunasin Hydrolase (PH DL1.4 PRO) (US Patent No. 6,797, 859), Thioredoxin H promoter from cucumber and rice (Fukuda A et al. (2005). Plant Cell Physiol. 46(11):1779-86), Rice (RSs1) (Shi, T. Wang et al. (1994). J. Exp. Bot. 45(274): 623-631) and maize sucrose synthese -1 promoters (Yang., N-S. et al. (1990) PNAS 87:4144-4148), PP2 promoter from pumpkin Guo, H. et al. (2004) Transgenic Research 13:559-566), At SUC2 promoter (Truernit, E. et al. (1995) Planta 196(3):564-70., At SAM-1 (S-adenosylmethionine synthetase) (Mijnsbrugge KV. et al. (1996) Planr. Cell. Physiol. 37(8): 1108-1115), and the Rice tungro bacilliform virus (RTBV) promoter (Bhattacharyya-Pakrasi et al. (1993) Plant J. 4(1):71-79).

The expression cassette can also comprise a selectable marker gene for the selection of transformed cells. Selectable marker genes are utilized for the selection of transformed cells or tissues. Marker genes include genes encoding antibiotic resistance, such as those encoding neomycin phosphotransferase II (NEO) and hygromycin phosphotransferase (HPT), as well as genes conferring resistance to herbicidal compounds, such as glufosinate ammonium, bromoxynil, imidazolinones, and 2,4-dichlorophenoxyacetate (2,4-D). Additional selectable markers include phenotypic markers such as β-galactosidase and fluorescent proteins such as green fluorescent protein (GFP) (Su et al. (2004) Biotechnol Bioeng 85:610-9 and Fetter et al. (2004) Plant Cell 16:215-28), cyan florescent protein (CYP) (Bolte et al. (2004) J. Cell Science 117:943-54 and Kato et al. (2002) Plant Physiol 129:913-42), and yellow florescent protein (PhiYFP™ from Evrogen, see, Bolte et al. (2004) J. Cell Science 117:943-54). For additional selectable markers, see generally, Yarranton (1992) Curr. Opin. Biotech. 3:506-511; Christopherson et al. (1992) Proc. Natl. Acad. Sci. USA 89:6314-6318; Yao et al. (1992) Cell 71:63-72; Reznikoff (1992) Mol. Microbiol. 6:2419-2422; Barkley et al. (1980) in The Operon, pp. 177-220; Hu et al. (1987) Cell 48:555-566; Brown et al. (1987) Cell 49:603-612; Figge et al. (1988) Cell 52:713-722; Deuschle et al. (1989) Proc. Natl. Acad. Sci. USA 86:5400-5404; Fuerst et al. (1989) Proc. Natl. Acad. Sci. USA 86:2549-2553; Deuschle et al. (1990) Science 248:480-483; Gossen (1993) Ph.D. Thesis, University of Heidelberg; Reines et al. (1993) Proc. Natl. Acad. Sci. USA 90:1917-1921; Labow et al. (1990) Mol. Cell. Biol. 10:3343-3356; Zambretti et al. (1992) Proc. Natl. Acad. Sci. USA 89:3952-3956; Baim et al. (1991) Proc. Natl. Acad. Sci. USA 88:5072-5076; Wyborski et al. (1991) Nucleic Acids Res. 19:4647-4653; Hillenand-Wissman (1989) Topics Mol. Struc. Biol. 10:143-162; Degenkolb et al. (1991) Antimicrob. Agents Chemother. 35:1591-1595; Kleinschnidt et al. (1988) Biochemistry 27:1094-1104; Bonin (1993) Ph.D. Thesis, University of Heidelberg; Gossen et al. (1992) Proc. Natl. Acad. Sci. USA 89:5547-5551; Oliva et al. (1992) Antimicrob. Agents Chemother. 36:913-919; Hlavka et al. (1985) Handbook of Experimental Pharmacology, Vol. 78 (Springer-Verlag, Berlin); Gill et al. (1988) Nature 334:721-724. Such disclosures are herein incorporated by reference. The above list of selectable marker genes is not meant to be limiting. Any selectable marker gene can be used in the present invention.

### VI. Compositions Comprising Silencing Elements

One or more of the polynucleotides comprising the silencing element can be provided as an external composition such as a spray or powder to the plant, plant part, seed, a pest, or an area of cultivation. In another example, a plant is transformed with a DNA construct or expression cassette for expression of at least one silencing element. In either composition, the silencing element, when ingested by an insect, can reduce the level of a target pest sequence and thereby control the pest (i.e., a Coleopteran plant pest including a *Diabrotica* plant pest, such as, *D. virgifera virgifera*, *D. barberi*, *D. virgifera zeae, D. speciosa,* or *D. undecimpunctata howardi*). It is recognized that the composition can comprise a cell (such as plant cell or a bacterial cell), in which a polynucleotide encoding the silencing element is stably incorporated into the genome and operably linked to promoters active in the cell. Compositions comprising a mixture of cells, some cells expressing at least one silencing element are also encompassed. In other embodiments, compositions comprising the silencing elements are not contained in a cell. In such embodiments, the composition can be applied to an area inhabited by a pest. In one embodiment, the composition is applied externally to a plant (i.e., by spraying a field or area of cultivation) to protect the plant from the pest.. Methods of applying nucleotides in such a manner are known to those of skill in the art.

The composition of the invention can further be formulated as bait. In this embodiment, the compositions comprise a food substance or an attractant which enhances the attractiveness of the composition to the pest.

The composition comprising the silencing element can be formulated in an agriculturally suitable and/or environmentally acceptable carrier. Such carriers can be any material that the animal, plant or environment to be treated can tolerate. Furthermore, the carrier must be such that the composition remains effective at controlling a pest. Examples of such carriers include water, saline, Ringer's solution, dextrose or other sugar solutions, Hank's solution, and other aqueous physiologically balanced salt solutions, phosphate buffer, bicarbonate buffer and Tris buffer. In addition, the composition may include compounds that increase the half-life of a composition. Various insecticidal formulations can also be found in, for example, US Publications 2008/0275115, 2008/0242174, 2008/0027143, 2005/0042245, and 2004/0127520, each of which is herein incorporated by reference.

It is recognized that the polynucleotides comprising sequences encoding the silencing element can be used to transform organisms to provide for host organism production of these components, and subsequent application of the host organism to the environment of the target pest(s). Such host organisms include baculoviruses, bacteria, and the like. In this manner, the combination of polynucleotides encoding the silencing element may be introduced via a suitable vector into a microbial host, and said host applied to the environment, or to plants or animals.

The term "introduced" in the context of inserting a nucleic acid into a cell, means "transfection" or "transformation" or "transduction" and includes reference to the incorporation of a nucleic acid into a eukaryotic or prokaryotic cell where the nucleic acid may be stably incorporated into the genome of the cell (e.g., chromosome, plasmid, plastid, or mitochondrial DNA), converted into an autonomous replicon, or transiently expressed (e.g., transfected mRNA).

Microbial hosts that are known to occupy the "phytosphere" (phylloplane, phyllosphere, rhizosphere, and/or rhizoplana) of one or more crops of interest may be selected. These microorganisms are selected so as to be capable of successfully competing in the particular environment with the wild-type microorganisms, provide for stable maintenance and expression of the sequences encoding the silencing element, and desirably, provide for improved protection of the components from environmental degradation and inactivation.

Such microorganisms include bacteria, algae, and fungi. Of particular interest are microorganisms such as bacteria, e.g., *Pseudomonas, Erwinia, Serratia, Klebsiella, Xanthomonas, Streptomyces, Rhizobium, Rhodopseudomonas, Methylius, Agrobacterium, Acetobacter, Lactobacillus, Arthrobacter, Azotobacter, Leuconostoc,* and *Alcaligenes,* fungi, particularly yeast, e.g., *Saccharomyces, Cryptococcus, Kluyveromyces, Sporobolomyces, Rhodotorula,* and *Aureobasidium.* Of particular interest are such phytosphere bacterial species as *Pseudomonas syringae, Pseudomonas fluorescens, Serratia marcescens, Acetobacter xylinum, Agrobacteria, Rhodopseudomonas spheroides, Xanthomonas campestris, Rhizobium melioti, Alcaligenes entrophus, Clavibacter xyli* and *Azotobacter vinlandir,* and phytosphere yeast species such as *Rhodotorula rubra, R. glutinis, R. marina, R. aurantiaca, Cryptococcus albidus, C. diffluens, C. laurentii, Saccharomyces rosei, S. pretoriensis, S. cerevisiae, Sporobolomyces rosues, S. odorus, Kluyveromyces veronae,* and *Aureobasidium pollulans.* Of particular interest are the pigmented microorganisms.

A number of ways are available for introducing the polynucleotide comprising the silencing element into the microbial host under conditions that allow for stable maintenance and expression of such nucleotide encoding sequences. For example, expression cassettes can be constructed which include the nucleotide constructs of interest operably linked with the transcriptional and translational regulatory signals for expression of the nucleotide constructs, and a nucleotide sequence homologous with a sequence in the host organism, whereby integration will occur, and/or a replication system that is functional in the host, whereby integration or stable maintenance will occur.

Transcriptional and translational regulatory signals include, but are not limited to, promoters, transcriptional initiation start sites, operators, activators, enhancers, other regulatory elements, ribosomal binding sites, an initiation codon, termination signals, and the like. See, for example, U.S. Patent Nos. 5,039,523 and 4,853,331; EPO 0480762A2; Sambrook et al. (2000); Molecular Cloning: A Laboratory Manual (3rd ed.; Cold Spring Harbor Laboratory Press, Plainview, NY); Davis et al. (1980) Advanced Bacterial Genetics (Cold Spring Harbor Laboratory, Cold Spring Harbor, NY); and the references cited therein.

Suitable host cells include the prokaryotes and the lower eukaryotes, such as fungi. Illustrative prokaryotes, both Gram-negative and Gram-positive, include *Enterobacteriaceae,* such as *Escherichia, Erwinia, Shigella, Salmonella, and Proteus*; *Bacillaceae*; *Rhizobiceae,* such as *Rhizobium*; *Spirillaceae,* such as photobacterium, *Zymomonas* , *Serratia*, *Aeromonas, Vibrio*, *Desulfovibrio, Spirillum; Lactobacillaceae*; *Pseudomonadaceae,* such as *Pseudomonas* and *Acetobacter; Azotobacteraceae* and *Nitrobacteraceae.* Among eukaryotes are fungi, such as *Phycomycetes* and *Ascomycetes,* which includes yeast, such as *Saccharomyces* and *Schizosaccharomyces;* and *Basidiomycetes* yeast, such as *Rhodotorula*, *Aureobasidium, Sporobolomyces,* and the like.

Characteristics of particular interest in selecting a host cell for purposes of the invention include ease of introducing the coding sequence into the host, availability of expression systems, efficiency of expression, stability in the host, and the presence of auxiliary genetic capabilities. Characteristics of interest for use as a pesticide microcapsule include protective qualities, such as thick cell walls, pigmentation, and intracellular packaging or formation of inclusion bodies; leaf affinity; lack of mammalian toxicity; attractiveness to pests for ingestion; and the like. Other considerations include ease of formulation and handling, economics, storage stability, and the like.

Host organisms of particular interest include yeast, such as *Rhodotorula spp*., *Aureobasidium spp., Saccharomyces spp.,* and *Sporobolomyces spp.,* phylloplane organisms such as *Pseudomonas spp., Erwinia spp.,* and *Flavobacterium spp.,* and other such organisms, including *Pseudomonas aeruginosa*, *Pseudomonas fluorescens, Saccharomyces cerevisiae, Bacillus thuringiensis, Escherichia coli, Bacillus subtilis,* and the like.

The sequences encoding the silencing elements encompassed by the invention can be introduced into microorganisms that multiply on plants (epiphytes) to deliver these components to potential target pests. Epiphytes, for example, can be gram-positive or gram-negative bacteria.

The silencing element can be fermented in a bacterial host and the resulting bacteria processed and used as a microbial spray in the same manner that *Bacillus thuringiensis* strains have been used as insecticidal sprays. Any suitable microorganism can be used for this purpose. By way of example, *Pseudomonas* has been used to express *Bacillus thuringiensis* endotoxins as encapsulated proteins and the resulting cells processed and sprayed as an insecticide Gaertner et al. (1993), in Advanced Engineered Pesticides, ed. L. Kim (Marcel Decker, Inc.).

Alternatively, the components of the invention are produced by introducing heterologous genes into a cellular host. Expression of the heterologous sequences results, directly or indirectly, in the intracellular production of the silencing element. These compositions may then be formulated in accordance with conventional techniques for application to the environment hosting a target pest, e.g., soil, water, and foliage of plants. See, for example, EPA 0192319, and the references cited therein.

In the present invention, a transformed microorganism can be formulated with an acceptable carrier into separate or combined compositions that are, for example, a suspension, a solution, an emulsion, a dusting powder, a dispersible granule, a wettable powder, and an emulsifiable concentrate, an aerosol, an impregnated granule, an adjuvant, a coatable paste, and also encapsulations in, for example, polymer substances.

Such compositions disclosed above may be obtained by the addition of a surface-active agent, an inert carrier, a preservative, a humectant, a feeding stimulant, an attractant, an encapsulating agent, a binder, an emulsifier, a dye, a UV protectant, a buffer, a flow agent or fertilizers, micronutrient donors, or other preparations that influence plant growth. One or more agrochemicals including, but not limited to, herbicides, insecticides, fungicides, bactericides, nematicides, molluscicides, acaracides, plant growth regulators, harvest aids, and fertilizers, can be combined with carriers, surfactants or adjuvants customarily employed in the art of formulation or other components to facilitate product handling and application for particular target pests. Suitable carriers and adjuvants can be solid or liquid and correspond to the substances ordinarily employed in formulation technology, e.g., natural or regenerated mineral substances, solvents, dispersants, wetting agents, tackifiers, binders, or fertilizers. The active ingredients of the present invention (i.e., at least one silencing element) are normally applied in the form of compositions and can be applied to the crop area, plant, or seed to be treated. For example, the compositions may be applied to grain in preparation for or during storage in a grain bin or silo, *etc.* The compositions may be applied simultaneously or in succession with other compounds. Methods of applying an active ingredient or a composition that contains at least one silencing element include, but are not limited to, foliar application, seed coating, and soil application. The number of applications and the rate of application depend on the intensity of infestation by the corresponding pest.

Suitable surface-active agents include, but are not limited to, anionic compounds such as a carboxylate of, for example, a metal; carboxylate of a long chain fatty acid; an N-acylsarcosinate; mono- or di-esters of phosphoric acid with fatty alcohol ethoxylates or salts of such esters; fatty alcohol sulfates such as sodium dodecyl sulfate, sodium octadecyl sulfate, or sodium cetyl sulfate; ethoxylated fatty alcohol sulfates; ethoxylated alkylphenol sulfates; lignin sulfonates; petroleum sulfonates; alkyl aryl sulfonates such as alkyl-benzene sulfonates or lower alkylnaphtalene sulfonates, e.g., butyl-naphthalene sulfonate; salts of sulfonated naphthalene-formaldehyde condensates; salts of sulfonated phenol-formaldehyde condensates; more complex sulfonates such as the amide sulfonates, e.g., the sulfonated condensation product of oleic acid and N-methyl taurine; or the dialkyl sulfosuccinates, e.g., the sodium sulfonate or dioctyl succinate. Non-ionic agents include condensation products of fatty acid esters, fatty alcohols, fatty acid amides or fatty-alkyl- or alkenyl-substituted phenols with ethylene oxide, fatty esters of polyhydric alcohol ethers, e.g., sorbitan fatty acid esters, condensation products of such esters with ethylene oxide, e.g., polyoxyethylene sorbitan fatty acid esters, block copolymers of ethylene oxide and propylene oxide, acetylenic glycols such as 2,4,7,9-tetraethyl-5-decyn-4,7-diol, or ethoxylated acetylenic glycols. Examples of a cationic surface-active agent include, for instance, an aliphatic mono-, di-, or polyamine such as an acetate, naphthenate or oleate; or oxygen-containing amine such as an amine oxide of polyoxyethylene alkylamine; an amide-linked amine prepared by the condensation of a carboxylic acid with a di- or polyamine; or a quaternary ammonium salt.

Examples of inert materials include, but are not limited to, inorganic minerals such as kaolin, phyllosilicates, carbonates, sulfates, phosphates, or botanical materials such as cork, powdered corncobs, peanut hulls, rice hulls, and walnut shells.

The compositions comprising the silencing element can be in a suitable form for direct application or as a concentrate of primary composition that requires dilution with a suitable quantity of water or other dilutant before application.

The compositions (including the transformed microorganisms) can be applied to the environment of an insect pest (such as a Coleoptera plant pest or a *Diabrotica* plant pest) by, for example, spraying, atomizing, dusting, scattering, coating or pouring, introducing into or on the soil, introducing into irrigation water, by seed treatment or general application or dusting at the time when the pest has begun to appear or before the appearance of pests as a protective measure. For example, the composition(s) and/or transformed microorganism(s) may be mixed with grain to protect the grain during storage. It is generally important to obtain good control of pests in the early stages of plant growth, as this is the time when the plant can be most severely damaged. The compositions can conveniently contain another insecticide if this is thought necessary. In an embodiment of the invention, the composition(s) is applied directly to the soil, at a time of planting, in granular form of a composition of a carrier and dead cells of a *Bacillus* strain or transformed microorganism of the invention. Another embodiment is a granular form of a composition comprising an agrochemical such as, for example, a herbicide, an insecticide, a fertilizer, in an inert carrier, and dead cells of a *Bacillus* strain or transformed microorganism of the invention.

### VII. Plants, Plant Parts, and Methods of Introducing Sequences into Plants

In one embodiment, the methods of the invention involve introducing a polynucleotide into a plant. "Introducing" is intended to mean presenting to the plant the polynucleotide in such a manner that the sequence gains access to the interior of a cell of the plant. The methods of the invention do not depend on a particular method for introducing a sequence into a plant, only that the polynucleotide or polypeptides gains access to the interior of at least one cell of the plant. Methods for introducing polynucleotides into plants are known in the art including, but not limited to, stable transformation methods, transient transformation methods, and virus-mediated methods.

"Stable transformation" is intended to mean that the nucleotide construct introduced into a plant integrates into the genome of the plant and is capable of being inherited by the progeny thereof. "Transient transformation" is intended to mean that a polynucleotide is introduced into the plant and does not integrate into the genome of the plant or a polypeptide is introduced into a plant.

Transformation protocols as well as protocols for introducing polypeptides or polynucleotide sequences into plants may vary depending on the type of plant or plant cell, i.e., monocot or dicot, targeted for transformation. Suitable methods of introducing polypeptides and polynucleotides into plant cells include microinjection (Crossway et al. (1986) Biotechniques 4:320-334), electroporation (Riggs et al. (1986) Proc. Natl. Acad. Sci. USA 83:5602-5606, *Agrobacterium-mediated* transformation (U.S. Patent No. 5,563,055 and U.S. Patent No. 5,981,840), direct gene transfer (Paszkowski et al. (1984) EMBO J. 3:2717-2722), and ballistic particle acceleration (see, for example, U.S. Patent Nos. 4,945,050; U.S. Patent No. 5,879,918; U.S. Patent No. 5,886,244; and, 5,932,782; Tomes et al. (1995) in Plant Cell, Tissue, and Organ Culture: Fundamental Methods, ed. Gamborg and Phillips (Springer-Verlag, Berlin); McCabe et al. (1988) Biotechnology 6:923-926); and Lecl transformation (WO 00/28058). Also see Weissinger et al. (1988) Ann. Rev. Genet. 22:421-477; Sanford et al. (1987) Particulate Science and Technology 5:27-37 (onion); Christou et al. (1988) Plant Physiol. 87:671-674 (soybean); McCabe et al. (1988) Bio/Technology 6:923-926 (soybean); Finer and McMullen (1991) In Vitro Cell Dev. Biol. 27P:175-182 (soybean); Singh et al. (1998) Theor. Appl. Genet. 96:319-324 (soybean); Datta et al. (1990) Biotechnology 8:736-740 (rice); Klein et al. (1988) Proc. Natl. Acad. Sci. USA 85:4305-4309 (maize); Klein et al. (1988) Biotechnology 6:559-563 (maize); U.S. Patent Nos. 5,240,855; 5,322,783; and, 5,324,646; Klein et al. (1988) Plant Physiol. 91:440-444 (maize); Fromm et al. (1990) Biotechnology 8:833-839 (maize); Hooykaas-Van Slogteren et al. (1984) Nature (London) 311:763-764; U.S. Patent No. 5,736,369 (cereals); Bytebier et al. (1987) Proc. Natl. Acad. Sci. USA 84:5345-5349 (Liliaceae); De Wet et al. (1985) in The Experimental Manipulation of Ovule Tissues, ed. Chapman et al. (Longman, New York), pp. 197-209 (pollen); Kaeppler et al. (1990) Plant Cell Reports 9:415-418 and Kaeppler et al. (1992) Theor. Appl. Genet. 84:560-566 (whisker-mediated transformation); D'Halluin et al. (1992) Plant Cell 4:1495-1505 (electroporation); Li et al. (1993) Plant Cell Reports 12:250-255 and Christou and Ford (1995) Annals of Botany 75:407-413 (rice); Osjoda et al. (1996) Nature Biotechnology 14:745-750 (maize via *Agrobacterium tumefaciens*); all of which are herein incorporated by reference.

In specific embodiments, the silencing element sequences of the invention can be provided to a plant using a variety of transient transformation methods. Such transient transformation methods include, but are not limited to, the introduction of the protein or variants and fragments thereof directly into the plant or the introduction of the transcript into the plant. Such methods include, for example, microinjection or particle bombardment. See, for example, Crossway et al. (1986) Mol Gen. Genet. 202:179-185; Nomura et al. (1986) Plant Sci. 44:53-58; Hepler et al. (1994) Proc. Natl. Acad. Sci. 91: 2176-2180 and Hush et al. (1994) The Journal of Cell Science 107:775-784, all of which are herein incorporated by reference. Alternatively, polynucleotides can be transiently transformed into the plant using techniques known in the art. Such techniques include viral vector systems and the precipitation of the polynucleotide in a manner that precludes subsequent release of the DNA. Thus, the transcription from the particle-bound DNA can occur, but the frequency with which it is released to become integrated into the genome is greatly reduced. Such methods include the use of particles coated with polyethylimine (PEI; Sigma #P3143).

In other embodiments, the polynucleotide of the invention may be introduced into plants by contacting plants with a virus or viral nucleic acids. Generally, such methods involve incorporating a nucleotide construct of the invention within a viral DNA or RNA molecule. Further, it is recognized that promoters of the invention also encompass promoters utilized for transcription by viral RNA polymerases. Methods for introducing polynucleotides into plants and expressing a protein encoded therein, involving viral DNA or RNA molecules, are known in the art. See, for example, U.S. Patent Nos. 5,889,191, 5,889,190, 5,866,785, 5,589,367, 5,316,931, and Porta et al. (1996) Molecular Biotechnology 5:209-221; herein incorporated by reference.

Methods are known in the art for the targeted insertion of a polynucleotide at a specific location in the plant genome. In one embodiment, the insertion of the polynucleotide at a desired genomic location is achieved using a site-specific recombination system. See, for example, WO99/25821, WO99/25854, WO99/25840, WO99/25855, and WO99/25853, all of which are herein incorporated by reference. Briefly, the polynucleotide of the invention can be contained in transfer cassette flanked by two non-recombinogenic recombination sites. The transfer cassette is introduced into a plant having stably incorporated into its genome a target site which is flanked by two non-recombinogenic recombination sites that correspond to the sites of the transfer cassette. An appropriate recombinase is provided and the transfer cassette is integrated at the target site. The polynucleotide of interest is thereby integrated at a specific chromosomal position in the plant genome.

The cells that have been transformed may be grown into plants in accordance with conventional ways. See, for example, McCormick et al. (1986) Plant Cell Reports 5:81-84. These plants may then be grown, and either pollinated with the same transformed strain or different strains, and the resulting progeny having constitutive expression of the desired phenotypic characteristic identified. Two or more generations may be grown to ensure that expression of the desired phenotypic characteristic is stably maintained and inherited and then seeds harvested to ensure expression of the desired phenotypic characteristic has been achieved. In this manner, the present invention provides transformed seed (also referred to as "transgenic seed") having a polynucleotide of the invention, for example, an expression cassette of the invention, stably incorporated into their genome.

As used herein, the term plant includes plant cells, plant protoplasts, plant cell tissue cultures from which plants can be regenerated, plant calli, plant clumps, and plant cells that are intact in plants or parts of plants such as embryos, pollen, ovules, seeds, leaves, flowers, branches, fruit, kernels, ears, cobs, husks, stalks, roots, root tips, anthers, and the like. Grain is intended to mean the mature seed produced by commercial growers for purposes other than growing or reproducing the species. Progeny, variants, and mutants of the regenerated plants are also included within the scope of the invention, provided that these parts comprise the introduced polynucleotides.

The present invention may be used for transformation of any plant species, including, but not limited to, monocots and dicots. Examples of plant species of interest include, but are not limited to, corn (*Zea mays*), *Brassica* sp. (e.g., *B. napus, B. rapa, B. juncea*), particularly those *Brassica* species useful as sources of seed oil, alfalfa (*Medicago sativa*), rice (*Oryza sativa*), rye (*Secale cereale*), sorghum (*Sorghum bicolor, Sorghum vulgare*), millet (e.g., pearl millet (*Pennisetum glaucum*), proso millet (*Panicum miliaceum*), foxtail millet (*Setaria italica*), finger millet (*Eleusine coracana*)), sunflower (*Helianthus annuus*), safflower (*Carthamus tinctorius*), wheat (*Triticum aestivum*), soybean (*Glycine max*), tobacco (*Nicotiana tabacum*), potato (*Solanum tuberosum*), peanuts (*Arachis hypogaea*)*,* cotton (*Gossypium barbadense*, *Gossypium hirsutum*), sweet potato (*Ipomoea batatus*), cassava *(Manihot esculenta),* coffee (*Coffea* spp.), coconut (*Cocos nucifera*), pineapple (*Ananas comosus*), citrus trees (*Citrus* spp.), cocoa (*Theobroma cacao*), tea *(Camellia sinensis),* banana (*Musa* spp.), avocado *(Persea americana),* fig (*Ficus casica*), guava (*Psidium guajava*), mango *(Mangifera indica),* olive (*Olea europaea*), papaya (*Carica papaya*), cashew (*Anacardium occidentale*), macadamia (*Macadamia integrifolia*), almond (*Prunus amygdalus*), sugar beets *(Beta vulgaris),* sugarcane (*Saccharum* spp.), oats, barley, vegetables, ornamentals, and conifers.

Vegetables include tomatoes (*Lycopersicon esculentum*), lettuce (e.g., *Lactuca sativa*), green beans (*Phaseolus vulgaris*), lima beans (*Phaseolus limensis*), peas (*Lathyrus* spp.), and members of the genus *Cucumis* such as cucumber (*C*. *sativus),* cantaloupe (*C*. *cantalupensis*), and musk melon (*C*. *melo*). Ornamentals include azalea (*Rhododendron* spp.), hydrangea (*Macrophylla hydrangea*), hibiscus (*Hibiscus rosasanensis*), roses (*Rosa* spp.), tulips (*Tulipa* spp.), daffodils (*Narcissus* spp.), petunias (*Petunia hybrida*), carnation (*Dianthus caryophyllus*), poinsettia (*Euphorbia pulcherrima*), and chrysanthemum.

Conifers that may be employed in practicing the present invention include, for example, pines such as loblolly pine (*Pinus taeda*), slash pine (*Pinus elliotii*), ponderosa pine (*Pinus ponderosa*), lodgepole pine (*Pinus contorta*), and Monterey pine (*Pinus radiata*); Douglas-fir (*Pseudotsuga menziesii*); Western hemlock (*Tsuga canadensis*); Sitka spruce (*Picea glauca*); redwood (*Sequoia sempervirens*); true firs such as silver fir (*Abies amabilis*) and balsam fir (*Abies balsamea*); and cedars such as Western red cedar (*Thuja plicata*) and Alaska yellow-cedar (*Chamaecyparis nootkatensis*). In specific embodiments, plants of the present invention are crop plants (for example, corn, alfalfa, sunflower, *Brassica,* soybean, cotton, safflower, peanut, sorghum, wheat, millet, tobacco, etc.). In other embodiments, corn and soybean plants and sugarcane plants are optimal, and in yet other embodiments corn plants are optimal.

Other plants of interest include grain plants that provide seeds of interest, oil-seed plants, and leguminous plants. Seeds of interest include grain seeds, such as corn, wheat, barley, rice, sorghum, rye, etc. Oil-seed plants include cotton, soybean, safflower, sunflower, *Brassica,* maize, alfalfa, palm, coconut, etc. Leguminous plants include beans and peas. Beans include guar, locust bean, fenugreek, soybean, garden beans, cowpea, mungbean, lima bean, fava bean, lentils, chickpea, etc.

### VIII. Stacking of Traits in Transgenic Plant

Transgenic plants may comprise a stack of one or more target polynucleotides as set forth in SEQ ID NOS: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants or fragments thereof, or complements thereof, as disclosed herein with one or more additional polynucleotides resulting in the production or suppression of multiple polypeptide sequences. Transgenic plants comprising stacks of polynucleotide sequences can be obtained by either or both of traditional breeding methods or through genetic engineering methods. These methods include, but are not limited to, breeding individual lines each comprising a polynucleotide of interest, transforming a transgenic plant comprising an expression construct comprising various target polynucleotides as set forth in SEQ ID NOS: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants or fragments thereof, or complements thereof, as disclosed herein with a subsequent gene and co-transformation of genes into a single plant cell. As used herein, the term "stacked" includes having the multiple traits present in the same plant (i.e., both traits are incorporated into the nuclear genome, one trait is incorporated into the nuclear genome and one trait is incorporated into the genome of a plastid or both traits are incorporated into the genome of a plastid). In one non-limiting example, "stacked traits" comprise a molecular stack where the sequences are physically adjacent to each other. A trait, as used herein, refers to the phenotype derived from a particular sequence or groups of sequences. Co-transformation of polynucleotides can be carried out using single transformation vectors comprising multiple polynucleotides or polynucleotides carried separately on multiple vectors. If the sequences are stacked by genetically transforming the plants, the polynucleotide sequences of interest can be combined at any time and in any order. The traits can be introduced simultaneously in a co-transformation protocol with the polynucleotides of interest provided by any combination of transformation cassettes. For example, if two sequences will be introduced, the two sequences can be contained in separate transformation cassettes (trans) or contained on the same transformation cassette (cis). Expression of the sequences can be driven by the same promoter or by different promoters. It is further recognized that polynucleotide sequences can be stacked at a desired genomic location using a site-specific recombination system. *See,* for example, WO 1999/25821, WO 1999/25854, WO 1999/25840, WO 1999/25855 and WO 1999/25853, all of which are herein incorporated by reference.

In some embodiments the various target polynucleotides as set forth in SEQ ID NOS: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants or fragments thereof, or complements thereof, as disclosed herein, alone or stacked with one or more additional insect resistance traits can be stacked with one or more additional input traits (e.g., herbicide resistance, fungal resistance, virus resistance, stress tolerance, disease resistance, male sterility, stalk strength, and the like) or output traits (e.g., increased yield, modified starches, improved oil profile, balanced amino acids, high lysine or methionine, increased digestibility, improved fiber quality, drought resistance, and the like). Thus, the polynucleotide embodiments can be used to provide a complete agronomic package of improved crop quality with the ability to flexibly and cost effectively control any number of agronomic pests.

Transgenes useful for stacking include, but are not limited to, to those as described herein below.

### i. Transgenes that Confer Resistance to Insects or Disease

(A) Plant disease resistance genes. Plant defenses are often activated by specific interaction between the product of a disease resistance gene (R) in the plant and the product of a corresponding avirulence (Avr) gene in the pathogen. A plant variety can be transformed with cloned resistance gene to engineer plants that are resistant to specific pathogen strains. *See,* for example, Jones, et al., (1994) Science 266:789 (cloning of the tomato Cf-9 gene for resistance to Cladosporium fulvum); Martin, et al., (1993) Science 262:1432 (tomato Pto gene for resistance to Pseudomonas syringae pv. tomato encodes a protein kinase); Mindrinos, et al., (1994) Cell 78:1089 (Arabidopsis RSP2 gene for resistance to Pseudomonas syringae), McDowell and Woffenden, (2003) Trends Biotechnol. 21(4):178-83 and Toyoda, et al., (2002) Transgenic Res. 11(6):567-82. A plant resistant to a disease is one that is more resistant to a pathogen as compared to the wild type plant.
(B) Genes encoding a Bacillus thuringiensis protein, a derivative thereof or a synthetic polypeptide modeled thereon. *See,* for example, Geiser, et al., (1986) Gene 48:109, who disclose the cloning and nucleotide sequence of a Bt delta-endotoxin gene. Moreover, DNA molecules encoding delta-endotoxin genes can be purchased from American Type Culture Collection (Rockville, Md.), for example, under ATCC Accession Numbers 40098, 67136, 31995 and 31998. Other non-limiting examples of Bacillus thuringiensis transgenes being genetically engineered are given in the following patents and patent applications and hereby are incorporated by reference for this purpose: U.S. Pat. Nos. 5,188,960; 5,689,052; 5,880,275; 5,986,177; 6,023,013, 6,060,594, 6,063,597, 6,077,824, 6,620,988, 6,642,030, 6,713,259, 6,893,826, 7,105,332; 7,179,965, 7,208,474; 7,227,056, 7,288,643, 7,323,556, 7,329,736, 7,449,552, 7,468,278, 7,510,878, 7,521,235, 7,544,862, 7,605,304, 7,696,412, 7,629,504, 7,705,216, 7,772,465, 7,790,846, 7,858,849 and WO 1991/14778; WO 1999/31248; WO 2001/12731; WO 1999/24581 and WO 1997/40162.
   Genes encoding pesticidal proteins may also be stacked including but are not limited to: insecticidal proteins from Pseudomonas sp. such as PSEEN3174 (Monalysin, (2011) PLoS Pathogens, 7:1-13), from Pseudomonas protegens strain CHAO and Pf-5 (previously fluorescens) (Pechy-Tarr, (2008) Environmental Microbiology 10:2368-2386: Gen Bank Accession No. EU400157); from Pseudomonas Taiwanensis (Liu, et al., (2010) J. Agric. Food Chem. 58:12343-12349) and from Pseudomonas pseudoalcligenes (Zhang, et al., (2009) Annals of Microbiology 59:45-50 and Li, et al., (2007) Plant Cell Tiss. Organ Cult. 89:159-168); insecticidal proteins from Photorhabdus sp. and Xenorhabdus sp. (Hinchliffe, et al., (2010) The Open Toxinology Journal 3:101-118 and Morgan, et al., (2001) Applied and Envir. Micro. 67:2062-2069), U.S. Pat. No. 6,048,838, and U.S. Pat. No. 6,379,946; and .delta.-endotoxins including, but not limited to, the Cry1, Cry2, Cry3, Cry4, Cry5, Cry6, Cry7, Cry8, Cry9, Cry10, Cry11, Cry12, Cry13, Cry14, Cry15, Cry16, Cry17, Cry18, Cry19, Cry20, Cry21, Cry22, Cry23, Cry24, Cry25, Cry26, Cry27, Cry 28, Cry 29, Cry 30, Cry31, Cry32, Cry33, Cry34, Cry35, Cry36, Cry37, Cry38, Cry39, Cry40, Cry41, Cry42, Cry43, Cry44, Cry45, Cry 46, Cry47, Cry49, Cry 51 and Cry55 classes of delta-endotoxin genes and the B. thuringiensis cytolytic Cyt1 and Cyt2 genes. Members of these classes of B. thuringiensis insecticidal proteins include, but are not limited to Cry1Aa1 (Accession #Accession #M11250), Cry1Aa2 (Accession #M10917), Cry1Aa3 (Accession #D00348), Cry1Aa4 (Accession #X13535), Cry1Aa5 (Accession #D17518), Cry1Aa6 (Accession #U43605), Cry1Aa7 (Accession #AF081790), Cry1Aa8 (Accession #126149), Cry1Aa9 (Accession #AB026261), Cry1Aa10 (Accession #AF154676), Cry1Aa11 (Accession #Y09663), Cry1Aa12 (Accession #AF384211), Cry1Aa13 (Accession #AF510713), Cry1Aa14 (Accession #AY197341), Cry1Aa15 (Accession #DQ062690), Cry1Ab1 (Accession #M13898), Cry1Ab2 (Accession #M12661), Cry1Ab3 (Accession #M15271), Cry1Ab4 (Accession #D00117), Cry1Ab5 (Accession #X04698), Cry1Ab6 (Accession #M37263), Cry1Ab7 (Accession #X13233), Cry1Ab8 (Accession #M16463), Cry1Ab9 (Accession #X54939), Cry1Ab10 (Accession #A29125), Cry1Ab11 (Accession #I12419), Cry1Ab12 (Accession #AF059670), Cry1Ab13 (Accession #AF254640), Cry1Ab14 (Accession #U94191), Cry1Ab15 (Accession #AF358861), Cry1Ab16 (Accession #AF375608), Cry1Ab17 (Accession #AAT46415), Cry1Ab18 (Accession #AAQ88259), Cry1Ab19 (Accession #AY847289), Cry1Ab20 (Accession #DQ241675), Cry1Ab21 (Accession #EF683163), Cry1Ab22 (Accession #ABW87320), Cry1Ab-like (Accession #AF327924), Cry1Ab-like (Accession #AF327925), Cry1Ab-like (Accession #AF327926), Cry1Ab-like (Accession #DQ781309), Cry1Ac1 (Accession #M11068), Cry1Ac2 (Accession #M35524), Cry1Ac3 (Accession #X54159), Cry1Ac4 (Accession #M73249), Cry1Ac5 (Accession #M73248), Cry1Ac6 (Accession #U43606), Cry1Ac7 (Accession #U87793), Cry1Ac8 (Accession #U87397), Cry1Ac9 (Accession #U89872), Cry1Ac10 (Accession #AJ002514), Cry1Ac11 (Accession #AJ130970), Cry1Ac12 (Accession #I12418), Cry1Ac13 (Accession #AF148644), Cry1Ac14 (Accession #AF492767), Cry1Ac15 (Accession #AY122057), Cry1Ac16 (Accession #AY730621), Cry1Ac17 (Accession #AY925090), Cry1Ac18 (Accession #DQ023296), Cry1Ac19 (Accession #DQ195217), Cry1Ac20 (Accession #DQ285666), Cry1Ac21 (Accession #DQ062689), Cry1Ac22 (Accession #EU282379), Cry1Ac23 (Accession #AM949588), Cry1Ac24 (Accession #ABL01535), Cry1Ad1 (Accession #M73250), Cry1Ad2 (Accession #A27531), CrylAel (Accession #M65252), CrylAfl (Accession #U82003), CrylAgl (Accession #AF081248), CrylAhl (Accession #AF281866), Cry1Ah2 (Accession #DQ269474), CrylAil (Accession #AY174873), Cry1A-like (Accession #AF327927), Cry1Ba1 (Accession #X06711), Cry1Ba2 (Accession #X95704), Cry1Ba3 (Accession #AF368257), Cry1Ba4 (Accession #AF363025), Cry1Ba5 (Accession #AB020894), Cry1Ba6 (Accession #ABL60921), Cry1Bb1 (Accession #L32020), Cry1Bc1 (Accession #Z46442), Cry1Bd1 (Accession #U70726), Cry1Bd2 (Accession #AY138457), Cry1Be1 (Accession #AF077326), Cry1Be2 (Accession #AAQ52387), Cry1Bf1 (Accession #AX189649), Cry1Bf2 (Accession #AAQ52380), Cry1Bg1 (Accession #AY176063), Cry1Ca1 (Accession #X07518), Cry1Ca2 (Accession #X13620), Cry1Ca3 (Accession #M73251), Cry1Ca4 (Accession #A27642), Cry1Ca5 (Accession #X96682), Cry1Ca6 [1] (Accession #AF215647), Cry1Ca7 (Accession #AY015492), Cry1Ca8 (Accession #AF362020), Cry1Ca9 (Accession #AY078160), Cry1Ca10 (Accession #AF540014), Cry1Ca11 (Accession #AY955268), Cry1Cb1 (Accession #M97880), Cry1Cb2 (Accession #AY007686), Cry1Cb3 (Accession #EU679502), Cry1Cb-like (Accession #AAX63901), Cry1Da1 (Accession #X54160), Cry1Da2 (Accession #176415), Cry1Db1 (Accession #Z22511), Cry1 Db2 (Accession #AF358862), Cry1 Dc1 (Accession #EF059913), Cry1Ea1 (Accession #X53985), Cry1Ea2 (Accession #X56144), Cry1Ea3 (Accession #M73252), Cry1Ea4 (Accession #U94323), Cry1Ea5 (Accession #A15535), Cry1Ea6 (Accession #AF202531), Cry1 Ea7 (Accession #AAW72936), Cry1 Ea8 (Accession #ABX11258), CrylEbl (Accession #M73253), Cry1Fa1 (Accession #M63897), Cry1Fa2 (Accession #M73254), Cry1Fb1 (Accession #Z22512), Cry1Fb2 (Accession #AB012288), Cry1Fb3 (Accession #AF062350), Cry1Fb4 (Accession #173895), Cry1Fb5 (Accession #AF336114), Cry1Fb6 (Accession #EU679500), Cry1Fb7 (Accession #EU679501), Cry1Ga1 (Accession #Z22510), Cry1Ga2 (Accession #Y09326), Cry1Gb1 (Accession #U70725), Cry1Gb2 (Accession #AF288683), CrylGc (Accession #AAQ52381), Cry1Ha1 (Accession #Z22513), Cry1Hb1 (Accession #U35780), Cry1H-like (Accession #AF182196), Cry1Ia1 (Accession #X62821), Cry1Ia2 (Accession #M98544), Cry1Ia3 (Accession #L36338), Cry1Ia4 (Accession #L49391), Cry1Ia5 (Accession #Y08920), Cry1Ia6 (Accession #AF076953), Cry1Ia7 (Accession #AF278797), Cry1Ia8 (Accession #AF373207), Cry1Ia9 (Accession #AF521013), Cry1Ia10 (Accession #AY262167), Cry1Ia11 (Accession #AJ315121), Cry1Ia12 (Accession #AAV53390), Cry1Ia13 (Accession #ABF83202), Cry1Ia14 (Accession #EU887515), Cry1Ib1 (Accession #U07642), Cry1Ib2 (Accession #ABW88019), Cry1Ib3 (Accession #EU677422), Cry1Ic1 (Accession #AF056933), Cry1Ic2 (Accession #AAE71691), Cry1Id1 (Accession #AF047579), Cry1Ie1 (Accession #AF211190), Cry1If1 (Accession #AAQ52382), Cry1I-like (Accession #190732), Cry1I-like (Accession #DQ781310), Cry1Ja1 (Accession #L32019), Cry1Jb1 (Accession #U31527), Cry1Jc1 (Accession #190730), Cry1Jc2 (Accession #AAQ52372), Cry1Jd1 (Accession #AX189651), Cry1Ka1 (Accession #U28801), Cry1La1 (Accession #AAS60191), Cryl-like (Accession #190729), Cry2Aa1 (Accession #M31738), Cry2Aa2 (Accession #M23723), Cry2Aa3 (Accession #D86064), Cry2Aa4 (Accession #AF047038), Cry2Aa5 (Accession #AJ 132464), Cry2Aa6 (Accession #AJ 132465), Cry2Aa7 (Accession #AJ132463), Cry2Aa8 (Accession #AF252262), Cry2Aa9 (Accession #AF273218), Cry2Aa10 (Accession #AF433645), Cry2Aa11 (Accession #AAQ52384), Cry2Aa12 (Accession #DQ977646), Cry2Aa13 (Accession #ABL01536), Cry2Aa14 (Accession #ACF04939), Cry2Ab1 (Accession #M23724), Cry2Ab2 (Accession #X55416), Cry2Ab3 (Accession #AF164666), Cry2Ab4 (Accession #AF336115), Cry2Ab5 (Accession #AF441855), Cry2Ab6 (Accession #AY297091), Cry2Ab7 (Accession #DQ119823), Cry2Ab8 (Accession #DQ361266), Cry2Ab9 (Accession #DQ341378), Cry2Ab10 (Accession #EF157306), Cry2Ab11 (Accession #AM691748), Cry2Ab12 (Accession #ABM21764), Cry2Ab13 (Accession #EU909454), Cry2Ab14 (Accession #EU909455), Cry2Ac1 (Accession #X57252), Cry2Ac2 (Accession #AY007687), Cry2Ac3 (Accession #AAQ52385), Cry2Ac4 (Accession #DQ361267), Cry2Ac5 (Accession #DQ341379), Cry2Ac6 (Accession #DQ359137), Cry2Ac7 (Accession #AM292031), Cry2Ac8 (Accession #AM421903), Cry2Ac9 (Accession #AM421904), Cry2Ac10 (Accession #BI 877475), Cry2Ac11 (Accession #AM689531), Cry2Ac12 (Accession #AM689532), Cry2Ad1 (Accession #AF200816), Cry2Ad2 (Accession #DQ358053), Cry2Ad3 (Accession #AM268418), Cry2Ad4 (Accession #AM490199), Cry2Ad5 (Accession #AM765844), Cry2Ae1 (Accession #AAQ52362), Cry2Af1 (Accession #EF439818), Cry2Ag (Accession #ACH91610), Cry2Ah (Accession #EU939453), Cry3Aa1 (Accession #M22472), Cry3Aa2 (Accession #J02978), Cry3Aa3 (Accession #Y00420), Cry3Aa4 (Accession #M30503), Cry3Aa5 (Accession #M37207), Cry3Aa6 (Accession #U10985), Cry3Aa7 (Accession #AJ237900), Cry3Aa8 (Accession #AAS79487), Cry3Aa9 (Accession #AAWO5659), Cry3Aa10 (Accession #AAU29411), Cry3Aa11 (Accession #AY882576), Cry3Aa12 (Accession #ABY49136), Cry3Ba1 (Accession #X17123), Cry3Ba2 (Accession #A07234), Cry3Bb1 (Accession #M89794), Cry3Bb2 (Accession #U31633), Cry3Bb3 (Accession #I15475), Cry3Ca1 (Accession #X59797), Cry4Aa1 (Accession #Y00423), Cry4Aa2 (Accession #D00248), Cry4Aa3 (Accession #AL731825), Cry4A-like (Accession #DQ078744), Cry4Ba1 (Accession #X07423), Cry4Ba2 (Accession #X07082), Cry4Ba3 (Accession #M20242), Cry4Ba4 (Accession #D00247), Cry4Ba5 (Accession #AL731825), Cry4Ba-like (Accession #ABC47686), Cry4Ca1 (Accession #EU646202), Cry5Aa1 (Accession #L07025), Cry5Ab1 (Accession #L07026), Cry5Ac1 (Accession #134543), Cry5Ad1 (Accession #EF219060), Cry5Ba1 (Accession #U19725), Cry5Ba2 (Accession #EU121522), Cry6Aa1 (Accession #L07022), Cry6Aa2 (Accession #AF499736), Cry6Aa3 (Accession #DQ835612), Cry6Ba1 (Accession #L07024), Cry7Aa1 (Accession #M64478), Cry7Ab1 (Accession #U04367), Cry7Ab2 (Accession #U04368), Cry7Ab3 (Accession #BI 1015188), Cry7Ab4 (Accession #EU380678), Cry7Ab5 (Accession #ABX9555), Cry7Ab6 (Accession #FJ194973), Cry7Ba1 (Accession #ABB70817), Cry7Ca1 (Accession #EF486523), Cry8Aa1 (Accession #U04364), Cry8Ab1 (Accession #EU044830), Cry8Ba1 (Accession #U04365), Cry8Bb1 (Accession #AX543924), Cry8Bc1 (Accession #AX543926), Cry8Ca1 (Accession #U04366), Cry8Ca2 (Accession #AAR98783), Cry8Ca3 (Accession #EU625349), Cry8Da1 (Accession #AB089299), Cry8Da2 (Accession #BD133574), Cry8Da3 (Accession #BD133575), Cry8 Db1 (Accession #AB303980), Cry8Ea1 (Accession #AY329081), Cry8Ea2 (Accession #EU047597), Cry8Fa1 (Accession #AY551093), Cry8Ga1 (Accession #AY590188), Cry8Ga2 (Accession #DQ318860), Cry8Ga3 (Accession #FJ198072), Cry8Ha1 (Accession #EF465532), Cry81a1 (Accession #EU381044), Cry8Ja1 (Accession #EU625348), Cry8 like (Accession #ABS53003), Cry9Aa1 (Accession #X58120), Cry9Aa2 (Accession #X58534), Cry9Aa like (Accession #AAQ52376), Cry9Ba1 (Accession #X75019), Cry9Bb1 (Accession #AY758316), Cry9Ca1 (Accession #Z37527), Cry9Ca2 (Accession #AAQ52375), Cry9Da1 (Accession #D85560), Cry9Da2 (Accession #AF042733), Cry9 Db1 (Accession #AY971349), Cry9Ea1 (Accession #AB011496), Cry9Ea2 (Accession #AF358863), Cry9Ea3 (Accession #EF157307), Cry9Ea4 (Accession #EU760456), Cry9Ea5 (Accession #EU789519), Cry9Ea6 (Accession #EU887516), Cry9Eb1 (Accession #AX189653), Cry9Ec1 (Accession #AF093107), Cry9Ed1 (Accession #AY973867), Cry9 like (Accession #AF093107), Cry10Aa1 (Accession #M12662), Cry10Aa2 (Accession #E00614), Cry10Aa3 (Accession #AL731825), Cry10A like (Accession #DQ167578), Cry1IAa1 (Accession #M31737), Cry1IAa2 (Accession #M22860), Cry1IAa3 (Accession #AL731825), Cry1IAa-like (Accession #DQ166531), Cry11Ba1 (Accession #X86902), Cry11Bb1 (Accession #AF017416), Cry12Aa1 (Accession #L07027), Cry13Aa1 (Accession #L07023), Cry14Aa1 (Accession #U13955), Cry15Aa1 (Accession #M76442), Cry16Aa1 (Accession #X94146), Cry17Aa1 (Accession #X99478), Cry18Aa1 (Accession #X99049), Cry18Ba1 (Accession #AF169250), Cry18Ca1 (Accession #AF169251), Cry19Aa1 (Accession #Y07603), Cry19Ba1 (Accession #D88381), Cry20Aa1 (Accession #U82518), Cry21Aa1 (Accession #132932), Cry21Aa2 (Accession #166477), Cry21Ba1 (Accession #AB088406), Cry22Aa1 (Accession #134547), Cry22Aa2 (Accession #AX472772), Cry22Aa3 (Accession #EU715020), Cry22Ab1 (Accession #AAK50456), Cry22Ab2 (Accession #AX472764), Cry22Ba1 (Accession #AX472770), Cry23Aa1 (Accession #AAF76375), Cry24Aa1 (Accession #U88188), Cry24Ba1 (Accession #BAD32657), Cry24Ca1 (Accession #AM158318), Cry25Aa1 (Accession #U88189), Cry26Aa1 (Accession #AF122897), Cry27Aa1 (Accession #AB023293), Cry28Aa1 (Accession #AF132928), Cry28Aa2 (Accession #AF285775), Cry29Aa1 (Accession #AJ251977), Cry30Aa1 (Accession #AJ251978), Cry30Ba1 (Accession #BAD00052), Cry30Ca1 (Accession #BAD67157), Cry30Da1 (Accession #EF095955), Cry30 Db1 (Accession #BAE80088), Cry30Ea1 (Accession #EU503140), Cry30Fa1 (Accession #EU751609), Cry30Ga1 (Accession #EU882064), Cry31Aa1 (Accession #AB031065), Cry31Aa2 (Accession #AY081052), Cry31Aa3 (Accession #AB250922), Cry31Aa4 (Accession #AB274826), Cry31Aa5 (Accession #AB274827), Cry31Abl (Accession #AB250923), Cry31Ab2 (Accession #AB274825), Cry31Acl (Accession #AB276125), Cry32Aal (Accession #AY008143), Cry32Ba1 (Accession #BAB78601), Cry32Ca1 (Accession #BAB78602), Cry32Da1 (Accession #BAB78603), Cry33Aa1 (Accession #AAL26871), Cry34Aa1 (Accession #AAG50341), Cry34Aa2 (Accession #AAK64560), Cry34Aa3 (Accession #AY536899), Cry34Aa4 (Accession #AY536897), Cry34Ab1 (Accession #AAG41671), Cry34Ac1 (Accession #AAG50118), Cry34Ac2 (Accession #AAK64562), Cry34Ac3 (Accession #AY536896), Cry34Ba1 (Accession #AAK64565), Cry34Ba2 (Accession #AY536900), Cry34Ba3 (Accession #AY536898), Cry35Aa1 (Accession #AAG50342), Cry35Aa2 (Accession #AAK64561), Cry35Aa3 (Accession #AY536895), Cry35Aa4 (Accession #AY536892), Cry35Ab1 (Accession #AAG41672), Cry35Ab2 (Accession #AAK64563), Cry35Ab3 (Accession #AY536891), Cry35Ac1 (Accession #AAG50117), Cry35Ba1 (Accession #AAK64566), Cry35Ba2 (Accession #AY536894), Cry35Ba3 (Accession #AY536893), Cry36Aa1 (Accession #AAK64558), Cry37Aa1 (Accession #AAF76376), Cry38Aa1 (Accession #AAK64559), Cry39Aa1 (Accession #BAB72016), Cry40Aa1 (Accession #BAB72018), Cry40Ba1 (Accession #BAC77648), Cry40Ca1 (Accession #EU381045), Cry40Da1 (Accession #EU596478), Cry41Aa1 (Accession #AB116649), Cry41Ab1 (Accession #AB116651), Cry42Aa1 (Accession #AB116652), Cry43Aa1 (Accession #AB115422), Cry43Aa2 (Accession #AB176668), Cry43Ba1 (Accession #AB115422), Cry43-like (Accession #AB115422), Cry44Aa (Accession #BAD08532), Cry45Aa (Accession #BAD22577), Cry46Aa (Accession #BAC79010), Cry46Aa2 (Accession #BAG68906), Cry46Ab (Accession #BAD35170), Cry47Aa (Accession #AY950229), Cry48Aa (Accession #AJ841948), Cry48Aa2 (Accession #AM237205), Cry48Aa3 (Accession #AM237206), Cry48Ab (Accession #AM237207), Cry48Ab2 (Accession #AM237208), Cry49Aa (Accession #AJ841948), Cry49Aa2 (Accession #AM237201), Cry49Aa3 (Accession #AM237203), Cry49Aa4 (Accession #AM237204), Cry49Ab1 (Accession #AM237202), Cry50Aa1 (Accession #AB253419), Cry51Aa1 (Accession #DQ836184), Cry52Aa1 (Accession #EF613489), Cry53Aa1 (Accession #EF633476), Cry54Aa1 (Accession #EU339367), Cry55Aa1 (Accession #EU121521), Cry55Aa2 (Accession #AAE33526).
   Examples of delta-endotoxins also include but are not limited to Cry1A proteins of U.S. Pat. Nos. 5,880,275 and 7,858,849; a DIG-3 or DIG-11 toxin (N-terminal deletion of alpha-helix 1 and/or alpha-helix 2 variants of Cry proteins such as Cry1A) of U.S. Pat. Nos. 8,304,604 and 8,304,605, Cry1B of U.S. patent application Ser. No. 10/525,318; CrylC of U.S. Pat. No. 6,033,874; CrylF of U.S. Pat. Nos. 5,188,960, 6,218,188; Cry1A/F chimeras of U.S. Pat. Nos. 7,070,982; 6,962,705 and 6,713,063); a Cry2 protein such as Cry2Ab protein of U.S. Pat. No. 7,064,249); a Cry3A protein including but not limited to an engineered hybrid insecticidal protein (eHIP) created by fusing unique combinations of variable regions and conserved blocks of at least two different Cry proteins (US Patent Application Publication Number 2010/0017914); a Cry4 protein; a Cry5 protein; a Cry6 protein; Cry8 proteins of U.S. Pat. Nos. 7,329,736, 7,449,552, 7,803,943, 7,476,781, 7,105,332, 7,378,499 and 7,462,760; a Cry9 protein such as such as members of the Cry9A, Cry9B, Cry9C, Cry9D, Cry9E, and Cry9F families; a Cry15 protein of Naimov, et al., (2008) Applied and Environmental Microbiology 74:7145-7151; a Cry22, a Cry34Ab1 protein of U.S. Pat. Nos. 6,127,180, 6,624,145 and 6,340,593; a CryET33 and CryET34 protein of U.S. Pat. Nos. 6,248,535, 6,326,351, 6,399,330, 6,949,626, 7,385,107 and 7,504,229; a CryET33 and CryET34 homologs of US Patent Publication Number 2006/0191034, 2012/0278954, and PCT Publication Number WO 2012/139004; a Cry35Ab1 protein of U.S. Pat. Nos. 6,083,499, 6,548,291 and 6,340,593; a Cry46 protein, a Cry 51 protein, a Cry binary toxin; a TIC901 or related toxin; TIC807 of US 2008/0295207; ET29, ET37, TIC809, TIC810, TIC812, TIC127, TIC128 of PCT US 2006/033867; AXMI-027, AXMI-036, and AXMI-038 of U.S. Pat. No. 8,236,757; AXMI-***031,*** AXMI-039, AXMI-040, AXMI-049 of U.S. Pat. No. 7,923,602; AXMI-018, AXMI-020, and AXMI-021 of WO 2006/083891; AXMI-010 of WO 2005/038032; AXMI-003 of WO 2005/021585; AXMI-008 of US 2004/0250311; AXMI-006 of US 2004/0216186; AXMI-007 of US 2004/0210965; AXMI-009 of US 2004/0210964; AXMI-014 of US 2004/0197917; AXMI-004 of US 2004/0197916; AXMI-028 and AXMI-029 of WO 2006/119457; AXMI-007, AXMI-008, AXMI-0080rf2, AXMI-009, AXMI-014 and AXMI-004 of WO 2004/074462; AXMI-150 of U.S. Pat. No. 8,084,416; AXMI-205 of US20110023184; AXMI-011, AXMI-012, AXMI-013, AXMI-015, AXMI-019, AXMI-044, AXMI-037, AXMI-043, AXMI-033, AXMI-034, AXMI-022, AXMI-023, AXMI-041, AXMI-063, and AXMI-064 of US 2011/0263488; AXMI-R1 and related proteins of US 2010/0197592; AXMI221Z, AXMI222z, AXMI223z, AXMI224z and AXMI225z of WO 2011/103248; AXMI218, AXMI219, AXMI220, AXMI226, AXMI227, AXMI228, AXMI229, AXMI230, and AXMI231 of WO11/103,247; AXMI-115, AXMI-113, AXMI-005, AXMI-163 and AXMI-184 of U.S. Pat. No. 8,334,431; AXMI-001, AXMI-002, AXMI-030, AXMI-035, and AXMI-045 of US 2010/0298211; AXMI-066 and AXMI-076 of US20090144852; AXMI128, AXMI130, AXMI131, AXMI133, AXMI140, AXMI141, AXMI142, AXMI143, AXMI144, AXMI146, AXMI148, AXMI149, AXMI152, AXMI153, AXMI154, AXMI155, AXMI156, AXMI157, AXMI158, AXMI162, AXMI165, AXMI166, AXMI167, AXMI168, AXMI169, AXMI170, AXMI171, AXMI172, AXMI173, AXMI174, AXMI175, AXMI176, AXMI177, AXMI178, AXMI179, AXMI180, AXMI181, AXMI182, AXMI185, AXMI186, AXMI187, AXMI188, AXMI189 of U.S. Pat. No. 8,318,900; AXMI079, AXMI080, AXMI081, AXMI082, AXMI091, AXMI092, AXMI096, AXMI097, AXMI098, AXMI099, AXMI100, AXMI101, AXMI102, AXMI103, AXMI104, AXMI107, AXMI108, AXMI109, AXMI110, AXMI111, AXMI112, AXMI114, AXMI116, AXMI117, AXMI118, AXMI119, AXMI120, AXMI121, AXMI122, AXMI123, AXMI124, AXMI1257, AXMI1268, AXMI127, AXMI129, AXMI164, AXMI151, AXMI161, AXMI183, AXMI132, AXMI138, AXMI137 of US 2010/0005543; Cry proteins such as Cry1A and Cry3A having modified proteolytic sites of U.S. Pat. No. 8,319,019; and a Cry1Ac, Cry2Aa and CrylCa toxin protein from Bacillus thuringiensis strain VBTS 2528 of US Patent Application Publication Number 2011/0064710. Other Cry proteins are well known to one skilled in the art (see, Crickmore, et al., "Bacillus thuringiensis toxin nomenclature" (2011), at lifesci.sussex.ac.uk/home/Neil_Crickmore/Bt/which can be accessed on the world-wide web using the "www" prefix). The insecticidal activity of Cry proteins is well known to one skilled in the art (for review, see, van Frannkenhuyzen, (2009) J. Invert. Path. 101:1-16). The use of Cry proteins as transgenic plant traits is well known to one skilled in the art and Cry- transgenic plants including but not limited to Cry1Ac, Cry1Ac+Cry2Ab, Cry1Ab, Cry1A.105, Cry1F, Cry1Fa2, Cry1F+Cry1Ac, Cry2Ab, Cry3A, mCry3A, Cry3Bb1, Cry34Ab1, Cry35Ab1, Vip3A, mCry3A, Cry9c and CBI-Bt have received regulatory approval (see, Sanahuja, (2011) Plant Biotech Journal 9:283-300 and the CERA (2010) GM Crop Database Center for Environmental Risk Assessment (CERA), ILSI Research Foundation, Washington D.C. at cera-gmc.org/index.php?action=gm_crop_database which can be accessed on the world-wide web using the "www" prefix). Pesticidal proteins also include insecticidal lipases including lipid acyl hydrolases of U.S. Pat. No. 7,491,869, and cholesterol oxidases such as from Streptomyces (Purcell et al. (1993) Biochem Biophys Res Commun 15:1406-1413). Pesticidal proteins also include VIP (vegetative insecticidal proteins) toxins of U.S. Pat. Nos. 5,877,012, 6,107,279, 6,137,033, 7,244,820, 7,615,686, and 8,237,020, and the like. Other VIP proteins are well known to one skilled in the art (see, lifesci.sussex.ac.uk/home/Neil_Crickmore/Bt/vip.html which can be accessed on the world-wide web using the "www" prefix). Pesticidal proteins also include toxin complex (TC) proteins, obtainable from organisms such as Xenorhabdus, Photorhabdus and Paenibacillus (see, U.S. Pat. Nos. 7,491,698 and 8,084,418). Some TC proteins have "stand alone" insecticidal activity and other TC proteins enhance the activity of the stand-alone toxins produced by the same given organism. The toxicity of a "stand-alone" TC protein (from Photorhabdus, Xenorhabdus or Paenibacillus, for example) can be enhanced by one or more TC protein "potentiators" derived from a source organism of a different genus. There are three main types of TC proteins. As referred to herein, Class A proteins ("Protein A") are stand-alone toxins. Class B proteins ("Protein B") and Class C proteins ("Protein C") enhance the toxicity of Class A proteins. Examples of Class A proteins are TcbA, TcdA, XptA1 and XptA2. Examples of Class B proteins are TcaC, TcdB, XptB1Xb and XptC1Wi. Examples of Class C proteins are TccC, XptC1Xb and XptB1Wi. Pesticidal proteins also include spider, snake and scorpion venom proteins. Examples of spider venom peptides include but are not limited to lycotoxin-1 peptides and mutants thereof (U.S. Pat. No. 8,334,366).
(C) A polynucleotide encoding an insect-specific hormone or pheromone such as an ecdysteroid and juvenile hormone, a variant thereof, a mimetic based thereon or an antagonist or agonist thereof. See, for example, the disclosure by Hammock, et al., (1990) Nature 344:458, of baculovirus expression of cloned juvenile hormone esterase, an inactivator of juvenile hormone.
(D) A polynucleotide encoding an insect-specific peptide which, upon expression, disrupts the physiology of the affected pest. For example, see the disclosures of, Regan, (1994) J. Biol. Chem. 269:9 (expression cloning yields DNA coding for insect diuretic hormone receptor); Pratt, et al., (1989) Biochem. Biophys. Res. Comm. 163:1243 (an allostatin is identified in Diploptera puntata); Chattopadhyay, et al., (2004) Critical Reviews in Microbiology 30(1):33-54; Zjawiony, (2004) J Nat Prod 67(2):300-310; Carlini and Grossi-de-Sa, (2002) Toxicon 40(11):1515-1539; Ussuf, et al., (2001) Curr Sci. 80(7):847-853 and Vasconcelos and Oliveira, (2004) Toxicon 44(4):385-403. See also, U.S. Pat. No. 5,266,317 to Tomalski, et al., who disclose genes encoding insect-specific toxins.
(E) A polynucleotide encoding an enzyme responsible for a hyperaccumulation of a monoterpene, a sesquiterpene, a steroid, hydroxamic acid, a phenylpropanoid derivative or another non-protein molecule with insecticidal activity.
(F) A polynucleotide encoding an enzyme involved in the modification, including the post-translational modification, of a biologically active molecule; for example, a glycolytic enzyme, a proteolytic enzyme, a lipolytic enzyme, a nuclease, a cyclase, a transaminase, an esterase, a hydrolase, a phosphatase, a kinase, a phosphorylase, a polymerase, an elastase, a chitinase and a glucanase, whether natural or synthetic. *See,* PCT Application WO 1993/02197 in the name of Scott, et al., which discloses the nucleotide sequence of a callase gene. DNA molecules which contain chitinase-encoding sequences can be obtained, for example, from the ATCC under Accession Numbers 39637 and 67152. *See* also, Kramer, et al., (1993) Insect Biochem. Molec. Biol. 23:691, who teach the nucleotide sequence of a cDNA encoding tobacco hookworm chitinase and Kawalleck, et al., (1993) Plant Molec. Biol. 21:673, who provide the nucleotide sequence of the parsley ubi4-2 polyubiquitin gene, and U.S. Pat. Nos. 6,563,020; 7,145,060 and 7,087,810.
(G) A polynucleotide encoding a molecule that stimulates signal transduction. For example, see the disclosure by Botella, et al., (1994) Plant Molec. Biol. 24:757, of nucleotide sequences for mung bean calmodulin cDNA clones, and Griess, et al., (1994) Plant Physiol. 104:1467, who provide the nucleotide sequence of a maize calmodulin cDNA clone.
(H) A polynucleotide encoding a hydrophobic moment peptide. *See,* PCT Application WO 1995/16776 and U.S. Pat. No. 5,580,852 disclosure of peptide derivatives of Tachyplesin which inhibit fungal plant pathogens) and PCT Application WO 1995/18855 and U.S. Pat. No. 5,607,914 (teaches synthetic antimicrobial peptides that confer disease resistance).
(I) A polynucleotide encoding a membrane permease, a channel former or a channel blocker. For example, see the disclosure by Jaynes, et al., (1993) Plant Sci. 89:43, of heterologous expression of a cecropin-beta lytic peptide analog to render transgenic tobacco plants resistant to Pseudomonas solanacearum.
(J) A gene encoding a viral-invasive protein or a complex toxin derived therefrom. For example, the accumulation of viral coat proteins in transformed plant cells imparts resistance to viral infection and/or disease development effected by the virus from which the coat protein gene is derived, as well as by related viruses. See, Beachy, et al., (1990) Ann. Rev. Phytopathol. 28:451. Coat protein-mediated resistance has been conferred upon transformed plants against alfalfa mosaic virus, cucumber mosaic virus, tobacco streak virus, potato virus X, potato virus Y, tobacco etch virus, tobacco rattle virus and tobacco mosaic virus. Id.
(K) A gene encoding an insect-specific antibody or an immunotoxin derived therefrom. Thus, an antibody targeted to a critical metabolic function in the insect gut would inactivate an affected enzyme, killing the insect. Cf. Taylor, et al., Abstract #497, SEVENTH INT'L SYMPOSIUM ON MOLECULAR PLANT-MICROBE INTERACTIONS (Edinburgh, Scotland, 1994) (enzymatic inactivation in transgenic tobacco via production of single-chain antibody fragments).
(L) A gene encoding a virus-specific antibody. See, for example, Tavladoraki, et al., (1993) Nature 366:469, who show that transgenic plants expressing recombinant antibody genes are protected from virus attack.
(M) A polynucleotide encoding a developmental-arrestive protein produced in nature by a pathogen or a parasite. Thus, fungal endo alpha-1,4-D-polygalacturonases facilitate fungal colonization and plant nutrient release by solubilizing plant cell wall homo-alpha-1,4-D-galacturonase. See, Lamb, et al., (1992) Bio/Technology 10:1436. The cloning and characterization of a gene which encodes a bean endopolygalacturonase-inhibiting protein is described by Toubart, et al., (1992) Plant J. 2:367.
(N) A polynucleotide encoding a developmental-arrestive protein produced in nature by a plant. For example, Logemann, et al., (1992) Bio/Technology 10:305, have shown that transgenic plants expressing the barley ribosome-inactivating gene have an increased resistance to fungal disease.
(O) Genes involved in the Systemic Acquired Resistance (SAR) Response and/or the pathogenesis related genes. Briggs, (1995) Current Biology 5(2), Pieterse and Van Loon, (2004) Curr. Opin. Plant Bio. 7(4):456-64 and Somssich, (2003) Cell 113(7):815-6.
(P) Antifungal genes (Cornelissen and Melchers, (1993) P1. Physiol. 101:709-712 and Parijs, et al., (1991) Planta 183:258-264 and Bushnell, et al., (1998) Can. J. of Plant Path. 20(2):137-149. Also see, U.S. patent application Ser. Nos. 09/950,933; 11/619,645; 11/657,710; 11/748,994; 11/774,121 and U.S. Pat. Nos. 6,891,085 and 7,306,946. LysM Receptor-like kinases for the perception of chitin fragments as a first step in plant defense response against fungal pathogens (US 2012/0110696).
(Q) Detoxification genes, such as for fumonisin, beauvericin, moniliformin and zearalenone and their structurally related derivatives. For example, see, U.S. Pat. Nos. 5,716,820; 5,792,931; 5,798,255; 5,846,812; 6,083,736; 6,538,177; 6,388,171 and 6,812,380.
(R) A polynucleotide encoding a Cystatin and cysteine proteinase inhibitors. See, U.S. Pat. No. 7,205,453.
(S) Defensin genes. See, WO 2003/000863 and U.S. Pat. Nos. 6,911,577; 6,855,865; 6,777,592 and 7,238,781.
(T) Genes conferring resistance to nematodes. See, e.g., PCT Application WO 1996/30517; PCT Application WO 1993/19181, WO 2003/033651 and Urwin, et al., (1998) Planta 204:472-479, Williamson, (1999) Curr Opin Plant Bio. 2(4):327-31; U.S. Pat. Nos. 6,284,948 and 7,301,069 and miR164 genes (WO 2012/058266).
(U) Genes that confer resistance to Phytophthora Root Rot, such as the Rps 1, Rps 1-a, Rps 1-b, Rps 1-c, Rps 1-d, Rps 1-e, Rps 1-k, Rps 2, Rps 3-a, Rps 3-b, Rps 3-c, Rps 4, Rps 5, Rps 6, Rps 7 and other Rps genes. See, for example, Shoemaker, et al., Phytophthora Root Rot Resistance Gene Mapping in Soybean, Plant Genome IV Conference, San Diego, Calif. (1995).
(V) Genes that confer resistance to Brown Stem Rot, such as described in U.S. Pat. No. 5,689,035 and incorporated by reference for this purpose.
(W) Genes that confer resistance to Colletotrichum, such as described in US Patent Application Publication US 2009/0035765 and incorporated by reference for this purpose. This includes the Rcg locus that may be utilized as a single locus conversion.

### ii. Transgenes that Confer Resistance to a Herbicide.

(A) A polynucleotide encoding resistance to a herbicide that inhibits the growing point or meristem, such as an imidazolinone or a sulfonylurea. Exemplary genes in this category code for mutant ALS and AHAS enzyme as described, for example, by Lee, et al., (1988) EMBO J. 7:1241 and Miki, et al., (1990) Theor. Appl. Genet. 80:449, respectively. See also, U.S. Pat. Nos. 5,605,011; 5,013,659; 5,141,870; 5,767,361; 5,731,180; 5,304,732; 4,761,373; 5,331,107; 5,928,937 and 5,378,824; U.S. patent application Ser. No. 11/683,737 and International Publication WO 1996/33270.
(B) A polynucleotide encoding a protein for resistance to Glyphosate (resistance imparted by mutant 5-enolpyruvl-3-phosphikimate synthase (EPSP) and aroA genes, respectively) and other phosphono compounds such as glufosinate (phosphinothricin acetyl transferase (PAT) and Streptomyces hygroscopicus phosphinothricin acetyl transferase (bar) genes), and pyridinoxy or phenoxy proprionic acids and cyclohexones (ACCase inhibitor-encoding genes). See, for example, U.S. Pat. No. 4,940,835 to Shah, et al., which discloses the nucleotide sequence of a form of EPSPS which can confer glyphosate resistance. U.S. Pat. No. 5,627,061 to Barry, et al., also describes genes encoding EPSPS enzymes. See also, U.S. Pat. Nos. 6,566,587; 6,338,961; 6,248,876 B1; 6,040,497; 5,804,425; 5,633,435; 5,145,783; 4,971,908; 5,312,910; 5,188,642; 5,094,945, 4,940,835; 5,866,775; 6,225,114 B1; 6,130,366; 5,310,667; 4,535,060; 4,769,061; 5,633,448; 5,510,471; Re. 36,449; RE 37,287 E and 5,491,288 and International Publications EP 1173580; WO 2001/66704; EP 1173581 and EP 1173582, which are incorporated herein by reference for this purpose.
   Glyphosate resistance is also imparted to plants that express a gene encoding a glyphosate oxido-reductase enzyme as described more fully in U.S. Pat. Nos. 5,776,760 and 5,463,175, which are incorporated herein by reference for this purpose. In addition glyphosate resistance can be imparted to plants by the over expression of genes encoding glyphosate N-acetyltransferase. See, for example, U.S. Pat. Nos. 7,462,481; 7,405,074 and US Patent Application Publication Number US 2008/0234130. A DNA molecule encoding a mutant aroA gene can be obtained under ATCC Accession Number 39256, and the nucleotide sequence of the mutant gene is disclosed in U.S. Pat. No. 4,769,061 to Comai. EP Application Number 0 333 033 to Kumada, et al., and U.S. Pat. No. 4,975,374 to Goodman, et al., disclose nucleotide sequences of glutamine synthetase genes which confer resistance to herbicides such as L-phosphinothricin. The nucleotide sequence of a phosphinothricin-acetyltransferase gene is provided in EP Application Numbers 0 242 246 and 0 242 236 to Leemans, et al.; De Greef, et al., (1989) Bio/Technology 7:61, describe the production of transgenic plants that express chimeric bar genes coding for phosphinothricin acetyl transferase activity. See also, U.S. Pat. Nos. 5,969,213; 5,489,520; 5,550,318; 5,874,265; 5,919,675; 5,561,236; 5,648,477; 5,646,024; 6,177,616 B1 and 5,879,903, which are incorporated herein by reference for this purpose. Exemplary genes conferring resistance to phenoxy proprionic acids and cyclohexones, such as sethoxydim and haloxyfop, are the Acc1-S1, Acc1-S2 and Acc1-S3 genes described by Marshall, et al., (1992) Theor. Appl. Genet. 83:435.
(C) A polynucleotide encoding a protein for resistance to herbicide that inhibits photosynthesis, such as a triazine (psbA and gs+genes) and a benzonitrile (nitrilase gene). Przibilla, et al., (1991) Plant Cell 3:169, describe the transformation of Chlamydomonas with plasmids encoding mutant psbA genes. Nucleotide sequences for nitrilase genes are disclosed in U.S. Pat. No. 4,810,648 to Stalker and DNA molecules containing these genes are available under ATCC Accession Numbers 53435, 67441 and 67442. Cloning and expression of DNA coding for a glutathione S-transferase is described by Hayes, et al., (1992) Biochem. J. 285:173.
(D) A polynucleotide encoding a protein for resistance to Acetohydroxy acid synthase, which has been found to make plants that express this enzyme resistant to multiple types of herbicides, has been introduced into a variety of plants (see, e.g., Hattori, et al., (1995) Mol Gen Genet. 246:419). Other genes that confer resistance to herbicides include: a gene encoding a chimeric protein of rat cytochrome P4507A1 and yeast NADPH-cytochrome P450 oxidoreductase (Shiota, et al., (1994) Plant Physiol 106:17), genes for glutathione reductase and superoxide dismutase (Aono, et al., (1995) Plant Cell Physiol 36:1687) and genes for various phosphotransferases (Datta, et al., (1992) Plant Mol Biol 20:619).
(E) A polynucleotide encoding resistance to a herbicide targeting Protoporphyrinogen oxidase (protox) which is necessary for the production of chlorophyll. The protox enzyme serves as the target for a variety of herbicidal compounds. These herbicides also inhibit growth of all the different species of plants present, causing their total destruction. The development of plants containing altered protox activity which are resistant to these herbicides are described in U.S. Pat. Nos. 6,288,306 B1; 6,282,837 B1 and 5,767,373 and International Publication WO 2001/12825.
(F) The aad-1 gene (originally from Sphingobium herbicidovorans) encodes the aryloxyalkanoate dioxygenase (AAD-1) protein. The trait confers tolerance to 2,4-dichlorophenoxyacetic acid and aryloxyphenoxypropionate (commonly referred to as "fop" herbicides such as quizalofop) herbicides. The aad-1 gene, itself, for herbicide tolerance in plants was first disclosed in WO 2005/107437 (see also, US 2009/0093366). The aad-12 gene, derived from Delftia acidovorans, which encodes the aryloxyalkanoate dioxygenase (AAD-12) protein that confers tolerance to 2,4-dichlorophenoxyacetic acid and pyridyloxyacetate herbicides by deactivating several herbicides with an aryloxyalkanoate moiety, including phenoxy auxin (e.g., 2,4-D, MCPA), as well as pyridyloxy auxins (e.g., fluoroxypyr, triclopyr).
(G) A polynucleotide encoding a herbicide resistant dicamba monooxygenase disclosed in US Patent Application Publication 2003/0135879 for imparting dicamba tolerance.
(H) A polynucleotide molecule encoding bromoxynil nitrilase (Bxn) disclosed in U.S. Pat. No. 4,810,648 for imparting bromoxynil tolerance.
(I) A polynucleotide molecule encoding phytoene (crtl) described in Misawa, et al., (1993) Plant J. 4:833-840 and in Misawa, et al., (1994) Plant J. 6:481-489 for norflurazon tolerance.

### iii. Transgenes that Confer or Contribute to an Altered Grain Characteristic

(A) Altered fatty acids, for example, by (1) Down-regulation of stearoyl-ACP to increase stearic acid content of the plant. See, Knultzon, et al., (1992) Proc. Natl. Acad. Sci. USA 89:2624 and WO 1999/64579 (Genes to Alter Lipid Profiles in Corn); (2) Elevating oleic acid via FAD-2 gene modification and/or decreasing linolenic acid via FAD-3 gene modification (see, U.S. Pat. Nos. 6,063,947; 6,323,392; 6,372,965 and WO 1993/11245); (3) Altering conjugated linolenic or linoleic acid content, such as in WO 2001/12800; (4) Altering LEC1, AGP, Dek1, Superal1, mil ps, various Ipa genes such as Ipa1, Ipa3, hpt or hggt. For example, see, WO 2002/42424, WO 1998/22604, WO 2003/011015, WO 2002/057439, WO 2003/011015, U.S. Pat. Nos. 6,423,886, 6,197,561, 6,825,397 and US Patent Application Publication Numbers US 2003/0079247, US 2003/0204870 and Rivera-Madrid, et al., (1995) Proc. Natl. Acad. Sci. 92:5620-5624; (5) Genes encoding delta-8 desaturase for making long-chain polyunsaturated fatty acids (U.S. Pat. Nos. 8,058,571 and 8,338,152), delta-9 desaturase for lowering saturated fats (U.S. Pat. No. 8,063,269), Primula .DELTA.6-desaturase for improving omega-3 fatty acid profiles; (6) Isolated nucleic acids and proteins associated with lipid and sugar metabolism regulation, in particular, lipid metabolism protein (LMP) used in methods of producing transgenic plants and modulating levels of seed storage compounds including lipids, fatty acids, starches or seed storage proteins and use in methods of modulating the seed size, seed number, seed weights, root length and leaf size of plants (EP 2404499); (7) Altering expression of a High-Level Expression of Sugar-Inducible 2 (HSI2) protein in the plant to increase or decrease expression of HSI2 in the plant. Increasing expression of HSI2 increases oil content while decreasing expression of HSI2 decreases abscisic acid sensitivity and/or increases drought resistance (US Patent Application Publication Number 2012/0066794); (8) Expression of cytochrome b5 (Cb5) alone or with FAD2 to modulate oil content in plant seed, particularly to increase the levels of omega-3 fatty acids and improve the ratio of omega-6 to omega-3 fatty acids (US Patent Application Publication Number 2011/0191904); and (9) Nucleic acid molecules encoding wrinkled1-like polypeptides for modulating sugar metabolism (U.S. Pat. No. 8,217,223).
(B) Altered phosphorus content, for example, by the (1) introduction of a phytase-encoding gene would enhance breakdown of phytate, adding more free phosphate to the transformed plant. For example, see, Van Hartingsveldt, et al., (1993) Gene 127:87, for a disclosure of the nucleotide sequence of an Aspergillus niger phytase gene; and (2) modulating a gene that reduces phytate content. In maize, this, for example, could be accomplished, by cloning and then re-introducing DNA associated with one or more of the alleles, such as the LPA alleles, identified in maize mutants characterized by low levels of phytic acid, such as in WO 2005/113778 and/or by altering inositol kinase activity as in WO 2002/059324, US Patent Application Publication Number 2003/0009011, WO 2003/027243, US Patent Application Publication Number 2003/0079247, WO 1999/05298, U.S. Pat. No. 6,197,561, U.S. Pat. No. 6,291,224, U.S. Pat. No. 6,391,348, WO 2002/059324, US Patent Application Publication Number 2003/0079247, WO 1998/45448, WO 1999/55882, WO 2001/04147.
(C) Altered carbohydrates affected, for example, by altering a gene for an enzyme that affects the branching pattern of starch or, a gene altering thioredoxin such as NTR and/or TRX (see, U.S. Pat. No. 6,531,648. which is incorporated by reference for this purpose) and/or a gamma zein knock out or mutant such as cs27 or TUSC27 or en27 (see, U.S. Pat. No. 6,858,778 and US Patent Application Publication Number 2005/0160488, US Patent Application Publication Number 2005/0204418, which are incorporated by reference for this purpose). See, Shiroza, et al., (1988) J. Bacteriol. 170:810 (nucleotide sequence of Streptococcus mutant fructosyltransferase gene), Steinmetz, et al., (1985) Mol. Gen. Genet. 200:220 (nucleotide sequence of Bacillus subtilis levansucrase gene), Pen, et al., (1992) Bio/Technology 10:292 (production of transgenic plants that express Bacillus licheniformis alpha-amylase), Elliot, et al., (1993) Plant Molec. Biol. 21:515 (nucleotide sequences of tomato invertase genes), Sogaard, et al., (1993) J. Biol. Chem. 268:22480 (site-directed mutagenesis of barley alpha-amylase gene) and Fisher, et al., (1993) Plant Physiol. 102:1045 (maize endosperm starch branching enzyme II), WO 1999/10498 (improved digestibility and/or starch extraction through modification of UDP-D-xylose 4-epimerase, Fragile 1 and 2, Ref1, HCHL, C4H), U.S. Pat. No. 6,232,529 (method of producing high oil seed by modification of starch levels (AGP)). The fatty acid modification genes mentioned herein may also be used to affect starch content and/or composition through the interrelationship of the starch and oil pathways.
(D) Altered antioxidant content or composition, such as alteration of tocopherol or tocotrienols. For example, see, U.S. Pat. No. 6,787,683, US Patent Application Publication Number 2004/0034886 and WO 2000/68393 involving the manipulation of antioxidant levels and WO 2003/082899 through alteration of a homogentisate geranyl geranyl transferase (hggt).
(E) Altered essential seed amino acids. For example, see, U.S. Pat. No. 6,127,600 (method of increasing accumulation of essential amino acids in seeds), U.S. Pat. No. 6,080,913 (binary methods of increasing accumulation of essential amino acids in seeds), U.S. Pat. No. 5,990,389 (high lysine), WO 1999/40209 (alteration of amino acid compositions in seeds), WO 1999/29882 (methods for altering amino acid content of proteins), U.S. Pat. No. 5,850,016 (alteration of amino acid compositions in seeds), WO 1998/20133 (proteins with enhanced levels of essential amino acids), U.S. Pat. No. 5,885,802 (high methionine), U.S. Pat. No. 5,885,801 (high threonine), U.S. Pat. No. 6,664,445 (plant amino acid biosynthetic enzymes), U.S. Pat. No. 6,459,019 (increased lysine and threonine), U.S. Pat. No. 6,441,274 (plant tryptophan synthase beta subunit), U.S. Pat. No. 6,346,403 (methionine metabolic enzymes), U.S. Pat. No. 5,939,599 (high sulfur), U.S. Pat. No. 5,912,414 (increased methionine), WO 1998/56935 (plant amino acid biosynthetic enzymes), WO 1998/45458 (engineered seed protein having higher percentage of essential amino acids), WO 1998/42831 (increased lysine), U.S. Pat. No. 5,633,436 (increasing sulfur amino acid content), U.S. Pat. No. 5,559,223 (synthetic storage proteins with defined structure containing programmable levels of essential amino acids for improvement of the nutritional value of plants), WO 1996/01905 (increased threonine), WO 1995/15392 (increased lysine), US Patent Application Publication Number 2003/0163838, US Patent Application Publication Number 2003/0150014, US Patent Application Publication Number 2004/0068767, U.S. Pat. No. 6,803,498, WO 2001/79516.

### iv. Genes that Control Male-Sterility

There are several methods of conferring genetic male sterility available, such as multiple mutant genes at separate locations within the genome that confer male sterility, as disclosed in U.S. Pat. Nos. 4,654,465 and 4,727,219 to Brar, et al., and chromosomal translocations as described by Patterson in U.S. Pat. Nos. 3,861,709 and 3,710,511. In addition to these methods, Albertsen, et al., U.S. Pat. No. 5,432,068, describe a system of nuclear male sterility which includes: identifying a gene which is critical to male fertility; silencing this native gene which is critical to male fertility; removing the native promoter from the essential male fertility gene and replacing it with an inducible promoter; inserting this genetically engineered gene back into the plant; and thus creating a plant that is male sterile because the inducible promoter is not "on" resulting in the male fertility gene not being transcribed. Fertility is restored by inducing or turning "on", the promoter, which in turn allows the gene that confers male fertility to be transcribed. Non-limiting examples include: (A) Introduction of a deacetylase gene under the control of a tapetum-specific promoter and with the application of the chemical N-Ac-PPT (WO 2001/29237); (B) Introduction of various stamen-specific promoters (WO 1992/13956, WO 1992/13957); and (C) Introduction of the barnase and the barstar gene (Paul, et al., (1992) Plant Mol. Biol. 19:611-622). For additional examples of nuclear male and female sterility systems and genes, see also, U.S. Pat. Nos. 5,859,341; 6,297,426; 5,478,369; 5,824,524; 5,850,014 and 6,265,640, all of which are hereby incorporated by reference.

### v. Genes that Create a Site for Site Specific DNA Integration.

This includes the introduction of FRT sites that may be used in the FLP/FRT system and/or Lox sites that may be used in the Cre/Loxp system. For example, see, Lyznik, et al., (2003) Plant Cell Rep 21:925-932 and WO 1999/25821, which are hereby incorporated by reference. Other systems that may be used include the Gln recombinase of phage Mu (Maeser, et al., (1991) Vicki Chandler, The Maize Handbook ch. 118 (Springer-Verlag 1994), the Pin recombinase of E. coli (Enomoto, et al., 1983) and the R/RS system of the pSRi plasmid (Araki, et al., 1992).

### vi. Genes that affect abiotic stress resistance

Including but not limited to flowering, ear and seed development, enhancement of nitrogen utilization efficiency, altered nitrogen responsiveness, drought resistance or tolerance, cold resistance or tolerance and salt resistance or tolerance and increased yield under stress. Non-limiting examples include: (A) For example, see: WO 2000/73475 where water use efficiency is altered through alteration of malate; U.S. Pat. Nos. 5,892,009, 5,965,705, 5,929,305, 5,891,859, 6,417,428, 6,664,446, 6,706,866, 6,717,034, 6,801,104, WO 2000/060089, WO 2001/026459, WO 2001/035725, WO 2001/034726, WO 2001/035727, WO 2001/036444, WO 2001/036597, WO 2001/036598, WO 2002/015675, WO 2002/017430, WO 2002/077185, WO 2002/079403, WO 2003/013227, WO 2003/013228, WO 2003/014327, WO 2004/031349, WO 2004/076638, WO 199809521; (B) WO 199938977 describing genes, including CBF genes and transcription factors effective in mitigating the negative effects of freezing, high salinity and drought on plants, as well as conferring other positive effects on plant phenotype; (C) US Patent Application Publication Number 2004/0148654 and WO 2001/36596 where abscisic acid is altered in plants resulting in improved plant phenotype such as increased yield and/or increased tolerance to abiotic stress; (D) WO 2000/006341, WO 2004/090143, U.S. Pat. Nos. 7,531,723 and 6,992,237 where cytokinin expression is modified resulting in plants with increased stress tolerance, such as drought tolerance, and/or increased yield. Also see, WO 2002/02776, WO 2003/052063, JP 2002/281975, U.S. Pat. No. 6,084,153, WO 2001/64898, U.S. Pat. No. 6,177,275 and U.S. Pat. No. 6,107,547 (enhancement of nitrogen utilization and altered nitrogen responsiveness); (E) For ethylene alteration, see, US Patent Application Publication Number 2004/0128719, US Patent Application Publication Number 2003/0166197 and WO 2000/32761; (F) For plant transcription factors or transcriptional regulators of abiotic stress, see, e.g., US Patent Application Publication Number 2004/0098764 or US Patent Application Publication Number 2004/0078852; (G) Genes that increase expression of vacuolar pyrophosphatase such as AVP1 (U.S. Pat. No. 8,058,515) for increased yield; nucleic acid encoding a HSFA4 or a HSFA5 (Heat Shock Factor of the class A4 or A5) polypeptides, an oligopeptide transporter protein (OPT4-like) polypeptide; a plastochron2-like (PLA2-like) polypeptide or a Wuschel related homeobox 1-like (WOX1-like) polypeptide (U. Patent Application Publication Number US 2011/0283420); (H) Down regulation of polynucleotides encoding poly (ADP-ribose) polymerase (PARP) proteins to modulate programmed cell death (U.S. Pat. No. 8,058,510) for increased vigor; (I) Polynucleotide encoding DTP21 polypeptides for conferring drought resistance (US Patent Application Publication Number US 2011/0277181); (J) Nucleotide sequences encoding ACC Synthase 3 (ACS3) proteins for modulating development, modulating response to stress, and modulating stress tolerance (US Patent Application Publication Number US 2010/0287669); (K) Polynucleotides that encode proteins that confer a drought tolerance phenotype (DTP) for conferring drought resistance (WO 2012/058528); (L) Tocopherol cyclase (TC) genes for conferring drought and salt tolerance (US Patent Application Publication Number 2012/0272352); (M) CAAX amino terminal family proteins for stress tolerance (U.S. Pat. No. 8,338,661); (N) Mutations in the SAL1 encoding gene have increased stress tolerance, including increased drought resistant (US Patent Application Publication Number 2010/0257633); (O) Expression of a nucleic acid sequence encoding a polypeptide selected from the group consisting of: GRF polypeptide, RAA1-like polypeptide, SYR polypeptide, ARKL polypeptide, and YTP polypeptide increasing yield-related traits (US Patent Application Publication Number 2011/0061133); and (P) Modulating expression in a plant of a nucleic acid encoding a Class III Trehalose Phosphate Phosphatase (TPP) polypeptide for enhancing yield-related traits in plants, particularly increasing seed yield (US Patent Application Publication Number 2010/0024067).

Other genes and transcription factors that affect plant growth and agronomic traits such as yield, flowering, plant growth and/or plant structure, can be introduced or introgressed into plants, see e.g., WO 1997/49811 (LHY), WO 1998/56918 (ESD4), WO 1997/10339 and U.S. Pat. No. 6,573,430 (TFL), U.S. Pat. No. 6,713,663 (FT), WO 1996/14414 (CON), WO 1996/38560, WO 2001/21822 (VRN1), WO 2000/44918 (VRN2), WO 1999/49064 (GI), WO 2000/46358 (FR1), WO 1997/29123, U.S. Pat. No. 6,794,560, U.S. Pat. No. 6,307,126 (GAI), WO 1999/09174 (D8 and Rht) and WO 2004/076638 and WO 2004/031349 (transcription factors).

### vii. Genes that Confer Increased Yield

Non-limiting examples of genes that confer increased yield are: (A) A transgenic crop plant transformed by a 1-AminoCyclopropane-1-Carboxylate Deaminase-like Polypeptide (ACCDP) coding nucleic acid, wherein expression of the nucleic acid sequence in the crop plant results in the plant's increased root growth, and/or increased yield, and/or increased tolerance to environmental stress as compared to a wild type variety of the plant (U.S. Pat. No. 8,097,769); (B) Over-expression of maize zinc finger protein gene (Zm-ZFP1) using a seed preferred promoter has been shown to enhance plant growth, increase kernel number and total kernel weight per plant (US Patent Application Publication Number 2012/0079623); (C) Constitutive over-expression of maize lateral organ boundaries (LOB) domain protein (Zm-LOBDP1) has been shown to increase kernel number and total kernel weight per plant (US Patent Application Publication Number 2012/0079622); (D) Enhancing yield-related traits in plants by modulating expression in a plant of a nucleic acid encoding a VIM1 (Variant in Methylation 1)-like polypeptide or a VTC2-like (GDP-L-galactose phosphorylase) polypeptide or a DUF1685 polypeptide or an ARF6-like (Auxin Responsive Factor) polypeptide (WO 2012/038893); (E) Modulating expression in a plant of a nucleic acid encoding a Ste20-like polypeptide or a homologue thereof gives plants having increased yield relative to control plants (EP 2431472); and (F) Genes encoding nucleoside diphosphatase kinase (NDK) polypeptides and homologs thereof for modifying the plant's root architecture (US Patent Application Publication Number 2009/0064373).

### IX. Methods of Use

Methods of the invention comprise methods for controlling a pest (i.e., a Coleopteran plant pest, including a *Diabrotica* plant pest, such as, *D. virgifera virgifera, D. barberi*, *D. virgifera zeae, D. speciosa,* or *D. undecimpunctata howardi*). The method comprises feeding or applying to a pest a composition comprising a silencing element of the invention, wherein said silencing element, when ingested or contacted by a pest (i.e., a Coleopteran plant pest including a *Diabrotica* plant pest, such as, *D. virgifera virgifera*, *D. barberi*, *D. virgifera zeae, D. speciosa,* or *D. undecimpunctata howardi),* reduces the level of a target polynucleotide of the pest and thereby controls the pest. The pest can be fed the silencing element in a variety of ways. For example, in one embodiment, the polynucleotide comprising the silencing element is introduced into a plant. As the Coleopteran plant pest or *Diabrotica* plant pest feeds on the plant or part thereof expressing these sequences, the silencing element is delivered to the pest. When the silencing element is delivered to the plant in this manner, it is recognized that the silencing element can be expressed constitutively or alternatively, it may be produced in a stage-specific manner by employing the various inducible or tissue-preferred or developmentally regulated promoters that are discussed elsewhere herein. In specific embodiments, the silencing element is expressed in the roots, stalk or stem, leaf including pedicel, xylem and phloem, fruit or reproductive tissue, silk, flowers and all parts therein or any combination thereof.

In another method, a composition comprising at least one silencing element of the invention is applied to a plant. In such embodiments, the silencing element can be formulated in an agronomically suitable and/or environmentally acceptable carrier, which is preferably, suitable for dispersal in fields. In addition, the carrier can also include compounds that increase the half life of the composition. In specific embodiments, the composition comprising the silencing element is formulated in such a manner such that it persists in the environment for a length of time sufficient to allow it to be delivered to a pest. In such embodiments, the composition can be applied to an area inhabited by a pest. In one embodiment, the composition is applied externally to a plant (i.e., by spraying a field) to protect the plant from pests.

In certain embodiments, the constructs of the present invention can be stacked with any combination of polynucleotide sequences of interest in order to create plants with a desired trait. A trait, as used herein, refers to the phenotype derived from a particular sequence or groups of sequences. For example, the polynucleotides of the present invention may be stacked with any other polynucleotides encoding polypeptides having pesticidal and/or insecticidal activity, such as other *Bacillus thuringiensis* toxic proteins (described in U.S. Patent Nos. 5,366,892; 5,747,450; 5,737,514; 5,723,756; 5,593,881; and Geiser et al. (1986) Gene 48:109), lectins (Van Damme et al. (1994) Plant Mol. Biol. 24:825, pentin (described in U.S. Patent No. 5,981,722), and the like. The combinations generated can also include multiple copies of any one of the polynucleotides of interest. The polynucleotides of the present invention can also be stacked with any other gene or combination of genes to produce plants with a variety of desired trait combinations including, but not limited to, traits desirable for animal feed such as high oil genes (e.g., U.S. Patent No. 6,232,529); balanced amino acids (e.g., hordothionins (U.S. Patent Nos. 5,990,389; 5,885,801; 5,885,802; and 5,703,409); barley high lysine (Williamson et al. (1987) Eur. J. Biochem. 165:99-106; and WO 98/20122) and high methionine proteins (Pedersen et al. (1986) J. Biol. Chem. 261:6279; Kirihara et al. (1988) Gene 71:359; and Musumura et al. (1989) Plant Mol. Biol. 12:123)); increased digestibility (e.g., modified storage proteins (U.S. Application Serial No. 10/053,410, filed November 7, 2001); and thioredoxins (U.S. Application Serial No. 10/005,429, filed December 3, 2001)); the disclosures of which are herein incorporated by reference.

The polynucleotides of the present invention can also be stacked with traits desirable for disease or herbicide resistance (e.g., fumonisin detoxification genes (U.S. Patent No. 5,792,931); avirulence and disease resistance genes (Jones et al. (1994) Science 266:789; Martin et al. (1993) Science 262:1432; Mindrinos et al. (1994) Cell 78:1089); acetolactate synthase (ALS) mutants that lead to herbicide resistance such as the S4 and/or Hra mutations; inhibitors of glutamine synthase such as phosphinothricin or basta (e.g., bar gene); and glyphosate resistance (EPSPS gene)); and traits desirable for processing or process products such as high oil (e.g., U.S. Patent No. 6,232,529); modified oils (e.g., fatty acid desaturase genes (U.S. Patent No. 5,952,544; WO 94/11516)); modified starches (e.g., ADPG pyrophosphorylases (AGPase), starch synthases (SS), starch branching enzymes (SBE), and starch debranching enzymes (SDBE)); and polymers or bioplastics (e.g., U.S. Patent No. 5.602,321; beta-ketothiolase, polyhydroxybutyrate synthase, and acetoacetyl-CoA reductase (Schubert et al. (1988) J. Bacteriol. 170:5837-5847) facilitate expression of polyhydroxyalkanoates (PHAs)); the disclosures of which are herein incorporated by reference. One could also combine the polynucleotides of the present invention with polynucleotides providing agronomic traits such as male sterility (e.g., see U.S. Patent No. 5.583,210), stalk strength, drought resistance (e.g., U.S. Patent No. 7,786,353), flowering time, or transformation technology traits such as cell cycle regulation or gene targeting (e.g., WO 99/61619, WO 00/17364, and WO 99/25821); the disclosures of which are herein incorporated by reference.

These stacked combinations can be created by any method including, but not limited to, cross-breeding plants by any conventional or TopCross methodology, or genetic transformation. If the sequences are stacked by genetically transforming the plants (i.e., molecular stacks), the polynucleotide sequences of interest can be combined at any time and in any order. For example, a transgenic plant comprising one or more desired traits can be used as the target to introduce further traits by subsequent transformation. The traits can be introduced simultaneously in a co-transformation protocol with the polynucleotides of interest provided by any combination of transformation cassettes. For example, if two sequences will be introduced, the two sequences can be contained in separate transformation cassettes (trans) or contained on the same transformation cassette (cis). Expression of the sequences can be driven by the same promoter or by different promoters. In certain cases, it may be desirable to introduce a transformation cassette that will suppress the expression of the polynucleotide of interest. This may be combined with any combination of other suppression cassettes or overexpression cassettes to generate the desired combination of traits in the plant. It is further recognized that polynucleotide sequences can be stacked at a desired genomic location using a site-specific recombination system. See, for example, WO99/25821, WO99/25854, WO99/25840, WO99/25855, and WO99/25853, all of which are herein incorporated by reference.

The following examples are offered by way of illustration and not by way of limitation.

### EXPERIMENTAL

### Example 1: In vitro transcript dsRNA screening method

A cDNA library was produced from neonate western corn rootworm larvae by standard methods. A selected cDNA clone containing an expressed sequence tag was amplified in a PCR using universal primers to the plasmid backbone and flanking the EST insert. The universal primers also contained T7 RNA polymerase sites. The product of the PCR reaction was used as the template for an in vitro transcription (IVT) reaction to produce long double stranded RNAs. Following enzymatic digestion and removal of the DNA template and single stranded RNA, the IVT reaction products were incorporated into artificial insect diet as described below.

Different target selection strategies were used in this invention to identify RNAi active targets with insecticidal activities in corn rootworn diet based assay. cDNA libraries were produced from neonate or midgut of 3rd instar western corn rootworm larvae by standard methods. Randomly selected cDNA clones containing an expressed sequence tag (EST) were amplified in a PCR using target specific primers (forward and reverse Table 1), and provided in the sequence listing included herein, to generate DNA templates. The target specific primers also contain T7 RNA polymerase sites (T7 sequence at 5' end of each primer). Second set of cDNA clones was selected based on homology to known lethal genes from other insects, primarily Drosophila melanogaster. A third set of genes was tested based on involvement in proteasome functions. Identification of these genes was based on a progressive homology search beginning with a list of proteosome genes identified in humans cross referenced to the Tribolium genome database. Hits from Tribolium were then used to parse western corn rootworm sequence database. Proteosome genes were categorized as 26S subunit non ATPase, 26S subunit ATPase, alpha type, and beta type genes.

Region(s) of WCRW genes were produced by PCR followed by in vitro transcription 5 (IVT) to produce long double stranded RNAs. The IVT reaction products are quantified in gel and incorporated into artificial insect diet for first-round IVT screening (FIS) as described below.

### Insect bioassays

dsRNAs were incorporated into standard WCRW artificial diet at a final concentration of 50ppm in a 96 well microtiter plate format. 5 µl of the IVT reaction (300 ng/ul) were added to a given well of a 96 well microtiter plate. 25 µl of molten lowmelt Western corn rootworm diet were added to the sample and shaken on an orbital shaker to mix the sample and diet. Once the diet has solidified, eight wells were used for each RNA sample. Preconditioned 1^{st} instar WCRW (neonate insects were placed on neutral diet for 24 hours prior to transfer to test material) were added to the 96 well microtiter plates at a rate of 3-5 insects/well. Plates were sealed with mylar which was then punctured twice above each well of the microtiter plate using a superfine insect collection pin. To prevent drying of the diet, plates were first placed inside a plastic bag with a slightly damp cloth and the bags were placed inside an incubator set at 28 °C and 70% RH. The assay was scored for mortality and stunting affects after 7 days and an average was determined based on assignment of numeric values to each category of impact (3=mortality, 2=severe stunting, 1=stunting, 0= no affect). The number reported in this and all diet assay tables reflect the average score across all observations. A score of 3 represents complete mortality across all observations. A score of 2.5 would indicate half the wells demonstrating mortality and half scored as severe stunting. The assay results can be found in Table 1A.

DNA sequences which encode double stranded RNAs which were shown to have insecticidal activity (average score above 1.5) against corn rootworms using the assay described in Example 1 are listed in Table 1. To identify full length of cDNA or full open-reading frame of RNAi active target gene, full insert sequencing for EST clones and transcriptome analyses of midgut RNA samples were conducted. Sequences of all target transcripts containing full length cDNA or longer transcripts were also listed in Table 1. Some of these sequences were used for RNAi active fragment search.

### Example 2. Sequences Having Insecticidal Activity

DNA sequences which encode double stranded RNAs which were shown to have insecticidal activity against corn rootworms using the assay described in Example 1 are set forth below. Non-limiting examples of target polynucleotides are set forth below in Table 1A and B, and SEQ ID NOS: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 15 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof, including, for example, SEQ ID NOS: 1, 9, 37, 45, 49, 61, 65, 77, 101, 113, 137, 141, 145, 149, 153, 157, 169, 173, 181, 185, 189, 205, 217, 225,233, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 5 684, 685, 686, 687, 688, 689, 690, 691, 692, and active variants and fragments thereof, and complements thereof, and SEQ ID NOS: 4, 140, 144, 148, 693, 694, 695, 696, 697, 700, 701, 702, 703, 706, 707, 708, 709, 712, 713, 714, 715, 718, 719, 720, 721, 724, 725, 726, 727, 728, and active variants and fragments thereof, and complements thereof.

Subregions of efficacious dsRNAs were designed to improve insecticidal activities in diet and dsRNA expression in planta. These fragments were assayed in the same manner as the original FIS assays described above. Regions demonstrating a severe impact on larval phenotype (mortality or severe growth retardation) were advanced to informal inhibitory concentration (IC₅₀) assays. IC₅₀ assays used doses starting at 50 ppm and progressing downward by ½ step dilutions through 25, 12.5, 6, 3, 1.5, and 0.75pppm. 12 observations were included for each rate. Assay methods were the same as described above for primary screens. Calculations of inhibition relied on scoring for both mortality and severe stunting. Selected fragments were advanced to formal dose response assays where both LC₅₀ and IC₅₀ values were calculated and described in Table 2 (Seq No. 561 to 728). These assays included an initial range finding assay followed by dose response assays for selected ranges including 3 replications of the experiment. Fragments with confirmed IC₅₀ values below 2 ppm were advanced to plant transformation vector construction.

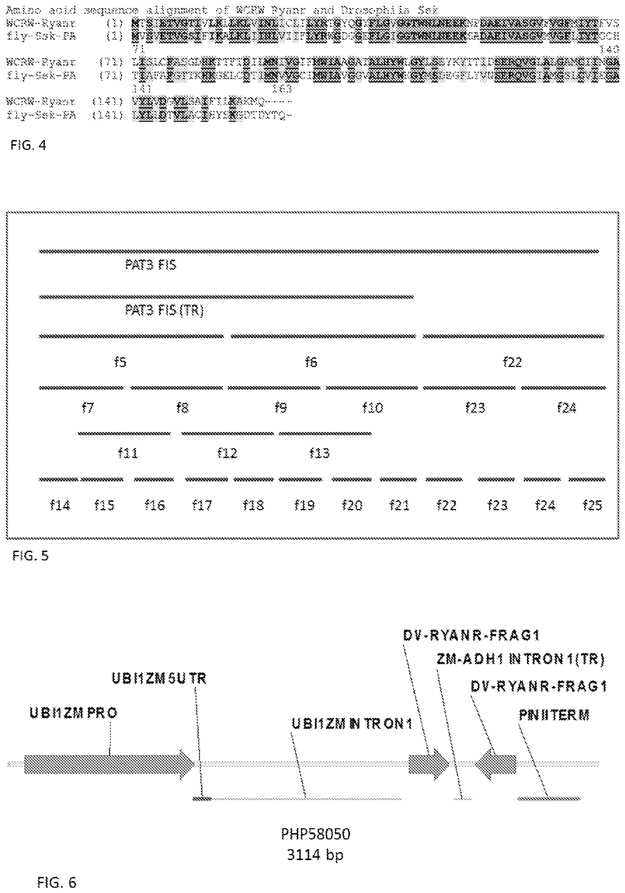

The proteosome alpha subunit type 3 (PAT3) target gene was used as a model for gene and construct optimization. As a first step, the gene was divided into 1/3, 1/6, and 1/12 size fragments (f). In addition, f11-13 represent spanning segments over the boundaries of the first four 1/6^{th} fragments. Figure 5 provides a diagram of the fragments of PAT3.

Plant preferred fragments were identified from active RNAi gene targets and tested in dsRNA artificial diet assays. Selection of these plant preferred regions was based on avoiding destabilizing elements and motifs, or regions with unsuitable base composition. Homology assessments were also employed to avoid potential non target organisms. Finally, fragments with a size range of 150-250 bp were preferred. All rules were considered in selecting fragments but fragments were not excluded from consideration based on any one rule. The Table 2 includes data for initial FIS samples and subsequent fragments insecticidal assay. Selected samples were advanced to IC₅₀ and LC₅₀ determinations.

### Example 3. Identify RNAi active targets from other insects

To identify RNAi active genes from other important corn pests or no-target insects, transcriptome experiments were completed using 3rd instar larvae from Northern corn rootworm (Diabrotica barberi), Southern corn rootworm (*Diabrotica undecimpunctata*), Mexican Bean Beetle (*Epilachna varivestis*), Colorado potato beetle (*Leptinotarsa decemlineata*), Insidious flower bug (*Orius insidiosus*) and Spotted Lady Beetle (*Coleomegilla maculata*, [CMAC]). Homologous transcripts of RNAi active leads were listed in Table 3 (Seq No.693 to 723). This sequence data is important for designing fragments to suppress target pest genes and avoid knockdown same gene in no target insects.

### Example 4. Insecticidal RNA targets in WCRW midgut

Two RNAi active targets Ryanr and HP2 (Table 1 and Table 2) were identified through random cDNA FIS screening. Ryanr was identified in a previous FIS screening (US 2011/0054007A1). Fragments of these targets showed very strong insecticidal activities. Homologous searches reveal that Ryanr and HP2 showed 54% and 49% identity to Drosophila Ssk and Mesh, respectively. The Mesh-Ssk protein complex is required for septate junction formation in the Drosophila midgut. See the amino acid sequence alignment of WCRW Ryanr and Drosophila Ssk in Figure 4.

### Example 5. Transformation of Maize

Immature maize embryos from greenhouse donor plants are bombarded with a plasmid containing the silencing element of the invention operably linked to either a tissue specific, tissue selective, or constitutive promoter and the selectable marker gene PAT (Wohlleben et al. (1988) Gene 70:25-37), which confers resistance to the herbicide Bialaphos. In one embodiment, the constructs will express a long double stranded RNA of the target sequence set forth in Table 3. Such a construct can be linked to the UBIZM promoter. Alternatively, the selectable marker gene is provided on a separate plasmid. Transformation is performed as follows. Media recipes follow below.

### Preparation of Target Tissue

The ears are husked and surface sterilized in 30% Clorox bleach plus 0.5% Micro detergent for 20 minutes, and rinsed two times with sterile water. The immature embryos are excised and placed embryo axis side down (scutellum side up), 25 embryos per plate, on 560Y medium for 4 hours and then aligned within the 2.5cm target zone in preparation for bombardment.

A plasmid vector comprising the silencing element of interest operably linked to either the tissue specific, tissue selective, or constitutive promoter is made. This plasmid DNA plus plasmid DNA containing a PAT selectable marker is precipitated onto 1.1 µm (average diameter) tungsten pellets using a CaCl2 precipitation procedure as follows: 100 µl prepared tungsten particles in water; 10 µl (1 µg) DNA in Tris EDTA buffer (1 µg total DNA); 100 µl 2.5 M CaCl2; and, 10 µl 0.1 M spermidine.

Each reagent is added sequentially to the tungsten particle suspension, while maintained on the multitube vortexer. The final mixture is sonicated briefly and allowed to incubate under constant vortexing for 10 minutes. After the precipitation period, the tubes are centrifuged briefly, liquid removed, washed with 500 ml 100% ethanol, and centrifuged for 30 seconds. Again the liquid is removed, and 105 µl 100% ethanol is added to the final tungsten particle pellet. For particle gun bombardment, the tungsten/DNA particles are briefly sonicated and 10 µl spotted onto the center of each macrocarrier and allowed to dry about 2 minutes before bombardment.

The sample plates are bombarded at level #4 in a particle gun. All samples receive a single shot at 650 PSI, with a total of ten aliquots taken from each tube of prepared particles/DNA.

Following bombardment, the embryos are kept on 560Y medium for 2 days, then transferred to 560R selection medium containing 3 mg/liter Bialaphos, and subcultured every 2 weeks. After approximately 10 weeks of selection, selection-resistant callus clones are transferred to 288J medium to initiate plant regeneration. Following somatic embryo maturation (2-4 weeks), well-developed somatic embryos are transferred to medium for germination and transferred to the lighted culture room. Approximately 7-10 days later, developing plantlets are transferred to 272V hormone-free medium in tubes for 7-10 days until plantlets are well established. Plants are then transferred to inserts in flats (equivalent to 2.5" pot) containing potting soil and grown for 1 week in a growth chamber, subsequently grown an additional 1-2 weeks in the greenhouse, then transferred to classic 600 pots (1.6 gallon) and grown to maturity.

Plants are monitored and scored for the appropriate marker, such as the control of a Coleoptera plant pest, such as a *Diabrotica* plant pest and have insecticidal activity. For example, R0 plant roots are fed to western corn rootworm larvae (WCR, Diabrotica virgifera). Transgenic corn roots are handed-off in Petri dishes with MSOD medium containing antibiotics and glyphosate for in vitro selection. Two WCR larvae are infested per root in each dish with a fine tip paintbrush. The dishes are sealed with Parafilm to prevent the larvae from escaping. The assays are placed into a 27° C, 60% RH Percival incubator incomplete darkness. Contamination and larval quality are monitored. After six days of feeding on root tissue, the larvae are transferred to WCR diet in a 96 well plate. The larvae are allowed to feed on the diet for eight days making the full assay fourteen days long. Larval mass and survivorship are recorded for analysis. A one-way ANOVA analysis and a Dunnett's test is performed on the larval mass data to look for statistical significance compared to an untransformed negative control (HC69). WCR larvae stunting is measured after feeding on two events and compared to growth of larvae fed on negative control plants.

In other assays, transgenic corn plants (R0) generated are planted into 10-inch pots containing Metromix soil after reaching an appropriate size. When plants reach the V4 growth stage, approximately 400 Western corn rootworm (WCR, Diabrotica virgifera) eggs are infested into the root zone. Non-transgenic corn of the same genotype is infested at a similar growth stage to serve as a negative control. Eggs are pre-incubated so hatch occurs within 24 hours of infestation. Larvae are allowed to feed on the root systems for 3 weeks. Plants are removed from the soil and washed so that the roots can be evaluated for larval feeding. Root damage is rated using a Node Injury Scale (NIS) to score the level of damage where a 0 indicates no damage, a 1 indicates that one node of roots is pruned to within 1.5 inches, a 2 indicates that 2 nodes are pruned, while a 3 indicates that 3 nodes are pruned. Because the plants being used for evaluation are directly out of tissue culture after transformation and because transformation events are unique, only a single plant is evaluated per event at this time. The plants in the assay that present signs or symptoms of larval feeding indicate that a successful infestation is obtained. Negative control plant roots are moderately to severely damaged averaging whereas roots of the transgenic plants provide substantial control of larval feeding, with about 0.2 or less on the Corn Rootworm Nodal Injury Score ("CRWNIS").

Bombardment medium (560Y) comprises 4.0 g/l N6 basal salts (SIGMA C-1416), 1.0 ml/l Eriksson's Vitamin Mix (1000X SIGMA-1511), 0.5 mg/l thiamine HCl, 120.0 g/l sucrose, 1.0 mg/l 2,4-D, and 2.88 g/l L-proline (brought to volume with D-I H2O following adjustment to pH 5.8 with KOH); 2.0 g/l Gelrite (added after bringing to volume with D-I H2O); and 8.5 mg/l silver nitrate (added after sterilizing the medium and cooling to room temperature). Selection medium (560R) comprises 4.0 g/l N6 basal salts (SIGMA C-1416), 1.0 ml/l Eriksson's Vitamin Mix (1000X SIGMA-1511), 0.5 mg/l thiamine HCl, 30.0 g/l sucrose, and 2.0 mg/l 2,4-D (brought to volume with D-I H2O following adjustment to pH 5.8 with KOH); 3.0 g/l Gelrite (added after bringing to volume with D-I H2O); and 0.85 mg/l silver nitrate and 3.0 mg/l bialaphos (both added after sterilizing the medium and cooling to room temperature).

Plant regeneration medium (288J) comprises 4.3 g/l MS salts (GIBCO 11117-074), 5.0 ml/l MS vitamins stock solution (0.100 g nicotinic acid, 0.02 g/l thiamine HCl, 0.10 g/l pyridoxine HCl, and 0.40 g/l glycine brought to volume with polished D-I H2O) (Murashige and Skoog (1962) *Physiol. Plant.* 15:473), 100 mg/l myo-inositol, 0.5 mg/l zeatin, 60 g/l sucrose, and 1.0 ml/l of 0.1 mM abscisic acid (brought to volume with polished D-I H2O after adjusting to pH 5.6); 3.0 g/l Gelrite (added after bringing to volume with D-I H2O); and 1.0 mg/l indoleacetic acid and 3.0 mg/l bialaphos (added after sterilizing the medium and cooling to 60°C). Hormone-free medium (272V) comprises 4.3 g/l MS salts (GIBCO 11117-074), 5.0 ml/l MS vitamins stock solution (0.100 g/l nicotinic acid, 0.02 g/l thiamine HCl, 0.10 g/l pyridoxine HCl, and 0.40 g/l glycine brought to volume with polished D-I H2O), 0.1 g/l myo-inositol, and 40.0 g/l sucrose (brought to volume with polished D-I H2O after adjusting pH to 5.6); and 6 g/l bacto-agar (added after bringing to volume with polished D-I H2O), sterilized and cooled to 60 °C.

### Example 6. Agrobacterium-mediated Transformation of Maize

For *Agrobacterium*-mediated maize transformation with the disclosed polynucleotide constructs comprising a silencing element as disclosed herein, the method of Zhao was employed (US Patent Number 5,981,840 and International Patent Publication Number WO 1998/32326, the contents of which are hereby incorporated by reference). Briefly, immature embryos were isolated from maize and the embryos contacted with an *Agrobacterium* suspension, where the bacteria were capable of transferring the desired disclosed polynucleotide constructs comprising a silencing element as disclosed herein, e.g. the polynucleotide construct shown in Figure 6, to at least one cell of at least one of the immature embryos (step 1: the infection step). In this step the immature embryos were immersed in an *Agrobacterium* suspension for the initiation of inoculation. The embryos were co-cultured for a time with the *Agrobacterium* (step 2: the co-cultivation step). The immature embryos were cultured on solid medium following the infection step. Following this co-cultivation period an optional resting step was contemplated. In this resting step, the embryos were incubated in the presence of at least one antibiotic known to inhibit *Agrobacterium* growth without a plant transformant selective agent (step 3: resting step). The immature embryos were cultured on solid medium with antibiotic, but without a selecting agent, for *Agrobacterium* elimination and for a resting phase for the infected cells. Next, inoculated embryos were cultured on medium containing a selective agent and growing transformed callus is recovered (step 4: the selection step). The immature embryos were cultured on solid medium with a selective agent resulting in the selective growth of transformed cells. The callus was then regenerated into plants (step 5: the regeneration step), and calli grown on selective medium were cultured on solid medium to regenerate the plants.

### Example 7 Expression of Silencing Elements in Maize

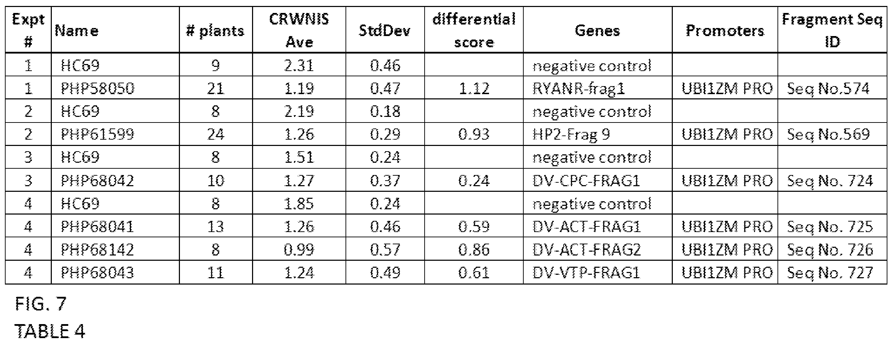

The silencing elements were expressed in a maize plant as hairpins using the transformation techniques described herein above in Example 6, and the plant was tested for insecticidal activity against corn root worms. The data from these studies is shown in Table 4 (Figure 7).

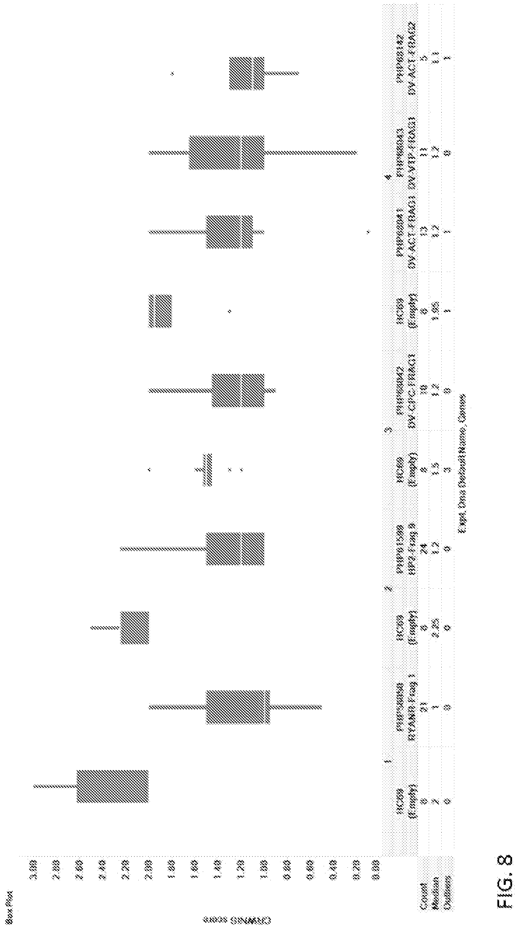

Maize plants were transformed with plasmids containing genes listed in Table 4 (Figure 7), and plants expressing the silencing elements were transplanted from 272V plates into greenhouse flats containing Fafard Superfine potting mix. Approximately 10 to 14 days after transplant, plants (now at growth stage V2-V3) were transplanted into treepots containing Fafard Superfine potting mix. At 14 days post greenhouse send date, plants were infested with 200 eggs of western corn root worms (WCRW)/plant. For later sets, a second infestation of 200 eggs WCRW/plant was done 7 days after the first infestation and scoring was at 14 days after the second infestation. 21 days post infestation, plants were scored using CRWNIS. Those plants with a score of ≤ 0.5 were transplanted into large pots containing SB300 for T1 seed. The data in Table 4 and Figure 8 showed that PHP58050, PHP61599, PHP68041, PHP68142 and PHP68043 showed significant reduced CRWNIS compared to non-transgenic HC6 control plants.

The sequences referred to herein, SEQ. ID NOs: 1-731 are filed concurrently herewith in a textfile and are incorporated herein in their entirities.

As used herein the singular forms "a", "and", and "the" include plural referents unless the context clearly dictates otherwise. Thus, for example, reference to "a cell" includes a plurality of such cells and reference to "the protein" includes reference to one or more proteins and equivalents thereof known to those skilled in the art, and so forth. All technical and scientific terms used herein have the same meaning as commonly understood to one of ordinary skill in the art to which this invention belongs unless clearly indicated otherwise.

All publications and patent applications mentioned in the specification are indicative of the level of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, certain changes and modifications may be practiced within the scope of the appended claims.

### EMBODIMENTS OF THE INVENTION

1. An expression cassette comprising a polynucleotide comprising:
   (a) the nucleotide sequence comprising any one of SEQ ID NOs: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof;
   (b) the nucleotide sequence comprising at least 90% sequence identity to any one of nucleotides SEQ ID NOs: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof; or
   (c) the nucleotide sequence comprising at least 19 consecutive nucleotides of any one of SEQ ID NOs: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof;
   wherein said polynucleotide encodes a silencing element having insecticidal activity against a Coleoptera plant pest.
2. The expression cassette of embodiment 1, wherein said Coleoptera plant pest is a *Diabrotica* plant pest.
3. The expression cassette of embodiment 1, wherein said polynucleotide is operably linked to a heterologous promoter.
4. The expression cassette of embodiment 1, wherein said polynucleotide is expressed as a double stranded RNA.
5. The expression cassette of embodiment 1, wherein said polynucleotide comprise a silencing element which is expressed as a hairpin RNA.
6. The expression cassette of embodiment 5, wherein the silencing element comprises, in the following order, a first segment, a second segment, and a third segment, wherein
   (a) said first segment comprises at least about 19 nucleotides having at least 90% sequence complementarity to a target sequence set forth in any one of SEQ ID NOs: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof;
   (b) said second segment comprises a loop of sufficient length to allow the silencing element to be transcribed as a hairpin RNA; and,
   (c) said third segment comprises at least about 19 nucleotides having at least 85% complementarity to the first segment.
7. The expression cassette of embodiment 1, wherein said polynucleotide is flanked by a first operably linked convergent promoter at one terminus of the polynucleotide and a second operably linked convergent promoter at the opposing terminus of the polynucleotide, wherein the first and the second convergent promoters are capable of driving expression of the polynucleotide.
8. A host cell comprising a heterologous expression cassette of embodiment 1.
9. A plant cell having stably incorporated into its genome a heterologous polynucleotide comprising a silencing element, wherein said silencing element comprises
   (a) a fragment of at least 19 consecutive nucleotides of any one SEQ ID NOs: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof; or,
   (b) the nucleotide sequence comprising at least 90% sequence identity to any one of SEQ ID NOs: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof;
   wherein said silencing element, when ingested by a Coleoptera plant pest, reduces the level of a target sequence in said Coleoptera plant pest and thereby controls the Coleoptera plant pest.
10. The plant cell of embodiment 9, wherein the Coleoptera plant pest is a *Diabrotica* plant pest.
11. The plant cell of embodiment 9, wherein said silencing element comprises,
   (a) a polynucleotide comprising the sequence set forth in any one of SEQ ID NOs: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof; or
   (b) a polynucleotide comprising at least 130 consecutive nucleotides of the sequence set forth in any one SEQ ID NOs: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof.
12. The plant cell of embodiment 9, wherein said plant cell comprises the expression cassette of embodiment 6.
13. The plant cell of embodiment 9, wherein said silencing element expresses a double stranded RNA.
14. The plant cell of embodiment 9, wherein said silencing element expresses a hairpin RNA.
15. The plant cell of embodiment 14, wherein said polynucleotide comprising the silencing element comprises, in the following order, a first segment, a second segment, and a third segment, wherein
   (a) said first segment comprises at least about 19 nucleotides having at least 90% sequence complementarity to a target sequence set forth in any one of SEQ ID NOs: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof,;
   (b) said second segment comprises a loop of sufficient length to allow the silencing element to be transcribed as a hairpin RNA; and,
   (c) said third segment comprises at least about 19 nucleotides having at least 85% complementarity to the first segment.
16. The plant cell of embodiment 9, wherein said silencing element is operably linked to a heterologous promoter.
17. The plant cell of embodiment 9, wherein said plant cell is from a monocot.
18. The plant cell of embodiment 17, wherein said monocot is maize, barley, millet, wheat or rice.
19. The plant cell of embodiment 9, wherein said plant cell is from a dicot.
20. The plant cell of embodiment 19, wherein said plant is soybean, canola, alfalfa, sunflower, safflower, tobacco, *Arabidopsis,* or cotton.
21. A plant or plant part comprising a plant cell of embodiment 9.
22. A transgenic seed from the plant of embodiment 21.
23. A method for controlling a Coleoptera plant pest comprising feeding to a Coleoptera plant pest a composition comprising a silencing element, wherein said silencing element, when ingested by said Coleoptera plant pest, reduces the level of a target Coleoptera plant pest sequence and thereby controls the Coleoptera plant pest, wherein said target Coleoptera plant pest sequence comprise a nucleotide sequence comprising at least 90% sequence identity to any one of SEQ ID NOs: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof.
24. The method of embodiment 23, wherein said Coleoptera plant pest comprises a Diabrotica plant pest.
25. The method of embodiment 23, wherein said silencing element comprises:
   (a) a fragment of at least 19 consecutive nucleotides of any one SEQ ID NOs: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof; or,
   (b) a nucleotide sequence comprising at least 90% sequence identity to any one of SEQ ID NOs: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof,.
26. The method of embodiment 25, wherein said *Diabrotica* plant pest comprises *D. virgifera virgifera*, *D. virgifera zeae, D. speciosa*, *D. barberi, D. virgifera zeae,* or *D. undecimpunctata howardi.*
27. The method of embodiment 23, wherein said composition comprises a plant or plant part having stably incorporated into its genome a polynucleotide comprising said silencing element.
28. The method of embodiment 27, wherein said silencing element comprises
   (a) a polynucleotide comprising the sense or antisense sequence of the sequence set forth in any one SEQ ID NOs: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof;
   (b) a polynucleotide comprising the sense or antisense sequence of a sequence having at least 95% sequence identity to the sequence set forth in any one of SEQ ID NOs: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof; or
   (c) a polynucleotide comprising the sense or antisense sequence of a sequence having at least 130 contiguous nucleotides of any one of SEQ ID NOs: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof.
29. The method of embodiment 27, wherein said silencing element expresses a double stranded RNA.
30. The method of embodiment 27, wherein said silencing element comprises a hairpin RNA.
31. The method of embodiment 30, wherein said polynucleotide comprising the silencing element comprises, in the following order, a first segment, a second segment, and a third segment, wherein
   (a) said first segment comprises at least about 19 nucleotides having at least 90% sequence complementarity to the target polynucleotide;
   (b) said second segment comprises a loop of sufficient length to allow the silencing element to be transcribed as a hairpin RNA; and,
   (c) said third segment comprises at least about 19 nucleotides having at least 85% complementarity to the first segment.
32. The method of embodiment 27, wherein said silencing element is operably linked to a heterologous promoter.
33. The method of embodiment 27, wherein said silencing element is flanked by a first operably linked convergent promoter at one terminus of the silencing element and a second operably linked convergent promoter at the opposing terminus of the polynucleotide, wherein the first and the second convergent promoters are capable of driving expression of the silencing element.
34. The method of embodiment 27, wherein said plant is a monocot.
35. The method of embodiment 34, wherein said monocot is maize, barley, millet, wheat or rice.
36. The method of embodiment 27, wherein said plant is a dicot.
37. The method of embodiment 36, wherein said plant is soybean, canola, alfalfa, sunflower, safflower, tobacco, *Arabidopsis,* or cotton.
38. An isolated polynucleotide comprising a nucleotide sequence comprising:
   (a) the nucleotide sequence comprising any one of SEQ ID NOs: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof;
   (b) the nucleotide sequence comprising at least 90% sequence identity to any one of nucleotides SEQ ID NOs: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof; or
   (c) the nucleotide sequence comprising at least 19 consecutive nucleotides of any one of SEQ ID NOs: 1, 4, 5, 8, 9, 12, 13, 16, 17, 20, 21, 24, 25, 28, 29, 32, 33, 36, 37, 40, 41, 44, 45, 48, 49, 52, 53, 54, 55, 56, 57, 60, 61, 64, 65, 68, 69, 72, 73, 76, 77, 80, 81, 84, 85, 88, 89, 92, 93, 96, 97, 100, 101, 104, 105, 108, 109, 112, 113, 116, 117, 120, 121, 124, 125, 128, 129, 132, 133, 136, 137, 140, 141, 144, 145, 148, 149, 152, 153, 156, 157, 160, 161, 164, 165, 168, 169, 172, 173, 176, 177, 180, 181, 184, 185, 188, 189, 192, 193, 196, 197, 200, 201, 204, 205, 208, 209, 212, 213, 216, 217, 220, 221, 224, 225, 228, 229, 232, 233, 236, 237, 240, 241, 244, 245, 248, 249, 252, 253, 256, 257, 260, 261, 264, 265, 268, 269, 272, 273, 276, 277, 280, 281, 284, 285, 288, 289, 292, 293, 296, 297, 300, 301, 304, 305, 308, 309, 312, 313, 316, 317, 320, 321, 324, 325, 328, 329, 332, 333, 336, 337, 340, 341, 344, 345, 348, 349, 352, 353, 356, 357, 360, 361, 364, 365, 368, 369, 372, 373, 376, 377, 380, 381, 384, 385, 388, 389, 392, 393, 396, 397, 400, 401, 404, 405, 408, 409, 412, 413, 416, 417, 420, 421, 424, 425, 428, 429, 432, 433, 436, 437, 440, 441, 444, 445, 448, 449, 452, 453, 456, 457, 460, 461, 464, 465, 468, 469, 472, 473, 476, 477, 480, 481, 484, 485, 488, 489, 492, 493, 496, 497, 500, 501, 504, 505, 508, 509, 512, 513, 516, 517, 520, 521, 524, 525, 528, 529, 532, 533, 536, 537, 540, 541, 544, 545, 548, 549, 552, 553, 556, 557, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 700, 701, 702, 703, 706, 707, 708, 709, 712, 713, 714, 715, 718, 719, 720, 721, 724, 725, 726, 727, 728, or active variants and fragments thereof, and complements thereof;
   wherein said polynucleotide encodes a silencing element having insecticidal activity against a Coleoptera plant pest.
39. The isolated polynucleotide of embodiment 38, wherein said Coleoptera plant pest is a *Diabrotica* plant pest.

## Claims

1. An expression cassette comprising a polynucleotide encoding a sense or antisense strand of a double stranded RNA wherein the polynucleotide consists of:
(a) a nucleotide sequence set forth in any one of SEQ ID NOs: 1, 4, 561, 562, 563, 564, 565, 566, 567, 568, 569, 570, 571, 572, and complements thereof;
(b) a nucleotide sequence having at least 90% sequence identity to any one of nucleotides SEQ ID NOs: 1, 4, 561, 562, 563, 564, 565, 566, 567, 568, 569, 570, 571, 572, and complements thereof; or
(c) at least 19 consecutive nucleotides of any one of SEQ ID NOs: 1, 4, 561, 562, 563, 564, 565, 566, 567, 568, 569, 570, 571, 572, and complements thereof;
wherein said double stranded RNA has insecticidal activity against a Coleoptera plant pest.

2. The expression cassette of claim 1, wherein:
(a) said Coleoptera plant pest is a *Diabrotica* plant pest;
(b) said polynucleotide is operably linked to a heterologous promoter;
(c) said polynucleotide comprises a silencing element which is expressed as a hairpin RNA; or
(d) said polynucleotide is flanked by a first operably linked convergent promoter at one terminus of the polynucleotide and a second operably linked convergent promoter at the opposing terminus of the polynucleotide, wherein the first and the second convergent promoters are capable of driving expression of the polynucleotide.

3. The expression cassette of claim 2(c), wherein the double stranded RNA comprises, in the following order, a first segment, a second segment, and a third segment, wherein:
(a) said first segment consists of a sense or antisense nucleotide sequence having at least 19 nucleotides having at least 90% sequence complementarity to a sequence set forth in any one of SEQ ID NOs: 1, 4, 561, 562, 563, 564, 565, 566, 567, 568, 569, 570, 571, 572, and complements thereof;
(b) said second segment consists of a loop of sufficient length to allow the silencing element to be transcribed as a hairpin RNA; and,
(c) said third segment consists of at least 19 nucleotides having at least 85% complementarity to the first segment.

4. A host cell comprising a heterologous expression cassette of claim 1.

5. A plant cell having stably incorporated into its genome a heterologous polynucleotide encoding a sense or antisense strand of a double stranded RNA, wherein said polynucleotide consists of:
(a) a fragment of at least 19 consecutive nucleotides of any one SEQ ID NOs: 1, 4, 561, 562, 563, 564, 565, 566, 567, 568, 569, 570, 571, 572, and complements thereof; or,
(b) a nucleotide sequence having at least 90% sequence identity to any one of SEQ ID NOs: 1, 4, 561, 562, 563, 564, 565, 566, 567, 568, 569, 570, 571, 572, and complements thereof; wherein said double stranded RNA, when ingested by a Coleoptera plant pest, reduces the level of a target sequence in said Coleoptera plant pest and thereby controls the Coleoptera plant pest.

6. The plant cell of claim 5, wherein:
(a) the Coleoptera plant pest is a *Diabrotica* plant pest;
(b) said plant cell comprises the expression cassette of claim 3;
(c) said double stranded RNA expresses a hairpin RNA;
(d) said double stranded RNA is operably linked to a heterologous promoter;
(e) said plant cell is from a monocot; optionally wherein said monocot is maize, barley, millet, wheat or rice; or
(f) said plant cell is from a dicot; optionally wherein said plant is soybean, canola, alfalfa, sunflower, safflower, tobacco, *Arabidopsis,* or cotton.

7. The plant cell of claim 5, wherein said double stranded RNA comprises:
(a) a polynucleotide consisting of the sense or antisense sequence of a sequence set forth in any one of SEQ ID NOs: 1, 4, 561, 562, 563, 564, 565, 566, 567, 568, 569, 570, 571, 572, and complements thereof; or
(b) a polynucleotide consisting of at least 130 consecutive nucleotides of the sequence set forth in any one SEQ ID NOs: 1, 4, 561, 562, 563, 564, 565, 566, 567, 568, 569, 570, 571, 572, and complements thereof.

8. The plant cell of claim 6(c), wherein said double stranded RNA comprises, in the following order, a first segment, a second segment, and a third segment, wherein:
(a) said first segment consists of at least 19 nucleotides having at least 90% sequence complementarity to a target sequence set forth in any one of SEQ ID NOs: 1, 4, 561, 562, 563, 564, 565, 566, 567, 568, 569, 570, 571, 572, and complements thereof;
(b) said second segment consists of a loop of sufficient length to allow the silencing element to be transcribed as a hairpin RNA; and,
(c) said third segment consists of at least 19 nucleotides having at least 85% complementarity to the first segment.

9. A plant or plant part comprising a plant cell of claim 5, or a transgenic seed comprising the heterologous polynucleotide as defined in claim 5.

10. A method for controlling a Coleoptera plant pest comprising feeding to a Coleoptera plant pest a composition comprising a double stranded RNA, which when ingested by said Coleoptera plant pest, reduces the level of a target Coleoptera plant pest sequence and thereby controls the Coleoptera plant pest, wherein the sense or antisense sequence of the double stranded RNA consists of a nucleotide sequence having at least 90% sequence identity to the sense or antisense sequence of any one of SEQ ID NOs: 1, 4, 561, 562, 563, 564, 565, 566, 567, 568, 569, 570, 571, 572, and complements thereof.

11. The method of claim 10, wherein:
(I) said Coleoptera plant pest comprises a Diabrotica plant pest;
optionally wherein said *Diabrotica* plant pest comprises *D. virgifera virgifera*, *D. virgifera zeae, D. speciosa*, *D. barberi, D. virgifera zeae,* or *D. undecimpunctata howardi*; or
(II) said composition comprises a plant or plant part having stably incorporated into its genome a polynucleotide comprising said double stranded RNA.

12. The method of claim 11(II), wherein:
(I) said double stranded RNA comprises a hairpin RNA; optionally wherein said polynucleotide encoding the double stranded RNA comprises, in the following order, a first segment, a second segment, and a third segment, wherein:
(a) said first segment consists of at least 19 nucleotides having at least 90% sequence identity to the sense or antisense sequence of any one of SEQ ID NOs: 1, 4, 561, 562, 563, 564, 565, 566, 567, 568, 569, 570, 571, 572, and complements thereof;
(b) said second segment consists of a loop of sufficient length to allow the silencing element to be transcribed as a hairpin RNA; and,
(c) said third segment consists of at least 19 nucleotides having at least 85% complementarity to the first segment;
(II) said silencing element is encoded by a polynucleotide operably linked to a heterologous promoter;
(III) said silencing element is encoded by a polynucleotide, wherein the polynucleotide is flanked by a first operably linked convergent promoter at one terminus of the polynucleotide and a second operably linked convergent promoter at the opposing terminus of the polynucleotide, wherein the first and the second convergent promoters are capable of driving expression of the silencing element;
(IV) said plant is a monocot, optionally wherein said monocot is maize, barley, millet, wheat or rice; or
(V) said plant is a dicot, optionally wherein said plant is soybean, canola, alfalfa, sunflower, safflower, tobacco, *Arabidopsis,* or cotton.

13. An isolated polynucleotide comprising a nucleotide sequence consisting of:
(a) the sequence set forth in any one of SEQ ID NOs: 1, 4, 561, 562, 563, 564, 565, 566, 567, 568, 569, 570, 571, 572, and complements thereof;
(b) the nucleotide sequence having at least 90% sequence identity to any one of nucleotides SEQ ID NOs: 1, 4, 561, 562, 563, 564, 565, 566, 567, 568, 569, 570, 571, 572, and complements thereof; or
(c) at least 19 consecutive nucleotides of any one of SEQ ID NOs: 1, 4, 561, 562, 563, 564, 565, 566, 567, 568, 569, 570, 571, 572, and complements thereof;
wherein said polynucleotide encodes a silencing element which is expressed as a double stranded RNA having insecticidal activity against a Coleoptera plant pest.

14. The isolated polynucleotide of claim 13, wherein said Coleoptera plant pest is a *Diabrotica* plant pest.

15. A double stranded RNA targeting a Coleopteran plant pest target polynucleotide, wherein the double stranded RNA comprises a polynucleotide consisting of:
(a) a sense or antisense sequence of a nucleotide sequence set forth in any one of SEQ ID NO: 1, 4, 561, 562, 563, 564, 565, 566, 567, 568, 569, 570, 571, 572;
(b) a sense or antisense sequence of a nucleotide sequence having at least 95% sequence identity to SEQ ID NO: 1, 4, 561, 562, 563, 564, 565, 566, 567, 568, 569, 570, 571, 572; or
(c) a sense or antisense sequence of a nucleotide sequence consisting of at least 25 consecutive nucleotides from nucleotides 50, 25-75,75-125,50-100,125-175, 100-150 or 150-200 of any one of SEQ ID NO: 1, 4, 561, 562, 563, 564, 565, 566, 567, 568, 569, 570, 571, 572;
wherein said double stranded RNA has insecticidal activity against a Coleopteran plant pest.

16. The double stranded RNA of claim 15, wherein:
(a) said Coleopteran plant pest is a *Diabrotica* plant pest;
(b) the double stranded RNA comprises a hairpin RNA;
(c) the double stranded RNA is expressed in a plant, plant part, or plant cell; or
(d) the double stranded RNA is expressed in a microorganism.

17. A composition comprising the double stranded RNA of claim 15, further comprising an agriculturally acceptable carrier.


SEQUENCE LISTING

<110>  Pioneer Hi-Bred International
       Diehn, Scott
       Harnack, Michelle
       Hu, Xu
       Presnail, James
       Richtman, Nina
       Procyk, Lisa

<120>  Novel RNAi Gene Targets from the Western Corn Rootworm Diabrotica
        virgifera

<130>  36446.0007P1

<150>  61/785,680
<151>  2013-03-14

<160>  731

<170>  PatentIn version 3.5

<210>  1
<211>  593
<212>  DNA
<213>  Diabrotica virgifera


<220>
<221>  misc_feature
<222>  (1)..(593)
<223>  RNAi target sequence

<220>
<221>  misc_feature
<222>  (480)..(480)
<223>  n can be any nucleotide

<220>
<221>  misc_feature
<222>  (495)..(495)
<223>  n can be any nucleotide

<400>  1
tgacttcaca cttccagatg gttctagagc agcaatagtg aataacataa atgacatgga      60

aagggtttac aatgattttg gttccaaatg gatgttggag gatgtgttag atcctcaaaa     120

gggtagagca ttgttccaca gggaattcgg aaggacttca gcgacttaca acaacaaaac     180

gttcaagcct caattcctaa tgaatccgga agatttccta ccaaccaaca gatctacaga     240

tcttaagcga atatccgaaa tatgtcctct aaagttgtac gaatgttact acgattacgc     300

tatgacggtt gacagggatt tggcgcatta caccaagaat tataaagcca cgatctatca     360

atataaagaa acgacaagga ggaaagttgt gtcttgcgga gtactagaaa caccgagatt     420

cggacgaaag agtactttcc tgttcgtgcc cggtacgaag gttacctatg aatgcatccn     480

agagttcgtg ttggntaggt gatcagcgga gagagtgtca agcggatgga acatggaata     540

ttcctgaata cggatacaca tattgcttac gtcagcagga atactctcaa cga            593

<210>  2
<211>  22
<212>  DNA
<213>  Artificial Sequence

<220>
<223>  primer


<220>
<221>  misc_feature
<222>  (1)..(22)
<223>  forward primer

<400>  2
tgacttcaca cttccagatg gt                                                22


<210>  3
<211>  22
<212>  DNA
<213>  Artificial Sequence

<220>
<223>  primer


<220>
<221>  misc_feature
<222>  (1)..(22)
<223>  reverse primer

<400>  3
tcgttgagag tattcctgct ga                                                22


<210>  4
<211>  5689
<212>  DNA
<213>  Diabrotica virgifera


<220>
<221>  misc_feature
<222>  (1)..(5689)
<223>  WCRW transcript ta0120.010_diavv

<400>  4
tgttcatcac caacgctgac caacgtgaga gtagcaactt tttttgatgt gatgtgcgcc      60

agtttgtagt gccaaataac caaaatgtgg ttcaagtact ttgttatttt tgttttgttt     120

agtgttagcg ttaaaggact agatggtgat cttccaccag atgtcgaaac tatcgatgtg     180

gagactacga atgtaagtaa cgcggatcca gacgcaggga aacctattcc ggtggaagat     240

acgcctaatg gagtccctcc agtgaataca aactatgaag tagataaatc cttagttaat     300

gtaaccaata gtttggtaaa tgtgactaaa aatcccagac ggacttatca gacagttaat     360

aaaatgggca aatattcgac attttatgct gcagattacg atcctatgac ctcagacaca     420

gctccaccag acactgacca acgaggagga ggaggaactc catacactat atcagaaccg 480

agactggcag aaataaggaa acattttatg tacccatttt tcgataaagg aggcagtgat 540

aataacttgg gagatttcca aaaggacatt caatcttcca ttcctcaggt gcacaagaat 600

ttaaacttcc agttgccttt ctttggattc aggtataatt acacaagagt ttcagtgaat 660

gggtatctgg aattcagcga tccacctcca aattatgact atccattagt atttccaatt 720

aaagaatggc ccaaaaagaa tgatcctgcc tttattggca ttttcttcag cagatgcaga 780

atcggtaatc tacgtgatgg agacattgat caaagaacac ccggggtcta ttttagaatg 840

gaaagagatt taagaacgag gcaagacaga atgggtgtag aaattagaga aagacttaag 900

tgggatatca gaacaggagt cattggatca gaaacattcg atcctaagca cgccgttatt 960

gtcacttgga aaaatgttac tttcactgga ggattcgcta atgctaaata caagacaaac 1020

accttccaaa tggttcttgc aactgacgaa gtcttcactt acgcgatgtt caactacctc 1080

aacttggact ggacctccca cactgaagcg ggtggtgatt cccaaaacgg ggaaggtggt 1140

gtcagtgcct atgttggttt taacgctgga aacggtacca gaagctacga atacaatcct 1200

tacagtcaag cttccgtcat tcgagatctc acatcagttg gatttggaaa tggttttaag 1260

ggaagacata ttttccgtat agacgaagac attttgcttg gatcgtgcaa caaagatata 1320

gatggtgcca accttcccct gaaattcgct cctgaaagtg gtaacatgtt gggtggaaca 1380

gtggtaaata taactggtcc atgcttcaat ttgaatgata ggattcgatg caagtttgac 1440

gtatcaaatg aagtatttgg gtatgttgtt gataaaaaca gagctatctg tgtacaacct 1500

caactttatg ccgaaggatg ggtgaatctt caaattgctg ttaattccga agcattcaag 1560

tggaagggga agtattatgt agaatcacct gctagtgcga ctcaaaaaat cttctttaag 1620

gacatgaaat tacacgaaaa atctccgagc gaattaaaaa tcacttggga gaagcagaat 1680

ttgaccacaa acgacaacgc taacatccga atttctctct ggggttacag agaaacgact 1740

atgaaaccag tgtttgttta tatcaccgat atcgctgata atgttcaaaa cattggagaa 1800

cacacgattg ttccttctca gtttagaact agagtcaatc aatatctgac tgatattaag 1860

tttggattcc tccaaattaa tttaactgag tctatcacag ttaatacata tactacaagc 1920

cagagcagca tagaattaac accggtaata tggagccgac ccattccact cggttggtac 1980

ttccagttcc aatgggaaaa ccagtacggt aaaaattggc ccaaatatct atgtgacgat 2040

tggctgagaa cggacagata tctaaaaaac ttcgcccacg aacttgctca atgtccctgc 2100

accgtcgaac aggctttagc tgacaagggc aggtttatgc cagacttcga ttgcgacaaa 2160

gattcgaacc cgatttgtta ctataacaac caagcacttc attgtgtgaa aactggatct 2220

cccactttag aaggctccga acaacaatgt tgctatgata aaaacggctt tttgatgctc 2280

tcttacgatc agcaatgggg ttctagtccg agacgttgcc acaacttggg taaaatgcct 2340

tacaatgaag cgacaaaagt gcctactctc tcacaatggt ttaatgacat ggtacccaaa 2400

tatttgtgtt gtctttggca agacgagcaa gctgtgggat gtgaaacact gagatttgaa 2460

cggagaccta cacaggattg tgtagcttac caagcaccag gtgtagcagg agtctacggt 2520

gatccacact ttgttacttt cgacgatgta gaatatacgt ttaatggaaa gggagaattc 2580

gcattggtga aatcagttac gcaaacggac aatctggaaa tccaaggaag atttgagcaa 2640

atggacccta acacttatgg tgaggttaga gctactcagc tgacttctgt agtagctaaa 2700

ggtaataaca caatagcaat tgaagtcaga aggagacctc tggattctag atggagatat 2760

aggttggatg ttatagcaga taatagaaga ctttattttg atagaccctc actaaaattc 2820

cagcacttcc aaggtgttac tatttacact ccaagctaca tccttaatca atcagaagtc 2880

atcattatgt ttgacaatgg agctggaatg gaggttgtcg acaatcaagg attcatgagt 2940

gcaagagtat ttttgccttg gtcgtttata aataaaacca taggcctttt tggaaattgg 3000

agttttaaca aagaagatga cttcacactt ccagatggtt ctagagcagc aatagtgaat 3060

aacataaatg acatggaaag ggtttacaat gattttggtt ccaaatggat gttggaggat 3120

gtgttagatc ctcaaaaggg tagagcattg ttccacaggg aattcggaag aacttcagcg 3180

acttacaaca acaaaacgtt caagcctcaa ttcctaatga atccggaaga tttcctacca 3240

accaacagat ctacagatct taagcgaata tccgaaatat gtcctctaaa gttgtacgaa 3300

tgttactacg attacgctat gacggttgac agggatttgg cgcattacac caagaattat 3360

aaagccacga tctatcaata taaagaaacg acaaggagga aagttgtgtc ttgcggagta 3420

ctagaaacac cgagattcgg acgaaagagt actttcctgt tcgtgcccgg tacgaaggtt 3480

acctatgaat gcatccaaga gttcgtgttg gtaggtgatc agcggagaga gtgtcaagcg 3540

gatggaacat ggaatattcc tgaatacgga tacacatatt gcttacgtca gcaggaatac 3600

tctcaacgac aagctggttt ggcttccggg ataattatgg ctattcttat cccgctcatc 3660

ttgcttttcg tctacctggc gtatcgtttc ttacaaagaa ggcagaaaga aaaagaatct 3720

gaaaaattac aagagcaaca gttcatggaa tcacaaagga gagcccaaga agcagctact 3780

aagaaattaa cagaagctga atatgcttca gaagatgaag acagtaatgt tacaagcacg 3840

ggagcaaaag aaacaaccgt atactaacac ctatagaata taatgattca acctatgaat 3900

tgtaatattt taaagtttaa tctttgtcaa aactattata taaaataagt actatattat 3960

gttgtagtta atatttaata ggacttttat tttttattgt atttcagatt atatttcatg 4020

cagagtgtga tttagattat atttcatgca aagtgtagag tttcagatat agccttttta 4080

accacaacgt aatccgattc ttatagtaaa ttcaacatta tttaagatac tttttcattg 4140

aattgtatac ctatctaatc ctgcaagacc cacatatctt tttcccaatt aaaagagtaa 4200

ataggaagat acgaaagcac aaagttaaaa taaattaacg aatttcataa aacccagtag 4260

aaagctatgt tctgcagtta ttatctaaat tagaatacta ttaagtaacc atatcaccct 4320

tctcaagttt acattaacat caagatctac tggtggcatc cataggtagg tgcctactcg 4380

tatatttagt aaacaacagt ttgttgatcc atcaacttct tgttggtaag attcagctca 4440

acgttgaggg caaccggccc ttcttcttaa agttccatct tctatcagag gttggatatc 4500

ataatgggtt tggtcactat gttggctgct gctctgaata gttgtaataa actacagtta 4560

aaccattatc taaggttctt tagccaggag atgcgtcttc ttccttggat ccttccttgc 4620

ataataattc tcagcaactc atatttttct cctctggtaa tgtgacccaa atattccagt 4680

tttctatgtt tttatactgt tcataatttc taactcctta tttaaacgtc gtagcacttc 4740

aacattggta atcctttgaa cccactgaat tttcaatatt cttctgtaac accacatttc 4800

gaacgcttct atttttctta tgtgttgtct cttcaacgtc caagcttcca ttccgtatag 4860

taatatagaa aatatgtagc atcttagagc cctcaatctg agaggcaact gaaggtcttt 4920

gtttgtaagc agtgtcttca tttataaatg cttgccttgc agtttcaatt cggactttaa 4980

tttctttgct ttggtcattt ttgtcatcaa cccaggttcc cagatattta tatgtctaac 5040

tttttctatt tgggtttgct ctctatcatt aacctttcat ttccatgttg tgtttttgag 5100

acaatcataa atttagtttt tttcttgttt atttttagtc cgtatcgaat gcaataatcg 5160

ttaattctat ttagcaattc ttgtagcgat cccagggtgt ctgccattat aaggatgtca 5220

tcagcgaatc ttgtgttatt tactattttt ccgtttatag agattctatc acttagctcc 5280

gctatcgctt cctgaaatat ggcttcactg tacacgttaa acagtagcgg cgacagaaca 5340

caaccctgtc ttactcctct ctttatatca atattctcgg attcttgttc ttctatcttt 5400

atattggcct tttgattcca atacagattg atgataattc gcaaatctcg taaaaaagca 5460

gctctcattt aatttaacga agaaccatgt gtaggaaacc catcagcaga atgtagcagg 5520

ctctggttgc caataataaa gaagtcagca acaagaatat accaacatta acggattagt 5580

agaaagttgg ggtcacttct tcttttggca tgataagcct agatgggtct ttgcctgtct 5640

ggctatgttc cattcagatc tctcttgtgc tcttctccat tgtctgttc 5689

<210> 5
<211> 446
<212> DNA
<213> Diabrotica virgifera

<220>
<221> misc_feature
<222> (1)..(446)
<223> RNAi target sequence

<400>  5
aagagtagcg atgggtggtt tacctcaagg aggaatatta agccctttgt tatatttaat      60

ttacacttct gatatagaaa aaaatttaaa ctcaacaaaa attttacaat ttgcagatga     120

tgtagttatt tatcaagaaa acattaaaat agaaaatgca gtcaaatcca ttgaagaagg     180

agacaaacat attaaaatat ggagtgaatt acatggacta aatatatctg attccaaaac     240

caaattatgt atttttacaa gaaaacgaaa agaaataccc aatcacatct taataaacaa     300

tatatcctat cctgtacaaa gttatgttaa atatttgggt attactctgg atagacagct     360

tctctggaaa gaacatatag acaatatagt taaaaaaaca gaaaaaggaa taaaccttct     420

tcgattcgta tcacacttac gttggg                                          446


<210>  6
<211>  22
<212>  DNA
<213>  Artificial Sequence

<220>
<223>  primer


<220>
<221>  misc_feature
<222>  (1)..(22)
<223>  forward primer

<400>  6
aagagtagcg atgggtggtt ta                                              22


<210>  7
<211>  22
<212>  DNA
<213>  Artificial Sequence

<220>
<223>  primer


<220>
<221>  misc_feature
<222>  (1)..(22)
<223>  reverse primer

<400>  7
cccaacgtaa gtgtgatacg aa                                              22


<210>  8
<211>  7388
<212>  DNA
<213>  Diabrotica virgifera


<220>
<221>  misc_feature
<222>  (1)..(7388)

<223>  WCRW transcript ta0145.001_diavv

<400>  8
catatattcc aacgtttttt actcaaaaga aaggtagtta taaaattagg tagataaaga      60

gatagaagat aatgatttat tatcattaat taaacctaga tttggaatta aattcgaagt     120

attacatgta aaaagaatta tgcggaaagt gattcaagat aatggtaata ctaattatag     180

taaaaacagg aaccgtaatt gtcactttta aaggtcagtc tataccaaaa aatgtaataa     240

tagaaaaaaa tgatatacga ggtggaaaat tatacaccca gaataatcca atgtttaaag     300

tgtttgagat ttgggcatat aagtgcccaa tgtagaggaa aagataggtg tgaaagatgt     360

ggcgaagaac acaacaaatc taattgttcc aatccgaata atttactttg tgtattgtgc     420

aaaggaaaac acagcgctac tgacaaggga gcagattgta cagaaagaca aaaacaggaa     480

tatattaaaa aaattatgaa taatgaaaat ataacatatt atgaagctaa taataaatat     540

aaaaaatgtt attcaacagt aagtaaagac caagaaaata cttcaaataa atatacaata     600

tctaaacgaa aaagatcaag tagtattgat tctagtagtg tagataaaga aacaatggaa     660

gcacgcagaa aaattttgca aacaccaaga atacaaagtc agccttgtta taattttaat     720

aatcccattt attctaacac aacacaaaca caaaacgata atcaaaataa tataggagaa     780

ataatagtaa actttatttc agcaacatta aatcaaatta atcacaattt agatcaaaaa     840

acgttggaat tattaaaaaa caatatttca caattattat tacctcgtga tggctaacgt     900

ttcatttctt caatggaatg cgagatcaat taaaacaaat aagggttatt tagaaaaatt     960

cttgtatgac aataaaatag atataggatt aatatcagaa acttggttaa aaaaaagtaa    1020

ttttattaac tttactggtt acaatgtctt taggaatgat agagatgatg gatatggtgg    1080

agtagccatc ccttccaaaa aattaataaa attttacaac agtcctactt ttcaccaaat    1140

taatgacata atgtgcaata gcatatcaat taaaattcaa tccggaaaaa agttaaacat    1200

ttattcaata tacgtaaaac caaatcttaa aatcactcta aatgaatgga aaaagttttt    1260

taatagtcta gagaagcctt ttataattgg tggtgatttt aatagccaca attatgtttg    1320

gggttgtagt actgtagata ctataggaaa aaatctgtta gaagctattg aggactgtaa    1380

tcttgtaatt ttaaataatg gtaaagaaac tttgatgcgt agaccgaata gcaataataa    1440

atctgcaata gatcttacaa tatgctcagc aaatattagt cattttttcg attgggatgt    1500

tgtggacgag acattaggat caaatcattt tgctattatt attaagtcaa atattaatgt    1560

taataaagcg gaatgtgtaa atacaaaatt ccaatggaac ataaataaag cggattggtc    1620

tcttttctct tctatcactg ataatttcat ggaaatagat aataatttaa attaccaaca    1680

atttttaaat aaattaaacg aatattgtaa gatatgtata ccggagaaga gaacagggac    1740

taatccacga tttagaaaaa cttggtggaa tgacaattgt aaaaaggtaa tagaagaaca    1800

aaaactagct ttacgcaagt ttaaactaca gtctaatata caaaattata taaattataa 1860

taaatgtgta gcgaaaacca aaaaagttgt attagagagt aagcgtaatg catggagaaa 1920

tttttgtaaa actttaaaca aaaatacacc aattaaagat atgtggaatc aagccaaacg 1980

attacaaaac atttttaaac cacaagtaaa tccagtgact gaaggagaat gggttgagga 2040

gtttttaaga aaattatgtc cagataatat attttcaaaa attaatgtaa tggaaagaaa 2100

aaactctatg catccacttt caattccatt tagtttctgt gaattagaaa taagccttaa 2160

gaataagaaa aatacttctc caggtataga taatgtacat tatattatgt tggccaattt 2220

acatcaaaaa gggaaagaaa cactattaaa tttttttaat caaatatggt tatcagaaga 2280

tattccagat aactggagag agtaccgggt gattcctatt ctcaaaacaa atcggaatcc 2340

tgattcagca gaatcttata gaggtatttc attgagctcc tgcataacaa aaacactgga 2400

aagaatgata aaacttaggc tcgaaagttg gctagaaaaa aacaataaat tatcacaaac 2460

acaatttgga tttcgaaaaa atcattctac tgtggaagct gtgagtcatt tggtaacaga 2520

tataaattta gcgtttacga aaaattcttc agtaatcgct cttttattag atgttgaagc 2580

agcatacgac aacgttaatt taaatatact atataacaaa atgatacaaa taggtctgcc 2640

agaatgcttc tgtcaaaaaa taataaaatt gtatgactgt agaaaaattt atatatcggt 2700

aaataataac acatttggtc caagagtagc gatgggtggt ttacctcaag gaggaatatt 2760

aagccctttg ttatatttaa tttacacttc tgatatagaa aaaaatttaa actcaacaaa 2820

aattttacaa tttgcagatg atatagttat ttatcaagaa aacattaaaa tagaaaatgc 2880

agtcaaatcc attgaagaag gagacaaaca tattaaaata tggagtgaat tacatggact 2940

aaatatatct gattccaaaa ccaaattatg tatttttaca agaaaacgaa aagaaatacc 3000

caatcacatc ttaataaaca atatatccta tcctgtacaa agttatgtta aatatttggg 3060

tattactctg gatagacagc ttctctggaa agaacatata gaccatatag ttaaaaaaac 3120

agaaaaagga ataaaccttc ttcgattcgt atcacactta cgttggggag cagatccaaa 3180

tatttctttg ttgttattta aaaataatat aagagctata ttcgactacg gatcaatatt 3240

atacagtgac gcatcacagt gtcatttaaa taaaatagac aaaatcatta ataaatccct 3300

tagattgtgt ataggagccc taagaagtac accgataaat aatctacaag ttgaatgctg 3360

tgaaatgccg atggatataa gacgaaaaaa acttggcatc aaccttttag ttagattgta 3420

tgaaaaaaag gcaagtttaa tttccaaaat acatcaactg tttttagcag acttgacaga 3480

aaaatattgg ataaaaaaga aaactctaaa tatggtagat ttatattcaa aaatgacaat 3540

atatgataaa caactttata aatatgacat aaacccaaat tataatattg actttaatag 3600

tatggaacaa gttcaggtat actttttaag ggactatagc aagttgccta tatctttaag 3660

aaaattagtg tttatctccg acagttcaca aatgttcaaa gattattgta tgctatatac 3720

agatgcatca aaaaatattg aaggtgtggg atgtgcctat tgggatagca gtaataaaat 3780

tagtaaaatg tttaaactaa attctattat gagtatatac acagctgaat taatagcgat 3840

aatggaagca ctaaaatatt tatctaatat aaatcattca aagtttataa tttttagtga 3900

cagtaaaagt tctcttagta aaattagtgc tataaatcct aaatcaaaag tgaactacat 3960

tgaattatat atcataaata aaattaaaga acttcagcaa caaggaaagt ctgttaagtt 4020

ggcatgggtt aaaagccact gtggaataac tggaaatgaa atggtagatg aattggctaa 4080

aaacgcggtg acacaaggag tattaaccca ttatctttgt acgccaaaag atattgaatc 4140

aaatttattc aaagagatta agaaagaatg gaaagaaagg tatattgatg aaaacgtaaa 4200

tacaggaaac cactacagat caatcagaaa ctatataacg gataaacctt ggttttctaa 4260

aatggagtcg acaaaagata tgataagaac catatgcaga atgagatttg accactgtct 4320

tactccatca tatttattta aaattaatat agctgatagt ccacaatgta tttgtggaca 4380

gttgggcaat ctacaacaca aaattttgac ctgttctctt atctctaata aagttaatat 4440

gtttttaaat tcactgtatt ctttgactga tgtccaccat cccattaatt taaattattt 4500

attaacatta gaaaataatg agttggtata ccgactattg tataaacata ttttagatat 4560

taagcttaaa ttgtaattgt aaaatataga gtaagtattt cttgtaaatt gttatatgtt 4620

aaaagaaaaa gaaaagaaaa gaaaaaaaaa gaaaagaaat ataaaaaaat aataaaataa 4680

aataaaaaaa aaaataataa ataaataaaa aaataataaa aaaagaaaga aaaaatataa 4740

aaaagatata taaaaaaaat agaaaaaaaa atataataat taaaaaaata taaaaaaaaa 4800

tatgtagata aaaatgaatg acgaacttgg acagtccata gtaaaatagc tttcgaatga 4860

agtagagggc taaagaagaa tgttgcagtt tttgctgtgg aactctgaga acatcatttg 4920

gaaaaaaatt aataaaatat atatataata aaaattatta aaaaaaatac aaaaataatt 4980

atttattagt agaattgttt attatattag tattagtttt aggataagat acgaaaaaat 5040

gactaataaa ataaaaaata gtaaaattgg cgaatggatt tagtgtccgc agtcatataa 5100

acccaaacaa acaacaaaag atcaacgaat accataaatc tgagtaatat taattgattt 5160

ttgaagaaat cgtgtcaaaa ttatttttat tatcaatcta atagtaaact taaagccagt 5220

agaagaacaa ccggttttta ctggacatta tttagaccag tcaaatggtt taggttctat 5280

atgagtagaa taacaagtta aaacaattat atattatcaa aaaattgaaa aaaggatcgg 5340

ctattttatg gtaaagggtt ttttggtcaa tttacttatt aaatgttaag gaggtaaaca 5400

ttttaaaatg gaataaattg ttaatcaatt taaaaaaaaa attctacgag ttttaaattt 5460

gcgtcaaaag gaggcttttg tagtggaaaa tatgtgataa aaatttcaaa actattcgtt 5520

aaatagtttt taaacaattc aatttgctta ttataacttt gcgaaaatga agaatctttt 5580

acaactaaaa atattttacc acatctcatt ttgaaggttt ttcaataaat caaccgattc 5640

ttttatcagt ttttttgata aactatataa caaacatcag cattttgcaa ggattgagac 5700

tattgatggg agcacatgtt atccacttat tttacttttt ataatacaat attacaataa 5760

atctaactta aaatataaaa tttgggatat ggcatttaaa aatattaatt tcattaccgc 5820

cgaatttaga tttagatgcg cttaaattca ttaataaaat ttagattaaa ccattacttg 5880

aactagcttt acaaacaaag agagcaacag aatttgacca aaaaatatat acacctgctt 5940

atgagatcag tcagtggcgt gaagcatgta cgtgatcgcg atcatgactg ggacagcgat 6000

cacataatag ggcttttcat tcacaatcat ttgtttcgag cttctgtcat gtgtcacata 6060

atattaatat acagggtgag tcatgaggac tcctacctcg tatagaggcc cctatgggga 6120

acaacaaacg accactaaaa agtgtctgct cccattgttc aacaatatac agggcgagtt 6180

cgcattttga cagaaattca tattcgtcac aattcctgaa cggtcagatc gatgtgtctc 6240

ccattctggc caatcgccac actactacca cccaatcaac taattcattc aaactagaaa 6300

aaaacaaggc ccgccccaaa aaaattagtt cgtttgggtc ccagaaaaaa cttcaccctg 6360

tatacactcc ttgaaaactc caatatgaat tttacaaatt agacaaatag gcaattaaaa 6420

tggcatattt atttttttcc ccacacgact actcaatttt tattaaaaaa tcaaatttgt 6480

caaaatcgga attttaacct aaaaataaaa aaaattaaat cagcgtttaa ctcgctacaa 6540

ctctgttcca ttttaatatt tttttctgaa atttttacag cacatacctc ttaccattgt 6600

gaagactatg aaaaattgtt gtagactttc agtcttcttc tcgtaaaagt tatgaatttt 6660

taaaaataaa aggtgcagat tcgtgaattg caaagttaaa tcgcaaaaat taagtgaaaa 6720

aatttaaaat ttatctattt gatcacgtct atgttaaact atagagtccc aaaagaagtg 6780

ttatgggaag ttttagatct agacatgtta tagaaaaaaa aaatggtaaa acttttaatt 6840

tgaggaaaaa tgttgttttt gtaaattaat ttttgtgaaa atcgttaatt tttttaaatc 6900

tatgcaaaaa ctttgccaaa ctttttatgc atagtcaaat ttgatttttt aactaaaaaa 6960

ctaagtaatc gtgtggggaa aaaataaata tgccatttca atcgcctacc tgtctccctg 7020

taaaattcat attagagccc tctaaaaaac gttattttta ctttttaagg tttttcgttt 7080

taaatttttt cctaagaccc aaacaaacta atttttcaaa tccgggcctg atttctctcc 7140

agtttgaaca aatcagccga ttgggtgata acacaaatta aatatttgct caaaaaaatt 7200

catttttgta ataaaagcta tttttttcaa tgcatggttg actttaccaa actttcaatt 7260

attcatagtc aaatttgatt tttttataaa aaatcaagta atcgtgtagg gaaaaaataa 7320

tatggaacag agttgtagcg agttaaaccg tgagttcatt tttttttcat ttttaggtta 7380

aaattccg 7388

<210>  9
<211>  445
<212>  DNA
<213>  Diabrotica virgifera


<220>
<221>  misc_feature
<222>  (1)..(445)
<223>  RNAi target sequence

<400>  9
gtttcgtttc atcatcattt gttgtaattt ttgtagtaaa gcacattaaa aaaaaaatct      60

gttttaaagc catggctgat gaagaatttg acgaaaatga tgtagcagat gatttcgatg     120

acgacgtaga ggatgataat atcgaagaac tcgaacaacc cgaggaagat ggagataaca     180

tcgatatcct tgctccagga caagcaggag gtggtgtacc aaaaaacaag aggataacaa     240

ctaaatatat gaccaaatat gagagagcca gagtattagg tactagagcc ttgcaaatag     300

ccatgtgtgc cccagttatg gttgaactag atggtgaaac tgatcctctg caaattgcca     360

tgaaggaatt aaaacagaga aagattccaa ttattattag aagatattta cctgaccatt     420

cctatgaaga ttggggaata gacga                                           445


<210>  10
<211>  20
<212>  DNA
<213>  Artificial Sequence

<220>
<223>  primer


<220>
<221>  misc_feature
<222>  (1)..(20)
<223>  forward primer

<400>  10
gtttcgtttc atcatcattt                                                  20


<210>  11
<211>  22
<212>  DNA
<213>  Artificial Sequence

<220>
<223>  primer


<220>
<221>  misc_feature
<222>  (1)..(22)
<223>  reverse primer

<400>  11
tcgtctattc cccaatcttc at                                               22

<210> 12
<211> 544
<212> DNA
<213> Diabrotica virgifera

<220>
<221> misc_feature
<222> (1)..(544)
<223> WCRW transcript ta02059.001_diavv

<400> 12
ggtttcgttt catcatcatt tgttgtaatt tttgtagtaa agcacattaa aaaaaaaatc 60

tgttttaaag ccatggctga tgaagaattt gacgaaaatg atgtagcaga tgatttcgat 120

gacgacgtag aggatgataa tatcgaagaa ctcgaacaac ccgaggaaga tggagataac 180

atcgatatcc ttgctccagg acaagcagga ggtggtgtac caaaaaacaa gaggataaca 240

actaaatata tgaccaaata tgagagagcc agagtattag gtactagagc cttgcaaata 300

gccatgtgtg ccccagttat ggttgaacta gatggtgaaa ctgatcctct gcaaattgcc 360

atgaaggaat taaaacagag aaagattcca attattatta gaagatattt acctgaccat 420

tcctatgaag attggggaat agacgagctc attattatag atcactagat tgtaattttt 480

atgtggatat tatttaatac ataggttttt ataacaaaaa aaaaaaaaaa aaaaaaaaaa 540

aaaa 544

<210> 13
<211> 563
<212> DNA
<213> Diabrotica virgifera

<220>
<221> misc_feature
<222> (1)..(563)
<223> RNAi target sequence

<400> 13
ttggtgattc tggtgtagga aaaagtaact tattgtcaag atttactaga aatgaattca 60

atttggagtc taaatctact ataggtgtcg aattcgctac cagaagtata caggtggatg 120

gtaaaactat taaagcacaa atatgggaca cggcaggtca agagagatac cgagcaatca 180

cggcggccta ttatcgtggt gcagtcggtg ctctactcgt ctacgatatc gccaaacatc 240

tgacatacga aaacgttgaa cgttggttgc gcgaattgcg cgaccacgca gatcagaaca 300

ttgtgatcat gttggtcgga aacaaatccg atctgaggca tttgcgcgcc gtgctgacag 360

aagaagctaa ggctttcgcc gaaagaaacg gcctcagctt catcgagact tcagccctag 420

attcgaccaa tgtcgataat gctttccaaa atatactcac agagatctat agaattgttt 480

cccaaaaaca gatcagagac cctccggaaa ataatatgat gccccaacac atgatgaacc 540

aaaaaatgat gcaaccagac atg 563

<210> 14
<211> 22
<212> DNA
<213> Artificial Sequence

<220>
<223> primer

<220>
<221> misc_feature
<222> (1)..(22)
<223> forward primer

<400> 14
ttggtgattc tggtgtagga aa 22

<210> 15
<211> 22
<212> DNA
<213> Artificial Sequence

<220>
<223> primer

<220>
<221> misc_feature
<222> (1)..(22)
<223> reverse primer

<400> 15
catgtctggt tgcatcattt tt 22

<210> 16
<211> 689
<212> DNA
<213> Diabrotica virgifera

<220>
<221> misc_feature
<222> (1)..(689)
<223> WCRW transcript ta47051.001_diavv

<400> 16
agttgtcctt attggtgatt ctggtgtagg aaaaagtaac ttattgtcaa gatttactag 60

aaatgaattc aatttggagt ctaaatctac tataggtgtc gaattcgcta ccagaagtat 120

acaggtggat ggtaaaacta ttaaagcaca aatatgggac acggcaggtc aagagagata 180

ccgagcaatc acggcggcct attatcgtgg tgcagtcggt gctctactcg tctacgatat 240

cgccaaacat ctgacatacg aaaacgttga acgttggttg cgcgaattgc gcgaccacgc 300

agatcagaac attgtgatca tgttggtcgg aaacaaatcc gatctgaggc atttgcgcgc 360

cgtgctgaca gaagaagcta aggctttcgc cgaaagaaac ggcctcagct tcatcgagac 420

ttcagcccta gattcgacca atgtcgataa tgctttccaa aatatactca cagagatcta 480

tagaattgtt tcccaaaaac agatcagaga ccctccggaa aataatatga tgccccaaca 540

catgatgaac caaaaaatga tgcaaccaga catgatgatg aataacgaaa tgatgaacga 600

aaaaatgatg atgccccaac acatgatgaa ccaaaaaatg atgcaaccag acatgatgat 660

gaataacgaa atgatgaacg aaaaaatga 689

<210> 17
<211> 204
<212> DNA
<213> Diabrotica virgifera

<220>
<221> misc_feature
<222> (1)..(204)
<223> RNAi target sequence

<400> 17
cccaatgccg agatatattg ataccgaaca aggcggatcc caagccagat ttttgttgtc 60

gaaagtaaat ccaagtcaaa ctcataacaa catgtattcc tacggaggtg attctggagc 120

tccagttttg acagatgatg tatcccttca agtattcatg gaccatctaa agaaattggc 180

agtttcgtcc acagcataat acct 204

<210> 18
<211> 20
<212> DNA
<213> Artificial Sequence

<220>
<223> primer

<220>
<221> misc_feature
<222> (1)..(20)
<223> forward primer

<400> 18
cccaatgccg agatatattg 20

<210> 19
<211> 20
<212> DNA
<213> Artificial Sequence

<220>
<223> primer

<220>
<221>  misc_feature
<222>  (1)..(20)
<223>  reverse primer

<400>  19
aggtattatg ctgtggacga                                                   20


<210>  20
<211>  2636
<212>  DNA
<213>  Diabrotica virgifera


<220>
<221>  misc_feature
<222>  (1)..(2636)
<223>  WCRW transcript ta01733.001_diavv

<400>  20
gcacgaggca gacacgacat cgaatgacgt ggaagttcaa attctgaaac aattaggtgt      60

aatttttagg agctcaataa tagtgttatt tacatgatag aatcctaata atatatattg     120

aggaatttcc ttagggaatt ccgacttgta atcttcaaaa atgagcacat atgaagagta     180

tatacaacaa aatgaagatc gagatgggat tagatttacc tggaatgtat ggccttcaag     240

cagaattgaa gctacccgtc tcgtagtacc cttagcttgt ctgtaccagc ctataaagga     300

acgtctggat cttccaccaa tacaatatga ccctgtttta tgtactagaa atacttgtag     360

agcaatatta aacccactgt gtcaggtaga ttatcgagca aaactctggg tatgcaactt     420

ttgtttccag agaaatccat ttccacctca atatgctgct atttcagaac aacatcaacc     480

agcggaattg atgcctatgt tttccaccat tgaatacaca ataactagag ctcaatgttt     540

accaccaata tttttgtatg ttgttgacac ctgcatggat gatgaagaac tgggttccct     600

gaaagactca ttgcaaatgt cccttagttt gttgccacct aatgcgttaa taggactaat     660

aacatttggg aaaatggttc aagttcatga acttggcact gaaggttgta gtaagtcata     720

tgtgttcaga ggtacaaaag atcttagtgc taaacaggtt caagaaatgc tgggaatagg     780

caaagtggct ttaggtcagc aagcccctca acagccaggg cagcctctaa gacctgggca     840

aatgcaacct actgttgttg caccaggaag caggtttcta caacctgtat ccaaatgcga     900

tatgaatcta acagacctaa taggagaaca acagaaagat ccttggcctg ttcatcaggg     960

taaaaggtat ttaagatcta caggtgtagc tttatcgatt gccattggtt tgttagaatg    1020

tacatattcc aatactggcg cccgagttat gctatttgtt ggaggacctt gctcacaagg    1080

acctggtcag gtagttaatg atgatttaaa acagcctatt agatcacatc atgatattca    1140

gaaagataat gcaaaatata tgaagaaagg tattaaacat tatgatgcgt tagcaatgag    1200

agccgcaact aatggtcact ctgttgatat ttattcttgt gctttggatc agacaggtct    1260

gatggaaatg aagcaatgct gtaattctac tgggggacac atggtaatgg gggattcatt 1320

taattcttcc ttgtttaagc aaactttcca acgtgtgttt accagagatc aaaaaagtga 1380

tctgaaaatg gcatttaacg gtactttgga agtgaagtgt tcccgagaat taaaagttca 1440

aggaggtatc ggttcgtgtg tatcacttaa cgtgaagagc cccttggttt ccgacacaga 1500

aataggaatg ggtaatactg tgcaatggaa aatgtgtact ttaacgccaa gtactaccat 1560

gtctttattc tttgaggtcg taaatcaaca ttctgctccc atacctcaag gtggtagagg 1620

ttgtatacaa tttattacgc agtaccagca ttcaagtggt caaagaaaaa tcagagtaac 1680

aacagtggct cgaaattggg ctgacgcaac tgctaatata caccatatca gtgccggatt 1740

cgatcaagaa gctgctgctg taataatggc taggatggcc gtttataggg cagaatctga 1800

tgatagtcca gatgttctta gatgggttga cagaatgctg attagattgt gtcaaaaatt 1860

cggagaatac aataaggacg accccaattc attcagactt ggtcaaaact tcagtcttta 1920

cccacagttc atgtatcact taagaagatc tcaatttctt caagtattca ataattctcc 1980

ggacgagact tcattctaca gacacatgtt gatgagggaa gatcttactc aatctttgat 2040

aatgattcaa cctattttgt atagttatag tttcaatggt ccaccagagc ctgtattact 2100

agatactagc tccattcaac ctgacagaat attacttatg gatactttct tccaaatatt 2160

aattttccat ggagagacta tcgcccaatg gcgtagttta aaatatcaag acatgccaga 2220

atatgaaaac tttagacagc tactacaggc tccagtagat gatgcacaag aaattttgca 2280

aactaggttc ccaatgccga gatatattga taccgaacaa ggcggatccc aagccagatt 2340

tttgttgtcg aaagtaaatc caagtcaaac tcataacaac atgtattcct acggaggtga 2400

ttctggagct ccagttttga cagatgatgt atcccttcaa gtattcatgg accatctaaa 2460

gaaattggca gtttcgtcca cagcataata cctatatatt acaattagat acatttgaca 2520

taatacagtt tttgaattta ttcaatatat tatattttaa gcttaatttt tttgtatatt 2580

tatttcatag atagtttata tatttggtaa tgtgatacaa taaatttttg ttttcc 2636

<210> 21
<211> 599
<212> DNA
<213> Diabrotica virgifera

<220>
<221> misc_feature
<222> (1)..(599)
<223> RNAi target sequence

<400> 21
acaaatgtgg tggtatcgac aaacgtacca tcgaaaagtt cgaaaaagaa gcccaagaaa 60

tgggtaaagg ttcattcaaa tatgcctggg tactcgacaa acttaaggcc gagagagaac 120

gtggtattac cattgatatt gctttgtgga aattcgaaac agctaaatac tatgtaacca 180

tcattgatgc ccctggacac agagatttca ttaagaacat gatcactggt acatcacaag 240

ctgactgtgc tgtactcatt gttgcagctg gtactggtga atttgaagca ggtatttcaa 300

agaatggaca aacacgtgaa catgctcttc ttgctttcac ccttggtgta aaacaactta 360

ttgttggtgt caacaaaatg gactcgactg aaccagcata cagtgaatca cgtttcgagg 420

aaatcaagaa ggaagtatcc tcatacatca agaaaattgg ttacaaccca gctgccgttg 480

ctttcgtacc aatttcagga tggcacggag acaacatgtt agaaggatct gacaagatgc 540

catggttcaa gggatggcaa atcgaacgta aagaaggaaa agctgaagga aagtgcttg 599

<210> 22
<211> 22
<212> DNA
<213> Artificial Sequence

<220>
<223> primer

<220>
<221> misc_feature
<222> (1)..(22)
<223> forward primer

<400> 22
acaaatgtgg tggtatcgac aa 22

<210> 23
<211> 22
<212> DNA
<213> Artificial Sequence

<220>
<223> primer

<220>
<221> misc_feature
<222> (1)..(22)
<223> reverse primer

<400> 23
caagcacttt ccttcagctt tt 22

<210> 24
<211> 1698
<212> DNA
<213> Diabrotica virgifera

<220>
<221> misc_feature
<222> (1)..(1698)
<223> WCRW transcript ta04714.004_diavv

<400>  24
gggatcttcc tttttaattt gctcgtcgta cggtgtggac ctcgcgcctg gtgttacatc      60

ccaagtagtg ttccttttat tctaagttta atttcgaaca gttgcattta ctttatttcc     120

aaacaatcaa aatgggtaaa gaaaagattc atattaacat cgttgtcatt ggacacgtag     180

attctggtaa atctactact actggacatt taatttacaa atgtggtggt atcgacaaac     240

gtaccatcga aaagttcgaa aaagaagccc aagaaatggg taaaggttca ttcaaatatg     300

cctgggtact cgacaaactt aaggccgaga gagaacgtgg tattaccatt gatattgctt     360

tgtggaaatt cgaaacagct aaatactatg taaccatcat tgatgcccct ggacacagag     420

atttcattaa gaacatgatc actggtacat cacaagctga ctgtgctgta ctcattgttg     480

cagctggtac tggtgaattt gaagcaggta tttcaaagaa tggacaaaca cgtgaacatg     540

ctcttcttgc tttcaccctt ggtgtaaaac aacttattgt tggtgtcaac aaaatggact     600

cgactgaacc agcatacagt gaatcacgtt tcgaggaaat caagaaggaa gtatcctcat     660

acatcaagaa aattggttac aacccagctg ccgttgcttt cgtaccaatt tcaggatggc     720

acggagacaa catgttagaa ggatctgaca agatgccatg gttcaaggga tggcaaatcg     780

aacgtaaaga aggaaaagct gaaggaaagt gcttgattga ggctttggat gctatccttc     840

ccccatctcg tccaactgag aaacccctcc gtcttccact ccaggatgtc tacaaaattg     900

gtggtattgg aacagtacct gtaggtcgtg ttgaaactgg tgtattgaaa cctggtatgg     960

ttgtagtatt cgctccagcc aatttgacga ctgaagtaaa atccgttgaa atgcatcacg    1020

aagccctcca ggaagcagta cctggtgaca atgttggttt caacgtcaag aatgtctctg    1080

ttaaagaatt gcgtcgtggt tacgtagctg gagacaccaa gaacaaccca cccagaggag    1140

cttcagactt cactgcccaa gtcattgttc tcaaccaccc tggtcaaatc tcaaacggat    1200

atacccctgt actcgactgt cacacagctc acattgcctg caaattcgct gaaatcaaag    1260

aaaaggttga ccgtcgttct ggtaagacca ctgaagaaaa tcccaaagcc atcaaatctg    1320

gtgatgccgc cattgtcaac ttggtaccca ccaagcccat gtgtgtagaa tcattccaag    1380

aattcccacc ccttggacgt tttgctgtcc gtgacatgag gcaaaccgtt gcagtaggag    1440

tcatcaagag cgttgccttc aaagatccca gcgccggaaa agtcacaaaa gctgcagaaa    1500

aagcccaaaa gaagaaatag tctatttgga ctccactact taaataattt tgatatgttt    1560

tgtttctttt atatattaat attggagtat caatgtattt ttaataacta atggacttat    1620

tacttgcatt gcattatttg tgatctgtaa attaaacttg gacaaataaa gaaaaaaatt    1680

aaaaaaaaaa aaaaaaaa                                                  1698

<210>  25
<211>  550

<212>  DNA
<213>  Diabrotica virgifera

<220>
<221>  misc_feature
<222>  (1)..(550)
<223>  RNAi target sequence

<400>  25
ccagggaaag agagaagtac gataatttag cagatttgta tgctgtaata aacactttgc      60

aacaattaga gaaggcatat attcgagatt gtgtagtggc aaaggaatat acaggagctt     120

gtagtaaatt attagttcag tataaggctg cgtttcgaca agtcaagagt gatgaatttc     180

ctacaccaga cgcctttgtt aagaagtaca ggcttgactg tcctgcagct ttagaacgta     240

tcaaggaaga taggccaatc actatcaaag acgacaaagg caacactagt cgatgcattg     300

ctgatatcgt atcgctattc atcactataa tggacaaact gcggttggac attcgttcga     360

tggataactt acatcctgac gtgagggact tagtagatac tatgaacaga cttagcattc     420

tgccatcgga tttcgatgga aaggtaaaag tggctgaatg gttaaatact ttaaatgaaa     480

tgcaggcctc tgatgaatta tccgaagctc aagtgaggca actgttgttt gatcttgaaa     540

gtgcgtatgc                                                            550

<210>  26
<211>  22
<212>  DNA
<213>  Artificial Sequence

<220>
<223>  primer

<220>
<221>  misc_feature
<222>  (1)..(22)
<223>  forward primer

<400>  26
ccagggaaag agagaagtac ga                                               22

<210>  27
<211>  22
<212>  DNA
<213>  Artificial Sequence

<220>
<223>  primer

<220>
<221>  misc_feature
<222>  (1)..(22)
<223>  reverse primer

<400>  27
gcatacgcac tttcaagatc aa                                                22

<210>  28
<211>  769
<212>  DNA
<213>  Diabrotica virgifera

<220>
<221>  misc_feature
<222>  (1)..(769)
<223>  WCRW transcript ta01620.001_diavv

<400>  28
atataaatag aaaaatgtca gcagaaaatc gccctgagct gtatgaggaa gtcaagctat       60

atcacaatgc cagggaaaga gagaagtacg ataatttagc agatttgtat gctgtaataa      120

acactttgca acaattagag aaggcatata ttcgagattg tgtagtggca aaggaatata      180

caggagcttg tagtaaatta ttagttcagt ataaggctgc gtttcgacaa gtcaagagtg      240

atgaatttcc tacaccagac gcctttgtta agaagtacag gcttgactgt cctgcagctt      300

tagaacgtat caaggaagat aggccaatca ctatcaaaga cgacaaaggc aacactagtc      360

gatgcattgc tgatatcgta tcgctattca tcactataat ggacaaactg cggttggaca      420

ttcgttcgat ggataactta catcctgacg tgagggactt agtagatact atgaacagac      480

ttagcattct gccatcggat ttcgatggaa aggtaaaagt ggctgaatgg ttaaatactt      540

taaatgaaat gcaggcctct gatgaattat ccgaagctca agtgaggcaa ctgttgtttg      600

atcttgaaag tgcgtatgcg tcctttaata aagtccttca taattctgcc taaatgtacg      660

agatagttgt tatgattgtt acacgtttat gtaaatatta taagcaaaaa aaattgagct      720

agatagttat tggcaatgta tgcatttatc aattttaacc ctaattgga                  769

<210>  29
<211>  578
<212>  DNA
<213>  Diabrotica virgifera

<220>
<221>  misc_feature
<222>  (1)..(578)
<223>  RNAi target sequence

<400>  29
aatccaaaag ctgaagtggc tagagctgca caagccctag ccgttaatat atcagcggca       60

aaaggcattc aagatgtaat gaagtcaaac cttggtccca aaggaacagt gaaaatgttg      120

gtatctggtg ccggcgatat aaaaataacc aaagatggaa acgttctatt gcacgaaatg      180

caaattcaac accccacggc ttcccttatt gctcgagcct caactgccca agatgatatg      240

accggcgacg gcactacatc gactgtgtta gtcataggtg aactattaaa acaagcagat 300

atttacatag cagaaggtct ccaccctaga ttgttaacag atggctttga taaggctagg 360

acaaaagcgt tggaagtact ggagaaaatg aagatttcaa ttgaagcgaa caaagaaact 420

ttaacagata tttgcaggac gagtttaaga acaaagctgc atcaaaaact ggctgatgtt 480

ttgacagacg tttgtgtgga tgctgttcta gctataaaga aagatggcaa acctgttgat 540

cttcatatgg ttgaattaat ggaaatgcag cacaaaac 578

<210> 30
<211> 20
<212> DNA
<213> Artificial Sequence

<220>
<223> primer

<220>
<221> misc_feature
<222> (1)..(20)
<223> forward primer

<400> 30
aatccaaaag ctgaagtggc 20

<210> 31
<211> 20
<212> DNA
<213> Artificial Sequence

<220>
<223> primer

<220>
<221> misc_feature
<222> (1)..(20)
<223> reverse primer

<400> 31
gttttgtgct gcatttccat 20

<210> 32
<211> 1864
<212> DNA
<213> Diabrotica virgifera

<220>
<221> misc_feature
<222> (1)..(1864)
<223> WCRW transcript ta00896.001_diavv

<400> 32
gggggtcaaa aattttgggt tggctcaaca cgagttttct attttatttg aaaaaatagt 60

attttaaata atggctatca gtttgttaaa tccaaaagct gaagtggcta gagctgcaca 120

agccctagcc gttaatatat cagcggcaaa aggcattcaa gatgtaatga agtcaaacct 180

tggtcccaaa ggaacagtga aaatgttggt atctggtgcc ggcgatataa aaataaccaa 240

agatggaaac gttctattgc acgaaatgca aattcaacac cccacggctt cccttattgc 300

tcgagcctca actgcccaag atgatatgac cggcgacggc actacatcga ctgtgttagt 360

cataggtgaa ctattaaaac aagcagatat ttacatagca gaaggtctcc accctagatt 420

gttaacagat ggctttgata aggctaggac aaaagcgttg gaagtactgg agaaaatgaa 480

gatttcaatt gaagcgaaca aagaaacttt aacagatatt tgcaggacga gtttaagaac 540

aaagctgcat caaaaactgg ctgatgtttt gacagacgtt tgtgtggatg ctgttctagc 600

tataaagaaa gatggcaaac ctgttgatct tcatatggtt gaattaatgg aaatgcagca 660

caaaacagaa acagatactc agttaattaa gggtttagtc ctggaccatg gctcccgtca 720

tccagatatg cctaaaagtc tcaacaactg ctacatttta acatgtaatg tcagcatgga 780

atatgaaaaa agtgaagtca attcaggatt cttctacaaa actgcagaag agagagaaaa 840

attagtgact gcagagcgtg aattcattga gcagagagtt aagaaagtca ttgaactgaa 900

aaggtcactc tgtaatggaa ctgataagac ttttgttgtt attaaccaaa aaggtattga 960

ccccatgagc ctggatatgc ttgccaaaga aggaataata gctctcagaa gagctaagag 1020

acgtaacatg gagcgtctat cattggcttg tggtggagta gctatgaaca gctttgatga 1080

tctgcaagaa tctcaacttg gtagagccgg acatgtatat gaacatgtgt tgggagaaaa 1140

taagtataca tttgtggaag attgcaaaat tcctcaatca gttactattt taatgaaagg 1200

gccaaataaa catactttga ctcagattaa agatgcagtt agagatggat taagggctat 1260

caataatgcc attgacgata aagcagttat tccaggtgct ggtgcatttg aagtcacagc 1320

caataaagaa ttgctagcat ttaaagacac tgttaagggt aaagctcgtc taggagttgc 1380

cgcttatgca gaagctttac tggtagttcc caagactttg gctgttaact ctggatttga 1440

tgcacaggac accattctaa aactacaaga agaatcccgt ttgaatccag acccaatcgg 1500

tttagatcta acctctgggg aacccatcaa tcctaaagat gctggcatct atgacaacta 1560

tattgttaaa aaacaaataa tcaattcctg ttcggtcatt gccagcaacc tcttattggt 1620

tgatgaaatt atgagggctg gaatgagcag cttaaaggga taaaatcgaa gtattcaaat 1680

ttaaatctgt atttttgtgc ttgttttaat ttatcattac attatgtatt ttcttaacct 1740

ctgaaaggtt gctacttaat atttagtgga catacacata atgctgtaac gttgataaca 1800

tacacacgta acatttataa tatttgcaat aataaaatgt caaaaaaaaa aaaaaaaaat 1860

gcga 1864

<210>  33
<211>  709
<212>  DNA
<213>  Diabrotica virgifera

<220>
<221>  misc_feature
<222>  (1)..(709)
<223>  RNAi target sequence

<400>  33
gaaaatgcca accaaagtct tcgagaagag ctgtccttta aggatcagat ccatcaacaa      60

cagttgacag aaactcgtac tcgtcgtcaa gtagaaatca gcgaaatcga cggtcgcctg     120

gcagaaaaat atgaggctaa gcttcaagag gctctacaag aactcagaga tcaatatgaa     180

agtcaaatgg ccaacaaccg tggcgaaatc gagttgcttt acgaacagaa gattaagaac     240

ttgcaatcag ctaatgacag acatgctgcc tctgcctccg gtgctcttga cgaattgaga     300

caagttagaa ccagaatcga tactttgaat tctcgaatta gcgatttaga gaaccagaac     360

gctggcttag cagctcgtgc tagagatttg gagaagttgt tagagaatga gcgcatgcgc     420

catgtggaag acctcgcaat gttagaaaga gaattgcata gactccgaga agaaatggct     480

aatcaacttc aagaatacca ggatcttatg gacattaagg tttctcttga tttagagata     540

gctgcttatc ggaaactgct cgaatctgaa gaagctcgtc ttaatattac cccaacaggt     600

agtgaagttc atgaagtaac tcacagtagg agccaacgca gctcgtcgca acgtcgcact     660

ccagtcagat tgggagccaa aagaaagcga acactactcg aagaaagcc                 709

<210>  34
<211>  22
<212>  DNA
<213>  Artificial Sequence

<220>
<223>  primer

<220>
<221>  misc_feature
<222>  (1)..(22)
<223>  forward primer

<400>  34
gaaaatgcca accaaagtct tc                                               22

<210>  35
<211>  22
<212>  DNA
<213>  Artificial Sequence

<220>
<223>  primer

<220>
<221>  misc_feature
<222>  (1)..(22)
<223>  reverse primer

<400>  35
ggctttcttc gagtagtgtt cg                                                22


<210>  36
<211>  2662
<212>  DNA
<213>  Diabrotica virgifera


<220>
<221>  misc_feature
<222>  (1)..(2662)
<223>  WCRW transcript ta03354.001_diavv

<400>  36
aaaaaccgtt aaacgccgct cccatcatat catctccaaa aatagtttga ttacctcggg      60

tagaccgcga cataatttgt gcttcgaccg atctctctct ttgagttgtc gcttttcgag     120

gtatttagtt gcaggcgtgg tgtatacgtt gaaatagtga tttttgttaa aagtgagtaa     180

atatggcatc gaaatcaaaa aaagggtcaa caccgttagc ttcacagcgg ccggcgagcc     240

ccttaagtcc taccagacat tctagattac aggagaagca tgaacttcag aatcttaacg     300

accgtttggc gggatatata gatcgagtaa ggtatttaga aggtgaaaac aacagattaa     360

cgagggaaat acaaagtacc caggaaacag taacaaggga agtttcaaac attaaatcta     420

tgtatgacca tgaattatca gatgctagaa aactattaga cgagacccac cgtgaaaaag     480

cacgtttaga gatagatgtt aaacgtcttt tagaagagaa tgacgaactc aagtctgagt     540

tagcaaagaa gaacaaggat ttggctctag ctgagaattc tgccaggata tacgagacca     600

gatgtaacga gttacagctc aaagcaaatc aagcggctgc tgatgctaag aaagctattg     660

cagatgctaa ggaacttgag aaagaacgtg ataagctacg caaacttctt gataatcatc     720

gtaaacagtt agaagaagaa tcattagcga gagtggaagt cgaaaatgcc aaccaaagtc     780

ttcgagaaga gctgtccttt aaggatcaga tccatcaaca acagttgaca gaaactcgta     840

ctcgtcgtca agtagaaatc agcgaaatcg acggtcgcct ggcagaaaaa tatgaggcta     900

agcttcaaga ggctctacaa gaactcagag atcaatatga aagtcaaatg gccaacaacc     960

gtggcgaaat cgagttgctt tacgaacaga agattaagaa cttgcaatca gctaatgaca    1020

gacatgctgc ctctgcctcc ggtgctcttg acgaattgag acaagttaga accagaatcg    1080

atactttgaa ttctcgaatt agcgatttag agaaccagaa cgctggctta gcagctcgtg    1140

ctagagattt ggagaagttg ttagagaatg agcgcatgcg ccatgtggaa gacctcgcaa    1200

tgttagaaag agaattgcat agactccgag aagaaatggc taatcaactt caagaatacc    1260

aggatcttat ggacattaag gtttctcttg atttagagat agctgcttat cggaaactgc 1320

tcgaatctga agaagctcgt cttaatatta ccccaacagg tagtgaagtt catgaagtaa 1380

ctcacagtag gagccaacgc agctcgtcgc aacgtcgcac tccagtcaga ttgggagcca 1440

aaagaaagcg aacactactc gaagaaagcc aagagtcaag catgaatgac tatacggtga 1500

acagctcctc aaaaggtgac attgaagtcg ctgatgtcga tccagaagga cactatgtta 1560

agttgcacaa taaaagtaat caagaagtcg cacttggagg atggcaagtc attagacgtg 1620

ttggtgagga ggaaactaca ttcaaatttc acaggtcact aaagcttgaa ggtaatggat 1680

ttgtaacaat atggagctct gacctcaaca aagatcatga acctccaact aatttagtta 1740

tgaagagtca gaaatgggtt gtaggtgata acatggtcac cacattattg aataataacg 1800

gagatgaagt agctgtctcc gaaagagtta aaaggcaatt aacaagctcc catctaagac 1860

acagagaact agcagctggc tacctctcag aagaactcca ccatcaacag ggtgatccac 1920

aagtagatga aagatgtagg cttatgtaaa gttaacacat ctcatttaat ttttttattt 1980

acagttaagg ttggaatgtt acagaagtag gttgtgctcc ttacttacgc tttttaaact 2040

attttttaag tgatgttctt aatattataa gattctagga cagaaggaac acaacttata 2100

ctgttattga caaaaaactt actgaaaatt gcaattagac aacagtagta agtttaatac 2160

taagaataca ggatgaggta ataattatta ttctttatat aaactgtctt tttaggagtt 2220

ttatatatat tttttaaata tcttgcatat aagtcttgaa ttacagttgt catttttata 2280

aatttaaacc tgaaaaatac attatttcgc attaatacct catcctgttc gatagtttct 2340

taagatagat aacaaactgc cagttcgata agttttcttt taactaacac aattgtttat 2400

tagtataata taattttgaa aaattactgt actgttgtcc ccaaaacatt aaagtgttct 2460

gcatttattt aaagaataat aaacttttta tattaatggt agttcttttg tttttgaggt 2520

ttttcactca gctgtttacg cttgttttag ttagataggt gttgcaaaaa ctatagggat 2580

aaaaatctag atttggtttc agttacttgt attatatttt attgtatatc tttattcttg 2640

atcttcgaaa cttaaatctg ta 2662

<210> 37
<211> 635
<212> DNA
<213> Diabrotica virgifera

<220>
<221> misc_feature
<222> (1)..(635)
<223> RNAi target sequence

<400> 37
aatggcttta cacgtcccta aagcaccggg ctttgcccag atgttaaaag aaggagccag 60

gcactattct ggtttggaag aagctgttat aagaaacata atagcatgca aggaatttgc     120

acaatccgta aaaactgcct atggtccaaa tggtatgaat aaaatggtca ttaatcacat     180

tgagaaacaa tttgtcacaa gtgatgcagc tacaattatg cgagaactag atattgaaca     240

tccagcagcc aaactcatga tactaggaag tcaaatgcaa gattctgagg tgggtgatgg     300

aactaacttt gtgattatac ttgctggagc acttttggag catgctgaag aactaattcg     360

tcttggagta actcccacag aaatagcaga aggttatgag aaagcactag acaaatgtct     420

agaaatcctt cctaatctta tttgctacac aatcaaggat ttcagaaaca tcgaagaagt     480

caaaaagggc atacaaacct caattcaatc taagcagtat ggcaatgaag agtttctatc     540

aaaacttatt gcagaagctt gtgtgtcaat tttgccagaa gaaacaacat ttaatgttga     600

taatgttaga atatgtaaag ttttggggtc tggtt                                635

<210>  38
<211>  22
<212>  DNA
<213>  Artificial Sequence

<220>
<223>  primer


<220>
<221>  misc_feature
<222>  (1)..(22)
<223>  forward primer

<400>  38
aatggcttta cacgtcccta aa                                               22


<210>  39
<211>  22
<212>  DNA
<213>  Artificial Sequence

<220>
<223>  primer


<220>
<221>  misc_feature
<222>  (1)..(22)
<223>  reverse primer

<400>  39
aaccagaccc caaaacttta ca                                               22


<210>  40
<211>  1866
<212>  DNA
<213>  Diabrotica virgifera

<220>
<221>  misc_feature
<222>  (1)..(1866)
<223>  WCRW transcript ta04788.001_diavv

<400>  40
ggaactctat ggtcttcagt cactctaaat aaaatcctat aacctttttt gggatttcaa      60

ttcgaattaa ataaatttct tcagaaacat acaagaaaca aatacagaca atggctttac     120

acgtccctaa agcaccgggc tttgcccaga tgttaaaaga aggagccagg cactattctg     180

gtttggaaga agctgttata agaaacataa tagcatgcaa ggaatttgca caatccgtaa     240

aaactgccta tggtccaaat ggtatgaata aaatggtcat taatcacatt gagaaacaat     300

ttgtcacaag tgatgcagct acaattatgc gagaactaga tatagaacac ccagcagcca     360

aactcatgat actaggaagt caaatgcaag attctgaggt gggtgatgga actaactttg     420

tgattatact tgctggagca cttttggagc atgctgaaga actaattcgt cttggagtaa     480

ctcccacaga aatagcagaa ggttatgaga aagcactaga caaatgtcta gaaatccttc     540

ctaatcttat ttgctacaca atcaaggatt tcagaaacat cgaagaagtc aaaaagggca     600

tacaaacctc aattcaatct aagcagtatg gcaatgaaga gtttctatca aaacttattg     660

cagaagcttg tgtgtcaatt ttgccagaag aaacaacatt taatgttgat aatgttagaa     720

tatgtaaagt tttggggtct ggtttacata actctcaggt tgtacaaggt atggttttca     780

aaagacaagt tgaaggggaa ataactaaag cagaaaaagc taaaattgct gtattcagct     840

gtgctgttga tattatgcaa acagaaacta aaggaactgt acttatcaaa actgcagatg     900

aacttatgaa ctttagtagg ggagaagaaa atcttcttga attacaaatc aaatgtatag     960

cagatgctgg agctaaagtt gtagttgcag gagccaaatt tggagatatg gctctacatt    1020

acttacataa atatggcatg atggttgtca ggttgaactc caaatttgat ttgagaagat    1080

tatgcaaaac tgttggtgct acttgtcttc cacgcctaac agctcccaca tctcaagaac    1140

ttggttacgc agatattgta tgtgtagaag aattgggtga tacccctata gtttctttca    1200

gattggaggg caaagaatca agaatttcca caattgtcat cagaggtgct actgacaatt    1260

acatggatga cattgaaaga gcaattgatg atggagttaa tacatttaag ggaatctctc    1320

gagatggcag atttgttcct ggtgccggcg ccgttgaagc agagttagct gttcagctga    1380

ctaaatatgc agacactctt ccaggcttag aacagtatgc tgtcagaaaa ttcgcatctg    1440

cccttgaaag ttttcccaaa acgttggcag ataatagtgg acacaaatcc acagcagtat    1500

tagaaaaaat attggaagct caccagaatg gtcagaaaaa tgttggagtt gacatagaag    1560

ctgaaagtgc agtatgtgac gctttggaaa agaacatctt ggacctttat caatgtaaat    1620

attggggact gaagtatgct gtaggagcag cagcaaccat tttaagagtc gaccaaatta    1680

ttatggcgaa aagggcaggt gggccaaaag ttaggcaacc aaagggaagt gatgacgaat 1740

cttgaaatcg ttaattttgc gcttggtttc tcgtatacac ttaaattcac cttacaactc 1800

acttgtatta atctgttaat ttaatttttt aattaatatt tcttaaccaa aaaaaaaaaa 1860

aaaaaa 1866

<210> 41
<211> 579
<212> DNA
<213> Diabrotica virgifera

<220>
<221> misc_feature
<222> (1)..(579)
<223> RNAi target sequence

<400> 41
ccagagactt catatcccaa ccaagactca cttacaaaac tgtatctggt gtcaacggtc 60

cactagtaat cctcgaagat gtcaaatttc ctaagttcaa cgaaattgtc caactcagat 120

tatctgacgg aagtttaaga tcaggtcaag tacttgaagt cagcggttcc aaggctgtag 180

tacaagtatt cgaaggtaca tcaggtatcg atgccaaaca cactgtctgt gagttcacag 240

gagatattct cagaactcca gtctctgagg atatgttggg aagagtattc aacggctctg 300

gaaaacctat tgacaaaggt cccccaattc tggctgaaga ctacctagat attcaaggac 360

aacccatcaa cccatggtcc cgtatctacc cagaggaaat gatccaaact ggtatcactg 420

ccatcgatgt gatgaactcg attgcccgtg gtcagaagat tcccattttc tcagctgctg 480

gtctgccaca taacgatatt gctgcccaaa tttgtaggca agccggtctt gtcaaaatcc 540

caggcaaatc agttttggat gaccacgaag acaactttg 579

<210> 42
<211> 22
<212> DNA
<213> Artificial Sequence

<220>
<223> primer

<220>
<221> misc_feature
<222> (1)..(22)
<223> forward primer

<400> 42
ccagagactt catatcccaa cc 22

<210> 43
<211> 22
<212> DNA

<213>  Artificial Sequence

<220>
<223>  primer


<220>
<221>  misc_feature
<222>  (1)..(22)
<223>  reverse primer

<400>  43
caaagttgtc ttcgtggtca tc                                                22


<210>  44
<211>  2471
<212>  DNA
<213>  Diabrotica virgifera


<220>
<221>  misc_feature
<222>  (1)..(2471)
<223>  WCRW transcript ta00620.009_diavv

<400>  44
tcttaaactt ccgtcagata atctgagttg ctaatttttt actgtctttg atagacgtct      60

cctgtacacg taaaaatggc ttcgacgata tcggcaagac aggctaacag agaacatgtt     120

ttggccgtat ccagagactt catatcccaa ccaagactca cttacaaaac tgtatctggt     180

gtcaacggtc cactagtaat cctcgaagat gtcaaatttc ctaagttcaa cgaaattgtc     240

caactcagat tatctgacgg aagtttaaga tcaggtcaag tacttgaagt cagcggttcc     300

aaggctgtag tacaagtatt cgaaggtaca tcaggtatcg atgccaaaca cactgtctgt     360

gagttcacag gagatattct cagaactcca gtctctgagg atatgttggg aagagtattc     420

aacggctctg gaaaacctat tgacaaaggt cccccaattc tggctgaaga ctacctagat     480

attcaaggac aacccatcaa cccatggtcc cgtatctacc cagaggaaat gatccaaact     540

ggtatcactg ccatcgatgt gatgaactcg attgcccgtg gtcagaagat tcccattttc     600

tcagctgctg gtctgccaca taacgatatt gctgcccaaa tttgtaggca agccggtctt     660

gtcaaaatcc caggcaaatc agttttggat gaccacgaag acaactttgc catcgtgttc     720

gctgctatgg gtgttaacat ggagactgct agattcttca aacaagattt cgaagaaaat     780

ggttctatgg aaaacgtatg tttgttcttg aacttggcta acgatccaac catcgaaaga     840

atcatcaccc cacgtcttgc tctaacagcc gctgaattct tggcttacca gtgcgaaaaa     900

cacgtgttag tcattttaac tgacatgtct tcatacgctg aagctttgcg tgaggtatct     960

gccgccagag aggaagtacc cggtcgtcgt ggtttccccg gttacatgta caccgatttg    1020

gccaccatct acgaacgtgc cggtcgtgtc gaaggccgta acggttccat cactcaaatc    1080

cccatcttga ctatgcctaa cgacgatatt actcacccta ttcccgattt gacaggttat 1140

attaccgaag gacagatcta cgtcgaccgt caattgcaca acaggcagat ctacccacca 1200

attaacgtac tgccatctct ctcacgtttg atgaagtctg ctatcggcga aggtatgacc 1260

aggaaggacc actctgacgt ttctaaccag ttgtatgctt gctatgctat cggtaaggac 1320

gtccaggcca tgaaagccgt agtaggtgaa gaagctctaa cacccgacga tcttctctac 1380

ctcgagttcc taacgaaatt cgaaaagaac ttcatcaccc aaggaaacta cgaaaaccgc 1440

accgtcttcg agtctttgga catcggctgg caactactcc gtatcttccc taaggaaatg 1500

ttgaaacgta tccctgcaag cacattggcg gaattctatc caagagactc tcgtcactag 1560

ttggtttatg tataaaattt atatatattt aggaagtttg ttaggtttgt tacaaatatg 1620

tccacttatt gtatcgatta ttttcaattg agagatattt taccaaaaca atttgagccg 1680

atgagattct acggtgtaat ttttgtaaat acttaaagag cgtgtttcaa aaatgcgctt 1740

aatataatat attatcaata cagagcaacc tttttttctt tattgttttt ttcactccaa 1800

agttcttgaa ctcctaaaag gagcatagag cttcagtgaa aaggcgccat cccctcattc 1860

caagactttc cttgacctct taccaattgt tggaaaaatt ataactcaga ttaattttat 1920

taggtgacag ttccataagt aaaaatatgc aatggctata tattgcgtac taaacttaag 1980

aagaaaaatt gtgtcgctct ggaagaaaga ttacttgcat aagaatgaaa atcagtgtca 2040

tattttgcgt ttatgacatc cctagacgat actatttttt cgttactata ttttttttaa 2100

agagatcaag agcaacaatt tggagactga tattcttatg cgagttgttt tcctactttg 2160

tcctattttg aagagtctac aattatttac ttaccatttg attaaattag atgaacataa 2220

gacaatacta tatttttatc atgtaagttg atattcccta tgtaaattat tggcacaatg 2280

taacctccaa cttgatttat acatttattt tacttttgtt tgtctatttt ttctgtaata 2340

gagtggaaga attaacattc cattaaatgt cattgcccaa tgtataaagc aatatacctc 2400

ttaccattcg atgctttttc agtattttaa ttctagatgg tgaacggaga tttttcttga 2460

tgtaggtttt g 2471

<210> 45
<211> 670
<212> DNA
<213> Diabrotica virgifera

<220>
<221> misc_feature
<222> (1)..(670)
<223> RNAi target sequence

<400> 45
tcgtaatttt tggttgtgct tgagaaaatc gtcaaacatc caaaatggtg aacttcaccg 60

tagatgaaat tagggccatg atggacaaaa agaggaacat ccgtaacatg tccgtcattg 120

cccatgtgga ccatggtaaa tctacattaa cagattctct agtatcaaaa gctggtatta 180

ttgctggtgc caaagctgga gaaaccagat tcacagacac aaggaaagat gaacaggaac 240

gttgtattac catcaaatca actgccatat ctatgttctt cgagttagat gataaagatc 300

ttgtgttcat caccaacgct gaccaacgtg agaaagatgt gaaaggtttc ttgattaacc 360

ttattgactc tcctggacac gttgatttct catcagaagt aacagctgcc cttcgtgtca 420

ctgatggtgc ccttgtagta gtagattgtg tatctggtgt atgtgtacag actgaaactg 480

tactccgtca agctattgca gaacgtatta agccaatttt attcatgaac aagatggacc 540

gtgccctctt agaattacag ttggaatctg aagaacttta ccagactttc caacgtattg 600

ttgaaaacgt caacgtcatt attgccacct acaacgatga ttcaggtcca atgggtgaag 660

tcagagttga 670

<210> 46
<211> 22
<212> DNA
<213> Artificial Sequence

<220>
<223> primer

<220>
<221> misc_feature
<222> (1)..(22)
<223> forward primer

<400> 46
tcgtaatttt tggttgtgct tg 22

<210> 47
<211> 22
<212> DNA
<213> Artificial Sequence

<220>
<223> primer

<220>
<221> misc_feature
<222> (1)..(22)
<223> reverse primer

<400> 47
tcaactctga cttcacccat tg 22

<210> 48
<211> 4535
<212> DNA
<213> Diabrotica virgifera

<220>
<221>  misc_feature
<222>  (1)..(4535)
<223>  WCRW transcript ta02030.005_diavv

<400>  48
ggggggcaga gagtcggtct ctttctttca cgggagtgcg ccatcgttgg cgtatcgtaa      60

tttttggttg tgcttgagaa aatcgtcaaa catccaaaat ggtgaacttc accgtagatg     120

aaattagggc catgatggac aaaaagagga acatccgtaa catgtccgtc attgcccatg     180

tggaccatgg taaatctaca ttaacagatt ctctagtatc aaaagctggt attattgctg     240

gtgccaaagc tggagaaacc agattcacag acacaaggaa agatgaacag gaacgttgta     300

ttaccatcaa atcaactgcc atatctatgt tcttcgagtt agatgataaa gatcttgtgt     360

tcatcaccaa cgctgaccaa cgtgagaaag atgtgaaagg tttcttgatt aaccttattg     420

actctcctgg acacgttgat ttctcatcag aagtaacagc tgcccttcgt gtcactgatg     480

gtgcccttgt agtagtagat tgtgtatctg gtgtatgtgt acagactgaa actgtactcc     540

gtcaagctat tgcagaacgt attaagccaa ttttattcat gaacaagatg gaccgtgccc     600

tcttagaatt acagttggaa tctgaagaac tttaccagac tttccaacgt attgttgaaa     660

acgtcaacgt cattattgcc acctacaacg atgattcagg tccaatgggt gaagtcagag     720

ttgaccccag caaaggttca gttggttttg gatcaggtct ccacggatgg gctttcactc     780

tcaaacaatt ctctgaaatg tatgccgaga aattcaagat tgatgtagtt aaactaatga     840

accgtttgtg gggtgagaac ttcttcaacc ccaaaaccaa gaaatggtcc aaacaaaagg     900

atgatgataa caaacgttca ttctgtatgt acatcttaga tccaatctac aagatttttg     960

actccatcat gaactacaag aaagaagaat atgaagcact ccttccaaaa ttgggtattg    1020

ccttaaaaca tgaagataaa gacaaagatg gtaaacaatt attgaaagta gttatgcgta    1080

cctggttgcc agctggagaa gctttacttc agatgatagc cattcactta ccatcacctg    1140

tggtagcaca gaaatacaga atggaaatgt tgtacgaagg acctcatgat gatgaagcag    1200

ctgttggtat caagaactgt gatccaaatg cacctcttat gatgtatgta tctaaaatgg    1260

tacccacttc tgacaaaggt cgtttctatg cttttggtcg tgtattctct ggtaaagtag    1320

ctactggtat gaaagcccgt atcatgggac ccaactacac ccctggcaaa aaggaagatt    1380

tatatgaaaa ggctatccaa cgtaccattc tcatgatggg tcgttatgtt gaagctattg    1440

aagatgtccc atctggtaac atctgtggtt tggttggtgt tgatcaattc ttggttaaga    1500

ctggtaccat taccactttc aaggatgccc acaacttgaa ggtcatgaaa ttcagtgtat    1560

ctcctgtagt acgtgttgct gttgaaccta agaaccctgc tgatttgcca aaacttgtag    1620

aaggtttgaa acgtttgacc aaatccgatc ctatggtcca atgtatcatt gaagaatctg    1680

gtgagcacat cattgctggt gccggagaat tgcatcttga aatttgcctt aaggatcttg 1740

aagaagatca tgcctgcatt cccattaaga aatctgaccc tgttgtatcc taccgtgaga 1800

cagtaagtga agagtctgat cagatgtgcc tctcaaaatc acccaacaaa cacaacagat 1860

tgttcatgaa agcttgtcct atgcctgatg gacttgctga agatattgat gatggtaaag 1920

ttaacccacg tgatgacttc aaagtccgtg cccgttatct tactgagaaa tatgactatg 1980

atataactga agcccgtaag atctggtgct ttggacctga tggtactgga cccaacatcc 2040

ttgttgattg tactaaggga gtgcaatatc ttaatgaaat caaagactct gtggtagctg 2100

gtttccaatg ggcaaccaaa gaaggtgtct tgtctgaaga aaacttgaga ggtgttagat 2160

tcaacattta tgatgttacc ttacacgctg atgctatcca tcgtggaggt ggtcaaatca 2220

ttccaacaac ccgtaggtgt ttgtatgctt gtttaatgac agctgcaccc agacttatgg 2280

aacctatcta caagtgtgaa attatgtgtc ctgaagttgc tgttggtggt atttatggtg 2340

tattgaacag aagacgtggt cacgtatttg aagaagctca aatcgctggt acacctatgt 2400

tcttagtaaa agcttactta cctgtaaacg aatcattcgg tttcactgct gatttacgtt 2460

ccaacactgg aggtcaggcc ttcccacaat cagtcttcga tcactggcaa atcttacctg 2520

gtgacccaat ggaacccagt accaaaccct atggaattgt gcaggacaca cgtaaaagaa 2580

agggtcttaa agaaggactt ccagacctgg cacaatatct ggataaatta taaacaacta 2640

agaaacttaa tttatgtaca gattatttaa taaaattatt tcaatttact caacggtttt 2700

tatagttaat tgtatttttg atatttttat tctgataagt tttcagttct ctcaaattga 2760

tggcaacact agaaatgaaa taaactaaat aatctgacta atattttatg tttgttatat 2820

atatttttgt taacagtcca gatatttgta tatttattaa tttgacatca aaagtaaatc 2880

tgatgaaaca tttttgagta tttatttgtg gatacaaatt cgtcttgttt tatttttact 2940

cccttgaaac attttattag tgtaattttt atttgagcag tactgattaa tgttaagtat 3000

attttttgga tatgtgtata ataaaactca gttttgtaag gaatctagtt ttctttaatg 3060

tgggtatcca aatcaattac gttaagtaaa taaaatacta tacccaaagt tatacaaggt 3120

tagagagtaa acaataaaac attaaatatt ctgtacatac attctagact cggataagat 3180

gaccgctgaa aataaatggg ccctcttgta ctactatatc tggtacatag tgttactaca 3240

gaactagagg attttttaaa attgaaggag ctaataccac atcagaaaaa cacccacatg 3300

ctttataaaa cattaggaag gaaagagtca ttacgtataa aaattggagc atactttaca 3360

gtaaatgaaa caaagaaacc ttatctaaac aaatattcac aaagtcctgg ataaattcat 3420

taagatcaat cacattcaat ataacagaat ttcttcattg ttttgaactc tttgtagata 3480

tctcttggaa acattaatat gctgattgga attctttaat tattttatga aaatattatg 3540

gttgatttat ggcacctgac tgatttcaga tctccaaata cacattagga aatcatgatg 3600

aagaaaccat ttttattttt ccatgtagca aattttctat agaaatatta gcttgctaat 3660

atccactaac tagtagaagt ttgtaatgtc acatttattt aaagaaatgg caggatagca 3720

aggattttat tttctttaaa aatgataaat tgcatacctt tgaagaagac aaaatattac 3780

tcctatacat ttcatacaaa caataaaaag aagaaaccac gataccagat gaggaaaaac 3840

atggaattca gttcatttag aattgattgt tttgaaaagc ttaaactaat tgatttttgc 3900

aagttgacag ctgacaagga atgctcctat acaaactgaa agatcgacga ttaggatatt 3960

ttttaaaggt tttgtaatga gtaacatatt cctaggagta tatcttgaga cataatgtga 4020

aagatctaaa atatcctatg gggcagattc ctgttgtatt taaaaaaagg gctgcaaatt 4080

aatagccact aaagctgact ttgctgtttt tgaaacaaaa agtctgcgaa ttaataacca 4140

ctgaggctga ctttgctgtt ttttaaagaa aaggttgtta attaataacc ggtgagacta 4200

accctgatgt ttttgaaaga aaggggttgc taattcataa tccatggcgc aaactctact 4260

gtttttgaaa taagagattt ttgagaaatt gtactgaaaa tgtgcctgtt cgtattaata 4320

tgaactgaaa acaacaaagg caaatcaggg atctaaccta ccgatcttta aaaggctgtt 4380

cccaaaaata aatgattctg atggtttcat ggacttaaga gtgaaatcaa gagaaggtgt 4440

ctgttcaata tagaaagttt tggggatgaa attctttctc tctggaatag tttctgttat 4500

tcaaggtgtt aaactaagaa tatttcagga taaaa 4535

<210> 49
<211> 528
<212> DNA
<213> Diabrotica virgifera

<220>
<221> misc_feature
<222> (1)..(528)
<223> RNAi target sequence

<220>
<221> misc_feature
<222> (499)..(499)
<223> n can be any nucleotide

<220>
<221> misc_feature
<222> (500)..(500)
<223> n can be any nucleotide

<400> 49
cgtcaaaagt atttgtcgca tcaactgaag acatgagaaa ataatttttt acaacataat 60

attttcagat attaaatcaa gatggtacta gcagatttag gtcgtaaaat tacgaccgcc 120

ttgcagtctt taagcaaggc aacaattatt aatgaagagg tcttaaatgg aatgctaaat 180

gatatttgtc gggcactcat tgaggctgat gttaatatca aattagttaa atctctcagg     240

gaaaatgtta aatctgttat tgactttgat gaaatggctg gtggtctcaa caagaggagg     300

atgatccaaa gtgcagtatt taaagagctt gtcaaattgg tagacccagg agtaaagcca     360

tatcaacctg ttaaaggcaa accaaatgtt attatgtttg ttggtttaca aggttctggt     420

aaaacaacca cttgtaccaa actagcttat cattatcaga aaaagaactg gaaatcatgt     480

ttggtatgtg cagatacann cagagctggt gcctatgatc aggtaaaa                  528

<210>  50
<211>  22
<212>  DNA
<213>  Artificial Sequence

<220>
<223>  primer

<220>
<221>  misc_feature
<222>  (1)..(22)
<223>  forward primer

<400>  50
cgtcaaaagt atttgtcgca tc                                              22

<210>  51
<211>  22
<212>  DNA
<213>  Artificial Sequence

<220>
<223>  primer

<220>
<221>  misc_feature
<222>  (1)..(22)
<223>  reverse primer

<400>  51
ttttacctga tcataggcac ca                                              22

<210>  52
<211>  1927
<212>  DNA
<213>  Diabrotica virgifera

<220>
<221>  misc_feature
<222>  (1)..(1927)
<223>  WCRW transcript ta05283.001_diavv

<400>  52
ggggggtcgc atcaactgaa gacatgagaa aataattttt tacaacataa tattttcaga      60

tattaaatca agatggtact agcagattta ggtcgtaaaa ttacgaccgc cttgcagtct 120

ttaagcaagg caacaattat taatgaagag gtcttaaatg gaatgctaaa tgatatttgt 180

cgggcactca ttgaggctga tgttaatatc aaattagtta aatctctcag ggaaaatgtt 240

aaatctgtta ttgactttga tgaaatggct ggtggtctca acaagaggag gatgatccaa 300

agtgcagtat ttaaagagct tgtcaaattg gtagacccag gagtaaagcc atatcaacct 360

gttaaaggca aaccaaatgt tattatgttt gttggtttac aaggttctgg taaaacaacc 420

acttgtacca aactagctta tcattatcag aaaaagaact ggaaatcatg tttggtatgt 480

gcagatacat tcagagctgg tgcctacgat caggtaaaac agaactgtac aaaagctagg 540

ataccctttt atggaagtta cacagaagtt gatcctgtag tcatagccca ggatggtgtt 600

gatatgttta aaaaggaagg ttttgaaatt ataatagtag atacaagtgg tagacacaag 660

caagaagaat ctttgtttga agaaatgttg gcagtttcta atgctgtgag accagacaat 720

attattttcg ttatggacgc cacaattggt caagcctgcg aagcccaagc aaaagccttt 780

aaagaaaaag tagatgtagg atccgtaatt atcacaaaat tggatggtca tgcaaaagga 840

ggtggtgcac ttagtgccgt tgcagctact aacagtccag ttatatttat cggtacagga 900

gagcacatag acgatttaga tccatttaaa acaaaacctt tcattagtaa attattgggg 960

atgggtgaca tagaaggttt aattgacaaa gtaaacgaat taaagttaga agataatgaa 1020

gaattgttag aaaaaattaa acatggtcaa tttacgctca gagatatgta tgaacaattt 1080

caaaatataa tgaagatggg acctttttca caaattatgg gtatgatccc aggatttagc 1140

caagatttta tgtcaaaagg aagcgaacag gaatcaatgg cacgattaaa aagattaatg 1200

acaataatgg acagtatgaa tgattacgag ttggacaaca gggatggcgc aaaattgttt 1260

actaaacaag ttggtagagt ggtcagggtc gcacaaggat ctggtgtaac agaaagagaa 1320

gtaaaagatc tcattacgca gtacacaaaa ttcgcagcgg tggtaaagaa aatgggaggc 1380

atcaaaggtc tcttcaaagg tggcgatatg gctaaaaacg tcaaccacaa ccagatggcg 1440

aaacttaatc aacaaatggc caaaatgatg gaccctagag tcctccaaca aatgggcggc 1500

atggcaggtt tgcaaaatat gatgagacaa ctccaagcag gcgcagctgg tggtttaggg 1560

ggcttaggta atcttatggg gggctttggt aataaatgat tgttaaatat tttttatgat 1620

tattttgaat tgccatttga tacttctttc agaagccata tgttgtgtca agcaacattt 1680

tttatttgta gaataatcta ataattaacg cagactttat cattaccgaa gcaaactgct 1740

acaatatgta taaagaaagt taatggcatt tgaaagaatt attgagtgat ttgtttctaa 1800

atggatgagg aagcagagta tttttgtttt gttttgaatg tgtagaaaat attttatatt 1860

gacattgtaa aaacggtaca ctgtttgtaa cttattccta aaaataaaca gttttctaat 1920

aaaaaaa 1927

<210>  53
<211>  605
<212>  DNA
<213>  Diabrotica virgifera

<220>
<221>  misc_feature
<222>  (1)..(605)
<223>  RNAi target sequence

<400>  53
aggctgtaag agaacctatg cagtctggaa ttaaggctgt agattctctt gtaccaattg      60

gtcgtggtca gcgtgagttg atcattggtg atcgtcaaac tggtaaaact gctttggcta     120

ttgatacaat catcaaccaa aaacgattca acgatgctga agacgaaaag aagaaattgt     180

actgcattta cgtcgccatc ggtcaaaaga gatctactgt ggctcaaatc ttgaagcgtc     240

tcacagacac tggtgccatc aactacacaa tcattgtatc agctactgca tctgatgctg     300

cacctttgca atacctcgcc ccatactccg gatgtgctat gggtgaattt ttcagagata     360

atggcaaaca tgccctcatc atctatgatg atttgtccaa acaagccgta gcctaccgtc     420

agatgtctct gttgcttcgt cgtcctccag ggcgtgaagc ctaccctggt gatgtattct     480

accttcactc ccgtcttttg gaacgtgccg ccaagatgtc tgaagcccac ggtggtggtt     540

cattgactgc cttgcccgta attgaaaccc aagctggtga tgtatcagcc tacattccaa     600

ccaat                                                                 605

<210>  54
<211>  23
<212>  DNA
<213>  Artificial Sequence

<220>
<223>  primer

<220>
<221>  misc_feature
<222>  (1)..(23)
<223>  forward primer

<400>  54
aggctgtaag agaacctatg cag                                              23

<210>  55
<211>  22
<212>  DNA
<213>  Artificial Sequence

<220>
<223>  primer

<220>
<221>  misc_feature
<222>  (1)..(22)
<223>  reverse primer

<400>  55
attggttgga atgtaggctg at                                              22


<210>  56
<211>  2003
<212>  DNA
<213>  Diabrotica virgifera


<220>
<221>  misc_feature
<222>  (1)..(2003)
<223>  WCRW transcript ta00263.005_diavv

<400>  56
   ggggggatcg tgcgcagttc ggcctattcg ggtactcgtt cagtgttcgt cacttaattt      60
   tcagtactgg ggcgactggc aaaaaaatgt ctttgctttc cgcacgatta gctgcctcat     120
   tggcaagaaa tgcccctaaa aatgtaacac agattgcggg agctgtcttc cctgctgcat     180
   ccgtcgcagc ccggaacttc catgctactc cacaaaacaa aggtgctgaa atttcatcca     240
   tccttgaaga gcgcatcctt ggtgctgcac caaaagccaa cctcgatgaa acaggacgag     300
   tcttaagtat tggtgacggt atcgcccgtg tatatgggtt gaacaacatc caagccgatg     360
   agatggtgga attctcctcc ggtctcaaag gtatggccct taacttggaa cctgacaacg     420
   ttggtgttgt agtatttggt aacgacaaac tcattaagga aggtgacatt gtcaagagaa     480
   ctggtgccat tgtagatgtg cctgtaggtg accaaatttt gggacgtgta gttgatgcct     540
   taggtaaccc aattgatggc aaaggaccat tgaacaccaa acaacgtttc cgtgtaggta     600
   tcaaagcccc tggtatcatt ccacgtatct ctgtaagaga acctatgcag tctggaatta     660
   aggctgtaga ttctcttgta ccaattggtc gtggtcagcg tgagttgatc attggtgatc     720
   gtcaaactgg taaaactgct ttggctattg atacaatcat caaccaaaaa cgattcaacg     780
   atgctgaaga cgaaaagaag aaattgtact gcatttacgt cgccatcggt caaaagagat     840
   ctactgtggc tcaaatcttg aagcgtctca cagacactgg tgccatcaac tacacaatca     900
   ttgtatcagc tactgcatct gatgctgcac ctttgcaata cctcgcccca tactccggat     960
   gtgctatggg tgaatttttc agagataatg gcaaacatgc cctcatcatc tatgatgatt    1020
   tgtccaaaca agccgtagcc taccgtcaga tgtctctgtt gcttcgtcgt cctccagggc    1080
   gtgaagccta ccctggtgat gtattctacc ttcactcccg tcttttggaa cgtgccgcca    1140
   agatgtctga agcccacggt ggtggttcat tgactgcctt gcccgtaatt gaaacccaag    1200
   ctggtgatgt atcagcctac attccaacca atgtcatttc catcactgat ggacaaatct    1260
   tcttggaaac tgaattgttc tacaaaggta tcagacctgc cattaacgta ggtttgtctg 1320
   tatccagagt aggatcagct gctcaaacca aggccatgaa acaggttgct ggttccatga 1380
   aactggaatt ggcccaatac cgtgaagtag ctgcttttgc ccaattcggt tccgatcttg 1440
   atgctgctac tcagcaattg ttgaacagag gtgtacgtct tactgaattg ttgaaacaag 1500
   gtcaatacgt acccatggct attgaagaac aagtagccat catctactgt ggtgtcagag 1560
   gacatttgga caaagttgat ccatcaaaga ttacagtatt tgaaaaacaa ttctcagaac 1620
   acattaagac tagccacaaa aacatcttgc agagcattgc caaggaagga aaaatcacag 1680
   acgatactga tgcccaactc aagaaagtcg ttcaagattt cttggccagc ttctctggtt 1740
   aaatataaat aatacaatta ttattaatac ctaaatcgtc acttttgttg atgtgatctg 1800
   taaacgttta agaacttcat gagctgtaat cctttcaaat tagaataatt aggcaggact 1860
   cctatgttat ccaataaggc acgtgaaaga atttcttttt ctccatttta caatatttat 1920
   cgatcattgt tttttaagga tatcttattt gtaaaaataa aaaataaact ttatattttt 1980
   taaaaaaaaa aaaaaaaaat cgg 2003

<210> 57
<211> 583
<212> DNA
<213> Diabrotica virgifera

<220>
<221> misc_feature
<222> (1)..(583)
<223> RNAi target sequence

<400> 57
gcacgaggct ttattagaca gaggtattca tcccataagg attgctgatg gtttcgagct 60

agcttgtcaa acagctgtag ctcatttgga aaaaattgca gaactatttc cggtatcagc 120

agaaaacact gaaccattaa taaaagttgc aatgaccact ctgggatcta aaatcattaa 180

caagtgtcat aaacagatgg cagaaatagc tgttaatgca gtcttagctg ttgcagattt 240

agaacagaaa gatgttaatt ttgaacttat taaagttgaa ggcaaggttg gtggaaaatt 300

ggaagacaca acattagtta aaggtgtagt tattgacaaa acaatgtccc atccacaaat 360

gcccaaagaa ctaaaagatg taaaattggc tattctcacc tgcccgttcg aaccaccaaa 420

accaaagacc aaacataagt tggatgtcag cagtgtggaa gattataagg aactaagaca 480

gtatgaacaa gaaaagttca aagaaatcgt tggtttggta aagaaagctg gagctacatt 540

agcaatttgt caatggggct tcgatgatga agccaatcac tta 583

<210> 58
<211> 23

<212>  DNA
<213>  Artificial Sequence

<220>
<223>  primer


<220>
<221>  misc_feature
<222>  (1)..(23)
<223>  forward primer

<400>  58
gcacgaggct ttattagaca gag                                              23


<210>  59
<211>  22
<212>  DNA
<213>  Artificial Sequence

<220>
<223>  primer


<220>
<221>  misc_feature
<222>  (1)..(22)
<223>  reverse primer

<400>  59
taagtgattg gcttcatcat cg                                               22


<210>  60
<211>  1889
<212>  DNA
<213>  Diabrotica virgifera


<220>
<221>  misc_feature
<222>  (1)..(1889)
<223>  WCRW transcript ta05506.001_diavv

<400>  60
aaaaatatca aatgtcagtt ttctgtcact ttcatgggag tagccagttc gaaacaaaaa      60

taaaatttta attaatctgg aacatctccc aattttttag aaaacttaca ttttcaatat      120

gagtgctata ccaggaagta ttgcatttga tgaatatgga cgacccttca taattcttag      180

agaccaagat aagcaaaaga ggttgaccgg gatagatgca cttaagtcac atattttggc      240

agcgagacaa atcgctcaaa ctataagcac ttctttgggt cccaaaggtt tagacaagat      300

gatggtgtca cctgacggtg aagtaacagt cactaatgat ggtgctacta ttttgaaaat      360

gatggatgtt gaacatgaag tcgctaaatt gctagtacag ctatcacagt cccaagatga      420

tgaaattggt gatggtacca caggtgttgt agttttggct ggtgccctgt tagagcaagc      480

aggatcttta ttagacagag gtattcatcc cataaggatt gctgatggtt ttgagctagc      540

ttgtcaaaca gctgtagctc atttggaaaa aattgcagaa ctatttccag tatcagcaga 600

aaacactgaa ccattaataa aagttgcaat gaccactctg ggatctaaaa tcattaacaa 660

gtgtcataaa cagatggcag aaatagctgt taatgcagtc ttagctgttg cagatttaga 720

acagaaagat gttaatttgg aacttattaa agttgaaggc aaggttggtg gaaaattgga 780

agacacaaca ttagttaaag gtgtagttat tgacaaaaca atgtcccatc cacaaatgcc 840

caaagaacta aaagatgtaa aattggctat tctcacctgc ccgttcgaac caccaaaacc 900

aaagaccaaa cataagttgg atgtcagcag tgtggaagat tataaggaac taagacagta 960

tgaacaagaa aagttcaaag aaatcgttgg tttggtaaag aaagctggag ctacattagc 1020

aatttgtcaa tggggcttcg atgatgaagc caatcactta ttgttacaac aagaattacc 1080

agctgtaaga tgggttggag gtccagaaat tgaacttata gcaatagcta ctgggggtag 1140

gatagttccc cgttttgaag agcttactcc tgaaaaactt ggtcatgctg gtttggtacg 1200

agaaattgca tttggcacca ctaaggatag aatgttggtt attgaagaat gttcaaattc 1260

acgggcagtt acgattcttg tcagaggagg taatcaaatg ttagtagctg aagccaagag 1320

aagtctccat gatgcattat gtgttgtcag gagtttagtt caagaaccaa gagtagtgta 1380

tggtggtgga gcagcagaaa ttgcctgctg tttggcagtc gcggctcaag cagatcaact 1440

agcttccttg gaacagtacg cctatcgtgc atttgctgaa gccttagaaa gtataccact 1500

tgctttggct gataacagtg ggctatctgc gatccatacg ctaggagaag tcaaggctaa 1560

acaagcagct acaggagatc cagctctcgg tatcgattgt atgaatactg gaaacctcaa 1620

catgagagaa caacatgtta tcgaatcact cagatcaaaa agacaacaat ttttgttggc 1680

cacccagttg gttaaaatga tattgaaaat tgacgatgta cgccaacctg catcgggata 1740

aaatgacact tatttttgta ctaccggcgt taatttctaa ttctcactaa taatgacttt 1800

tataagttga tctgcgtaat gcttctttgt tcttataatc actgaaagta ttaatataat 1860

ttatagaaaa aaaaaaaaaa aaaatgcga 1889

<210> 61
<211> 588
<212> DNA
<213> Diabrotica virgifera


<220>
<221> misc_feature
<222> (1)..(588)
<223> RNAi target sequence

<400> 61
agagaacagt cagcacaagc agttattgat atcgatgatt tagataattt ggctacacct 60

gaaagtatat tgttgagtgc tgtttctgga gaagatgccc aagaccgttc agatcgtaca 120

attcttactc cgtgggttaa gttcctttgg gaatcttact gtcaatgttt ggaacttttg 180

cgtactaatg cacatgttga gaacttatat catgatatag ccaggatggc tttccagttt 240

tgtttgaaat ataatagaaa aacagaattt agaaaattat gtgataaatt gaggaaacat 300

ttagaggata tctgcaaatt accaacacaa atggcaaatg tttctatgtc taaaccagaa 360

acccaacaac ttaatcttga aacgaggctc catcaactag attttgctat tcagatggag 420

ttatggcaag aagcctacaa ggccatcgaa gacatccata acttgatgaa tttgtccaag 480

aagagtccag taccaaaaac catggcaaac tattaccaga aactagccat ggtgttctgg 540

aaagctggta attatctatt ccatgctgct gctttgttca agttcttc 588

<210> 62
<211> 22
<212> DNA
<213> Artificial Sequence

<220>
<223> primer

<220>
<221> misc_feature
<222> (1)..(22)
<223> forward primer

<400> 62
agagaacagt cagcacaagc ag 22

<210> 63
<211> 22
<212> DNA
<213> Artificial Sequence

<220>
<223> primer

<220>
<221> misc_feature
<222> (1)..(22)
<223> reverse primer

<400> 63
gaagaacttg aacaaagcag ca 22

<210> 64
<211> 3733
<212> DNA
<213> Diabrotica virgifera

<220>
<221> misc_feature
<222> (1)..(3733)

<223>  WCRW transcript ta05000.002_diavv

<400>  64
attttgtctt tcaactgacg tttgctttga cgaaatttca gaagataata gttttattta      60

ctattctata tatttaaatg tgaagtgaat acaacaaata aacacagtaa agatggcccg     120

atacggtcag cgtccggaaa acgctttaaa gcgagcgaat gaattcatcg aagttggaaa     180

accagctaga gcccttgata ctctccaaga agtttttcgt aataaaaaat gggcgtacaa     240

ttggtcagaa tccgttttgg aacctattat gttcaaatat ctggatctat gtgtagaact     300

gaaaaaatct cacattgcca aggaaggtct tttccaatac aggaacatgt tccagtcagt     360

gaatgtagga tcacttgaaa atgttataag agggtatttg cggatggcag aagaaagaac     420

agagaatgct agagaacagt cagcacaagc agttattgat atcgatgatt tagataattt     480

ggctacacct gaaagtatat tgttgagtgc tgtttctgga gaagatgccc aagaccgttc     540

agatcgtaca attcttactc cgtgggttaa gttcctttgg gaatcttact gtcaatgttt     600

ggaacttttg cgtacaaatg cacatgttga gaacttgtat catgatatag ccaggatggc     660

tttccagttt tgtttgaaat ataatagaaa aacagaattt agaaaattat gtgataaatt     720

gaggaaacat ttagaggata tctgcaaatt accaacacaa atggcaaatg tttctatgtc     780

taaaccagaa acccaacaac ttaatcttga aacgaggctc catcaactag attttgctat     840

tcagatggag ttatggcaag aagcctacaa ggccatcgaa gacatccata acttgatgaa     900

tttgtccaag aagagtccag taccaaaaac catggcaaac tattaccaga aactagccat     960

ggtgttctgg aaagctggta attatctatt ccatgctgct gctttgttca agttcttcca    1020

gttgtcaaag gagatgaaga agaacatcac tccagaggaa ttacaaagaa tggcctgtag    1080

ggtattaatc gctacactag caataccgtt gccttccgct caccctgagt ttgatagatt    1140

cattgaaacc gacaagtcac ctttagagaa ggctcagcgt ttagctgttt tattggggct    1200

gattcaacca cctactagag ctagtctttt gagagatctt gtaagagtta atgttgtgaa    1260

cctggcaagt ccacaactac aagatttgta caattggctg gaagtggagt tccatccttt    1320

actattgtgt cagagagtgc atgaggtaat caaatctctc gaatctgaag aaaatgcagc    1380

cctccaacaa tatgtgccag ctttacaaga tgtaactctg gtgcgtctag tgagacagat    1440

agcacaagtc tatcagacca tagaatttgc taggttgatc cagattgcca aatttactac    1500

accttttcat ctggagagac ttttggttga ttgtgtgaga cacaatgata tgcaaattcg    1560

tattgatcat ggcaaagact gtatccactt tggaatggac ctgagtgaaa gccaaagaga    1620

ggacaaacca gagggaccca ctttgcaagt tatgcctagt gagcaagtca gaaatcaact    1680

tattaacatg tctactgtat tgaatcaagc catacaagtc attaatccta ataagaagaa    1740

gcttgaacgc gaaaaactta gagctgttat ggttcagaat tatcacgaaa caaaggttag    1800

agaacaccag aaaattttac aaaggcacaa gattattgag gacagaaagg aatatattga 1860

gcgtttgaat acagtcaggg aagaagagga gcagaaaaga ttggaagaaa tgcaacggca 1920

gcacttactg gccgaacaaa aacgattgga ccaagaaaga gaagaaagag agcggaaacg 1980

tgctctaaat gaaatccagc aggttaaaga cagacactta aaggaaaaac tacaacaaat 2040

cagacagact ggtcatggtc agaagattct gaagaaaatg gatgaagacg atatcaaaaa 2100

gcttgatgca gaccaaatag cggtgaaaga agcagaagag ctacaaaagg aacgtcgcga 2160

acttcaagcc aagcttaaat ctcaagaaaa gaaggtagac tatttcgagc gtgccaagcg 2220

gttggaagaa atccctctac ttcaagccaa tatgcaagaa cgtcaattac aggatcagaa 2280

cttctgggag caacaggaga aagagcgaat cgctgcagca attgaagagc gcaagctggc 2340

cgtcgcaacg agagaccgtt tgctacgcat gaaagtcgac aaagatgaat tcatctcgaa 2400

attaaagaag gagagaaaca ttgtctacga agacaatctt aaggaattcg agaagatgtt 2460

gagcgaggaa agaaagaaga ggcttttgga aagaaagatc caaagaaaag aggagcgccg 2520

tgctacatat attaaagaga aggaagaaga agccgaacgt agagccaacg aaaagagacg 2580

tcgcgaagaa gaagaaaggg agagattaga ggaaatagcg agaaaagaac aggagcaaca 2640

agaacgcgca ctaaaggagg aacaagaaag gaagaagaag gaacatcaag agagtttgga 2700

tagagcttgg acaatgcaga aagctaaaga ggaagaaatc gaaaagaaat tagcaatgga 2760

gaaagaaagc agcgacaaag gaaaagattc ttggaggaaa gaccccaaga atagctcttc 2820

ttggaggagg gacgagaaac ctgctgacga acccaaaaat gaaccttgga gagcatctcg 2880

tttccgatca aatgctgaag aacccccaaa aggcgacaac accactactt cttcttggag 2940

gaatgacgat aaaagggaag atcgcagaga cgatcgccaa ggtgaacgtc gcggcggaga 3000

cggcggaaga tttggcaacc gcgacagcgg aagatttggc gacagagaca gagaaggtgg 3060

tgtcagattc acgagagata atcgggataa tcgtggggat cgcagggacg atagggagag 3120

gagagacgac agggataaca gggataggag agacgacaga gatcgtagag atgataggga 3180

taggagagac gacaggagcg gtagagatga tagagagaga agagatgacc gtggcggaag 3240

agatgataac cgggaccgcc gagacgatcg tgatcgtaga gacgacaggg accgaagagg 3300

cggggacgaa ggtacttggc gtagaaagac tgacgatgac cgtcgttcta ctcctatgag 3360

aagagaagct gctccagaat cagcatggag gagtgctaac cgagagaccg atcgtagaga 3420

tgattctaag agagacgata gaaggggaac tccgatgacc agagaaaaac cagcagctcg 3480

cgagcagcaa tctcagccag aaggcgagga gggctggtcc caagttggaa ggagacgtta 3540

agccattata taatctagac tagagaatta agttacacaa ttataccgat attttattta 3600

cctacaggtt acgagggttt tcatttatag taatcattgt tttaatgcgg aactggcttg 3660

aactcgttga taaggcagtg ctgtgtgcta attaacccat atagttaata aaatttcaga 3720

tattataaaa aaa                                                        3733

<210>  65
<211>  716
<212>  DNA
<213>  Diabrotica virgifera

<220>
<221>  misc_feature
<222>  (1)..(716)
<223>  RNAi target sequence

<400>  65
ttgaaaaaga tagggctcca aagtcctatt gccaacaaaa aaacaacgca agacattaaa      60

caactttttt caagcccatc tgtatttaaa aaaacagtgc aaaatgtttc tgtgggactg     120

gtttacggga atgctgggat atctaggatt gtggaagaaa cctggaaaac tattattctt     180

aggactggat aacgcaggca aaactaccct tctacatatg ctcaaggatg acagactggc     240

ccagcatctt cccacgttac atcccacatc agaggagctt tccattggta acatgaggtt     300

tacgacgttc gatttggggg gccacgagca ggctaggaga gtgtggaggg actactttcc     360

agcagtcgat gccatagtgt tccttgttga tgccaacgac agctcaagat ttgtagaaag     420

ccaggaacag ctaaatgccc tcctctcaga cgaaactcta tcaaactgtc caatacttat     480

cttaggtaat aaaattgatc tcccaggtgc tgcttcggaa gatgaattac gaactagatt     540

cggcttgttt ggccaaacca caggcaaagg caaagtagcc agaaatgatc tacccggtag     600

gcctctagaa ctatttatgt gctctatact caaaagacaa ggttatggag aaggtttccg     660

ttggttggca caatatatcg attaattatg tatttttcca tttcgttctg tcattg         716

<210>  66
<211>  22
<212>  DNA
<213>  Artificial Sequence

<220>
<223>  primer

<220>
<221>  misc_feature
<222>  (1)..(22)
<223>  forward primer

<400>  66
ttgaaaaaga tagggctcca aa                                               22

<210>  67
<211>  22
<212>  DNA
<213>  Artificial Sequence

<220>
<223>  primer


<220>
<221>  misc_feature
<222>  (1)..(22)
<223>  reverse primer

<400>  67
caatgacaga acgaaatgga aa                                                22


<210>  68
<211>  2438
<212>  DNA
<213>  Diabrotica virgifera


<220>
<221>  misc_feature
<222>  (1)..(2438)
<223>  WCRW transcript ta00303.001_diavv

<400>  68
ggggggaaaa agatagggct ccaaagtcct attgccaaca aaaaaacaac gcaagacatt        60

aaacaacttt tttcaagccc atctgtattt aaaaaaacag tgcaaaatgt ttctgtggga      120

ctggtttacg ggaatgctgg gatatctagg attgtggaag aaatctggaa aactattatt      180

cttaggactg gataacgcag gcaaaactac ccttctacat atgctcaagg atgacagact      240

ggcccagcat cttcccacgt tacatcccac atcagaggag ctttccattg gtaacatgag      300

gtttacgacg ttcgatttgg ggggccacga gcaggctagg agagtgtgga gggactactt      360

tccagcagtc gatgccatag tgttccttgt tgatgccaac gacagctcaa gatttgtaga      420

aagccaggaa cagctaaatg ccctcctctc agacgaaact ctatcaaact gtccaatact      480

tatcttaggt aataaaattg atctcccagg tgctgcttcg gaagatgaat tacgaactag      540

attcggcttg tttggccaaa ccacaggcaa aggcaaagta gccagaaatg atctacccgg      600

taggcctcta gaactattta tgtgctctat actcaaaaga caaggttatg gagaaggttt      660

ccgttggttg gcacaatata tcgattaatt atgtattttt ccatttcgtt ctgtcattga      720

gttaggatat taatgtttga ggaactattg gcaacactgc aactacctga ttcatttcag      780

atcttaggta ctttacataa atatctaaat atagatgttg gcaatgtaat tttgaacaac      840

agtatataca ttcaatgtaa attatatatt tttagttaag ttaccttctt aaatggtgtt      900

tgagtggcta tggtactgaa atagtttgac tttttgtgtt cttcgataac taaaaatgat      960

ttcttgtgga aagttacact cagaattaca tagttaactt ctttatcagc agttgttgtc     1020

aagatttcca ttttgagacg attgttttgt aaattaggtg ggaattttta aaatgttgga     1080

ctgtttttta acatgccttt ctcaactttt gttacaaata atgttttgtc cagttgtaaa     1140

tgttttattt aactagagac tttctaggtt gtgttaataa tagtcattgg tcaaatacga 1200

tagaagccaa aatttgaaga aaaggaacaa tgtactaggg tggtccgata agtattttgc 1260

cttcaaaaga aaagtggcga tttatttttc aatattgtct ccttctagct caatacacgt 1320

gacccagcga ggcttcaccc agattcagcg gcttcaattt ctgaaggtct gtaaagtaga 1380

catcaatagc agcgatgaac tcactaaatc gacttacatt tctgcccgac gagtgacttt 1440

tcaaatttgg aaacgaaaag aaatcgcaca gggccaaatc tggagaatac agtggatggg 1500

atagtagtta gtagtccgaa tcgaaaaatt tggccatggc gacggcgaag gtgtgagcgg 1560

gtgcgttgtc gtggaagagt actttttatc ttccccaatt ggggctgttt tttctgcaag 1620

tcggtgtcga atcgacccaa taattcgcca aagtaaagcc ctgtgaccgt tttgcccttt 1680

ttttcaggaa gctgatatat atcacacttt gtgaatccca gaaaacggtg gtcatcactt 1740

ttcggtcgtt aggacagtct ttgccttctt cggagtatgt tcgcggggcg acgtccactg 1800

tatcaactgt tccttggttt ctggtgagta ctggtggatc caagtttcat cgatgattat 1860

gaaacaatgt ataaacaata ggaaggttta atttatttct aattctgagt aggaatggtc 1920

acttaaaggt tttgtaattt aacttaagta cctaatgaat ttattttgga ctttttcaag 1980

cttggctata tggacttcat aagcgggtga ccaaacaact gagcaatact caagaacaga 2040

tctaactagg gaaatgtaaa tcagtctgat tgaattaatg ttatgcaaac atctagaagt 2100

tctaataatg aagcctaaca tgtgaaatgc cgcaccaacc acatcaacac aatgcgcata 2160

aacaaaataa tgaagaatat aactcaaaaa acagatacag cacaggtgac acaacacaca 2220

gcagtcaagc acaaagtgaa agcacaaaaa actacaaaac caaaaaacaa gaacaaaatc 2280

acaggcaaat atcaaacgaa gtgaattatt caacaaacca aaaataaacc aaatgccaca 2340

cccagtcaaa acaaaacaac gaaggttgag attttgacat agataggcca tctacaagaa 2400

tgtattacat atacagggtg cgccaagcct ctggtttt 2438

<210> 69
<211> 619
<212> DNA
<213> Diabrotica virgifera

<220>
<221> misc_feature
<222> (1)..(619)
<223> RNAi target sequence

<400> 69
tcggtcgatt taggtagtag gtgtaataaa ttttgaaagt gataaagaca gtttgtgata 60

aactgtagtg cagttctttt cgtttatttt ctcacctctc acataacaat taaactgaaa 120

caatggcccc cccgggtatc gggaattcgg tggagatccg ccccgaggcg acagcatcgt 180

cggcagagag gagcagtagc aacggagtcg ctacattcca aaaggacatc gtcgttttgg 240

atgggggctt cgctactcaa ttaggctgcc acgtccaaca accgattgat ggagacgtcc 300

tatggagttc caggttcttg gccaccaatc cagaggctgt tattgatact cacttagatt 360

ttctcagagc tggtgcggat gtaattatta ctaacacata tcaagcagat gtcaacttgt 420

ttgccaaaca tctcaatatg accaaagaag aaggatacaa cttaataaaa gactcagtga 480

agatggccaa aacagctgtt gaaagatatt taaaagaata tccggaagca agaaaacctc 540

ttatagctgg ttcagtgggt ccttacggag cttcactaca tgatggttcg gaatacacag 600

gagcctatgc gaaaactac 619

<210> 70
<211> 23
<212> DNA
<213> Artificial Sequence

<220>
<223> primer

<220>
<221> misc_feature
<222> (1)..(23)
<223> forward primer

<400> 70
tcggtcgatt taggtagtag gtg 23

<210> 71
<211> 22
<212> DNA
<213> Artificial Sequence

<220>
<223> primer

<220>
<221> misc_feature
<222> (1)..(22)
<223> reverse primer

<400> 71
gtagttttcg cataggctcc tg 22

<210> 72
<211> 1246
<212> DNA
<213> Diabrotica virgifera

<220>
<221> misc_feature
<222> (1)..(1246)

<223>  WCRW transcript ta08045.004_diavv

<400>  72
ggtcactcgt cgccgactgc cgacttgatc ggtcgattta ggtagtaggt gtaataaatt      60

ttgaaagtga taaagacagt ttgtgataaa ctgtagtgca gttcttttcg tttattttct     120

cacctctcac ataacaatta aactgaaaca atggcccccc cgggtatcgg gaattcggtg     180

gagatccgcc ccgaggcgac agcatcgtcg gcagagagga gcagtagcaa cggagtcgct     240

acattccaaa aggacatcgt cgttttggat gggggcttcg ctactcaatt aggctgccac     300

gtccaacaac cgattgatgg agacgtccta tggagttcca ggttcttggc caccaatcca     360

gaggctgtta ttgatactca cttagatttt ctcagagctg gtgcggatgt aattattact     420

aacacatatc aagcagatgt caacttgttt gccaaacatc tcaatatgac caaagaagaa     480

ggatacaact taataaaaga ctcagtgaag atggccaaaa cagctgttga aagatattta     540

aaagaatatc cggaagcaag aaaacctctt atagctggtt cagtgggtcc ttacggagct     600

tcactacatg atggttcgga atacacagga gcctatgcga aaactacttc ggcagaaacc     660

atgagagaat ggcacgggcc cagaataaaa gccttggtgg aaggaggagt tgatctctta     720

gcaattgaaa ctattccgaa tgcagttgaa gctgagatgt tggtccagtt aataaaagaa     780

gaatatccta atgttaaagc gtggcttgct tttagtgtag cgcaagacgg aaaatcaata     840

gcctacggag aagacttcaa aacaatcgcc cgaaaatgtt atgactccaa tcctgagcaa     900

ttagtagcca taggaatcaa ctgcacggcc ccacgactaa tcgaacccct tctcaccggg     960

ataaatgatg atagaaaaga caacccccga ccacttattg tctaccctaa cagcggagaa    1020

agttacaatg tcgaactcgg ggatgattcc agatggcata accgagaaaa ctgcgagcca    1080

gtagacacaa atattcacaa atggttagat ctgggagtca cgtgggtcgg aggctgctgt    1140

agaacctacg ccgtggatgt cacaagaata cgcgaccaag tgcagttatg gaaggagaaa    1200

cagtagctaa actttgaatt tatattttaa tttttatttt taataa                   1246


<210>  73
<211>  680
<212>  DNA
<213>  Diabrotica virgifera


<220>
<221>  misc_feature
<222>  (1)..(680)
<223>  RNAi target sequence

<400>  73
attgtcaacc tggaaaacga cttgtcgaag tcgcatagtt tttataagtt taaataaact      60

aaattaaata taaatacttc gagaatgcaa taattattat tctttaacta gacccacagc     120

ttattaatta gcagaagtag tagcagactt atactaacta gcataaggag aaacatatta     180

acatagcatg gcagacttca tagattctga agcagaagaa agtagtgagg aggaggaatt 240

agatcatagg gatcgtaaaa aagcccaaaa agccaaagtt gtagatagtt cagatgaaga 300

tgatgaagat gatgacgaaa gactgagaga ggaattaaag gatttgattg atgataatcc 360

tattgaagaa agtgatgctg agtctgatgc ttcaggaagg gaaaaacgta agaaatctga 420

cgacgaggat ttggatgatc gactggaaga tgaagattat gatttgcttg aagaaaattt 480

gggtgttaaa gttgaaagaa ggaaattcaa gcgactgcgg cgttttgaag atgaagaaag 540

tgaaggagaa gaagaacatg atcctgaaca agatagggaa caaattgcta tggatatatt 600

ttcagatgat gacgatgaaa gacgatcaga acgaagtcac aggcctgccg tcgaacaaga 660

aacttatggt gtaggcgagg 680

<210> 74
<211> 22
<212> DNA
<213> Artificial Sequence

<220>
<223> primer

<220>
<221> misc_feature
<222> (1)..(22)
<223> forward primer

<400> 74
attgtcaacc tggaaaacga ct 22

<210> 75
<211> 22
<212> DNA
<213> Artificial Sequence

<220>
<223> primer

<220>
<221> misc_feature
<222> (1)..(22)
<223> reverse primer

<400> 75
cctcgcctac accataagtt tc 22

<210> 76
<211> 6054
<212> DNA
<213> Diabrotica virgifera

<220>

<221>  misc_feature
<222>  (1)..(6054)
<223>  WCRW transcript ta02840.001_diavv

<400>  76
agtttaaata aactaaatta aatataaata cttcgagaat gcaataatta ttattcttta      60

actagaccca cagcttatta attagcagaa gtagtagcag acttatacta actagcataa     120

ggagaaacat attaacataa catggcagac ttcatagatt ctgaagcaga agaaagtagt     180

gaggaggagg aattagatca tagggatcgt aaaaaagccc aaaaagccaa agttgtagat     240

agttcagatg aagatgatga agatgatgac gaaagactga gagaggaatt aaaggatttg     300

attgatgata atcctattga agaaagtgat gctgagtctg atgcttcagg aagggaaaaa     360

cgtaagaaat ctgacgacga ggatttggat gatcgactgg aagatgaaga ttatgatttg     420

cttgaagaaa atttgggtgt taaagttgaa agaaggaaat tcaagcgact gcggcgtttt     480

gaagatgaag aaagtgaagg agaagaagaa catgatcctg aacaagatag ggaacaaatt     540

gctatggata tattttcaga tgatgacgat gaaagacgat cagaacgaag tcacagacct     600

gccgtcgaac aagaaactta tggtgtaggc gaggaagaag aggaagggga gtactcggat     660

gccgatgatt ttatagttga cgatgacggt agaccgatag ctgaaaaaaa aaaaaaaaaa     720

aaaaaaaaaa aaacagatgc cgctctccaa gaggctcaag aaaccttcgg tgtcgatttt     780

gattatgatg aatttagtaa atacgatgaa gatgattacg aagatgaaga ggaggaggat     840

gacgaatacg aggaagatga tgtagagaaa aggaaacggc ctaaaaagac ttcaaagaaa     900

aaaccgacga agaaatccat ttttgaagtg tatgaaccta gtgaacttaa aagagggttc     960

tttaccgatc tcgataatga aatccgaaac actgatattc ccgaaagaat gcaacttcgt    1020

gatgttccaa tcaccgctgt tccggatgac tcaactgaac ttgatgatga agcagaatgg    1080

atttacaggc aagcgttttg taacagaact gtttccaatg tggattctca tttatcatca    1140

gaggcaagag agaaattaaa gaagactcat catgccatcg gaaaaatcag aaaagcatta    1200

gattttataa gaaatcaaca attagaagta ccgtttattg ctttttatag aaaggagtat    1260

gttcaaccgg aacttaatat taacgatttg tggaaagtat ataaatacga tgcaaagtgg    1320

tgccaattga aaacacgcaa agaaaacctc ttgaagcttt ttgaaaaaat gaggtcatat    1380

caaactgacc acataatgaa agatccagat gcaccaattc cagacaacct tcgtatcatg    1440

actgagtccg acattgagcg attgaaaaat gttcaaaccg ccgaagagtt aaatgacgtt    1500

cacaatcatt tcattttata ttatgctgca gatttgccag ccatgcatgc cgcgtggaga    1560

gtcaaagaaa gagaaaggag aagacaggaa agaaaggagg ctagacttaa gctcatcgct    1620

gaaagtgaag aaggtgctga aattcctgaa gagcctgagg aaattgacga tgatgaacca    1680

gaagctgaaa ccttaaaata cgccaataga tcaggcagct atgcactatg taataaagga    1740

ggtttgggtc ctctagcgaa gaaatttggt ttaacgcctg aagaatttgc cgaaaacctg 1800

agagataatt atcagaggca cgaagtagat caagaacata tagaacctgt agaagtcgct 1860

aaagaattcg tatcgccgaa atttcctaca gtggaagaag tgttgcaagc tactaaacac 1920

atggtagctt tacaaatagc aagagaacca ttagtaagga aatgcgtaag agaaattttc 1980

tttgaacgag ctagattaaa cgtttatcca accaaaaagg gtgtgaaagt tatagatgaa 2040

gctcataatt gctatagtat gaagtatgta aaaaataaac cagtaagaga tcttgcaggc 2100

gaccaatttt taaaattatg tttagccgaa gaggagaacc tccttactat taccatcaat 2160

gaccatattc aaggaaacac tactaacaat tacattgatg aagtcaaaca attatatata 2220

aaggatgaat tcagcaaaca cgttcaggat tggaatgcgc taagaatgga atcggtagaa 2280

agggctttaa cgaaaagtgt cttaccagat ttaagatctg aattaaaacg aacgttgctc 2340

acagaggcta aagaattcgt attaaaagct tgctgtagaa aattatataa ttggataaag 2400

attgctccat acgcaataac ttttcccgat gaagacgaag atgattggga tacatccaaa 2460

ggtgttagaa ctatgggtgt agcatacgta ccagagcaca cagtatcagc ttttacctgt 2520

atttcagcac cagacggaga tataactgat tatctcagat taccaaatat tctaaaaaga 2580

aaaaatagct ttcgaactga agaaaaactt atgaaggaag ctgatcttca agcactgaaa 2640

aatttcatat tccttaaaaa gcctcatgtg atagcagtag ggggtgagtc cagagaagcc 2700

ttaatgatcg ctgatgatat cagaggagta ataagtgaac taatagaatc cgaccaattc 2760

ccacagatta gggttgaaat tattgataat gaattagcca aagtgtacgc aaattccatt 2820

aaaggttcaa ctgatttcag agattatcca gagttgttaa ggcaagctat ttcattagct 2880

cgaagaatgc aagatccttt ggttgaattt tcccaattat gtaatagcga tgaggaaatt 2940

ttgagtctga ggtttcatcc cttgcaggaa caagtccaga aagaagaact actagaagct 3000

ctctgtttag aatttgtcaa cagaacaaat gaagtaggtg tagatataaa tcttgccgtt 3060

cagcagattc ataaaaagta gtttagttca attcatatgc ggtctaggac cgcgtaaagg 3120

tcaagcgtta cttaaagttc tgaaacaaac taatcagagg cttgaaaaca gaacccaatt 3180

ggttacattt tgtcatatgg gtccaaaagt ttttattaat tgttctggat tcataaagat 3240

tgataccaat agtttaggag acagtactga ggcatatgtt gaaatattgg atggctctcg 3300

agttcatccc gaaacttatg aatgggcacg aaaaatggct gtggatgctt tagaatatga 3360

cgatgatgag ggagctaatc cggcgggagc tttagaggaa attctcgagg cgccagagag 3420

gttaaaagat cttgacttgg atgcatttgc ggaggaattg gaaagacaag gaatttggta 3480

acaagagtat aacattgtat gacattagag cagaactgaa ctcgcgatat aaagatttga 3540

gacaaccttt tcgttctgca aatcctgaag aactattcga tatgcttact aaagaaactc 3600

ccgaaacatt ttatattgga aaaatggtta catctaccgt gtttggcatt gcaaggagaa 3660

aaccaaagtc agaccagctc gatcaagcta atccggtccg taatgacgaa actggtttgt 3720

ggcagtgccc cttctgtttg aaaaatgatt ttcctgaatt atctgatgta tggaatcatt 3780

ttgatgcagg agcatgtcct ggtcaagcta ctggagttaa actaagactg gataatggta 3840

tattaggtta tatttatata aaaaatataa gcgacaaacc agttgctaat ccggaagaaa 3900

gagtaggcat aggacaatta attcactgta gaataataaa aattgacgta gagaagttta 3960

gtgttgattg tacatcgaaa tctagcgatc ttgctgataa aaatcatgaa tggagacctc 4020

aacgagatcc tcattatgat caagaacgtg aagacaaaga caatcgatta gaagccgaga 4080

agaagaaaga gaaacagcgt atgacgtaca tcaagagggt tatcgttcat ccggcgttcc 4140

ataacatatc ctatgccgaa gccgaaaaat gtatggttaa tatggaccag ggtgaagtta 4200

tcattaggcc ttcgaggaag ggcccggaac aatcgaacat aacgaagaag gttaaggaag 4260

gaacatatca acacaaagat actaaagaaa aacgaaaact gaacaatggt acattaggaa 4320

aatcattatg gattggaaat gaagaatttg aagatcttga tgaaataata gctaggcacg 4380

ttacaccaat ggcggcgcat gctagagatt tactgtattt taggtattac aaagatttcc 4440

aaggtggtca taaagataag gctgaggaat atctaaaaga tgaaaaaaag aaaaatgctt 4500

ctaaaattca ttatgtagtt agtgctgcta agaatatacc tggtaaattt cttctttcgt 4560

accttccacg caacaaagtt agacacgaat atgtaacagt tactccagaa ggctttcggt 4620

ttagacaaca aatgtttgat tccgtttctt cgttatttaa atggttcaaa gaacatttcc 4680

gggagcctcc accaggcggt gcaacaccag gtagcacgcc aaggatggct tcaagcagaa 4740

ctggatatgg aagtgccaca cccgcgtata gtatgaataa tgaagctatt caaagagttg 4800

ctcaaaattt accaagtcat gtagttcaag cgttatctgc tgccaccaac caaactccac 4860

attatcccca cacccctggt tatggaggca attatattaa tacgccttac actccaagtg 4920

gtcaaactcc atacatgact ccgtacgcta caccccatac gcagcaaact ccccgctatg 4980

gtcatcaaac accttcccaa cacatggcga gctcagcacc acaaggtctc aacaatccct 5040

ttttacatcc tggcgcggtg actccctccc aacgaactcc tatttatcgc aaccatcctg 5100

cacaatctcc agtaatgctt cctacaagcc ctgtaccaag tccaggttcc cagagttcat 5160

acagtagtca tttaagtcat aatcagcgaa gtggaagtta tgctgaatcc ttaagattcc 5220

aacctcccga atcgccgaga agctcagtga gtaatagaag ctttcaaact gatagatacg 5280

gcggtgatag gtatggtaaa ggaggaagtc atagatatgg tggaagctca aacgaagata 5340

gatatggtaa gggaggagga ggaaatgaaa atacggattg gcagaaagct gcagaagcat 5400

gggctcgatc tagatctact ccgagaagtg atggccgtaa tactccaaga tctgtaggtc 5460

aacgaactcc tagatacgac aacgacgccg aacgttctag aatgaaacac ctcagcaaga 5520

gcccgaggtc tgttaggtct actcctcgaa ccaatacatc tccacattct atgtccctag 5580

gtgatgctac ccctctgtat gacgaaagta tctaaattac tttgtatttg gttatatagt 5640

agggtaacag tgatattgta aatgctaaaa tcaacaatac agattcgcga tattaagtca 5700

caatgacgac ctctcgtgta tttcggctga actagcatcg aggggtcttt tcaatgtcaa 5760

acgctgagcg cacatgcatt tgaaacaagt aggcaatatt cacttattta tttttacatg 5820

ttttaaatta attattgggt aatataaaat gattaacatg gaatcttcac gtttcaataa 5880

aagttggcgt cgatattgta tataaaatta aaaaaaatat gtaaaaataa ataatattac 5940

ctacttgtct caaatgcacg tgcgcccagc gtttgacatt gaaatcaccc tcaaagctag 6000

ttcagacgaa atagacgaga ggtcgtcgtt gtgacttaac atcgtgaata gaaa 6054

<210> 77
<211> 319
<212> DNA
<213> Diabrotica virgifera

<220>
<221> misc_feature
<222> (1)..(319)
<223> RNAi target sequence

<400> 77
tggtaaggga ggcaaagtta agggaaaagc aaagtcccga tcaaatcgtg ctggtttaca 60

atttcctgta ggtcgtattc atcgtttatt gagaaaagga aattatgccg aaagagttgg 120

tgctggagct cctgtatact tggcagctgt tatggaatat ttagctgctg aagttttgga 180

attggcagga aatgcagcta gagatagcaa aaagacccgt ataattccta gacatttaca 240

attggccata agaaatgacg aggaattgaa caaattactg tcaggagtta ccatcgccca 300

aggtggagta ttgcctaat 319

<210> 78
<211> 22
<212> DNA
<213> Artificial Sequence

<220>
<223> primer

<220>
<221> misc_feature
<222> (1)..(22)
<223> forward primer

<400> 78
tggtaaggga ggcaaagtta ag 22

<210> 79

<211>  22
<212>  DNA
<213>  Artificial Sequence

<220>
<223>  primer


<220>
<221>  misc_feature
<222>  (1)..(22)
<223>  reverse primer

<400>  79
attaggcaat actccacctt gg                                                22


<210>  80
<211>  456
<212>  DNA
<213>  Diabrotica virgifera


<220>
<221>  misc_feature
<222>  (1)..(456)
<223>  WCRW transcript ta07804.001_diavv

<400>  80
gggggaaggg aggggagtat tcacttgatc ttcaaggtag attaacgcaa gtagaaatct        60

aaaacatgtc tggacgtggt aagggaggca aagttaaggg aaaagcaaag tcccgatcaa       120

atcgtgctgg tttacaattt cctgtaggtc gtattcatcg tttattgaga aaaggaaatt       180

atgccgaaag agttggtgct ggagctcctg tatacttggc agctgttatg gaatatttag       240

ctgctgaagt tttggaattg gcaggaaatg cagctagaga taacaaaaag acccgtataa       300

ttcctagaca tttacaattg gccataagaa atgacgagga attgaacaaa ttactgtcag       360

gagttaccat cgcccaaggt ggagtattgc ctaatataca agcagtactt ttacctaaaa       420

agaccgaaaa aaaaaaaaaa aaaaaaaaaa aaaaaa                                 456


<210>  81
<211>  759
<212>  DNA
<213>  Diabrotica virgifera


<220>
<221>  misc_feature
<222>  (1)..(759)
<223>  RNAi target sequence

<400>  81
atcatcctct gtgtttccgt tttgaactta acttgtattt agtttaaaaa ataatcatgt        60

ctagaggaag cagtgcaggt tttgaccgac acataacaat tttctcgccc gaaggccgac       120

tctatcaagt agagtatgct tttaaagcca ttaaccaagc cggccccact tcggtagcag       180

tccgaggagt agatgctgcg gcgtgtgtga cccagagaaa gatcccggat aagctgattg 240

atcccaacac aattacacat ctgttccagt taacagaaca cactggatgt gtgatgactg 300

gcatgattgc tgacagcaag tcccaggtgc agagagctag atatgaggct gccgagttca 360

aatataagtt tggatatgag atgccaatcg atgccttgtg taggagagta tcggatattt 420

cccaggttta tacgcagaat gctgagatga gacctttggg ttgctccatg cttctgatag 480

gatatgacca agaaatggga ccatgtgtcc acaaagctga ccctgctggc tattactgtg 540

gctacagagc agtaagtgta ggatccaaac aaactgaagc caacagctac ctagagaaga 600

agctaaagaa gaaaactgat ctgtccaaag atgaagtcat ccaattggca atcagttgtc 660

tttcaagtgt tttatctgta gattttaaac caacagaagt cgaagttggg gttgttacta 720

aggatgagcc caagtttagg aaattgacgg aacaagaaa 759

<210> 82
<211> 22
<212> DNA
<213> Artificial Sequence

<220>
<223> primer


<220>
<221> misc_feature
<222> (1)..(22)
<223> forward primer

<400> 82
atcatcctct gtgtttccgt tt 22


<210> 83
<211> 22
<212> DNA
<213> Artificial Sequence

<220>
<223> primer


<220>
<221> misc_feature
<222> (1)..(22)
<223> reverse primer

<400> 83
tttcttgttc cgtcaatttc ct 22


<210> 84
<211> 957
<212> DNA
<213> Diabrotica virgifera

<220>
<221>  misc_feature
<222>  (1)..(957)
<223>  WCRW transcript ta04410.001

<400>  84
ggggccccgg gggacagaag acagaacgtg acaacagaaa aatatagaaa atcatcctct      60

gtgtttccgt tttgaactta acttgtattt agtttaaaaa ataatcatgt ctagaggaag     120

cagtgcaggt tttgaccgac acataacaat tttctcgccc gaaggccgac tctatcaagt     180

agagtatgct tttaaagcca ttaaccaagc cggccccact tcggtagcag tccgaggagt     240

agatgctgcg gcgtgtgtga cccagagaaa gatcccggat aagctgattg atcccaacac     300

aattacacat ctgttccagt taacagaaca cactggatgt gtgatgactg gcatgattgc     360

tgacagcaag tcccaggtgc agagagctag atatgaggct gccgagttca aatataagtt     420

tggatatgag atgccaatcg atgccttgtg taggagagta tcggatattt cccaggttta     480

tacgcagaat gctgagatga gacctttggg ttgctccatg cttctgatag gatatgacca     540

agaaatggga ccatgtgtct acaaagctga ccctgctggc tattactgtg gctacagagc     600

agtaagtgta ggatccaaac aaactgaagc caacagctac ctagagaaga agctaaagaa     660

gaaaactgat ctgtccaaag atgaagtcat ccaattggca atcagttgtc tttcaagtgt     720

tttatctgta gattttaaac caacagaagt cgaagttggg gttgttacta aggatgagcc     780

caagtttagg aaattgacgg aacaagaaat agatttccat ctgacagcta ttgctgagag     840

ggattagata gattttttat attcactctt tgtaatgtta agaaatggtc aaattttgag     900

ttaagtaaga aataaatttt tctcttataa aaaaaaaaaa aaaaaaaaaa aaaaaaa       957


<210>  85
<211>  587
<212>  DNA
<213>  Diabrotica virgifera


<220>
<221>  misc_feature
<222>  (1)..(587)
<223>  RNAi target sequence

<400>  85
aactttgtgt gttttggcag gtccgatggc tgcaataaga aaaaaattgg tgattgttgg      60

tgacggagca tgtggaaaaa catgtctttt aatagtattt agtaaagatc agttcccaga     120

agtatacgtg cccactgtat tcgaaaacta tgtggcggat attgaagtag atggaaagca     180

ggtagaactc gccttatggg ataccgcagg tcaagaagat tatgatcgtc tgcggcccct     240

ttcatatcca gacaccgatg tgattttaat gtgcttctca gtagactccc ccgactcatt     300

agaaaacatc ccagaaaaat ggaccccaga ggtaaaacac ttttgtccaa atgtgccaat     360

tattctggta ggcaataaga aggatctgcg taacgaccct actaccatta acgaacttaa 420

aaagatgaaa caagaaccag taaaacctca agacggaaga tccatggcag agaaaatcaa 480

tgcttttgca tatctagaat gttctgccaa aagtaaggag ggtgtaagag aggtttttga 540

aaatgctacc cgagctgcac tgcaagtcaa aaagaagaag aagcccc 587

<210> 86
<211> 22
<212> DNA
<213> Artificial Sequence

<220>
<223> primer

<220>
<221> misc_feature
<222> (1)..(22)
<223> forward primer

<400> 86
aactttgtgt gttttggcag gt 22

<210> 87
<211> 20
<212> DNA
<213> Artificial Sequence

<220>
<223> primer

<220>
<221> misc_feature
<222> (1)..(20)
<223> reverse primer

<400> 87
ggggcttctt cttctttttg 20

<210> 88
<211> 1530
<212> DNA
<213> Diabrotica virgifera

<220>
<221> misc_feature
<222> (1)..(1530)
<223> WCRW transcript ta15897.001_diavv

<400> 88
   ggggatttaa tgtccgccat tttcgttcgt agttgaatag gcgttaactt tgtgtgtttt 60
   ggcaggtccg atggctgcaa taagaaaaaa attggtgatt gttggtgacg gagcatgtgg 120
   aaaaacatgt cttttaatag tatttagtaa agatcagttc ccagaagtat acgtgcccac 180
   tgtattcgaa aactatgtgg cggatattga agtagatgga aagcaggtag aactcgcctt 240
   atgggatacc gcaggtcaag aagattatga tcgtctgcgg cccctttcat atccagacac 300
   cgatgtgatt ttaatgtgct tctcagtaga ctcccccgac tcattagaaa acatcccaga 360
   aaaatggacc ccagaggtaa aacacttttg tccaaatgtg ccaattattc tggtaggcaa 420
   taagaaggat ctgcgtaacg atcctactac cattaacgaa cttaaaaaga tgaaacaaga 480
   accagtaaaa cctcaagacg gaagatccat ggcagagaaa atcaatgctt ttgcatatct 540
   agaatgttct gccaaaagta aggagggtgt aagagaggtt tttgaaaatg ctacccgagc 600
   tgcactgcaa gtcaaaaaga agaagaagcc ccgttgtgta atgttttgag ccactacata 660
   ttctatattt tatattaata atcgttacgt gtgttaatta atttcaaggg ggcttctgca 720
   tgatgtattg actaacaaaa taaaccccta cttttctctt cagttctatt tgttcaaact 780
   ccttttctcc ttgtggacaa atttaagtat taagttatat tttcatatgt atttaaccca 840
   ttgtttattg gttcttagag ttacacgacc cagaaattta attttaaaac aacacaacga 900
   tgtagcaagt tactttttag agtttaatat agaatagatc attattttgt ttctttattc 960
   cccaccaaac tagtattgca cattgcaatt atttggctat gaattttaat tgattaatgt 1020
   aaaattttga tttttgaaat atccttagtt gtctcgattt ttgatgaact tccagtgagg 1080
   ggtgtgtaac ttataagctt tttttataca atggtaaatg atataatcag tgtcatgtgc 1140
   acaaaactta ttgaacgtcc acttagatca tcattgtagt actaatttac aagcatacgt 1200
   tgattcattt tatagtattt atattttata acctgatcaa tatattttgt ttcaaaaatt 1260
   tttttctgta tctatagtta tcttttacag atattcagag tacccattga tttttattta 1320
   gttcaatgac tgcagcacat ttattatgtt ttgattagaa gtcactgcaa atgaaacaag 1380
   tggattaaaa cttttgacta acagatacac aaaaaaatgt ttgtgttgca gttattgcac 1440
   tgggactaaa aggactaaca gtgatgtcat ctcttgtgta agggtagtta ctaccacctg 1500
   ttgtccttta agtctcaaaa ttaattttga 1530

<210> 89
<211> 444
<212> DNA
<213> Diabrotica virgifera


<220>
<221> misc_feature
<222> (1)..(444)
<223> RNAi target sequence

<400> 89
   cggcgttgta aaccttttgt aaacattaaa cattttgaaa attactgtat tgctgcagcg 60
   atttacgtag aagtatttta agatgaatgc gcctccgacc tttgaatcgt ttttattata 120
   cgatggagag aaaaagatca ttctagaaca agatacgaaa gttccaaatg cagccatttt 180
   tactatcaac aaagaggacc acacacttgg taatacgatc agaaaccaac ttttaaaaga 240
   tcccaacgta ttattcgctg gttacaaact acctcatcca ttggaacaca aatctgtctt 300
   gcgaattcaa acaacatcag actattcacc tcaagaagcc ttaatgcacg ctatcactga 360
   tttaatttca gaattgtcct tgttcgaaga aagatttaag gaagcgatca aagaaaagaa 420
   ggaaggattg gattaggcag tgaa 444

<210> 90
<211> 21
<212> DNA
<213> Artificial Sequence

<220>
<223> primer

<220>
<221> misc_feature
<222> (1)..(21)
<223> forward primer

<400> 90
cggcgttgta aacctttgt a 21

<210> 91
<211> 22
<212> DNA
<213> Artificial Sequence

<220>
<223> primer

<220>
<221> misc_feature
<222> (1)..(22)
<223> reverse primer

<400> 91
ttcactgcct aatccaatcc tt 22

<210> 92
<211> 575
<212> DNA
<213> Diabrotica virgifera

<220>
<221> misc_feature
<222> (1)..(575)
<223> WCRW transcript ta06570.001_diavv

<400> 92
caaataacag gaaattgaca ctgacagttc gttggacagg aatgattttt tcggcgttgt 60

aaaccttttg taaacattaa acattttgaa aattactgta ttgctgcagc gatttacata 120

gaagtatttt aagatgaatg cgcctccgac ctttgaatcg tttttattat acgatggaga 180

gaaaaagatc attctagaac aagatacgaa agttccaaat gcagccattt ttactatcaa 240

caaagaggac cacacacttg gtaatatgat cagaaaccaa cttttaaaag atcccaacgt 300

attattcgct ggttacaaac tacctcatcc attggaacac aaatttgtct tgcgaattca 360

aacaacatca gactattcac ctcaagaagc cttaatgcac gctatcactg atttaatttc 420

agaattgtcc ttgttcgaag aaagatttaa ggaagcgatc aaagaaaaga aggaaggatt 480

ggattaggca gtgaattttt actttgattt agcttttgta attaaggtta atgtgactaa 540

taaatatagt tttattcaaa aaaaaaaaaa aaaaa 575

<210> 93
<211> 453
<212> DNA
<213> Diabrotica virgifera

<220>
<221> misc_feature
<222> (1)..(453)
<223> RNAi target sequence

<400> 93
ctggttgtca taggttgtac gcagttaaat tttttgtgat aaaacttagt gttcctttgt 60

tatcaattaa catatataaa aatagtaatg gctagtcgga agaagacttt gttgaaggtc 120

accatcctgg gtgattccag tgtgggaaag acttccttga tgaaccagta cgtaaatagg 180

aaattctcca accaatacaa agcgaccata ggtgctgatt tccttacaaa agaactcaca 240

gtggacgatc gaactgttac aatgcaaata tgggatactg caggtcagga aaggtttcag 300

tccttaggtg tagcctttta cagaggggca gactgctgtg tgttagtatt tgatgtaact 360

tcccccacct cattcaaatc cctggacagt tggcgagacg aattcctcat ccaagcatcg 420

ccccgagagc cagaaaactt cccattgacc tca 453

<210> 94
<211> 22
<212> DNA
<213> Artificial Sequence

<220>
<223> primer

<220>
<221> misc_feature
<222> (1)..(22)
<223> forward primer

<400>  94
ctggttgtca taggttgtac gc                                                22


<210>  95
<211>  22
<212>  DNA
<213>  Artificial Sequence

<220>
<223>  primer


<220>
<221>  misc_feature
<222>  (1)..(22)
<223>  reverse primer

<400>  95
tgaggtcaat gggaagtttt ct                                                22


<210>  96
<211>  640
<212>  DNA
<213>  Diabrotica virgifera


<220>
<221>  misc_feature
<222>  (1)..(640)
<223>  WCRW transcript ta40942.001_diavv

<400>  96
tagtaatggc tagtcggaag aagactttgt tgaaggtcac catcctgggt gattccagtg      60

tgggaaagac ttccttgatg aaccagtacg taaataggaa attctccaac caatacaaag     120

cgaccatagg tgctgatttc cttacaaaag aactcacagt ggacgatcga actgttacaa     180

tgcaaatatg ggatactgca ggtcaggaaa ggtttcagtc cttaggtgta gccttttaca     240

gaggggcaga ctgctgtgtg ttagtatttg atgtaacttc ccccacctca ttcaaatccc     300

tggacagttg gcgagacgaa ttcctcatcc aagcatcgcc ccgagagcca gaaaacttcc     360

cattgacctc aagcgttatt atccttataa tacccttggt accttaataa ctataagtac     420

aagaagattc cactataata ttgttacaaa ttaagaaagt aacatattag atatattcaa     480

tcctttatat ctctgaaaat attaaaaaaa gcatacatct gatttacaaa agaaataagt     540

tgtaaaaatg cataagctct cagctgtaaa tgtttttttg tgtcctttag tattattcca     600

gtgtttttaa tatatctcat aatattggaa tattttttaa                           640


<210>  97
<211>  601
<212>  DNA
<213>  Diabrotica virgifera

<220>
<221>  misc_feature
<222>  (1)..(601)
<223>  RNAi target sequence

<400>  97
gtgaacgcca ttgtgaactc taacgacttt ggaaccaagc tgaagtaatc cgtgattttt      60

tgtgccatcg gtgtttccta gtacttttta ttcacaagtt taaattcaaa aaatcatggc     120

tggcgaacaa gatatgccca ccttcaaatg cgtcttggtc ggagatggag gtactggcaa     180

aactaccttt gtaaaaagac acatgactgg tgaattcgaa aagaaatatg tagcaacctt     240

aggagtagaa gttcaccctt tagttttcca cacaaataga ggacaaattc gttttaacgt     300

atgggacaca gcaggccaag aaaagtttgg tggtctgcgt gatggatact acatccaagg     360

acaatgtgcc ataattatgt ttgatgttac atctagagtc acttacaaaa atgttccaaa     420

ctggcacaga gatcttgtta gagtttgtga aaatatccca attgtattgt gtggtaacaa     480

agttgacatc aaagatagga aagttaaggc aaagagcatt gtcttccata ggaagaagaa     540

tttacaatac tatgacatct ctgcaaaatc caactacaac tttgaaaaac ccttcttgtg     600

g                                                                     601


<210>  98
<211>  22
<212>  DNA
<213>  Artificial Sequence

<220>
<223>  primer


<220>
<221>  misc_feature
<222>  (1)..(22)
<223>  forward primer

<400>  98
gtgaacgcca ttgtgaactc ta                                               22


<210>  99
<211>  22
<212>  DNA
<213>  Artificial Sequence

<220>
<223>  primer


<220>
<221>  misc_feature
<222>  (1)..(22)
<223>  reverse primer

<400>  99
ccacaagaag ggtttttcaa ag                                               22

<210>  100
<211>  1075
<212>  DNA
<213>  Diabrotica virgifera

<220>
<221>  misc_feature
<222>  (1)..(1075)
<223>  WCRW transcript ta05966.001_diavv

<400>  100
   tatttggcgc gcattcctga cacctacgcg ggtcttcttg tgaacgccat tgtgaactct      60
   aacgactttg gaaccaagct gaagtaatcc gtgatttttt gtgccatcgg tgtttcctag     120
   tactttttat tcacaagttt aaattcaaaa aatcatggct ggcgaacaag atatgcccac     180
   cttcaaatgc gtcttggtcg gagatggagg tactggcaaa actacctttg taaaaagaca     240
   catgactggt gaattcgaaa agaaatatgt agcaacctta ggagtagaag ttcacccttt     300
   agttttccac acaaatagag gacaaattcg ttttaacgta tgggacacag caggccaaga     360
   aaagtttggt ggtctgcgtg atggatacta catccaagga caatgtgcca taattatgtt     420
   tgatgttaca tctagagtca cttacaaaaa tgttccaaac tggcacagag atcttgttag     480
   agtttgtgaa aatatcccaa ttgtattgtg tggtaacaaa gttgacatca aagataggaa     540
   agttaaggca aagagcattg tcttccatag gaagaagaat ttacaatact atgacatctc     600
   tgcaaaatcc aactacaact ttgaaaaacc cttcttgtgg ttggctagga aactcattgg     660
   agatcccaac ttagaattcg tagctgcacc tgccttgctt cctccagaag tccagatgga     720
   tccccaatgg cagcaacaaa tcgaaaagga tcttaaagaa gcctcagaaa ctgcactgcc     780
   cgatgatgac gaagatttat aaactcatta accaattact aaaactctgt catcgttcca     840
   aagcgcgtgt gtaacaattg atatcttaca agacttaagt taaattacat tttatgtatt     900
   tctgaagcag aactgttgat atttttatat aaatgttttc taaatttgtt gaaaggtttg     960
   tctggagtct ggacgtatgt tcttcaatat attagtggta tggttgttag agtgttagag    1020
   tcagcaagct accgtaacag ttcttgtcac ataggagtgt agtcttaatt tcaat         1075

<210>  101
<211>  614
<212>  DNA
<213>  Diabrotica virgifera

<220>
<221>  misc_feature
<222>  (1)..(614)
<223>  RNAi target sequence

<400>  101

caacaatttt cgcatctttc tgtctgttcc cggcgttctt tgaaaaagtt ttcaataata      60

ctctaaaaaa tggcagacgc tgatgatcta ttagattatg aagatgagga acagacagaa      120

caaaccgcaa ctgaaacggc aactacagag gtacagaaaa agggtgtcaa gggcacatat      180

gtatcaatac acagttctgg gtttagagat tttctgttaa aaccagcaat tctcagagct      240

atagtggact gcgggttcga acatccttca gaagttcaac atgaatgtat tcctcaagct      300

gtcattggca tggatattct gtgccaagct aaatccggta tgggaaaaac ggctgttttt      360

gtattagcta cactccaagt aatagatcct acagaaaatg ttgtatatgt tctcgtcatg      420

tgccatacca gagagttagc cttccagata agcaaagagt acgaacgttt cagtaaatat      480

atgcccaata ttaaagtagg ggtcttcttt ggtggcttgc ctatccagaa agatgaggaa      540

acgttaaaaa ataattgccc gcatatcgtt gtgggtactc caggaagaat tttagcattg      600

gtcagatcga aaaa                                                        614

<210>  102
<211>  22
<212>  DNA
<213>  Artificial Sequence

<220>
<223>  primer

<220>
<221>  misc_feature
<222>  (1)..(22)
<223>  forward primer

<400>  102
caacaatttt cgcatctttc tg                                               22

<210>  103
<211>  22
<212>  DNA
<213>  Artificial Sequence

<220>
<223>  primer

<220>
<221>  misc_feature
<222>  (1)..(22)
<223>  reverse primer

<400>  103
tttttcgatc tgaccaatgc ta                                               22

<210>  104
<211>  1515
<212>  DNA
<213>  Diabrotica virgifera

<220>
<221>  misc_feature
<222>  (1)..(1515)
<223>  WCRW transcript ta03347.002_diavv

<400>  104
ttggaaaacg caatatatct atagaaaaca agcggtttat ttgccgccat tttgacaagc      60

ttacgtacct acatttttaa ttcgttgttt tatttttagt tttcaataat actctaaaaa     120

atggcagacg ctgatgatct attagattat gaagatgagg aacagacaga acaaaccgca     180

actgaaacgg caactacaga ggtacagaaa aagggtgtca agggcacata tgtatcaata     240

cacagttctg ggtttagaga ttttctgtta aaaccagcaa ttctcagagc tatagtggac     300

tgcgggttcg aacatccttc agaagttcaa catgaatgta ttcctcaagc tgtcattggc     360

atggatattc tgtgccaagc taaatccggt atgggaaaaa cggctgtttt tgtattagct     420

acactccaag taatagatcc tacagaaaat gttgtatatg ttctcgtcat gtgccatacc     480

agagagttag ccttccagat aagcaaagag tacgaacgtt tcagtaaata tatgcccaat     540

attaaagtag gggtcttctt tggtggcttg cctatccaga aagatgagga aacgttaaaa     600

aataattgcc cgcatatcgt tgtgggtact ccaggaagaa ttttagcatt ggtcagatcg     660

aaaaaactta atctcaaaca tctaaagcat tttattttgg atgaatgtga taaaatgttg     720

gagttattag acatgagacg tgatgttcaa gaaatatatc gtaacactcc ccacgaaaaa     780

caagtcatga tgttcagtgc caccttaagt aaagaaatta gaccagtttg caagaaattt     840

atgcaagatc ccatggaagt atatgttgac gacgaagcta aattaacatt gcatggtcta     900

cagcaacatt atgtaaaact aaaagaaaac gaaaagaata aaaagttatt tgagttacta     960

gatgttttgg agttcaacca ggttgtcatt ttcgtaaaat ccgtacaaag gtgtgtggcc    1020

ctggcacaat tactgactga acagaacttc ccagcgatag gaattcacag aggaatggac    1080

caaaaagaac gactgtcccg gtatgaacaa ttcaaggatt tccaaaagag gattctagta    1140

gccacaaact tatttggacg tggaatggat atcgaaagag tgaacatcgt cttcaactat    1200

gacatgcccg aagattctga cacttacctg cacagagtag cccgagctgg acgtttcggt    1260

accaaaggtc tagccattac tttcgtatca gaagagggag atgccaaaat attgaacgaa    1320

gtacaagaaa gatttgatgt taatattact gaattacctg acgaaataga tctcagttca    1380

tacattgaag gacgataaga atttcaagat ctttgaagaa ttaattttaa gatttagaat    1440

aagatttcca ctaggtttaa cgatttcaat ttgtattatg tttcaatgtt attgcattag    1500

tattaaaata aatgg                                                     1515

<210>  105
<211>  683

<212>  DNA
<213>  Diabrotica virgifera


<220>
<221>  misc_feature
<222>  (1)..(683)
<223>  RNAi target sequence

<400>  105
ggcatcttgt ctcgtttatc gtaactcaaa ttaaacgtaa agatgtcggg aggaatcgag      60

gctttaagcc ttaaagagga cgatgttacc aagatgttgg cggcttccac ccatcttgga     120

acagcaaatg ttgattttca aatggaacag tatgtataca aacgtcgtca tgacggtact     180

cacatcatca acttaaggaa aacgtgggaa aagattctct tggcagctcg tgccattgtt     240

gctattgaac accctggtga agttttcgta atttcatctc gtccccatgg ccagcgtgct     300

gttcttaagt ttgctgctca cactggagct acccctattg ctggaagatt tactcctggt     360

gcttttacca atcagatcca agctgcattc agggaacctc gtctgttgat cgtcactgac     420

cctgcttttg atcaccaacc tatcactgaa gcctcttacg taaacattcc agttattgct     480

ttgtgcaata ctgattctcc attacgtttt gttgatattg ccattcccgg aaacaacaaa     540

gcctcacaca gtattggttt actgtggtgg cttttggcta gagaggttct ccgtcttaga     600

ggaatcattc cacgggatac caaatgggat gttgtagtag atcttttctt ctaccgtgaa     660

ccagaagagg ctgaaaagga aga                                             683


<210>  106
<211>  22
<212>  DNA
<213>  Artificial Sequence

<220>
<223>  primer


<220>
<221>  misc_feature
<222>  (1)..(22)
<223>  forward primer

<400>  106
ggcatcttgt ctcgtttatc gt                                               22


<210>  107
<211>  22
<212>  DNA
<213>  Artificial Sequence

<220>
<223>  primer


<220>

<221>  misc_feature
<222>  (1)..(22)
<223>  reverse primer

<400>  107
tcttcctttt cagcctcttc tg                                                22


<210>  108
<211>  822
<212>  DNA
<213>  Diabrotica virgifera


<220>
<221>  misc_feature
<222>  (1)..(822)
<223>  WCRW transcript ta15911.001_diavv

<400>  108
gggggacaga gacaggacat aagcattaaa agggacgtaa aaacaacaaa ggggctcttt      60

ctcgtgcaaa taacccaagt ggcatcttgt ctcgtttatc gtaactcaaa ttaaacgtaa     120

agatgtcggg aggaatcgag gctttaagcc ttaaagagga cgatgttacc aagatgttgg     180

cggcttccac ccatcttgga acagcaaatg ttgattttca aatggaacag tatgtataca     240

aacgtcgtca tgacggtact cacatcatca acttaaggaa aacgtgggaa aagattctct     300

tggcagcccg tgccattgtt gctattgaac accctggtga agttttcgta atttcatctc     360

gtccctatgg ccagcgtgct gttcttaagt ttgctgctca cactggagct acccctattg     420

ctggaagatt tactcctggt gcttttacca atcagatcca agctgcattc agggaacctc     480

gtctgttgat cgtcactgac cctgcttttg atcaccaacc tatcactgaa gcctcttacg     540

taaacattcc agttattgct ttgtgcaata ctgattctcc attacgtttt gttgatattg     600

ccattcccgg aaacaacaaa gcctcacaca gtattggttt actgtggtgg cttttggcta     660

gagaggttct ccgtcttaga ggaatcattc cacgggatac caaatgggat gttgtagtag     720

atcttttctt ctaccgtgaa ccagaagagg ctgaaaagga agaacaagct gccaaagaag     780

ctgtggctgc tgttgtcaaa cctgtagata tcccagctcc tc                        822


<210>  109
<211>  559
<212>  DNA
<213>  Diabrotica virgifera


<220>
<221>  misc_feature
<222>  (1)..(559)
<223>  RNAi target sequence

<400>  109
ggatggttta ttgtctggaa gctcattcta agtagattcc gttttattaa agaactgttg      60

aagggagcaa gtgactctac aattacggaa ttgaaaaaca ctaggagtaa actcaagagg 120

acaagaaagg actgatttat cttctcaaaa tgaatattcg atgtgcaaaa ccagaagatc 180

ttatgaacat gcagcactgc aatctgttat gtctaccaga aaactatcag atgaaatatt 240

acttttatca tggccttagt tggccccaac taagttatgt agctgaagac gaaaaaggta 300

acatagttgg ttatgtccta gcaaaaatgg aagaagacca agaagatctc aagcatggtc 360

acataacctc cctggcagtg aagcgatccc acaggcgatt agggctagcc cagaaactga 420

tggatcaagc ttcagaagcc atggtcgaat gtcttgacgc aaaatatgtt tcgctgcatg 480

ttcgtaaaag caatcgcgcc gctttaaatc tatacaaaaa ctctttgaaa tttgaaactg 540

tcgaaattga gccaaaata 559

<210> 110
<211> 22
<212> DNA
<213> Artificial Sequence

<220>
<223> primer


<220>
<221> misc_feature
<222> (1)..(22)
<223> forward primer

<400> 110
ggatggttta ttgtctggaa gc 22


<210> 111
<211> 22
<212> DNA
<213> Artificial Sequence

<220>
<223> primer


<220>
<221> misc_feature
<222> (1)..(22)
<223> reverse primer

<400> 111
tattttggct caatttcgac ag 22


<210> 112
<211> 903
<212> DNA
<213> Diabrotica virgifera


<220>
<221> misc_feature

<222>   (1)..(903)
<223>   WCRW transcript ta05802.003_diavv

<400>   112
aaatatgcac ttgtgacggc ctttgaccta aatgtttagt taggaacaaa atggcgaaat      60

gttggaactg ataaacatca ctgccttttt gtaatagtta agaattagat ttttaattgg     120

aaaattgtaa tgtagcaaat tacttaagcg cctaactcca agacccatta atgcattcag     180

gttaaacagt ttattttatt taggatggtt tattgtctgg aagctcattc taagtagatt     240

ccgttttatt aaagaactgt tgaagggagc aagtgactct acaattacgg aattgaaaaa     300

cactaggagt aaactcaaga ggacaagaaa ggactgattt atcttctcaa aatgaatatt     360

cgatgtgcaa aaccagaaga tcttatgaac atgcagcact gcaatctgtt atgtctacca     420

gaaaactatc agatgaaata ttacttttat catggcctta gttggcccca actaagttat     480

gtagctgaag acgaaaaagg taacatagtt ggttatgtcc tagcaaaaat ggaagaagac     540

caagaagatc tcaagcatgg tcacataacc tccctggcag tgaagcggtc ccacaggcga     600

ttagggctag cccagaaact gatggatcaa gcttccgaag ccatggtcga atgttttgac     660

gcaaaatatg tttcgctgca tgttcgtaaa agcaatcgcg ccgctttaaa tctatacaaa     720

aactctttga aatttgaaac tgtcgaaatt gagccaaaat attatgcaga tggtgaagat     780

gcatattcca tgagacgaga cttatctgaa ttcgctaaga aagttaaacc taagtctgag     840

actaaaaacg aagaatagtg taattatttg taatttttgt atttatagcg aatataatat     900

aca                                                                   903


<210>   113
<211>   368
<212>   DNA
<213>   Diabrotica virgifera


<220>
<221>   misc_feature
<222>   (1)..(368)
<223>   RNAi target sequence

<400>   113
ggttgcagaa gatttcacaa aactttatta tgatactctg gataaacgac ggcatttgat      60

agcaagactg tatttggaga cgggattatt atcatggaat ggtaatggtg taactgggaa     120

tgaaaatatt ttgaaattta tgattgattt accaccgtca tcttttacta tacaaactct     180

agacgcacag cctgttttag attctgcagt aaatggacaa ctgaccttca tcactcaagt     240

gactggcagt gtaaaatacc aagagaaaac accaaaaacc tttcagcgaa acttcataat     300

tacagcacag ggcgataaat ggaaaattgt cagtgattgt atgaggctgc aagaaccatt     360

aaacgcta                                                              368

<210>  114
<211>  21
<212>  DNA
<213>  Artificial Sequence

<220>
<223>  primer


<220>
<221>  misc_feature
<222>  (1)..(21)
<223>  forward primer

<400>  114
gttgcagaag atttcacaaa a                                                21


<210>  115
<211>  22
<212>  DNA
<213>  Artificial Sequence

<220>
<223>  primer


<220>
<221>  misc_feature
<222>  (1)..(22)
<223>  reverse primer

<400>  115
tagcgtttaa tggttcttgc ag                                               22


<210>  116
<211>  470
<212>  DNA
<213>  Diabrotica virgifera


<220>
<221>  misc_feature
<222>  (1)..(470)
<223>  WCRW transcript ta08838.001_diavv

<400>  116
atttatgaga aatggaagat ttaaggctaa aaattgatca ggcctgtaag gttgcagaag      60

atttcacaaa actttattat gatactctgg ataaacgacg gcatttgata gcaagactgt     120

atttggagac gggattatta tcatggaatg gtaatggtgt aactgggaat gaaaatattt     180

tgaaatttat gattgattta ccaccgtcat cttttactat acaaactcta gacgcacagc     240

ctgttttaga ttctgcagta aatggacaac tgaccttcat cactcaagtg actggcagtg     300

taaaatacca agagaaaaca ccaaaaacct ttcagcaaaa cttcataatt acagcacagg     360

gcgataaatg gaaaattgtc agtgattgta tgaggctgca agaaccatta aacgctaaga     420

actaacaact gtatttttaa gtattttgat aaataaagta attggtagac 470

<210> 117
<211> 589
<212> DNA
<213> Diabrotica virgifera

<220>
<221> misc_feature
<222> (1)..(589)
<223> RNAi target sequence

<400> 117
acctggctga tgcaatagat ttggtaaaag ggttttctac ctataaattt acaatatatt 60

attataaata gactaaactc atcaataaaa aagtttaagc aaatagccat atagctacaa 120

tggccgaaga cgatgcaatc tttgatccaa ccctcaaaaa aaagaaaaag aagaagaaga 180

ccactttcga cattgatgct gctctagctg aaggtaacat tgaaaatgat tcagggaatg 240

ccccagctgc agaagctgat aatattgaat caaaacctga cgatgatcag gatgatggaa 300

atgtggacat tgatttagat ttcactaaaa agaaaaagaa gaagaaaaag cctttctcag 360

ctgatgattt agattcttcc ccagctgata ataaagctga aactactcaa gatggagctg 420

gggacaatga tgggggtatg gatgataact ttgatttaga tatggacttt agtaaaaaga 480

agaagaagaa aaccaagaaa cctaagaatt tagacgaact tattgcagaa actgaggaga 540

aggatgatga tcaggagaat gtagatgaaa attcaacaca atgggtcaa 589

<210> 118
<211> 22
<212> DNA
<213> Artificial Sequence

<220>
<223> primer

<220>
<221> misc_feature
<222> (1)..(22)
<223> forward primer

<400> 118
acctggctga tgcaatagat tt 22

<210> 119
<211> 22
<212> DNA
<213> Artificial Sequence

<220>
<223> primer

<220>
<221>  misc_feature
<222>  (1)..(22)
<223>  reverse primer

<400>  119
ttgacccatt gtgttgaatt tt                                                22


<210>  120
<211>  1418
<212>  DNA
<213>  Diabrotica virgifera


<220>
<221>  misc_feature
<222>  (1)..(1418)
<223>  WCRW transcript ta00006.001_diavv

<400>  120
   ggggtggaga agggtttcgt gtgaacagcc gttgcacacg agtcagtcga tcctaagccc      60
   taggcgaaag ccgatgttga tgtggttggg ggacaacctg gctgatgcaa tagatttggt     120
   aaaagggttt tctacctata aatttacaat atattattat aaatagacta aactcatcaa     180
   taaaaaagtt taagcaaata gccatatagc tacaatggcc gaagacgatg caatctttga     240
   tccaaccctc aaaaaaaaga aaaagaagaa gaagaccact ttcgacattg atgctgctct     300
   agctgaaggt aacattgaaa atgattcagg gaatgcccca gctgcagaag ctgataatat     360
   tgaatcaaaa cctgacgatg atcaggatga tggaaatgtg gacattgatt tagatttcac     420
   taaaaagaaa aagaagaaga aaaagccttt ctcagctgat gatttagatt cttccccagc     480
   tgataataaa gctgaaacta ctcaagatgg agctggggac aatgatgggg gtatggatga     540
   taactttgat ttagatatgg actttagtaa aaagaagaag aagaaaacca agaaacctaa     600
   gaatttagac gaacttattg cagaaactga ggagaaggat gatgatcagg agaatgtaga     660
   tgaaaattca acacaatggg tcaattccga tcgggactac acatatgacg aattactaaa     720
   ccgtgccttt gaaataatga gagataaaaa tccagatatg gctgccggta agaaacagaa     780
   gttcgtcatg agacctcctc aagtagtcaa aatcggcgcc aaaaagacgt catttgcgaa     840
   tttcacagag atatgcaaaa tgcttcatcg tcaacccaag catcttttag attttttact      900
   agctgaattg ggtaccagcg ggtctgtgga cggtaataat cagctcatca ttaaaggaag     960
   gtttcaacag aaacaaattg aaaatgtact caggagatac atcaaggaat acgtcacttg    1020
   tcatacatgt agatccccag atacaattct tcaaaaagac actagattgt tcttcttaca    1080
   atgtgaaacg tgtggttcta ggtgttctgt tgctagtatt aaatcaggtt tccaggctgt    1140
   cacttccaaa cgtgctgcaa ttcgagccaa gacagcatag gaaccttaga tattgcaata    1200
   caataataaa taggcactgt tacagaagaa atttcaaagt gctaaagaat aggtgttgta    1260
   tgtgttatat aatttgtact ataaatctag ctgaattgtt aaatggaatt aacaatcata 1320
   ttaatatcaa caattttaat tatataactt ttttattgat tattaaattt taataaacag 1380
   taaataatta tttatatttt tcgtccataa aaaaaaaa 1418

<210> 121
<211> 610
<212> DNA
<213> Diabrotica virgifera

<220>
<221> misc_feature
<222> (1)..(610)
<223> RNAi target sequence

<400> 121
gtagcggaac cgtcaaaaag tcgggccctc gcaagagagt tcgtcggggt aacccaaaag 60

gacctggaga cgtcgtcgag agatccggga agaatctcgc actcttctcg aacaagccga 120

ccgtgctcgt cgccaagctg aacaagaatt gggagatgct cacgaacaat tgaacgatct 180

tggagcccaa aacgcttcct tgtctgccgc caagaggaaa ttggaaaccg aattgcaaac 240

cctccactca gaccttgacg aacttctcaa cgaagccaag aactccgaag agaaggccaa 300

gaaggctatg gtagatgccg ctcgtctcgc tgatgaactc cgcgctgaac aagaccatgc 360

ccaaacacaa gagaaactcc gtaaagctct cgaagctcaa atcaaggacc tccaagtacg 420

cttggacgaa gctgaagcca atgcccttaa aggaggaaag aaggctattg ccaaattgga 480

acaacgtgtt agagaattag aaaatgaatt agacggtgaa caaaggcggc acgcggatgc 540

ccaaaagaac ttgagaaagt ctgaacgtcg tatcaaggaa cttagcttcc aagctgaaga 600

agaccgcaag 610

<210> 122
<211> 21
<212> DNA
<213> Artificial Sequence

<220>
<223> primer

<220>
<221> misc_feature
<222> (1)..(21)
<223> forward primer

<400> 122
gtagcggaac cgtcaaaaag t 21

<210> 123
<211> 20
<212> DNA

<213>  Artificial Sequence

<220>
<223>  primer


<220>
<221>  misc_feature
<222>  (1)..(20)
<223>  reverse primer

<400>  123
cttgcggtct tcttcagctt                                                   20


<210>  124
<211>  669
<212>  DNA
<213>  Diabrotica virgifera


<220>
<221>  misc_feature
<222>  (1)..(669)
<223>  WCRW transcript ta41130.001_diavv

<400>  124
gggcacaccc attgggcgaa agggaatccg gttcctattc cggaacccgg tagcggaacc      60

gtcaaaaagt cgggccctcg caagagagtt cgtcggggta acccaaaagg acctggagac     120

gtcgtcgaga gatccgggaa gaatctcgca ctcttctcga acaagccgac cgtgctcgtc     180

gccaagctga acaagaattg ggagatgctc acgaacaatt gaacgatctt ggagcccaaa     240

acgcttcctt gtctgccgcc aagaggaaat tggaaaccga attgcaaacc ctccactcag     300

accttgacga acttctcaac gaagccaaga actccgaaga gaaggccaag aaggctatgg     360

tagatgccgc tcgtctcgct gatgaactcc gcgctgaaca agaccatgcc caaacacaag     420

agaaactccg taaagctctc gaagctcaaa tcaaggacct ccaagtacgc ttggacgaag     480

ctgaagccaa tgcccttaaa ggaggaaaga aggctattgc caaattggaa caacgtgtta     540

gagaattaga aaatgaatta gacggtgaac aaaggcggca cgcggatgcc caaaagaact     600

tgagaaagtc tgaacgtcgt atcaaggaac ttagcttcca agctgaagaa gaccgcaaga     660

accacgaga                                                             669


<210>  125
<211>  628
<212>  DNA
<213>  Diabrotica virgifera


<220>
<221>  misc_feature
<222>  (1)..(628)
<223>  RNAi target sequence

<400>  125
ccagtattta gaccggcatc tcgtttttcc cttattggaa tttctgtccg caaaaggaat      60

ttacaatgag accgaactgt taaaagccaa attagatatt ctaagcaaga ctaatatgat     120

agattatgct attgatatca gaaaacaact gtaccctgat caggaaatac ccgaggacct     180

aaaacagagg cgtaatcatg ttgtgcagca gctagcagag ttgcaagaag aggttaagcc     240

tatccttaag attatggaat ctgaagaagt tatgaagaat atggaaaaca tgagagattc     300

gaaaacactt attacatatt tgtcaaaaga attcaagttt gaaattgaat tgatcgacag     360

tcttcacaaa ctagccaaat acagatacga ctgtggcaac tacacagtat ctacatccta     420

cctgtacttc tgtatgctgg tattacctcc taatgacagg aactatctta gtgccttgtg     480

gggaaaattc gcttcagaaa ttctgtacca aaactgggat tcagccctgg aagatctcaa     540

caaactgagg gaatatattg attccagtcc caatcagttt tctggcaaca gtttgcagct     600

tctccaacag aggacttggt tgatccat                                        628


<210>  126
<211>  22
<212>  DNA
<213>  Artificial Sequence

<220>
<223>  primer


<220>
<221>  misc_feature
<222>  (1)..(22)
<223>  forward primer

<400>  126
ccagtattta gaccggcatc tc                                               22


<210>  127
<211>  22
<212>  DNA
<213>  Artificial Sequence

<220>
<223>  primer


<220>
<221>  misc_feature
<222>  (1)..(22)
<223>  reverse primer

<400>  127
atggatcaac caagtcctct gt                                               22


<210>  128
<211>  1547
<212>  DNA

<213>  Diabrotica virgifera

<220>
<221>  misc_feature
<222>  (1)..(1547)
<223>  WCRW transcript ta05495.001_diavv

<400>  128
tttttagagg ctacttctcg tagctctgct tgttgaattt taaaatattg aaagtagtaa      60

atattgaaga aagtattaat aacacaatca tggcaaagtt cgacttaact tcgaagatgg     120

gccagtattt agaccggcat ctcgtttttc ccttattgga atttctgtcc gcaaaaggaa     180

tttacaatga gaccgaactg ttaaaagcca aattagatat tctaagcaag actaatatga     240

tagattatgc tattgatatc agaaaacaac tgtaccctga tcaggaaata cccgaggacc     300

taaaacagag gcgtaatcat gttgtgcagc agctagcaga gttgcaagaa gaggttaagc     360

ctatccttaa gattatggaa tctgaagaag ttatgaagaa tatggaaaac atgagagatt     420

cgaaaacact tattacatat ttgtcaaaag aattcaagtt tgaaattgaa ttgatcgaca     480

gtcttcacaa actagccaaa tacagatacg actgtggcaa ctacacagta tctacatcct     540

acctgtactt ctgtatgctg gtattacctc ccaatgacag gaactatctt agtgccttgt     600

ggggaaaatt cgcttcagaa attctgtacc aaaactggga ttcagccctg gaagatctca     660

acaaactgag ggaatatatt gattccagtc ccaatcagtt ttctggcaac agtttgcagc     720

ttctccaaca gaggacttgg ttgatccatt ggtctctctt tgtgtttttc aatcatgtta     780

tgggcagaga attgattatt gaaatgttct tgtatagacc acactacctc aacgccatcc     840

agaccatgtg tcctcatatc ttgaggtatt tggctacagc tgtaattatt aatcgtggaa     900

ggagatcagc actgaaggac ttagttaaag ttattcagca agaaagctac acttacagag     960

atcccattac agagtttgtg gaacaccttt acgtcaactt cgatttcgat ggagcccgtc    1020

aaaaattaca cgaatgccaa acagttttac taaacgactt cttcctaatt tcctgtttag    1080

acgaattcgt agaaaacgcc agacttatga tcttcgagac attctgcaga attcatcaat    1140

gcatcagtat aggcatgtta gccgaaaagc tcaacatgaa tccagacgaa gctgaatgct    1200

ggatcgtgaa tttgatcaga aacgcacgat tggatgctaa gatcgattct aagttagggc    1260

atgtggttat gggtgctcaa cctttatcgc cataccagca acttattgaa aaaattgatt    1320

ccctttcggt caggtcagaa actctgtgtg ctcttataga tagaaagctc agagcgagga    1380

ctgacgtaag tttctaaagg tacgttaacg aaatgatgca agaatttgtc atttttgtta    1440

cagatacgtt ggggaaatcc agatttttaa atatctgtta ttttgtaata attttattgg    1500

atacaaataa aagatctttc cagtatgtca aaaaaaaaaa aaaaaaa                  1547

<210>  129

<211>  619
<212>  DNA
<213>  Diabrotica virgifera

<220>
<221>  misc_feature
<222>  (1)..(619)
<223>  RNAi target sequence

<400>  129
tcacattcca gttgtttaga gcacttagta gagatataca cctttaagca tgtcgtgttc      60

agcgatatat attttagatg taaaaggaaa agtcttaatc tctaggaact atagaggcga     120

tatagaccta ggtgtaatag agaagtttat gcctttatta atggaaaaag aagaagaagg     180

gcttctaact ccaattttgc aaatccctga atgcaccttc tcatatatta aaaccaacaa     240

tctatatata gtaagcacta cgaagaaaaa tggtaatatc gctcttgtat ttgtattttt     300

acataaaatc gttcaagtaa tgactgaata ttttaaagaa ttagaagaag aaagtatcag     360

agataatttt gtcgttattt atgagttgtt ggacgaactt ttagactttg gctatcctca     420

aacaactgat agcaaaattc ttcaagaata catcactcaa gaaggtcata aactagagat     480

acaaccaaga attcctgtag ctgtgacaaa tgcagtttca tggcgttctg agggaatcaa     540

atacaggaaa aatgaagtat tcttagatgt aatcgagtca gtaaatcttt tagcaagtgc     600

aaatggcaat gtactaaga                                                  619

<210>  130
<211>  20
<212>  DNA
<213>  Artificial Sequence

<220>
<223>  primer

<220>
<221>  misc_feature
<222>  (1)..(20)
<223>  forward primer

<400>  130
tcacattcca gttgtttaga                                                  20

<210>  131
<211>  20
<212>  DNA
<213>  Artificial Sequence

<220>
<223>  primer

<220>
<221>  misc_feature

<222>   (1)..(20)
<223>   reverse primer

<400>   131
tcttagtaca ttgccatttg                                                   20


<210>   132
<211>   2131
<212>   DNA
<213>   Diabrotica virgifera


<220>
<221>   misc_feature
<222>   (1)..(2131)
<223>   WCRW transcript ta08478.001_diavv

<400>   132
agtaaataat aatgattata atatggtttg caaatagtta aatgcagtat gtgattttct      60

gaagaagtga agaggttata tcacattcca gttgtttaga gcacttagta gagatataca     120

cctttaaaca tgtcgtgttc agcgatatat attttagatg taaaaggaaa agtcttaatc     180

tctaggaact atagaggcga tatagaccta ggtgtaatag agaagtttat gcctttatta     240

atggaaaaag aagaagaagg gcttctaact ccaattttgc aaatccctga atgcaccttc     300

tcatatatta aaaccaacaa tctatatata gtaagcacta cgaagaaaaa tggtaatatc     360

gctcttgtat ttgtattttt acataaaatc gttcaagtaa tgactgaata ttttaaagaa     420

ttagaagaag aaagtatcag agataatttt gtcgttattt atgagttgtt ggacgaactt     480

ttagactttg gctatcctca aacaactgat agcaaaattc ttcaagaata catcactcaa     540

gaaggtcata aactagagat acaaccaaga attcctgtag ctgtgacaaa tgcagtttca     600

tggcgttctg agggaatcaa atacaggaaa aatgaagtat tcttagatgt aatcgagtca     660

gtaaatcttt tagcaagtgc aaatggcaat gtactaagaa gcgaaatagt gggagcaata     720

aaaatgagag tgtatctttc tggtatgcct gaactgagat taggtctcaa tgataaagta     780

ttattcgaga gtacaggacg tggtaaatct aaatcggtag agttagatga tgttaagttc     840

catcaatgtg taagattatc aagattcgag atagatagaa caatctcttt tattccacca     900

gatggtgaat ttgaattaat gtcttatcgt ttaaataccc atgtgaaacc attaatatgg     960

attgaatctg ttatagaaag acatgcgcat agtagagtgg agtacatgat taaagctaaa    1020

tcgcaattca agagacgttc tactgctaac aatgttgaaa taatcattcc agtgccacat    1080

gatgcagact ctccgaagtt taaaaccacc atcggcagtg tcaagtatgc ccctgaacaa    1140

aatgccataa cttggagtat caaatcattc ccagggggta aagagtattt gatgagagcc    1200

cactttggtt tacctagtgt tgaatgtgaa gatactgagg gtaaaccacc tattcaggtc    1260

aaatttgaaa ttccatattt tacaacttct ggaatacaag tcagatactt gaagattata    1320

gagaaaagtg ggtatcaagc tctaccatgg gtgagatata ttacacagaa tggtgactac 1380

cagttgcgta ctaattaagc gttgttattg tacttttctg ctttttaagt tagaatataa 1440

tatattttta cttatacttt atttgtaggg tcttataaaa ctttgtacta tgtagcacta 1500

tattcagtta atatatttta aaataaacat tactttttca tgtactgttc tcacaattca 1560

ttataacttg tttcagcatt atctatttca aaatatttaa agtatgtact taaacatttg 1620

acattgaata aatttaccta ttttattctt aaattttggg tgctttgaga caagctcagc 1680

ggttggacaa ctgagtaagc ggtagcgttt agacaacaca atgacaagcc actgttaaat 1740

ttactaagcg tattttcttt ctgctgtgta actcagtggt tgcttgggta cagtacgcaa 1800

taatgagttt aaattttttt aaacttttca taaaatattc aattgaccca aaaaatggat 1860

ttacagtgga ataagagaag tacattttgg tactgtacga gaatatttat ttctagaagt 1920

attatgtaaa tattatgcaa atatgtttat tactactgga tctaagccgt ttttgtagtt 1980

aatgtcagcc actgaggtcg tctgaatgta cccattggtt atcctgtctt ttatcaagtt 2040

gccatgcctt caagcgtcgg aaaagttttg attttaagag agtaggcgca aaatttaact 2100

ggattcattt ttttcgaatc ctgagaaaac t 2131

<210> 133
<211> 660
<212> DNA
<213> Diabrotica virgifera

<220>
<221> misc_feature
<222> (1)..(660)
<223> RNAi target sequence

<400> 133
acctccaaaa gaccatcaag aaacaatccc tgcaactcac cgaaatccag gctcactatg 60

acgaaatcca acgtcaattg caagttactc ttgaccaact cagcgtatca caaagaaggg 120

tacaggcact taccgccgaa tctgaagaaa tcaggagcaa ttatgaaggt gctcttcgtg 180

gtaagaggca agccgagcaa atgtacgaag aagctggcac tcgcatcaac gagctcacca 240

ccatcaacgt caacctttct tcaatgaagg tcaagattga acaagaactg tcctcagttc 300

ttgctgacta cgatgaaatc accaaggaac tccgcatttc tgatgaacgc taccaacgtg 360

tacaggccga actcaaacat accgtcgaaa tcctacatga agagcaagag cgcgtggtca 420

agatcgaagc tatcaagaag agtctggaaa tcgaagtcaa gaacctctca gtccgtttgg 480

aagaagtcga agccaacgct atcgttggag gaaaacgtat catcagcaaa ctcgaagctc 540

gtatcaggga ttgcgaagct gaactcgatg aagaaaagag acgccacgcc gaaaccatca 600

agatcttgcg caagaaggaa cgcactgtta aggaagtcat gatccaatgt gaagaggacc 660

<210>  134
<211>  22
<212>  DNA
<213>  Artificial Sequence

<220>
<223>  primer


<220>
<221>  misc_feature
<222>  (1)..(22)
<223>  forward primer

<400>  134
acctccaaaa gaccatcaag aa                                                22


<210>  135
<211>  21
<212>  DNA
<213>  Artificial Sequence

<220>
<223>  primer


<220>
<221>  misc_feature
<222>  (1)..(21)
<223>  reverse primer

<400>  135
gtcctcttca cattggatca t                                                 21


<210>  136
<211>  3369
<212>  DNA
<213>  Diabrotica virgifera


<220>
<221>  misc_feature
<222>  (1)..(3369)
<223>  WCRW transcript ta00254.002_diavv

<400>  136
aatcaagtat attcaaatgg gaaacaagcc acaatttcac ctaaaaacga ctttatcgac      60

gtctcgacgc tcatatacga acgactctgc caccactgtc aggcggcgca ggcacgcgga     120

tttcaatctt gaaattttcg acggtcagtg caccaacgac acgactatac attcacaaaa     180

aagcaaaaaa atctcttgaa gatggcttct tcctctgtgg cgaggacctc caaatactct     240

taccgttcca caggagctgc aggaggcagt gctgacgtca gcatcgaata cagcgcggat     300

ctgacagcgc tttctaggct tgaggataaa attcgcctac ttcaggatga cctagaaatt     360

gaacgagaac ttagatctag aatcgaaaga gaaaaagccg accttagtgt acaagtaatc     420

caattatcag aacgtctaga agaagctgag ggtggtgcag aaagtcagtt tgaaattaac 480

aaaaaacgtg acacagaatt gctgaagctt cgcaaactat tagaagatgt acacatagag 540

tcagaagaaa ctgctctcat cctcaagaag aaacatcaag aaatcgttgt cgacttccaa 600

gaacagattg attccttagc taaagcaaaa tcaagagccg aaaaagaaaa gggcaaattc 660

caagccgaaa tctaccagct catcgcccag gtcgaaagcg taaccaaaga acgcttgatt 720

tccatcaaac aatgtgagaa actagaaatc accgtcaacg agctcaacat cagaatcgag 780

gagcttaacc gttccgtcgt tgacatcacc tcccacaaga cccgtctctc ccaagagaac 840

atcgagctca tcaaggaagt ccaagacctc aaggtcaata tcgaaaacgc catctacctc 900

aagagccagc tcgccggcca actagaggat gcccgccgcc gtgctgaaga cgatgaaaga 960

agacgctccc ttttggaggc caacctccac caggtcgaga tcgaacttga atctgtccgt 1020

gtgcaacttg aggaagaaag cgaagctcgt cttgaccttg aacgtcaatt ggtcaaatcc 1080

caaggtgagg ttcaagtcta caaatccaaa tatgaaaccg aagtcgttgc tcgtgctgaa 1140

gaagttgaag aaatccgtcg caaattcacc atcagaatcc aagaacaaga agaacacatt 1200

gaagccctct tggtcaaggt caacaacctc gagaagcaaa agtcccgcct ccaatccgaa 1260

gtcgaggttc ttatcattga cttggagaaa gcaaataaca ccgctagaga acttcaaaaa 1320

cgcgttgaac aactcgagag aatcaatatt gacttgaaga cccgtctcga tgaaacagtt 1380

caattgtacg agcaaagcca acgtgacctc cgtaacaagg ttactgaatt gcaacgcgct 1440

gtacacgaat tggacaaaac tcgcgaactg aaggaccaac ttgccagaga aaacaagaaa 1500

ttatgcgacg atctcagcga ctccaagatt accattaccg agcttactcg tcgtctccat 1560

gaactcgact tggaagttcg ccgtttggaa aatgaacgtg atgaacttac tgcagcctac 1620

aaagaagctg aagccggacg taaagctgaa gaacaacgtg cccaacgcct tgccgccgaa 1680

cttggtcaat tccgtcacga agccgaaaaa cgtcttcaag aaaaagacga agaaattgaa 1740

gctatccgca aggcaaccag catcgaaatt gaacagctca atgcccgcgt cgttgaagcc 1800

gaaaccaggc tcaagactga ggtcacccgc atcaagaaga agttgcagat ccaaatcacc 1860

gagctcgaga tgtccttgga ctccgccaac aaactcaaca tcgacctcca aaagaccatc 1920

aagaaacaat ccctgcaact caccgaaatc caggctcact atgacgaaat ccaacgtcaa 1980

ttgcaagtta ctcttgacca actcagcgta tcacaaagaa gagtacaggc acttaccgcc 2040

gaatctgaag aaatcaggag caattatgaa ggtgctcttc gtggtaagag gcaagccgag 2100

caaatgtacg aagaagctgg cactcgcatc aacgagctca ccaccatcaa cgtcaacctt 2160

tcttcaatga aggtcaagat tgaacaagaa ctgtcctcag ttcttgctga ctacgatgaa 2220

atcaccaagg aactccgcat ttctgatgaa cgctaccaac gtgtacaggc cgaactcaaa 2280

cataccgtcg aaatcctaca tgaagagcaa gagcgcgtgg tcaagatcga agctatcaag 2340

aagagtctgg aaatcgaagt caagaacctc tcagtccgtt tggaagaagt cgaagccaac 2400

gctatcgttg gaggaaaacg tatcatcagc aaactcgaag ctcgtatcag ggattgcgaa 2460

gctgaactcg atgaagaaaa gagacgtcac gccgaaacca tcaagatctt gcgcaagaag 2520

gaacgcactg ttaaggaagt catgatccaa tgtgaagagg accaaaagaa catcgctcta 2580

ttgcaagaaa ctttagacaa ggctaattcg aagattacta ttttcaagag acaactacaa 2640

gaagtcgagg gtgtatctag ccaaagcata actcgcgtac gtcgattcca gagggaattg 2700

gaagccgccg aagaccgtgc cgaaaccgcc gaaagcaatc tgtccctcat ccgcgccaaa 2760

caccgcactt tcgtcaccac cagcacagta cctggctccc aagtctactt ggtacaagaa 2820

caacgagcat cttccgaaat ctaatcatct cctattctag ctactaataa actaccccat 2880

ccggccgaat aacaacgtca aagacattta tccatacagc agcgacatct gcgaaggaaa 2940

acttgagttg gaagactcgt ttcccttcgt cagtttcttg ctgtgtgtaa acttgtcttt 3000

gggctcgcga catggtggaa gacatcatca tacaagatca tttttttttg ttcttcgtgc 3060

acataattaa ataatattta ccactctctt tgaaatatta ttattattat tataacgaaa 3120

cgtacatatt tcattaaata aattaataaa atgagtaagc gatttatatt tgtttgcaat 3180

cgcacgatga gtccatggta tggaaatttg taggtttcta ataaactgtt atgttatgta 3240

attgtactta tatcactgat gtgtgcttct attagaggta caagggttat tggaatgtaa 3300

tttataagtt tttgaatgta ccgtgtaaga ataaattgct tataaataaa aaaaaaaaaa 3360

aaaaatcgg 3369

<210> 137
<211> 589
<212> DNA
<213> Diabrotica virgifera

<220>
<221> misc_feature
<222> (1)..(589)
<223> RNAi target sequence

<400> 137
cgatttatca gcttcccaat tctctcctga tggaagagta tttcaagttg aatatgcaat 60

gaaagcagtt gaaaatagtg gcaccgtaat aggcctccga ggtacagatg gcattgtatt 120

ggctgctgaa aagctcatta tgtcaaaatt gcatgaacca agtacaaata aacgaatttt 180

caacattgat aaacacatag gaatggcatt ttcaggctta atagctgatg caaggcaaat 240

cgttgagatt gctagaaaag aagcatcaaa ttatagacat caatatggtt caaatattcc 300

tcttaaatac ctaaatgata gagtaagcat gtacatgcat gcatacactt tatacagtgc 360

tgttagacca tttggttgca gtgtcatctt ggccagttat gaagatagtg acccatctat 420

gtatctgatt gatccatctg gagttagcta tggatacttt ggatgtgcta caggtaaagc 480

aaaacagtct gcaaagactg aaatagaaaa attgaagatg gggaatctaa catgcaaaga 540

acttgttaaa gaagcagcca aaatcattta tttggtccat gatgagctg 589

<210> 138
<211> 22
<212> DNA
<213> Artificial Sequence

<220>
<223> primer

<220>
<221> misc_feature
<222> (1)..(22)
<223> forward primer

<400> 138
cgatttatca gcttcccaat tc 22

<210> 139
<211> 22
<212> DNA
<213> Artificial Sequence

<220>
<223> primer

<220>
<221> misc_feature
<222> (1)..(22)
<223> reverse primer

<400> 139
cagctcatca tggaccaaat aa 22

<210> 140
<211> 956
<212> DNA
<213> Diabrotica virgifera

<220>
<221> misc_feature
<222> (1)..(956)
<223> WCRW transcript ta06609.001_diavv

<400> 140
aaaaaatcaa aatgagttct attggaactg ggtacgattt atcagcttcc caattctctc 60

ctgatggaag agtatttcaa gttgaatatg caatgaaagc agttgaaaat agtggcaccg 120

taataggcct ccgaggtaca gatggcattg tattggctgc tgaaaagctc attatgtcaa 180

aattgcatga accaagtaca aataaacgaa ttttcaacat tgataaacac ataggaatgg 240

cattttcagg cttaatagct gatgcaaggc aaatcgttga gattgctaga aaagaagcat 300

caaattatag acatcaatat ggttcaaata ttcctcttaa atacctaaat gatagagtaa 360

gcatgtacat gcatgcatac actttataca gtgctgttag accatttggt tgcagtgtca 420

tcttggccag ttatgaagat agtgacccat ctatgtatct gattgatcca tctggagtta 480

gctatggata ctttggatgt gctacaggta aagcaaaaca gtctgcaaag actgaaatag 540

aaaaattgaa gatggggaat ctaacatgca aagaacttgt taaagaagca gccaaaatca 600

tttatttggt ccatgatgag ctgaaggata agaattttga actggaactt tcatgggtat 660

gcaaagatac gaatggttta cataccaaag tgcctgaatc agtgtttgct gatgcagaaa 720

aagctgccaa acaagcaatg gaagcagatt cagaatcaga tacagaagat atgtaataac 780

tacatttagt ttttaatatt tcgctgatgg tggctgttct tacaatattt cgtgtgttat 840

gttcatatat tatgtaatac tgtgagaatt tccatttcaa ggataggttt ataacttttt 900

tttctaataa atacataact ttatgtcaaa aaaaaaaaaa aaaaaaaaaa aaaaaa 956

<210> 141
<211> 577
<212> DNA
<213> Diabrotica virgifera

<220>
<221> misc_feature
<222> (1)..(577)
<223> RNAi target sequence

<400> 141
aattggtgca gatacacgtt tgagtactgg attttccatt tataccagag aacaaaacaa 60

acttttccca ctatcaggca ctactgtttt gggttgtgca ggatgttggt gtgacactct 120

aacattaacc agaatcctta aatctcgcat gcagatgtac caacaagagc ataacaaaac 180

aatgtctaca actgcatgtg cccagatgtt gtcaaccatg ctctactaca agagattctt 240

tccttattat atatcaaaca ttctagtagg tttagataat gaaggaaagg gctgtgttta 300

cagctatgat cctattggac attgtgaaaa agctacgtat agagcaggtg gttcagctgg 360

agctcttctt cagcctctgt tggacaatca aattggacag aagaacatgc ttaaaacatc 420

tggggaacct cttagtcagg agaaagctct gtctaccctt aaagatgtat ttatttctgc 480

tgctgaaaga gacatctaca ctggagatag cgtacttata aatattatta ctaaagatgg 540

agtaaaggaa gagtccttcc agttgagacg ggattag 577

<210> 142
<211> 22
<212> DNA
<213> Artificial Sequence

<220>
<223>  primer


<220>
<221>  misc_feature
<222>  (1)..(22)
<223>  forward primer

<400>  142
aattggtgca gatacacgtt tg                                                22


<210>  143
<211>  22
<212>  DNA
<213>  Artificial Sequence

<220>
<223>  primer


<220>
<221>  misc_feature
<222>  (1)..(22)
<223>  reverse primer

<400>  143
ctaatcccgt ctcaactgga ag                                                22


<210>  144
<211>  913
<212>  DNA
<213>  Diabrotica virgifera


<220>
<221>  misc_feature
<222>  (1)..(913)
<223>  WCRW transcript ta03835.001_diavv

<400>  144
gggggcagtt atttcgactt ttcatgcttg tcataaaata aaattaaaat atatccggcg      60

aggtgttgac tagcggattt ttttagattc aacaatctta ttttataaaa taattagtta     120

aaatgatgca aacagctaat aatgcatatt atcccgatta ttccactgct ccaatgcaac     180

gtcaaattaa cccctatgca gataatggag ggagtgtagt agcaatagca ggtgaagact     240

ttgtaataat tggtgcagat acacgtttga gtactggatt ttccatttat accagagaac     300

aaaacaaact tttcccacta tcaggcacta ctgttttggg ttgtgcagga tgttggtgtg     360

acactctaac attaaccaga atccttaaat ctcgcatgca gatgtaccaa caagagcata     420

acaaaacaat gtctacaact gcatgtgccc agatgttgtc aaccatgctc tactacaaga     480

gattctttcc ttattatata tcaaacattc tagtaggttt agataatgaa ggaaagggct     540

gtgtttacag ctatgatcct attggacatt gtgaaaaagc tacgtataga gcaggtggtt     600

cagctggagc tcttcttcag cctctgttgg acaatcaaat tggacagaag aacatgctta 660

aaacatctgg ggaacctctt agtcaggaga aagctttgtc tacccttaaa gatgtattta 720

tttctgctgc tgaaagagac atctacactg gagatagcgt acttataaat attattacta 780

aagatggagt aaaggaagag tccttccagt tgagacggga ttagaagcaa gtggttttgt 840

ttatattttc ttatgtgaaa ttcaaatata ctttctaaat aaacaaaaaa aaaaaaaaaa 900

aaaaaaaaaa aaa 913

<210> 145
<211> 542
<212> DNA
<213> Diabrotica virgifera

<220>
<221> misc_feature
<222> (1)..(542)
<223> RNAi target sequence

<400> 145
tcttcgttca ataccagctt cagtgatgat ccccagtctg actacacctc ctgaagaacc 60

atctcttgac atggcaagtg ccaatgtatt tgtgacaaac ttcacacatt cttccttgct 120

catattgggc ttgaagttgg catctacgta accataaaca taactggaac ctgatcctcc 180

gattgacact tcttgtctaa cacacatccc accaattggt atggaatata cttgtccgcc 240

tttcttttta tcccaacctg ctaccagtat accagccatt agcgaatctc tataattgta 300

gcaaagttct tggaaaatgg cggcacctac ttgtactttg ggttcttcac caagttccat 360

accatgaaaa ttaagatgat aagcaacaat gtctgcaatt gcttgtgtat ctgctgcaga 420

tcctgaacga caacagtata tatggtcagt gactttggtg agtttgtctg ctacccggtt 480

tgcaatgtag gccccagtag ttgtgcgaga atctgctcct ataacaacgc ctccatcaaa 540

ct 542

<210> 146
<211> 22
<212> DNA
<213> Artificial Sequence

<220>
<223> primer

<220>
<221> misc_feature
<222> (1)..(22)
<223> forward primer

<400> 146
tcttcgttca ataccagctt ca 22

<210>  147
<211>  22
<212>  DNA
<213>  Artificial Sequence

<220>
<223>  primer


<220>
<221>  misc_feature
<222>  (1)..(22)
<223>  reverse primer

<400>  147
agtttgatgg aggcgttgtt at                                                22


<210>  148
<211>  850
<212>  DNA
<213>  Diabrotica virgifera


<220>
<221>  misc_feature
<222>  (1)..(850)
<223>  WCRW transcript ta02145.001_diavv

<400>  148
attgcagggg gactggcaaa atattgcatt gtaggattac aagtgtaatt tttctaagct      60

tgtattcttt tcattaaact tgtaaaaaat gcctcttatg actgagtatg gtgcaatgac     120

tggtcctaat gattggagag atgccgctca cagtacagga acctctatta tggccgcgga     180

gtttgatgga ggcgttgtta taggagcaga ttctcgcaca actactgggg cttacattgc     240

aaaccgggta gcagacaaac tcaccaaagt cactgaccat atatactgtt gtcgttcagg     300

atctgcagca gatacacaag caattgcaga cattgttgct tatcatctta attttcatgg     360

tatggaactt ggtgaagaac ccaaagtaca agtaggtgcc gccattttcc aagaactttg     420

ctacaattat agagattcgc taatggctgg tatactggta gcaggttggg ataaaaagaa     480

aggcggacaa gtatattcca taccaattgg tgggatgtgt gttagacaag aagtgtcaat     540

cggaggatca ggttccagtt atgtttatgg ttacgtagat gccaacttca agcccaatat     600

gagcaaggaa gaatgtgtga agtttgtcac aaatacattg gcacttgcca tgtcaagaga     660

tggttcttca ggaggtgtag tcagactggg gatcatcact gaagctggta ttgaacgaag     720

agttgtttta ggaaatgaat tgcccaaatt ttatgaaggt taattgatta ttttttaagc     780

tttggtatgt tttggtatat attaaaataa aaaaatcagt tttacactaa aaaaaaaaaa     840

aaaaaatcgg                                                            850

<210>  149
<211>  559
<212>  DNA
<213>  Diabrotica virgifera

<220>
<221>  misc_feature
<222>  (1)..(559)
<223>  RNAi target sequence

<400>  149
ctgaattgac caacaaattg gattcaatta gagggttttt acattctcgt ttacgtaccg      60

ctacactcag gaacgacttt gaaggtcaag ctgtgtttat aaattgcctg ttgagaaatt     120

atttgcatta ttcgttgtat gatcaagctg acaaactagt gagtaaatgc gtatttcctg     180

aaactgcaag taacaatgaa tgggccagat tcttatacta cttgggcaga attaaggcgg     240

caagattaga atacagtgta gcacataaac atcttgtgca ggccatgaga aaagcacccc     300

agaatgctgc cattggtttc agacaggttg ttcaaaagct tactgttgta gttgaactac     360

tgctaggaga tataccagaa cgacagattt ttagacaatc tagtatgagg cactcccttg     420

ccccatactt tcaactaaca caagcagtta gaatgggcaa tttgcaaaga tttggtgagg     480

tgttagagaa cttcggccca caatttagac aagatcatac atttacacta attttgagac     540

tgcgccacaa tgttattaa                                                  559

<210>  150
<211>  22
<212>  DNA
<213>  Artificial Sequence

<220>
<223>  primer

<220>
<221>  misc_feature
<222>  (1)..(22)
<223>  forward primer

<400>  150
ctgaattgac caacaaattg ga                                              22

<210>  151
<211>  22
<212>  DNA
<213>  Artificial Sequence

<220>
<223>  primer

<220>
<221>  misc_feature
<222>  (1)..(22)

<223>  reverse primer

<400>  151
ttaataacat tgtggcgcag tc                                                22


<210>  152
<211>  1711
<212>  DNA
<213>  Diabrotica virgifera


<220>
<221>  misc_feature
<222>  (1)..(1711)
<223>  WCRW transcript ta08208.001_diavv

<400>  152
gggggagggg gattgtcaaa tagttgtcag cgttacacag cgaatatttt cctcatcagt      60

tcaatattaa caataaattg ttttgttgaa attgaaatca caaattactt tataaaatgg     120

taactctaga agacgttgaa atgaaaaatg cagacagtcc tccaggatta gaagctggtg     180

atacaaagaa agataccgac ctacaaagtg taatagaaat tcgtgaacat gcaagacaaa     240

tagaaaaatc agtcacaagt aaagaaaacc gtttcatctt acgagtttta cgttgcttgc     300

ccaacactag aaggaagctt aatggactgg tgctgagaag cctcattact caaatatatc     360

ctgtagctga acgtgatgcc ctccttagtt tcgtcgagga agcttctgga gaactcgacg     420

ccacccagtc acgagcaaga tcagctgtta agtcacctgt tcccgaggta gatacatata     480

taaatctttt aatactagta cgtttaattg ataccaataa gttagttgaa gcagagcgct     540

gttctcaagc tcttatgaat aaaataacta accaaaacag acgtactata gatcatattg     600

ctgccaagtg ttatttctat cactcaagag ttgctgaatt gaccaacaaa ttggattcaa     660

ttagagggtt tttacattct cgtctacgta ccgctacact caggaacgac tttgaaggtc     720

aagctgtgct tataaattgc ctgttgagaa attatttgca ttattcgttg tatgatcaag     780

ctgacaaact agtgagtaaa tgcgtatttc ctgaaactgc aagtaacaat gaatgggcca     840

gattcttata ctacttgggc agaattaagg cggcaagatt agaatacagt gtagcacata     900

aacatcttgt gcaggccatg agaaaagcac cccagaatgc tgccattggt ttcagacagg     960

ttgttcaaaa gcttactgtt gtagttgaac tactgctagg agatatacca gaacgacaga    1020

tttttagaca atctagtatg aggcactccc ttgccccata ctttcaacta acacaagcag    1080

ttagaatggg caatttgcaa agatttggtg aggtgttaga gaacttcggc ccacaattta    1140

gacaagatca tacatttaca ctaattttga gactgcgcca caatgttatt aaaacagcta    1200

taagatccat aggattatca tactccagaa tatctcccca agacatagct aagaaactgg    1260

ggctggattc agctgaagat gcagaattta tagtagcaaa agcaatcaga gatggggtga    1320

tagaagctac acttgatcca cagggaggtt acatgaggag caaagaaagt actgacatct    1380

actgcacaaa ggaacctcaa ctggcgtttc atcaaagaat atcgttttgc ttagatttgc 1440

acaatcaaag tgtcaaagct atgagatatc cccccaaggc gtatggcaaa gaacttgaat 1500

cagcagaaga gagaagggaa agagaacagc aggatttgga gttggccaag gaaatggctg 1560

aagaagatga tgatggtttt ccttaagttg gtgtagttat caatattaat tttataacat 1620

ttttgctttt gtatggtatt tatagacagt agatttatca aaatatactg agtaaaactc 1680

caaaaaaaaa aaaaaaaaaa aaaaaaaaaa a 1711

<210> 153
<211> 473
<212> DNA
<213> Diabrotica virgifera

<220>
<221> misc_feature
<222> (1)..(473)
<223> RNAi target sequence

<400> 153
atgtgtgatg actggcatga ttgctgacag caagtcccag gtgcagagag ctagatatga 60

ggctgccgag ttcaaatata agtttggata tgagatgcca atcgatgcct tgtgtaggag 120

agtatcggat atttcccagg tttatacgca gaatgctgag atgagacctt tgggttgctc 180

catgcttctg ataggatatg accaagaaat gggaccatgt gtccacaaag ctgaccctgc 240

tggctattac tgtggctaca gagcagtaag tgtaggatcc aaacaaactg aagccaacag 300

ctacctagag aagaagctaa agaagaaaac tgatctgtcc aaagatgaag tcatccaatt 360

ggcaatcagt tgtctttcaa gtgttttatc tgtagatttt aaaccaacag aagtcgaagt 420

tggggttgtt actaaggatg agcccaagtt taggaaattg acggaacaag aaa 473

<210> 154
<211> 22
<212> DNA
<213> Artificial Sequence

<220>
<223> primer

<220>
<221> misc_feature
<222> (1)..(22)
<223> forward primer

<400> 154
atgtgtgatg actggcatga tt 22

<210> 155
<211> 22

<212>  DNA
<213>  Artificial Sequence

<220>
<223>  primer


<220>
<221>  misc_feature
<222>  (1)..(22)
<223>  reverse primer

<400>  155
tttcttgttc cgtcaatttc ct                                                22


<210>  156
<211>  879
<212>  DNA
<213>  Diabrotica virgifera


<220>
<221>  misc_feature
<222>  (1)..(879)
<223>  WCRW transcript 454run3_isotig15710

<400>  156
gtgacaacag aaaaatatag aaaatcatcc tctgtgtttc cgttttgaac ttaacttgta        60

tttagtttaa aaaataatca tgtctagagg aagcagtgca ggttttgacc gacacataac       120

aattttctcg cccgaaggcc gactctatca agtagagtat gcttttaaag ccattaacca       180

agccggcccc acttcggtag cagtccgagg agtagatgct gcggcgtgtg tgacccagag       240

aaagatcccg gataagctga ttgatcccaa cacaattaca catctgttcc agttaacaga       300

acacactgga tgtgtgatga ctggcatgat tgctgacagc aagtcccagg tgcagagagc       360

tagatatgag gctgccgagt tcaaatataa gtttggatat gagatgccaa tcgatgcctt       420

gtgtaggaga gtatcggata tttcccaggt ttatacgcag aatgctgaga tgagaccttt       480

gggttgctcc atgcttctga taggatatga ccaagaaatg ggaccatgtg tctacaaagc       540

tgaccctgct ggctattact gtggctacag agcagtaagt gtaggatcca aacaaactga       600

agccaacagc tacctagaga agaagctaaa gaagaaaact gatctgtcca aagatgaagt       660

catccaattg gcaatcagtt gtctttcaag tgttttatct gtagatttta aaccaacaga       720

agtcgaagtt ggggttgtta ctaaggatga gcccaagttt aggaaattga cggaacaaga       780

aatagatttc catctgacag ctattgctga gagggattag atagattttt tatattcact       840

ctttgtaatg ttaagaaatg gtcaaatttt gagttaagt                              879


<210>  157
<211>  451
<212>  DNA
<213>  Diabrotica virgifera

<220>
<221>  misc_feature
<222>  (1)..(451)
<223>  RNAi target sequence

<400>  157
agataggcgg tttggtattc aagcccaaac ggtagctaca aatttccaaa aaaatctttg      60

aaatgggacc acatttatat gtgggtcttc caggattagc cacagatacc caaacagtta      120

tggaaaaact ccgtttccga aaaaacttgt acgaacttaa ggaaaatcga aaaatatctc      180

caaaagtatt tgcctctatg atatcaaata tgttgtatga aaaaagattt gggccatttt      240

ttgtagaacc tgtagtagct ggacttctac ctaatactta tgaacccttt atctgtaata      300

tggatttaat tggttgtata aaccaacctt cagactttgt tgttggtgga acagcgtcag      360

cacagttgta tggtatgtgt gaagcacttt gggagcctaa cctaggacct gaggatcttt      420

ttgaaaccat ctctcaagct ctcatcaatg c                                     451


<210>  158
<211>  22
<212>  DNA
<213>  Artificial Sequence

<220>
<223>  primer


<220>
<221>  misc_feature
<222>  (1)..(22)
<223>  forward primer

<400>  158
agataggcgg tttggtattc aa                                               22


<210>  159
<211>  22
<212>  DNA
<213>  Artificial Sequence

<220>
<223>  primer


<220>
<221>  misc_feature
<222>  (1)..(22)
<223>  reverse primer

<400>  159
gcattgatga gagcttgaga ga                                               22


<210>  160
<211>  829

<212>  DNA
<213>  Diabrotica virgifera


<220>
<221>  misc_feature
<222>  (1)..(829)
<223>  WCRW transcript ta03399.001_diavv

<400>  160
gtttgcagag agtgtattag tgtattgtat tttagtattt tacaattctt tgaattgcag      60

attatttagc ggtttagtac aaaaacctga agtaaattct ataacggaaa gtgcttaaat     120

tttagtggta aaatgtccat tctagcttac aatggtggtg ctatggtggc gatgaaggga     180

gaaaactgtg tagcaattgc agcagatagg cggtttggta ttcaagccca aacagtagct     240

acaaatttcc aaaaaatctt tgaaatggga ccacatttat atgtgggtct tccaggatta     300

gccacagata cccaaacagt tatggaaaaa ctccgtttcc gaaaaaactt gtacgaactt     360

aaggaaaatc gaaaaatatc tccaaaagta tttgcctcta tgatatcaaa tatgttgtat     420

gaaaaaagat ttgggccatt ttttgtagaa cctgtagtag ctggacttct acctaatact     480

tatgaaccct ttatctgtaa tatggattta attggttgta taaaccaacc ttcagacttt     540

gttgttggtg gaacagcgtc agcacagttg tatggtatgt gtgaagcact ttgggagcct     600

aacctaggac ctgaggatct ttttgaaacc atctctcaag ctctcatcaa tgcctttgac     660

agagatgcca tttctggttg gggagctaca gtgtacatca tagaaaaaaa taaggtgact     720

gtaaaaaatt tgaagacacg tatggactag ttaaatcttg ttttttcatt tacatttaat     780

tgataaaata aatgaaacta tttgataaaa aaaaaaaaaa aaaatgcga                 829


<210>  161
<211>  530
<212>  DNA
<213>  Diabrotica virgifera


<220>
<221>  misc_feature
<222>  (1)..(530)
<223>  RNAi target sequence

<400>  161
cttcttcgac taggaggtgg aatgccaggt ttaactcagg caccgccgcc ttctgatgct      60

cctgttgtgg atactgccga acaagtatac atatcctcct tagcactatt aaaaatgttg     120

aagcacggcc gagctggagt acccatggaa gtaatgggtc ttatgttagg agaattcgta     180

gatgactata ccgttcgtgt aatcgatgta tttgctatgc ctcagactgg taccggtgtc     240

agtgtagaag ctgctgatcc agtgtttcaa acaaaaatgc ttgacgtgtt aagacagact     300

ggtaggccag aaatggttgt ggggtggtat cattcccatc caggtttcgg atgttggttg     360

tctggagtgg atatcaacac ccagcaaagt tttgaggctc tcacggaaag agctgtagca 420

gtagtagttg atccaatcca gtcagtgaaa ggcaaagtcg ttattgatgc ctttaggtta 480

ataaatccga atatgatggt attgggccaa gaacctcgcc aaactacatc 530

<210> 162
<211> 22
<212> DNA
<213> Artificial Sequence

<220>
<223> primer

<220>
<221> misc_feature
<222> (1)..(22)
<223> forward primer

<400> 162
cttcttcgac taggaggtgg aa 22

<210> 163
<211> 22
<212> DNA
<213> Artificial Sequence

<220>
<223> primer

<220>
<221> misc_feature
<222> (1)..(22)
<223> reverse primer

<400> 163
gatgtagttt ggcgaggttc tt 22

<210> 164
<211> 1190
<212> DNA
<213> Artificial Sequence

<220>
<223> primer

<220>
<221> misc_feature
<222> (1)..(1190)
<223> WCRW transcript ta04924.001_diavv

<400> 164
tgacagatga cttgtaaggg ggaaaactaa aagtcatact cacggttgtt tttgtaaaac 60

ttgtaggtaa cgaattttcg tttttagtga attaacaaaa caacagagtg attttccaac 120

aagaataact tatttattag tttcattaat ttgataaaca ataaaatgga tagacttctt 180

cgactaggag gtggaatgcc aggtttaact caggcaccgc cgccttctga tgctcctgtt 240

gtggatactg ccgaacaagt atacatatcc tccttagcac tattaaaaat gttgaagcac 300

ggccgagctg gagtacccat ggaagtaatg ggtcttatgt taggagaatt cgtagatgac 360

tataccgttc gtgtaatcga tgtatttgct atgcctcaga ctggtaccgg tgtcagtgta 420

gaagctgttg atccagtgtt tcaagcaaaa atgcttgaca tgttaagaca gactggtagg 480

ccagaaatgg ttgtggggtg gtatcattcc catccaggtt tcggatgttg gttgtctgga 540

gtggatatca acacccagca aagttttgag gctctctcgg aaagagctgt agcagtagta 600

gttgatccaa tccagtcagt gaaaggcaaa gtcgttattg atgcctttag gttaataaat 660

ccgaatatga tggtattggg ccaagaacct cgccaaacta catccaactt aggacatcta 720

cagaaaccat cagtccaggc tttgattcat ggtctaaatc ggcattacta ctccatcagt 780

attaattaca gaaagaatga attggaacaa aaaatgctac taaatctcca taaaaaatcc 840

tggatggatg gtctaacttt agcagattac tctgaaaatt gtagtgtgaa tgaaaagact 900

gtagctgata tgttagaact agccaagaat tacaacaagg cattagaaga ggaggagaaa 960

atgactccag aacagctcgc tattaaaaat gttggcaaac aagatcctaa gaggcattta 1020

gaagaaaagg ttgatatttt gatgaccaat aatatcgtcc agtgcttggg agctatgcta 1080

gatacagttg tttttaagta attgctggat ctcttttttt actataattg caacttattt 1140

ataagatatt ttttcaataa aaacttttat tatgaatttg tgttgcgcag 1190

<210> 165
<211> 654
<212> DNA
<213> Diabrotica virgifera

<220>
<221> misc_feature
<222> (1)..(654)
<223> RNAi target sequence

<400> 165
acgatttttg aggttgtcgg ctagaagttc gcaaagtaaa tctaaattcc aacaattaaa 60

ccgaaatagt tctttaaaaa agatgtttcg aaaccaatac gatagtgatg tgacagtgtg 120

gagcccccag ggccgtctgc atcaggtaga atatgccatg gaagcagtta acctaggttc 180

agccacagtg ggactgaaaa gtaaaactca tgctgtactt atcgctttga agagggcttc 240

ttcagaactg tctgcctacc agaagaaaat tatcaacata gacgatcaca ttggtatcac 300

catctcggga cttacagctg atgctaggat ccttagtaga tatatgcgaa acgaatgctt 360

gaattataag tactctttgg atacttatat gccattgaat aggcttatca gtattttagg 420

taacaaaatg cagacatgta cccaaagata cgaccgcaga ccatttggtg taggtcttct 480

agtagctggt tatgacgaat ctggttctca tatctaccaa acgtgccctt ctgctaattt 540

ctacgattgc aaagctatgt ccattggagc tcgatcccag agtgccagga catacttaga 600

aaaacatctg gatgaattgc catctagttc tttagaagaa cttatcaaac atgg 654

<210> 166
<211> 22
<212> DNA
<213> Artificial Sequence

<220>
<223> primer

<220>
<221> misc_feature
<222> (1)..(22)
<223> forward primer

<400> 166
atttttgagg ttgtcggcta ga 22

<210> 167
<211> 22
<212> DNA
<213> Artificial Sequence

<220>
<223> primer

<220>
<221> misc_feature
<222> (1)..(22)
<223> reverse primer

<400> 167
ccatgtttga taagttcttc ta 22

<210> 168
<211> 1142
<212> DNA
<213> Diabrotica virgifera

<220>
<221> misc_feature
<222> (1)..(1142)
<223> WCRW transcript ta04466.001_diavv

<400> 168
gggggagggg gacagtcgac gtagcattac aaacgatttt tgaggttgtc ggctagaagt 60

tcgcaaagta aatctaaatt ccaacaatta aaccgaaata gttctttaaa aaagatgttt 120

cgaaaccaat acgatagtga tgtgacagtg tggagccccc agggccgtct gcatcaggta 180

gaatatgcca tggaagcagt taacctaggt tcagccacag tgggactgaa aagtaaaact 240

catgctgtac ttatcgcttt gaagagggct tcttcagaac tgtctgccta ccagaagaaa 300

attatcaaca tagacgatca cattggtatc accatctcgg gacttacagc tgatgctagg 360

atccttagta gatatatgcg aaacgaatgc ttgaattata agtactcttt ggatacttat 420

atgccattga ataggcttat cagtatttta ggtaacaaaa tgcagacatg tacccaaaga 480

tacgaccgca gaccatttgg tgtaggtctt ctagtagctg gttatgacga atctggttct 540

catatctacc aaacgtgccc ttctgctaat ttctacgatt gcaaagctat gtccattgga 600

gctcgatccc agagtgccag gacatactta gaaaaacatc tggatgaatt gccatctagt 660

tctttagaag aacttatcaa acatggattg agggctctta gagatactct acctccagag 720

gttgacctca caacaaaaaa tgtctccata ggatacactg gcaaaggtca gacctttaaa 780

atcctcgaag aagaagaaac ttccccctac ttgtccctta ttgagggtga agaacgtaga 840

ggaggaggag ctgcagctgc tgcaattgag gaccttcctg cagatccgtt gagagatgat 900

gcgggggacg agcccaggga tgcagtccca gctgtggaaa cttctgccat ggataccaca 960

gaatagaagg attagataaa gcattaaagc cacccgttga tcaggagagt gggtattgga 1020

aaggaatatt gccgtggctg tctctgttag tctaaaacag actgagacag tcacggcaga 1080

acatcgtttg ttatatccat actgtatttt ttaagctttt aataatattt tgattaataa 1140

at 1142

<210> 169
<211> 558
<212> DNA
<213> Diabrotica virgifera

<220>
<221> misc_feature
<222> (1)..(558)
<223> RNAi target sequence

<400> 169
tctgtgcaaa aacaaactga caagtttgac ataagtgaca agtgacatag cggttgtcaa 60

caattttgca agttgtgtaa attgttaatt tgaaggaagc ttttaacttt taagtaaaat 120

taactgaaaa tggctacagt tttgtgtcca gaaataccaa cgcctggttt ttcattcgaa 180

aattgtaaaa gaaatgcact tctggaaaat aagggccttg ctcttccaaa agccactaaa 240

acaggtacta cgatcgtagg aatagtctac aaagatggaa ttattcttgg agcagatact 300

agagctacag aagacaccac agttgcagat aagaactgtg aaaagattca cttccttgca 360

cccaatatgt attgttgtgg tgctggtact gcagcagata cagaaatgac cactcagatg 420

gtctctgcta aattagaact ccacagatta tacacaaacc gagttactag agtgtgtaca 480

gcaaaccaat tgttaaaaca atatctattt cgctaccagg gatatattgg ggctgcttta 540

atcctgggtg gtgttgat                                                    558

<210>  170
<211>  22
<212>  DNA
<213>  Artificial Sequence

<220>
<223>  primer

<220>
<221>  misc_feature
<222>  (1)..(22)
<223>  forward primer

<400>  170
tctgtgcaaa aacaaactga ca                                                22

<210>  171
<211>  22
<212>  DNA
<213>  Artificial Sequence

<220>
<223>  primer

<220>
<221>  misc_feature
<222>  (1)..(22)
<223>  reverse primer

<400>  171
atcaacacca cccaggatta aa                                                22

<210>  172
<211>  1578
<212>  DNA
<213>  Diabrotica virgifera

<220>
<221>  misc_feature
<222>  (1)..(1578)
<223>  WCRW transcript ta04816.001_diavv

<400>  172
gggggtttta aattttcgta gacacgattg ttcagtagtt caaaattcat tattattaat      60

gttctgtgca aaaacaaact gacaagtttg acataagtga gaagggacat agcggttgtc     120

aacaattttg caagttgtgt aaattgttaa tttgaaggaa gcttttaact tttaagtaaa     180

attaactgaa aatggctaca gttttgtgtc cagaaatacc aacgcctggt ttttcattcg     240

aaaattgtaa aagaaatgca cttctggaaa ataagggcct tgctcttcca aaagccacta     300

aaacaggtac tacgatcgta ggaatagtct acaaagatgg aattattctt ggagcagata     360

ctagagctac agaagacacc acagttgcag acaagaactg tgaaaagatt cacttccttg 420

cacccaatat gtattgttgt ggtgctggta ctgcagcaga tacagaaatg accactcaga 480

tggtctctgc taaattagaa ctccacagat tatacacaaa ccgagttact agagtgtgta 540

cagcaaacca attgttaaaa caatatctat ttcgctacca gggatatatt ggggctgctt 600

taatcctggg tggtgttgat attgaaggac cacatctata tatgatttat ccacatggat 660

ccagtgttga tcttccttat gctacaatgg gttctggttc cttagcagcc atatctgtct 720

ttgaatcccg ctggaagcct caaatggaag aggaagatgg tgtccaacta gtaagagatg 780

ctattgcagc tggtatattc aatgatctag gttcaggttc caatgtagac atctgtgtaa 840

taagaaaggg ttcagtagac tacaaacgaa cctatgacga ggctaacaaa aagggagtta 900

aacaaggatc ttatagatat caaagaggaa ccactgcagt gctcagtacc caggtggtgg 960

atgttgagga agtctcagtg attcccattg aagctgaaga aatggatgtt gcttaaatat 1020

tacagttaaa taaatgtcat tgtattcttt ttggtattta ctttaatata aaaataaaaa 1080

ataaatataa cagggtttaa atgttcaata atatagtaaa ttttaattta aaaaaatatt 1140

ttttcttaac aattacaata gccacatcta aatagatttg ctactcaagg atgttgagtt 1200

aataggatca gtcagtctat aaaactaaag aacacattta ttttgatcat tactttgggt 1260

ttgatagctg ccctttatta tatgttaatt ttaagtacaa tctatataac cctaaagttg 1320

tttacccaaa aatcattttt atcattttcg agagtaaata ttgttcaatt ttcacatgtc 1380

aaaaatatta ttatattttt tactgtgttt aatcttttat tacaaccctt aaggtactag 1440

tacacttcag aagaacaaaa ataatcattt ttttaaagaa ttttcctctc agaacctgta 1500

ttaaaaatga acacaaaacc tgtcacatac taatatctaa ctctcagaga gtacaagaaa 1560

tatatctctt ttcattca 1578

<210> 173
<211> 473
<212> DNA
<213> Diabrotica virgifera


<220>
<221> misc_feature
<222> (1)..(473)
<223> RNAi target sequence

<400> 173
aatataccga aaatgccgag tcaagaatta acaactaata aagtaatagt ccaccctttg 60

gttcttctaa gtgtggtgga ccattttaat cgcatgggca aaatcggcaa tcaaaagcgt 120

gttgttggtg ttcttcttgg ctgttggcgt gctaaaggag tcttagatgt ttctaacagt 180

tttgcagttc cttttgacga agatgacaag gataagtctg tgtggttctt ggaccatgat 240

tacttagaaa acatgtatgg aatgttcaaa aaagttaatg ccagggaacg agtagtaggt 300

tggtaccaca ccggccccaa acttcatcaa aatgatattg ccattaatga gttaatcaga 360

aggtattgtc caaattccgt tctagttatc atagacgcta aaccaaaaga tttaggttta 420

ccaactgaag cttatcaagc tgtagaagaa gtacacgatg acggttcacc tac 473

<210> 174
<211> 22
<212> DNA
<213> Artificial Sequence

<220>
<223> primer

<220>
<221> misc_feature
<222> (1)..(22)
<223> forward primer

<400> 174
aatataccga aaatgccgag tc 22

<210> 175
<211> 22
<212> DNA
<213> Artificial Sequence

<220>
<223> primer

<220>
<221> misc_feature
<222> (1)..(22)
<223> reverse primer

<400> 175
gtaggtgaac cgtcatcgtg ta 22

<210> 176
<211> 1221
<212> DNA
<213> Diabrotica virgifera

<220>
<221> misc_feature
<222> (1)..(1221)
<223> WCRW transcript ta03894.001_diavv

<400> 176
tagttaattg ggaacaaaga ttttattaga ataatatacc gaaaatgccg agtcaagaat 60

taacaactaa taaagtaata gtccaccctt tggttcttct aagtgtggtg gaccatttta 120

atcgcatggg caaaatcggc aatcaaaagc gtgttgttgg tgttcttctt ggctgttggc 180

gtgctaaagg agtcttagat gtttctaaca gttttgcagt tccttttgac gaagatgaca 240

aggataagtc tgtgtggttc ttggaccatg attacttaga aaacatgtat ggaatgttca 300

aaaaagttaa tgccagggaa cgagtagtag gttggtacca caccggcccc aaacttcatc 360

aaaatgatat tgccattaat gagttaatca gaaggtattg tccaaattcc gttctagtta 420

tcatagacgc taaaccaaaa gatttaggtt taccaactga agcttatcaa gctgtagaag 480

aagtacacga tgacggttca cctacatcta aaacatttga gcatgttcct agtgaaatcg 540

gagctgagga agctgaagaa gttggtgttg aacacttatt gagagatatt aaagacacaa 600

ctgttggtac tttgtcacag agaataacga atcagctttt agggttaaaa ggtctccatt 660

cacagttacg tgatattagg gactatttgg tacaagtgtg tggtaaccag cttccaatca 720

atcaccaaat catctaccaa ttacaagaca tatttaatct tttgcccgat ataaatcagg 780

atgcatttaa tacatcgttt tacgtgaaaa ataacgatca aatgttagtc gtttatctag 840

cagctttagt tagatctatt gtcgctctac acaatcttat taacaacaaa ctaacaaata 900

gagacgcgga agaaggaaaa aaggaggata gtaagaaaga aagcaaagat aaggacaagg 960

ataaagagaa ggaaaaggat aaagatgcta agaaggacga gaagaaaaag taattgtgta 1020

ttatatctgg ttctacattc aaatgttctc atgttgtgtt ttcaaacacg atattttaat 1080

ggtttcaaca ctttccattt gattcgagta taataattta ttttcatttt atatgctagt 1140

ttagtatata cctaacttcc ttactaagtt tgatttatat ttaggcaata aagttcttta 1200

tacaaaaaaa aaaaaaaaaa a 1221

<210> 177
<211> 553
<212> DNA
<213> Diabrotica virgifera

<220>
<221> misc_feature
<222> (1)..(553)
<223> RNAi target sequence

<400> 177
ttaattctgc tgctcctcct ttgtggcata acggccccgt acctggagaa ttttacaatt 60

ttcccggcaa acagaccagc tattccaacc ctataacaca tacacagtct cccgttacaa 120

catcaacttc tgtattagct attacttacg ataaaggagt attgatagcc ggagatctgg 180

ttgcttcata cggatcttta gctaggtacg gaaattgccc gagggttatt aaagtcaatc 240

agaatatcat cttgggagcc ggaggagact atgctgattt ccaatacgtc aagagtttca 300

ttgagcaaaa aataattgat gaagaatgca ttgatgatgg actaaaaatg aaaccaaaat 360

cattgtactg ctggcttact agagtaatgt accaacgcag gagtaaatta gacccttttt 420

ggaataatct ggttatagga ggaatccagg atggagtccc ctttcttgga accgtagata 480

aattgggaac tgcatataca gacaaagtta tatgtacagg ttatggagcc cacatagcct 540

tgcctcttct gag 553

<210> 178
<211> 22
<212> DNA
<213> Artificial Sequence

<220>
<223> primer

<220>
<221> misc_feature
<222> (1)..(22)
<223> forward primer

<400> 178
ttaattctgc tgctcctcct tt 22

<210> 179
<211> 22
<212> DNA
<213> Artificial Sequence

<220>
<223> primer

<220>
<221> misc_feature
<222> (1)..(22)
<223> reverse primer

<400> 179
ctcagaagag gcaaggctat gt 22

<210> 180
<211> 925
<212> DNA
<213> Diabrotica virgifera

<220>
<221> misc_feature
<222> (1)..(925)
<223> WCRW transcript ta01874.001_diavv

<400> 180
aaacaagtca tcgttgaatt ttctgaagtt agagattctt tgattacagt ttgttgtatt 60

aaatagaaaa tgtttaattc tgctgctcct cctttgtggc ataacggccc cgtacctgga 120

gaattttaca attttcccgg caaacagacc agctattcca accctataac acatacacag 180

tctcccgtta caacatcaac ttctgtatta gctattactt acgataaagg agtattgata 240

gccggagatc tggttgcttc atacggatct ttagctaggt acagaaattg cccgagggtt 300

attaaagtca atcagaatat catcttggga gccggaggag actatgctga tttccaatac 360

gtcaagagtt tcattgagca aaaaataatt gatgaagaat gcattgatga tggactaaaa 420

atgaaaccaa aatcattgta ctgctggctt actagagtaa tgtaccaacg caggagtaaa 480

ttagaccctt tttggaataa tctggttata ggaggaatcc aggatggagt cccctttctt 540

ggaaccgtag ataaattggg aactgcatat acagacaaag ttatatgtac aggttatgga 600

gcccacatag ccttgcctct tctgagagac tctttagata aaaatggagc tcttaacttg 660

gccgatgcca aaactctgat tcacaaatgc atggaagtat tgttctatag agatgccaga 720

agttttccta aatatcagct tggtgttatt gataaagatg atggtgttag tattgagggg 780

cctatttcag tcgaagagaa ctggagcatt gcccatatga cataattttt cttaatttcg 840

tttattttgt attgaaacta tattatacat tcatggaagt ctgtgtgaat aaatttttga 900

taaaactaaa aaaaaaaaaa aaaaa 925

<210> 181
<211> 420
<212> DNA
<213> Diabrotica virgifera

<220>
<221> misc_feature
<222> (1)..(420)
<223> RNAi target sequence

<400> 181
acaagcaatg gcgaaagtaa atgaagtggc taatttgtac aagcagttaa aatcagaatg 60

gaataccaac aacccaaatt taagtaaatg tgaaaaacta ctctcaactc ttaagcttga 120

gctcaccaat ctaatgtttc tgccgattac cacatctact gcgtccaaac aagaactact 180

tctagccaga gatgttctag agataggagt ccaatggagt atagcagcca atgatattac 240

atcttttgaa agatacatgg cacaattaaa atgttattac ttcgattata acaaccaact 300

accagaatca ccatttaagt accaactgct tggattaaac ttgttattcc tgctgtcaca 360

aaaccgagta gcagaatttc acactgaatt ggaactttta cctgccgatc atattcagaa 420

<210> 182
<211> 22
<212> DNA
<213> Artificial Sequence

<220>
<223> primer

<220>

<221>  misc_feature
<222>  (1)..(22)
<223>  forward primer

<400>  182
acaagcaatg gcgaaagtaa at                                                22


<210>  183
<211>  22
<212>  DNA
<213>  Artificial Sequence

<220>
<223>  primer


<220>
<221>  misc_feature
<222>  (1)..(22)

<400>  183
ttctgaatat gatcggcagg ta                                                22


<210>  184
<211>  1040
<212>  DNA
<213>  Diabrotica virgifera


<220>
<221>  misc_feature
<222>  (1)..(1040)
<223>  WCRW transcript ta06150.001_diavv

<400>  184
gacagctgtc acaaatattc aacggattcc atttgttcct agtcatagtg taatttgtgg      60

aagaattttg ttcgagtatt tatagctaaa attctgaaga tttaattaat taaacaagca     120

atggcgaaag taaatgaagt ggctaatttg tacaagcagt taaaatcaga atggaatacc     180

aacaacccaa atttaagtaa atgtgaaaaa ctactctcaa ctcttaagct tgagctcacc     240

aatctaatgt ttctgccgat taccacatct actgcgtcca aacaagaact acttctagcc     300

agagatgttc tagagatagg agtccaatgg agtatagcag ccaatgatat tacatctttt     360

gaaagataca tggcacaatt aaaatgttat tacttcgatt ataacaacca actaccagaa     420

tcaccattta agtaccaact gcttggatta aacttgttat tcctgctgtc acaaaaccga     480

gtagcagaat ttcacactga attggaactt ttacctgccg atcatattca gaatgatgtg     540

tacatcagac atcccttgtc tattgaacaa tatttaatgg aagggagtta taataaaata     600

tttctggcta aaggtaatgt acctgctaag agttataact tctttatgga tattttgctg     660

gacaccataa gagaagaaat tgcagactgt ttatcagtag cctatgaaaa gatctcagta     720

aaagacgtag ccagaatgct gtacctgcca aacgaagaag ctgctaaaac atttgccaaa     780

aaggctttcc agctcaaacc gtggaagctc aacaatgact ttttccactt tcttcctgaa 840

gagaagaccc acgaacccat accctcacag gaactggcac agcaggctat agagtatgcc 900

aaggaacttg aaatgattgt ctaataggtg tatttaattc ttatatttag gattttttta 960

tataaaaatc aatatttaat aaacaaacaa tttatttgtt ttgtacttta aaaaaaaaaa 1020

aaaaaaaaaa aaaaaatgcg 1040

<210> 185
<211> 716
<212> DNA
<213> Diabrotica virgifera

<220>
<221> misc_feature
<222> (1)..(716)
<223> RNAi target sequence

<220>
<221> misc_feature
<222> (608)..(608)
<223> n can be any nucleotide

<220>
<221> misc_feature
<222> (645)..(645)
<223> n can be any nucleotide

<400> 185
cattacatag cgctttttgt tattgcaaat tgtcggcccg agttattttg tacaatggag 60

tgtctacttg gtatcaaatt taatgattac gttcttgttg ctgcagatat gactgcagcg 120

caaagcataa ttgtaatgaa aacagatgaa aacaagcttc ataaactttc caataagctt 180

gttatggctg tttctggtga gtctggggat actacacagt ttgcagagta tatagcaaaa 240

aatattcaac tttacaaaat gaggaatacc tatgaattga gtcccaaaga agctgcagca 300

tttactagaa gaaatttggc agatgcactc agaagcagaa gtgcatacca tgtaaacctt 360

cttctagctg gatatgatga caaagaaggt cctcagctat attacatgga ctacctagca 420

tccatagcaa gcgttgatta tgctgctcat ggttatggag gattcttttc actgtcaatt 480

atggatcgta attatcggca gaaccttagt aaagaagaag gttatgaact tttgaaaaaa 540

tgtgtgaaag aagttcagaa aagattgatc atcaatttgc ctaactttaa ggtgcagtgt 600

gttgatanaa atggcatcca ggatatgcct aatatcactg ctaangattt ggtttaattt 660

agtttgatat aagttatgta ttttttttta ttattaaata aactgaattt tcttgt 716

<210> 186
<211> 20
<212> DNA
<213> Artificial Sequence

<220>
<223>  primer

<220>
<221>  misc_feature
<222>  (1)..(20)
<223>  forward primer

<400>  186
cattacatag cgctttttgt                                                   20

<210>  187
<211>  20
<212>  DNA
<213>  Artificial Sequence

<220>
<223>  primer

<220>
<221>  misc_feature
<222>  (1)..(20)
<223>  reverse primer

<400>  187
acaagaaaat tcagtttatt                                                   20

<210>  188
<211>  872
<212>  DNA
<213>  Diabrotica virgifera

<220>
<221>  misc_feature
<222>  (1)..(872)
<223>  WCRW transcript ta01941.001_diavv

<400>  188
aaaaatgggg ggctatcatt acatagcgct ttttgttatt gcaaattgtc ggcccgagtt       60

attttgtaca atggagtgtc tacttggtat caaatttaat gactacgttc ttgttgctgc      120

agatatgact gcagcgcaaa gcataattgt aatgaaaaca gatgaaaaca agcttcataa      180

actttccaat aagcttgtta tggctgtttc tggtgagtct ggggatacta cacagtttgc      240

agagtatata gcaaaaaata ttcaacttta caaaatgagg aatacctatg aattgagtcc      300

caaagaagct gcagcattta ctagaagaaa tttggcagat gcactcagaa gcagaagtgc      360

ataccatgta aaccttcttc tagctggata tgatgacaaa gaaggtcctc agctatatta      420

catggactac ctagcatcca tagcaagcgt tgattatgct gctcatggtt atggaggatt      480

cttttcactg tcaattatgg atcgtaatta tcggcagaac cttagtaaag aagaaggtta      540

cgaacttttg aaaaaatgtg tgaaagaagt tcagaaaaga ttgatcatca atttgcctaa      600

ctttaaggtg cagtgtgttg ataaaaatgg catccaggat atgcctaata tcactgctaa 660

ggatttggtt taatttagtt tgatataagt tatgtatttt tttattatta aataaactga 720

attttcttgt aaaaaaaaaa aaaaaagtag catttatttc tatattagga caagaaatta 780

gatagggtgg tatatgcttg taacaaaatt tactaattat acggtaaacg ccaccctact 840

agatacatag gaacattgag ctattacagt cc 872

<210> 189
<211> 364
<212> DNA
<213> Diabrotica virgifera

<220>
<221> misc_feature
<222> (1)..(364)
<223> RNAi target sequence

<220>
<221> misc_feature
<222> (274)..(274)
<223> n can be any nucleotide

<400> 189
ttggcatgac tgtgcaaata gtcggtccat tgtgcatgag taaagtgatt ctaaccataa 60

cttcaaaacg ctttagtttt aaatatttgt gaacattaat aacagaaaca gttcattcaa 120

aataatctat aagaatgggc gatacaaagg tagaacctac aaaggtggat aaaacaaagg 180

ggactgagga caaaaacaaa aatgaactgt cggaagaaga caaacagctt caagatgaat 240

taaatttgtg tgttgagaaa ctccaaggat aacnatgaaa agagatacct acttgctttg 300

caaagcttta tcaaatcata ttcaaagcct caactacttc gatgacttct gttcctaaag 360

ccgt 364

<210> 190
<211> 22
<212> DNA
<213> Artificial Sequence

<220>
<223> primer

<220>
<221> misc_feature
<222> (1)..(22)
<223> forward primer

<400> 190
ttggcatgac tgtgcaaata gt 22

<210> 191
<211> 22

<212>  DNA
<213>  Artificial Sequence

<220>
<223>  primer


<220>
<221>  misc_feature
<222>  (1)..(22)
<223>  reverse primer

<400>  191
acggctttag gaacagaagt ca                                              22


<210>  192
<211>  3066
<212>  DNA
<213>  Diabrotica virgifera


<220>
<221>  misc_feature
<222>  (1)..(3066)
<223>  WCRW transcript ta44362.001_diavv

<400>  192
attgaatgct tggcatgact gtgcaaatag tcggtccatt gtgcatgagt aaagtgattc      60

taaccataac ttcaaaacgc tttagtttta aatatttgtg aacattaata acagaaacag     120

ttcattcaaa ataatctata agaatgggcg atacaaaggt agaacctaca aaggggactg     180

aggacaaaaa caaaaatgaa ctgtcggaag aagacaaaca gcttcaagat gaattaaatt     240

tgtgtgttga gaaactccag gataacgatg aaaagagata cctacttgct ttgcaagctt     300

tatcaaatca tattcaagcc tcaactactt cgatgacttc tgttcctaag ccgttaaaat     360

ttatgaggcc ccattacgaa accatgaaga cagtacatga aaaaattact gatcctgata     420

ctaaaaaagt ctgtgcagat attatatctg ttttagctat gacaatggga gaaggcagag     480

aatgtttgaa ataccgtctt ctttctaatt tggataaaat tggcgaatgg ggacatgaat     540

atgttaggca tttggcagga gagattgcag gagaatgggc cgagactgat tttatagtga     600

acactgaaat tagggaaaaa cttatcttac taacaaaaca aattgtgcca tataatatgg     660

cacacaatgc agaagcagaa gcatgtgact tgcttatgga aattgaaagg cttgatctac     720

tagaacagta tgttgatgaa aacatctacc atagagtgtg cttatatctc accagctgtg     780

taccctatgt ggcagatcct gagaatacta ctctactgca gactgctctt ttactgttta     840

gaaagtttga tcagtatccc caggcactga gattagcaat gcagttaaat gactttggat     900

taattgaaga aatttttgtc aactgcccag attcggacgt acaaaaacaa ctggcattta     960

tgctaggacg acaacagata tatgttgaaa ttcctgagca tacgcctgaa tacgaagatc    1020

taatcgagat aatggcaaac tgtcatctta ataaccactt cttgaacctc gctcgcgagt    1080

tggacattat ggatcctaaa acccccgaag acgtttacaa atcccattta gaaaatagtc 1140

gtcctccatt tggtggaaac caagttgatt cagccagaca aaacttagct gccagctttg 1200

ttaacggttt tgttaatgct gcttttggtc aagataagct cttaatggaa gatgggaata 1260

aatggcttta caaaaacaaa gaacatggaa tgcttagtgc tactgcttct ttgggactaa 1320

ttcttttatg ggatgtagac ggtggtctta ctcctatcga taaatacttg tactcttctg 1380

aagaccatat caagtctggc gctttattag cttgtggcat tgtaaactgc ggtgtcagga 1440

atgaatgtga tcccgcactt gcacttttaa gtgattatgt tcttcacaac acaaacataa 1500

tgagaatagg agctattgta ggtctaggtt tagcgtatgt tggttcaaat agggaagcag 1560

tattatctct aatcacgcct gtgctatctg accctaaatc aaatatggag gtcattggaa 1620

tggcggctct agcatgcgga atgatttctg caggatctgg taactcacat gtgacaacat 1680

caataatgca agttcttatg gaaaagtccg aaatagaatt aaaggacact tacgccagat 1740

ttttgcctct gggtcttgga ttgtgtcatt taggtaaaca agaaagcatt gacacaatta 1800

tagcagcatt agaagttata cctgaccctt tcagatccat ggcgactact atggtcgata 1860

tttgcgctta tgcaggcaca ggaaatgttt taaaagtaca acatttgtta catatatgtt 1920

cagaacatta cgatgctcct gaaactcctg acaaaacttc aaaggacaaa gataaaaaag 1980

aagcagagaa agaaaaagat aaggatcttt cagctagaca agctattgcg gttttgggta 2040

tagctttaat tagcatggga gaagacattg gtagcgagat ggctttccgg acatttggtc 2100

atttactaag atactgcgaa ccagcgatca gacgtgctgt tcccttagcc ctaggcttaa 2160

tctcagtttc aaatccaaaa ttaagtattc tcgatacttt gagtaaattt tcacatgata 2220

gcgatgctga agtggctcat aatgccatct ttgcgatggg tctggtggga gctggtacta 2280

acaatgcaag gttagcagct atgctaagac agttagcaca gtaccatgcc aaagatccca 2340

ataatttatt tatggttcgc atcgcccagg gtctcactca tcttggcaaa ggaactttaa 2400

ctctcagtcc attccacagt gataggcagc tgatgagtcc agtagctgta gctggtctaa 2460

tggcgacact catcggcttc ttggatgtta aaaacattat attggggaag tcacactact 2520

tactgtacac cttagcagct gcaatgcaac caaggatgtt agtcacattt gacgatgagc 2580

taaatcctct accagtgcct gtcagagttg gattggcagt ggatgttgtt ggccaagcag 2640

gaaaaccaaa gactataaca ggtttccaaa cccataccac tcctgtgctt ctggcttatg 2700

gtgagcgcgc tgagctcgca actgaagaat acattgcatt gacgccaatt atggaaggat 2760

ttgttatttt acgaaaaaat cctgaattta attcataaat atattatttc cgtcattgtt 2820

tatcatgttc cgcaattttt tgatttaata tacttttttg tattaatcga tatttattaa 2880

atattgctca cgttttgatt gaaaacccta attgttcata acctcttcaa tatctgtata 2940

tttgcattga aaatatctga atctgtatat tttaagaaaa aatcgcgaat ttaattcata 3000

aatatagata aaagtatagg gacaaaatta tggagcccat ttctctaaag tggacgattt 3060

tggaga 3066

<210> 193
<211> 358
<212> DNA
<213> Diabrotica virgifera

<220>
<221> misc_feature
<222> (1)..(358)
<223> RNAi target sequence

<220>
<221> misc_feature
<222> (267)..(267)
<223> n can be any nucleotide

<400> 193
cagcagtggt acagcaatca aatgtaaacg attatttact aaaaaaacaa caagaaagta 60

ctaaagaatt aggctcagaa tgggccgaac ttgaagaact tcataataaa aaattgtggc 120

atcagttgac tctaaaattg ctgaatttta ttaagaaacc agaactacag aagaatgata 180

acctcattca actgtacaat aactttattc aaagttttga aaataaaatc aatcctttgt 240

cattggttga aattgttgct atagtantcc aacaattcaa aaatccaaaa gatgctgtcg 300

cttttcttga aaagactgaa cctaaggtta aaattaattc aagatgccca aaacttgt 358

<210> 194
<211> 21
<212> DNA
<213> Artificial Sequence

<220>
<223> primer

<220>
<221> misc_feature
<222> (1)..(21)
<223> forward primer

<400> 194
cagcagtggt acagcaatca a 21

<210> 195
<211> 21
<212> DNA
<213> Artificial Sequence

<220>
<223> primer

<220>
<221>  misc_feature
<222>  (1)..(21)
<223>  reverse primer

<400>  195
acaagttttg ggcatcttga a                                                21


<210>  196
<211>  1241
<212>  DNA
<213>  Diabrotica virgifera


<220>
<221>  misc_feature
<222>  (1)..(1241)
<223>  WCRW transcript ta04679.001_diavv

<400>  196
taaaagcgaa aatggctgca gcagtggtac agcaatcaaa tgtaaacgat tatttactaa      60

aaaaacaaca agaaagtcct aaagaattag gctcagaatg ggccgaactt gaagaacttc     120

ataataaaaa attgtggcat cagttgactc taaaattgct gaattttatt aagaaaccag     180

aactacagaa gaatgataac ctcatacaac tgtacaataa ctttattcaa agttttgaaa     240

ataaaatcaa tcctttgtca ttggttgaaa ttgttgctat agtagtccaa caattcaaaa     300

atccaaaaga tgctgtcgct tttcttgaaa agactgaacc taaggttaaa attaattcag     360

atgcccaaaa cttgtgcaaa gttttagctg ggcagatata tatcgaaaag ttgaacgatt     420

tagatgctac taaaaagata attgaagaag tggaatctac attggacaat gctgatggtg     480

ttactccagt tcatggaaga ttttatttac ttgcctcaca atattataga attcaaggaa     540

accatgctca atattatagg acagcattga gatatcttgg atgcatagag ttggatacaa     600

taacatcaga agtgaaagtg caacacgctt tctttttggg tttggctgct ctattaggcg     660

aaggtgtcta taacttaggt gaattactcg cacatcccgt tttggagtcc ctaaaatcaa     720

cagaaaatgc gtggttggta gaattgttgt atgcttttaa ttctggcaat attaaaaagt     780

ttgagtccat gaaaccgcag tgggaagcta tagcagatct tgcagctgaa gaattatttt     840

tacgacaaaa aatatccctg ttgtgtctta tggagatgac atttaataga ccatcgcaca     900

acaggcaatt aacatttgca gaaatcgcaa aagaaacaaa attacctttg aatgaaattg     960

aattattgat tatgaaagcg ttgtcccaag gactagtcaa aggagccatt gatcaggttg    1020

ccaataccgt aaatatgacc tgggttcaac ctagagtttt ggattgtacg cagatttcca    1080

acatgattac taggcttaac gattggtgta aacatgttga taccatggag caattgctgg    1140

aagaaaaagc tagtgaaata ttaacattgt aatttgtaat tattaataat ttatatattc    1200

gtccttaatt tttttttaag gttttctcac tgataataaa a                        1241

<210>  197
<211>  410
<212>  DNA
<213>  Diabrotica virgifera

<220>
<221>  misc_feature
<222>  (1)..(410)
<223>  RNAi target sequence

<400>  197
tggaggtgat aacgaggtac aacgtactat gttagaactt atcaatcagt tggatggttt      60

tgatcctaga ggtaacatta aagtacttat ggctacaaat cgtccagata ctctagatcc     120

tgcccttatg agacctggtc gtttggatcg taaagttgaa tttggacttc cagaccttga     180

aggaagaact catattttca aaattcatgc taggtccatg tctgttgaaa gagatattcg     240

ttttgaactt ttggctaggt tgtgtccaaa ttccactgga gctgaaatca gaagtgtttg     300

taccgaagct ggtatgttcg ctataagagc aaggaggaaa gtagctaccg aaaaagactt     360

cttagaagct gtaaataaag taattaagtc atacgccaag ttctctgcta                410

<210>  198
<211>  22
<212>  DNA
<213>  Artificial Sequence

<220>
<223>  primer

<220>
<221>  misc_feature
<222>  (1)..(22)
<223>  forward primer

<400>  198
tggaggtgat aacgaggtac aa                                               22

<210>  199
<211>  22
<212>  DNA
<213>  Artificial Sequence

<220>
<223>  primer

<220>
<221>  misc_feature
<222>  (1)..(22)
<223>  reverse primer

<400>  199
tagcagagaa cttggcgtat ga                                               22

<210>  200
<211>  1452
<212>  DNA
<213>  Diabrotica virgifera

<220>
<221>  misc_feature
<222>  (1)..(1452)
<223>  WCRW transcript ta07921.001_diavv

<400>  200
   ttatttaaaa ctaaatctat gaaaaatatt gttcttaaaa tatttttcat agatttagtt      60
   ttaaataaag acaattaaga tgccggacca tttaggagat gatatgcgaa aaataaaaga     120
   acagaaagag gagcccgaaa aggaaattaa agccctggat gaaggtgata tagcacttct     180
   taaaacctat ggacaagggc aatacacaaa gacaattaaa acatgtgaag aagatatcca     240
   aacgataatc aaaagagtaa atgaattaac tggtataaaa gaatctgata ctggtttagc     300
   tccaccagct ttatgggatt tggcagctga caaacaaacc ttacaaaatg agcaaccctt     360
   acaagtggcc cgttgtacga aaattatcaa cgctgataca gatgatccca aatacattat     420
   aaacgttaaa cagtttgcca aattcgtagt agatttggca gactctgtgg cccctacaga     480
   catagaagaa ggcatgagag ttggagtcga ccgaaataag tatcaaattc atattcctct     540
   acctccaaaa attgatccca ctgtgactat gatgcaagtt gaggagaagc ctgatgttac     600
   atacagtgat gttggtggct gcaaggaaca aattgaaaag ttgagagaag ttgttgaaac     660
   acctttactc catcctgaaa aatttgtaaa acttggtatt gaaccaccaa aaggcgttct     720
   cctttttggc ccccctggaa ctggtaaaac tctttgtgct agagcagtag ccaatagaac     780
   agatgcctgc tttattcgtg taattggttc tgaactggtt cagaagtacg taggagaggg     840
   agcaagaatg gttagagaac ttttcgaaat ggctaggtct aagaaggcct gcttgatatt     900
   ctttgatgaa attgatgcca ttggtggagc cagatttgat gatggcgctg gaggtgataa     960
   cgaggtacaa cgtactatgt tagaacttat caatcagttg gatggttttg atcctagagg    1020
   taacattaaa gtacttatgg ctacaaatcg tccagatact ctagatcctg cccttatgag    1080
   acctggtcgt ttggatcgta aagttgaatt tggacttcca gaccttgaag gaagaactca    1140
   tattttcaaa attcatgcta ggtccatgtc tgttgaaaga gatattcgtt ttgaactttt    1200
   ggctaggttg tgtccaaatt ccactggagc tgaaatcaga agtgtttgta ccgaagctgg    1260
   tatgttcgct ataagagcaa ggaggaaagt agctaccgaa aaagacttct tagaagctgt    1320
   aaataaagta attaagtcat acgccaagtt ctctgctaca ccaagatata tgacttacaa    1380
   ttaattttaa agattttcta attatcgaat tatttaaatt gcgtttctaa ataaataatc    1440
   cttttttagt gt                                                         1452

<210>  201
<211>  458
<212>  DNA
<213>  Diabrotica virgifera


<220>
<221>  misc_feature
<222>  (1)..(458)
<223>  RNAi target sequence

<400>  201
tctcctgaag ggaggttgta tcaggtggag tatgccatgg aggctatttc tcatgcaggt      60

acttgtctgg gcattttagc aaatgatgga attttgttgg ctgctgagag gcgtaacact     120

aacaaattat tagatgaagt ttttatttca gaaaaaatat acaaactaaa tgatgatatg     180

gtttgtagtg tggctggaat aacatcagat gctaatgttt taacaaatga attgagactt     240

ataggtcaaa gatatttatt tcagtatgcc gaatctattc cttgcgaaca gttagtttcc     300

tggctttgtg atgtcaagca ggcatacact cagtatggag gtaaacgtcc atttggtgtt     360

tcaatattat atatgggatg ggataaacat tatggatacc agctatatca gtcagatccc     420

agtggcaact attctggttg gaaagctacc tgcatagg                             458


<210>  202
<211>  22
<212>  DNA
<213>  Artificial Sequence

<220>
<223>  primer


<220>
<221>  misc_feature
<222>  (1)..(22)
<223>  forward primer

<400>  202
tctcctgaag ggaggttgta tc                                               22


<210>  203
<211>  22
<212>  DNA
<213>  Artificial Sequence

<220>
<223>  primer


<220>
<221>  misc_feature
<222>  (1)..(22)
<223>  reverse primer

<400>  203
cctatgcagg tagctttcca ac                                                22

<210>  204
<211>  892
<212>  DNA
<213>  Diabrotica virgifera

<220>
<221>  misc_feature
<222>  (1)..(892)
<223>  WCRW transcript ta04612.001_diavv

<400>  204
tttaaatttt ctaggctcag tatttttagc tatattgagt atcaactaaa actatggctc       60

gtcgatatga taccagaacc acaatattct ctcctgaagg gaggttgtat caggtggagt      120

atgccatgga ggctatttct catgcaggta cttgtctggg cattttagca aatgatggaa      180

ttttgttggc tgctgagagg cgtaacacta acaaattatt agatgaagtt tttatttcag      240

aaaaaatata caaactaaat gatgatatgg tttgtagtgt ggctggaata acatcagatg      300

ctaatgtttt aacaaatgaa ttgagactta taggtcaaag atatttattt cagtatgccg      360

aatctattcc ttgcgaacag ttagtttcct ggctttgtga tgtcaagcag gcatacactc      420

agtatggagg taaacgtcca tttggtgttt caatattata tatgggatgg gataagcatt      480

atggatacca gctatatcag tcagatccca gtggcaacta ttctggttgg aaagctacct      540

gcataggcaa caacagttca gcagcaattt caagtttgaa acaagaatat aaagaaggtc      600

aaatgacttt gaaggatgca aagacgttag cagtaaaggt tctggcaaaa acattggata      660

tggcaaaact aacttcagaa aaagtggaaa tggctacact tacccgcaaa aatgataaaa      720

ccatgattaa aattttaagc ggcaaggaag tagaagaact gatttcagag tatgaaaaaa      780

gtgaagctgc cgcagaagct agcaaaaaag agcagaaggc atcagcttaa ttacatttat      840

ttggttttgt ttaggttcac taagtatgtc attgtcacaa tatgaataaa tg              892

<210>  205
<211>  453
<212>  DNA
<213>  Diabrotica virgifera

<220>
<221>  misc_feature
<222>  (1)..(453)
<223>  RNAi target sequence

<400>  205
tgcagcttat agtggctgaa aagagtcaaa atcttagacg tttgcaagct cagagaaatg       60

aactcaatgc caaagttcgt atgttgagag aagaactgat gttacttcaa gagcaaggaa      120

gctatgtcgg agaagttgta aagcccatgg ataagaaaaa agtccttgtg aaagttcatc     180

cagaaggaaa atttgtagtt gacattgaca aaaacataga cataaatgat gttactccaa     240

attgtcgagt agccctcaga aatgaatctt acacattgca caaaatactt ccaaacaagg     300

ttgatccttt agtttccttg atgatggtgg agaaagtccc tgattcaact tatgaaatgg     360

taggaggact tgacaagcaa attaaagaaa tcaaagaagt aattgaactt ccagttaaac     420

atccagaact tttgatgctc cttggtattg cac                                   453

<210>  206
<211>  22
<212>  DNA
<213>  Artificial Sequence

<220>
<223>  primer

<220>
<221>  misc_feature
<222>  (1)..(22)
<223>  forward primer

<400>  206
tgcagcttat agtggctgaa aa                                              22

<210>  207
<211>  22
<212>  DNA
<213>  Artificial Sequence

<220>
<223>  primer

<220>
<221>  misc_feature
<222>  (1)..(22)
<223>  reverse primer

<400>  207
gtgcaatacc aagagcatca aa                                              22

<210>  208
<211>  1322
<212>  DNA
<213>  Diabrotica virgifera

<220>
<221>  misc_feature
<222>  (1)..(1322)
<223>  WCRW transcript ta01297.001_diavv

<400>  208
aaactcccgg agtgcaaaaa actatcaaga atgacggtaa ttaacaagat ggaggtggac      60

ggaggtccga agggagaagg ttttcggtca tattatatta caaagattga agaattgcag 120

cttatagtgg ccgaaaagag tcaaaatctt agacgtttgc aagctcagag aaatgaactc 180

aatgccaaag ttcgtatgtt gagagaagaa ctgatgttac ttcaagagca aggaagctat 240

gtcggagaag ttgtaaagcc catggataag aaaaaagtcc ttgtgaaagt tcatccagaa 300

ggaaaatttg tagttgacat tgacaaaaac atagacataa atgatgttac tccaaattgt 360

cgagtagccc tcagaaatga atcttacaca ttgcacaaaa tacttccaaa caaggttgat 420

cctttagttt ccttgatgat ggtggagaaa gtccctgatt caacttatga aatggtagga 480

ggacttgaca agcaaattaa agaaatcaaa gaagtaattg aacttccagt taaacatcca 540

gaactttttg atgctcttgg tattgcacaa cctaagggtg tgttgttata tggaccacca 600

ggcactggaa aaactcttct ggcacgtgct gtagctcatc acacagagtg tacatttatt 660

agagtatctg gttcagaatt ggtacagaaa ttcataggag aaggatcccg tatggtaaga 720

gaactcttcg taatggccag agaacatgct ccttcaatta ttttcatgga tgaaattgat 780

tccattggat cttcccgtat tgaatcagga tctggtggtg attctgaagt ccaaagaacc 840

atgttggaac ttctaaatca attggatggt tttgaagcta ccaaaaacat caaagtaatt 900

atggctacca acagaattga tattttggat cccgctttac tcagaccagg aagaattgac 960

agaaaaattg aattcccccc accgaacgaa gaagctagat tggacatctt aaagattcac 1020

tccagaaaaa tgaacctcac aagaggaata aatctgagaa aaatagctga gttgatgcca 1080

ggtgctagtg gagctgaagt taagggtgtg tgtacggaag ctggtatgta tgcactaaga 1140

gaacgaaggg tgcatgttac tcaagaagat tttgaaatgg ccgtcgccaa agttatgcag 1200

aaggattctg agaagaatat gtccatcaag aagttgtgga aataagtttt ttgtactact 1260

taatatgtga ttatttttta agaaataaaa tatagcgaat agataaaaaa aaaaaaaaaa 1320

aa 1322

<210> 209
<211> 488
<212> DNA
<213> Diabrotica virgifera


<220>
<221> misc_feature
<222> (1)..(488)
<223> RNAi target sequence

<400> 209
gttaagatgc cggaccattt aggagatgat atgcgaaaaa taaaagaaca gaaagaggag 60

cccgaaaagg aaattaaagc cctggatgaa ggtgatatag cacttcttaa aacctatgga 120

caagggcaat acacaaagac aattaaaaca tgtgaagaag atatccaaac gataatcaaa 180

agagtaaatg aattaactgg tataaaagaa tctgatactg gtttagctcc accagcttta 240

tgggatttgg cagctgacaa acaaacctta caaaatgagc aacccttaca agtggcccgt 300

tgtacgaaaa ttatcaatgc tgatacagat gatcccaaat acattataaa cgttaaacag 360

tttgccaaat tcgtagtaga tttggcagac tctgtggccc ctactgacat agaagaaggc 420

atgagagttg gagtcgaccg aaataagtat caaattcata ttcctctacc tccaaaaatt 480

gatcccac 488

<210> 210
<211> 22
<212> DNA
<213> Artificial Sequence

<220>
<223> primer

<400> 210
gttaagatgc cggaccattt ag 22

<210> 211
<211> 22
<212> DNA
<213> Artificial Sequence

<220>
<223> primer

<220>
<221> misc_feature
<222> (1)..(22)
<223> reverse primer

<400> 211
gtgggatcaa tttttggagg ta 22

<210> 212
<211> 1425
<212> DNA
<213> Diabrotica virgifera

<220>
<221> misc_feature
<222> (1)..(1425)
<223> WCRW transcript 454run3_isotig16670

<400> 212
aaaatttgct tgtaatcttg cacaaaaata ttttaagaac aatatttttc atagatttag 60

ttttaaataa agacaattaa gatgccggac catttaggag atgatatgcg aaaaataaaa 120

gaacagaaag aggagcccga aaaggaaatt aaagccctgg atgaaggtga tatagcactt 180

cttaaaacct atggacaagg gcaatacaca aagacaatta aaacatgtga agaagatatc 240

caaacgataa tcaaaagagt aaatgaatta actggtataa aagaatctga tactggttta 300

gctccaccag ctttatggga tttggcagct gacaaacaaa ccttacaaaa tgagcaaccc 360

ttacaagtgg cccgttgtac gaaaattatc aacgctgata cagatgatcc caaatacatt 420

ataaacgtta aacagtttgc caaattcgta gtagatttgg cagactctgt ggcccctaca 480

gacatagaag aaggcatgag agttggagtc gaccgaaata agtatcaaat tcatattcct 540

ctacctccaa aaattgatcc cactgtgact atgatgcaag ttgaggagaa gcctgatgtt 600

acatacagtg atgttggtgg ctgcaaggaa caaattgaaa agttgagaga agttgttgaa 660

acacctttac tccatcctga aaaatttgta aaacttggta ttgaaccacc aaaaggcgtt 720

ctcctttttg gcccccctgg aactggtaaa actctttgtg ctagagcagt agccaataga 780

acagatgcct gctttattcg tgtaattggt tctgaactgg ttcagaagta cgtaggagag 840

ggagcaagaa tggttagaga acttttcgaa atggctaggt ctaagaaggc ctgcttgata 900

ttctttgatg aaattgatgc cattggtgga gccagatttg atgatggcgc tggaggtgat 960

aacgaggtac aacgtactat gttagaactt atcaatcagt tggatggttt tgatcctaga 1020

ggtaacatta aagtacttat ggctacaaat cgtccagata ctctagatcc tgcccttatg 1080

agacctggtc gtttggatcg taaagttgaa tttggacttc cagaccttga aggaagaact 1140

catattttca aaattcatgc taggtccatg tctgttgaaa gagatattcg ttttgaactt 1200

ttggctaggt tgtgtccaaa ttccactgga gctgaaatca gaagtgtttg taccgaagct 1260

ggtatgttcg ctataagagc aaggaggaaa gtagctaccg aaaaagactt cttagaagct 1320

gtaaataaag taattaagtc atacgccaag ttctctgcta caccaagata tatgacttac 1380

aattaatttt aaagattttc taattatcga attatttaaa ttgcg 1425

<210> 213
<211> 553
<212> DNA
<213> Diabrotica virgifera

<220>
<221> misc_feature
<222> (1)..(553)
<223> RNAi target sequence

<220>
<221> misc_feature
<222> (485)..(485)
<223> n can be any nucleotide

<400> 213
caaaggaact cgcagagctt ataaaggcca ccaggccttt tttgagttta ataagcaaag 60

caaaagctgc taaattggta aggtctctag ttgactactt cctagattta gaagctggta 120

ttggtatcga agtacaactc tgcaaagaat gtatagaatg ggcaaaggaa gagagaagaa 180

cttttctacg tcagtccctt gaagccaggc tgattgctct ttattttgac actggaatgt 240

acagcgaagc gctacaactg gaatcgacct tactgaaaga gttaaaaaag ttggatgaca 300

aaaatttgtt ggtggaagta caactacttg aaagcaaaac atatcatgct ttaagtaatt 360

taccgaaggc tagagctgca ctaacttcag cacgaaccac agctaacgct atctattgcc 420

ctccaaagat gcaggcagct ttagatttac aatccggagt attacacgct gcagatgaga 480

agganttcaa aacagcttat tcttattttt atgaagcttt tgaaggtttt gatagtgttg 540

aatccccaaa agc 553

<210> 214
<211> 22
<212> DNA
<213> Artificial Sequence

<220>
<223> primer

<220>
<221> misc_feature
<222> (1)..(22)
<223> forward primer

<400> 214
caaaggaact cgcagagctt at 22

<210> 215
<211> 22
<212> DNA
<213> Artificial Sequence

<220>
<223> primer

<220>
<221> misc_feature
<222> (1)..(22)
<223> reverse primer

<400> 215
gcttttgggg attcaacact at 22

<210> 216
<211> 1860
<212> DNA
<213> Diabrotica virgifera

<220>
<221> misc_feature
<222> (1)..(1860)
<223> WCRW transcript ta03917.001_diavv

<400> 216
gggtcagatc cgatcatatt aacgtgaaaa gcatatttat gacagacaaa ttaatggaag 60

tttagtagtg agtagtgaaa aaatatggcc ggagcaatgt tatttgaaag agccccgtct 120

gtttcttctc aacacgagaa gcttttaaaa ttatccaggg attcagatga tgaagatcaa 180

aatattgtca ataaagaaca agacatcctt aatcttggtg agaaatataa aaaggagggc 240

aaggcaaagg aactcgcaga gcttataaag gccaccaggc cttttttgag tttaataagc 300

aaagcaaaag ctgctaaatt ggtaaggtct ctagttgact acttcctaga tttagaagct 360

ggtattggta tcgaagtaca actctgcaaa gaatgtatag aatgggcaaa ggaagagaga 420

agaacttttc tacgtcagtc ccttgaagcc aggctgattg ctctttattt tgacactgga 480

atgtacagcg aagcgctaca actggaatcg accttactga aagagttaaa aaagttggat 540

gacaaaaatt tgttggtgga agtacaacta cttgaaagca aaacatatca tgctttaagt 600

aatttaccga aggctagagc tgcactaact tcagcacgaa ccacagctaa cgctatctat 660

tgccctccaa agatgcaggc agctttagat ttacaatccg gagtattaca cgctgcagat 720

gagaaggatt tcaaaacagc ttattcttat ttttatgaag cttttgaagg ttttgatagt 780

gttgaatccc caaaagccct cacagccctt aaatacatgc ttttgtcaaa aatcatgata 840

aacagtccgg aagacgtcca acaaatcgtt agtggtaaat tggcctttaa atacgctggt 900

caagatatag aggctatgaa atcagttgca caggctgcac acaaaagatc actagcagat 960

ttccaacaag ctgtaaaaca gtttaaacat gaattagaag atgatgttat tgttagagca 1020

catttaggaa cgttgtatga caatatgttg gagcagaacc tttgcagaat tatcgaacca 1080

tattctaggg tacaagtaga gtatgtagca aaaaccataa aactcccagt actacaagta 1140

gagaagaaat tgtcacagat gattttagat gccaagtttc atggaatttt ggaccaaggc 1200

gaaggtgtcc tcatagtatt cgaagaaagt gcagtagaca aaacatatga aatggcccta 1260

gaaacgatcc agagtatgag caaagtagta gatactcttt atcaaaaagc aaagaaattg 1320

tcataggtat aatgtagccg agaatgttat gaaaataata aaatgactgt aaatttttca 1380

gaactagatt ttttgtagac actgtgttta tataacctat ttaatttgct tttgttaatt 1440

cttgtctttt taaggattct tataactcat agttttcgga gcttgagggt tttggatgtg 1500

atggagtttt tttgttaata cagtatagat attttttaag atacttaaag cttatctaaa 1560

tctaggaaaa tatcatgcct aaagtcataa gagattcata atccatatgt aactaactca 1620

tagaactatt ttctttataa tattttttac aagcaatgaa ataaaagcct caaaaaatag 1680

actgaacttt agagacttta ggcattgagt tttggtcttt attgggaaaa tattaataca 1740

aaagttctat aagaaatatc tttgtatggt ttattaattc attttttaac ctgccaagac 1800

attgtgatct ctattactat cagtgtcatt ggcttgtttt catacaattt ttccctaata 1860

<210>  217
<211>  469
<212>  DNA
<213>  Diabrotica virgifera

<220>
<221>  misc_feature
<222>  (1)..(469)
<223>  RNAi target sequence

<220>
<221>  misc_feature
<222>  (294)..(294)
<223>  n can be any nucleotide

<220>
<221>  misc_feature
<222>  (295)..(295)
<223>  n can be any nucleotide

<220>
<221>  misc_feature
<222>  (296)..(296)
<223>  n can be any nucleotide

<220>
<221>  misc_feature
<222>  (385)..(385)
<223>  n can be any nucleotide

<220>
<221>  misc_feature
<222>  (386)..(386)
<223>  n can be any nucleotide

<400>  217
gtcaatacct tttcacccga aggaagatta tttcaagtac aatatgccat acaagctatt      60

aagttaggat ctaccgctat tggaatatgc accagtggtg gagtagtgct tgctgtacaa     120

aaacgcatta cttccccatt aatggaacct actacgattg agaaaattgt agaagttgac     180

aaacatattg gatgtgcggt ttcaggactt atggcagatt caaggactat gatagataga     240

gctcgtgtgg aatgtcacaa tcattggttt gtctacaatg aaaatataag tgtnnnatcc     300

tgtgcacaag gtgtatcaaa tttggctatt cagttcggat attctgatga agatggtgca     360

gccatgtcta gaccttttgg agtannaatt ttgtttgctg gtatagatga aaaaggacct     420

cagttgtacc atatggatcc atcaagaaca tttgttcagt ttgacgcaa                 469


<210>  218
<211>  22
<212>  DNA
<213>  Artificial Sequence

<220>
<223>  primer

<220>
<221>  misc_feature
<222>  (1)..(22)
<223>  forward primer

<400>  218
gtcaatacct tttcacccga ag                                                22


<210>  219
<211>  21
<212>  DNA
<213>  Artificial Sequence

<220>
<223>  primer


<220>
<221>  misc_feature
<222>  (1)..(21)
<223>  reverse primer

<400>  219
ttgcgtcaaa ctgaacaaat g                                                 21


<210>  220
<211>  850
<212>  DNA
<213>  Diabrotica virgifera


<220>
<221>  misc_feature
<222>  (1)..(850)
<223>  WCRW transcript ta09615.001_diavv

<400>  220
gctattacaa gtttcttttg tataagtttt ataaatattg ttaatcgtta acatagtcct      60

gtttacaaaa tgtttcttac aagaagtgaa tacgataggg gagtcaatac cttttcaccc     120

gaaggaagat tatttcaagt agaatatgcc atagaagcta ttaagttagg atctaccgct     180

attggaatat gcaccagtgg tggagtagtg cttgctgtag aaaaacgcat tacttcccca     240

ttaatggaac ctactacgat tgagaaaatt gtagaagttg acaaacatat tggatgtgcg     300

gtttcaggac ttatggcaga ttcaaggact atgatagata gagctcgtgt ggaatgtcag     360

aatcattggt ttgtctacaa tgaaaatata agtgtagaat cctgtgcaca aggtgtatca     420

aatttggcta ttcagttcgg agattctgat gaagatggtg cagccatgtc tagacctttt     480

ggagtagcaa ttttgtttgc tggtatagat gaaaaaggac ctcagttgta ccatatggat     540

ccatcaggaa catttgttca gtttgacgca aaggcgatag gatcaggatc cgagggtgca     600

cagcaaagtt tacaggaggc atatcatcgc agtatgaccc tggaagaagc taccacaact     660

gctttaacaa ttctaaaaca agttatggaa gaaaagttga attcttcaaa tgttgaggtt     720

atgactatga ctccaaaaga tatgttccac atgttcacaa aggaacaagt ggaagaggta     780

attgctaaat tgtcttaact taggaagtaa gctaaatttt gaaaagggtt aattttatat     840

aagttttttt                                                            850

<210>  221
<211>  404
<212>  DNA
<213>  Diabrotica virgifera

<220>
<221>  misc_feature
<222>  (1)..(404)
<223>  RNAi target sequence

<220>
<221>  misc_feature
<222>  (300)..(300)
<223>  n can be any nucleotide

<220>
<221>  misc_feature
<222>  (301)..(301)
<223>  n can be any nucleotide

<220>
<221>  misc_feature
<222>  (302)..(302)
<223>  n can be any nucleotide

<220>
<221>  misc_feature
<222>  (338)..(338)
<223>  n can be any nucleotide

<220>
<221>  misc_feature
<222>  (339)..(339)
<223>  n can be any nucleotide

<220>
<221>  misc_feature
<222>  (340)..(340)
<223>  n can be any nucleotide

<220>
<221>  misc_feature
<222>  (341)..(341)
<223>  n can be any nucleotide

<400>  221
ttcggaagta aaacaagtgc tgcaggatgt ggatactgaa gatttgtaca ctaaatataa      60

aaaacttcaa caacagttgg agttcttgga ggttcaagaa gagtacatta aagatgagca     120

aaggaatctc aaaaaggagt atttacatgc tcaggaagaa gtgaagcgta tccagtctgt     180

cccgcttgtt attggacagt ttttagaagc tgtagatcaa aacactggca ttgttggctc     240

aactacaggt tcaaattatt atgttagaat cttatcaaca attgatagag aactcttaan 300

nnccttctgc tagtgttgct cttcataaac acagcaannn ncttagtaga tgtcctacca 360

ccagaggcag attcttcaat aagcatgctc caggctgatg aaaa 404

<210> 222
<211> 22
<212> DNA
<213> Artificial Sequence

<220>
<223> primer

<220>
<221> misc_feature
<222> (1)..(22)
<223> forward primer

<400> 222
ttcggaagta aaacaagtgc tg 22

<210> 223
<211> 20
<212> DNA
<213> Artificial Sequence

<220>
<223> primer

<220>
<221> misc_feature
<222> (1)..(20)
<223> reverse primer

<400> 223
ttttcatcag cctggagcat 20

<210> 224
<211> 1364
<212> DNA
<213> Diabrotica virgifera

<220>
<221> misc_feature
<222> (1)..(1364)
<223> WCRW transcript ta03370.001_diavv

<400> 224
aaaaaagtca aaatttgaag aagaaaacaa ttttacaagt catcaaccat ggaggctatg 60

gatattgtta taccagaaaa agaagaaggt agtccttcgg aagtaaaaca agtgctgcag 120

gatgtggata ctgaagattt gtacactaaa tataaaaaac ttcaacaaca gttggagttc 180

ttggaggttc aagaagagta cattaaagat gagcaaagga atctcaaaaa ggagtattta 240

catgctcagg aagaagtgaa gcgtatccag tctgtcccgc ttgttattgg acagttttta 300

gaagctgtag atcaaaacac tggcattgtt ggctcaacta caggttcaaa ttattatgtt 360

agaatcttat caacaattga tagagaactc ttaaaacctt ctgctagtgt tgctcttcat 420

aaacacagca atgccttagt agatgtccta ccaccagagg cagattcttc aataagcatg 480

ctccaggctg atgaaaaacc agatgttcaa tacagtgaca ttggaggtat ggatatgcag 540

aaacaagaaa ttagggaagc tgtagagtta cctttgactc acttcgaatt gtataaacag 600

attggtattg atccccccag aggtgtgttg atgtatggac ctcctggatg cgggaagact 660

atgttggcca aagctgttgc tcatcatact acagctgcct ttattcgtgt agtcggatca 720

gaattcgtac agaaatatct tggtgaaggt cctcgaatgg ttagagacgt gttccgtcta 780

gcaaaagaaa attctccagc aatcatcttt attgatgaaa ttgatgcaat cgctaccaaa 840

cgttttgatg cccagacagg tgctgatcgt gaagtccaga gaattttatt ggaactgctt 900

aaccagatgg atggttttga tcagacaaca aatgttaagg ttatcatggc aactaacaga 960

gctgatacat tagatcctgc acttctgaga cctggtcgtc tggacagaaa aatcgaattt 1020

cctttaccag atagaagaca aaagcgtttg attttcagca ctatcacatc taaaatgaat 1080

ttgtcagagg aggtcgactt ggaggactat gtagctagac cagacagaat atcaggagca 1140

gacataaacg ccatctgcca ggaagccggt atgcatgccg tccgagaaaa ccgttacatc 1200

gttttaccta aagatttcga gaagggttat aagaataaca ttaagaagga tgagagcgag 1260

cacgagtttt acaaatagtt tatttatatt ttttattatt actaatctaa taaagacttg 1320

taaaatgtat ttgatgtcat tgaatcaagt ttacattaaa aaaa 1364

<210> 225
<211> 455
<212> DNA
<213> Diabrotica virgifera

<220>
<221> misc_feature
<222> (1)..(455)
<223> RNAi target sequence

<400> 225
cggaattaaa tagtgcccaa gatgaatatc acatctgcag cgggaattat ttccctcttg 60

gatgaaccaa gaccagaatt aaaagtattt gcactgaaga aactagattc catagtgggt 120

gagttttggc cggagatctc agaagctatc gaaaaaatcg aaatttgcca tgaagacaaa 180

gttttccagc aacaccagtt agctgcactt gtggcaagta aggtatatta ccatttaggc 240

gcttttgaag actctttaac ttatgctctt ggtgcgggtg atctctttga tgtcaatgcc 300

cggagtgagt atgtagaaac tattctagct aaatgtatcg actattacac tcagcaacgt 360

attgctgtag ctgaaggatt ggcagatgcc aaacccatag actctcgcct tgaagctatt 420

gttaatcgaa tgtttcagag gtgtcttgat gatgg 455

<210> 226
<211> 22
<212> DNA
<213> Artificial Sequence

<220>
<223> primer

<220>
<221> misc_feature
<222> (1)..(22)
<223> forward primer

<400> 226
cggaattaaa tagtgcccaa ga 22

<210> 227
<211> 22
<212> DNA
<213> Artificial Sequence

<220>
<223> primer

<220>
<221> misc_feature
<222> (1)..(22)
<223> reverse primer

<400> 227
ccatcatcaa gacacctctg aa 22

<210> 228
<211> 3242
<212> DNA
<213> Diabrotica virgifera

<220>
<221> misc_feature
<222> (1)..(3242)
<223> WCRW transcript ta00483.001_diavv

<400> 228
tgaatatcca catcaaaaat tgcattcatt caatgactgt gttgtttttc cgacgtgaaa 60

gtttaaaaat tacatttttc ggaattaaat agtgcccaag atgaatatca catctgcagc 120

gggaattatt tccctcttgg atgaaccaag accagaatta aaagtatttg cactgaagaa 180

actagattcc atagtgggtg agttttggcc ggagatctca gaagctatcg aaaaaatcga 240

aattttgcat gaagacaaag ttttccagca acaccagtta gctgcacttg tggcaagtaa 300

ggtatattac catttaggcg cttttgaaga ctctttaact tatgctcttg gtgcgggtga 360

tctctttgat gtcaatgccc ggagtgagta tgtagaaact attctagcta aatgtatcga 420

ctattacact cagcaacgta ttgctgtagc tgaaggattg gcagatgcca aacccataga 480

ctctcgcctt gaagctattg ttaatcgaat gtttcagagg tgtcttgatg atggtcaata 540

tcgtcaagct atgggcctgg ctctagaaac cagacgaatg gatatctttg agtcatctat 600

tactcagtct gatgatatta tcagcatgtt gacttatgca tttcaagtag cgatgagttt 660

gattcaaaat cgagcattta gaaacactgt tttgagatct cttgttggac tttatagagg 720

actggttaca cctgattacg ttaatatgtg ccagtgctta attttcctcg atgatccatt 780

gtctgtggct gaaattttag acaggcttgt aactggcaag gaggatactg aacttatggc 840

atatcaaatt gcttttgatc tctatgagtc agctactcaa cagtttttgg aaagagtatt 900

agttgcatta agagccacag caccagttcc atctttgctg gaagaaaaag caaagccaaa 960

gccttctggt gaaggagagg gtgctatgtc aaccgagact ggagaaccat ctacagaaac 1020

aaaagaagaa aaattaaaag aagaaaaatc tctggatagt ttgtctgatc aggataagga 1080

acatcagaaa agaattgaga aacttcatac tatactctca ggggaggtct ctatagaact 1140

gcaccttcaa ttcctcatca ggtcaaatca tgctgatcta ctgattctta aacaaacaaa 1200

agagactgtc agagttagta tttgccacac tgctactgta atcgcaaacg catttatgca 1260

tagtggtact actagcgatc aatttttaag agataactta gaatggttag ccagagcaac 1320

gaattgggcc aaacttacag ccacagcctc gctaggagtt attcatagag gccatgaaca 1380

agaagcatta acactaatgc aaagttactt gccgaaagaa gttggaccca gctcaggtta 1440

ttccgaaggg ggcggccttt atgctcttgg tcttatacat gccaaccatg gtacaaatat 1500

cattgattac cttcttggac aacttaagga tgcacagaat gagatggtga gacatggagg 1560

ttgtcttggt ctaggtttgg ctgctatggg cactcataga caggatgttt atgaacaact 1620

caaattcaat ttataccaag atgatgccaa tactggtgaa gcagctggta ttgcaatggg 1680

aatggtaatg ttgggttcaa gtaattctac cacaattatg gatatggttg catacgcaca 1740

agagagtcaa catgagaaaa ttcttagagg cttagctgtt ggtattagtt tttccatgta 1800

tggtagatta gaagaagcag atccactgat tcaacaactt acctctgata aagatcctat 1860

cctcagaaga tcaggaatgt atactttggg tatggcatat tgtggtactg gacacaacca 1920

agcaattaga aaactacttc atgtagctgt gtcagatgtc aacgatgatg ttagaagagc 1980

tgcagttaca gctttaggtt ttctgttatt cagaactcct gaacaatgtc ctagtgtagt 2040

atcccttcta gctgagagtt acaatccaca tgtacgttat ggagctgcta tggctcttgg 2100

tattgcatgt gcaggaactg gtctacgtga agctatagct cttctcgaac ctatggttat 2160

gtttgatcct gtaaacttcg taagacaggg tgctctcatt gcttctgcta tgattttaat 2220

tcaacagact gaacaaactt gccccaaagt cacattcttc agacagacat atgctcaagt 2280

catttccaac aaacacgaag atgtgatggc caagtttgga gctatcttag ctcaaggaat 2340

catcgatgca ggtggaagaa atgtaacttt gtcgctacag tccagaactg gccacacaaa 2400

tatgttagct gtagttggta cactggtgtt cactcagtac tggtactggt tccctctatc 2460

acattgttta gctttagcat tcactccaac atgtgtgatt ggtcttaatg cacaattgaa 2520

gatgcccaaa ttagacttca aatccaatgc caaacctagt ctatatgctt atccagctcc 2580

aatggaagaa aagaaaagag aagaaagaga aaaggtaact actgccgttt taagtattgc 2640

tgcccgccaa cgtagaagag accatgaaag aaagcatcgt gacgaaaaga tggaagtaga 2700

tgaagacaaa gatgacaaga aaacagaaaa agaagacaag gagaagaagt ctgaagagaa 2760

agataaaaag gatgaaaaga aagctgatga caaagataaa gataaaaaag cagatgacaa 2820

gaaagcatct acttctactg gcgatgataa gaagaaggat gaaaaggaag aaaagaagaa 2880

agaacctgaa ccaaacttcg aagttttaca gaatcctgct agagttatga ggcaacaatt 2940

aaaggtgatt acacttaatg atagcaatca acatgtttcg atgaaagatg tttctattgg 3000

aggaataatc atggtgagaa atttgaagcc tggagatgag gaactggttg aacctgttgc 3060

agcttttggt cccaagggcg aggatgaaaa ggaaccagaa cctccagagc cgtttgaatg 3120

gacagaagat tagatatcat cattaacatt tttataattt ttcatttaat ttactataaa 3180

tagatgtaat tatacgaaaa acgacttttg ttaacattta atattaaatt attagtttag 3240

cg 3242

<210> 229
<211> 701
<212> DNA
<213> Diabrotica virgifera

<220>
<221> misc_feature
<222> (1)..(701)
<223> RNAi target sequence

<400> 229
agctcgcttt tatatgagat ccgcaacgtt cataggcatt tcatctatct ttgtattata 60

gaactgttcg atatccttaa gcgtgcgttt atcatcgtcc gttacgaaat ttattgctac 120

acccttacga ccaaatcgac cacctcgacc aatcctgtga atgtaatttt ctctattaga 180

aggcaaatca taattaataa ctagggaaac ttgctgtaca tctatacctc tcgctaacag 240

atcagtggtt attaaaaccc tagatgatcc tgttctgaac tgacgcataa taacgtcacg 300

ctctctttgt tccatatcac catgcatagc ggaaacagtg aagtctctct tgtgcatgtt 360

ttctgttaac caatcgacct tgcgacgggt attgcaaaag attacagcct gcgtaatcga 420

aagagtatca tagagatcac ataatgtctc taatttccaa tcttcttttt ctacatatac 480

ataaaattgt ttaatacctt ctaaggtaag ttcctctttt ttgacaagta tccgaacagg 540

actcctcata aaggattttg tcacgtctag cacatcgttt ggcatggtag ccgaaagtaa 600

aattacctgt acgtcagtgt tcaacatttt gaacacgtca tgaatttgat ctttaaaacc 660

ccttgacaac atttcatcgg cttcatccaa aacaaacatt t 701

<210> 230
<211> 27
<212> DNA
<213> Artificial Sequence

<220>
<223> primer

<220>
<221> misc_feature
<222> (1)..(27)
<223> forward primer

<400> 230
agctcgcttt tatatgagat ccgcaac 27

<210> 231
<211> 28
<212> DNA
<213> Artificial Sequence

<220>
<223> primer

<220>
<221> misc_feature
<222> (1)..(28)
<223> reverse primer

<400> 231
aaatgtttgt tttggatgaa gccgatga 28

<210> 232
<211> 2338
<212> DNA
<213> Diabrotica virgifera

<220>
<221> misc_feature
<222> (1)..(2338)
<223> WCRW transcript ta03651.003_diavv

<400> 232
aaacgaccac agacagactc actttcaggt aaaaaaatcc taacttattt gttaccacaa 60

gttagaagtg gttaaaagca tcctagaagc cactaagtga attgcaatac ctgggcggag 120

cgacagtatt cctatcactt caaacttcaa ttcagggggc ttttccgttt agctatacgc 180

gaagtagaca tgtctatgcc agatcgtcgt gaagagtggg acaagaatgg cccagctaat 240

gaagacagcc agccccaagg ctatgacggc ccaccaggta tggagcctga tggcattatt 300

gaatccaact gggaagaggt ggtcgataat ttcgacgaca tgaacctaaa agaggaactt 360

ttaagaggaa tttatgccta tggttttgaa aaaccttctg ctatccaaca gagagccatc 420

attccttgtg taaagggaca cgatgttatt gctcaggctc agtcaggtac tggtaagact 480

gcaacattct ccatctctat tttacaacaa attgacacgt cagtgaggga gtgccaagct 540

cttatattgg caccaactcg tgaattggct caacagatcc aaaaagtagt aattgcccta 600

ggagatttca tgtcagcgca gtgtcatgca tgtattggtg gtaccaatgt acgagaagac 660

ataagaaaac ttgaaactgg cgtacatgtt gttgttggta cacctggtag agtttacgat 720

atgataacga ggagagccct aaggccgact catattaaaa tgtttgtttt ggatgaggct 780

gatgaaatgt tgtcaagggg ttttaaagat caaattcatg acgtgttcaa aatgttgaac 840

actgacgtac aggtaatttt actttcggct accatgccaa acgatgtgct agacgtgaca 900

aaatccttta tgaggagtcc tgttcggata cttgtcaaaa aagaggaact taccttagaa 960

ggtattaaac aattttatgt atatgtagaa aaagaagatt ggaaattaga gacattatgt 1020

gatctctatg atactctttc gattacgcag gctgtaatct tttgcaatac ccgtcgcaag 1080

gtcgattggt tgacagaaaa catgcacaag agagacttca ctgtttccgc tatgcatggt 1140

gatatggaac aaagagagcg tgacgttatt atgcgtcagt tcagaacagg atcatctagg 1200

gttttaataa ccactgatct gttagcgaga ggtatagatg tacaacaagt ttccctagtt 1260

attaattatg atttgccttc taatagagaa aattacattc acaggattgg tcgaggtggt 1320

cgatttggtc gtaagggtgt agcaataaat ttcgtaacgg acgatgataa acgcacgctt 1380

aaggatatcg aacagttcta taatacaaag atagatgaaa tgcctatgaa cgttgcggat 1440

ctcatataaa agcgagctaa taaagagtgt tggtgtgttc tacgacgtct cctgaagtat 1500

ctttggtgtg agtattagct ttctatgcta aatctgctgc gacatatata tgcgaatatt 1560

gtgcgctgcg ccccatcacg tttctttagg acaacgccgt tcgaggcaca atcatccacg 1620

aactctctga gtgtcaattt ttaaatatat ttaattgttt cggtttaatc tctagtgtat 1680

ttttaatttt ttgatataca tatagttagt gtttattatc attcgggaga cgtgtggctg 1740

tatggaggcc cgaggtcccc tcgtttcgag gggtaccgac ggtcgtaggc gtgttgtgat 1800

ttgctccaca cagttcgttc gtctctgtaa gggtttagtt ggtatctgga aaggttatta 1860

taatcgtaaa agatgtagaa ggaaattctg gattatcaca tagacttttt gtacattaag 1920

acttaataaa agtggtgata aaattcgttg cctttctttt atgttttatt ataactggtg 1980

tgtctaaaga tttgttgaat tgtgaatctg catatatact gtttagagag agactcacag 2040

gtgagtaatc gtactgtttg aacagaaact ttatagtaca ttcttagttt cattctgtgt 2100

tatatttgtt taataatcac agttaaagtt aacacaaaat gcaaaaaaca ctaacataaa 2160

cttttgatgt tgatagtttg ttgaaattat cttccgtgtt tgttatagtt ttttttggct 2220

ctagcttttg taaaagtaat aatcattagc gaactagtag gcctatggct tgattgacca 2280

ttcttcatcc tttcttgttc attgctttca tcctccaatt tgtgatgttg agtttttt 2338

<210> 233
<211> 669
<212> DNA
<213> Diabrotica virgifera

<220>
<221> misc_feature
<222> (1)..(669)
<223> RNAi target sequence

<400> 233
gtgaagagtg ggacaagaat ggcccagcta atgaagacag ccagccccaa ggctatgacg 60

gcccaccagg tatggagcct gatggcatta ttgaatccaa ctgggaagag gtggtcgata 120

atttcgacga catgaaccta aaagaggaac ttttaagagg aatttatgcc tatggttttg 180

aaaaaccttc tgctatccaa cagagagcca tcattccttg tgtaaaggga cacgatgtta 240

ttgctcaggc tcagtcaggt actggtaaga ctgcaacatt ctccatctct attttacaac 300

aaattgacac gtcagtgagg gagtgccaag ctcttatatt ggcaccaact cgtgaattgg 360

ctcaacagat ccaaaaagta gtaattgccc taggagattt catgtcagcg cagtgtcatg 420

catgtattgg tggtaccaat gtacgagaag acataagaaa acttgaaact ggcgtacatg 480

ttgttgttgg tacacctggt agagtttacg atatgataac gaggagagcc ctaaggccga 540

ctcatattaa aatgtttgtt ttggatgagg ctgatgaaat gttgtcaagg ggttttaaag 600

atcaaattca tgacgtgttt aaaatgttga acactgacgt acaggtaatt ttactttcgg 660

ctaccatgc 669

<210> 234
<211> 22
<212> DNA
<213> Artificial Sequence

<220>
<223> primer

<220>
<221> misc_feature
<222> (1)..(22)
<223> forward primer

<400>  234
gtgaagagtg ggacaagaat gg                                                22

<210>  235
<211>  22
<212>  DNA
<213>  Artificial Sequence

<220>
<223>  primer

<220>
<221>  misc_feature
<222>  (1)..(22)
<223>  reverse primer

<400>  235
gcatggtagc cgaaagtaaa at                                                22

<210>  236
<211>  1764
<212>  DNA
<213>  Diabrotica virgifera

<220>
<221>  misc_feature
<222>  (1)..(1764)
<223>  WCRW transcript 454run12_isotig05267

<400>  236
ttttcaccac ttttattaag tcttaatgta caaaaagtct atgtgataat ccagaatttc      60

cttctacatc ttttacgatt ataataacct ttccagatac caactaaacc cttacagaga     120

cgaacgaact gtgtggagca aatcacaaca cgcctacgac cgtcggtacc cctcgaaacg     180

aggggacctc gggcctccat acagccacac gtctcccgaa tgataataaa cactaactat     240

atgtatatca aaaaattaaa aatacactag agattaaacc gaaacaatta aatatattta     300

aaaattgaca ctcagagagt tcgtggatga ttgtgcctcg aacggcgttg tcctaaagaa     360

acgtgatggg gcgcagcgca caatattcgc atatatatgt cgcagcagat ttagcataga     420

aagctaatac tcacaccaaa gatacttcag gagacgtcgt agaacacacc aacactcttt     480

attagctcgc ttttatatga gatccgcaac gttcataggc atttcatcta tctttgtatt     540

atagaactgt tcgatatcct taagcgtgcg tttatcatcg tccgttacga aatttattgc     600

tacaccctta cgaccaaatc gaccacctcg accaatcctg tgaatgtaat tttctctatt      660

agaaggcaaa tcataattaa taactaggga aacttgctgt acatctatac ctctcgctaa     720

cagatcagtg gttattaaaa ccctagatga tcctgttctg aactgacgca taataacgtc     780

acgctctctt tgttccatat caccatgcat agcggaaaca gtgaagtctc tcttgtgcat     840

gttttctgtt aaccaatcga ccttgcgacg ggtattgcaa aagattacag cctgcgtaat 900

cgaaagagta tcatagagat cacataatgt ctctaatttc caatcttctt tttctacata 960

tacataaaat tgtttaatac cttctaaggt aagttcctct tttttgacaa gtatccgaac 1020

aggactcctc ataaaggatt ttgtcacgtc tagcacatcg tttggcatgg tagccgaaag 1080

taaaattacc tgtacgtcag tgttcaacat tttaaacacg tcatgaattt gatctttaaa 1140

accccttgac aacatttcat cagcctcatc caaaacaaac attttaatat gagtcggcct 1200

tagggctctc ctcgttatca tatcgtaaac tctaccaggt gtaccaacaa caacatgtac 1260

gccagtttca agttttctta tgtcttctcg tacattggta ccaccaatac atgcatgaca 1320

ctgcgctgac atgaaatctc ctagggcaat tactactttt tggatctgtt gagccaattc 1380

acgagttggt gccaatataa gagcttggca ctccctcact gacgtgtcaa tttgttgtaa 1440

aatagagatg gagaatgttg cagtcttacc agtacctgac tgagcctgag caataacatc 1500

gtgtcccttt acacaaggaa tgatggctct ctgttggata gcagaaggtt tttcaaaacc 1560

ataggcataa attcctctta aaagttcctc ttttaggttc atgtcgtcga aattatcgac 1620

cacctcttcc cagttggatt caataatgcc atcaggctcc atacctggtg ggccgtcata 1680

gccttggggc tggctgtctt cattagctgg gccattcttg tcccactctt cacgacgatc 1740

tggcatagac atgtctactc cgcg 1764

<210> 237
<211> 628
<212> DNA
<213> Diabrotica virgifera

<220>
<221> misc_feature
<222> (1)..(628)
<223> RNAi target sequence

<400> 237
tcattgtgtc ctaaaggcat gatcataacg ataagtcagc agatgcaaac atccaactta 60

gaacatgcca atttctcttt tttctagttt tgtaattttt tcttcattta tatctgggat 120

taaaaacttt attcctttaa atgtaacgtt aatatatgaa tctcataact agtgttaccc 180

cttagattta accaagagag aaaataacgc tctggtctac atataagtaa acattgccta 240

atttaaattg atgtttcaga acattcgtcg cagaactcag tttctatgtt aaacaacttg 300

gaagaactat tcatggaagg gatactggat ctaataaaca gactgcaagt aaaagttgtg 360

ccctatcagt tgtgcgacaa ctttttcact tgaaagtcat tgaagctttt tcaggtatgt 420

agaaatgtat atctttcttt ttaattcctt tctcttttta tacagagagg ggctaaattg 480

tggagtgaat tgattttatc tagaatggac cactttggag agaaatcgcg aaacgggtcg 540

atttttattt ttgaattaca atttttttgc atatgtttca tactggtgac gtcatccatc 600

tgggcgtgat gacgtagtcg gtgatttt 628

<210> 238
<211> 22
<212> DNA
<213> Artificial Sequence

<220>
<223> primer

<220>
<221> misc_feature
<222> (1)..(22)
<223> forward primer

<400> 238
tcattgtgtc ctaaaggcat ga 22

<210> 239
<211> 22
<212> DNA
<213> Artificial Sequence

<220>
<223> primer

<220>
<221> misc_feature
<222> (1)..(22)
<223> reverse primer

<400> 239
aaaatcaccg actacgtcat ca 22

<210> 240
<211> 697
<212> DNA
<213> Diabrotica virgifera

<220>
<221> misc_feature
<222> (1)..(697)
<223> WCRW transcript ta41281.001_diavv

<400> 240
ttaaaaaaat caccgactac gtcatcacgc ccagatggat gacgtcacca gtatgaaaca 60

tatgcaaaaa aattgtaatt caaaaataaa aatcgacccg tttcgcgatt tctctccaaa 120

gtggtccatt ctagataaaa tcaattcact ccacaattta gcccctctct gtataaaaag 180

agaaaggaat taaaaagaaa gatatacatt tctacatacc tgaaaaagct tcaatgactt 240

tcaagtgaaa aagttgtcgc acaactgata gggcacaact tttacttgca gtctgtttat 300

tagatccagt atcccttcca tgaatagttc ttccaagttg tttaacatag aaactgagtt 360

ctgcgacgaa tgttctgaaa catcaattta aattaggcaa tgtttactta tatgtagacc 420

agagcgttat tttctctctt ggttaaatct aaggggtaac actagttatg agattcatat 480

attaacgtta catttaaagg aataaagttt ttaatcccag atataaatga agaaaaaatt 540

acaaaactag aaaaaagaga aattggcatg ttctaagttg gatgtttgca tctgctgact 600

tatcgttatg atcatgcctt taggacacaa tgaagaaatg taactcacag tttacttctt 660

ttatttaggt ttttggataa ggattttttt gaatccc 697

<210> 241
<211> 610
<212> DNA
<213> Diabrotica virgifera

<220>
<221> misc_feature
<222> (1)..(610)
<223> RNAi target sequence

<220>
<221> misc_feature
<222> (560)..(560)
<223> n can be any nucleotide

<400> 241
ccttcaccaa tttcccctta ctcgggggta aatacaacag cggacagtac acttacaaaa 60

tctaccattt ggccagcaaa gtaccggcgt tcataaggct cctggcaccg aaggggagtt 120

tggagataca cgaagaggct tggaatgctt acccatattg taggaccgtc attacgaatc 180

ctggttatac gaaggagaat tttgttattt gtattgaaag tatgcatatt gctgataatg 240

gtcatcaggt taacgttcat gacttaccca gtgacaaatt gaaacaaaga gaagtggtac 300

acatcgacat cgctaatgat agtgtatcgt cgggagatta caaagatgac gaagatccta 360

cgaagtttaa gtctcaaaag acgggcaggg gaccgctcgt gggtcctgat tggaaggaca 420

aagtcaatcc tgtgatgacg tgttataaat tagtaactgt agaattcaaa tggtttggtt 480

tacagacgag gatagaaagt tttatacaaa aatctgaaag gcggctattt acaaatttcc 540

atagacaagt tttctgttgn atggaccgct ggcacggtct gacgatggaa gacatccgtg 600

caatagaaga 610

<210> 242
<211> 22
<212> DNA
<213> Artificial Sequence

<220>
<223> primer

<220>
<221>  misc_feature
<222>  (1)..(22)
<223>  forward primer

<400>  242
ccttcaccaa tttcccctta ct                                                22


<210>  243
<211>  23
<212>  DNA
<213>  Artificial Sequence

<220>
<223>  primer


<220>
<221>  misc_feature
<222>  (1)..(23)
<223>  reverse primer

<400>  243
tcttctattg cacggatgtc ttc                                               23


<210>  244
<211>  800
<212>  DNA
<213>  Diabrotica virgifera


<220>
<221>  misc_feature
<222>  (1)..(800)
<223>  WCRW transcript ta20868..001_diavv

<220>
<221>  misc_feature
<222>  (715)..(715)
<223>  n can be any nucleotide

<400>  244
aattgcagca aagaaaacat gctgattaaa gaataccgtg tgaccctacc cctcacagtc      60

gaagagtacc aagtggctca actattcagc gtggcagagg cttccaaaga caacactggc     120

ggcggagagg ggatagaagt attgaaaaat gaacccttca ccaatttccc cttactcggg     180

ggtaaataca acagcggaca gtacacttac aaaatctacc atttggccag caaagtaccg     240

gcgttcataa ggctcctggc accgaagggg agtttggaga tacacgaaga ggcttggaat     300

gcttacccat attgtaggac cgtcattacg aatcctggtt atacgaagga gaattttgtt     360

atttgtattg aaagtatgca tattgctgat aatggtcatc aggataacgt tcatgactta     420

cccagtgaca aattgaaaca aagagaagtg gtacacatcg acatcgctaa tgatagtgta     480

tcgtcgggag attacaaaga tgacgaagat cctacgaagt ttaagtctca aaagacgggc     540

agaggaccgc tcgtgggtcc tgattggaaa ggacaaagtc aatcctgtga tgacgtgtta 600

taaattagta actgtagaat tcaaatggtt tggtttacag acgaggatag aaagttttat 660

acaaaaatct gaaaggcggc tatttacaaa tttccataga caagttttct gttgnatgga 720

ccgctggcac ggtctgacga tggaagacat ccgtgcaata gaagataaaa caaaagaaga 780

actagaagct ctccgtcaaa 800

<210> 245
<211> 524
<212> DNA
<213> Diabrotica virgifera

<220>
<221> misc_feature
<222> (1)..(524)
<223> RNAi target sequence

<400> 245
ggttgccgtc gtttagtaga agtgtcattt ttgtctgctc tcgttttcat cttttgtgta 60

taaaattggt gctaaggtag ataaactata ttcgaatagg cgtttttatg tcgcaagaaa 120

gtgttttgta tatttgtgat acgtgatctc gtagagggag tccagaaatt cataatttat 180

agttaagtaa aaatggcggc aaacagaact ggacctgctc agagaccaaa tggcgctacc 240

caaggaaaga tatgtcagtt caaactggtc ctactaggcg aaagtgccgt cggtaagtcg 300

agtttggtac tgaggttcgt caaaggacag ttccacgaat accaggagag taccatagga 360

gcagctttcc ttacacaaac catatgcctc gacgatacaa ctgttaaatt tgaaatttgg 420

gacacagcgg gtcaagaaag gtaccacagt ttagctccta tgtactatag gggcgcacag 480

gcagctatag tcgtctacga cataaccaat caagacacat tcgg 524

<210> 246
<211> 22
<212> DNA
<213> Artificial Sequence

<220>
<223> primer

<220>
<221> misc_feature
<222> (1)..(22)
<223> forward primer

<400> 246
ggttgccgtc gtttagtaga ag 22

<210> 247
<211> 22
<212> DNA

<213>  Artificial Sequence

<220>
<223>  primer


<220>
<221>  misc_feature
<222>  (1)..(22)
<223>  reverse primer

<400>  247
ccgaatgtgt cttgattggt ta                                                22


<210>  248
<211>  3182
<212>  DNA
<213>  Diabrotica virgifera


<220>
<221>  misc_feature
<222>  (1)..(3182)
<223>  WCRW transcript ta01834.005_diavv

<400>  248
ctcaaatatt ggcagccttg ttgacgtcgt ttagtagaag tgtcattttt gtctgctctc      60

gttttcatct tttgtgtata aaattggtgc taaggtagat aaactatatt cgaataggcg     120

tttttatgtc gcaagaaagt gttttgtata tttgtgatac gtgatctcgt agagggagtc     180

cagaaattca taatttatag ttaagtaaaa aatggcggca aacagaactg gacctgctca     240

gagaccaaat ggcgctaccc aaggaaagat atgtcagttc aaactggtcc tactaggcga     300

aagtgccgtc ggtaagtcga gtttggtact gaggttcgtc aaaggacagt tccacgaata     360

ccaggagagt accataggag cagctttcct tacacaaacc atatgcctcg acgatacaac     420

tgttaaattt gaaatttggg acacagcggg tcaagaaagg taccacagtt tagctcctat     480

gtactatagg ggcgcacagg cagctatagt cgtctacgac ataaccaatc aagacacatt     540

cggcagggcg aaaacgtggg tgaaggaact tcaaaggcag gccagtccga cgatcgtgat     600

agctttggcc ggcaacaagc aagatttggc caacaaacgt atggtagaat acgaagaggc     660

gcagacgtat gctgacgaaa acggcttact ttttatggaa acttccgcaa agacggcaat     720

gaacgtcaac gatatatttt tagcaatagc taagaaactg cccaagaatg aacaaaccac     780

aggtcaaggc ggcagtgccc aaggcaggcg gctagcggag ggcgattcgg gcgccaaggc     840

acccggaaat tgttgcaagt gatggtatac gcctgcaggt cgagtgttgt tattaaaccg     900

tcacgagaaa ggactggcaa gtgcagcggc actctagtga tatctatgtg taataaaggt     960

ccttctatta acaaaaaaaa attaataaaa aaatatatta aacttcatat acactgtcac    1020

atattccatt tagatgatga aaaacaaaag agcagaagca ttttggtctc taacggtcat    1080

gttgagttgg aatgtctcgt ggggcattta aatttgtgat aattgtacct atattttgtt 1140

tttttatata tataattaat atatctgaca gtgtaagtta agcgtaaact gtttatatcg 1200

atcgtattag cagcaccaat taaaaaaata aataaaattg aagatctttt ttattgtttt 1260

tgtaatttta actctttggg gaaggttaca aaagaattga taacgttgtg ttgaaaacat 1320

tgcattataa taaagtaatt ccacccactt acgaattatt ttgaaaggag aatcaaaaat 1380

gtgtcaattg tcacctgtca tcttatgatg acactctatg agagtggggg caattgcagc 1440

gcgcgcatac tattcagtct agcttcatta tgtcaattaa atatcgatgt gcattagaaa 1500

tactgcctaa ttgaatattt tcgacatatg attaaaattt catcgattag tttgacagtt 1560

cccaaatgaa aaaacagtta cttccgcata aaaatgtatt gcttcaaaac ttactttcag 1620

tggcatttca gtgggtttta gccatcttat ggagagtggt ctaacacact ttctaggtac 1680

agctgtggtt tttaaaagga aatataatga atcgcttttg ttttcgaaat ttacattggg 1740

aatcccttag tgtccttata tttgccctac agagtttcat ttaaactgaa attgggtaat 1800

ttacatagac ctaaagcttt cacgtctata tagcctaatt agaccacaaa attgacttgc 1860

tttacgtgaa atgtatgaac cattcaaaca tatttatatg acaacaatat aatataatac 1920

aatataatat aaataactga caacatttaa aattagaatt tttacccaca atttttctct 1980

gccatgtgtc atctaataca agaagtatgg caatttagcc ctactcctct tattgtacat 2040

ttcttgtaat tcctcatttt ctttttcaat tacttcgatc ttttaaactt aattcaccaa 2100

gtttatttgc aatattgttc ttgaagtcta tagtttgaat tccttcaata tatttgtaat 2160

gtttttcttc agtgtcaatc cttgcattcc atgaaagaac acgaaaaaga tgcagcactt 2220

tatttgtcca tattcctgct acatagttat ttttcgatta acacattcac tgccagcgta 2280

tagtaaactg gcactttact atacgctaaa caaaaatttg gccttgcaga ttttcctaaa 2340

acacgtagca gctacgccac tgaatggact taggaccccc tatcgtttcg ctggttgcaa 2400

tagtccggta actagagaca aaagtgggtc attttgcaca cttcatataa ttctcgtttt 2460

ctgtacttca cgagtatttt ttttgtcgga tatgtattga atagtatcgg tgacaacaca 2520

ctgccctgcg tgacacccct ttcaaaacct atgtgggaac aatatgtatc tttagagaac 2580

tttttgtacc cttcccctaa aaactaaata ttttttcact tagtgcaatt ttaatattag 2640

tctcacctta tttctagaat acggtcgatc taaatgtata atctcaactt tcagcttata 2700

cttcgaaaac cgagaagtat aaactgtaag aatatttaac gttatatgta ttttattagg 2760

ataatgattg ttgaaagtat gtttcccaac acgtgtaagt tacatttcgt actctttcca 2820

tttttattac aaaaaaaaat gtttatttag ataaactgtt gcggaacata ctttctcgat 2880

gcatgactga aaagaacgtt aacatttata cgtatttgtt tttattttgt gtctgatgtg 2940

acaacccaga tcttagcgaa agcgtagagg acaaaagatg agtttttaag tttcccattg 3000

aactttttcc gtccttaaat caaatacaac aactattcac ttaagttttc taaacatttt 3060

tgctaatatg ggcccctgca ccattcccga cttaacagtc ttcatttgtt tttattaggt 3120

ttgttcgtag tacgatccaa acgatcggca accgttgcca accattagac gcatgcgcgt 3180

tc 3182

<210> 249
<211> 529
<212> DNA
<213> Diabrotica virgifera

<220>
<221> misc_feature
<222> (1)..(529)
<223> RNAi target sequence

<400> 249
atttttgagg ttgtcggcta gaagttcgca aagtaaatct aaattccaac aattaaaccg 60

aaatagttct ttaaaaaaga tgtttcgaaa ccaatacgat agtgatgtga cagtgtggag 120

cccccagggc cgtctgcatc aggtagaata tgccatggaa gcagttaacc taggttcagc 180

cacagtggga ctgaaaagta aaactcatgc tgtacttatc gctttgaaga gggcttcttc 240

agaactgtct gcctaccaga agaaaattat caacatagac gatcacattg gtatcaccat 300

ctcgggactt acagctgatg ctaggatcct tagtagatat atgcgaaacg aatgcttgaa 360

ttataagtac tctttggata cttatatgcc attgaatagg cttatcagta ttttaggtaa 420

caaaatgcag acatgtaccc aaagatacga ccgcagacca tttggtgtag gtcttctagt 480

agctggttat gacgaatctg gttctcatat ctaccaaacg tgcccttct 529

<210> 250
<211> 22
<212> DNA
<213> Artificial Sequence

<220>
<223> primer

<220>
<221> misc_feature
<222> (1)..(22)
<223> forward primer

<400> 250
atttttgagg ttgtcggcta ga 22

<210> 251
<211> 22
<212> DNA
<213> Artificial Sequence

<220>
<223>  primer


<220>
<221>  misc_feature
<222>  (1)..(22)
<223>  reverse primer

<400>  251
agaagggcac gtttggtaga ta                                                22


<210>  252
<211>  1051
<212>  DNA
<213>  Diabrotica virgifera


<220>
<221>  misc_feature
<222>  (1)..(1051)
<223>  WCRW transcript 454run3_isotig10716

<400>  252
cgatgttctg ccgtgactgt ctcggtctgc tttagactaa cagagacagc cacggcaata      60

ttcctttcca ataccccctc tcctgatcaa cgggtggctt taatgcttta tctaatcctt      120

ctattctgtg gtatccatgg cagaagtttc cacagctggg actgcatccc tgggctcgtc      180

ccccgcatca tctctcaacg gatctgcagg aaggtcctca attgcagcag ctgcagctcc      240

tcctcctcta cgttcttcac cctcaataag ggacaagtag ggggaagttt cttcttcttc      300

gaggatttta aaggtctgac ctttgccagt gtatcctatg gagacatttt ttgttgtgag      360

gtcaacctct ggaggtagag tatctctaag agccctcaat ccatgtttga taagttcttc      420

taaagaacta gatggcaatt catccagatg tttttctaag tatgtcctgg cactctggga      480

tcgagctcca atggacatag ctttgcaatc gtagaaatta gcagaagggc acgtttggta      540

gatatgagaa ccagattcgt cataaccagc tactagaaga cctacaccaa atggtctgcg      600

gtcgtatctt tgggtacatg tctgcatttt gttacctaaa atactgataa gcctattcaa      660

tggcatataa gtatccaaag agtacttata attcaagcat tcgtttcgca tatatctact      720

aaggatccta gcatcagctg taagtcccga gatggtgata ccaatgtgat cgtctatgtt      780

gataattttc ttctggtagg cagacagttc tgaagaagcc ctcttcaaag cgataagtac      840

agcatgagtt ttacttttca gtcccactgt ggctgaacct aggttaactg cttccatggc      900

atattctacc tgatgcagac ggccctgggg gctccacact gtcacatcac tatcgtattg      960

gtttcgaaac atctttttta aagaactatt tcggtttaat tgttggaatt tagatttact     1020

ttgcgaactt ctagccgaca acctcaaaaa t                                    1051

<210>  253
<211>  533
<212>  DNA
<213>  Diabrotica virgifera

<220>
<221>  misc_feature
<222>  (1)..(533)
<223>  RNAi target sequence

<400>  253
aggcataacg gtcaaagagg tagaggttat tataataata atcgggattc caggtatggc      60

gatagagact atcgaggcag aggcagaccg tacgggttcc aaaatagagg taatcgaaga     120

caacagcgcg gtagatatgg ttctagagga gggggaggat acagagatta tagggatcgc     180

agatattcaa gaagcagatc ccgcgaccgc gatcacagca ggtcatacag tcagagcaac     240

tcagacgcct ctcctccatc gaaacgagac aggtcttctc ctccatccga gaaagaaaag     300

atcaacaagt attctgacgg agacgcgccc atccagcatg aaggaccgct ttctgagggc     360

gaggacaggg acgattacat cagcaccgat accagcaagt tgatcgatag ggaaatcggt     420

ggtaaatggg ctgataaaga agctgacact agtaaggagg aagccaaaga gagtgggcaa     480

gggaatactg tggaagtaaa ggagtaaaga ggtggacttt cttttaagtc gtt            533

<210>  254
<211>  22
<212>  DNA
<213>  Artificial Sequence

<220>
<223>  primer

<220>
<221>  misc_feature
<222>  (1)..(22)
<223>  forward primer

<400>  254
aggcataacg gtcaaagagg ta                                               22

<210>  255
<211>  24
<212>  DNA
<213>  Artificial Sequence

<220>
<223>  primer

<220>
<221>  misc_feature
<222>  (1)..(24)
<223>  reverse primer

<400>  255
aacgacttaa aagaaagtcc acct                                              24

<210>  256
<211>  3832
<212>  DNA
<213>  Diabrotica virgifera

<220>
<221>  misc_feature
<222>  (1)..(3832)
<223>  WCRW transcript ta01498.002_diavv

<400>  256
ggggaagata aaataatttc ctggtaaata gcttgtcagt gattattaat ttatcgtttt      60

tattaaaagt atctatttta atctagaatt tctcattatt aatacaatat tttctaaata     120

attgtcactt aaattacaat ttttacagaa ataaagtatt gccgttgaaa aatatctaca     180

aaaccacttt ggggggtgtt caaaatggac gtcattcttc atattcgcta attgccggtg     240

aaaaatattg tctagagtta aaatggaaac agtaaattca cagttgttga ccaaagccat     300

aaactttcat ggtcaacagc tgcagaagtt gtgggaagga gaatttggag aaaatgattt     360

gacaagaaaa aatgtcaaag atttgaatta caatgtgtat agtcaacgcc agaagaacct     420

atcttttcaa gatagaggta aacggttgaa actccaacag tttttgataa agaaggctaa     480

ttttatctat agtttggaac ccacgaagca aaagaacaat gagaaagcga ttactgaaga     540

tatgtatgct gttatgcctc cttttgaaac ttacaccagt gtagacaaac aaaaaagagt     600

ggcattcttc atggagaatg tgaaagtagg taatctaatc ctgggcacca ttgtgagcag     660

acaacaatca ggaatgatgt tgaaagtgtt gtgtactact ggaaatggta acacttgttt     720

atatgctgct gatatcaacg tcaaggcatt ctgtccagtt gcaaatgtca ttaatgcagt     780

ggacaaaaag ggggtatcac gcaactacat gatgaatgac tatgtgtgtt gtgaagtgct     840

ggaaatcatg cctgatacag ataagctaat atgtggtatg atgggtgtga ctaggaaatc     900

atcagatcca gaacatcgac cacctctagg gcttattagt actgaagatt ttcctcttat     960

atacaaaaaa gcgttggatc acaagagtga gagttacgac agtgttttag agaagagcac    1020

cgggttcaat aaccccaata acattcaata cctctcagaa cagatggatg ttggtaaaca    1080

tctcagcttc atcacaggct taaagtacag gttcccggaa aacgaatatg ccaccgagtt    1140

gagacaagtc caggccagta aatgggctta ccgcaatgta gcagacggca tagatcattt    1200

taaggcggga aaacacgtgg aagcgttcca atgtctcaac aaagccctca atatagatcc    1260

caagaatgtc gaaggtctcg tagcgagagg tgcgctctat gctaacagtg gaaatttcca    1320

gaaggccatc gaagatttcg aaactgcttt gaagctgaat cctaatcatg gaaacgctcg    1380

aaaatatatg ggagaaactc tcgtcgcatt aggaaggagc tacgaggaag aaaataaatt    1440

agaggaagct agaaaagcgt accagagctg cttgactatc attccttacc acgaagaagc 1500

tcagaactct ttggatttct taaaatccaa agttcaacca aaaaatctta ttgaacccac 1560

tgaacttcta ctgccaagtt tgaacgttcc ctctaagcaa actgacatga atgacgcgct 1620

taaacaactt ctaaagaccg aggaagaaga caaaaaagat aagaagaaga aaaagaagag 1680

caagaagcgc aagagtcgaa aacgctccag ttctagctca agttcgagct cgtcggcaag 1740

tggagaatcc tcctcgagct cgtctagcag ctccagcgac tcaagctcta gctcgggcga 1800

ttccgatttt aaaaagcgca agaagcacag gtcgaggtct caccggcgta aagaacgcga 1860

gaattcgctc agtcctttga gcaagaggat ggctttgatg gatcagagcc atgacactcc 1920

caataccagt tacaatttca acaaaccagc aagttccagc tttgagttta gctttgaaca 1980

tccagagcct tctaaaccac ctgagaacga atacgaaaat aaattgaggg ccttcctgga 2040

tcaaaccaag ggcgactccg actatgagga caaactgaga aaattcctgg aagagacaaa 2100

atggaagaaa ggcaaaggcg aggacaaaaa gaagaagaaa aagaaagaga agaaggccaa 2160

gaaagagagc aagaaaaaga agaagaagga aaagatcaag agaaaacaga gcttcgattt 2220

gagcgaattg gacgaccaca agaagctaag ggacgccatt aagaaggaat tgagcaaaga 2280

aaagaaatcg aagaggcaca gtttggcgtc tgaagacgac gagtacatcc tgcagaagtc 2340

gggttatagt aagaacatta aagaacgaag cgcgagccct cccaagcgca ttatagactc 2400

cccgtctccc acaagggagc agaaggcgcg acaggcagta gccgaagcta cggcctctgc 2460

gggggcccag aagtggaaaa tgcaaattgc cggcagttcg ggcaattcca ggttcaaaaa 2520

gaagcccgaa agagcgcctc ctgcttctga ttggcagtct gatagagaca aacgagagcg 2580

ggagcgagaa cgcgatagag agagggaaag agagagagaa cgtgctaaga gctcttctgc 2640

ctctgacgat tctccaccac cgaaaaaaat gtctttgcct ccacttcctc cagctgagaa 2700

ggccgttagt gtgcgcaagg ccatggcatt gaaagaaccg ccactcaaaa agcctgagtc 2760

tcccaaaaag ttaaaactga ctccagctcc acccttgccg aaaataccgc agggacaagt 2820

tgtgcttgat aaatttggaa acttcaggct gatttctccg cctgaattga aaaagggaga 2880

aggaacgagt tcaggtcgcc gcccaccgga gcccccaggt cgcccgcgca gcgacagccg 2940

ctcgccgaaa cgccgcagtc gcagccgttc cagatcgtcg agcggatcgc gatctcgctc 3000

gcggtctcgc tctcgaagct accgctcgcg atcgcgcagc tactacagca ggtcgcgatc 3060

gcgcagcggc tcctactact cacgcagtcg cagccgctcg cgcagttact ccggagaccg 3120

gcgcaggtac aaccgcagag gcggcttcag aggcaggcat ctcaacgaca ggggcacgta 3180

ctacaaaccg aggttccaaa cgccaaggca taacggtcaa agaggtagag gttattataa 3240

taataatcgg gattccaggt atggcgatag agactatcga ggcagaggca gaccgtacgg 3300

gttccaaaat agaggtaatc gaagacaaca gcgcggtaga tatggttcta gaggaggggg 3360

aggatacaga gattataggg atcgcagata ttcaagaagc agatcccgcg accgcgatca 3420

cagcaggtca tacagtcaga gcaactcaga cgcctctcct ccatcgaaac gagacaggtc 3480

ttctcctcca tccgagaaag aaaagatcaa caagtattct gacggagacg cgcccatcca 3540

gcatgaagga ccgctttctg agggcgagga cagggacgat tacatcagca ccgataccag 3600

caagttgatc gatagggaaa tcggtggtaa atgggctgat aaagaagctg acactagtaa 3660

ggaggaagcc aaagagagtg ggcaagggaa tactgtggaa gtaaaggagt aaagaggtgg 3720

actttctttt aagtcgttaa agaagatttg ttttaagtgt ttgctatgta atataatttt 3780

ttcataatat agaaataaag gttaattgta cacgaaaaaa aaaaaaaaaa aa 3832

<210> 257
<211> 583
<212> DNA
<213> Diabrotica virgifera

<220>
<221> misc_feature
<222> (1)..(583)
<223> RNAi target sequence

<400> 257
tgggccagtt aagttcgtat tccacaacaa aagaatggac ccgtggaaga aaagcacttt 60

tgggaagctt agggcttatt gctggtggta ctggtgcctt gctgtttgct ttggaccaat 120

cagttaaagc atctgatctt gaattgcatc ctccaaagaa tgtttggagt cattctggaa 180

ctttcaattc cttggatcat gctagtgtaa gaagaggtta tgaagtatac aaacaagtat 240

gtgctgcctg ccactcaatg cgcttcattg cttaccgtaa cttaattgga gtttcccaca 300

ctgaagcaga agccaaagct gaagctgaag aacaaatgat tgaagatggt cccgatgaca 360

cgggtaactt tttcaaaaga ccaggaaagt tatctgacta cttccccagt ccatacccca 420

atgaagaagc agctcgtgct gccaacaatg gttcctaccc tccagatttg agctatattg 480

tatctgccag acatggaggc gaagattaca tatttgccct tttgacgggt tactgcgacg 540

cgccagcggg cgtggtcctc agagacggac aatacttcaa ccc 583

<210> 258
<211> 22
<212> DNA
<213> Artificial Sequence

<220>
<223> primer

<220>
<221> misc_feature

<222>  (1)..(22)
<223>  forward primer

<400>  258
tgggccagtt aagttcgtat tc                                                22


<210>  259
<211>  22
<212>  DNA
<213>  Artificial Sequence

<220>
<223>  primer


<220>
<221>  misc_feature
<222>  (1)..(22)
<223>  reverse primer

<400>  259
gggttgaagt attgtccgtc tc                                                22


<210>  260
<211>  1332
<212>  DNA
<213>  Diabrotica virgifera


<220>
<221>  misc_feature
<222>  (1)..(1332)
<223>  WCRW transcript ta01835.001_diavv

<400>  260
ggggggtacg tgctaggtgc agactggtat tacgtaaagt gtcaaaaaat tgtatatttt      60

ttcttagtag cttcctaaaa gtgcttaaat tctatcaaac tagctttcta tcacctaaat     120

acttcatgtt actctaatct aagggtacga ggggtacaaa ataaatttca cataacctat     180

ctgcatagtt tttgagggga ccctttaggc aacatggctg caacaatagg acgaatttgt     240

ggagctggtc ttttaaaacc aacaaactta tcctctgtgg gccagttaag ttcgtattcc     300

acaacaaaag aatggacccg tggaagaaaa gcacttttgg gaagcttagg gcttattgct     360

ggtggtactg gtgccttgct gtttgctttg gaccaatcag ttaaagcatc tgatcttgaa     420

ttgcatcctc caaagaatgt ttggagtcat tctggaactt tcaattcctt ggatcatgct     480

agtgtaagaa gaggttatga agtatacaaa caagtgtgtg ctgcctgcca ctcaatgcgc     540

ttcattgctt accgtaactt aattggagtt tcccacactg aagcagaagc caaagctgaa     600

gctgaagaac aaatgattga agatggtccc gatgacacgg gtaacttttt caaaagacca     660

ggaaagttat ctgactactt ccccagtcca taccccaatg aagaagcagc tcgtgctgcc     720

aacaatggtt cctaccctcc agatttgagc tatattgtat ctgccagaca tggaggcgaa     780

gattacatat ttgccctttt gacgggttac tgcgacgcgc cagcgggcgt ggtcctcaga 840

gacggacaat acttcaaccc ctatttcccc ggtggtgcca tttcgatggc acaagcccta 900

tataatgaaa ccatagaata cagtgacgga acaccagcaa ctgccagtca actcgccaaa 960

gacgtcgctg tgttcctaaa atggacgtca gagccagagc atgatgacag aaagaaaatg 1020

ttaatcaaag tgatcggcat cttttctctt cttattggta tcacttacta tgctaagaaa 1080

ctcaaattct cttcactgaa atcgagaaag attgaattta ggcccaagag taaataaata 1140

agtattgtag atgaatttta aacaaggttt agcgtgacta ccatagttat tattatctta 1200

aggtctaatt ttattgtaca tatttacaga ctttccaact gactggggtt tgcatttgcc 1260

accttggttt atttattttt tccaggcttt attattattg tgtcacgtta tgtttcaata 1320

aaaaaaaata at 1332

<210> 261
<211> 546
<212> DNA
<213> Diabrotica virgifera

<220>
<221> misc_feature
<222> (1)..(546)
<223> RNAi target sequence

<400> 261
gatcccctgc acttggatta tgttattgct gcagctaatt tgaaggctga agtttatggt 60

ataccaacta gtagaaatag agaacaaatc gcagaagtgg ttatgcaagt caatgttcca 120

gaattcatcc ctaaatctgg cgtaaagata gcagttacgg attctcaaat ggctatgaat 180

ggtaataatg tggatttgga tcgtgtttcc caaattaaag aagaattacc ctcagtagca 240

gaactaggtg gactacgtct tactccttta gaatttgaaa aggacgatga caccaatttc 300

cacatggact ttatagttgc tgcttcaaat cttcgtgcag ctaattacaa aataccgcct 360

gctgatagac ataaatcaaa acttattgct ggtaagatta ttcccgccat agcaacaaca 420

acctcagtag ttgctggaat ggtttgcttg gaactctaca aactcaccag aagctataca 480

tccctaactc cattcaaaaa taatttcatt aacctcgctc tgcctttctt cagttgctca 540

gaacca 546

<210> 262
<211> 21
<212> DNA
<213> Artificial Sequence

<220>
<223> primer

<220>
<221>  misc_feature
<222>  (1)..(21)
<223>  forward primer

<400>  262
gatcccctgc acttggatta t                                                21


<210>  263
<211>  22
<212>  DNA
<213>  Artificial Sequence

<220>
<223>  primer


<220>
<221>  misc_feature
<222>  (1)..(22)
<223>  reverse primer

<400>  263
tggttctgag caactgaaga aa                                               22


<210>  264
<211>  4199
<212>  DNA
<213>  Diabrotica virgifera


<220>
<221>  misc_feature
<222>  (1)..(4199)
<223>  WCRW transcript ta05794.001_diavv

<400>  264
tgagaacttg cgtggttcat ttctttcctt tgtcccatca ttctgattca ttttcagctt      60

aatttttcca tgtagtgccg gttaattgaa gtgatatatg atttatttat gtgattacat     120

ctaacagcag actcataaaa ttgtgtcttt ttgtgtccga tgtctagtgg tagtttagca     180

gacaactcca tcgatcctcc agccaaaaag cggaagcttg aatcaaagat tgctaccctc     240

gaaatggcga acaatgaagc ttcaggatcc tccaccagtg atattgatga gggactttac     300

tcgagacaac tgtacgtttt ggggcacgaa gctatgcgcc gcatggcaaa tagtgatgtt     360

ctaatatcag gtttaggtgg tttgggtgta gaagtagcta aaaatgtaat tcttggtggt     420

gttaaatctg ttacccttca cgatgaggct ctttgtacta ttaaggacct gtcatcccaa     480

ttttatttaa cagaggggga cattggtaaa aaccgcgcag aagcctgctg taagcagtta     540

tctgaactca acacttatgt ccccacaaag tcttacacag ggccccttga tgaagctttc     600

atcttaaagt tcagtgctgt tgtcttaacc acaagcaatc gtgaagagca actaagaatc     660

agtgagataa cacacgaaaa caacattgca ttaattatag cagacacgaa aggccttttt     720

gcacaagtat tttgtgattt cggaaaatcc tttactgttg tagatgtaaa cggagaacct 780

ttagtctctg cgatgatagc agacatttcg aatgatgtcg aaagtgtagt cacttgcatt 840

gacgatacaa ggcatggtat ggaggatgga gactatgtta ccttctcgga agttcaggga 900

atgactgaat taaataactg tgaaccaatc aaaatcaaag tattaggacc atacaccttt 960

agtattggcg ataccaccaa gttttctaaa tacaccagag gtggtattgt aagtcaagtt 1020

aaaatgccga aacatctgga attcaaatcg tttaaagatt ctcttgaaaa tcctgagata 1080

ccaattactg attattcaaa aatggatttt cctacgcagc ttcatctagc ttttaccacc 1140

ttacataagt ttgtagaaaa gtctgcaagg ctacctaaac catggaacaa tgaagatgca 1200

acagaatttg ttaatttagc taaatcgtta gctgcaaagg gtgattctga aggagaagtt 1260

aatgtagacc ttttagacac atttgccaag gtatcggctg gagatttaaa tcctataaat 1320

gctactattg gtggtattgt agcccaagaa gtaatgaagg catgttctgg aaaattccat 1380

cccattttcc aatggttcta ctatgatgcc atagaatgcc taccacaaga taaatcagaa 1440

atcactgaag aactagctgc acccactggt agcagatatg atggccaagt tgctgttttt 1500

ggaaaagaat tccaaaagaa gttagcatca ctgaaatatt ttattgttgg tgctggagct 1560

attggttgtg aattgttgaa gaactttgct atgatgggtg taggaacaca aggagaagtt 1620

acagtaactg atatggattt aattgaaaaa tctaatttga acaggcagtt ccttttcaga 1680

ccacataacg tccaacaacc aaaatctgcc acagctgcaa aggtcattaa aaagatgaat 1740

cctgatatca atgttgttgc acatgaaaat cgtgttggac ctgaaactga aagtgtctac 1800

aatgacgatt tctttgaaaa gcttgatgga gttgctaatg cactggataa cgtagatgct 1860

agaatttata tggatagaag atgtgtctac taccgcaagc ctcttctaga gtctggaacc 1920

ctaggaacta agggaaatac tcaagtggtg gcacctttcc tctctgaatc atacagttct 1980

tctcaagatc ctccagagaa aagcattcca atctgtactt taaagaattt ccctaatgct 2040

atcgaacata ctctacaatg ggcaagagat atgtttgaag gattgtttaa acaaaatgcc 2100

gaaaatgctt cgttgtacat aacagaccct gatttcctcg ataggactgt taagctacct 2160

ggtgtacaac ctttagaggt attggaatcc gtaaaggctg ccttaattga tgagagacct 2220

aaagatttaa atgattgcat cgcttgggca cgtaaccatt ggcaagaaca gtattccaac 2280

caaattaggc agttgttgtt caactttcct cctgatcaat taaccagtac ggggcagttg 2340

ttctggtcgg gaccgaagag atgcccagaa gcgcttactt ttgacgtaaa cgatcccctg 2400

cacttggatt atgttattgc tgcagctaat ttgaaggctg aagtttatgg tataccaact 2460

agtagaaata gagaacaaat cgcagaagtg gttatgcaag tcaatgttcc agaattcatc 2520

cctaaatctg gcgtaaagat agcagttacg gattctcaaa tggctatgaa tggtaataat 2580

gtggatttgg atcgtgtttc ccaaattaaa gaagaattac cctcagtagc agaactaggt 2640

ggactacgtc ttactccttt agaatttgaa aaggacgatg acaccaattt ccacatggac 2700

tttatagttg ctgcttcaaa tcttcgtgca gctaattaca aaataccgcc tgctgataga 2760

cataaatcaa aacttattgc tggtaagatt attcccgcca tagcaacaac aacctcagta 2820

gttgctggaa tggtttgctt ggaactctac aaactcacca gaagctatac atccctaact 2880

ccattcaaaa ataatttcat taacctcgct ctgcctttct tcagttgctc agaaccaatc 2940

gcagcccaac aaatggaata ctgtggtaac aaatggacac tctgggaccg cttcgacgtt 3000

cagggagaga tgacacttag tgaatttttg gaatacttca aggaaaaaca cggtcttgaa 3060

attacgatgt tgtcccaggg cgtctgcatg ctgtattcgt tcttcatggc aaaagctaaa 3120

gctcaagaac gtttcggtct ttcgatgtca gaagtcgtca gacgggtgtc aaagaagaaa 3180

atcgaacctc acgtaaaagc attggtattc gaactgtgtt gcaacgattc cactggtgaa 3240

gacgtggaag ttccatatgt taaatacaat ttatgttgag ccgactcagt tcaaaactca 3300

acttaaaaga cttaagagtt ttgttaggtc agtttctgga ttaagagtac gggaaaacta 3360

gccacgtttt actatttaca cattgtgaga ttgctgacca atagttggaa tacatattag 3420

cagcaagagc ttgtgtcttt tattatttat tatttatcag ctgtttttta atatttgcag 3480

acctttaaat attttttata acttttaaat gtcctttttg tattaacgtc atggcaacca 3540

ctgttagtat ttaacccttc cagctccgct gtaatatgtc cagcatcatg acgcacttac 3600

gcgtcaaata cactggccaa gtcatctttg gacgcgcgta tgcgtcaccg tagctgaaag 3660

agttaaatct ctgtaatttc tccaattttc gtttacccaa aaactgatct atactgaatt 3720

tgggtacttg actggaaagt gtgatctaaa ttattcttct caaagaaatt gtttgctgtc 3780

attttgagat aggttaatga aattaataac ggaaaggatt agcatttaaa cacgttgatg 3840

caatatactt ataataactt gtaggagtat gtttattttt atgaaaatag tagttttcag 3900

gtaacagtta ttattttctt aagaaatttt catgtgtgag aataaaataa atatttacaa 3960

aaagttcctt gttttcctta tatacctaca atcgtcgacg actgtagctc aacaacaata 4020

tactaagtcg attctatgct ccaacaacat tttcattttt aaaaatgata aaacaccaaa 4080

agtacacatt tgttctcatc ttactaaggc acgtcgaata atatatcgct tatactattt 4140

cagtgactta aaacgtaaga acagtaaacg taacagtaag aaattttcat gtgtgagaa 4199

<210> 265
<211> 644
<212> DNA
<213> Diabrotica virgifera

<220>
<221> misc_feature
<222> (1)..(644)

<223>  RNAi target sequence

<400>  265
gaatttgatt tggcattttg gtatgtagaa ataaaattat cttgtcaata aataatcaga      60

aatattgaaa gtagaatcgt tctaaataat tagaaattga aagtagaaca gtttgttcgt     120

tttactaggt agtaaagagt taaatagaca acaggacgct gttaaaaggt aagctattgc     180

cgccatactt ttcctgactg aatttattaa ttttttgttt acctaaaaac attattgttt     240

ttaaacacat tttgtttttg ataacatatt taatctaata ctagaaaagc caaaaattac     300

aaaataaggt aaagaatcta aatggtttat tactattagg gcccccaggc attaggcagt     360

ctatgtacat gtaacagcca gagccaattc gtgggcaagt tgtatccttc aagggatgta     420

gaagtgcaag aggtcataat tatttactat ttctctcgta gaaaactcaa catataagca     480

tcgaattcaa attcagcctc ttcttttata acatcacttg aaaggcattt caattatatt     540

ttggaagcat gtttgtaggt tcccagatat acaaaaatgt cagttcaatc gtttgataat     600

cttctgtata aattaattca atcaaggcca ctgatactcc tatg                      644


<210>  266
<211>  22
<212>  DNA
<213>  Artificial Sequence

<220>
<223>  primer


<220>
<221>  misc_feature
<222>  (1)..(22)
<223>  forward primer

<400>  266
gaatttgatt tggcattttg gt                                               22


<210>  267
<211>  22
<212>  DNA
<213>  Artificial Sequence

<220>
<223>  primer


<220>
<221>  misc_feature
<222>  (1)..(22)
<223>  reverse primer

<400>  267
cataggagta tcagtggcct tg                                               22


<210>  268

<211>  712
<212>  DNA
<213>  Diabrotica virgifera


<220>
<221>  misc_feature
<222>  (1)..(712)
<223>  WCRW transcript ta41459.001_diavv

<400>  268
tcagccagat ctatccccaa tgtttttaaa atgatttttg atactgaaaa attaaaatta      60

attcacatag gagtatcagt ggccttgatt gaattaattt atacagaaga ttatcaaacg     120

attgaactga catttttgta tatctgggaa cctacaaaca tgcttccaaa atataattga     180

aatgcctttc aagtgatgtt ataaaagaag aggctgaatt tgaattcgat gcttatatgt     240

tgagttttct acgagagaaa tagtaaataa ttatgacctc ttgcacttct acatcccttg     300

aaggatacaa cttgcccacg aattggctct ggctgttaca tgtacataga ctgcctaatg     360

cctgggggcc ctaatagtaa taaaccattt agattcttta ccttattttg taatttttgg     420

cttttctagt attagattaa atatgttatc aaaaacaaaa tgtgtttaaa aacaataatg     480

tttttaggta aacaaaaaat taataaattc agtcaggaaa agtatggcgg caatagctta     540

ccttttaaca gcgtcctgtt gtctatttaa ctctttacta cctagtaaaa cgaacaaact     600

gttctacttt caatttctaa ttatttagaa cgattctact ttcaatattt ctgattattt     660

attgacaaga taattttatt tctacatacc aaaatgccaa atcaaattcc cc             712


<210>  269
<211>  629
<212>  DNA
<213>  Diabrotica virgifera


<220>
<221>  misc_feature
<222>  (1)..(629)
<223>  RNAi target sequence

<220>
<221>  misc_feature
<222>  (568)..(568)
<223>  n can be any nucleotide

<220>
<221>  misc_feature
<222>  (594)..(594)
<223>  n can be any nucleotide

<400>  269
tgagaaaaac tgtcctttgc atttgcagcg ttaaagatcg gtctcagtaa taaaattatt      60

tgtagagttt gcaatttcct gaaaatagtg acctctgata aagttcacaa cagttcaata     120

attggtagtg gcgcatatca gtgtctttga cgaagccatg attaatatag ctggtggtgc     180

agtgatttct tcgtttgcgg tgtatttgtt attaagatac aggactagaa tgacgtccct 240

tttgacatgg attattggaa atattagatc tcaacaagct gtcagcagta catcacaaga 300

agaagagaaa tctgacaaat ccagcagcaa taaacccaag caacagttta aaggtggcaa 360

agttcgacag aggcctaaaa aacttaacga agatcccaca tttagtacaa gtttatcaac 420

ccccattgaa gatataatag aggaaacaat caatgaagaa gcagtagcag ttaataagaa 480

aaaaggaaaa tcatccaaaa agcaaactga agaattgcca gtgaaaaagg aaaaacccac 540

aaaagatcta gatgatgaca taggttantc atttttcaaa gttaaaactg tttnccaaag 600

gaaatccaga agccaagcct aagaaacaa 629

<210> 270
<211> 22
<212> DNA
<213> Artificial Sequence

<220>
<223> primer

<220>
<221> misc_feature
<222> (1)..(22)
<223> forward primer

<400> 270
tgagaaaaac tgtcctttgc at 22

<210> 271
<211> 22
<212> DNA
<213> Artificial Sequence

<220>
<223> primer

<220>
<221> misc_feature
<222> (1)..(22)
<223> reverse primer

<400> 271
ttgtttctta ggcttggctt ct 22

<210> 272
<211> 1750
<212> DNA
<213> Diabrotica virgifera

<220>
<221> misc_feature
<222> (1)..(1750)

<223>  WCRW transcript ta03339.001_diavv

<220>
<221>  misc_feature
<222>  (633)..(633)
<223>  n can be any nucleotide

<400>  272
aaaagtagcg aaggtacaac gtgtagagaa tagtcagtct gagaaaaact gtcctttgca      60

tttgcagcgt taaagatcgg tctcagtaat aaaattattt gtagagtttg caatttcctg     120

aaaatagtga cctctgataa agttcacaac agttcaataa ttggtagtgg cgcatatcag     180

tgtctttgac gaagccatga ttaatatagc tggtggtgca gtgatttctt cgtttgcggt     240

gtatttgtta ttaagataca ggactagaat gacgtccctt ttgacatgga ttattggaaa     300

tattagatct caacaagctg tcagcagtac atcacaagaa gaagagaaat ctgacaaatc     360

cagcagcaat aaacccaagc aacagtttaa aggtggcaaa gttcgacaga ggcctaaaaa     420

acttaacgaa gatcccacat ttagtacaag tttatcaacc cccattgaag atataataga     480

ggaaacaatc aatgaagaag cagtagcagt taataagaaa aaaggaaaat catccaaaaa     540

gcaaactgaa gaattgccag tgaaaaagga aaaacccaca aaagatctag atgatgacat     600

aggttattca tttttcaaag ttaaaactgt ttnccaaagg aaatccagaa gccaagccta     660

agaaacaagt ttctgttgac ctaaatgaag ataacacagc agatgatata ggtttctcat     720

ttttacctgt caaagaaaaa ggcaaaaaat ataaatctaa gagtaatcca gaaaataaag     780

accaagaact gactttaaac gcagtggtag atcagacact gaggaaagct aaatatgata     840

catcaaagcc gtcaagaata gaacattaca aaaagagaac aactgacaaa gctttttcca     900

aaaaaggctg caacaaaatt tgaaagtcct tcagtatcta caatcgtaga aaagagattg     960

ttgtatgaaa gcaaacttaa aagtgaaact aaggagagca aaaaagttac atcttctatt    1020

tcctttgcta gcatagtcag taatgctact gacgcccctt caggttctga tattgaacaa    1080

aattcccagt tactaaatgg acctaagcct gatattcaac agcatatctt tgagtcttta    1140

gagtcagtaa aatctagtgc tgagtccgta gaagtaattg atgatagcaa ttctgaatat    1200

caaaacagtc aatccaccaa ttttgagaca atcaaagaaa aattaaatac tttactcgat    1260

gaggattctg attttaatca aactcaaata gagcaatcaa atgtatatga aatagatcaa    1320

tcttcaaagc attcttctgt atatattgta ggaagtaacg aggtattgga agaatcatcc    1380

tccacacaag attcttatat tggtcataca ggttttgaac aagaacctga acaaacattc    1440

agtcagaaaa ctctagaagc ttctatagac aacagattta taacagatgc cagtatatta    1500

gaaaaaagta catccactga agcttttctt agttttgaaa gacatatcga atcacaagat    1560

ctaacacaca gtcttgaatc cagtaattat tatcaaaata attattcaca gttaaatttt    1620

gacaatcaac ttcaaaactc ttcagaaagc catatttctg attacagcta ttcccaaaat    1680

caacagtccc taagctcaag cgacttttct agagactaca attctacaga ccattccttt 1740

acagaactag 1750

<210> 273
<211> 700
<212> DNA
<213> Diabrotica virgifera

<220>
<221> misc_feature
<222> (1)..(700)
<223> RNAi target sequence

<220>
<221> misc_feature
<222> (608)..(608)
<223> n can be any nucleotide

<400> 273
gagagcactt ttcgcagtta ccacagcgga ccggcttttc aacaaacaga aacaatcaga 60

ggaccggtgt aaaagtgatg gatccttcat ttgagaatgc tgggcaagtg gatggccttg 120

aaatatggag aattgaagac ttcaaacctg tgccttatcc taaaaatcaa tacggcaaat 180

tctacacagg agactcatac attatactaa ataccaaaac tacaagaagt ggtgccaaag 240

tacatgatct tcatttttgg ttaggtgctg aaactagcca ggatgaacaa ggatcagctg 300

ccatatatac agttcagttg gacgagcatt tgaacggaga accagtccaa catcgtgaaa 360

cccaaaacca cgaatctcaa ctgttcttat cttacttcaa aagcggtggt attcgatacc 420

ttcctggtgg tgttagctct ggcttccacc atgtcgatcc taatgctttc gagaagaggc 480

tgttccaagt taaaggctct agaaatatta gagttaagca agttacccca tcaatcgcct 540

ctatgaacac tggtgactgc tttattttgg acgttggaag agatatttat gtctatgttg 600

gtactaangg ctagaagagt tgaacaactc aagggctatt aatgctgcca accaaattcg 660

cgaccaagat catgccggaa aggccagagt aaccattgtc 700

<210> 274
<211> 22
<212> DNA
<213> Artificial Sequence

<220>
<223> primer

<220>
<221> misc_feature
<222> (1)..(22)
<223> forward primer

<400> 274

gagagcactt ttcgcagtta cc                                                22

<210>  275
<211>  22
<212>  DNA
<213>  Artificial Sequence

<220>
<223>  primer

<220>
<221>  misc_feature
<222>  (1)..(22)
<223>  reverse primer

<400>  275
gacaatggtt actctggcct tt                                                22

<210>  276
<211>  2169
<212>  DNA
<213>  Diabrotica virgifera

<220>
<221>  misc_feature
<222>  (1)..(2169)
<223>  WCRW transcript ta00090.001_diavv

<400>  276
gggggactag ctgtatgagc tttcgtaacg ttcggttgtg tgacagttca gtgtggaagt      60

gataattcaa aaaaaaacgc aggcgacaaa aacaaaatag gaactacata atagagagag     120

accgaggcga ataagatgga ccacccttaa accacccaaa ttagaacatt aaaaggtgtt     180

tttcaaagct ctcaaattca atagctagcg catgtccaaa atgtggggaa acggagcgtt     240

tagtcgcggc actttggtgt gtctcgcgct tttagtttat ctgaaagata gtagctgtgt     300

gactacaaac agacgagagc acttttcgca gttaccacag cggaccggct tttcaacaaa     360

cagaaacaat cagaggaccg gtgtaaaagt gatggatcct tcatttgaga atgctgggca     420

agtggatggc cttgaaatat ggagaattga agacttcaaa cctgtgcctt atcctaaaaa     480

tcaatacggc aaattctaca caggagactc atacattata ctaaatacca aaactacaag     540

aagtggtgcc aaagtacatg atcttcattt ttggttaggt gctgaaacta gccaggatga     600

acaaggatca gctgccatat atacagttca gttggacgag catttgaacg gagaaccagt     660

ccaacatcgt gaaacccaaa accacgaatc tcaactgttc ttatcttact tcaaaagcgg     720

tggtattcga taccttcctg gtggtgttag ctctggcttc caccatgtcg atcctaatgc     780

tttcgagaag aggctgttcc aagttaaagg ctctagaaat attagagtta agcaagttac     840

cccatcaatc gcctctatga acactggtga ctgctttatt ttggacgttg gaagagatat     900

ttatgtctat gttggtacta aggctagaag agttgaacaa ctcaaggcta ttaatgctgc 960

caaccaaatt cgcgaccaag atcatgccgg aaaggccaaa gtaaccattg tcgataccta 1020

cagtccagaa agtgactata ctacattctt tgaagccttg ggtgaaggtt ccagagactc 1080

agttcctgac tataccacca gtgacgacga tgaatctttc gaacgcacag aggaaaatca 1140

tgcttcactt tacaaggtat cagatgctac tggctctgtt aaagtaacca aagttggaca 1200

aaaacctttg gaagctagtt cattggaccc cagcgactgc tttattttgg ataccactga 1260

tgccttattg tatgtatgga tcggcagcaa atgtgatgaa agagaaaaga aggaagcgat 1320

gaataaagct gatgggttcc ttaatgcgca caatcaccct aaatggacac atgttcaaag 1380

aattgttcaa ggagctgagc ctactgcgtt tacacaatat ttcagaaatt ggcaggcctt 1440

tggagaaacc cacccaagat tactacgttc tgtcagcacc tcagcccacc tttaccattg 1500

ccaaatccgt tcaagatctc gcagacttaa agtcgaagag attagggact atgagcagga 1560

agacctctgg gaggatgaca tcatggtctt ggacgctggt gatgttgtgt ttgtttggaa 1620

cggcaaagga gcttcagatg aagaaaaatc tagaggacct aaatacgtac ataaaatgtt 1680

gaatcgtcaa ggtcgtgaag acgtaccagt aaaaataatc gagcaaggtg atgaaccaga 1740

agaattcaca atacacttcc cagcttggtc agcagatcac tgggacaatt taccagatgt 1800

aagaagctac atcaaaaatg aggaagactg aattctgtta tcatgattga ccaacgcgtc 1860

acttgtttta aataaggctt ttaagttgaa aaatgtgcgc aaaaatatta tagctttaac 1920

atttttaaat atgcatttat atagctgttt tagtttttta tatgccaatg ctctcggtta 1980

aatagaaata gtttcaaaat tattgttaca acactagaaa gaacttatga attgtgccta 2040

aaaaagattt ttaaatacta tatttaaatg atactttata tatgtactgt tgtgtaccta 2100

tcagtgttat tttttaaaat gtatttgtta agcttgtgtg gttacaaaaa ctataataaa 2160

aatattaat 2169

<210> 277
<211> 608
<212> DNA
<213> Diabrotica virgifera

<220>
<221> misc_feature
<222> (1)..(608)
<223> RNAi target sequence

<220>
<221> misc_feature
<222> (561)..(561)
<223> n can be any nucleotide

<220>
<221> misc_feature

<222>   (562)..(562)
<223>   n can be any nucleotide

<400>   277
caacctggaa ctttaactgc tgcagctgca acaccaggta aaggtaagaa gaaggaagaa      60

aagaaacaag agaagaagcc acaaacacct aaagaaaaga aggaaaagga acagccagaa     120

gaattagatg ctgcagaagc cgctcttgct gaagaaccca agagtaagga ccctttttct      180

gaactaccta aaggaacctt tgttatggat gactttaaac gtgtctattc aaatgaagaa     240

gagagcaaat caattgctta tttctgggaa aaattcgacc ctgaaaacta ttcaatctgg     300

tatggtgaat acaaataccc tgaagagctc tccaaagtgt tcatgagctg taacctaatt     360

actggcatgc aacagagact agataagatg agaaagcagg cctttgcatc tgtatgcctt     420

tttggagaag acaacaacag cacaatctcc ggtatctggg tatggagagg acaagaatta     480

gcttttacct taagtcccga ctggcaaata gattatgaat cctatgaatg gaagaagtta     540

gatgccaaat cagaagaaac nngaaattag tagagcaata cttctcatgg acaggtactg     600

acaagcaa                                                              608


<210>   278
<211>   22
<212>   DNA
<213>   Artificial Sequence

<220>
<223>   primer


<220>
<221>   misc_feature
<222>   (1)..(22)
<223>   forward primer

<400>   278
caacctggaa ctttaactgc tg                                               22


<210>   279
<211>   22
<212>   DNA
<213>   Artificial Sequence

<220>
<223>   primer


<220>
<221>   misc_feature
<222>   (1)..(22)
<223>   reverse primer

<400>   279
ttgcttgtca gtacctgtcc at                                               22

<210>  280
<211>  1508
<212>  DNA
<213>  Diabrotica virgifera

<220>
<221>  misc_feature
<222>  (1)..(1508)
<223>  WCRW transcript ta20625.001_diavv

<400>  280
ggggctcttt agctagtttt gagtgtagtg agaatggctg ccggaactct ttacacttat      60

cccgaaaatt tccgggccgc aaaaactctg attgccgcac aatatgccaa ggcaaacgtc     120

aaagtagcac ccaattttgt ttttggagaa actaataaaa ctaaagagtt caccaagaaa     180

tttccaagtg gaaaggttcc tgcttttgaa tcaaacgatg gcaagtacct tcaagacagc     240

aacgccatcg cttactatgt ttccaatgaa caactaagag gaaaaaacga atatgataga     300

gcacaaattt tgcaatggat tggctttgct gaaggtgaaa ttgttccagc agcatgtgcc     360

tgggtattcc ctatcttggg aattgttaaa aataacagtg gtaatgatga gactttccaa     420

agagcaaagg aagaccttaa atcctctttg tctattctca acagccactt acttgcccac     480

acttacttgg taggagagag aatcactcta gctgacattg tagttgcctg caacctattg     540

aatgcctaca aatatgtact tgatcccaac tttagaaaat cttttggtaa cgtcaataga     600

tggttcaata cacttattaa ccagccacaa ttcatagctg tacttggaga agttaaactt     660

tgcagtgcag ttgctcaacc tggaacttta actgctgcag ctgcaacacc aggtaaaggt     720

aagaagaagg aagaaaagaa acaagagaag aagccacaaa cacctaaaga aaagaaggaa     780

aaggaacagc cagaagaatt agatgctgca gaagccgctc ttgctgaaga acccaagagt     840

aaggaccctt tttctgaact acctaaagga acctttgtta tggatgactt taaacgtgtc     900

tattcaaatg aagaagagag caaatcaatt gcttatttct gggaaaaatt cgaccctgaa     960

aactattcaa tctggtatgg tgaatacaaa taccctgaag agctctccaa agtgttcatg    1020

agctgtaacc taattactgg catgcaacag agactagata agatgagaaa gcaggccttt    1080

gcatctgtat gcctttttgg agaagacaac aacagcacaa tctccggtat ctgggtatgg    1140

agaggacaag aattagcttt taccttaagt cccgactggc aaatagatta tgaatcctat    1200

gaatggaaga agttagatgc caaatcagaa gaaacaaaga aattagtaga gcaatacttc    1260

tcatggacag gtactgacaa acaaggtaga aaattcaatc aaggaaaaat ctttaaatga    1320

tctacatcta tttaataaat cttacaaaat gctgtactga tcatgtgaag caagagaaat    1380

aaatcccaat gttttgttaa taatattttt ttattaatat gtaattgttt tgatcgaagc    1440

ttattctttg ggctttactc cctgagttta taagacactt aaataaattt aataacaatt    1500

taaaaaaa                                                              1508

<210>  281
<211>  724
<212>  DNA
<213>  Diabrotica virgifera


<220>
<221>  misc_feature
<222>  (1)..(724)
<223>  RNAi target sequence

<400>  281
gtcagtgtca tcatttgacg tgtcaagttt ggggttaaat tttgttaatt tcaagaatta      60

aaatatttaa cataaaaatg gtttctatat taaattcaat agatactggt catgaagata     120

tgatacatga tgcagaagta gattattacg gtttaaggtt agccacttgt tcctcagata     180

acagcatcaa agtttatgat ataaaaagtg gagcttccac actactggat gatctaaaag     240

gccactatgg acctgtttgg caaatagctt ggagtcatcc taagtttggc aacttattag     300

cttcgtgttc ttatgatagg aaagtaataa tttggaaaga acagcaaaga aaatggataa     360

aatattatga gtatgcaaat catgattcca gtgttaactc tgttcagttt gcacctgctg     420

agctaggttt aattctggct tgtggaagta gcgatggttc aatttctctt ttaacttata     480

atgttggaag taatatttgg gatgccaaga agattcaaaa tgcccatgca ataggatgta     540

atgctgtaag ttgggctcca gccataaccc ctgtttttgg agatgagact acacctgtga     600

aacgtcttgt ttctggagga tgtgacaatt tagtaaaaat atggagggaa gatggtgaca     660

ggtggattga agagtccaaa cttgaagtac gctctgattg ggtaagagat gtagcttggg     720

ctcc                                                                  724


<210>  282
<211>  22
<212>  DNA
<213>  Artificial Sequence

<220>
<223>  primer


<220>
<221>  misc_feature
<222>  (1)..(22)
<223>  forward primer

<400>  282
gtcagtgtca tcatttgacg tg                                               22


<210>  283
<211>  21
<212>  DNA
<213>  Artificial Sequence

<220>
<223>  primer


<220>
<221>  misc_feature
<222>  (1)..(21)
<223>  reverse primer

<400>  283
gagcccaagc tacatctctt a                                                21


<210>  284
<211>  1151
<212>  DNA
<213>  Diabrotica virgifera


<220>
<221>  misc_feature
<222>  (1)..(1151)
<223>  WCRW transcript ta06970.001_diavv

<400>  284
gggggacagt tgtcggcggc ccgcagccaa aatccatgtt acgccgagac gttcgtattt      60

gacgtgtcaa gtttggggtt aaattttgtt aatttcaaga attaaaatat ttaacataaa     120

aatggtttct atattaaatt caatagatac tggtcatgaa gatatgatac atgatgcaga     180

agtagattat tacggtttaa ggttagccac ttgttcctca gataacagca tcaaagttta     240

tgatataaaa agtggagctt ccacactact ggatgatcta aaaggccact atggacctgt     300

ttggcaaata gcttggagtc atcctaagtt tggcaactta ttagcttcgt gttcttatga     360

taggaaagta ataatttgga aagaacagca aagaaaatgg ataaaatatt atgagtatgc     420

aaatcatgat tccagtgtta actctgttca gtttgcacct gctgagctag gtttaattct     480

ggcttgtgga agtagcgatg gttcaatttc tcttttaact tataatgttg gaagtaatat     540

ttgggatgcc aagaagattc aaaatgccca tgcaatagga tgtaatgctg taagttgggc     600

tccagccata acccctgttt ttggagatga gactacacct gtgaaacgtc ttgtttctgg     660

aggatgtgac aatttagtaa aaatatggag ggaagatggt gacaggtgga ttgaagagtc     720

caaacttgaa gtacactctg attgggtaag agatgtagct tgggctccat cagtaggact     780

ccacagatat accattgcca gctgttctca agacaggaga gttgttatat ggactagcaa     840

tgattgcatc aattggaatt caacagtctt acatacattt gatgatgtag tttggaatct     900

tagttggtct ttgaatggaa atattttagc tgtatctggt ggagataata aaattacctt     960

gtgggatcaa aactcagaag gtagttggca atgcattagt gatattgcca agggccaagg    1020

tcaaatcaat aatcaatgaa ttcgctattt aaatagattt cgaaagtgtt atattttgca    1080

ttttcttctg tattaagttt taatgaatgt atattttact actctaattt taaaattaaa    1140

ttaataaaaa t                                                          1151

<210>  285
<211>  501
<212>  DNA
<213>  Diabrotica virgifera

<220>
<221>  misc_feature
<222>  (1)..(501)
<223>  RNAi target sequence

<400>  285
tgtccaataa tgtgtgttgt gaagtgtaac aaatatgaaa aagactacac ttagaatatg        60

agttaaaatt ggttgaagat tcctttgtat ttagtttgta gttacacaac agtacagaca       120

tggcaaggga gcaggtaacc agaaaatggg aaatatttca aggtagaaat aggttttact       180

gcaatggaag gctgatgaca gcaccgcatt ctggggtatt tttgttgaca ctttttttta        240

attactgtaa cgagtgtgtt attttttgta tttgactgta aatatttagc agaaaatgtg        300

acaatagcca ttccgatcat cggagctttg ctgttcttat ttacagtgtc ttcccttttg        360

cgtacaagct tgtctgaccc aggtattata cctagggcgt cgtcagaaga ggcagcctac       420

gttgaaaagc aaatagaagt aacaaactct gccaacagtc ctacatatag accgccaccc       480

aggacgaaag aagttttggt t                                                 501

<210>  286
<211>  23
<212>  DNA
<213>  Artificial Sequence

<220>
<223>  primer

<220>
<221>  misc_feature
<222>  (1)..(23)
<223>  forward primer

<400>  286
tgtccaataa tgtgtgttgt gaa                                                23

<210>  287
<211>  22
<212>  DNA
<213>  Artificial Sequence

<220>
<223>  primer

<220>

<221>  misc_feature
<222>  (1)..(22)
<223>  reverse primer

<400>  287
aaccaaaact tctttcgtcc tg                                                22


<210>  288
<211>  2197
<212>  DNA
<213>  Diabrotica virgifera


<220>
<221>  misc_feature
<222>  (1)..(2197)
<223>  WCRW transcript ta06058.001_diavv

<400>  288
gggggtcaaa gtcaactgtc aaaattgttg agtcgtgaat gtcggataat cttcactttt      60

taggagtgtt tcttgtttat ttgtaaaaaa cgataatatg cgtatctcta aacaagatga     120

tatattaaat tctgtccaat aatgtgtgtt gtgaagtgta acaaatatga aaaagactac     180

acttagaata tgagttaaaa ttggttgaag attcctttgt atttagtttg tagttacaca     240

acagtacaga catggcaagg gagcaggtaa ccagaaaatg ggaaatattt caaggtagaa     300

ataggtttta ctgcaatgga aggctaatga cagcaccgca ttctggggta tttttgttga     360

cacttttttt aattactgta acgagtgtgt tattttttgt atttgactgt aaatatttag     420

cagaaaatgt gacaatagcc attccgatca tcggagcttt gctgttctta tttacagtgt     480

cttccctttt gcgtacaagc ttgtctgacc caggtattat acctagggcg tcgtcagaag     540

aggcagccta cgttgaaaag caaatagaag taacaaactc tgccaacagt cctacatata     600

gaccgccacc caggacgaaa gaagttttgg ttaaaggtca aaccattaaa cttaagtatt     660

gcttcacttg taaaatattt aggccgccta gagcttcgca ttgtagttta tgtgataatt     720

gtgtagatag gtttgatcat cactgtccat gggtggggaa ctgtgttggt agaagaaatt     780

ataggttttt ttacatgttt atagtatcac tggcgttttt agcagtattt atatttgcct     840

gtgcagttgc gcatcttatt ttaatgacga aggaagatcg agaattccta gatgccgtga     900

aggaatcccc tcccagcgtg ctagttgcag tcatttgttt ttttagtgtt tggagtattt     960

tgggcttggc cggattccat acgtacctta cggctagcaa ccaaaccact aatgaagata    1020

taaaaggatc ttttacaagc aaaagaggcc aagaaaacgt caacccttat tcggagggca    1080

acgtctgctt aaattgtttt cacattctct gtggaccggt tactccttcg ttaatcgaca    1140

gaagaggtgt tgtcacagaa agctaccaat acgaaaacag tagacctgtt accaatgcac    1200

caactttgcc tttgaagaaa tatggattgc ccttgcagaa tggtccccaa caagtgacac    1260

ctgacgcagc ggaaatgcca ggcatctacc gccaaaccac cgagaacacg gaaagcaaca    1320

cgggtagcgt aacccactta gtgtctagcg aacagcccct agccgtcgtt cccgccgcag 1380

tgcatctaca gagaacccac ggggacttgc aacagtacca gtcgcagacc agtctgaacc 1440

actccccggt ctacaacaac atcaccccaa cgcacgccgc tcccgcgcaa ctcagcaaat 1500

cacaaagctg tatcgacaat ataaattcca cgtcttcatc gaaagaaaac ggcgcgaaag 1560

acattacatt gaacatggaa gacctccatt tggatcctgt gatattgagg gagaaccacg 1620

tactggtcaa catcaacgac aaaggacggc tcgccgattt gaggctagac aacgatatga 1680

cagtgcaaga caaggagaac agtttgttga gtgttagtag gttgcgactg ttgcaagaca 1740

cgacgatgat cgagagtgct ttagacttgg attcgttaga tgggtctagt ttaggtacga 1800

atagtcaggc gggattgatg aaagtagacg tgtagttcat tcgagaactt ttagaagaga 1860

atgtttgtca tttgtttggc aacagccaac atatagtagt attgaataca gaagaacgta 1920

aaataactca tgatagtgca ataaaatggg atttctttgt tttcgactta ttttattggt 1980

caaaagtttt gtttatattt ttataacaga ataaatatga ttaaataatt gatttgcact 2040

ttaaaatgta taaaaatcat cagcagattt atctgtagca atgcaagaca cttttatagt 2100

aatctccagc tagggaaaac ttacgcagtg catcttagta tttcagaatt aaattttaaa 2160

tgaaaaagac ctttataaaa gatatattta ttttaaa 2197

<210> 289
<211> 628
<212> DNA
<213> Diabrotica virgifera

<220>
<221> misc_feature
<222> (1)..(628)
<223> RNAi target sequence

<400> 289
aatttgtgcc tcggaattgt attttttacg ttttttagtg gaatttgggc ttcagccgaa 60

aaaataccac tgggagcaat tttcgaacag ggcacagatg acgtccaaac agcatttaaa 120

tttgcgatgt tgcagcacaa tcaaaatatt tccagtagaa aatttgaatt tcaagcatat 180

gttgacgtta ttaatactgc ggatgcattt aaattgtcta gattaatttg cactcaattt 240

tcaagaggtg tatattcgat gttgggagca gtttctccag attcatttga tacgctacat 300

tcctatagta atacttttca aatgcctttt gtaacgcctt ggtttccaga aaaagtatta 360

gccccgtcat cgggatttat ggactatgct atttcaatgc gacctgaata ccacaaagcc 420

atcatagata cggttaagta ttacggatgg aggcagatta tatatttata tgattctaat 480

gatggtctcc taaggttaca acagatatac caaggattaa ctcctggcag tgagattttt 540

caagtcacta ctgtacggag aataaacaat gttagcgaag cattatcatt tcttagagga 600

atagaagaac atactcgatg gaccaaca                                          628

<210>  290
<211>  22
<212>  DNA
<213>  Artificial Sequence

<220>
<223>  primer

<220>
<221>  misc_feature
<222>  (1)..(22)
<223>  forward primer

<400>  290
aatttgtgcc tcggaattgt at                                                22

<210>  291
<211>  22
<212>  DNA
<213>  Artificial Sequence

<220>
<223>  primer

<220>
<221>  misc_feature
<222>  (1)..(22)
<223>  reverse primer

<400>  291
tgttggtcca tcgagtatgt tc                                                22

<210>  292
<211>  690
<212>  DNA
<213>  Diabrotica virgifera

<220>
<221>  misc_feature
<222>  (1)..(690)
<223>  WCRW transcript ta41486.001_diavv

<400>  292
gatcacgtca tgggcaccac aaatttgtgc ctcggaattg tattttttac gttttttagt      60

ggaatttggg cttcagccga aaaaatacca ctgggagcaa ttttcgaaca gggcacagat      120

gacgtccaaa cagcatttaa atttgcgatg ttgcagcaca atcaaaatat ttccagtaga      180

aaatttgaat ttcaagcata tgttgacgtt attaatactg cggatgcatt taaattgtct      240

agattaattt gcactcaatt ttcaagaggt gtatattcga tgttgggagc agtttctcca      300

gattcatttg atacgctaca ttcctatagt aatacttttc aaatgccttt tgtaacgcct      360

tggtttccag aaaaagtatt agccccgtca tcgggattta tggactatgc tatttcaatg 420

cgacctgaat accacaaagc catcatagat acggttaagt attacggatg gaggcagatt 480

atatatttat atgattctaa tgatggtctc ctaaggttac aacagatata ccaaggatta 540

actcctggca gtgagatttt tcaagtcact actgtacgga gaataaacaa tgttagcgaa 600

gcattatcat ttcttagagg aatagaagaa catactcgat ggaccaacaa atatgtggtt 660

ctagattgct ctactgagac tgctaaagaa 690

<210> 293
<211> 710
<212> DNA
<213> Diabrotica virgifera

<220>
<221> misc_feature
<222> (1)..(710)
<223> RNAi target sequence

<400> 293
gctctgttat gtgcgtcaag aatttagaaa ttttatatta tccgtaatgg tggtcgaccg 60

tgtgcattct aaaactatta aataatcatg tcgctagaga ctcgatctaa ttcagctctg 120

tttaaaagag cagaccaact aaaacgttgg gaagaatcag atacaaatcg agaaccagtg 180

tcaccgaaac aaaaaactcg gaaaattaaa ttttcttcgg gatgtgtgtt tctcgcggct 240

tgtgctgctg gtgataaaaa agaagtgttg gaattgttac ataaaggagc tgacatcaat 300

actgcaaatg tggacggatt aacagcccta catcaggcat gtatcgacga caatctcgac 360

atggtggagt tcctcgtgga gaacggggcg gacgtcaaca ggggcgacaa cgaaggttgg 420

acccctttgc acgccactgc cagctgcggg tttctctcga tcgcgaagta cctcatcgaa 480

aagggggccc acgtagcggc agttaataac gatggacaat tggcacttga tattgccgag 540

agccaaaaaa tggaaggact actcagggaa gaaattgagg atagaggcat cgactgcgaa 600

gcagctagga acgaagaaga attggtgatg ttgagggatg ccaaagattg gttagcgaca 660

aaaagttcct tagttaacgt accacaccct aaaaacggag ctacaccttt 710

<210> 294
<211> 22
<212> DNA
<213> Artificial Sequence

<220>
<223> primer

<220>
<221> misc_feature
<222> (1)..(22)

<223>  forward primer

<400>  294
gctctgttat gtgcgtcaag aa                                                22


<210>  295
<211>  23
<212>  DNA
<213>  Artificial Sequence

<220>
<223>  primer


<220>
<221>  misc_feature
<222>  (1)..(23)
<223>  reverse primer

<400>  295
aaaggtgtag ctccgttttt agg                                               23


<210>  296
<211>  3435
<212>  DNA
<213>  Diabrotica virgifera


<220>
<221>  misc_feature
<222>  (1)..(3435)
<223>  WCRW transcript ta00860.008_diavv

<400>  296
ttttatatta tccgtaatgg tggccgaccg tgtgcattct aaaactatta aataatcatg      60

tcgctagaga ctcgatctaa ttcagctctg tttaaaagag cagaccaact aaaacgttgg     120

gaagaatcag atacaaatcg agaaccagtg tcaccgaaac aaaaaactcg gaaaattaaa     180

ttttcttcgg gatgtgtgtt tctcgcggct tgtgctgctg gtgataaaaa agaagtgttg     240

gaattgttac ataaaggagc tgacatcaat actgcaaatg tggacggatt aacagcccta     300

catcaggcat gtatcgacga caatctcgac atggtggagt tcctcgtgga gaacggggcg     360

gacgtcaaca ggggcgacaa cgaaggttgg acccctttgc acgccactgc cagctgcggg     420

tttctctcga tcgcgaagta cctcatcgaa aagggggccc acgtagcggc agttaataac     480

gatggacaat tggcacttga tattgccgag agccaaaaaa tggaaggact actcagggaa     540

gaaattgagg atagaggcat cgactgcgaa gcagctagga acgaagaaga attggtgatg     600

ttgagggatg ccaaagattg gttagcgaca aaaagttcct tagttaacgt accacaccct     660

aaaaacggag ctacaccttt acatgtagcc tgtgccaaag gttatgccga cgttataaaa     720

atattattac aatgtggagc tgacattgat gcgcaggatg ttgacggctg gacccctcta     780

catgcagctg cgcactgggg ccaaaaggaa acgtgccaaa tattggctga aagtttggct     840

gacatggaag ctaaaaactt cgtaggacaa acagcatttg acttgtgtga tactgacatg 900

ttatcgacat tggaagatct taaggaaaaa aaaaaaaaaa aaaaaacgaa ttaatagaag 960

aatggccaag aagagggccg atattaacga taggaatttg gaatctgaga cgcctaccaa 1020

agtgaaaaag gttgagctcg aagggacgaa agtgattaga aatatagaaa agaaaaataa 1080

cctgaatgcc aacaatgaaa tcacaacgaa cactactgta ccccagaaga tcgttgaaga 1140

caaagaggat gttactttaa ggaagaaacc gtatacaaaa cagaattcag atttgagtcc 1200

aacaaaactg aatagtacga ataatttgac taattccacg gaatctataa agattgaacc 1260

agaagttact ctcaggaaac ctcctattgt acaaaatgaa aaaccgacta aaaaagaaga 1320

caacgataca ccggtcagta ccgctgcaac cacaaatacc acaactaaca tatcacaaga 1380

aaagacttca ccagtgacta gtccacagcc tactactaac aaccaacaac aattgcgaag 1440

cggtctaaag ttgcaaatgc cacctccgcc tacaacaacg gttaccgccc ctacaccggt 1500

aacgaattct tccacgccct ccactcctac aacgccggga ggaaccaggc ttcagatgct 1560

caaaaatttc tttaagtcgt ttgttcctcc agtaagggat gaagaaagtg aaactcaaag 1620

aaaagcgcat gccaagaggg taagggagac tcgacgatcc actcaggggg taactttaga 1680

agaaatcaaa tccgccgaac agttagtaaa acagaagaat caacctggtg gcggcaatga 1740

ggaaaagcct ggagcaaatg gagactcccc caacggaatc gtagttaccg ccaccatcac 1800

aaccggtact cccacagtta taacaactcg tgaaagtgag gaaccacccg caatacacga 1860

aaggcgacca tcgtggaggt tgagggtaga caatggtacc agcaagttct tgttagaaga 1920

cgcttcaaaa ccttcggacc cgccaaacac gccgtcgatt attcgtagga ccatcgcatc 1980

catccaaaga ccttcttcag cgcctctaga gactgcagat actacaatta cactgccttt 2040

aagaaaaccc aaatccgtag aagaaaaaga tcaagataag gagaacgata ttagaaatgc 2100

tcaagcaact ctagccacac aagcagctat tcaaaggaga agaaggccga agaggagatc 2160

gacaggggta gtttcgtttg ataatgttga tgatttggaa caggagaagg attcttcaag 2220

tggaaaagac tgtgatgata ctactaaaat gaatcatttg gagagcggca cagaccgaag 2280

tagccgatcg agaattggta gttcttcgga tattcgaaac gagaacggag aattggacta 2340

caaaaaactg tatgaacagc aactcgcaga gaacgaccga ctgaaagaca aattaaggaa 2400

atctgacgag gagttgcgag aaaccaaaca aactttggaa aaaattaact tggtcacaag 2460

taaaaattcc ttatccgaac tagaaaaacg cgaaaggaga gccatggaga gaaaacttag 2520

tgaaatggaa gaggaactaa agcaattaca aaagttgaaa gctgagaacg aacgcctaaa 2580

ggccgacaac agatcactca cacgtgtcat caataaactt accaactctg caatgtaata 2640

gaatttggaa aatcttaaat ccgagaacca gaggttgaag gatgaaaatg gtgctcttat 2700

cagagtgatc agcaaactgt caaagtagca taagttttag ttgttatcat tcatgcattt 2760

tatgttaagg tgactttaat aatttattgt tagaaaataa tgaaaattca ggatttccca 2820

tttttattca cgttgaaagt tagtttcttt aacacagatt tgtagtagtt aaattgaatc 2880

gtactattgt ggacgcgaat gaaaatagga cgcgaaaaaa agaatatgaa gctgaaatag 2940

aggctatcga gatggaaatt gtaaagaaaa tagcagatga agaagaagta acgagatcat 3000

taggtatata acaggtcaca aacaataaaa gaatataccc agacacaaaa aaataaacta 3060

atgtgatcgt agattccaga gacaacttta aagtaaatat tactagcatc caagaaagat 3120

atggatataa cacaccatat aagagatata tccccgaaaa aaagtaaaac tgttaagaga 3180

ggatctacag tccttatcta aagaaactca agttgtttac cgagttgtgt actttacgag 3240

ttttttgcgt acttagaggt tttttataaa ctgtacagaa gtaaacaaat acttcataaa 3300

aacgtttctg tggggattga tactcggcta ttttaaatat atttattaaa taaatacatg 3360

tttttttgtt tatacctcat aaaagtcatt cttatgatcc aaaaaaaaca aacaatccat 3420

ttggcatact tattt 3435

<210> 297
<211> 632
<212> DNA
<213> Diabrotica virgifera

<220>
<221> misc_feature
<222> (1)..(632)
<223> RNAi target sequence

<220>
<221> misc_feature
<222> (610)..(610)
<223> n can be any nucleotide

<400> 297
ggacagtatg gaagacgatt gggctgtgga taatcagagt ggtggagttg tcgccccaaa 60

aattgcagaa ctacctgaaa ttaagttgtt cgctagatgg aactgcgatg atgtccaagt 120

ttcagacatg tcccttcagg actacattgc agtgaaagaa aaaaatgcaa agtatttacc 180

ccattcagct ggtagatatg ctgcaaaaag gttccgtaaa gcacaatgcc caatcgttga 240

gaggttaaca aactctctaa tgatgcatgg acgtaacaat ggtaaaaaat tgatggctgt 300

cagaattgtt aaacatgctt ttgaaattat ccatttacta actggagaaa atccattaca 360

gattttagtt tctgctatta tcaattcagg acctagagaa gattctactc gtattggtag 420

agctggtact gtaagaagac aagctgttga tgtgtcaccc ttgagaaggg ttaaccaagc 480

aatttggttg ctctgcacag gtgctaggga agcagcattc cgtaatatta aaactattgc 540

tgaatgtttg gctgatgaat taatcaatgc tgccaaggga tcatcaaatt catatgctat 600

caaaaagaan ggatgaactt gaacgtgtag cc                                    632


<210>  298
<211>  21
<212>  DNA
<213>  Artificial Sequence

<220>
<223>  primer


<220>
<221>  misc_feature
<222>  (1)..(21)
<223>  forward primer

<400>  298
gacagtatgg aagacgattg g                                                21


<210>  299
<211>  22
<212>  DNA
<213>  Artificial Sequence

<220>
<223>  primer


<220>
<221>  misc_feature
<222>  (1)..(22)
<223>  reverse primer

<400>  299
ggctacacgt tcaagttcat cc                                               22


<210>  300
<211>  806
<212>  DNA
<213>  Diabrotica virgifera


<220>
<221>  misc_feature
<222>  (1)..(806)
<223>  WCRW transcript ta21523.001_diavv

<400>  300
gcacgaggag cgattgccgg agtggaagtc gctctcaaaa aaagagaaaa ttagaaaagc      60

aaccttttcc ggttcacgcc gtcgttcagc aagagcttgc gatttttatt tttgaaaaat      120

agagagtatt ctctaatatt taaggacagt atggaagacg attgggctgt ggataatcag      180

agtggtggag ttgtcgcccc aaaaattgca gaactacctg aaattaagtt gttcgctaga      240

tggaactgcg atgatgtcca agtttcagac atgtcccttc aggactacat tgcagtgaaa      300

gaaaaaaatg caaagtattt accccattca gctggtagat atgctgcaaa aaggttccgt      360

aaagcacaat gcccaatcgt tgagaggtta acaaactctc taatgatgca tggacgtaac 420

aatggtaaaa aattgatggc tgtcagaatt gttaaacatg cttttgaaat tatccattta 480

ctaactggag aaaatccatt acagatttta gtttctgcta ttatcaattc aggacctaga 540

gaagattcta ctcgtattgg tagagctggt actgtaagaa gacaagctgt tgatgtgtca 600

cccttgagaa gggttaacca agcaatttgg ttgctctgca caggtgctag ggaagcagca 660

ttccgtaata ttaaaactat tgctgaatgt ttggctgatg aattaatcaa tgctgccaag 720

ggatcatcaa attcatatgc tatcaaaaag aaggatgaac ttgaacgtgt agccaaatcc 780

aaccgttaaa tttatttctc atttta 806

<210> 301
<211> 546
<212> DNA
<213> Diabrotica virgifera

<220>
<221> misc_feature
<222> (1)..(546)
<223> RNAi target sequence

<400> 301
caagtagggc taaaatgaag caaattgtaa caaaccaaac ggtaaaaatt cctgagggaa 60

ttacagtaac cactaagtca agggtagtaa ctgtaaaagg accccgaggt aatctgaaga 120

gatccttcaa acatcttacc ctggacattc gaatgatcaa ccccaggtta ctgaaagtag 180

agaaatggtt cggtaccaag aaggaattag ctgctgtcag aacagtatgc tctcatgttg 240

aaaacatgtt aaagggtgtt acaaaaggat accaatacaa gatgagagca gcatatgctc 300

actttcccat taactgtgtc accactgaag gaaacacagt tatcgaaatt agaaatttct 360

tgggagaaaa atacatcaga agagtaaaga tggccccagg tgttacagta gtaaactcca 420

ccaaacaaaa ggatgaactt atcattgaag gaaactcatt ggaggatgtt tcaaagtcag 480

ctgcccttat tcaacaatct acaacagtta aaaataagga tatccgtaaa ttcttagatg 540

ggcttt 546

<210> 302
<211> 23
<212> DNA
<213> Artificial Sequence

<220>
<223> primer

<220>
<221> misc_feature
<222> (1)..(23)

<223>  forward primer

<400>  302
caagtagggc taaaatgaag caa                                              23


<210>  303
<211>  23
<212>  DNA
<213>  Artificial Sequence

<220>
<223>  primer


<220>
<221>  misc_feature
<222>  (1)..(23)
<223>  reverse primer

<400>  303
aaagcccatc taagaattta cgg                                              23


<210>  304
<211>  789
<212>  DNA
<213>  Diabrotica virgifera


<220>
<221>  misc_feature
<222>  (1)..(789)
<223>  02181.002_diavv

<400>  304
ggggaaaact cttcggaata gaagtcgctt tttctgtcca ttttatgtca caagtagggc      60

taaaatgaag caaattgtaa caaaccaaac ggtaaaaatt cctgagggaa ttacagtaac     120

cactaagtca agggtagtaa ctgtaaaagg accccgaggt aatctgaaga gatccttcaa     180

acatcttacc ctggacattc gaatgatcaa ccccaggtta ctgaaagtag agaaatggtt     240

cggtaccaag aaggaattag ctgctgtcag aacagtatgc tctcatgttg aaaacatgtt     300

aaagggtgtt acaaaaggat accaatacaa gatgagagca gcatatgctc actttcccat     360

taactgtgtc accactgaag gaaacacagt tatcgaaatt agaaatttct tgggagaaaa     420

atacatcaga agagtaaaga tggccccagg tgttacagta gtaaactcca ccaaacaaaa     480

ggatgaactt atcattgaag gaaactcatt ggaggatgtt tcaaagtcag ctgcccttat     540

tcaacaatct acaacagtta aaaataagga tatccgtaaa ttcttagatg ggctttatgt     600

atctgaaaaa acaactgttg tacaagaaga ataacttgta tttttgaaat aaatttaagt     660

ttatatttaa aaaaaaaaaa aaaaaatccg ttcaacaatc tacaacagtt aaaaataagg     720

atatccgtaa attcttagat gggctttatg tatctgaaaa aacaactgtt gtacaagaag     780

aataacttg                                                             789

<210>  305
<211>  409
<212>  DNA
<213>  Diabrotica virgifera

<220>
<221>  misc_feature
<222>  (1)..(409)
<223>  RNAi target sequence

<400>  305
ctcttttgac aacggccaac gttctaaagt tgcatcatgg cgtcttcaga aggtgcaggc      60

gtgactacag gtcaaccaaa tttacacaaa caagatttat ctaaattgga tgttaaaaga     120

ttgacggctt tatctccaga agttataagt agacaagcta ccattaacat tggtacaatt     180

ggacatgtag cgcatggaaa atcgactgtg gttaaggcca tatctggggt acaaactgtc     240

agatttaaga atgaactgga aaggaacatt accattaagc taggttatgc taacgcaaaa     300

atctacaaat gtgataatcc gaaatgcccc aggccctcct gctttatttc gggcagttct     360

tcaaaggacg acagtttccc ttgtttacgt ccagcatgtt cgggtagat                409

<210>  306
<211>  20
<212>  DNA
<213>  Artificial Sequence

<220>
<223>  primer

<220>
<221>  misc_feature
<222>  (1)..(20)
<223>  forward primer

<400>  306
ctcttttgac aacggccaac                                                  20

<210>  307
<211>  20
<212>  DNA
<213>  Artificial Sequence

<220>
<223>  primer

<220>
<221>  misc_feature
<222>  (1)..(20)
<223>  reverse primer

<400>  307
atctacccga acatgctgga                                                  20

<210>  308
<211>  1691
<212>  DNA
<213>  Diabrotica virgifera

<220>
<221>  misc_feature
<222>  (1)..(1691)
<223>  WCRW transcript ta02330.001_diavv

<400>  308
ggacaagtga cgtgttttca tgaacgttag atgtcaaaat tattggccgc ctcttttgac      60

aacggccaac gttctaaagt tgcatcatgg cgtcttcaga aggtgcaggc gtgactacag     120

gtcaaccaaa tttacacaaa caagatttat ctaaattgga tgttaaaaga ttgacggctt     180

tatctccaga agttataagt agacaagcta ccattaacat tggtacaatt ggacatgtag     240

cgcatggaaa atcgactgtg gttaaggcca tatctggggt acaaactgtc agatttaaga     300

atgaactgga aaggaacatt accattaagc taggttatgc taacgcaaaa atctacaaat     360

gtgataatcc gaaatgcccc aggccctcct gctttatttc gggcagttct tcaaaggacg     420

acagtttccc ttgtttacgt ccagcatgtt cgggtagatt ccaattggtc agacatgtca     480

gctttgtaga ttgccctggt cacgacattc ttatggctac catgcttaac ggagccgctg     540

tgatggacgc agcattgtta ttaatagcag gaaacgaatc atgtcctcaa cctcaaacta     600

gtgaacattt ggctgctatc gaaatcatga agttgaaaca catcctcatt cttcaaaata     660

aaatagattt ggtgaaggaa gggcaagcca aggagcaaca tgagcaaatt gtgaaatttg     720

tccaaggaac tgtagctgaa ggagccccta taattcccat atccgctcag ttgaaatata     780

acatcgaggt tttgtgcgaa tatatcacta aaaaaattcc aataccaatg agagatttta     840

cttctgaacc aaggctgatc gtgatcagat cttttgatgt taataaacct ggttgtgaag     900

ttaacgattt aaaaggaggt gtagctggtg gcagtatttt aagaggagtg ttgaaggtgg     960

gacaagaaat tgaagtaaga cctggactag ttagcaagga ctcagaagga aagctggcct    1020

gccgaccaat attctctaga atagtatctc tatataccga acaaaacgaa ctgcagtttg    1080

cagttccagg aggactcatt ggtgtcggta ccaaaatcga accaaccctt tgcagagccg    1140

atcgacttgt aggtcaagtt ttgggagcag ttggtgctct acctagtatc tttacagaac    1200

ttgaggtgag ttactatcta ttgaaacgtt tgctgggagt ccgtacagaa ggagacaaga    1260

aaggggccaa agtccagaga ttaaccagaa gtgaagtact ccttgtcaac attggatccc    1320

tcagcacggg aggacgagtt gtagccacta aggccgattt gtgcaagatc gctttaacga    1380

atccagtctg tacggaaatc ggcgaaaaga ttgcactcag tagacgtgtt gaaaaacatt    1440

ggcgtctcat cggatggggg cagatccgcg gaggggagac aatcgagcct accacaagct    1500

aggcttttta tactcgtacc ggtcttttca ggacacttta agccaatttt tgttttctat 1560

ttaatatcgt tttattaaag aagtgtttac gttttgttta tatcttttag gttgattttt 1620

attactgcta tagtgtactg attatccgat tcaaatataa tgtctataaa actaaaaaaa 1680

aaaaaaaaaa a 1691

<210> 309
<211> 670
<212> DNA
<213> Diabrotica virgifera

<220>
<221> misc_feature
<222> (1)..(670)
<223> RNAi target sequence

<400> 309
attgcagtca ttgctgtagt gcacacgtaa tttaagtatt tatttaagaa ttgtctgcaa 60

ataattaatt ataagatggc taaagcccca gctgtaggta ttgatttagg aaccacatac 120

tcctgtgtag gagttttcca acatggcaaa gttgaaatta ttgccaacga ccaaggtaac 180

agaaccacac catcatatgt ggccttcaca gacacagaac gtctcatcgg agatgctgcc 240

aagaaccagg tagccatgaa ccccaataac acaatttttg atgccaaacg tcttattggg 300

cgtcgctttg atgacagtgc tgtacagtct gacatgaaac attggccatt tgaagtagta 360

aatgatgcag gtaaaccaaa gattaaagtt gcatacaagg gcgaagacaa atccttctac 420

ccagaagaag tcagttccat ggtccttaca aaaatgaagg aaacagcaga agcatactta 480

ggaaagaatg tcaccaacgc tgtcatcaca gtacctgcat atttcaatga ctcacaacgt 540

caagctacca aagatgctgg aaccattgct ggactccagg tattacgtat tattaacgaa 600

cccactgctg ctgccatcgc ctatggttta gacaaaaagg gccaaggtga aagaaacgtc 660

ctcattttcg 670

<210> 310
<211> 22
<212> DNA
<213> Artificial Sequence

<220>
<223> primer

<220>
<221> misc_feature
<222> (1)..(22)
<223> forward primer

<400> 310
attgcagtca ttgctgtagt gc 22

<210>  311
<211>  22
<212>  DNA
<213>  Artificial Sequence

<220>
<223>  primer

<220>
<221>  misc_feature
<222>  (1)..(22)
<223>  reverse primer

<400>  311
cgaaaatgag gacgtttctt tc                                                22

<210>  312
<211>  2609
<212>  DNA
<213>  Diabrotica virgifera

<220>
<221>  misc_feature
<222>  (1)..(2609)
<223>  WCRW transcript ta00491.010_diavv

<220>
<221>  misc_feature
<222>  (19)..(19)
<223>  n can be any nucleotide

<220>
<221>  misc_feature
<222>  (49)..(49)
<223>  n can be any nucleotide

<400>  312
ggcccatgga actgacttnt tctgggtaga aggatttgtc tttcgcccnt tgtatgcaaa      60

ctttaatctt ttggtttacc ttgcatcatt tattactttc aaatggccaa tgtttcatgt     120

cagactgtac agcactgtca tcaaagcgac gcccaataag acgtttggca tcaaaaattg     180

tgttattggg gttcatggct acctggttct tggcagcatc tccgatgaga cgttctgtgt     240

ctgtgaaggc cacatatgat ggtgtggttc tgttaccttg gtcgttggca ataatttcaa     300

ctttgccatg ttggaaaact cctacacagg agtatgtggt tcctaaatca atacctacag     360

ctggggcttt agccatcttg gggggatttg attgcagtca ttgctgtagt gcacacgtaa     420

tttaagtatt tatttaagaa ttgtctgcaa ataattaatt ataagatggc taaagcccca     480

gctgtaggta ttgatttagg aaccacatac tcctgtgtag gagttttcca acatggcaaa     540

gttgaaatta ttgccaacga ccaaggtaac agaaccacac catcatatgt ggccttcaca     600

gacacagaac gtctcatcgg agatgctgcc aagaaccagg tagccatgaa ccccaataac     660

acaatttttg atgccaaacg tcttattggg cgtcgctttg atgacagtgc tgtacagtct 720

gacatgaaac attggccatt tgaagtagta aatgatgcag gtaaaccaaa gattaaagtt 780

gcatacaagg gcgaagacaa atccttctac ccagaagaag tcagttccat ggtccttaca 840

aaaatgaagg aaacagcaga agcatactta ggaaagaatg tcaccaacgc tgtcatcaca 900

gtacctgcat atttcaatga ctcacaacgt caagctacca aagatgctgg aaccattgct 960

ggactccagg tattacgtat tattaacgaa cccaccgctg ctgccatcgc ctatggttta 1020

gacaaaaagg gccaaggtga aagaaacgtc ctcattttcg atctgggtgg tggtactttt 1080

gatgtatcaa tcttaacaat tgaagatggt atctttgaag tcaaatcaac tgctggagac 1140

actcacttgg gaggtgaaga cttcgacaac agaatggtca atcactttgt tggagaattc 1200

aagaggaaat acaagaagga ccttaccaca aacaaacgtg ccctccgtcg tttgagaaca 1260

agctgtgaaa gagctaagcg taccctttcc tcatcaaccc aagccagcat tgaaatcgat 1320

tctctatttg agggtattga cttctacacc tcaatcacca gggctagatt tgaagaactc 1380

aacgctgatt tgttcagatc taccatggaa cctgtagaaa aggccatcag ggatgccaag 1440

atggacaaat ctcaagttca cgatattgtg ttggttggtg gatctacccg tattccaaag 1500

gtacaaaaac tgttgcaaga tttcttcaat ggtaaagaat tgaacaagtc gatcaacccc 1560

gatgaagctg ttgcttatgg tgcagccgtc caagccgcca tcttgcacgg agataagtct 1620

gaggaagtac aggacttgct cttgctcgat gtcactccac tctcattggg tattgaaact 1680

gccggaggtg ttatgactgc cctcatcaaa cgtaacacta ccattcccac caagcaaacc 1740

cagaccttca ctacttactc tgacaaccaa cccggagtac ttattcaggt atatgaaggt 1800

gaacgtgcca tgaccaagga caacaacctc ttgggaaaat tcgaacttac tggcattcct 1860

cctgcgcccc gtggtgtccc tcagattgaa gtaaccttcg acattgatgc caacggtatc 1920

ttgaacgtca cagctattga aaaatccacc aacaaagaaa acaagatcac catcaccaat 1980

gataaaggtc gtctcagcaa agaagacatt gaaagaatgg tcaacgatgc agaaaaatac 2040

cgtaacgaag atgataagca aaaggctacc atctctgcca agaacgtcct cgaatcatac 2100

tgtttcaaca tcaagagcac catggaagat gacaaaatca aggacaagat cagcgaaagt 2160

gacaaaacta ccgtcatgga gaaatgcaac gaggttattg cttggttgga tgctaaccaa 2220

ttagccgaca aggaagaata tgaacacaaa caaaaggagc tcgaaaacat ctgcaacccc 2280

atcatcacca agttgtacca aggtgccggt ggagctccag gtggcatgcc cggtggtttc 2340

ccaggtggag cagctccagg agctgcaggc gccgctggag gtgctggacc aaccattgaa 2400

gaagtcgatt aaactattca ttccattttt taaacttttg ttaaccgttg tgcagtatta 2460

cttgcagaca aagcttttta aaattacttt ttttacacaa tttttcaatt gttaaacaac 2520

gtttttggta acagttcaaa aattactgca ataattatgt tcgttcatga ataaaataaa     2580

aagtcaaatc cgaaaaaaaa aaaaaaaaa                                      2609

<210>  313
<211>  672
<212>  DNA
<213>  Diabrotica virgifera

<220>
<221>  misc_feature
<222>  (1)..(672)
<223>  RNAi target sequence

<220>
<221>  misc_feature
<222>  (648)..(648)
<223>  n can be any nucleotide

<220>
<221>  misc_feature
<222>  (649)..(649)
<223>  n can be any nucleotide

<400>  313
tacacgtaaa aatggcttcg acgatatcgg caagacaggc taacagagaa catgttttgg      60

ccgtatccag agacttcata tcccaaccaa gactcactta caaaactgta tctggtgtca     120

acggtccact agtaatcctc gaagatgtca aatttcctaa gttcaacgaa attgtccaac     180

tcagattatc tgacggaagt ttaagatcag gtcaagtact tgaagtcagc ggttccaagg     240

ctgtagtaca agtattcgaa ggtacatcag gtatcgatgc caaacacact gtctgtgagt     300

tcacaggaga tattctcaga actccagtct ctgaggatat gttgggaaga gtattcaacg     360

gctctggaaa acctattgac aaaggtcccc caattctggc tgaagactac ctagatatcc     420

aaggacaacc catcaaccca tggtcccgta tctacccaga ggaaatgatc caaactggta     480

tcacagctat cgatgtgatg aactcgattg cccgtggtca gaagattccc attttctcag      540

ctgctggtct gccacataac gatattgctg cccaaatttg taggcaagcc ggtcttgtca     600

aaatcccagg caaatcagtt ttggatgacc acgaagannc tttgccatcg tgttcgctgc     660

tatgggtgtt aa                                                         672

<210>  314
<211>  22
<212>  DNA
<213>  Artificial Sequence

<220>
<223>  primer

<220>
<221>  misc_feature

<222>   (1)..(22)
<223>   forward primer

<400>   314
tacacgtaaa aatggcttcg ac                                                22


<210>   315
<211>   21
<212>   DNA
<213>   Artificial Sequence

<220>
<223>   primer


<220>
<221>   misc_feature
<222>   (1)..(21)
<223>   reverse primer

<400>   315
ttaacaccca tagcagcgaa c                                                 21


<210>   316
<211>   2583
<212>   DNA
<213>   Diabrotica virgifera


<220>
<221>   misc_feature
<222>   (1)..(2583)
<223>   WCRW transcript ta00620.001_diavv

<400>   316
tcttaaactt ccgtcagatg ggggcagttg ctaatttttt actgtctttg atagacgtct      60

cctgtacacg taaaaatggc ttcgacgata tcggcaagac aggctaacag agaacatgtt     120

ttggccgtat ccagagactt catatcccaa ccaagactca cttacaaaac tgtatctggt     180

gtcaacggtc cactagtaat cctcgaagat gtcaaatttc ctaagttcaa cgaaattgtc     240

caactcagat tatctgacgg aagtttaaga tcaggtcaag tacttgaagt cagcggttcc     300

aaggctgtag tacaagtatt cgaaggtaca tcaggtatcg atgccaaaca cactgtctgt     360

gagttcacag gagatattct cagaactcca gtctctgagg atatgttggg aagagtattc     420

aacggctctg gaaaacctat tgacaaaggt cccccaattc tggctgaaga ctacctagat     480

attcaaggac aacccatcaa cccatggtcc cgtatctacc cagaggaaat gatccaaact     540

ggtatcactg ccatcgatgt gatgaactcg attgcccgtg gtcagaagat tcccattttc     600

tcagctgctg gtctgccaca taacgatatt gctgcccaaa tttgtaggca agccggtctt     660

gtcaaaatcc caggcaaatc agttttggat gaccacgaag acaactttgc catcgtgttc     720

gctgctatgg gtgttaacat ggagactgct agattcttca aacaagattt cgaagaaaat     780

ggttctatgg aaaacgtatg tttgttcttg aacttggcta acgatccaac catcgaaaga 840

atcatcaccc cacgtcttgc tctaacagcc gctgaattct tggcttacca gtgcgaaaaa 900

cacgtgttag tcattttaac tgacatgtct tcatacgctg aagctttgcg tgaggtatct 960

gccgccagag aggaagtacc cggtcgtcgt ggtttccccg gttacatgta caccgatttg 1020

gccaccatct acgaacgtgc cggtcgtgtc gaaggccgta acggttccat cactcaaatc 1080

cccatcttga ctatgcctaa cgacgatatt actcacccta ttcccgattt gacaggttat 1140

attaccgaag gacagatcta cgtcgaccgt caattgcaca acaggcagat ctacccacca 1200

attaacgtac tgccatctct ctcacgtttg atgaagtctg ctatcggcga aggtatgacc 1260

aggaaggacc actctgacgt ttctaaccaa ttgtatgctt gttatgctat cggtaaggac 1320

gtccaggcca tgaaagccgt agtaggtgaa gaagctctaa cacccgacga tcttctctac 1380

ctcgagttcc taacgaaatt cgaaaagaac ttcatcaccc aaggaaacta cgaaaaccgc 1440

accgtcttcg agtctttgga catcggctgg caactactcc gtatcttccc taaggaaatg 1500

ttgaaacgta tccctgcaag cacattggcg gaattctatc caagagactc tcgtcactag 1560

ttggtttatg tataaaattt atatatattt aggaagtttg ttaggtttgt tacaaatatg 1620

tccacttatt gtatcgatta ttttcaattg agagatattt taccaaaaca atttgagccg 1680

atgagattct acggtgtaat ttttgtaaat acttaaagag cgtgtttcaa aaatgcgctt 1740

aatataatat attatcaata cagagcaacc tttttttctt tattattttt tcttgttgaa 1800

caatatataa agttcttgaa ctccctaagt ggaacataga gcttcagtga aaatgcacca 1860

tcacctcatt ccaagacttt ccttgacttc ttaccaattg ttggacaatt ataactcaga 1920

ttaattttac taggtgacag ttccataagt aaaaatatgc aatggctata tattgcgtac 1980

taaacttaag aagaaaaatt gtgtcgctct ggaagaaaga ttacttgcat aagaatgaaa 2040

atcagtgtca tattttgcgt ttatgacatc cctagacgat actatttttt cgttactata 2100

ttttttttaa agagatcaag agcaacaatt tggagactga tattcttatg cgagttgttt 2160

tcctactttg tcctattttg aagagtctac aattatttac ttaccatttg attaaattag 2220

atgaacataa gacaatacta tatttttatc atgtaagttg atattcccta tgtaaattat 2280

tggcacaatg taacctccaa cttgatttat acatttattt tacttttgtt tgtctatttt 2340

ttctgtaata gagtggaaga attaacattc cattaaatgt cattgcccaa tgtataaagc 2400

aatatacctc ttaccattcg atgctttttc agtattttaa ttctagatgg tgaacggaga 2460

tttttcttga tgtaggtttt gacattttcg atgttactgt aaaaatctaa ttttatgtta 2520

aatagattag tgtacaaaaa tgtgaataaa cactatacat atgttaaaaa aaaaaaaaaa 2580

aaa 2583

<210>  317
<211>  500
<212>  DNA
<213>  Diabrotica virgifera

<220>
<221>  misc_feature
<222>  (1)..(500)
<223>  RNAi target sequence

<400>  317
actttgacga gaggcgttgt tagttacttc gttgtgtcga catttttctg tttagtgtac      60

cgattaatct tcaaatatta caatggctga ccaactcacc gaagaacaaa ttgccgaatt      120

caaagaagct ttctcactat tcgataaaga tggtgatggt acaattacga ctaaagaatt      180

aggaacagta atgagatctc taggacaaaa tccaacagag gctgaattac aggatgtgac      240

caatgaagta gatgccgatg gtaacggcac gatcgatttc ccagaatttt taacgatgat      300

ggcacgtaaa atgaaagata ccgatagtga ggaagaaatt cgtgaagcat tccgagtgtt      360

cgacaaagac ggcaatggtt tcatctcagc agcagaattg cgccacgtca tgaccaactt      420

gggtgaaaaa ttgacagacg aagaagtcga tgaaatgatt cgggaggccg atatcgatgg      480

tgatggtcaa gtcaattacg                                                  500

<210>  318
<211>  20
<212>  DNA
<213>  Artificial Sequence

<220>
<223>  primer

<220>
<221>  misc_feature
<222>  (1)..(20)
<223>  forward primer

<400>  318
actttgacga gaggcgttgt                                                  20

<210>  319
<211>  22
<212>  DNA
<213>  Artificial Sequence

<220>
<223>  primer

<220>
<221>  misc_feature
<222>  (1)..(22)
<223>  reverse primer

<400>  319
cgtaattgac ttgaccatca cc                                                22

<210>  320
<211>  713
<212>  DNA
<213>  Diabrotica virgifera

<220>
<221>  misc_feature
<222>  (1)..(713)
<223>  WCRW transcript ta02046.005_diavv

<400>  320
gcagcagcag ttcccccctc tcttctctta cgtcgatcct aaacaaaacg agaaaagaca        60

gttggctggt gcactttgac gagaggcgtt gttagttact tcgttgtgtc gacatttttc       120

tgtttagtgt accgattaat cttcaaatat tacaatggct gaccaactca ccgaagaaca       180

aattgctgaa ttcaaagaag ctttctcact attcgataaa gatggtgatg gtacaattac       240

gactaaagaa ttaggaacag taatgagatc tctaggacaa aatccaacag aggctgaatt       300

acaggatatg atcaatgaag tagatgccga tggtaacggc acgatcgatt tcccagaatt       360

tttaacgatg atggcacgta aaatgaaaga taccgatagt gaggaagaaa ttcgtgaagc       420

attccgagtg ttcgacaaag acggcaatgg tttcatctca gcagcagaat tgcgccacgt       480

catgaccaac ttgggtgaaa aattgacaga cgaagaagtc gatgaaatga ttcgggaggc       540

cgatatcgat ggtgatggtc aagtcaatta cgaagagttt attagaagtc acctcttggc       600

caactaagat attattgatt acaaagaaaa atagaagaat atattataat ttacagatac       660

tatattaatg ataatagaat aagaataaaa tgtaaataaa tagttttgct cat             713

<210>  321
<211>  514
<212>  DNA
<213>  Diabrotica virgifera

<220>
<221>  misc_feature
<222>  (1)..(514)
<223>  RNAi target sequence

<400>  321
tttggcaaca ttgtgtatga tgtcatagga gcggggtcag ataggaatgt tgtagttttg        60

aatgatatac acatagatat aatggactat atagtgcctg ctagttgtac agatagcgag       120

tttatgagaa tgtgggcgga atttgaatgg gaaaataagg taaccgttaa cacacccctc       180

acggaacttt cagaatacct cgaacatcta ctcaaaagca caaatttgaa atgtttaaca       240

tcagaaaaag ctctgagcgg gcagtgtggt tttatggcag ccaatttata tgcaaaatcc       300

atttttggag aagacgcttt ggccaactta agtatagaga aaccttttaa taaacccgat     360

gcgccagtaa gcggtcatat tagaataagg gccaaaagtc agggcatggc cttaagttta     420

ggagacaaag tcaatatgac acagaagagc acacaacata aagtagtagc tgcataaata     480

aaaacgtttt tttctgtttc aacccttttc ttca                                 514


<210>  322
<211>  22
<212>  DNA
<213>  Artificial Sequence

<220>
<223>  primer


<220>
<221>  misc_feature
<222>  (1)..(22)
<223>  forward primer

<400>  322
tttggcaaca ttgtgtatga tg                                              22


<210>  323
<211>  23
<212>  DNA
<213>  Artificial Sequence

<220>
<223>  primer


<220>
<221>  misc_feature
<222>  (1)..(23)
<223>  reverse primer

<400>  323
tgaagaaaag ggttgaaaca gaa                                             23


<210>  324
<211>  3271
<212>  DNA
<213>  Diabrotica virgifera


<220>
<221>  misc_feature
<222>  (1)..(3271)
<223>  WCRW transcript ta00934.001_diavv

<400>  324
gggggtcgca tcaaaacgtc aacaatctga cacgtctgta atgtttagcc tgaattttga      60

gtaaaattac cgaaaaacat aattaaaatt gatttattaa gagtacataa taacgtacga     120

aaatgaccgc ggtagaacaa ccttgttaca cactaataaa cttgccaaca gattcggagc     180

cctacaatga aatgcaacta aaaatggatt tagaaaaggg tgaggttaaa gtaaaaataa 240

gagcattaga aaaaataatt cacatgattc tggcaggaga aaggttgccg aatggattcc 300

taatgaccat cataagaaac gttttacctc tacaagatca tttggcaaaa aaactattat 360

tgattttctg ggaaatagtt ccaaaaacaa atccagaagg taaactacta caagagatga 420

ttttggtatg tgatgcctat agaaaagatc tgcaacaccc aaatgaattt ttgagaggtt 480

ctacacttcg cttcttgtgc aaactgaagg aaccagaatt gttggaacca ttaatgccca 540

gtattagagc ttgtttggat cataggcaca gctatgtgag gaggaatgct gtactggcaa 600

tttttaccat ttacaaaaat tttgaagccc tcattccaga tgctcctgaa ctgatctcca 660

attatttgga tggtgagcaa gacatgtctt gtaaaagaaa tgcgttttta atgcttcttc 720

atgctgacca agaaagggcg ttgtcgtatt tggcatcatg tttagatcaa gtaaattcat 780

ttggagatat tctacaactg gtcatcgttg agttgatata taaggtgtgt cattccaatc 840

ctgcggaaag atctagattt attagatgta tatataactt gttgaactca agcagtcctg 900

ctgtcaggta cgaagctgca ggaactttag tcaccctctc cagtgccccg actgccgtta 960

aagctgctgc tagctgttac attgagttaa ttatcaaaga aagtgacaac aatgtaaaac 1020

tcatcgtttt ggacaggctg atagcactta aggagcttcc taatcacgaa agaattctgc 1080

aggatttagt tatggacata ctgagagtac tctctgctcc tgacttagaa gtccgcaaga 1140

agactttaag tctagccctt gaattagtct cttcacggaa catagaagaa atggtattag 1200

tattaacaaa ggaagtgagt aaaacggtag acagtgaaca tgaggataca ggaaagtaca 1260

ggcaattgtt agtaaggact ctacattcgt gttccattaa gttcccagat atcgcacgta 1320

gtgttatacc agtcttgatt gaatttttat ccgataataa tgaactggct gccacagatg 1380

tattgctgtt cttaagggaa gccatacaga agtttaaaga attgcaaccg ttaattattg 1440

agaaactcat cgaaactttc aaagacatta aattggtcaa agtccataga gcagcaattt 1500

ggattttggg agaatacgcg agtactgctt ccgatataga agttatcgtt ggagaaatta 1560

acagattgtt gggtgaagga tccctcgttg aagctgagca gaagttaata gcaggagata 1620

cggaagagaa tgctcctgca cctgctgcag gcgccaccac tttagttact tccgatggaa 1680

catatgctac ccaatcagct ttcaacactg tcagccaaac cactaaagaa gcacgacctc 1740

ctctaagaca atacctcatg gatggtgatt ttttcatcgg agcctctttg gcatctacat 1800

taaccaaact gtctttgcgg tatgaggacc tcacctctcc tgctgctagc aatggattca 1860

atgccaaaat tatgcttatt atggctggaa ttcttcactt gggaaaatca ggacttccca 1920

caaaatcaat aaccaacgac gataaagacc acattctgtt ctgtttacga gtcctatctg 1980

atcgttctcc aatcattgtt gaaattttca aaaaattgtg ccgctcggca ctaaatgaga 2040

tgcttctagc taaggaatcg gtagaagcga tctcgcaaaa gagcaaagaa aaaaacaagc 2100

gtacgattca aactgacgac gctataagct tcctgcaatt agagacagat aaaagtggag 2160

agctaggaga aaacgtattc gagatgtcgc tgtcacaagc tttagtagga ggtcgaacgg 2220

gaggtggcga atcagtatta agttccaata aattagataa aatcacacaa ctgactggtt 2280

tttccgatcc agtttattcc gaagcatacg ttcacgtgaa tcagtacgat atcgtgcttg 2340

atgtcttaat cgtaaaccaa actaacgata ctttacaaaa ctgcacgcta gagctggcta 2400

ctttaggcga tttgaagttg gtagagaagc cacaacctgt cgtattggcg cccaaagact 2460

tttgcaacat taaagctaac gtgaaagtgg cctcaactga aaacggaatt atatttggca 2520

acattgtgta tgatgtcata ggagcggggt cagataggaa tgttgtagtt ttgaatgata 2580

tacacataga tataatggac tatatagtgc ctgctagttg tacagatagc gagtttatga 2640

gaatgtgggc ggaatttgaa tgggaaaata aggtaaccgt taacacaccc ctcacggaac 2700

tttcagaata cctcgaacat ctactcaaaa gcacaaattt gaaatgttta acatcagaaa 2760

aagctctgag cgggcagtgt ggttttatgg cagccaattt atatgcaaaa tccatttttg 2820

gagaagacgc tttggccaac ttaagtatag agaaaccttt taataaaccc gatgcgccag 2880

taagcggtca tattagaata agggccaaaa gtcagggcat ggccttaagt ttaggagaca 2940

aagtcaatat gacacagaag agcacacaac ataaagtagt agctgcataa ataaaaacgt 3000

ttttttctgt ttcaaccctt ttcttcattt taaatcatat tctgtaattt aattaattta 3060

atatcatatt actggttaca cattgttaga ttataaatat cttttaaaga ataaaattta 3120

tttagctttt acaaagagcg gaaagcaatc tttatcttct tctaataaga tcaacataaa 3180

gttttctgga aaatataata tataatttaa aaatgtttag catttattag tgaaatacat 3240

ggcttactat tttgatgaca aagtcagtgt a 3271

<210> 325
<211> 606
<212> DNA
<213> Diabrotica virgifera

<220>
<221> misc_feature
<222> (1)..(606)
<223> RNAi target sequence

<220>
<221> misc_feature
<222> (577)..(577)
<223> n can be any nucleotide

<400> 325
gccctcatca aacgtaacac taccattccc accaagcaaa cccagacctt cactacttac 60

tctgacaacc aacccggagt acttattcag gtatatgaag gtgaacgtgc catgaccaag 120

gacaacaacc tcttgggaaa attcgaactt actggcattc ctcctgcgcc ccgtggtgtc 180

cctcagattg aagtaacctt cgacattgat gccaacggta tcttgaacgt cacagctatt 240

gaaaaatcca ccaacaaaga aaacaagatc accatcacca atgataaagg tcgtctcagc 300

aaagaagaca ttgaaagaat ggtcaacgat gcagaaaaat accgtaacga agatgataag 360

caaaaggcta ccatctctgc caagaacgtc ctcgaatcat actgtttcaa catcaagagc 420

accatggaag atgacaaaat caaggacaag atcagcgaaa gtgacaaaac taccgtcatg 480

gagaaatgca acgaggttat tgcttggttg gatgctaacc aattagccga caaggaagaa 540

tatgaacaca aacaaaagga gctcgaaaac atctgcnacc ccatcatcac caagttgtac 600

caaggt 606

<210> 326
<211> 22
<212> DNA
<213> Artificial Sequence

<220>
<223> primer

<220>
<221> misc_feature
<222> (1)..(22)
<223> forward primer

<400> 326
gccctcatca aacgtaacac ta 22

<210> 327
<211> 22
<212> DNA
<213> Artificial Sequence

<220>
<223> primer

<220>
<221> misc_feature
<222> (1)..(22)
<223> reverse primer

<400> 327
accttggtac aacttggtga tg 22

<210> 328
<211> 2209
<212> DNA
<213> Diabrotica virgifera

<220>
<221> misc_feature

<222>   (1)..(2209)
<223>   WCRW transcript 454run12_isotig00560

<400>   328
gatttgattg cagtcattgc tgtagtgcac acgtaattta agtatttatt taagaattgt      60

ctgcaaataa ttaattataa gatggctaaa gccccagctg taggtattga tttaggaacc     120

acatactcct gtgtaggagt tttccaacat ggcaaagttg aaattattgc caatgaccaa     180

ggtaacagaa ccacaccatc atatgtggcc ttcacagaca cagaacgtct catcggagat     240

gctgccaaga accaggtagc catgaacccc aataacacaa tttttgatgc caaacgtctt     300

attgggcgtc gctttgatga cagtgctgta cagtctgaca tgaaacattg gccatttgaa     360

gtagtaaatg atgcaggtaa accaaagatt aaagttgcat acaagggcga agacaaatcc     420

ttctacccag aagaagtcag ttccatggtc cttacaaaaa tgaaggaaac agcagaagca     480

tacttaggaa agaatgtcac caacgctgtc atcacagtac ctgcatattt caatgactca     540

caacgtcaag ctaccaaaga tgctggaacc attgctggac tccaggtatt acgtattatt     600

aacgaaccca ctgctgctgc catcgcctat ggtttagaca aaaagggcca aggtgaaaga     660

aacgtcctca ttttcgatct gggtggtggt acttttgatg tatcaatctt aacaattgaa     720

gatggtatct ttgaagtcaa atcaactgct ggagacactc acttgggagg tgaagacttc     780

gacaacagaa tggtcaatca ctttgttgga gaattcaaga ggaaatacaa gaaggacctt     840

accacaaaca aacgtgccct ccgtcgtttg agaacaagct gtgaaagagc taagcgtacc     900

ctttcctcat caacccaagc cagcattgaa atcgattctc tatttgaggg tattgacttc     960

tacacctcaa tcaccagggc tagatttgaa gaactgaacg ctgatttgtt cagatctacc    1020

atggaacctg tagaaaaggc catcagggat gccaagatgg acaaatctca agttcacgat    1080

attgtgttgg ttggtggatc tacccgtatt ccaaaggtac aaaaactgtt gcaagatttc    1140

ttcaatggta aagaattgaa caagtcgatc aaccccgatg aagctgttgc ttatggtgca    1200

gccgtccaag ccgccatctt gcacggagat aagtctgagg aagtacagga cttgctcttg    1260

ctcgatgtca ctccactttc attgggtatt gaaactgccg gaggtgttat gactgccctc    1320

atcaaacgta acactaccat tcccaccaag caaacccaga ccttcactac ttactctgac    1380

aaccaacccg gagtacttat tcaggtatat gaaggtgaac gtgccatgac caaggacaac    1440

aacctcttgg gaaaattcga acttaccggc attcctcctg cgccccgtgg tgtccctcag    1500

attgaagtaa ccttcgacat tgatgccaac ggtatcttga acgtcacagc tattgaaaaa    1560

tccaccaaca aagaaaacaa gatcaccatc accaatgata aaggtcgtct cagcaaagaa    1620

gacattgaaa gaatggtcaa cgatgcagaa aaataccgta acgaagatga taagcaaaag    1680

gctaccatct ctgccaagaa cgtcctcgaa tcatactgtt tcaacatcaa gagcaccatg    1740

gaagatgaca aaatcaagga caagatcagc gaaagtgaca aaactaccgt catggagaaa    1800

tgcaacgagg ttattgcttg gttggatgct aaccaattag ccgacaagga agaatatgaa 1860

cacaaacaaa aggagctcga aaacatctgc aaccccatca tcaccaagtt gtaccaaggt 1920

gccggtggag ctccaggtgg catgcccggt ggtttcccag gtggagcagc tccaggagct 1980

gcaggcgccg ctggaggtgc tggaccaacc attgaagaag tcgattaaac tattcattcc 2040

attttttaaa cttttgttaa ccgttgtgca gtattacttg cagacaaagc tttttaaaat 2100

tacttttttt acacaatttt tcaattgtta aacaacgttt ttggtaacag ttcaaaaatt 2160

actgcaataa ttatgttcgt tcatgaataa aataaaaagt caaatccgc 2209

<210> 329
<211> 718
<212> DNA
<213> Diabrotica virgifera

<220>
<221> misc_feature
<222> (1)..(718)
<223> RNAi target sequence

<400> 329
gttgggattg ggatttaaca aatacgtcgt tctaaatttc taaaactgca tacaaaaaat 60

ccttaaaaaa ctagtgaaga ttactaaagt gcaatagata caaaaacaat atttttacgt 120

attcaccata ataatataaa cgtagaaaat gaatcccgag tatgattatt tattcaaact 180

tctgctgatt ggagattcag gagtaggaaa atcttgtctt ctactgagat ttgcagatga 240

tacctacaca gaaagctata ttagtaccat tggcgtagat tttaaaatca ggacaatcga 300

tttagatgga aagacaatta aattgcaaat ttgggataca gcaggtcagg aaaggtttag 360

aacgattaca tcaagttatt accgaggagc acatggtatt attgtagtgt acgattgcac 420

agaccaagat tcattcaata acgttaaaca atggctcgaa gaaatcgacc gttatgcgtg 480

tgacaatgta aacaaattac tggtagggaa taaaagcgat ttgacaacta agaaagttgt 540

cgacttcact acagccaagg agtatgccga ccaattgggt ataccatttt tggaaacctc 600

agctaagaat gcaaccaatg tagaacaggc ctttatgact gtggccgctg aaataaaaaa 660

tagagtagga cctccatctt ctgcggtaga ccaaggaaat aaggttaggt tcgatcaa 718

<210> 330
<211> 22
<212> DNA
<213> Artificial Sequence

<220>
<223> primer

<220>

<221>  misc_feature
<222>  (1)..(22)
<223>  forward primer

<400>  330
gttgggattg ggatttaaca aa                                                22


<210>  331
<211>  24
<212>  DNA
<213>  Artificial Sequence

<220>
<223>  primer


<220>
<221>  misc_feature
<222>  (1)..(24)
<223>  reverse primer

<400>  331
ttgatcgaac ctaaccttat ttcc                                              24


<210>  332
<211>  4464
<212>  DNA
<213>  Diabrotica virgifera


<220>
<221>  misc_feature
<222>  (1)..(4464)
<223>  WCRW transcript ta00232.001_diavv

<400>  332
gaatcaaggg gttgggattg ggatttaaca aatacgtcgt tctaaatttc taaaactgca      60

tacaaaaaat ccttaaaaaa ctagtgaaga ttactaaagt gcaatagata caaaaacaat     120

atttttacgt attcaccata ataatataaa cgtagaaaat gaatcccgag tatgattatt     180

tattcaaact tctgctgatt ggagattcag gagtaggaaa atcttgtctt ctactgagat     240

ttgcagatga tacctacaca gaaagctata ttagtaccat tggcgtagat tttaaaatca     300

ggacaatcga tttagatgga aagacaatta aattgcaaat ttgggataca gcaggtcagg     360

aaaggtttag aacgattaca tcaagttatt accgaggagc acatggtatt attgtagtgt     420

acgattgcac agaccaagat tcattcaata acgttaaaca gtggctcgaa gaaatcgacc     480

gttatgcgtg tgacaatgta aacaaattac tggtagggaa taaaagcgat ttgacaacta     540

agaaagttgt cgacttcact acagccaagg agtatgccga ccaattgggt ataccatttt     600

tggaaacctc agctaagaat gcaaccaatg tagaacaggc ctttatgact atggccgctg     660

aaataaaaaa tagagtagga cctccatctt ctgcggtaga ccaaggaaat aaggttaggt     720

tcgatcaaag tcgcccagtc gaaacaacca aatccggttg ctgctgaaaa tatttagttc     780

tctttggttg aaggtggtca ctcatgtcat ctcttttgta cggtagcagc gctacagtta 840

ctcttattct agtaatatcc gtctgtatgt atagttattt taaagttttt gatttgattt 900

tcactttaga tttttttttc aaaaatatag aaaacaccaa tatctcttag cctaatctta 960

actcccttag ataaatatgt aattaacaaa aatatttata aaaccatttt tactaaacta 1020

ttagtccaca gtatatcgtt ccttttcagg aatgattgca aaagtcttaa aaatggtttt 1080

aattgtttcg tttaaacgag tctaccactt tatgaaaaat ttcagaaaga ttatacgttg 1140

aaataaaata aattattttt gtcggacaaa aaagaggggc aaacacccgc aagatgtcac 1200

tctagcgcgg tacagttttg ttaattacaa aactcataat tttcaattga aagtattgat 1260

aattactaaa tattgtggat ctcttgtccg acaaatttgc agcctcgttt ctttagtagg 1320

acaacggaaa tatgttaatt atggtaggtt atggtttgcg gttacaaaag tcataatttt 1380

aattttttaa cgttcaaact gttcaaaaag taattgtccg acaaaaaaat tgttcgacgc 1440

aaacttataa caaaaacagt gacagtctac ttgtgtttgt cggtataatc gcaaattacc 1500

tacctctagt atagatgctt agtctagtaa ttagtctgtt ccgctctgcg gaattgagta 1560

aatggaatta attaaaaata agtgtaaact atgaacaata atatatttat ttgtgtcgat 1620

aaaaagacgt gttttgctcg aaagggtgtg ttgttgacgc accgcatcta tcccgacgcc 1680

cgctggggtg tgactgccga cgggaggttg taaatcgacc cgtggttatg tatcgactga 1740

cgctgtggct gaaaaaaacc acaaacgcta atatgtttgt acttctaaca tctggccaga 1800

aaagcgataa attactacat ctgtagtttt aagaaacgtc ttcgaatgca tttccatgca 1860

ttgacatgga atctgataag gtaattaatt catggggaat tcagtttgtt aaactgaaca 1920

caccaaaaat atggttaggg atacatttcg tttcgtaaaa cttcttagca tctggtttgt 1980

atattaaatg tgaatttata tgaccgtttt gagaaatgat attaaaaaat aaaattagca 2040

aaaatagtaa ttaaagcgtg tcgccacaag tctatgttta cattcggttt ctatacattt 2100

tctgacgttc taaacgtcac tggcataaca gtctaaacag tgcaacaagt aaagggtcca 2160

gggctatgtt agctcaattt atctagtact gtggcggttg cagtaaaaaa agttttgaaa 2220

ttaatttgtc ggactggcta tctgataccc aatttttgtc ggacattgct ctatttggat 2280

aaagaaccct ccaatctatc actttcgtgt tcgaaatttt ataatcgcaa tctgcataat 2340

tgttaattat taacatattt gcgttgttcg gctaaagagg ggaggctgca aatttgtctg 2400

acaagagatc aacaatctag taattaatta ttaataaact aaaagtgggt agttccctgg 2460

gttggctgta taccatgtag ttaaaaaact ctaaacatgt agtaaggcca cttctgtgta 2520

aaaggtgtat gtactgaaat tttaaaaatc ccgatggatt gaataaacta ggttcattca 2580

gaacgttttc ggacctgtca gtccatcatc agtgaattca tatatacttg cgctaactag 2640

tcccagaacc aaacaatggt tgtacttata aatcaatcta cattatagtg ttatgtattt 2700

ggaaaggcta agcgccgatg ttctgatgga ctgataggtc cgaaaacgtt ctgaattgga 2760

acctatttta atcaattcat tgggattttt agggttgtag tagatatacc ttttacacag 2820

aagtggtttt atttcatgtt ttttgtccga cgaaaataat ttattttatt ctaacgcgta 2880

attttttgat taaaattaaa aattatagtt ttgcaatctc aaaacaaccg cgctagagct 2940

acaggtttaa aaggaagtca tcaaaaccat ttttaagact tttgtaatca tttttgaaaa 3000

acgacgatat actgtggact acatatatga gatcattaga acgagaaaga tatactcatc 3060

gactgttaat tttgtattgt ttttagtata cctttctctg ttttataaat ccagatcatg 3120

atagattgac gttagaaatt attgttcgct tttcaaacgt gtattagcag aattttggca 3180

aattaaatat aaaaattcga gatcctttat aaaaaggtat atttaaaatt ccctaagtaa 3240

gggttatatt aagtaacaga acgttttcgg attaaaaaat ccaccatcag tgttacaaaa 3300

aaaaaatagc atgcctgagg caccgaaatg ttacgggtaa aaacccttta aatgttgaca 3360

gttgttgtct tatactatga tgttgctaat attcctggat attacccagg gcaacacagg 3420

actcttccca cgcggttgga atttgagaat ttcaatcacg aatttgacac ttccaaaata 3480

cgtaaagttt tccacatctt taatctgcat gttgtgaata gtaaatagag tgtcgttcct 3540

tgcattggtt ctcatggact tgattttact aatattaatt ttcaaatcta ttttattggc 3600

ttcagtggaa agtgaatttt gaaaaaaaat ctgctaaaaa tagtttaaaa aatatttttt 3660

tttagttgtg acactataca gataaaataa cgaaaaattt acgaaataat atttatcaaa 3720

agtggctctt gcagtacaga atataatatc attattatta tctagttaat agactcgtac 3780

tttttaacga tgtaacttta aaaacaatta acaacgttaa aaaaatactg caaaaaggaa 3840

gataaataag tgataaatgt gtcacttttc ttgagcacta cgcagattgt ttggaataaa 3900

gctatcacgt attatactgt gtaaactcca caaacgacac tttatacaca tcttagatga 3960

acaaatgcct ggcacaagtc gatatgtgac actgtttatg ttcggtgcac cgtcagtttt 4020

ggttaatcat tagtgtcagc taacgaaaat actttaacta taaacttgac actttttgag 4080

ataagatttt gtagttttat taaactgttg gtgcaataaa accgttttta aaattttttg 4140

agttgtgaca ctatttgagc ggaggtgcga catgtggaaa atatttacaa ttatttcata 4200

tatcacttgt tctatacaat aaaaaaacgt catttctaac atgattttta gtgatttctg 4260

ttggtccaaa acaatttatt atattatcca tatatacttg ttatttttta tgtaatgatt 4320

tttcttattc gctacacatt aacgtcattt tttgtttata tcaaataaaa agtaatattg 4380

taagtaatat gtgattgtaa atcttggtca ataggttttt tgtaaattaa agttattctg 4440

aactggaaaa aaaaaaaaaa aaaa 4464

<210>  333
<211>  704
<212>  DNA
<213>  Diabrotica virgifera

<220>
<221>  misc_feature
<222>  (1)..(704)
<223>  RNAi target sequence

<400>  333
ttttgggtgt gaatctgtct atttaattta ctcctcctgg ttaaaaaata aacattagta      60

gattatatct agggtgctcg gtaaaaggag tataagtcaa aaatggtcaa aatattattt     120

ttgcagaata agactataaa ggactatatc tttaccttgg tatcaataga acaagtcaat     180

atgctaacat aaattaaatc aaattaattg gtctttttag tacaactact taaatgaact     240

tttaatttgg taacaacagt ttatgtcaac ttatactgtt tatacacttc gagtttgtga     300

actattcttg gctctaatag tacatgtcga catatgtact gttttgacca ttgatactga     360

ataatatcca agtaagacgg tcgacttata ctaagacgat cgctttcaga tacgtgtgtc     420

aagcttacta agcaaatatt actcttttct gtacgagatt ttcaagaaac aactgataac     480

tattcgcgtt tggctgataa ctaaaccgtg ggaattttac caattaaaga ttacacttaa     540

tagggacagc gagaataagt aatactaact tcttacgtag gtttaatttg ataaaaatag     600

atgagtaagt ggtttatttt ctggaccact gtgcaatatg ggtgatcgta acaaacgtgt     660

tgcatatatt gttagcctta tagactccga accacctaaa atca                      704

<210>  334
<211>  24
<212>  DNA
<213>  Artificial Sequence

<220>
<223>  primer

<220>
<221>  misc_feature
<222>  (1)..(24)
<223>  forward primer

<400>  334
ttttgggtgt gaatctgtct attt                                             24

<210>  335
<211>  22
<212>  DNA
<213>  Artificial Sequence

<220>
<223>  primer

<220>
<221>  misc_feature
<222>  (1)..(22)
<223>  reverse primer

<400>  335
tgattttagg tggttcggag tc                                                22


<210>  336
<211>  737
<212>  DNA
<213>  Diabrotica virgifera


<220>
<221>  misc_feature
<222>  (1)..(737)
<223>  WCRW transcript ta41917.001_diavv

<400>  336
gggattattc tagtgacttt tttgggtgtg aatctgtcta tttaatttac tcctcctggt       60

taaaaaataa acattagtag attatatcta gggtgctcgg taaaaggagt ataagtcaaa      120

aatggtcaaa atattatttt tgcagaataa gactataaag gactatatct ttaccttggt      180

atcaatagaa caagtcaata tgctaacata aattaaatca aattaattgg tctttttagt      240

acaactactt aaatgaactt ttaatttggt aacaacagtt tatgtcaact tatactgttt      300

atacacttcg agtttgtgaa ctattcttgg ctctaatagt acatgtcgac atatgtactg      360

ttttgaccat tgatactgaa taatatccaa gtaagacggt cgacttatac taagacgatc      420

gctttcagat acgtgtgtca agcttactaa gcaaatatta ctcttttctg tacgagattt      480

tcaagaaaca actgataact attcgcgttt ggctgataac taaaccgtgg gaattttacc      540

aattaaagat tacacttaat agggacagcg agaataagta atactaactt cttacgtagg      600

tttaatttga taaaaataga tgagtaagtg gtttattttc tggaccactg tgcaatatgg      660

gtgatcgtaa caaacgtgtt gcatatattg ttagccttat agactccgaa ccacctaaaa      720

tcactagaaa aaggacc                                                     737


<210>  337
<211>  221
<212>  DNA
<213>  Diabrotica virgifera


<220>
<221>  misc_feature
<222>  (1)..(221)
<223>  RNAi target sequence

<400>  337
aatgaaaaac ataataacgg aagccaaaaa attccgactt aaaataaatg aagaaaagac       60

aaagtatatg ataatgggag agatccaaaa agaaaaagag aataacatca tagtacaaat 120

agatggagaa aaaacatact cgttcaaaag agccaaagaa attatttacc tgggagccaa 180

aatagatgaa aatggtcatg aagaagggga gataaaggca a 221

<210> 338
<211> 24
<212> DNA
<213> Artificial Sequence

<220>
<223> primer

<220>
<221> misc_feature
<222> (1)..(24)
<223> forward primer

<400> 338
aatgaaaaac ataataacgg aagc 24

<210> 339
<211> 20
<212> DNA
<213> Artificial Sequence

<220>
<223> primer

<220>
<221> misc_feature
<222> (1)..(20)
<223> reverse primer

<400> 339
ttgcctttat ctccccttct 20

<210> 340
<211> 3813
<212> DNA
<213> Diabrotica virgifera

<220>
<221> misc_feature
<222> (1)..(3813)
<223> WCRW transcript ta03247.005_diavv

<400> 340
cttagcgaga tgcgtagaga gcagaaagaa tatagagatg aaataagaga gctacgccag 60

gagaatgtaa cattaagaga agaaaacacg aaactaaaac aggcagttta tcaaatagaa 120

gaacggcttg aaagtttaga aaatcaaaaa aggcggaaaa atataattat acaaggattg 180

aacatagata caaatgggcc agatatccta aaacatgcaa tgaaaaattt tatgcagaca 240

gaattaaaag tagatgtaca aatagagaag gctctgaagt taggagaaaa agtttgtctg 300

gttgagcttg aaaaggaaga agataaaaga aaaatcatgc gaaacaaaac caaattgaga 360

aatcgagaag aaggtctagt ttatatcaac gatgacctat caaagaacga cagaattgta 420

cagaaacatg tcagacaaaa agcgcaagaa ctaagaaagg aagggagaaa agtgaaagta 480

gggtataaga aaatatatgc cgatcatgaa atatggcgct gggatggaat gaaccaaacg 540

ttggtggtag agaccaaagc aaaaaactaa gcaatagcaa tgaaaggaaa caaatgacga 600

cgaaaaatgc aaagaatagg attacgaact atggaaatag aaacaaatca agacagcaat 660

aaaaaagaaa agccgaaaaa tgacaagata cagtttgcaa cagggataaa taagacggaa 720

acgacgcagg caatggacaa ggcaataagt aaagataaca aaaagaaaac taaagtcaac 780

ttgggtacat ggaatgtaag aggcacatac gaaacaggaa aactcaaaga attaattaga 840

gaaattaaaa gatataatgt agaaataata gctttacaag agacaaaaca attggaaaca 900

gaaatagtag aaatagaaga cgtggtattt tttaaaagcg ggggagaaac cagaaggtta 960

ggaacaggtt ttataataca gcgaaaatgg aaggaaagag taatggcatt ccaaccaata 1020

tcagatagat tatgcacaat aagattaaaa ggggacaaac gaaacataag tataataaat 1080

gtacacgcac cgattgaaga tgccacagaa gaagaaaaag atgaattcta cgagcaattg 1140

gaaagagaat atgataaatt accagggttt gacatcaaaa taataatggg agactgcaac 1200

gccaaagtcg gaagagagaa tatatacgaa cacataatag ggaaatatag taagcatgag 1260

gtgtcgaacg ataatggcca aagaataata ggctttgcaa cagaaaggca aatggtgata 1320

cgaagcacac aattacgccg aaaagatata tataaaggaa cgtggatttc ccctgataag 1380

aagacagtaa atcagataga ccatatttta atagagaaga agcatgcaaa caatgtaata 1440

aatgtaaaga gcatgagagg agcggactgt aactctgacc actacctggt gagagcagaa 1500

tataaaatcg agcataacat aaagcgaaac aataaaacaa cagaaaaaat tggaaaacaa 1560

tttaacatag gactactaaa agataacaac acacagaaga agtatgaaca gggaataacc 1620

aacagactaa tcagggaaca agaaacgtca agcccagaca aacaatggca aaatatcaaa 1680

gaagcaatct tgaacacaag taaagcaatc ctagcgaaaa ccaaaagaaa acacaaaaca 1740

aaagattggt atgatgaaga atgtcaagaa gtagcagaag caataagaaa ggtaagactc 1800

aaaaatctca caaatgacga agaaagcact agagaagaat acagagaatt gagaaaaatc 1860

atgaaaagaa aatgtagaag caaaaagaga aaatacaacg agaacaaact gaaagaaata 1920

gaagaaaaat ttcaaaaaaa agaaataaga tcattctacc aggaagcaaa aaaaatggaa 1980

agaggatatc aaaaaaacaa cccatatatg agagatgata aagggatgtt gctaaatgag 2040

cctgaaaaaa taatggaaaa ctggcaaaac tatttcacag aactgctaaa taagagcgaa 2100

gtacaaaagg aaacaaacca tgaaattgaa gatgatcaga tagcaataga aatacccaca 2160

gaacaagaaa taaagaacga aataaagagc ttgaaaaata acaaaagccc aggaataaca 2220

gaaatatcgg ccgaaatgat aaaatcagga gggaaaagac tacagaaaga gatatatgac 2280

ctgataaaaa gaatatggga agtagaaaaa atgccagaag agtggaccat agcaagaata 2340

tgccctatac ataaaaaagg tgatagaatg ctatgcgaaa actacagagg catcgcacta 2400

ttagaaatag tttacaaaat cttggcacaa agtataagta aaaggctaag tcattattcg 2460

gaaaaaatac tgggggaata ccaggcaggt tttagaaaca acagatcaac gactgaccaa 2520

atatttgcct taaaagaaat acagactacc tgttacgaac ataaaacagt actatatgct 2580

ttgttcatag actttaagca ggcttacgac acagtaaata gacagcagat gtacgaactg 2640

atgaaagaat tggggatacc aagtaaaatt gtcaggatga taaagatgac gatggaaaac 2700

acaacaaacg aaatagcttg gaaagggtat acatccaaaa agtttgaaac caaggaagga 2760

ttacgacaag gagacccact atcaacaatg gcattcaatc taacattgga aggaataatc 2820

aggaaaagca gaataaacat gcaagaaacg atatttaaaa acggccacca atgcatagca 2880

tttgcagatg atctgacgct attagcaaca agcaaaaaag aactacaaaa gttaatgaaa 2940

aacataataa cagaagccaa aaaattcgga cttaaaataa atgaagaaaa gacaaagtat 3000

atgataatgg gagagatcca aaaagaaaaa gagaataaca tcatagtaca aatagatgga 3060

gaaaaaacat actcgtttaa aagagccaaa gaaattattt acctgggagc caaaatagat 3120

gaaaatggtc atgaagaagg ggagataaag gcaagaatag ctaaaggaaa taaaaaatat 3180

ggagcattac gcacattatt gaaatccaaa tacgtatcaa gaaaaacaaa aataagaatt 3240

tataagactg ttatcagacc tacagtaaca tacgcatgcg aaacatgggt gctcaaaaag 3300

tcagaaacag accttttaga aagatgggag agaaaaatgc aaagagcaat atatggaggt 3360

gtgaaaatag aaggtcagtg gagaagaaga accaataaag aattggaaga actatatcaa 3420

gagccaacga ttacgacgac gatcaaagca cagagaatac gatacttggg gcacatcgag 3480

agaatgggaa gtaaacgaat gccaaaaatg gtactctcac gaagaccaat acaaaagagg 3540

aggaaaggca ggccaaggaa aagatggaag gacagtgtat atgaagactt gaagaaaagt 3600

aacattgaga gatggaaaga actagcattg gacagaagga gatggagaga ggtggtgaag 3660

gagtgtataa aaaaactgaa gtgtaattaa ataaaattta taagttatgt aagttatatt 3720

aagttataaa caatataacc caaggaataa aaaacctaaa cagcttaaca aaaaaaaaac 3780

aaacactatt gccctgaaca aaaaaagttt tat 3813

<210> 341
<211> 656
<212> DNA
<213> Diabrotica virgifera

<220>
<221>  misc_feature
<222>  (1)..(656)
<223>  RNAi target sequence

<400>  341
tgcaagtaat catggcttca gagagatata gcttttcttt aactactttc agcccctctg      60

gaaagttggt tcagattgaa tatgcattag ccgctgtagc ggcgggtgct ccatcagtag     120

gaattaaagc atcaaatggt gtggttattg caacagagaa caagcacaaa tcaattctgt     180

atgatgaaca cagtgtccat aaagtagaaa tgatcacaaa acacattggt atggtttatt     240

ctggtatggg tcctgactat cgccttcttg tgagacaagc acgcaagatg gctcagcagt     300

attacctagt ataccatgaa cccattccta cagtacagtt ggtgcaaaga gtagcgactg     360

taatgcaaga atacacccaa tcaggaggag ttagaccatt tggagtgtcg ctactgattt     420

gtggttggga taacaacaga ccttacttgt tccaatgcga tccttctgga gcttactttg     480

cttggaaagc tactgcaatg ggaaagaatt atgtcaatgg aaaaacgttc ttagaaaaaa     540

ggtacagtga agagctagaa ctcgacgatg ctgtgcacac tgctatcctt accctgaaag     600

aaagttttga aggccagatg acagctgaca acatagaagt tggtatttgc gatgct         656


<210>  342
<211>  22
<212>  DNA
<213>  Artificial Sequence

<220>
<223>  primer


<220>
<221>  misc_feature
<222>  (1)..(22)
<223>  forward primer

<400>  342
tgcaagtaat catggcttca ga                                               22


<210>  343
<211>  22
<212>  DNA
<213>  Artificial Sequence

<220>
<223>  primer


<220>
<221>  misc_feature
<222>  (1)..(22)
<223>  reverse primer

<400>  343

agcatcgcaa ataccaactt ct                                              22

<210>  344
<211>  857
<212>  DNA
<213>  Diabrotica virgifera

<220>
<221>  misc_feature
<222>  (1)..(857)
<223>  WCRW transcript ta03948.001_diavv

<400>  344
ggtcaaacat caaatccaca agtcatcaac acgaagagga aagttgaaaa ttgtgtttgt      60

aattgcaagt aatcatggct tcagagagat atagcttttc tttaactact ttcagcccct     120

ctggaaagtt ggttcagatt gaatatgcat tagccgctgt agcggcgggt gctccatcag     180

taggaattaa agcatcaaat ggtgtggtca ttgcaacaga gaacaagcac aaatcaattc     240

tatatgatga acacagtgtc cataaagtag aaatgatcac aaaacacatt ggtatggttt     300

attctggtat gggccctgac tatcgccttc tcgtgagaca ggcacgcaag atggctcagc     360

agtattacct agtataccat gaacccattc ctacagtaca gttggtgcaa agagtagcga     420

ctgtaatgca agaatacacc caatcaggag gagttagacc atttggagtg tcactactga     480

tttgtggttg ggataacaac agaccttact tgttccaatg cgatccttct ggagcttact     540

ttgcttggaa agctactgca atgggaaaga attatgtcaa tggaaaaacg ttcttagaaa     600

aaaggtacag tgaagagcta gaactcgacg atgctgtgca cactgctatc cttaccctga     660

aagaaagttt tgaaggccag atgacagctg acaacataga agttggtatt tgcgatgcta     720

atggattcag aaggtttgat ccatctactg ttaaggattt cttagctaat attccttaaa     780

tttttttata gtttcgattt tttaatatta cttggattat taaatttact ataagtatca     840

aaaaaaaaaa aaaaaaa                                                    857

<210>  345
<211>  512
<212>  DNA
<213>  Diabrotica virgifera

<220>
<221>  misc_feature
<222>  (1)..(512)
<223>  RNAi target sequence

<400>  345
acattcttaa acccggagac aaagtaggag cttccgaagc cacattactc aacatgttaa      60

acatttctcc cttctcatat ggtcttcaag ttgagcaagt ctatgactca ggcactgtat     120

ttgctccagc tattttggac atcaaaccag aagatttgag ggagaaattc ttggctggag     180

tagccaattt ggctgctgtt tgtttgtcca ttggacaccc aacagttgct tctgctccac 240

acagcattgc caatggattc aagaacttgt tggctattgc tgctgttact gatgttgact 300

tcaaggaagc caagaccatc aaagaataca tcaaggatcc cagcaaattt gctgctgctg 360

ctcctgtagc tgcagccgcc gctgcaccag ctgccgagtc taagaaagaa gagaagaagg 420

aggaatctga aagtgaagat gacgatatgg gcttgggtct ctttgactag aaagttttat 480

atttttgata cgttatttgc tgttttgtcg ag 512

<210> 346
<211> 22
<212> DNA
<213> Artificial Sequence

<220>
<223> primer

<220>
<221> misc_feature
<222> (1)..(22)
<223> forward primer

<400> 346
acattcttaa acccggagac aa 22

<210> 347
<211> 22
<212> DNA
<213> Artificial Sequence

<220>
<223> primer

<220>
<221> misc_feature
<222> (1)..(22)
<223> reverse primer

<400> 347
ctcgacaaaa cagcaaataa cg 22

<210> 348
<211> 1042
<212> DNA
<213> Diabrotica virgifera

<220>
<221> misc_feature
<222> (1)..(1042)
<223> WCRW transcript ta20424.001_diavv

<220>
<221> misc_feature

<222>  (427)..(427)
<223>  n can be any nucleotide

<400>  348
atacgagtcc ctaaccactt caaagatggg tagggaggac aaggctactt ggaagtcaaa      60

atacttcacc aaacttatcc aattgctaga agactaccca aagtgtttca ttgtgggcgc     120

tgacaatgtc ggctcaaaac aaatgcagca aattcgtatt tcattgcgag gaaatgctgt     180

cgttcttatg ggaaaaaata ccatgatgcg taaagctatc aagggccata ttgaacacaa     240

ccctgctctg gaaaagatct tgccccacat caagggtaat gttggttttg ttttcacccg     300

aggtgacttg gttgaaatca gagacaaact attggaaaac aaagtcaaag cccctgctag     360

agctggtgcc attgctccat tatctgtcat cattcctgcc caaaacactg gattgttccc     420

tgagaanact tctttcttcc aggctttgag tagccccacc aagatttcca agggtactat     480

tgaaatcatc aatgatgtcc acattcttaa acccggagac aaagtaggag cttccgaagc     540

cacattactc aacatgttaa acatttctcc cttctcatat ggtcttcaag ttgagcaagt     600

ctatgactca agcactgtat ttgctccagc tattttggac atcaaaccag aagatttgag     660

ggagaaattc ttggctggag tagccaattt ggctgctggt tgtttgtcca ttggacaccc     720

aacagttgct tctgctccac acagcattgc caatggattc aagaacttgg tggctattgc     780

tgctggtact gatgttgact tcaaggaagc caagaccatc aaagaataca tcaaggatcc     840

cagcaaattt gctgctgctg ctcctgtagc tgcagccgcc gctgcaccag ctgccgagtc     900

taagaaagaa gagaagaagg aggaatctga aagtgaagat gacgatatgg gcttgggtct     960

ctttgactag aaagttttat atttttgata cgttatttgc tgttttgtcg agtcagactg    1020

tcaaataaat gaaacaggtc gc                                             1042


<210>  349
<211>  700
<212>  DNA
<213>  Diabrotica virgifera


<220>
<221>  misc_feature
<222>  (1)..(700)
<223>  RNAi target sequence

<220>
<221>  misc_feature
<222>  (471)..(471)
<223>  n can be any nucleotide

<400>  349
ggggagtaca atgtcggcta ccagactgga gaacggtgcg ggtgattata ttcaaacaga      60

attgatatcg ggcatcgatt tcgttaataa aaacaattct aaaggacatt gcgccaatgg     120

agaaactggg ttaaatgaac acactaaata caagaatgga tttaaaaagt cggtcatttc     180

caatggacat gtcacatcat cagacgaaga aacgacaaaa aataggacaa aaaatggtta 240

caaaacagac acatcaacag acgaaccaaa aatccgacaa atcaaagaat ccttcgaacc 300

gcctacgttg atcaccaaag cactcaccca catcagtttc tacatcctca tgttcttagg 360

atacctcagc caggcgctgt ttccacctaa gttggtcaaa gaaaaaaaat cgggaaggct 420

atcgaccgtt gtctgatcgt tttgccgctt tctattccca gtacgtctac nggcgtatca 480

aggattgttg gaacttaccc atttgcagcg taccagggga tgaagtcgta cttaaagata 540

ggattacgag agatcattgc tggacctttg aatttacggg gtccaaaacg aaatgcataa 600

atttggcttc ctacaactac ttggggttcg cagaagccac aggtccctgc gcagaacatg 660

cgattcggac gatttacacg gatggcatta gtaccggaag 700

<210> 350
<211> 18
<212> DNA
<213> Artificial Sequence

<220>
<223> primer

<220>
<221> misc_feature
<222> (1)..(18)
<223> forward primer

<400> 350
gagtacaatg tcggctac 18

<210> 351
<211> 21
<212> DNA
<213> Artificial Sequence

<220>
<223> primer

<220>
<221> misc_feature
<222> (1)..(21)
<223> reverse primer

<400> 351
cttccggtac taatgccatc c 21

<210> 352
<211> 2113
<212> DNA
<213> Diabrotica virgifera

<220>

<221>  misc_feature
<222>  (1)..(2113)
<223>  WCRW transcript ta00669.001_diavv

<400>  352
ggggacagac accagccgca gacctccagg catgcgctta ttcagcaagg tggtgtgact      60

gctgtgagat ggctgtgtcg gctgtacgaa attacaggtt agattaggtt tgggttggga     120

gttttctttg atttgttcgt gtgttgtgcc taaaaacttc agtacaatgt cggctaccag     180

actggagaac ggtgcgggtg attatattca aacagaattg atatcgggca tcgatttcgt     240

taataaaaac aattctaaag gacattgcgc caatggagaa actgggttaa atgaacacac     300

taaatacaag aatggattta aaaagtcggt catttccaat ggacatgtca catcatcaga     360

cgaagaaacg acaaaaaata ggacaaaaaa tggttacaaa acagacacat caacagacga     420

accaaaaatc cgacaaatca aagaatcctt cgaaccgcct acgttgatca ccaaagcact     480

cacccacatc agtttctaca tcctcatgtt cttaggatac ctcagccagg cgctgtttcc     540

acctaagttg gtcaaagaaa aaaatcggga aggctatcga ccgttgtctg atcgttttgc     600

cgctttctat tcccagtacg tctacaggcg tatcaaggat tgttggaact tacccatttg     660

cagcgtacca ggggatgaag tcgtacttaa agataggatt acgagagatc attgctggac     720

ctttgaattt acggggtcca aaacgaaatg cataaatttg gcttcctaca actacttggg     780

gttcgcagaa gccacaggtc cctgcgcaga acatgcgatt cggacgattt acacggatgg     840

cattagtacc ggaagtacca gacaacagta cggaacgtgt gcactccatg acgaactgga     900

gcagctgata gccgaattcg taggtgccga agattccatc acctttggaa tgggtttcgc     960

gacgaactca ctcaacattc caacactcgc atcgcctggt tgtctgatat tgagtgacga    1020

aaagaatcat gccagcttga tattgggtat caaacttaat catcccaccg ttagagtatt    1080

taaacacaat aatgttaagc atctagaaaa actcctccaa gaagccatct actacggcca    1140

acccggccaa cccaacaacg agtacaaacc ttggaagaaa atcattattt ttatcgaagg    1200

cgtgtatagt atggaaggaa ccataggccg ccttccggaa attatagctc ttaagaaaaa    1260

gtacaaagct tatttatatt tggacgaggc ccacagcata ggggccatgg gcaagcacgg    1320

caaaggtgtt gtagattatt tcaactgtga tcccaaagat atcgacattt tgatgggtac    1380

gtttacgaag agttttggag gcgcgggagg atacattgca ggttcaaagg aattcatatc    1440

tttcattcgc gaacatagtc acgcgtctgg tcacgcttgg gcgatgtcgc cgccagtcgc    1500

agcacagatc atatcagttc taaagataat catgggcaaa gatggtacaa acgaaggaca    1560

aaagagaatc gaaaggttag ctaggaattc cagatacgtc aggttgcgtc tcgaacagat    1620

gggtttgatc gtacacggta accaagattc tccagtagtt cccattttag tttatcgtta    1680

ctcaaacgta gcggtagtgg taagaacttt aattaaagaa aaaatagcaa cagtaggcgt    1740

aggatatcct gcaacgccgc ttgcggaatg cagaataaga atctgcatat cggcgggcca 1800

cacgaaagag cagctggatt atgccttatc agttatagaa aaagtagcag atgaaatagg 1860

tattaagtat tctagaaaac cgagagatcc aaaaccgatt gattacaata aaatcaaaat 1920

atatcaggat ttgttttaga gagagacttt tagatgaagc ttcaagagaa caccagcctg 1980

cgataggagg ttttgttgtt tttataaatt ttataaatgc atttaatatt tgatttcaaa 2040

agctatttat taagaatcgt ttttggctct gttttatttt aatactttgc aaaataaaat 2100

ataaaaacag aaa 2113

<210> 353
<211> 440
<212> DNA
<213> Diabrotica virgifera

<220>
<221> misc_feature
<222> (1)..(440)
<223> RNAi target sequence

<400> 353
gggtaatgaa agattccgtt gtccagaagc cctcttccaa ccatcctttt tgggaatgga 60

agccaatggt atacatgaga ctacctacaa ttccatcatg aagtgtgatg ttgatatccg 120

taaagactta tatgccaata ctgtactctc tggaggtacc accatgtacc ctggtattgc 180

tgacagaatg cagaaagaaa taacctcctt ggcacccagc acaatgaaaa tcaaaattat 240

tgctccacca gaaaggaaat actctgtttg gattggtggt tcaattttag cctctctttc 300

tactttccaa cagatgtgga tttccaaaca ggaatatgat gaatctggac catccattgt 360

tcacaggaag tgcttttaat ttttatctat gtttaagtat attctttata tatcttgttt 420

tttatatccc ccatcttggg 440

<210> 354
<211> 20
<212> DNA
<213> Artificial Sequence

<220>
<223> primer

<220>
<221> misc_feature
<222> (1)..(20)
<223> forward primer

<400> 354
gtaatgaaag attccgttgt 20

<210> 355

<211>  21
<212>  DNA
<213>  Artificial Sequence

<220>
<223>  primer


<220>
<221>  misc_feature
<222>  (1)..(21)
<223>  reverse primer

<400>  355
cccaagatgg gggatataaa a                                                21


<210>  356
<211>  1465
<212>  DNA
<213>  Diabrotica virgifera


<220>
<221>  misc_feature
<222>  (1)..(1465)
<223>  WCRW transcript ta15851.001_diavv

<400>  356
ggagggcatt ccagtcttgt gatatttagt gcttacttgg tacagcagtt tcagtgctgt      60

gctttagaat aatttatttt ttaacattta tatagaaatc aaatactaac caatcaacat     120

gtgtgaagaa gaagttgccg ctttagtcgt agacaatgga tccggtatgt gcaaagctgg     180

ttttgctggg gatgatgcac ctcgtgctgt attcccttca attgttggac gcccaagaca     240

tcagggtgtg atggtaggaa tgggacaaaa agattcctat gtaggtgatg aagctcaaag     300

taaaagaggt atccttacct taaaataccc catcgagcac ggaatagtca caaactggga     360

tgatatggag aaaatttggc atcatacatt ctacaatgaa ctcagagtag ccccagaaga     420

acaccctgtt ctgttgacag aagctcctct caaccccaag gccaacaggg aaaagatgac     480

acaaataatg tttgaaactt tcaacacccc agccatgtat gttgccatcc aggctgtact     540

ctccttgtat gcatctggtc gtactactgg tatcgtattg gattctggtg atggtgtatc     600

ccacactgtc ccaatctatg aaggttatgc acttccccat gcaatccttc gtttggactt     660

ggctggcaga gatttaactg attacctcat gaaaatcttg actgaacgtg gctactcttt     720

caccaccaca gcagaaagag aaattgttag ggatattaaa gaaaaactct gctatgtagc     780

tttggacttc gaacaagaaa tggcaacggc tgctagttcc agttcccttg aaaagagtta     840

cgaacttcct gatggacaag tcatcaccat tggtaatgaa agattccgtt gtccagaagc     900

cctcttccaa ccatcctttt tgggaatgga agccaatggt atacatgaga ctacctacaa     960

ttccatcatg aagtgtgatg ttgatatccg taaagactta tatgccaata ctgtactctc    1020

tggaggtacc accatgtacc ctggtattgc tgacagaatg cagaaagaaa taacctcctt 1080

ggcacccagc acaatgaaaa tcaaaattat tgctccacca gaaaggaaat actctgtttg 1140

gattggtggt tcaattttag cttctctttc tactttccaa cagatgtgga tttccaaaca 1200

ggaatatgat gaatctggac catccattgt tcacaggaag tgcttttaat ttttatctat 1260

gtttaagtat attctttata tatcttgttt tttatatccc ccatcttggg gcaagaactg 1320

cctatgttat atgagtgatc ttgtgattta ttacacgttc agcataagac ttaatgagct 1380

ttaattttaa gaagtgtaag ttgaatactc tgtttcatat ttattttacc tttgtaatat 1440

aataaacaaa aaaaaaaaaa aaaaa 1465

<210> 357
<211> 600
<212> DNA
<213> Diabrotica virgifera

<220>
<221> misc_feature
<222> (1)..(600)
<223> RNAi target sequence

<400> 357
gttcgctagt tccgtagtgt gttgtgcaca tttaacatcg ttctggcctg tagatactcc 60

taataactta aaaaatggca gatgacaaac accccgtcta tggacccttc tttggagtca 120

tgggagccgc ggctgctatc attttcagtt cgctgggcgc cgcctatggt acggcaaaat 180

caggaaccgg tattgccgct atgtcggtca tgaggccgga acttatcatg aaatgtatta 240

tcccagttgt catggcgggt atcattgcca tctacggtct ggtcatagct gtactgatct 300

ctggtaacat ccaagacagc tctacatatt cattgtacag tggttttgtc catttgggtg 360

ctggtctttc agtaggattt tcaggattgg cggcaggttt cgctattggt atcgtaggtg 420

acgctggtgt aagaggtacc gcacaacagc caagactctt cgtaggaatg atcttgatcc 480

tcattttcgc cgaagtatta ggtctttatg gtctcatcgt cgccatttac ctctatacta 540

gagcttaagc gcccagctcc cctaaatttt catctcctaa acacagcgta ccaaaagttg 600

<210> 358
<211> 23
<212> DNA
<213> Artificial Sequence

<220>
<223> primer

<220>
<221> misc_feature
<222> (1)..(23)
<223> forward primer

<400>  358
gttcgctagt tccgtagtgt gtt                                              23


<210>  359
<211>  22
<212>  DNA
<213>  Artificial Sequence

<220>
<223>  primer


<220>
<221>  misc_feature
<222>  (1)..(22)
<223>  reverse primer

<400>  359
caacttttgg tacgctgtgt tt                                               22


<210>  360
<211>  2938
<212>  DNA
<213>  Diabrotica virgifera


<220>
<221>  misc_feature
<222>  (1)..(2938)
<223>  WCRW transcript ta15854.001_diavv

<400>  360
ggacctgaaa ttggcggatc gtcatatggg ggattcttgc ggggggcagt cgtgtaagcc      60

atgggggtca gtgggttcgc tagttccgta gtgtgttgtg cacatttaac atcgttctgg     120

cctgtaaata ctcctaataa cttaaaaaat ggcagatgac aaacaccccg tctatggacc     180

cttctttgga gtcatgggag ccgcggctgc tatcattttc agttcgctgg gcgccgccta     240

tggtacggca aaatcaggaa ccggtattgc cgctatgtcg gtcatgaggc cggaacttat     300

catgaaatgt attatcccag ttgtcatggc gggtatcatt gccatctacg gtctggtcat     360

agctgtactg atctctggta acatccaaga cagctctaca tattcattgt acagtggttt     420

tgtccatttg ggtgctggtc tttcagtagg attttcagga ttggcggcag gtttcgctat     480

tggtatcgta ggtgacgctg gtgtaagagg taccgcacaa cagccaagac tcttcgtagg     540

aatgatcttg atcctcattt tcgccgaagt attaggtctt tatggtctca tcgtcgccat     600

ttacctctat actagagctt aagcgcccag ctcccctaaa ttttcatctc ctaaacacag     660

cgtaccaaaa gttgcgcatc tcgccccgat cattttgttt ttaatgtcta catgtaaaaa     720

taaaaactgt ataaatggta gcaggcgatg aaagtgactg aggcgggcaa tgtaaataat     780

ttagttatga tggggcccgt atttttcgcg tacggggcaa aacaaattaa taattttagt     840

aaaaaagact gggcctcttc atcgctgctg tgtccacaca cacgactaat aattccttat 900

ttccatattc atggaaccgg tcgagttttc ttgacaggtt tttgtattaa aacgaatttt 960

cttcctaggc aaaataaagg tcttagttac taataggttg ttcaaatgaa aatgtacgaa 1020

acgacactgt gggtataaat gaagtcttta ttcaaagtat tcaccattta cctgagcaca 1080

acgatgccgt tgattagccg aaggattcat tgatcccaga actcctgtga ccatgaacag 1140

tcttccccgt atacatccgg cgttgaatag caactggttt aactaacacc ttccgccgtc 1200

agaaacctga taacgcctca cgaattttgc aacatgaacc ccatgatgtg cccacacgaa 1260

ctgtcgcgtc aattgaacgg ctctcttata agagcctcta ttctctcctg cgcatgcggg 1320

cgcccgggaa tgccccgatc gaatccgaag gctacatttg cctccctgga tgtctcacta 1380

cgttatccca taacggcata ggcaattatg tttgcggtta agttgttacg acgtttcgtg 1440

ccttttcatt tgatcagtcg cagtataata tacaagagaa gagggaaaac aaattttgat 1500

ttattttgcc tcgtacgtta caatacctgt gctatcattg taaataattt taaacatact 1560

tagaagcctc tatttcattt tcatggcggg tcaccactat tattaaatta attgtatagt 1620

tagtagaatt taggttaccg tttttctttt aatataaaag ctagactcta ttgagcctag 1680

gaagtatttg aataaagtta aaaaagtagc acaatttatt tttaatttca caaaaatact 1740

gtacatttgt tttatcatta gtgtgttata cttgatatat cattttcatg gccttacgaa 1800

ggcttggtct ctttatacaa caaatggttc gggaatgtta tttttgacaa tttttttgaa 1860

tagcaagttt taagccagta aaactgtggt attttttttc aatgcatcat cttttttaat 1920

aaaaaaaact ttggttaatg gggcattaca tcgcaaaacc gtaatttcta aataatatcg 1980

tcttaattta tttatctaac aatgtactgt ctaaataaca ttcgtaaacc ctgtacattt 2040

ttttaatagt caatttttat ttgttcccat cctcatcagt tttgcttacc tttttttctc 2100

tctcaaaaat atgatttcaa aataatctat gttccattac cttccaaaaa taatataaaa 2160

cggttttatt atatcagcat tgttgtaaat atgacaactt atgtgtatcg atggtttttt 2220

tgatgtccat agctgtacgt gttagcctaa agtgtagtca tagctttggc aaaaaacatc 2280

cccgttttca acactgacga aacttctatt taatagacag acggtgatgc tttttgactt 2340

tagcagataa gaatgttata cagggttaac aaaaatgaga atactacgtc tgatatatcc 2400

tgtaacacac aaaatacttt aaagctcagt tacacgatgc taataacttg ttttactgga 2460

atataaaaca ccaatttaca caaggagagt actaaaagtc agtacatact cggttttagt 2520

gactcacaag tgttactagc atggtgtaac tgagccttta tcctggcatt ccaattactg 2580

gacaatgcta tcatataaat accatgccat ctaaataggt aatctacttg ttagtcctaa 2640

gagtttatca ctatttaagt aaacgatgaa ttgaattggc tgttttgtgt tacaaggcta 2700

gtaagttttc tcttttcaag tcaaccctgt agtatctcct ttttatttta gtcatgaatc 2760

atcatctcag cacgaagctt gttgggcaag tgtctgccgg ttttcggtgg attcggtcca 2820

tcgaaatagg cagacattgt gtcctattgt tgtagagtat tatattataa tgtagattgg 2880

ttctgttgta agaagggaac agcaaaatta tgttgaaaat aaataatttg tacattcc 2938

<210> 361
<211> 525
<212> DNA
<213> Diabrotica virgifera

<220>
<221> misc_feature
<222> (1)..(525)
<223> RNAi target sequence

<400> 361
gggatctcca tcttcctttt tcatttagtg taaagcatgc ctttccaaag gtttgtagaa 60

accggccgcg tggccgttgt atcggaaggc ccacataagg gcaaacttgt tagtattgta 120

gacgtaatcg accagacaag ggttttagtt gatggcccca atagcaatgt acccagagga 180

cagcttaggt taaatgaatt gcatttaaca aagttccgca ttaggtatcc attttcagct 240

ccaactcgtg ttgtaagaaa agcatggact gatgctaaga ttgatgaaaa atggaatgaa 300

tccgtctggg ccaaaaaagt agcagccaag gaaaagagat cacaacttac agatcttgac 360

cggttcaaac ttcgtagggc tcgttcacga agaaaccaca tccgtacagc tactttcctt 420

tctttaagga aggccgcatc aagaaacggt ttcctctatg gtaaaaagaa gatggctaaa 480

ggaggtgaca aacctaaacc aagcaagaag gcgaagaaag aaaag 525

<210> 362
<211> 22
<212> DNA
<213> Artificial Sequence

<220>
<223> primer

<220>
<221> misc_feature
<222> (1)..(22)
<223> forward primer

<400> 362
gggatctcca tcttcctttt tc 22

<210> 363
<211> 22
<212> DNA
<213> Artificial Sequence

<220>

<223>  primer

<220>
<221>  misc_feature
<222>  (1)..(22)
<223>  reverse primer

<400>  363
cttttctttc ttcgccttct tg                                                22

<210>  364
<211>  689
<212>  DNA
<213>  Diabrotica virgifera

<220>
<221>  misc_feature
<222>  (1)..(689)
<223>  WCRW transcript ta01695.001_diavv

<400>  364
aagaatacga agagctacta acattactta aataaactat agtttaagat tttaatctgt      60

caacaactgt gatagccagg ttcctttttc atttagtgta aagcatgcct ttccaaaggt     120

ttgtagaaac cggccgcgtg gccgttgtat cggaaggccc acataagggc aaacttgtta     180

gtattgtaga cgtaatcgac caaacaaggg ttttggttga tggccccaac agcaatgtac     240

ccagaggaca gcttaggtta aacgaattgc atttaacaaa gttccgcatt aggtatccat     300

tttcagctcc aactcgtgtt gtaagaaaag catggactga tgctaagatt gatgaaaaat     360

ggaatgaatc tgtctgggcc aaaaaagtag cagccaagga aaagagatca caactcacag     420

atcttgaccg gttcaaactt cgtagggctc gttcacgaag aaaccacatc cgtacagcaa     480

ctttccttgc tttaaggaag gccgcatcaa gaaacggttt cctctatggt aaaaagaaga     540

tggctaaagg aggtgacaaa cctaaaccaa gcaagaaggc gaagaaagaa aagcctgcat     600

ccaagaagaa ataattttta gattaaatga caatatattt gtttaaaaaa caaaaaaaaa     660

aaaaaaaaat agaaaccatc attaactat                                       689

<210>  365
<211>  551
<212>  DNA
<213>  Diabrotica virgifera

<220>
<221>  misc_feature
<222>  (1)..(551)
<223>  RNAi target sequence

<400>  365
ggggatcttt agtcagtgct ttagaggcca cgaagcaatt tctaggatag acgaacgacg      60

ctcgcgacga cgcgctgcga cgcacacgac aaaaaaatac acaatacgac accacgcgcc 120

attttgccat tttctgtgtg tgcagtgaag tgaagtgcta ctaaaatctt caaaattcaa 180

cccttctaag aaggccgatt tcatgtgttc tatatcgaag taaaagagca atagcatggg 240

agtggcagag gatttcgctc ccagcttcac gcaaaagcct caattgaggc aggaggacga 300

tggaaacaaa ctcattttcg aatgccagtt actggctgct ccgaaaccgg aaatcgaatg 360

gtttcgaagc gatataccac tttcagaaga cagtagaact aattttaaaa ttcaatccat 420

aggcaccaac aaatttttag tagtactcga attagatgat gttattgaaa ccgacgctgg 480

cctttacaag gtcaaagcga aaaataccat gggggaaata gcagcctcca tcaatctcaa 540

ctacagcccc a 551

<210> 366
<211> 19
<212> DNA
<213> Artificial Sequence

<220>
<223> primer

<220>
<221> misc_feature
<222> (1)..(19)
<223> forward primer

<400> 366
gatctttagt cagtgcttt 19

<210> 367
<211> 21
<212> DNA
<213> Artificial Sequence

<220>
<223> primer

<220>
<221> misc_feature
<222> (1)..(21)
<223> reverse primer

<400> 367
tggggctgta gttgagattg a 21

<210> 368
<211> 6974
<212> DNA
<213> Diabrotica virgifera

<220>
<221> misc_feature

<222>  (1)..(6974)
<223>  WCRW transcript ta35139.001_diavv

<400>  368
acaatacgac accacgcgcc attttgccat tttctgtgtg tgcagtgaag tgaagtgcta      60

ctaaaatctt caaaattcaa cccttctaag aaggccgatt tcatgtgttc tatatcgaag     120

taaaagagca atagcatggg agtggcagag gatttcgctc ccagcttcac gcaaaagcct     180

caattgaggc aggaggacga tggaaacaaa ctcattttcg aatgccagtt actggctgct     240

ccgaaaccgg aaatcgaatg gtttcgaagc gatataccac tttcagaaga cagtagaact     300

aattttaaaa ttcaatccat aggcaccaac aaatttttag tagtactcga attagatgat     360

gttattgaaa ccgacgctgg cctttacaag gtcaaagcga aaaataccat gggggaaata     420

gcagcctcca tcaatctcaa cttcagcccc atggacgaac caaaagaaaa acaaatagac     480

ggcctagcac ccacttttgc gaagaaacca gctattcgcc aagaagatga tggcaaaaaa     540

ttattattcg aatgtaggat acaggccgat ccccgtccaa ccgtcagttg gtcccacaat     600

ggcaacgctg ttagcgaagg tccacgtcac aagttgagga tagataaaga tggccattca     660

tattttgcga cccttgaaat aatcgatgtc acggtagagg atgctggcaa atacaaggtg     720

accgcaaaaa atgacttggg agaaagtaac gccacaatca gccttaactt tgacagtgga     780

gatagcgctg atggctttgc gccttctttc cttgagaaac ccaaaatcat acctaatgag     840

agtggcactc ttattactat gaaatgtaaa tgcaaagcta aacctaaacc tgacgtcacg     900

tggttccgcg gaaccacagc cgtcaaggaa tcttccaaaa ttaaaatcca gatcgttgat     960

ctcgaagaag acaaattcga actgtcctta gaaatcaagg atccatcggc agctgatggg    1020

ggtacttaca gatgccatgt gaagaacgaa tacggagaaa gtaatgcaaa tctgaaccta    1080

aatatcgaag cagaaccaga accagaagga gaaggaccaa cgttcgtcga aaaacccagg    1140

ataacctctc acgatggagg caaactcgtt gtcatggagt gtaaagttcg tgctaatcct    1200

aaacctacta tagtttggta cagagaaagc aaagaagtca cagaatcatc caaaattaag    1260

atcagtatta aacaaacaga agaagatata tattacgtca aattggaact caatgatccg    1320

gggattgatg actctggctt gtacaaatgc aatataagga acacacttgg tgaactcaac    1380

gccaacctca ccttaaacat cgagattatt cctgttatca aagaaaaacc caaagttatt    1440

aaaatcatta agaagaaaac tgttattgtt gaatgtaaag ttctcagcaa gtttgcacct    1500

gattgtacat ggtttaagga aagcgatgcc gttaaagaag attcaagaca tactgttcac    1560

gttgaccaag ttaaagacgg cgaatttact gttaaactcg aaattaatga agttgagaaa    1620

aaagacaaag gtatgtacaa attggttgct aaaaacgaaa agggtgaggc aacttcacaa    1680

gtcgttgaag tcactgagtt acctccagag gagaaaccca aaggagacaa gccgaaactg    1740

accaaactaa ccaatatcgt tactgacgaa ggaaaatcag ttgattttat aacttctctc    1800

aaaatcgaag acaaaacagt caaaatcaca tggtacaaga acaccactgt gataaccgaa 1860

tcttcagaaa tcaaaatctc ttttgatggc actgtgacgc gacttagcat tagtaaatgt 1920

aaagtatcac attccgctac atacaagtgc gttgccaaaa acgaatttgg cgaagacgaa 1980

ataagcgcta cacttaaagt aaacgaagct aaagaggaag atgaagaaga agaagaatcc 2040

gaagaggagg ttatcgaaga aaagaaagag gaaaagaaag aagaaaagaa agaagaaaaa 2100

caggaaaaga aagttgaaga gaaaaaagaa gaaaaaaggt agaaaagaaa gaagagaagg 2160

ttgaaaagaa agtagaaaag aaagaggaaa agaaaacaga agaaaagaaa gcagttaaaa 2220

agatagaaaa gaaagaggaa agaaaagaag aggagaaaat gataattaag aaggacgaag 2280

aaaagaaaaa agacgaaaag attgtggcca aaaaggaaga ggaaaagaaa gatgttgaga 2340

agaaaataag gcgtacatcg gttcaacaga acgaagaagt aagaaccgaa tcacgcagaa 2400

gctcaattat acaaaccgaa gaaaaagtta ttaaagatgg aaaagttagt aggaaatctt 2460

ccatttcaaa agtcaatgag gagagtaaga ctgagtccag gcgcacatca ttagtagatg 2520

acaagaaaaa gataaccaaa gataccaaaa aattggaaac cacaaaagaa gtgtcagaag 2580

aaaagctggg agtagccgaa gttaaaaagc tggaaaaacc taaaagggta atacccccta 2640

aagaagaagc tcaaaagggt tcgttcttga acattcaatt gaaaccagtt caatcggagg 2700

gtaaaaaagc tgaaaaggcg aataaacttg aagttgactt gaagaaagga gcagttgaag 2760

agaaaaagac aacagtcaaa aaatcttctg ccaaaaaaga agattatgat attaatgctt 2820

gggaaaatat ccctgattac gacagaccag tgttagagaa attcgaaaaa catgatgctc 2880

cagaatatgc tggtagagaa aagagtaaat tctctaagga aagaccatct atctcagtcg 2940

aaggaaaaga aaaagctcct tctcagtcag ctatatctga acaacctaga cctagccttc 3000

ctcaaattaa gactccagat gcacccaaaa tcgaagtgag tagggaaaaa actcccgaac 3060

ccagaaagaa atctctcgag cctggctcag gtccaggaag tcgtagagga tctcttattc 3120

ctccagaagc catgggcaga cgtgccagtc ttatcatctc cgatgaggga ggaaaattac 3180

gtcctgggga agtgctagat ccaaagcgtc gtcgtccgag ctccgacgta cgtcgaccaa 3240

gtttagctga attggaggat ctcgtgaaca aaccctcgac tcctttgaaa ccttccggtc 3300

caaagggagc agcacctcaa attgtcgatg tccaagaaaa ttactcctca gttgaagatc 3360

aaactgctta cattactatc caagttgagg gtgatccacc accgacattc aaattctata 3420

agggtatgac tcaacttatt gagggtggta gattcagatt tgtaaccgat ggtgaaacaa 3480

acttgatcac actctgcatt agaaaggtaa agcccaatga tgagggtacc tataaagttg 3540

tcatagccaa cgtccatggt gaagactccg ctgaaatgac cctcttcgta gctggtgctg 3600

gaggtgttga tttccgttct atgctcatga agagaaagta cgccaaatgg gataaggata 3660

aggaagaccc caactgggga gacttaaaag aggttgaaaa accagtgcca gcattaaaaa 3720

aagtcgaaaa gaaacaagaa agtttcctta aaccattggt agatcagttc gctaaagaag 3780

gaaaagataa gaaagttgtg ttcgacgctg gtttctcaaa aaccaactgt aaaccaaaat 3840

ggctgttcag gaaagacgaa cttttcccag gatctaaata caaatttaag aatgaaggtg 3900

acgtgtacca gctgatcatc agcaatccta aagtagagga tactggaaaa tacacaattg 3960

aaatcggagg agtcagctct accggtttcc tgacagtaga tgaaccagat ccatgttaca 4020

cctttaccaa agcactgaag aaaaaatatg atggatacac caaacatgaa gttgaactgg 4080

cttgtactgt aagcagtagt ttggccattg ttggttggta taaaggagag acaaagttgg 4140

aagataatga taaatttagt atctctaaag atattaacgg agtctgtaga ctgactatta 4200

aagatgcgac atttgatgat gctggtgatt acacctgcaa actagaaaaa caaaccgaaa 4260

agacacaaac taaagttaat attgtagact acccatataa attcgtgaaa gtacttaagc 4320

atcaacaact tatagaaaaa gaaaatgtta cattagtatg tgagctcaac gatgctggtg 4380

gtgatgtaca gtggtttaag aacggagaag aaatcaaacc agataaacga gtagttatca 4440

gtaaagatgg tcgcaaacgc aaactggtaa ttaaagacgc caaagttact gatgctggta 4500

tgtactcttg tactaccaat gcagacaaaa ccgaagctga gcttattgtg aactacttga 4560

accgctttaa caaaaaactg aaagacactg ttgctgttga acgagaaaaa ctagttttgg 4620

acattgaact acaagatcaa acagccccgt gcgagtggtt gttcaatggc gaaccaatca 4680

agccctctga gagagtcgag atcaaaaact tgggaggtgg caagcaccaa ctcatattta 4740

atggtttaga tatggcagat gacggtgaaa ttaagtgtgt atcaggaaaa cttgaatctt 4800

ccatgatgct ttccgtcaaa aaaggtgaaa gcaagcctga aattgacttc ccatcagact 4860

tcgctgcacc aattagcaaa cctattgtcc ttgaagtacc atacaaaatt agtggaacca 4920

gacagacacc tttggaagct aaacttatca aagacggaaa ggtactacct ctaaaagacg 4980

tagagacaat aatttcagac gacaaagtca tattcaaaat taaaaaacca tccagagacc 5040

aatctggtcc ataccagatc aaattatcga acgggcaagg tgaggatgtt aaagatgtca 5100

agatcaccat gcaggacgtt cctgctccac cagaagacgt taatgtaaca gatatattcc 5160

aagacaactg taaaatatcc tggaaaccac ccaaagataa tggtggagca cctcttctcc 5220

attatgttgt tgaacgacaa gatctcagtc tcaaagctgg ttgggaaaat gttggtgaag 5280

taaaagccaa tgaaccaact acattcaaag ttgaaaaact aattcccaag aaagaatata 5340

aattcagaat cagagctgtt aaccagctag gtcctagcga acctgctcta ttcggtaaac 5400

cagtattagc taaagatcct tgggatgaag ccagcaagcc taaagatgta gaagtagttg 5460

actgggatgt tgaccacgca gacattactt ggaagacacc tgacagcgat ggtggagctc 5520

ctattactgg ctacgttatt gaattttaaa gaaaaattcg ccaaagattg gaaaaccggt 5580

gttgaagtac caggtgacca actgaaagga actgttccta aactcaagga aaacagtcag 5640

tacgaattta gagtaagggc tattaataag gctggaccag gagaacctag tgatcccacc 5700

aaacctatca tagctaagtg tagattcgta aaaccattta ttattggtaa tgacttaaac 5760

agtattatta tcaagaaggg acaagttgta aagtacgata ttaagtacgg gggtgaacca 5820

gaacccgagg tgcaatggtt cctcgataaa aaagaaatca aacccgactt ggaagaaaga 5880

gttactatcg acaaatatga cagaaacact attattacag tacgcagatg tacaagagct 5940

gacagtggaa aatatcgttt ggtacttacc aacagctctg gtacttgtga aggggtggcg 6000

gaagttattg ttttagataa acctacacca cccaaagcta tgaaagtcga ggaaatccgt 6060

gcagatcacg ttaaagttgc atggcaaaaa ccagaagata tgggtggtac tgacctaact 6120

ggttatatat tagaaaaaat ggatatggac acaggaagat ggattcctgc aggtgaagtt 6180

ggtcctgatg aatcaaactt tacgtttaag aacctgacac ctaagaagaa atacaaattt 6240

agagttcgag ctaaaaataa agaaggagaa tctgaacctt tggaaaccga tgactttatt 6300

ctagccaaga atccttacga tgagcccgga gcacctggac aaccagaaat ctttgactat 6360

gacaacaaga gtgccagcct taaatgggct aaaccagata gcgatggtgg gcgccctatc 6420

acccattaca cagtcgaaat gaaagataag tttgcagctg attggactga ggtagccaag 6480

acgcctgacg ctactcctga agtggtcgta cctggtttaa aagaaaatat ggtatatcaa 6540

ttccgtgtga aagcacacaa taaatcagga cctagtgtac cttccgcacc aacagctaac 6600

cacctctgca aacacaaaaa cttaaaacca agaattgatc gtgaaacatt tagagctgtc 6660

actataaaag ctggccgtac tcataagtgg gcagttgact ttatgggaga gcctccacca 6720

tctgtcaaat gggtatggag agatgacatt ccattatcaa atagtgaacg tatcaagata 6780

gaaaactccg actaccatac ggacttcaca ctcctcaacg ctgttcgtaa agactcagga 6840

aaatacacgc ttactattga aaacgttaac ggaaaggata ctgaaacagt tgaactgact 6900

gtattgggca aaccagcagc ccctgaagga cctttagttg tatctgatgt gactgcagaa 6960

aaagctaaag ttag 6974

<210> 369
<211> 586
<212> DNA
<213> Diabrotica virgifera

<220>
<221> misc_feature
<222> (1)..(586)
<223> RNAi target sequence

<400> 369
gggggagtag tcggtatgta tttgaattta tcttaatttg tttaatatta gtagttaaat 60

attaaactat agttagaagt tgttatatat ttgaccatta gttgactccc caaaatgcag 120

atcttcgtta aaaccttaac gggtaagacc atcacccttg aggtcgagcc ctcagatact 180

atcgaaaatg tgaaagctaa aatccaggat aaagaaggaa ttcccccaga ccagcaacgt 240

ctcatcttcg ctggaaaaca actcgaagat ggtcgtacct tgtctgacta taatattcag 300

aaagaatcaa cccttcactt ggtgttgaga ttgagaggag gtgctaagaa acgtaagaag 360

aagaattact ccacccccaa gaaaatcaag cacaagaaga agaaggttaa gttagctgta 420

ttgaaatttt ataaggttga cgaaaatggt aaaatccacc gattgagacg tgaatgcccc 480

gctgaacaat gtggagctgg tgtcttcatg gcagccatgg aagacaggca ttactgtggc 540

aagtgcggtt acactcttgt cttctccaaa ccaggagatg agaaat 586

<210> 370
<211> 19
<212> DNA
<213> Artificial Sequence

<220>
<223> primer

<220>
<221> misc_feature
<222> (1)..(19)
<223> forward primer

<400> 370
gagtagtcgg tatgtattt 19

<210> 371
<211> 22
<212> DNA
<213> Artificial Sequence

<220>
<223> primer

<220>
<221> misc_feature
<222> (1)..(22)
<223> reverse primer

<400> 371
atttctcatc tcctggtttg ga 22

<210> 372
<211> 683
<212> DNA
<213> Diabrotica virgifera

<220>

<221>  misc_feature
<222>  (1)..(683)
<223>  WCRW transcript ta06472.004_diavv

<400>  372
ggagtagtcg gtatgtattt gaatttatct taatttgttt aatattagta gttaaatagg      60

aaactatagt tagaagttgt tatatatttg actattagtt gactccccaa aatgcagatc     120

ttcgttaaaa ccttaacggg taagaccatc actcttgagg tcgagccctc agatactatc     180

gaaaatgtga aagctaaaat ccaggataaa gaaggaattc ccccagacca gcaacgtctc     240

atcttcgctg gaaaacaact cgaagatggt cgtaccttgt ctgactataa tattcaaaaa     300

gaatcaaccc ttcacttggt gttgagattg agaggaggtg ctaagaaacg taagaagaag     360

aattactcca cccccaagaa aatcaagcac aagaagaaga aggttaagtt agctgtattg     420

aaattttata aggttgacga aaatggtaaa atccaccgat tgagacgtga atgccccgct     480

gaacaatgtg gagctggtgt cttcatggca gccatggaag acaggcatta ctgtggcaag     540

tgcggttaca ctcttgtctt ctccaaacca ggagatgaga aatagatata tgtccttgta     600

tattgtttaa gaaaaaaata gaaaaacctt tgtttatttg aataaaatat tcgaggaaat     660

atatctattt ctcatctcct ggt                                             683


<210>  373
<211>  611
<212>  DNA
<213>  Diabrotica virgifera


<220>
<221>  misc_feature
<222>  (1)..(611)
<223>  RNAi target sequence

<400>  373
ctccgaagac agacagtcga ttttattcag tgattctgat tgttcagttt ttgtagttgt      60

acagttttcg tattaggaaa taaatcaaat atggcatcag gagtaactgt agcagatgcc     120

tgcaaaaagg tccatgaaga aatcaaaagg gacaaaaaac acagatttgc aattttctac     180

atcaaggatg gcaaacagat tgatgttgaa cacataggag aaagaaatgc agagtatgat     240

gatttcctaa ctaaactgca agagggagga gctaatgaat gtcgttatgg acttttcgat     300

tttgaataca tgcaccagtg ccagggaacc tcagagtcaa ccaagaaaca aaagttattc     360

ctgatgtcgt ggtgtcctga aactgcatct gtcaaaaaga agatggtgta tgctagctca     420

ttcgaatatc ttaaaaaagc tttggtaata cagaaatcta tccaagcgac agatttagcg     480

gaagcgagta taacagcggt agaagaaaaa ttacgatcca ctgatcgtca gtaacgcctt     540

ttatttttat tatttttctt tttgtatctc gactgtctac tcgcgcgccg acctgccgag     600

gtgtattatt t                                                          611

<210>  374
<211>  21
<212>  DNA
<213>  Artificial Sequence

<220>
<223>  primer


<220>
<221>  misc_feature
<222>  (1)..(21)
<223>  forward primer

<400>  374
ctccgaagac agacagtcga t                                                21


<210>  375
<211>  22
<212>  DNA
<213>  Artificial Sequence

<220>
<223>  primer


<220>
<221>  misc_feature
<222>  (1)..(22)
<223>  reverse primer

<400>  375
aaataataca cctcggcagg tc                                               22


<210>  376
<211>  1099
<212>  DNA
<213>  Diabrotica virgifera


<220>
<221>  misc_feature
<222>  (1)..(1099)
<223>  WCRW transcript ta00957.001_diavv

<400>  376
gggaaatcgc gagaatacgg gcgaggtttg aagtttttaa ttttttgttc atatcaccac      60

ttaaagctat tgttatcatt ttagcatata taaaacaata gcttctgtgt tggggattaa     120

cagtcgcggt tgagctaaaa aatatatatt tgttaatgtt ataaataata ttcaaaaagt     180

ttgtttaatt caaaactggc gcatttcgta cgcatgaaaa aatagttcga attgacattg     240

gaaacatttt gatagttgtg gtgacgtttg ctgtaaatct atttaatttt tggtgattta     300

ataattgaaa atatttctgg gaattaacgt agtttctaga aaaaatatcg gtgacttatt     360

ttggcgtagt aaaattgact cgcgcaggcg tacccccagc tgccggtagg acaacgtccc     420

caacagatct ccgaagacag acagtcgatt ttattcagtg attctgattg ttcagttttt 480

gtagttgtac agttttcgta ttaggaaata aatcaaatat ggcatcagga gtaactgtag 540

cagatgcctg caaaaaggtc catgaagaaa tcaaaaggga caaaaaacac agatttgcaa 600

ttttctacat caaggatggc aaacagattg atgttgaaca cataggagaa agaaatgcag 660

agtatgatga tttcctaact aaactgcaag agggaggtgc taatgaatgt cgttatggac 720

ttttcgattt tgaatacatg caccagtgcc agggaacctc agagtcaacc aagaaacaaa 780

agttattcct gatgtcgtgg tgtcctgaaa ctgcatctgt caaaaagaag atggtgtatg 840

ctagctcatt cgaatatctt aaaaaagctt tggtaataca gaaatctatc caagcgacag 900

atttagcgga agcgagtata acagcggtag aagaaaaatt acgatccact gatcgtcagt 960

aacgcctttt atttttatta tttttctttt tgtatctcga ctgtctactc acgcaccgac 1020

ctaccgaggt gtattattta tataagtatt tgaatattta ataaagcttc atttataaaa 1080

aaaaaaaaaa aaaaaaaaa 1099

<210> 377
<211> 357
<212> DNA
<213> Diabrotica virgifera

<220>
<221> misc_feature
<222> (1)..(357)
<223> RNAi target sequence

<400> 377
aaaatgtcca agagaggacg tggtggttcc gcgggagcga agtttaggat ctcccttgct 60

cttcctgtag gagcagtaat taattgtgca gataatacag gagcaaagaa cttatatgtt 120

atcgctgtac aaggtattgg tggacgactt aaccgtctgc cagctgcagg atctggtgac 180

atgattgtag ccacagtcaa aaagggtaaa ccagaactca ggaaaaaagt catgcctgca 240

gtagtcatca ggcagcggaa accatttaga aggaaggatg gtgtcttcat ttactttgaa 300

gataatgctg gggttatagt caataacaaa ggtgaaatga aaggatcagc catcact 357

<210> 378
<211> 22
<212> DNA
<213> Artificial Sequence

<220>
<223> primer

<220>
<221> misc_feature
<222> (1)..(22)

<223>  forward primer

<400>  378
aaaatgtcca agagaggacg tg                                                22


<210>  379
<211>  22
<212>  DNA
<213>  Artificial Sequence

<220>
<223>  primer


<220>
<221>  misc_feature
<222>  (1)..(22)
<223>  reverse primer

<400>  379
agtgatggct gatcctttca tt                                                22


<210>  380
<211>  667
<212>  DNA
<213>  Diabrotica virgifera


<220>
<221>  misc_feature
<222>  (1)..(667)
<223>  WCRW transcript ta01259.001_diavv

<400>  380
aaggtattgt ctctttaagg aatgcacagg gatctaatga ctggcaggct cgtttgcatc      60

tggttgcaaa cgtcacgggg gtctgggggc tgtcaaaata agttctttca ataatacaaa     120

atgtccaaga gaggacgtgg tggttccgcg ggagcgaagt ttaggatctc ccttgctctt     180

cctgtaggag cagtaattaa ttgtgcagat aatacaggag caaagaactt atatgttatc     240

gctgtacaag gtattggtgg acgacttaac cgtctgccag ctgcaggatc tggtgacatg     300

attgtagcca cagtcaaaaa gggtaaacca gaactcagga aaaaagtcat gcctgcagta     360

gttatcaggc agcggaaacc atttagaagg aaggatggtg tcttcattta ctttgaagat     420

aatgctgggg ttatagtcaa taacaaaggt gaaatgaaag gatcagccat cactggtcca     480

gttgcaaaag aatgtgcaga tttatggccc agaattgcat caaatgcaag cagtattgct     540

tgatgtgtat atgtttaaga aaaataaaat aggatattgt tcttaaaaaa aaaaaaaaaa     600

aatgcgattt tttatagaac ctatgtaagt ttacaattta ttcatgaaat aatgtaaaaa     660

tgacagg                                                               667


<210>  381
<211>  520

<212>  DNA
<213>  Diabrotica virgifera


<220>
<221>  misc_feature
<222>  (1)..(520)
<223>  RNAi target sequence

<400>  381
ggcccaaaca actgtaagac agggtgctca aggaggaact actgtatacc ccggtatgca      60

aaacacttac agagctactg cccgcccacc aaatcagtca acaaccatgc gtagcaacat     120

gagtgtacct aggcaaatta ctggacaaca accacaaaat gcaattgtac ctgctgctgt     180

taacagaaca gccaacttct aatacacttc aaacatgaga aatccaccac aacctttggg     240

aggtttacca ggtgctgctg ctccagtaca gcaggctgta cacactcaag gtcaggaacc     300

attgacagca accatgttgg ctgctgctcc accacaggag caaaaacaga tgttgggtga     360

aaggttgttt ccacttatcc aacgcatgta tccagacatg gcaggtaaaa tcactggtat     420

gttattggag attgacaaca ctgagctgct acacatgttg gaacaacagg agtcccttaa     480

gaacaaagtg gaagaagcag ttgctgtttt acaagcccat                           520


<210>  382
<211>  20
<212>  DNA
<213>  Artificial Sequence

<220>
<223>  primer


<220>
<221>  misc_feature
<222>  (1)..(20)
<223>  forward primer

<400>  382
gcccaaacaa ctgtaagaca                                                  20


<210>  383
<211>  22
<212>  DNA
<213>  Artificial Sequence

<220>
<223>  primer


<220>
<221>  misc_feature
<222>  (1)..(22)
<223>  reverse primer

<400>  383
atgggcttgt aaaacagcaa ct                                               22

<210>  384
<211>  2656
<212>  DNA
<213>  Diabrotica virgifera

<220>
<221>  misc_feature
<222>  (1)..(2656)
<223>  WCRW transcript ta16449.001_diavv

<400>  384
cgcatttttt tttttttttt ttgacttctg acaaatagac tttttaaacc tattgtgaag      60

aaaaactatt aaaaaaacaa aaaaaattgt aaaatagtgt aaaaaagtaa aaaaatatcc     120

aacgaagatg aatccggctg ccccaaatta tcctatggcg tctttatacg tcggcgattt     180

acattctgat attactgagg ccatgctttt tgagaaattt tcaaccgcgg gtcctgtgct     240

ttcgattcgc gtgtgtagag acttgattac acgtcgatcc ttgggttatg catatgtgaa     300

cttccaacaa cctgcagatg ctgagcgtgc tttggatacc atgaattttg acttgataaa     360

aggtaggcct atccgcatca tgtggtctca acgtgatcca tcattgagaa aatctggcgt     420

tggaaatgtt tttatcaaga atttagatcg ttccattgac aacaaggcca tgtacgatac     480

tttctcagct tttggtaaca ttcttagttg taaggtagcc caagatgaaa acagctctag     540

taaaggttat gggtttgtcc attttgaaac cgaagaagct gcaaataagt ctattgaaaa     600

ggtaaatggt atgttgttga acggaaaaaa agtatatgta ggccgcttta ttccacacaa     660

agaaagagaa aaggaactgg gagaaaaagc caagctgttc acaaatgttt atgttaaaaa     720

ttttggtgaa gattttgctg aggaacagtt aaaggttatg tttgaaaagt atggtaaaat     780

taccagctac aagataatga gtaaagaaga tggtaaatca aaaggatttg gttttgtagc     840

ctttgaaagt tcagaggcag cagaagcggc agttgaagct ttaaatggaa aagagttggt     900

tgaaggaaaa cctttgtatg ttggaagagc ccaaaagaga gctgagcgtc aacaagaact     960

aaaacgtcgc tttgaagcct taaaaatgga aagacttaat cgctatcagg gtgtaaatct    1020

ctatgttaaa aatctagatg atacaataga tgatgaacgt ctccgtaaag agtttactcc    1080

ttttggaacc ataacatctg ccaaagtgat gttggaagaa ggtagaagca agggttttgg    1140

ttttgtatgt ttttcctctc cagaagaagc tacaaaggcg gtaacagaaa tgaatggcag    1200

aattgttggt tctaagcctt tatatgttgc cttagctcag cgtaaagaag atcgtaaagc    1260

acatttaact tctcagtaca tgcagcgtat ggcaaatatg agaatgcacc agatgggtca    1320

gttcatacaa ccaggtgcat ctagcggtta ttttgttcct accattcctg ccgcacaaag    1380

attctatgga ccagcacaga tgactcaaat tcgtgctagt accagatggg ccgcccaaac    1440

aactgtaaga cagggtgctc aaggaggaac tactgtatac cccggtatgc aaaacactta    1500

cagagctact gcccgcccac caaatcagtc aacaaccatg cgtagcaaca tgagtgtacc 1560

taggcaaatt actggacaac aaccacaaaa tgcaattgta cctgctgctg ttaacagaac 1620

agccaacttc aaatacactt caaacatgag aaatccacca caacctttgg gaggtttacc 1680

aggtgctgct gctccagtac agcaggctgt acacattcaa ggtcaggaac cattgacagc 1740

aaccatgttg gctgctgctc caccacagga gcaaaaacag atgttgggtg aaagattgtt 1800

tccacttatc caacgcatgt atccagacat ggcaggtaaa atcactggta tgttattgga 1860

gattgacaac actgagctgc tacacatgtt ggaacaacag gagtccctta agaacaaagt 1920

ggaagaagca gttgctgttt tacaagccca tcaagccaag caagctgcta ctactcagat 1980

taagaaagag tagttgctga agcagtgatg attttatgtt ttatattttt aagcataaat 2040

aaaaagtact tgtgcatttt caaaaaaaaa aaaaaaaaat gcgtcaaatt agctgattgt 2100

agcttaaaca taatctttaa atctaatgtg tttattatta cagtttttgt ttagcatcaa 2160

acattgctta catctccata ttcacttctt gattattgtt taatagtttg tcatttgatt 2220

taaattatac aacaaagtta atgctttgtc atttattcag tttttgtatt ataaaaaaat 2280

atacaattct ggaccttgga ggtaaactac attatgttaa catggtgttg ttttgaataa 2340

gttcccttaa tccacttatt ttattagttg gattatacaa atttggttat atattattca 2400

aatatggtta cattgttttt caaacaattg agatttttat aacactcaaa gtgctctact 2460

gtaatttacc tccgaggtcc agatatgttt ttggaaatcg tcaaagtctc agtacggcta 2520

gctttgctac atttgtaaca catgattaat ttcatttaga atagttgatt acaaaaatgc 2580

cacaaggaaa tagcttcaga acatttcatt ttttaattat tataaattca ttttagaaag 2640

cattatcaaa ttgtag 2656

<210> 385
<211> 490
<212> DNA
<213> Diabrotica virgifera

<220>
<221> misc_feature
<222> (1)..(490)
<223> RNAi target sequence

<400> 385
tcaggtatgc cagtttattt tggaagatta tgtaatgtct gggcaaggag cctgggccaa 60

catctgcgtc acacaaccca gacgtatatc agccgtttcg gtatcagaaa gagtggctgc 120

cgaacgatgt gaagagctcg gtcagtctat cggttactca gtgcgttttg aatcagcttt 180

acctaaaccg tacggatcag taatgttctg tacagtagga gtgttgttga aaaagttgga 240

aaatggtctt agaggagtca gccacgttat tgtagatgag attcacgaac gagatgtcaa 300

tagtgacttt attatggttg ttttgagaga tatgatccac acatatcccg atttaagagt 360

aattcttatg tctgctacca tcgatacaac tttattctct aaatatttca acaactgccc 420

cgttatcgaa attccaggaa gagcttttcc tgttaaacag tatttcttgg aagatgctat 480

tgaactcacg 490

<210> 386
<211> 23
<212> DNA
<213> Artificial Sequence

<220>
<223> primer

<220>
<221> misc_feature
<222> (1)..(23)
<223> forward primer

<400> 386
tcaggtatgc cagtttattt tgg 23

<210> 387
<211> 22
<212> DNA
<213> Artificial Sequence

<220>
<223> primer

<220>
<221> misc_feature
<222> (1)..(22)
<223> reverse primer

<400> 387
cgtgagttca atagcatctt cc 22

<210> 388
<211> 4007
<212> DNA
<213> Diabrotica virgifera

<220>
<221> misc_feature
<222> (1)..(4007)
<223> WCRW transcript ta01430.001_diavv

<400> 388
gggggccaca agcacaagct aaagcgacac gccgggattt ggttccggta gaaaaattca 60

gaaaaaatct attttttcgt ttaaacaagc ataaaaaata attaccaaat attaataaaa 120

catttacaat cgtggtaaac gtattttgaa ttcatcatgt ctgatctaaa atcctttcta 180

aaccaatggt gtcagaagaa taaaaaggaa ccagcttatg aagtgagacc cacgggaccc 240

aagcataggc agagatttct ctgtgaacta agaattgatg gctacaacta tgttggagca 300

ggaaattcaa ccaacaaaaa ggatgcacaa tctaatgctt caaaggattt catctctttc 360

ttggttcggc aaggaatgat taacaaaaat gaggtaccag ctgatattga tgctgctcct 420

acacctgcca ttcaccacgg ccaacaagaa atgacaggtg gcgggccgca acatcaagtg 480

tttgagaaag gtatgggtcc tgccaatttt ggtgaagcat accgtcctcg tggtcgagaa 540

gatggagaat ggcattatat ggacagagct caagaacaga aacgaatgga agaagcagaa 600

gatttagatg tgaatgctca aattcatgga aattggactg tagagaatgc caaatctaag 660

ctacatcaat tcatgcaagc caataagatt aacacggatt ataaatatac acaagttggg 720

ccagatcact ctagaacatt cgtcgcagaa ctcagtttct atgttaaaca acttggaaga 780

actattcatg gaagggatac tggatctaat aaacagactg caagtaaaag ttgtgcccta 840

tcagttgtgc gacaactttt tcacttgaaa gtcattgaag ctttttcagg aacattaaaa 900

aaagaaaaga agtcagcaac agaaatgcct cctttccaag taagactaag tcctaaattg 960

aaggatcaaa tttctgactg tttgaggtca ttaaacgtga ctcccactcc agtcacctct 1020

aatcaagtgg acgataaagg cgaacctgta cccgtgtctc tactaagcag ccacgtttta 1080

gacgactttc atcccgcaaa acccacagcg gctggtgtgg ttccgtggtc gccacctcaa 1140

ccaaactgga actgctggac aggttgtaat atagacgaag ggtatttagc tagtgctacg 1200

ttggatgatt taagtgaaca gatgcttact gattggaaac acagaacagc gaatgattct 1260

tcgctgatca ctagtatgca agacagaatg attttgccgg tatatacgaa gaaatcagag 1320

attatggaag ctatcaacga acatcctgtt attatcataa ggggaaatac tggttgcggt 1380

aaaacaactc aggtatgcca gtttattttg gaagattatg taatgtctgg gcaaggagcc 1440

tgggccaaca tctgcgtcac acaacccaga cgtatatcag ccgtttcggt atcagaaaga 1500

gtggctgccg aacgatgtga agagctcggt cagtctatcg gttactcagt gcgttttgaa 1560

tcagctttac ctaaaccgta cggatcagta atgttctgta cagtaggagt gttgttgaaa 1620

aagttggaaa atggtcttag aggagtcagc cacgttattg tagatgagat tcacgaacga 1680

gatgtcaata gtgactttat tatggttgtt ttgagagata tgatccacac atatcccgat 1740

ttgagagtaa ttcttatgtc tgctaccatc gatacaactt tattctctaa atatttcaac 1800

aactgccccg ttatcgaaat tccaggaaga gcttttcctg ttaaacagta tttcttggaa 1860

gatgctattg aactcacgaa gttcgtgcca cccactgata ccagaaaaag aaaaaataaa 1920

ggtggtaaag atgatgacga agaagaaagt acaggcgttg aagaacctga cgaaatttta 1980

aacaaggttg taggaggaaa ttattcagat ctgaccagaa acgtcatgtc tagactgaat 2040

gaaaaagaag tgagttttga gcttctggaa gctcttttag actacatcaa aacacaatct 2100

atacctggag ctgtattagt tttccttcct ggctggaatt taatcttcgc tatgatgaaa 2160

catcttcaaa atcaccccgt ttatggagga agtcagtaca gtattttacc cttacattcc 2220

caaatcccta gagaagatca gaaaagggtc tttcaacctg ttcctgacca cgtaacaaaa 2280

gtcattcttg caacaaatat cgccgaaact tccatcacca taaacgacgt agtgtttgtg 2340

attgacagtt gcaaagcaaa aatgaagatg tttacttctc acaacaatat gactagttat 2400

gctacggttt gggcttccag gacaaacttg gaacagagaa aaggtcgagc tggtcgtgtt 2460

aggccaggta tttgtttcca tttgtgttcc aaggccagat tcgacaaact tgacgaacat 2520

atgacaccag agatgtttag aactccctta cacgagctgg ctttgagcat aaagctgtta 2580

cgtttgggct cgataggtca tttcctgtca aaggctattg aacctccacc attagatgcc 2640

gtcatcgaag cggaagtgct attaagagaa atgaaatgtc tggatcaaaa cgatgagttg 2700

actcctctgg gtagaatcat cgccaagctt cccattgaac cgagattggg gaaaatgatg 2760

gttttgggtt gtgtatttat gtgtggagga ccattggcta cgatggctgc aaactcttct 2820

acattccctg aaatattcac gctagacatg ggccaaagac ggttaagcta tcatcaaaga 2880

gccttatctg gggacaggca ctccgatcat gttgcgatgt taacagcatt tgagctgtgg 2940

gatcaagcca gacaaggtgg agaagaagca gaattaaggt tttgcgattt caaaggcatc 3000

agtatgccga ctatgagagt aacttgggaa gctaaacatc aactacaaca aatcttgaat 3060

aatgtaggtt ttccagaaga aactatggcg ctatttccta tggaaactgt tggtccagat 3120

cccagacttg atgttgtaat gtctcttatg tgttacggat tatatccaaa cgtttgtttc 3180

cacaaggaca aaagaaaggt attaacaaca gaaagcaaag ccgcacttat ccacaaaact 3240

tccgtcaact gcagtaacca ggatcaagga tttccatacc cattctttgt atttggagaa 3300

aaaatcagaa ccagagctgt atcttgcaaa cagatgacta tggttacacc ccttcatctt 3360

ctgctgttcg gttcccgaaa agtagaatgg gttgataacg tggttaggtt ggacaactgg 3420

atcaatttgg acatggatcc cgaggtagct tccatgatag tggctctaag acctgcgttg 3480

gaaagtttag ttattagaac ttctcagtct ccagaagcaa tcttggaaat gggagaaact 3540

gacgttaagg ttgtgtcaac gattaaagaa atatgcagat tgcacgcagg aagttttgaa 3600

ctagaaagag ccaaaggtag cctgcttcaa tcaaataggc ctcccagagg acacactctc 3660

ggagggttca gtggaccttc tcccaaattc ttcagaggtg gctttaatag tcgaggtggt 3720

ggtggaggtt tcagaggtgg attccgtggc gatagaaatg gggcggtttt tggcggcggc 3780

agaggtggat tccgaggtgg tcgaggcgga tttggaggag gcggtggtgg ctttggcggt 3840

tcccgtggtg gaagatatta gatatcttat aatatatata gatataactt aattactaat 3900

gtttcttagg tatttattgc attttaaaaa tgactgtttc caaacaatgt tttgcaatgt 3960

attagtatga cagtgtatca gttaatttaa gatttagtgt ttaattt                4007

<210>  389
<211>  658
<212>  DNA
<213>  Diabrotica virgifera

<220>
<221>  misc_feature
<222>  (1)..(658)
<223>  RNAi target sequence

<400>  389
gggggacttc attccagtct tgtgatattt agtgcttact tggtacagca gtttcagtgc      60

tgtgctttag aataatttat tttttaacat ttatatagaa atcaaatact aaccaatcaa     120

catgtgtgaa gaagaagttg ccgctttagt cgtagacaat ggatccggta tgtgcaaagc     180

tggttttgct ggggatgatg cacctcgtgc tgtattccct tcaattgttg gacgcccaag     240

acatcagggt gtgatggtag gaatgggaca aaaagattcc tatgtaggtg atgaagctca     300

aagtaaaaga ggtatcctta ccttaaaata ccccatcgag cacggaatag tcacaaactg     360

ggatgatatg gagaaaattt ggcatcatac attctacaat gaactcagag cagccccaga     420

agaacaccct gttctgttga cagaagctcc tctcaacccc aaggccaaca gggaaaagat     480

gacacaaata atgtttgaaa ctttcaacac cccagccatg tatgttgcca tccaggctgt     540

actctccttg tatgcatctg gtcgtacaac tggtattgtg ttggattctg gtgatggtgt     600

atcccacact gtcccaatct atgaaggtta tgctcttcct catgcaatcc ttcgtttg       658

<210>  390
<211>  22
<212>  DNA
<213>  Artificial Sequence

<220>
<223>  primer

<220>
<221>  misc_feature
<222>  (1)..(22)
<223>  forward primer

<400>  390
cttggtacag cagtttcagt gc                                               22

<210>  391
<211>  22
<212>  DNA
<213>  Artificial Sequence

<220>

<223>  primer

<220>
<221>  misc_feature
<222>  (1)..(22)
<223>  reverse primer

<400>  391
gcatgaggaa gagcataacc tt                                              22

<210>  392
<211>  1534
<212>  DNA
<213>  Diabrotica virgifera

<220>
<221>  misc_feature
<222>  (1)..(1534)
<223>  WCRW transcript ta02481.002_diavv

<400>  392
ggattccagt cttgtgatat ttagtgctta cttggtacag cagtttcagt gctgtgcttt      60

agaataattt attttttaac atttatatag aaatcaaata ctaaccaatc aacatgtgtg      120

aagaagaagt tgccgcttta gtcgtagaca atggatccgg tatgtgcaaa gctggttttg      180

ctggggatga tgcacctcgt gctgtattcc cttcaattgt tggacgccca agacatcagg      240

gtgtgatggt aggaatggga caaaaagatt cctatgtagg tgatgaagct caaagtaaaa      300

gaggtatcct taccttaaaa taccccatcg agcacggaat agtcacaaac tgggatgata      360

tggagaaaat ttggcatcat acattctaca atgaactcag agtagcccca gaagaacacc      420

ctgttctgtt gacagaagct cctctcaacc ccaaggccaa cagggaaaag atgacacaaa      480

taatgtttga aactttcaac accccagcca tgtatgttgc catccaggct gtactctcct      540

tgtatgcatc tggtcgtaca actggtattg tgttggattc tggtgatggt gtatcccaca      600

ctgtcccaat ctatgaaggt tatgctcttc ctcatgcaat ccttcgtttg gacttagctg      660

gtagagactt gactgattac ctcatgaaaa ttttgactga acgtggctac tctttcacca      720

ccacagcaga aagagaaatt gttagagata ttaaagaaaa actctgctat gtagctttgg      780

acttcgaaca agaaatggca acagctgcta gttccagttc ccttgaaaag agttatgaac      840

ttcctgatgg acaagtcatc accattggta atgaaagatt ccgttgccca gaagctctct      900

tccaaccatc cttcttgggt atggaagctg ctggtattca tgaaacaacc tacaattcca      960

tcatgaagtg tgatgttgat atccgtaagg acctatatgc caacactgta ctctctggag     1020

gtaccaccat gtaccctggt attgctgata gaatgcagaa agaaatcact gccttggctc     1080

ctagcacaat gaagattaag atcatcgccc caccagaaag aaaatactct gtatggatcg     1140

gtggttccat tttggcctct ctatccacct tccaacaaat gtggatttcc aaacaagaat     1200

atgatgaatc tggtccatcc atcgtccaca gaaaatgctt ctaatttgtt tttgtccgca 1260

tttatattct ttatttctat ttacataagc ccgcccattt tccgggcgcg aactgccgtg 1320

tttatcgtat gggatcgaga ccataaaata tccacactag gccaaagtat tactatctta 1380

atttttgtaa tttattacct gtgcaaagta agactattat attattaggc taatactatg 1440

ggaaaataaa aaaaatagta aaactcaaaa aaaaaaaaaa aaaatcagtt tacctttcta 1500

ataaaataaa taaaacaaaa aaaaaaaaaa aaaa 1534

<210> 393
<211> 601
<212> DNA
<213> Diabrotica virgifera

<220>
<221> misc_feature
<222> (1)..(601)
<223> RNAi target sequence

<400> 393
tgctccaaga tgaaggtact cattcttctt tccgttgttg ccttgagcag ctgcttacca 60

gcatccagaa atcaacaata cgttgatgca gactacaaca gctacaacgc tcatcccgtc 120

tacaacttct ggaggacttg gaatcagcaa cactatggac aagaaccatg gaaccaatgg 180

gtacaatatg gtgtccagaa ccaacccggt aactactgga ggcagtacgt gcaacgcagc 240

aaccaagctg aaaatgtcga tttaccttgg atgtatcact cttgggatgt ccaggatgtg 300

caaaacttaa ctcccgttca acgtgttcag ttgctactca actacgtcag acttcaccaa 360

caatggaacc aatggactca tgatgtaact gccaatgaac gtcttcagaa tatgttgtat 420

caacaccttc ctgaacactt atggagctcc aacgagcaaa ttgtaccata catgatgaac 480

tacttcaacc aatacccaat gaaatacaac gacattatgc atgaagttat gcaaacccca 540

tgggctcacc aacaacttat gttccacgtt atgtctgaat tataccagca cttcggtcaa 600

g 601

<210> 394
<211> 22
<212> DNA
<213> Artificial Sequence

<220>
<223> primer

<220>
<221> misc_feature
<222> (1)..(22)
<223> forward primer

<400>  394
tgctccaaga tgaaggtact ca                                                22

<210>  395
<211>  22
<212>  DNA
<213>  Artificial Sequence

<220>
<223>  primer

<220>
<221>  misc_feature
<222>  (1)..(22)
<223>  reverse primer

<400>  395
cttgaccgaa gtgctggtat aa                                                22

<210>  396
<211>  1208
<212>  DNA
<213>  Diabrotica virgifera

<220>
<221>  misc_feature
<222>  (1)..(1208)
<223>  WCRW transcript ta08003.008_diavv

<400>  396
   ggggggacat ttcagtacac tgctccaaga tgaaggtact cattcttctt tccgttgttg      60
   ccttgagcag ctgcttacca gcatccagaa atcaacaata cgttgatgta gactacaaca      120
   gctacaacgc tcatcccgtc tacaacttct ggaggacttg gaatcagcaa cactatggac      180
   aagaaccatg gaaccaatgg gtacaatatg gtgtccagaa ccaacccggt aactactgga      240
   ggcagtacgt gcaacgcagc aaccaagctg aaaatgtcga tttaccttgg atgtatcact      300
   cttgggatgt ccaggatgtg caaaacttaa ctcccgttca acgtgttcag ttgctactca      360
   actacgtcag acttcaccaa caatggaacc aatggactca tgatgtaact gccaatgaac      420
   gtcttcagaa tatgttgtat caacaccttc ctgaacactt atggagctcc aacgagcaaa      480
   ttgtaccata catgatgaac tacttcaacc aatacccaat gaaatacaac gacattatgc      540
   atgaagttat gcaaacccca tgggctcacc aacaacttat gttccacgtt atgtctgaat      600
   tataccagca cttcggtcaa gccccatggt cccacgaaca aataatgaac cagatcatgc      660
   aacaccaata cctctacggt acaaccttat cccaacaaca actccaacaa ttggtagaat      720
   tcgtactcca cattcaatac gaacttactc ctttacatta ccaacaactt gaacaagttc      780
   aaccctggaa ccgtgaacaa gtactttcat ggaaccaagg acaagtactc ccatgggcct      840
   acagacaact tcttccattg aactatgaaa ccgttttacc atggacccgc cagtattccg      900
   cacaaaacaa catgaaccta gagtcccaat acaactacaa tctccaccaa caacaaatgc 960
   cattgtccca atactggaac cagagatacg gacaacaata cccacaacat tatgaagcca 1020
   tggaacaacc cggatggtgg aaccaagaat ggacccaaaa cttggttgga caagagaatc 1080
   attatcctct cttggggcaa aatatgccct tagtatatta aaaagtattc tgttctaagc 1140
   atatgattta aaataaaaaa aggcatatat acttgttagt tttattttta acccgtatac 1200
   aaagagtt 1208

<210> 397
<211> 600
<212> DNA
<213> Diabrotica virgifera

<220>
<221> misc_feature
<222> (1)..(600)
<223> RNAi target sequence

<400> 397
atttctttca aactgggatt cggtgaagac gtgacagctc agggggcgtt taatgaaaaa 60

actgtgattt attagtgaaa tagtggttaa aacccagcaa aatggggatt aaatttttgg 120

aagttataaa gcctttctgc agcatactgc ctgaaatagc caagccagaa agaaaaatcc 180

aattcaggga gaaagtacta tggactgcaa tcactctttt catcttttta gtatgttgtc 240

agattccatt attcggtata atgagttcag attctgcaga tcctttctac tggattcgtg 300

ttattcttgc atcaaacaga ggtaccctta tggaacttgg tatttcccca attgtaactt 360

caggtctgat tatgcagctg ttggccggtg ccaaaattat cgaggttggt gacactccaa 420

agacagagcc ttattcaatg gtgcccaaaa attgtttggt atggttatta ctgtcggtca 480

agccatcgtg tatgtaatga caggcatgta tggtgatccg gctgaaattg gagctggagt 540

gtgccttctc atcatcatcc aactgttcgt cgccggtctc attgtattgc ttctcgatga 600

<210> 398
<211> 22
<212> DNA
<213> Artificial Sequence

<220>
<223> primer

<220>
<221> misc_feature
<222> (1)..(22)
<223> forward primer

<400> 398
atttctttca aactgggatt cg 22

<210>  399
<211>  22
<212>  DNA
<213>  Artificial Sequence

<220>
<223>  primer


<220>
<221>  misc_feature
<222>  (1)..(22)
<223>  reverse primer

<400>  399
tcatcgagaa gcaatacaat ga                                                22


<210>  400
<211>  2110
<212>  DNA
<213>  Diabrotica virgifera


<220>
<221>  misc_feature
<222>  (1)..(2110)
<223>  WCRW transcript ta03313.003_diavv

<400>  400
ccaacatcat cgatcacgtg acccgtgtgc tgtgctacgt gtacgacgtg tagacgtaaa      60

acagagacaa gaagtaccgt aatttctttc aaactgggat tcggtgaaga cgtgacagct     120

caggggggcg tttaatgaaa aaactgtgat ttattagtga aatagtggtt aaaacccagc     180

aaaatgggga ttaaattttt ggaagttata aagcctttct gcagcatact gcctgaaata     240

gccaagccag aaagaaaaat ccaattcagg gagaaagtac tatggactgc aatcactctt     300

ttcatctttt tagtatgttg tcagattcca ttattcggta taatgagttc agattctgca     360

gatcctttct actggattcg tgttattctt gcatcaaaca gaggtaccct tatggaactt     420

ggtatttccc caattgtaac ttcaggtctg attatgcagc tgttggccgg tgccaaaatt     480

atcgaggttg gtgacactcc aaagacagag ccttattcaa tggtgcccaa aaattgtttg     540

gtatggttat tactgtcggt caagccatcg tgtatgtaat gacaggcatg tatggtgatc     600

cggctgaaat tggagctgga gtgtgccttc tcatcatcat ccaactgttc gtcgccggtc     660

tcattgtatt gcttctcgat gaacttctcc agaaaggata tggtttaggt tctggtattt     720

ccctcttcat tgcaacaaac atttgtgaaa ctattatttg gaaagccttc tcaccagcaa     780

cagtaaacac tggaagagga acagaatttg aaggagctgt cattgcctta ttccacttgt     840

tgaccaccag acaagacaaa atcagaggat tacgtgaagc tttctacagg caaaatcttc     900

ccaatttgat gaacttgttg gcgacagtac tcgtttttgc tatagtaata tacttccaag     960

gattcagagt agatctaccc atcaagagtg caagataccg tggacaatat tccagctatc 1020

caatcaaatt gttctacact tcgaacatcc ctattattct acaatctgct cttgtatcta 1080

acttgtatgt catctcacag atgttggctg ttaaattcca agggaacttc ttaattaatc 1140

tattaggagt ctgggctgac gttggaggcg gtggcccagc taggtcttac cccattggag 1200

gattatgtta ctacttatct ccacctgaaa gtgtaagtca cattctagaa gatccaatcc 1260

acgcaatgct ctacatcgtc ttcatgttgg gatcttgtgc tttcttctcc aaaacatgga 1320

tcgaagtatc tggaagctca gccaaagatg tcgctaagca attgaaagaa caacagatgg 1380

taatgagagg acacagagat aactccatga ttcacgaatt gaacaggtac attcccaccg 1440

ccgcagcttt tggaggtctc tgtattggag ccttgtcagt actcgctgat ttcatgggag 1500

ccattggttc aggtactggt atcttgttag ctgtcaccat catctaccaa tacttcgaaa 1560

tcttcgtcaa agaacaaagc gagatgggtg gaatgggagc gttactcttc tagataaatt 1620

gaagatatca caaagctata gcttgactga tattgtgatt ttgtttggta aatagtaact 1680

attgtggtta ttatttgttt agtttttgta cctcagtgtc tccactatag ttattattta 1740

tgtggaacaa cttatatgtc actttgtata aagtgtgaaa catttttttg tactaaaaga 1800

ttgaaaatag acaaatatat ttcattgtac atttacatga ttttgtgttt aagaagcgag 1860

ttatttataa tgtgcggatg tcccatacta ttagtttatt ttttacattt tttcattctc 1920

gttcatgcag tacggttgtg tattgatcat ttattatttg ttgttagata taatttattt 1980

taaatacaga aatttctgaa atcctgtttt tcatttgatg ctaatgtata ctgtaaatgt 2040

ttcgagataa gggcatctat catttaaatg ttgaaatatt attgaaatgt acaaatttac 2100

atgttctttt 2110

<210> 401
<211> 707
<212> DNA
<213> Diabrotica virgifera

<220>
<221> misc_feature
<222> (1)..(707)
<223> RNAi target sequence

<400> 401
gggggacctt ttatatttga catcctgaaa gatggttgag aaaaaggctg tacagaagcc 60

cgctggggat gcaccaaaaa aggtgcacaa accaggaaaa cctagaaatt atgatttggg 120

aaatggagta ctcagatttt ctagatctaa aatgttccac aaaaaagcat tgtacaaatt 180

tgttggcaag aaggtagaag ctaccaagaa acctcaaaag ccccaggtta ttgaaaaacc 240

aattggtgga gagaaaaatg gaggaaagag atttgttttg gtcaagaaac gtagggcatc 300

ctatccaacc caagacaaaa ttaaggccag gccagctagg agaacattta gccaacacac 360

aaggtctatt agaaatacaa tcactccagg aacagttctc atccttttag ctggttctca 420

caaaggaaag agggttgtat tccttaaaca actgtactct ggtctacttt tggtaactgg 480

accttttgga atcaatgctt gcccattgag gcgcattagc caacgatatg taattgctac 540

atctaccaaa attgacgtta gtggagtaca gctgccagaa aaccttaatg atgaatattt 600

tgcaagaaag aggcagaaga gatccaagaa agaagaaggt gatattttcc aatccaaaaa 660

agaaggttac aaagtaagtg aagataggaa agctgaccaa aagaaag 707

<210> 402
<211> 18
<212> DNA
<213> Artificial Sequence

<220>
<223> primer

<220>
<221> misc_feature
<222> (1)..(18)
<223> forward primer

<400> 402
   gaccttttat atttgaca 18

<210> 403
<211> 22
<212> DNA
<213> Artificial Sequence

<220>
<223> primer

<220>
<221> misc_feature
<222> (1)..(22)
<223> reverse primer

<400> 403
ctttcttttg gtcagctttc ct 22

<210> 404
<211> 1036
<212> DNA
<213> Diabrotica virgifera

<220>
<221> misc_feature
<222> (1)..(1036)
<223> WCRW transcript ta17558.001_diavv

<400>  404
aaggagaggg aggttttcct gtttgtgcac cttttttgtg cagacagaac gaccacctgg      60

acacgcatgc tatgggggac cttttatatt tgacatcctg aaagatggtt gagaaaaagg     120

ctgtacagaa gcccgctggg gatgcaccaa aaaaggtgca caaaccagga aaacctagaa     180

actatgattt gggaaatgga gtactcagat tctctaggtc taaaatgttc cacaaaaaag     240

cattatacaa attcgttggc aagaaagtag aagctaccaa gaaacctcaa aagccccagg     300

ttattgaaaa accaattggt ggagagaaaa atggaggaaa gagatttgtt ttggtcaaga     360

aacgtagagc atcctatcct acccaagaca aaattaaggc caggccagct aggagaacat     420

ttagccaaca cacgaggtct attagaaata caatcactcc aggaacagtt ctcatccttt     480

tagctggttc tcacaaagga aagagggttg tattccttaa acaactgtac tctggtctac     540

ttttggtaac tggacctttt ggaattaatg cctgtccatt gagacgtatt agccaacgtt     600

atgtaattgc tacatctacc aaaattgacg ttagtggagt acagctgcca gaaaacctaa     660

atgatgaata ttttgcaaga aagaggcaga agagatcaaa gaaagaagaa ggtgatattt     720

tccaatccaa aaaagaaggt tacaaagtaa gcgaagatag gaaagctgac caaaagaaag     780

tagatacaca agttctggct gcaatcaaga agcaatcaga ccgcaaagta cttttagctt     840

atttgtcagc catgtgggga ctcagatcat cacagtaccc acacagattg aagttttaga     900

taagagatac cataaattgt ttatcttttg acttgtatag agaggaaagt gataaataaa     960

ttatggattt ataaatgagg tttttttatg tgactgttgt tataatacaa taatttaatt    1020

cttaaaaaaa aaaaaa                                                    1036

<210>  405
<211>  671
<212>  DNA
<213>  Diabrotica virgifera

<220>
<221>  misc_feature
<222>  (1)..(671)
<223>  RNAi target sequence

<400>  405
gggaacctta ttttatggtg tcgtgccagc agaaaatttt aaaaattctt gtaattgttt      60

agacatttcg caacaattgt gaacaatttt ggaaaatatt gcttgaaagc tataattcta     120

gatgtaagag caaagttttg tagtcaaaat ggctggagta ttcgatttag aactgcatga     180

tgttgaaact gtagaagtag atgattcaga cgatgcaatc gaaattgaag aggatgaata     240

tgatcaaaat cctaatatta atgcaattgt agaatccgat gatacagaaa cggttcatct     300

gactgaacaa attgtgaacc cgggccagga aaaaaccgga ccccatgact ttgaattacg     360

caaagtcctg ggcaaaggag gttatggcaa agtatttcaa gttagaaaaa ttacggggaa     420

agatgccaat actgtctttg ccatgaaggt tctccgcaag gcttccatag ttagaaatca 480

aaaagatact gcccatacga aagcagaacg aaatatttta gaagaagtta agcatccttt 540

tatagtggat ttgatatatg ccttccaaac gggtggaaaa ttgtacttaa ttttagaata 600

cctcagcggg ggcgaacttt ttatgcattt agaaagagaa ggaatatttt tagaggatac 660

cgcttgcttt t 671

<210> 406
<211> 21
<212> DNA
<213> Artificial Sequence

<220>
<223> primer

<220>
<221> misc_feature
<222> (1)..(21)
<223> forward primer

<400> 406
gaaccttatt ttatggtgtc g 21

<210> 407
<211> 22
<212> DNA
<213> Artificial Sequence

<220>
<223> primer

<220>
<221> misc_feature
<222> (1)..(22)
<223> reverse primer

<400> 407
aaaagcaagc ggtatcctct aa 22

<210> 408
<211> 2032
<212> DNA
<213> Diabrotica virgifera

<220>
<221> misc_feature
<222> (1)..(2032)
<223> WCRW transcript ta00443.001_diavv

<400> 408
gggggagaca taaccttatt ttatggtgtc gtgccagcag aaaattttaa aaattcttgt 60

aattgtttag acatttcgca acaattgtga acaattttgg aaaatattgc ttgaaagcta 120

taattctaga tgtaagagca aagttttgta gtcaaaatgg ctggagtatt cgatttagaa 180

ctgcatgatg ttgaaactgt agaagtagat gattcagacg atgcaatcga aattgaagag 240

gatgaatatg atcaaaatcc taatattaat gcaattgtag aatccgatga tacagaaacg 300

gttcatctga ctgaacaaat tgtgaacccg ggccaggaaa aaaccggacc ccatgacttt 360

gaattacgca aagtcctggg caaaggaggt tatggcaaag tatttcaagt tagaaaaatt 420

acggggaaag atgccaatac tgtctttgcc atgaaggttc tccgcaaggc ttccatagtt 480

agaaatcaaa aagatactgc ccatacgaaa gcagaacgaa atattttaga agaagttaag 540

catcctttta tagtggattt gatatatgcc ttccaaacgg gtggaaaatt gtacttaatt 600

ttagaatacc tcagcggggg cgaacttttt atgcatttag aaagagaagg aatattttta 660

gaggataccg cttgctttta tttatccgaa atcattctcg ccctagaaca tttacatgga 720

caaggaatta tttatagaga tctaaagcct gaaaatattt tacttgacgc ccacggtcac 780

gttaaattga cagatttcgg tctgtgtaaa gaacacatta acgaaggcat attgacccac 840

acgttttgtg gaactattga atatatggca cctgaaattt taactagaca tggccatgga 900

aaagaggttg attggtggtc attaggggca ttgatgtacg atatgttaac tggtgcacct 960

ccgtttaccg ctgaaaatag aaaaaagaca atagaaaaaa ttactaaagg gaaaactaaa 1020

ccttccgcct tatttgaccg ccgatgctcg tgatttagta cgcaaattac taaagaggca 1080

ggttccttcg agattagggg ctcctcccga tgatgcaaat gctgtcaaac gacatcaatt 1140

cttcaaacac atcgtctggg atgatgtcct gagaagaaaa tatgaacctc catttaaacc 1200

aagtcttgct agtgatgatg atgtctctca gtttgatacc agatttacaa aacaaactcc 1260

agtagattcg ccggacgaat cgactcttag tgaaagtgcc aacatgatat tccagggctt 1320

tacctacata gcaccctccg tcttggaaga tatctataaa ccaacgatag taaaagctcg 1380

ctctccccgt aaaggttcct atcgccctca tttcggtttt acgcctactc atcatagtca 1440

tcccaataac ctggatccga attgcagcta cgtagcagga atgtcttctg cgcacgctgc 1500

gcactccaat cacgtgcctc cacatgctgc acacggactt gcgcatcatc cacatgcgca 1560

ccatcataac ccgtttgctc cacaagcagt ctttaatata ccaacaaatg catgcgcaga 1620

cgagatgatg gaagtcggca gcggactacc tcatgtatag tatatatatg atagattagg 1680

accggtcttt ttatataaga gttgatagga agtttttgat aggtatcgat tcattagttc 1740

aattttgttt tttatgatta atattatttt gactggaggt gtcaaatttc ctaatgatag 1800

atttagcttc ccttgatcga tcacattata aaatacaata atttttgaac tgaagtatcg 1860

catgtgtcaa aagttgatat ctttggaaaa taaagactat attatgtgtt gttatacatt 1920

taatttgttg aagtctagtt acaaaaggct caattttgtt aaaataggtt tggcaagtgc 1980

acctaggtca gtatgggttg cgaatttaaa atggttataa aaaatatgct ca 2032

<210> 409
<211> 612
<212> DNA
<213> Diabrotica virgifera

<220>
<221> misc_feature
<222> (1)..(612)
<223> RNAi target sequence

<400> 409
gagcttgcga tttttatttt tgaaaaatag agagtattct ctaatattta aggacagtat 60

ggaagacgat tgggctgtgg ataatcagag tggtggagtt gtcgccccaa aaattgcaga 120

actacctgaa attaagttgt tcgctagatg gaactgcgat gatgtccaag tttcagacat 180

gtcccttcag gactacattg cagtgaaaga aaaaaatgca aagtatttac cccattcagc 240

tggtagatat gctgcaaaaa ggttccgtaa agcacaatgc ccaatcgttg agaggttaac 300

aaactctcta atgatgcatg gacgtaacaa tggtaaaaaa ttgatggctg tcagaattgt 360

taaacatgct tttgaaatta tccatttact aactggagaa aatccattac agattttagt 420

ttctgctatt atcaattcag gacctagaga agattctact cgtattggta gagctggtac 480

tgtaagaaga caagctgttg atgtgtcacc cttgagaagg gttaaccaag caatttggtt 540

gctctgcaca ggtgctaggg aagcagcatt ccgtaatatt aaaactattg ctgaatgttt 600

ggctgatgaa tt 612

<210> 410
<211> 23
<212> DNA
<213> Artificial Sequence

<220>
<223> primer

<220>
<221> misc_feature
<222> (1)..(23)
<223> forward primer

<400> 410
gagcttgcga tttttatttt tga 23

<210> 411
<211> 22
<212> DNA
<213> Artificial Sequence

<220>
<223> primer

<220>
<221>  misc_feature
<222>  (1)..(22)
<223>  reverse primer

<400>  411
aattcatcag ccaaacattc ag                                                22


<210>  412
<211>  780
<212>  DNA
<213>  Diabrotica virgifera


<220>
<221>  misc_feature
<222>  (1)..(780)
<223>  WCRW transcript 454run3_isotig03469

<400>  412
aagttttatc catatttatt attggaaata aaatataaaa tgagaaataa atttaacggt       60

tggatttggc tacacgttca agttcatcct tctttttgat agcatatgaa tttgatgatc      120

ccttggcagc attgattaat tcatcagcca aacattcagc aatagtttta atattacgga      180

atgctgcttc cctagcacct gtgcagagca accaaattgc ttggttaacc cttctcaagg      240

gtgacacatc aacagcttgt cttcttacag taccagctct accaatacga gtagaatctt      300

ctctaggtcc tgaattgata atagcagaaa ctaaaatctg taatggattt tctccagtta      360

gtaaatggat aatttcaaaa gcatgtttaa caattctgac agccatcaat tttttaccat      420

tgttacgtcc atgcatcatt agagagtttg ttaacctctc aacgattggg cattgtgctt      480

tacggaacct ttttgcagca tatctaccag ctgaatgggg taaatacttt gcattttttt      540

ctttcactgc aatgtagtcc tgaagggaca tgtctgaaac ttggacatca tcgcagttcc      600

atctagcgaa caacttaatt tcaggtagtt ctgcaatttt tggggcgaca actccaccac      660

tctgattatc cacagcccaa tcgtcttcca tactgtcctt aaatattaga gaatactctc      720

tatttttcaa aaataaaaat cgcaagctct tgctgaacga cggcgtgaac cggaaaaggt      780


<210>  413
<211>  630
<212>  DNA
<213>  Diabrotica virgifera


<220>
<221>  misc_feature
<222>  (1)..(630)
<223>  RNAi target sequence

<400>  413
gcttcttttt cgttggatgt ctttgtaggt tttaaaaatg ggagcctaca agtatataca       60

ggagttgtat agaaaaaaac aaagtgatgt tcttagattt ttactgagag tgagagtctg 120

gcaatatcgc caattgacta aactccacag aactcccaga cccagtcggc ctgacaaggc 180

tcgcagaatg ggttacaggg ccaagcaagg ttttgttatt taccgtatta gagtacgacg 240

tggaggacgc aagcgtccag taccaaaagg tgccacctac ggtaaaccca aaagccatgg 300

agttaatgaa ttgaaacctg tccgtaactt acaagctatt gctgaggaac gtgtaggaag 360

aaggtgtggt ggattaagag tcctaaactc ctactgggta gcccaagatt caacatacaa 420

atattttgaa atcattttgg tcgacccctc acataacgct attcgcaagg accccaaaat 480

taactggatt gtcaactcag tacacaaaca cagagaactc cgtggcaaaa cttcctctgg 540

caaatcatct cgtggtttgg gcaagggtca cagatactct caaaccaagg gaggatccag 600

gcgtgccgct tggctcagaa ggaactcact 630

<210> 414
<211> 22
<212> DNA
<213> Artificial Sequence

<220>
<223> primer

<220>
<221> misc_feature
<222> (1)..(22)
<223> forward primer

<400> 414
gcttcttttt cgttggatgt ct 22

<210> 415
<211> 22
<212> DNA
<213> Artificial Sequence

<220>
<223> primer

<220>
<221> misc_feature
<222> (1)..(22)
<223> reverse primer

<400> 415
agtgagttcc ttctgagcca ag 22

<210> 416
<211> 1374
<212> DNA
<213> Diabrotica virgifera

<220>
<221>  misc_feature
<222>  (1)..(1374)
<223>  WCRW transcript ta18374.001_diavv

<400>  416
tatataactc ggattaccac agtcaaagtg cataaaactt aacaatacgc aaaaaccgta      60

gcagtccgtt tatttctttc ctgcggcctt ggctggagcc ttcttggcct tcttaggctt     120

tgatttctta tctttcaaga tttgtgccct tctcctttct tgcaattttc tggatctaat     180

cacgacgttg tcggcactca cggtaatacc acgtttcttg gccaaaagtt cttccctgtt     240

taactgtctc ttttgattct tcaaaatggc ttcacgtttg agtacagcag catatggatt     300

caacttaagc atggccttag cgttggtcaa tggattgaga cgacgtacac gacgtacaac     360

cttcttttga ggagcacgta atacagcttt gatttcatca gccttcaaca atctagatag     420

atcagtgttg gccattttag gctggggtag attgtaaccc ttcttttcca atgatgctgt     480

tttccatgtg ccgaacaatt tatctaaacg ttggaaagct gattcagtcc aaataacaaa     540

gcgacctaca tgaccaccag gagcaagttt caacaaattt aacttctgta cgctaatgag     600

gtcaatgcct gggatgttac ggaaagcacg acgaagacct acaaggggga cttgcgcgtt     660

gaactttctt tttcgttgga tgtctttgta ggttttaaaa atgggagcct acaagtatat     720

acaggagttg tatagaaaaa aacaaagtga tgttcttaga tttttactga gagtgagagt     780

gtggcaatat cgccaattga ctaaactcca cagaactccc agacccagtc ggcctgacaa     840

ggctcgcaga atgggttaca gggccaagca aggttttgtt atttaccgta ttagagtacg     900

acgtggagga cgcaagcgtc cagtaccaaa aggtgccacc tacggtaaac ccaaaagcca     960

tggagttaat gaattgaaac ctgtccgtaa cttacaagct attgctgagg aacgtgtagg    1020

aagaaggtgt ggtggattaa gagtcctaaa ctcctactgg gtagcccaag attcaacata    1080

caaatatttt gaaatcattt tggtcgaccc ctcacataac gctattcgca aggaccccaa    1140

aattaactgg attgtcaact cagtacacaa acacagagaa ctccgtggca aaacttcctc    1200

tggcaaatca tctcgtggtt tgggcaaggg tcacagatac tctcaaacca agggaggatc    1260

caggcgtgcc gcttggctca gaaggaactc actgcaattg cgcagaaaac gttaaaattt    1320

atttttttat aattagacca ataaaattta cggattaaaa aaaaaaaaaa aaaa          1374

<210>  417
<211>  663
<212>  DNA
<213>  Diabrotica virgifera

<220>
<221>  misc_feature
<222>  (1)..(663)

<223>  RNAi target sequence

<220>
<221>  misc_feature
<222>  (570)..(570)
<223>  n can be any nucleotide

<400>  417
gggcgaaact gttcttcatt atcctttcta ggtcgtgtaa aatacgtgaa ggccaaaatg      60

ggtagacgac ctgcgaggtg ctaccgttat tgtaaaaaca aaccctaccc caaaagtcgt     120

ttctgcagag gtgttccaga tccaaaaatc cgtattttcg atttgggtaa gaagaaggct     180

tccgtagaag actttcctct atgtgtacat ttagtttctg acgaatatga acagctaagt     240

tctgaagcat tagaagcagg acgtatctgt tgtaacaaat atttggtaaa gaactgtggt     300

aaagatcaat tccacattcg tatgagactc catcctttcc atgttatcag aatcaacaag     360

atgttgtcat gtgctggagc tgatagactc cagactggaa tgagaggagc tttcggcaag     420

ccccaaggta cagttgctcg tgtccgcatt ggtcaaccta ttatgagtgt acgctctagt     480

gatcgtttca aggctgcagt tattgaggct ctccgtcgtg ccaagttcaa gttcccaggt     540

cgtcaaaaga tctatgtatc gaagaaatgn ggattcacca agtatgaacg tgaacaatat     600

gaaaacttaa aacaacaagg tagattagct cctgatggtt gcaatgttca atacaggcca     660

gag                                                                   663


<210>  418
<211>  20
<212>  DNA
<213>  Artificial Sequence

<220>
<223>  primer


<220>
<221>  misc_feature
<222>  (1)..(20)
<223>  forward primer

<400>  418
gcgaaactgt tcttcattat                                                  20


<210>  419
<211>  22
<212>  DNA
<213>  Artificial Sequence

<220>
<223>  primer


<220>
<221>  misc_feature
<222>  (1)..(22)

<223>  reverse primer

<400>  419
ctctggcctg tattgaacat tg                                                22


<210>  420
<211>  726
<212>  DNA
<213>  Diabrotica virgifera


<220>
<221>  misc_feature
<222>  (1)..(726)
<223>  WCRW transcript ta04337.003_diavv

<400>  420
gaaggtttgt ttcttaattt attttaacaa tcttacgttt ttagttgtag tgatatattt      60

ttataaacta aatactgaat acgtaataac gtatatgtgt tagtttttta atctacatat     120

ttattttcta ttttcaggcc aaaatgggta gacgacctgc gaggtgctac cgttattgta     180

aaaacaaacc ctaccccaaa agtcgtttct gcagaggtgt cccagatcca aaaatccgta     240

ttttcgattt gggtaagaag aaggcttcag tagaagactt tcctctatgt gtacatttag     300

tttctgacga atatgaacag ctaagttctg aagcattaga agcaggacgt atctgttgta     360

acaaatattt ggtaaagaac tgtggtaaag atcaattcca cattcgtatg agactccatc     420

ctttccatgt tatcagaatc aacaagatgt tgtcatgtgc tggagctgat cgactccaga     480

ctggaatgag aggagctttc ggcaagcccc aaggtacagt tgctcgtgtc cgcattggtc     540

aacctattat gagtgtacgc tctagtgatc gtttcaaggc tgcagttatc gaggctctcc     600

gtcgtgccaa gttcaagttc ccaggtcgtc aaaagatcta tgtatcaaag gaaatgggga     660

ttcaccaagt atgaacgtga acaatatgaa aacttaaaac aacaaggtag attagctcct     720

gatggt                                                                 726


<210>  421
<211>  625
<212>  DNA
<213>  Diabrotica virgifera


<220>
<221>  misc_feature
<222>  (1)..(625)
<223>  RNAi target sequence

<400>  421
gcgaaagttg tgtcttgaaa agtaaaaatg acgtctaaag tatctcgtga tactctatac      60

gagtgtgtga atggagtctt ggaaaatgcc aaggagaaga aaaggaactt tttggaaacc     120

gtggaactcc agatcggtct taagaactac gatccccaaa aggacaagcg tttcagcgga     180

accgtcaaac taaagcacat tcctcgtcca aagatgcagg tatgcatctt gggagatcag 240

caacattgtg atgaagctaa cgctaacaat gttccattca tggatgtaga agccttgaaa 300

aaattgaaca aaaacaagaa attggttaag aaactagcca agaaatatga tgctttcctc 360

gcttctgaag ctctcatcaa acagatcccc aggttgttgg gtccaggttt gaacaaggct 420

ggtaaattcc ctggtcttct ctcccaccag gaatccatga cacagaaaat tgatgaagta 480

aaagcaacaa tcaaattcca aatgaagaag gtactctgcc tctctgttgc tgttggtcat 540

gtaggcatga atcaagatga attggttcaa aatgtccatc tttcaatcaa cttcttggtc 600

tcactgttga agaaacattg gcaga 625

<210> 422
<211> 22
<212> DNA
<213> Artificial Sequence

<220>
<223> primer

<220>
<221> misc_feature
<222> (1)..(22)
<223> forward primer

<400> 422
gcgaaagttg tgtcttgaaa ag 22

<210> 423
<211> 22
<212> DNA
<213> Artificial Sequence

<220>
<223> primer

<220>
<221> misc_feature
<222> (1)..(22)
<223> reverse primer

<400> 423
tctgccaatg tttcttcaac ag 22

<210> 424
<211> 1055
<212> DNA
<213> Diabrotica virgifera

<220>
<221> misc_feature
<222> (1)..(1055)
<223> WCRW transcript ta02345.005_diavv

<400>  424
aagactagta ccttcacata atatggagaa tctcctgggt tgaacataga aaaaaatgta      60

taatgtatcc ataatagaac aactcaatat aaaagatagg ttgcttaaac aaacacaaca     120

gagatacagt attttggaca catttcctga agaacaggaa cactttatca ttcctggtgc     180

atttcgttaa cgtagacaag ccgcaaaagt tgtgtcttga aaaataaaaa tgacgtctaa     240

agtatctcgt gatactctat acgagtgtgt gaatggagtc ttggaaaatg ccaaggagaa     300

gaaaaggaac tttttggaaa ccgtggaact ccagatcggt cttaagaact acgatcccca     360

aaaggataag cgtttcagcg gaaccgtcaa actaaagcac attcctcgtc caaagatgca     420

ggtatgcatc ttgggagatc agcaacattg tgacgaagct aacgccaaca atgttccatt     480

catggatgta gaagccttga aaaaattgaa caaaaacaag aaattggtta agaaactagc     540

caagaaatat gatgctttcc ttgcttctga agccctcatc aaacagatcc ccaggttgtt     600

gggtccaggt ttgaacaagg ctggtaaatt ccctggtctt ctctcccacc aggaatccat     660

gacacagaaa attgatgaag taaaagcaac aatcaaattc caaatgaaga aggtactctg     720

cctttctgtt gctgttggtc atgtaggcat gaatcaagat gaattggttc aaaatgtcca     780

tctttcaatc aacttcttgg tctcactgtt gaagaaacat tggcagaacg ttaggtctct     840

ccacgtcaaa tcatctatgg gacccccaca aagattgtat taagttataa tttttatact     900

gagactttta tttacacact taaatgtggg tatctaaata aacatcgttt ttaagttctt     960

taaaaaaaaa aaaaatagaa aaaaaaaatg attggatgta catattatac taccctacta    1020

agtaaaaaaa tacatctaaa aaaaaaaaaa aaaaa                               1055


<210>  425
<211>  693
<212>  DNA
<213>  Diabrotica virgifera


<220>
<221>  misc_feature
<222>  (1)..(693)
<223>  RNAi target sequence

<400>  425
gggggacatt ttcgtttgta aataacaaat aacagtaaag taaagaataa aaattgaaga      60

attttatatg attcctttaa ttttcgtctt caaatagcat ataagttata ttctgagttt     120

ataaaattaa caccatgcct acgataatat tgctggacgt gtctctttct atgacgaggc     180

ctgttcaagt tggtgagggc tttgaaacag tctcaagaaa gcaactggcg gaattaggga     240

ttaatgcatt tttggattat cttagcatac attccaaatt agagtttata tctttgatgg     300

ctttttcgtc attgtacgag gagaggtgtc cttttaccag agattacaat gctattaaag     360

cagaattaaa gaatatagaa gactatgata aaacttgcat tgaaacagcg cttcatggtg 420

ttaatcacat ggttcttgga gagtggggga ataacacggc ttgtcaggta ttattagtaa 480

cggatggaaa tactggaatt ggaacgaatt ccttgaaaga agcaattgct acaatgaatc 540

aaaggaataa taaaatacca tttcctgttc ctttttcatt cccaggaaaa ttacatgtgg 600

tgtgtctcac ttctccctgt gatttaagtt ttattaaatc taaaccactt tttcagaggt 660

tgattgattt aagtggttac gacggatcaa ttt 693

<210> 426
<211> 21
<212> DNA
<213> Artificial Sequence

<220>
<223> primer

<220>
<221> misc_feature
<222> (1)..(21)
<223> forward primer

<400> 426
gggggacatt ttcgtttgta a 21

<210> 427
<211> 22
<212> DNA
<213> Artificial Sequence

<220>
<223> primer

<220>
<221> misc_feature
<222> (1)..(22)
<223> reverse primer

<400> 427
aaattgatcc gtcgtaacca ct 22

<210> 428
<211> 1551
<212> DNA
<213> Diabrotica virgifera

<220>
<221> misc_feature
<222> (1)..(1551)
<223> WCRW transcript ta10911.001_diavv

<400> 428
ggacattttc gtttgtaaat aacaaataac agtaaagtaa agaataaaaa ttgaagaatt 60

ttatatgatt cctttaattt tcgtcttcaa atagcatata agttatattc tgagtttata 120

aaattaacac catgcctacg ataatattgc tggacgtgtc tctttctatg acgaggcctg 180

ttcaagttgg tgagggcttt gaaacagtct caagaaagca actggcggaa ttagggatta 240

atgcattttt ggattatctt agcatacatt ccaaattaga gtttatatct ttgatggctt 300

tttcgtcatt gtacgaggag aggtgtcctt ttaccagaga ttacaatgct attaaagcag 360

aattaaagaa tatagaagac tatgataaaa cttgcattga aacagcgctt catggtgtta 420

atcacatggt tcttggagag tgggggaata acacggcttg tcaggtatta ttagtaacgg 480

atggaaatac tggaattgga acgaattcct tgaaagaagc aattgctaca atgaatcaaa 540

ggaataataa aataccattt cctgttcctt tttcattccc aggaaaatta catgtggtgt 600

gtctcacttc tccctgtgat ttaagtttta ttaaatctaa accacttttt cagaggttga 660

ttgatttaag tggttacgac ggatcaattt tggtgccaga caatattcat agtgaatcga 720

gcgtaattag tttatttcag aaacttgctg aagacatgta cacaacattt aagggcaccc 780

taaagtgtgg caatttagaa tcaaaaataa tattgtcgcc agctcctgtg gcttatacaa 840

agtttacgga ttttgaatgc aacacatata acatgtcaga actaatagaa gtgtgcggtt 900

tcgtatcagt tacggatgta gggtcaccga tggccattag ccgtcatcta attcttccag 960

ccagttctcc aaccaaacaa gaaagtgttg ccaaaaatga cacggaagcc accgaagatg 1020

acagcactga cgaagcgaaa ataccatcat tttgtgtact acttcatgga gctttaagag 1080

tagagaatat ggcagcattg gtgaatatag gagaaaactg gttcggattc gtatattcct 1140

gggcagattc gaagaaaaag tcaaatttga tgctaactat tttaatgcca ggcactgata 1200

tcataccttg gttaggagat ttaaattgtt tgggatccgc agaatcttat tcgtctgagc 1260

aaataggcag ttttcctatt aaacctatgg agaagaggag ttactctcag aacggagtcg 1320

tttggattcg ccaagcaggt ctgcaatcgg atattcagaa aatattacga catgcaagaa 1380

agttgcccga gaaaactcag caattctata aggaacttaa caggcttaga aaagcagcaa 1440

tatccttagg tttcttggat cttttaaacg gattggctta tatttttgaa caagaatgtt 1500

tacagttacc tccaagtgca catccagatt gtgctgtcca gttacaacat g 1551

<210> 429
<211> 651
<212> DNA
<213> Diabrotica virgifera

<220>
<221> misc_feature
<222> (1)..(651)
<223> RNAi target sequence

<400> 429

gggggctctt agttatccat cttggcttcg tttaggtttt agcttagcac tagcaccacg      60

ttttcgtttt aagttgactg ttgactttgg ccattgtttc aagtgtagtt gtcttatcgt     120

tatcgttaca atttgaaatt tggattgagt ttgagttgtt aggaccagga ccaggtttat     180

aacacaagtg gatatctatt catcatggca attgattggg aaaaaattgt aactgaacta     240

agtgctgtgt tccaagatga gcaagagaca tttgcgagag ttttgtttta tttaggagaa     300

gaagaaaagg tgtataaaga tgccgataaa ctaggaccgc agatgaagat cttaatgtca     360

tctatgaaag tagcatcttt gtttagtcag tacgccaaac ggttagcaga ggacacaaag     420

agggctgatt cggatgaaaa gttcaaacga gtgatgggtc taatctgggc ccaaatgatg     480

gacgctaaca aacagacaga tgacaaagat aaaaccccag atgtaagcat tattgccaaa     540

ttagaacacc tgtctctaga agcttcgcca ggaatggttc gcgccttgag cattattcca     600

tatgaaagcc aaaagcgttt tttagtaatg ctcaaagaca tcgtaccaac a              651

<210>  430
<211>  22
<212>  DNA
<213>  Artificial Sequence

<220>
<223>  primer

<220>
<221>  misc_feature
<222>  (1)..(22)
<223>  forward primer

<400>  430
gggggctctt agttatccat ct                                               22

<210>  431
<211>  22
<212>  DNA
<213>  Artificial Sequence

<220>
<223>  primer

<220>
<221>  misc_feature
<222>  (1)..(22)
<223>  reverse primer

<400>  431
tgttggtacg atgtctttga gc                                               22

<210>  432
<211>  1310
<212>  DNA
<213>  Diabrotica virgifera

<220>
<221>  misc_feature
<222>  (1)..(1310)
<223>  WCRW transcript ta05539.003_diavv

<400>  432
cagagacttc atgctcatgc tccttctctt agttatccat cttggcttcg tttaggtttt      60

agcttagcac tagcaccacg ttttcgtttt aagttgactg ttgactttgg ccattgtttc     120

aagtgtagtt gtcttatcgt tatcgttaca atttgaaatt tggattgagt ttgagttgtt     180

aggaccagga ccaggtttat aacacaagtg gatatctatt catcatggca attgattggg     240

aaaaaattgt aactgaacta agtgctgtgt tccaagatga gcaagagaca tttgcgagag     300

ttttgtttta tttaggagaa gaagaaaagg tgtataaaga tgccgataaa ctaggaccgc     360

agatgaagat cttaatgtca tctatgaaag tagcatcttt gtttagtcag tacgccaaac     420

ggttagcaga ggacacaaag agggctgatt cggatgaaaa gttcaaacga gtgatgggtc     480

taatctgggc ccaaatgatg gacgctaaca aacagacaga tgacaaagat aaaaccccag     540

atgtaagcat tattgccaaa ttagaacacc tgtctctaga agcttcgcca ggaatggttc     600

gcgccttgag cattattcca tatgaaagcc aaaagcgttt tttagtaatg ctcaaagaca     660

tcgtaccaac aacagtgcta tcgaatgtag agtatgttgt tgaaactgtt aacaaagtag     720

gtggtgctgt agtaactgta gctttagcta gtatacaatt ggcatatgaa gctattgtta     780

acattagaag atggtggaat ggagagattt caggcaagag atgttgtaaa aatattatag     840

attcacttgc tacaattggt ggtagcgttg gtggaggatt tgctggtgta gctttgggag     900

cacttggagg tccagttggc atgataatcg gaggtattgg tggaggaatg ttgggcggag     960

aattggccaa gcgtctgtcg caatggatga ctgaaaaggt atttgatgtt ccaaaatctg    1020

aggcgctaga aaatgcttat aatttcttta atttgcgcaa tcatgcttcc aatcatcaga    1080

taaatacagc ttacagacag aagtgtctag aataccatcc tgataaagga ggttctgagg    1140

aaatgttcca taaagtgcag atggaaatgg cggttattaa agctgccaaa ggagaattgt    1200

attcataata attagtttgt atttttgtac ttagatttag attatataac atattttgta    1260

ctatactatt ttatgtacac attatatttg tacagaaata aaaattaacg               1310

<210>  433
<211>  658
<212>  DNA
<213>  Diabrotica virgifera

<220>
<221>  misc_feature
<222>  (1)..(658)
<223>  RNAi target sequence

<220>
<221>  misc_feature
<222>  (546)..(546)
<223>  n can be any nucleotide

<400>  433
atgtttttaa gatgggccag tgccagtcac acattgtgtt taagtttatt ttcgtgtttc      60

ttgctgggta cgaccgagta acgaacaacc taggatatca cgccaaattc acaagtaaca     120

agttccagtc ctggagtagg agcagccctg gcggcgttag gaagtctcgg tggaatcttc     180

ggcaatcact tacaaatgtt aggaagctta ccagctttcc agagtccgca gagcctgcaa     240

cagctgcagc agctggcgat gctgcagcag gctagcggca accagatgaa tccgcaggct     300

cagtttttat ttcaaaatca gggttttcta caagggttcc ctagaacaag tataccttct     360

agacccccac atacaatgca ggtacaacaa atagcacaag ctgcccagca actacagcaa     420

ctgcagaaag tacagcaaaa cacaaaccaa cacaatacca gcattcctca aaatacgacg     480

ttacctcatc aaacgcagac cacgcctcca aacaaccaca atatacctgc cagcatgctt     540

actccnagca cgcctagttc tggtggactt acgccgcagc atcttaaaac accctcaagg     600

atactggaac catctccaga agaaaccaca gatttggaag aacttgaaca gtttgcga       658


<210>  434
<211>  22
<212>  DNA
<213>  Artificial Sequence

<220>
<223>  primer


<220>
<221>  misc_feature
<222>  (1)..(22)
<223>  forward primer

<400>  434
atgtttttaa gatgggccag tg                                               22


<210>  435
<211>  22
<212>  DNA
<213>  Artificial Sequence

<220>
<223>  primer


<220>
<221>  misc_feature
<222>  (1)..(22)
<223>  reverse primer

<400>  435

tcgcaaactg ttcaagttct tc                                              22

<210>  436
<211>  698
<212>  DNA
<213>  Diabrotica virgifera

<220>
<221>  misc_feature
<222>  (1)..(698)
<223>  WCRW transcript ta21806.001_diavv

<220>
<221>  misc_feature
<222>  (566)..(566)
<223>  n can be any nucleotide

<400>  436
ggagtgcata ttgcccgcag atgtttttaa gatgggccag tgccagtcac acattgtgtt      60

taagtttatt ttcgtgtttc ttgctgggta cgaccgagta acgaacaacc taggatatca     120

cgccaaattc acaagtaaca agttccagtc ctggagtagg agcagccctg gcggcgttag     180

gaagtctcgg tggaatcttc ggcaatcact tacaaatgtt aggaagctta ccagctttcc     240

agagtccgca gagcctgcaa cagctgcagc agctggcgat gctgcagcag gctagcggca     300

accagatgaa tccgcaggct cagtttttat ttcaaaatca gggttttcta caagggttcc     360

ctagaacaag tataccttct agacccccac atacaatgca ggtacaacaa atagcacaag     420

ctgcccagca actacagcaa ctgcagaaag tacagcaaaa cacaaaccaa cacaatacca     480

gcattcctca aaatacgacg ttacctcatc aaacgcagac cacgcctcca aacaaccaca     540

atatacctgc cagcatgctt actccnagca cgcctagttc tggtggactt acgccgcagc     600

atcttaaaac accctcaagg atactggaac catctccaga agaaaccaca gatttggaag     660

aacttgaaca gtttgcgaag acgttcaaac agagaagg                             698

<210>  437
<211>  647
<212>  DNA
<213>  Diabrotica virgifera

<220>
<221>  misc_feature
<222>  (1)..(647)
<223>  RNAi target sequence

<220>
<221>  misc_feature
<222>  (524)..(524)
<223>  n can be any nucleotide

<400>  437
aatttcagcc gtcatctgtt gtgttggtgt gtctgtattt ttgtgaattt cgcaaatatt      60

tttgattcta ataacaaaac cttttgctaa attcagtgaa tcatagtgct actttcttgt 120

taaattcata agggaaagaa gcatcaacca acaaaaaatt gtgaatgttc tcctagagag 180

atttcaacta acttcagagc aactctatat cattcaagga aataaggagt ttttattgac 240

tcctgaaata tttaatgtac tagataagat ccaagaaata cgcaatgatt gtaaaatact 300

tatgcagtct ggtcttcaaa cgttggcttt agatattatg gagcaaatga ccacatatca 360

ggaggcaggg ttggaaaaat tatatagatg gacccaaagt cactgtagaa atgttgaaaa 420

ccctgaatta acagatctag ttacacaatc aatggcaagg cttcaacaca gacaagtttt 480

gtttaagtat gtaatcgatg aatactgcat atgtcgcaga agcnagataa ctgatgattt 540

tatcaacgct ctaacacgag gagggccatc tggaaatcct gctcctatag aaatgagagc 600

tcacgattct cagatctatg tcacagatat gctcgtttgg ttgaaca 647

<210> 438
<211> 22
<212> DNA
<213> Artificial Sequence

<220>
<223> primer

<220>
<221> misc_feature
<222> (1)..(22)
<223> forward primer

<400> 438
aatttcagcc gtcatctgtt gt 22

<210> 439
<211> 22
<212> DNA
<213> Artificial Sequence

<220>
<223> primer

<220>
<221> misc_feature
<222> (1)..(22)
<223> reverse primer

<400> 439
tgttcaacca aacgagcata tc 22

<210> 440
<211> 727
<212> DNA
<213> Diabrotica virgifera

<220>
<221>  misc_feature
<222>  (1)..(727)
<223>  WCRW transcript ta21805.001_diavv

<220>
<221>  misc_feature
<222>  (540)..(540)
<223>  n can be any nucleotide

<400>  440
ggcaatcgat accaaaaatt tcagccgtca tctgttgtgt tggtgtgtct gtatttttgt      60

gaatttcgca aatatttttg attctaataa caaaaccttt tgctaaattc agtgaatcat     120

agtgctactt tcttgttaaa ttcataaggg aaagaagcat caaccaacaa aaaattgtga     180

atgttctcct agagagattt caactaactt cagagcaact ctatatcatt caaggaaata     240

aggagttttt attgactcct gaaatattta atgtactaga taagatccaa gaaatacgca     300

atgattgtaa aatacttatg cagtctggtc ttcaaacgtt ggctttagat attatggagc     360

aaatgaccac atatcaggag gcagggttgg aaaaattata tagatggacc caaagtcact     420

gtagaaatgt tgaaaaccct gaattaacag atctagttac acaatcaatg gcaaggcttc     480

aacacagaca agttttgttt aagtatgtaa tcgatgaata ctgcatatgt cgcagaagcn     540

agataactga tgattttatc aacgctctaa cacgaggagg gccatctgga aatcctgctc     600

ctatagaaat gagagctcac gattctcaga tctatgtcac agatatgctc gtttggttga     660

acaaagcgat tgctttggaa aagcagaatg ttagtttatt gttgaaattg tgcaataaag     720

tagagct                                                               727

<210>  441
<211>  711
<212>  DNA
<213>  Diabrotica virgifera

<220>
<221>  misc_feature
<222>  (1)..(711)
<223>  RNAi target sequence

<400>  441
gggggataag ataataaatc gaacatacct gtttcgactc tatgtggaat ttgtcagtgc      60

atggttatgc aatttagtta aaggttaagg ataataatta gtggaaaaaa tgagttgcgg     120

ttttgattca caaagaattt atatgctagg tgaaaaatat tcaggtctaa aagctagagg     180

tgctctccaa aattatgaag aaattagaaa gaaatatctg ctgaacaatg cattattcga     240

agactcacaa tttcaagtag aaaaagtaga actcggacaa gagtgggaca atcataagaa     300

caacattaag tggctccgac cccaggaaat ggtttccgat ccagtttttc tagaagctca     360

tgaagacagg ttcgatatta aacaaggaac tttgggcgat tgctggtttt tagcagcttt 420

ggcaaacata acagacaata ataaaataat gcagcgtatt ataccagaca atcaaagctt 480

ttctcaagat tatgcaggag tatttcattt caggttttgg caatatggta gatggataga 540

cgtggttatt gacgacaaat tacctactat agatggtaaa cttatcttta ctaagaccaa 600

atttagcaac gtattttgga cacctttatt ggaaaaagca tatgcaaaac tgtatggatc 660

ttataaaaat attgaggggg gctttatttc tgaagctctg gaagatttca c 711

<210> 442
<211> 24
<212> DNA
<213> Artificial Sequence

<220>
<223> primer

<220>
<221> misc_feature
<222> (1)..(24)
<223> forward primer

<400> 442
gggggataag ataataaatc gaac 24

<210> 443
<211> 23
<212> DNA
<213> Artificial Sequence

<220>
<223> primer

<220>
<221> misc_feature
<222> (1)..(23)
<223> reverse primer

<400> 443
gtgaaatctt ccagagcttc aga 23

<210> 444
<211> 2478
<212> DNA
<213> Diabrotica virgifera

<220>
<221> misc_feature
<222> (1)..(2478)
<223> WCRW transcript ta05594.001_diavv

<400> 444
gggggataag ataataaatc gaacatacct gtttcgactc tatgtggaat ttgtcagtgc 60

atggttatgc aatttagtta aaggttaagg ataataatta gtggaaaaaa tgagttgcgg 120

ttttgattca caaaaaattt atatgctagg tgaaaaatat tcaggtctaa aagctagagg 180

tgctctccaa aattatgaag aaattagaaa gaaatatctg ctgaacaatg cattattcga 240

agactcacaa tttcaagtag aaaaagtaga actcggacaa gagtgggaca atcataagaa 300

caacattaag tggctccgac cccaggaaat ggtttccgat ccagtttttc tagaagctca 360

tgaagacagg ttcgatatta aacaaggaac tttgggcgat tgctggtttt tagcagcttt 420

ggcaaacata acagacaata ataaaataat gcagcgtatt ataccagaca atcaaagctt 480

ttctcaagat tatgcaggag tatttcattt caggttttgg caatatggta gatggataga 540

cgtggttatt gacgacaaat tacctactat agatggtaaa cttatcttta ctaagaccaa 600

atttagcaac gtattttgga cacctttatt ggaaaaagca tatgcaaaac tgtatggatc 660

ttataaaaat attgaggggg gctttatttc tgaagctctg gaagatttca ccggtggact 720

aaatgaaaga taccttgatt tagcaaacat ttcaaaacat gaggaccttt tcaaaatgat 780

gctaatttca tttatgagaa aatcgtacat gggctgtggt atcctggatt ctggtgaagt 840

tgaaggagaa agaaaagatg gactttattc aagtcatgct tacagtataa acaatgtagt 900

ggaggttacc tcaaaagctg aaaaagtcta taagttgctt aggcttagga atccatgggg 960

cacgggtgag tggacaggcg actggagtga ccattcaaaa acctgggacc ttcttccaga 1020

cgaaactaaa aataagttat tagtgataaa ggatgatgga gaatttggga tgcaacacga 1080

ggatttcatt aaagattata atttgttaga attttgtcac cctaatcccg actcactaga 1140

atatgaagaa gatgaatcac ataaatggaa catttttatg cttgatggca gttgggttgc 1200

aaaaattagt aatggaggtc agccacatag tacaagtttt tggcataact cgcaatattt 1260

gttaaaaata aatgatagta gtaatgctat acacagagta gtaattggat taatgcagaa 1320

gaatagaaga ctgtacaatc ttcccaagat tcctatagga tttgctgtgt atgaactaaa 1380

atccgtttct ggacgtttaa cgcataattt tttctcctca attaaaccta tatatattag 1440

agaaccagag aacaatacac aggttacggg acacatagat ttaccaaaag gatcctactg 1500

cataatacca tttacagatg aaccacaagc aagctgtgaa tttcttttaa gggtttactc 1560

tactgttaca tttgatgtta atgaacacga agtagatgtt cgattgatag atccaacagt 1620

accattagga gggttacgtg cacctgtaga cgcagtagat ggtgcggctg ccgataaagg 1680

aataaaacat gtattcgaga actatgctga taaagaatta gaaataggtt ggaaggaact 1740

agtatctata ctagaacttc ttttctcaac agaacaacac atattttcaa aggaactgtg 1800

tcgaagtatg gttgcattag cggatactga cctttcagga aaactcaatt ttcaggaatt 1860

ctatgagctt tgggaacaaa ttggagaatt aaaaggcatt ttcaaaaatt ttgattcaga 1920

tagttccaac ggaatgtcag tgcgtgaact tagacaggct cttagagtag ctaatttcca 1980

acttagcaat aaaattttat ccctaattgg actgagatac gcaaacgaaa aagatgagtt 2040

ggaattagaa gactttttgc attgttgcgt taaattaaaa ttaatgatag atatttacaa 2100

ctccaaaaag aaaagagaat atggcttgga ggaatggtta cttgaaacaa tgtatgctta 2160

aggtgcaaga atatttttgt tatatttttt attggtttga taaaataaaa tgaatattcc 2220

agtatataaa tatatgagtt gattgaaata tccataatat ttcaatcaac catattttat 2280

aagtatttaa catgtctgta aaaaaaagaa atctaataga acttttaacg ttattattaa 2340

tactgatgat agatggcatc gcatgtacgt gtataaaata aaaattgaat actgcaaatg 2400

aaagagaata tacataatat tatgtacata gtttggaaat agtatatttg tttgaaacga 2460

tttatactaa cagtaatc 2478

<210> 445
<211> 579
<212> DNA
<213> Diabrotica virgifera

<220>
<221> misc_feature
<222> (1)..(579)
<223> RNAi target sequence

<400> 445
cgccattttt gttttcgtag ttggaggcga tgtagggaac tcaatcgatt catacaaaac 60

ttttactgga atgaagcatt tcagcggaaa cctcatgttt ctgtcctgag ccgtttagaa 120

gttgtacgta gttgaaatgg gggaaaatat aagtggacac ccggtataat atgcagatga 180

atgtagaaaa aacacaatat ttatgcctag gtgaagattc aactgatatg atattggaca 240

atataatcag aaaataacta gttgtggtaa atataagtat ttgggaatta atttcagcaa 300

agaagatgcg gacgatgcag aaattaagag cacagtcaat aaagaaagaa cgataattag 360

agatataatt aaatcattga acggagtttt gtggagtacc aagatagaga aacaaagaaa 420

aatgagaata tacaacacca tgattaagag cacattggta tagggatccg aaacatggag 480

agtaaaggaa caccaaaaaa gtaaaataga agctaccgag atggacgccc taagaagagc 540

aagtagaaca tcgagattgg accgggttag aaatgagta 579

<210> 446
<211> 22
<212> DNA
<213> Artificial Sequence

<220>
<223> primer

<220>

<221>  misc_feature
<222>  (1)..(22)
<223>  forward primer

<400>  446
cgccattttt gttttcgtag tt                                                22


<210>  447
<211>  22
<212>  DNA
<213>  Artificial Sequence

<220>
<223>  primer


<220>
<221>  misc_feature
<222>  (1)..(22)
<223>  reverse primer

<400>  447
tactcatttc taacccggtc ca                                                22


<210>  448
<211>  628
<212>  DNA
<213>  Diabrotica virgifera


<220>
<221>  misc_feature
<222>  (1)..(628)
<223>  WCRW transcript ta21896.001_diavv

<400>  448
gtaaattcat tatttgcttt acttactcat ttctaacccg gtccaatctc gatgttctac      60

ttgctcttct tagggcgtcc atctcggtag cttctatttt acttttttgg tgttccttta     120

ctctccatgt ttcggatccc tataccaatg tgctcttaat catggtgttg tatattctca     180

tttttctttg tttctctatc ttggtactcc acaaaactcc gttcaatgat ttaattatat     240

ctctaattat cgttctttct ttattgactg tgctcttaat ttctgcatcg tccgcatctt     300

ctttgctgaa attaattccc aaatacttat atttaccaca actagttatt ttctgattat     360

attgtccaat atcatatcag ttgaatcttc acctaggcat aaatattgtg ttttttctac     420

attcatctgc atattatacc gggtgtccac ttatattttc ccccatttca actacgtaca     480

acttctaaac ggctcaggac agaaacatga ggtttccgct gaaatgcttc attccagtaa     540

aagttttgta tgaatcgatt gagttcccta catcgcctcc aactacgaaa acaaaaatgg     600

cggatttttg aaaaaaactc tgttgccc                                        628


<210>  449
<211>  539

<212>  DNA
<213>  Diabrotica virgifera


<220>
<221>  misc_feature
<222>  (1)..(539)
<223>  RNAi target sequence

<400>  449
ctatatgaga cgaggccaga caccatatgg aggagtacat cttgctgatg gcgttgtagc      60

tgatggttta tgggacgttt acaacaaatt ccatatgggt aactgcacag aaaacacagc     120

aaagaagctg aacatctcca gggctgaaca ggatgagtat gctatcaaca gttacaagaa     180

aagtgccgaa gcatacaaaa acaatgcttt tgcttctgaa ttagtatcag ttaatgtacc     240

acaaaggaaa ggacaaccag atttggtggt ttcagtagat gaagagtaca aaagggttaa     300

ctttgaaaag tttaacaaac tgccaacggt tttccaaaag gaaaacggca cagtaacagc     360

tggaaacgct tcaactttaa acgacggtgc agctgctttg atcttaacca ctgccgaaaa     420

tgccaagagg ttaaacgctg aacctctagc tcgagttgtt ggttaccaag acggtgaatg     480

tgacccaatc gatttcccaa tagcccctgc cgtagctatt ccaaaattgc tcgaaaaga      539


<210>  450
<211>  22
<212>  DNA
<213>  Artificial Sequence

<220>
<223>  primer


<220>
<221>  misc_feature
<222>  (1)..(22)
<223>  forward primer

<400>  450
ctatatgaga cgaggccaga ca                                               22


<210>  451
<211>  22
<212>  DNA
<213>  Artificial Sequence

<220>
<223>  primer


<220>
<221>  misc_feature
<222>  (1)..(22)
<223>  reverse primer

<400>  451
tcttttcgag caattttgga at                                               22

<210>  452
<211>  1541
<212>  DNA
<213>  Diabrotica virgifera

<220>
<221>  misc_feature
<222>  (1)..(1541)
<223>  WCRW transcript ta05758.001_diavv

<400>  452
ttattgtaaa ataagtgttg cagttattta aaatcgtatg tttataattt tttcatatta      60

gttgaacaag ggtagggtca aagtttaatc caaagtggtg gggaaggaga atactttatc     120

acttatttgt tctggaaata ttttgagaat tgtcgttcac ttattttaga agataggctt     180

aaaaatgatc atgtcaaagc caactaaggt tattcaaatg agcagaagat tctacagctc     240

tggctctaaa aacgatattg ttattatttc tgcagttcga actccaatgg gtacgtttct     300

gggaccctta gcacctttat cagctcccag attgggagca gttgccatac aagcagctgt     360

tgaacgagct ggaattccca aagaagaagt caaagaagtc ttcatgggcc atgtttgtca     420

aggaggcgct ggacaggccc cagccagaca ggctacagtt tttgcaggtc ttccaaagtc     480

aacaatatgc acaactgtca acaaagtatg ctcatcaggc atgaaatctg taatgtttgc     540

aacacagagc ttgctactcg gcgaccaaga cgtcatacta gccgggggaa tggaatccat     600

gtcgaatgta ccatactata tgagacgagg ccagacacca tatggaggag tacatcttgc     660

tgatggcgtt gtagctgatg gtttatggga cgtttacaac aaattccata tgggtaactg     720

cgcagaaaac acagcaaaga agctgaacat ctccagggct gaacaggatg agtatgctat     780

caacagttac aagaaaagtg ccgaagcata caaaaacaat gcttttgctt ctgaattagt     840

atcagttaat gtaccacaaa ggaaaggaca accagatttg gtggtttcag tagatgaaga     900

gtacaaaagg gttaactttg aaaagtttaa caaactgcca acggttttcc aaaaggaaaa     960

cggcacagta acagctggaa acgcttcaac tttaaacgac ggtgcagctg ctttgatctt    1020

aaccactgcc gaaaatgcca agaggttaaa cgctaaacct ctagctcgag ttgttggtta    1080

ccaagacggt gaatgtgacc caatcgattt cccaatagcc cctgccgtag ctattccaaa    1140

attgctcgaa aagaccggag tcagcaaaga cgaggttgcc atgtgggaaa tcaatgaagc    1200

gttcagcgtt gttgtcttag ctaaccaaaa aatccttggg ctcaatccag acaaagttaa    1260

catccacgga ggtgctgtca gtttgggcca tcccatcggt atgtctggtg ccagacttat    1320

cactcatctc gttcactctc tcaaggctga agagaaagga gtcggtgcta tctgtaatgg    1380

aggaggagga gcttcttcca tgatgatcga gaaattgtaa gtggatcttg gatctcagaa    1440

ctgaatctta ggtttaagtg caaaaatcat atatttttta agaattttag atgtgcattt    1500

atgaactgat attataattt tataaactat tttaaatcct g                    1541


<210>  453
<211>  603
<212>  DNA
<213>  Diabrotica virgifera



<220>
<221>  misc_feature
<222>  (1)..(603)
<223>  RNAi target sequence

<400>  453
ggggtgtgta tctctgtgct ataagtttag tgtaaatact gtaaccttga aaattttgaa      60

gtaacctgtg ttttctagaa aattaaggaa acatcaatga aactttggat cccaagttga     120

ccttgagcga ttatcagatc accgaaattt acgtggtcgg aaaaggaaca cacagtctta     180

ctcaagcatt ttccagtagc gacattatgg cattaagaaa agaagaagaa aggaaacaaa     240

tgcataacaa gactggagga ggtgtattca acttaatttt taggagagga aaatcgagta     300

tgggttcggg ttcaatctcc agcgatacac gttctatttc gccgactcac agcgacgatt     360

cccgctcagt tacaccacct gcagtcattc agcaacctat tctgccaccg ccgaagcaag     420

atccgcctcc cgaacgaccg aaacctccac aacgtaaacg gagacccgct ccgagacctc     480

cgcagctaac ccctccacag caggagatac ctaaggagga aactgccatt gatgctgaag     540

attctaaaga atctcgtcga aattcggaag aaaaaccagc gagcaccgtt tcagaagtta     600

gtg                                                                   603


<210>  454
<211>  22
<212>  DNA
<213>  Artificial Sequence

<220>
<223>  primer


<220>
<221>  misc_feature
<222>  (1)..(22)

<400>  454
ggggtgtgta tctctgtgct at                                               22


<210>  455
<211>  22
<212>  DNA
<213>  Artificial Sequence

<220>
<223>  primer

<220>
<221>  misc_feature
<222>  (1)..(22)
<223>  reverse primer

<400>  455
cactaacttc tgaaacggtg ct                                              22


<210>  456
<211>  736
<212>  DNA
<213>  Diabrotica virgifera


<220>
<221>  misc_feature
<222>  (1)..(736)
<223>  WCRW transcript ta14242.001_diavv

<220>
<221>  misc_feature
<222>  (627)..(627)
<223>  n can be any nucleotide

<220>
<221>  misc_feature
<222>  (629)..(629)
<223>  n can be any nucleotide

<220>
<221>  misc_feature
<222>  (630)..(630)
<223>  n can be any nucleotide

<220>
<221>  misc_feature
<222>  (631)..(631)
<223>  n can be any nucleotide

<400>  456
ggtgtgtatc tctgtgctat aagtttagtg taaatactgt aaccttgaaa attttgaagt      60

aacctgtgtt ttctagaaaa ttaaggaaac atcaatgaaa ctttggatcc caagttgacc     120

ttgagcgatt atcagatcac cgaaatttac gtggtcggaa aaggaacaca cagtcttact     180

caagcatttt ccagtagcga cattatggca ttaagaaaag aagaagaaag gaaacaaatg     240

cataacaaga ctggaggagg tgtattcaac ttaattttta ggagaggaaa atcgagtatg     300

ggttcgggtt caatctccag cgatacacgt tctatttcgc cgactcacag cgacgattcc     360

cgctcagtta caccacctgc agtcattcag caacctattc tgccaccgcc gaagcaagat     420

ccgcctcccg aacgaccgaa acctccacaa cgtaaacgga gacccgctcc gagacctccg     480

cagctaaccc ctccacagca ggagatacct aaggaggaaa ctgccattga tgctgaagat     540

tctaaagaat ctcgtcgaaa ttcggaagaa aaaccagcga gcaccgtttc agaagctagt     600

gagttgcagt cgaaaaaaat cgaaacngnn ntagtcatct gtcattccgg aaatagttca 660

gatagttcag gataccatga agcatcagtt ttaagtgaca actgcaacac gtcgttgcca 720

cgaaggccaa aatcgg 736

<210> 457
<211> 519
<212> DNA
<213> Diabrotica virgifera

<220>
<221> misc_feature
<222> (1)..(519)
<223> RNAi target sequence

<400> 457
cctcgttggt ggagtagtca attcagcatt gtacaatgtt gatggaggtc acagggcagt 60

tattttcgac aggttcgccg gtattaagaa acaagttata ggcgaaggta cacatttctt 120

tataccttgg gtacagaaac ctattatctt tgatgtacgg tctcgcccta gaaatatccc 180

ggtaataaca ggtagcaaag atctgcaaaa cgtaaatatc acactccgta ttctcttcag 240

accagttcca gacgaattac caaagatcta cacaatttta ggccaggatt atgaagaaag 300

agttttacca tcaatcacta atgaagtcct aaaagctgtg gtggcacaat tcgatgccag 360

tgaactgatt actcagagag atgtagtttc ccaaaaagtc agtgaagatt taactgaaag 420

agctactcaa tttggtgtta tccttgatga tatatcatta acccacttaa cctttggtag 480

ggaattcaca caagctgtag aattgaaaca agttgccca 519

<210> 458
<211> 22
<212> DNA
<213> Artificial Sequence

<220>
<223> primer

<220>
<221> misc_feature
<222> (1)..(22)
<223> forward primer

<400> 458
cctcgttggt ggagtagtca at 22

<210> 459
<211> 22
<212> DNA
<213> Artificial Sequence

<220>
<223> primer

<220>
<221>  misc_feature
<222>  (1)..(22)
<223>  reverse primer

<400>  459
tgggcaactt gtttcaattc ta                                                22


<210>  460
<211>  1170
<212>  DNA
<213>  Diabrotica virgifera


<220>
<221>  misc_feature
<222>  (1)..(1170)
<223>  WCRW transcript ta06694.001_diavv

<400>  460
cacaaatcta aatcggcatt ttgatcatca attttgtggt tttttattat aaattcagct      60

gttaggttga aaaatatttc agtctaaaaa ttacgttata aaagacatcg aaatcttcga     120

tcgattataa ccataaaagc attttagttt gtttgcattt ttacataact taacctctca     180

tagtgaactt cacatgtaaa gatgtctgca gctcaaattt tcaacagaat cggccaagtt     240

ggtctaggaa tagccctcgt tggtggagta gtcaattcag cattgtacaa tgttgatgga     300

ggtcacaggg cagttatttt cgacaggttc gccggtatta agaaacaagt tataggcgaa     360

ggtacacatt tctttatacc ttgggtacag aaacctatta tctttgatgt acggtctcgc     420

cctagaaata tcccagtaat aacaggtagc aaagatctgc aaaacgtaaa tatcacactc     480

cgtattctct tcagaccagt tccagacgaa ttaccaaaga tctacacaat tttaggccag     540

gattatgaag aaagagtttt accatcaatc actaatgaag tcctaaaagc tgtggtggca     600

caattcgatg ccagtgaact gattactcag agagatgtag tttcccaaaa agtcagtgaa     660

gatttaactg aaagagctac tcaatttggt gttatccttg atgatatatc attaacccac     720

ttaacctttg gtagggaatt cacacaagct gtagaattga aacaagttgc ccaacaggaa     780

gctgagaaag ctaggttttt ggtagaaaaa gctgaacaga caaagaaagc aactgtaatc     840

tcagcggagg gtgatgccca agctgctgtg cttcttgcca aggcttttgg tgatgctgga     900

gaaggtttag tagaactgag acgtatagaa gcagctgaag atatcgcata ccaactatca     960

aggtcaaggc aggtttccta cttgcctgga ggacaaaatt tgttgttgaa tgtgccaaca    1020

cctggtaatt agatggatag agtaacaaac aaaattggag ctagttgatt atttggctct    1080

atgaatgaga gagttgccca tttagctttt atggttctat tattttttat aaaagtatgt    1140

taataaattg ttgataaatt ttaaaaaaaa                                      1170

<210>  461
<211>  602
<212>  DNA
<213>  Diabrotica virgifera

<220>
<221>  misc_feature
<222>  (1)..(602)
<223>  RNAi target sequence

<400>  461
gggaagactc ccaagatatg gtcgacgagt ccacagatca cgtttccaga acgaacgaaa      60

aatcgacgat attcgaccag gttaaacagt ttttaaagaa taaccatcaa attcctggca     120

tggcagattt agaagctaag caagaagaag ttttaaaaca gcttgcagac ctcaagaaac     180

aaattctctc aattaaattg gatttgaaaa ttaatgcaaa ctcagtagga acaaacaaga     240

cctgtccaag tatttcagca gaaaagctga atgctcttcc tgatcttgtt gtgaatgcaa     300

gtccaacaaa tccgccatat tcattactga taattcaaaa gctactacgt gacattgtcg     360

aattgaaggt tacatcccat atacactcct cagttccaac attgaatgaa aatgccaaac     420

agctgggtga ctcattggca agttacgtac cgaaacgtgg tgtaccaagt gttaacatta     480

aattgatatg gaaaaatgtg ggcatcgaca cagaactcat agtatcacac gttccaattt     540

ttggagaagt caacttatta cggtatctaa ccagggtaat cagttcccca ctcagataca     600

gt                                                                    602

<210>  462
<211>  20
<212>  DNA
<213>  Artificial Sequence

<220>
<223>  primer

<220>
<221>  misc_feature
<222>  (1)..(20)
<223>  forward primer

<400>  462
gaagactccc aagatatggt                                                  20

<210>  463
<211>  23
<212>  DNA
<213>  Artificial Sequence

<220>
<223>  primer

<220>
<221>  misc_feature
<222>  (1)..(23)
<223>  reverse primer

<400>  463
actgtatctg agtggggaac tga                                              23


<210>  464
<211>  1170
<212>  DNA
<213>  Diabrotica virgifera


<220>
<221>  misc_feature
<222>  (1)..(1170)
<223>  WCRW transcript ta07588.001_diavv

<400>  464
taattatgta attaggctga gtaaatatga agttttgcct cttttaagga taacatacaa      60

tatcatctgg accccttttt aagaacgcca gtgaaaaatt atttagcaga cgaagactga     120

ttaccaaaaa aattatatat cgaatttcta aagttaattg taacatttta gcgaagaaaa     180

atgaatgggc cgataaaaat gtatcgaaca cgacaaataa ttcaccacga ccaaaagatt     240

gacaagccga agtgtatgta tgctttaaaa aatatctact caaaagactc ccaagatatg     300

gtcgacgagt ccacagatca cgtttccaga acgaacgaaa aatcgacgat attcgaccag     360

gttaaacagt ttttaaagaa taaccatcaa attcctggca tggcagattt agaagctaag     420

caagaagaag ttttaaaaca gcttgcagac ctcaagaaac aaattctctc aattaaattg     480

gatttgaaaa ttaatgcaaa ctcagtagga acaaacaaga cctgtccaag tatttcagca     540

gaaaagctga atgctcttcc tgatcttgtt gtgaatgcaa gtccaacaaa tccgccatat     600

tcattactga taattcaaaa gctactacat gacattgtcg aattgaaggt tacatcccat     660

atacactcct cagttccaac attgaatgaa aatgccaaac agctgggtga ctcattggca     720

agttacgtac cgaaacgtgg tgtaccaagt gttaacatta aattgatatg gaaaaatgtg     780

ggcatcgaca cagaactcat agtatcacac gttccaattt ttggagaagt caacttatta     840

cggtatctaa ccagggtaat cagttcccca ctcagataca gttctgactc tgatagtgta     900

gaaatcgatt cgatattaga cacttgttat cttcttctga gagctaaaac gaaaactgaa     960

aaggcaggac atattgccac attaaataaa tccttaggaa aatcccaatg gttagcaggt    1020

agaagtaggc atagtctggg ggatctagcc gcatactctg cccttaaaca ggttacaagt    1080

ggtagtgaaa ttaatgcaaa tatgacgaag tggtatcagc ggtgtgaaac tgttgtataa    1140

aattattttt gttataaata taagctttaa                                     1170


<210>  465

<211>  508
<212>  DNA
<213>  Diabrotica virgifera


<220>
<221>  misc_feature
<222>  (1)..(508)
<223>  RNAi target sequence

<400>  465
caaaacgaat gtgttgagtg tgtttttgtg gatatggttc tggttaataa ggcgcttgat      60

ttatctcata gataacataa tttttgttaa aactggtata actgctgatt attattattc     120

tctttgtcaa caagagtgtt acgtatagtg ttatgtatag aacattcacg aagggttgct     180

tcagcttccg gtcagaccat gcccgtctta cccacgaccc acgatgaggt agcaagacat     240

gatttcgaca ccgtcaccat cgattcatat ggaatgagcc ccttggcctt gcagaaaatt     300

agggagcaga tggctttgag cctggagcga accaagcagt tggaggacca gatcaagagc     360

atgcacaaat tggaggagga ggtcaacttc ctgaaggatg aaaattgtag gttacgaaac     420

agtttgaaat cagaaaacgg aaaaaaccaa gccacaataa atgacaatta tatcgaacat     480

tacaaacgac agaggtctca atcattca                                        508


<210>  466
<211>  22
<212>  DNA
<213>  Artificial Sequence

<220>
<223>  primer


<220>
<221>  misc_feature
<222>  (1)..(22)
<223>  forward primer

<400>  466
caaaacgaat gtgttgagtg tg                                               22


<210>  467
<211>  22
<212>  DNA
<213>  Artificial Sequence

<220>
<223>  primer


<220>
<221>  misc_feature
<222>  (1)..(22)
<223>  reverse primer

<400>  467

tgaatgattg agacctctgt cg                                              22

<210>  468
<211>  3569
<212>  DNA
<213>  Diabrotica virgifera

<220>
<221>  misc_feature
<222>  (1)..(3569)
<223>  WCRW transcript ta01520.001_diavv

<400>  468
ggggaactgt gttttgttca acaaaaaata agtacctgtc acaaagtaca tatatggaga      60

acataaacaa aacgaatgtg ttgagtgtgt ttttgtggat atggttctgg ttaataaggc     120

gcttgattta tctcatagat aacataattt ttgttaaaac tggtataact gctgattatt     180

attattctct ttgtcaacaa gagtgttacg tatagtgtta tgtatagaac attcacgaag     240

ggttgcttca gcttccggtc agaccatgcc cgtcttaccc acgacccacg atgaggtagc     300

aagacatgat ttcgacaccg tcaccatcga ttcatatgga atgagcccct tggccttgca     360

gaaaattagg gagcagatgg ctttgagcct ggagcgaacc aagcagttgg aggaccagat     420

caagagcatg cacaaattgg aggaggaggt caacttcctg aaggatgaaa attgtaggtt     480

acgaaacagt ttgaaatcag aaaacggaaa aaaccaagcc acaataaatg acaattatat     540

cgaacattac aaacgacaga ggtctcaatc attcacaaac gtaaacaatt tggagatctc     600

aatgcgaaaa gaatcatccc caccacctcc accaccacga aaagattttg gagtgatgtg     660

tggggtgttg acgaggaaca taggagtagg caatcagaca cctaacacaa agaatgtttc     720

tacggctact tttgaaaatg aattcgctga taagtggctg ccagaaaagg tcaaatttct     780

taacaatcag aacttgaagt catctagtaa aatcacgata tcaaaagaaa cgcaaaccct     840

aggacaaaag tcaagctcag ataaaggtgt ccaattccaa acaaagagtc ctacagtgcc     900

taagttcagc acctacacgc aaactacaac aactaaaaaa caactagtgg ataaagggat     960

cttagcaact gtcaaagtat ctcatatttc tgtccagaaa tgtgttgaat gttactctat    1020

tggcacttca gatgacacag taaatgatat catttgcgaa aaatgcaatg ctacaaaaaa    1080

gatctatagc aataggtcct gaatctaatg aagatagcca tacgtcgcct atatccttag    1140

cgtcccttaa cttaagaagc aaatcgttca atatgggcga agatcgactt aaccttacca    1200

ctagaacccg ttccatagca tcacagtacg aatctagtag catcagcaga gcttgtcagg    1260

cgaaacttaa gaaggattct tctacgaaat cttgccaagc tgaaacgaaa atgactgaca    1320

aaaacaccca gtacgtaaat tacactgtct caaggcatac cgacactcaa gactttataa    1380

caccaaagaa aaacgtcagt tgcgaagcag ttgaacaaaa accggaaacc attgaggtgg    1440

cttgtaatac cgcgactgat ttgctgacct gcgtcaaatg tgaaaaagaa aaagaaaagg 1500

aggcgctgaa gaaagacgga ggcagcccca ctccatctag aataccgcga ccacaaattc 1560

cgactactcc cgtagaaaat agaaagttta ggagacaaga tacttatact aaagtatatt 1620

caccaactga gaaagctaca tcacctacta gtccaagatt gtctggttta gatctatcac 1680

ctaaagtcat atcaaacgat tcaaatccct tagataaagt taaaagtaac ctcaaactag 1740

tagaaccaat accacatgct caaaatggtg tcaatttcac agcgcctatt accaaagttc 1800

aaattcaaga aattacactg ccaacttcag aatctgtttt ccaacccatt acacaaaatc 1860

aaaataggaa aaaagtcatg ccaagtaagg aaatgcaggg tgcaatgaaa gtcctgaacg 1920

atcatcttat gaagaaccct tccaaaaaca tcaagaaccc aactgctgaa aatattattc 1980

aaacagaatg gttcaaaatc tcgagctcag taacagccaa tccattggaa gtggaggatt 2040

acctggatgc tttcgaagac gtgtccagct tgttactgga atacatagta aacatgagtg 2100

attatagtgg gaacacagcc atgcactatg ctgtttccca tggcaacttt gatgtcgtat 2160

ccattttgtt agattctaaa gtttgtgata tcaataaaca aaataaggct ggatatacaa 2220

gtgttatgtt agtttctttg gcggaagttc ggtcccaaac acatgccaac gttgtccgaa 2280

gactgttcca gttagctgat gtgaatatta gagcgaaaga gcatggtcaa acagcactga 2340

tgttagcagt aagccacaat aggttgaaca tggtcaagat gttgttggaa gcaggtgcag 2400

atataaatat tcaggacgaa gatggcagta cggcgttaat gtgtggtgct gaacacggcc 2460

atatcgaaat agttaaacat ttcctttccc aacctgactg caattcgacc attatggata 2520

tggatggcag cacagctcta gcaatagcta tggaagccgg tcaccgacac atcggagtcc 2580

tgctctacgc tcatgagagg aatagccaaa agtctcataa cgtgaagtta aaaaaagcaa 2640

aatcaacaaa tagtccgaaa accccttcct caccactggc tgcaagaagt agccatcgtg 2700

ccttaacaga tacgaaacct aaatagtact aacccatttc taactgtgtt ttaacttaag 2760

aaggtaatgc gtgttgtttc gctgttaacg attctgttaa ccatcgtgat gtattcatat 2820

acgatggttc tagatttttg gcatgtaaaa tgcatgttta ttcagtgtca tcaagatagg 2880

gaacccaaag ttattcatcc ggtccaaaca actctatcta gatgaacata ttcttatgta 2940

taatggaaaa gaatcttagt cgatatatgg cctggcgcaa atttgaccca accgagacac 3000

aatacactcc ggggccacgg atgacccttg catttatttt tctactgcag cgcatattga 3060

acccaaccgt gggctgtcgc cgtggccagt tgctaaaatg tagtgatgtt gattatagtt 3120

attttcaagt atctatgtat tcgttacttt tgtgtaaagt aataatttac attattcgtt 3180

acattgatta ctatgaatac ttcgaatatt tatgtatttc actaacagta atcatataga 3240

aaatcggaat tcctcagtat tttgaatata ccttacaata ttgtaacaca agtgagcgct 3300

tccaacaaat ttaaacatat tttaagtgat atgcgttact attgataata caaaaataaa 3360

aaccgtgaat acataaaaac aatacaatat ataataaaac aatgaaatac ataaaaacaa 3420

tgaaatacat gtaaaattaa agttgttgat tatagtgtag atacattatc atttccacat 3480

atttttagtc aatctaaagc tattttttat ctaagactaa taatatacaa ccgcgtaacc 3540

aggatgatcc taaggggggg ggggttacg 3569

<210> 469
<211> 525
<212> DNA
<213> Diabrotica virgifera

<220>
<221> misc_feature
<222> (1)..(525)
<223> RNAi target sequence

<400> 469
gggaaccatc tgataccatt gagaatgtca aagctaagat ccaagataag gaaggtatcc 60

caccagacca gcaaagattg atctttgcag gaaaacagct agaagatggt agaaccttgt 120

ctgactataa catccagaaa gagtccactc ttcacttggt actgagattg agaggaggta 180

tgcagatctt cgtcaagaca ctaactggaa agactatcac tttggaagta gaaccatctg 240

atactattga gaatgtgaaa gctaagatcc aagataagga aggtatccca ccagaccagc 300

aaagattgat ctttgcaggg aaacagctag aagatggtag aaccttgtct gactataaca 360

tccagaaaga gtccactctt cacttggtac ttcgattgag aggaggtatg cagatcttcg 420

tcaagacact aactggaaag actatcactt tggaagtaga accatctgat accattgaga 480

atgtgaaagc caagatccaa gataaggaag gtatcccacc agatc 525

<210> 470
<211> 20
<212> DNA
<213> Artificial Sequence

<220>
<223> primer

<220>
<221> misc_feature
<222> (1)..(20)
<223> forward primer

<400> 470
gaaccatctg ataccattga 20

<210> 471
<211> 22
<212> DNA
<213> Artificial Sequence

<220>
<223>  primer


<220>
<221>  misc_feature
<222>  (1)..(22)
<223>  reverse primer

<400>  471
gatctggtgg gataccttcc tt                                                22


<210>  472
<211>  703
<212>  DNA
<213>  Diabrotica virgifera


<220>
<221>  misc_feature
<222>  (1)..(703)
<223>  WCRW transcript ta21603.001_diavv

<400>  472
gcacgaggct tggtacttag attgagagga ggtatgcaga tttttgtcaa gacattgact      60

ggtaaaacta tcacattaga aggggaacca tctgatacca ttgagaatgt caaagctaag     120

atccaagata aggaaggtat cccaccagac cagcaaagat tgatatttgc aggaaaacag     180

ctagaagatg gtagaacctt gtctgactat aacatccaga aagagtccac tcttcacttg     240

gtactgagat tgagaggagg tatgcagatc ttcgtcaaga cactaactgg aaagaccatc     300

accttagaag tagaaccatc agataccatt gagaatgtga aagctaagat ccaagataag     360

gaaggtatcc caccagacca gcaaagattg atctttgcag ggaaacagct agaagatggt     420

agaaccttgt ctgactataa catccagaaa gagtccactc ttcacttggt acttcgattg     480

agaggaggta tgcagatctt cgtcaagaca ctaactggaa agactatcac tttggaagta     540

gaaccatctg ataccattga gaatgtgaaa gccaagatcc aagataagga aggtatccca     600

ccagatcagc aaagattgat ctttgcaggt aaacaattgg aagatggaag aaccttatct     660

gattataaca tccagaaaga gtccactctt catttggtac tta                       703


<210>  473
<211>  653
<212>  DNA
<213>  Diabrotica virgifera


<220>
<221>  misc_feature
<222>  (1)..(653)
<223>  RNAi target sequence

<220>

<221>  misc_feature
<222>  (623)..(623)
<223>  n can be any nucleotide

<400>  473
gatcagctaa gaggctgaca ccacgccatt ttgctttgag aacacgtttt tggattttat      60

atacagtaaa ttagtctatt aatttatcaa aatgtctgga gaagaaacat ctgccgatag     120

aaatgttgag atatggaaaa taaagaaact tataaaaagt ttggaactag caagagggaa     180

tggaacaagt atgatctcac ttataattcc tccaaaagat caaatatcta gagtaagcaa     240

aatgttagct gatgaatttg gtacagcctc taatattaag tctcgagtaa atagactttc     300

agttcttggt gctatcacat cagtccagca ccgtttaaaa ctatatacca aggtccctcc     360

taatggatta gtcatttact gtggaactat tgtaactgat gaaggaaagg aaaagaaagt     420

aaacatagac tttgaaccat tcaaaccaat taatacctca ttgtatttat gtgacaataa     480

gttccataca gaagccctga cagcattatt ggcagatgat aataaatttg gttttatagt     540

tatggatggt aatggtgcat tattcggtac actacaagga aacacaaggg aggtacttca     600

caagtttact gtagatttac ccnagaaaca cggtagagga ggtcagtctg cac            653


<210>  474
<211>  22
<212>  DNA
<213>  Artificial Sequence

<220>
<223>  primer


<220>
<221>  misc_feature
<222>  (1)..(22)
<223>  forward primer

<400>  474
gatcagctaa gaggctgaca cc                                               22


<210>  475
<211>  22
<212>  DNA
<213>  Artificial Sequence

<220>
<223>  primer


<220>
<221>  misc_feature
<222>  (1)..(22)
<223>  reverse primer

<400>  475
gtgcagactg acctcctcta cc                                               22

<210>  476
<211>  2829
<212>  DNA
<213>  Diabrotica virgifera

<220>
<221>  misc_feature
<222>  (1)..(2829)
<223>  WCRW transcript ta00933.001_diavv

<400>  476
aaacaattta gggtggattt gaaattaaat ttaatcaaaa tttggtggac aaaataaaat      60

cccaattttc aaatttttgt tttttatgac agatatctga actgaattat caattggata      120

tgccaaatca cctaaacaga atattgttct ttacctgaat ttacagttaa ggccgtgaat      180

atccagttaa aaccagttgg tttcagttaa taactagtga aaagtgactt tcctggatcc      240

tcttgttgat cagctaagag gctgacacca cgccattttg ctttgagaac acgtttttgg      300

attttatata cagtaaatta gtctattaat ttatcaaaat gtctggagaa gaaacatctg      360

ccgatagaaa tgttgagata tggaaaataa agaaacttat aaaaagtttg gaactagcaa      420

gagggaatgg aacaagtatg atctcactta taattcctcc aaaagatcaa atatctagag      480

taagcaaaat gttagctgat gaatttggta cagcctctaa tattaagtct cgagtaaata      540

gactttcagt tcttggtgct atcacatcag tccagcaccg tttaaaacta tataccaagg      600

tccctcctaa tggattagtc atttactgtg gaactattgt aactgatgaa ggaaaggaaa      660

agaaagtaaa catagacttt gaaccattca aaccaattaa tacctcattg tatttatgtg      720

acaataagtt ccatacagaa gccctgacag cattattggc agatgataat aaatttggtt      780

ttatagttat ggatggtaat ggtgcattat tcggtacact acaaggaaac acaagggagg      840

tacttcacaa gtttactgta gatttaccca agaaacacgg tagaggaggt cagtctgcac      900

ttcgttttgc ccgtctgaga atggaaaaac gtcataacta tgttcgaaaa gtagcagaag      960

tagctacgca attatttata acaaatgaca aacctaacat agctggactc atcttggcgg     1020

gtagtgccga ttttaagact gaacttagtc aatctgatat gtttgatcca aggttacaag     1080

ctaaagtaat caaactagta gatgtctcgt atggtggtga gaatggattt aatcaagcca     1140

tcgaattagc agctgaatca cttcaaaatg taaagtttat tcaagaaaag aaactgatag     1200

gcagatattt cgatgaaatt agccaagata cggggaaata ctgttttgga gttgaagata     1260

ctcttagggg cttggaatta ggtgctgtag aaacactaat ctgttgggag aatttagata     1320

ttcagcgata tgtattgaaa aaccatgcta caggttcgga aactatactg catttaactc     1380

ctgaacaaga aaaagataaa tcacattttt cggataaaga gactggtgta gaattggaat     1440

tggtcgagtg tcaacctctt cttgaatggt tggcgaataa ttacaagaac ttcggtgcca     1500

ctttagaaat tattacagac aaatctcaag agggctccca atttgttcga gggtttggtg 1560

gaattggagg tttgctccgg tacaaagtag acttccaaag tttacaacta gacgatatgg 1620

ataacggcga agaatttgat ctagatgatt attaaatgta gagacaaatt tgtagaaatt 1680

ttcatttaaa acaatgtggg cccgttatgt aataaacacg tgtaaaaagt aaatgatggc 1740

aatatactaa tttatcctaa aggagtataa tatgatgtgc ttttttgtaa tattatttcc 1800

aaatttatgc tttcacaaac ttgaaagttt attccatgaa aagttttaat tatatcatta 1860

aattaataag tattattgat taattttatt tattgattaa tctatgttag taaaatgtac 1920

aaagcacaaa gataaagata gttaggataa ccctttacac caactgaaag tatagaaatt 1980

tccggattgt ttggtacggc aacaaacaaa ttcacacgaa attccaaatt tgataatgtc 2040

tactacagtt gaagatgaca atagataatt ccagaccacg acctatacgc tcagaaaaca 2100

atgtaccatt acaaaacggc tgcgaaagcc tacattcata cttgattcgt atacatactg 2160

tccatgggcg ctcataccca tgggcagggt agggggcgtg gccccctggc ttttcgggtg 2220

ctttaaacta tatgtcgtta tccaaagtat acatacctaa ggtaatgtgc aacatcttca 2280

cgtctgccct ccccctaaaa atttttatat gggcgcccgt gatactgtcc taagttacac 2340

atccattttt gtccacgtgt gtgaccagga gcgccattac caacggttcc caagactgag 2400

agtcatagct tactcggagc agtagggatt taaagaagaa aaaacgagct atgtacttgt 2460

cagtgtgtcg gatcacacac agatcggttt agtagtttgt ataaattata taaaataaaa 2520

atgtagattt ggaaataata ttacaataat atcggagtga tcatgtgttt tgtataaatt 2580

atattgtctc aaaaaatgtt taccgatatt tattatttaa ttgttaaaat tatgtatgta 2640

tattgcaagt gtttataatt aatagtgaaa aagaccagta tttctacagt ggcttgtttt 2700

tttcattggt ttttatatat ttagaacaga atattttttg tatttacttt ttttgttaac 2760

gaggtaataa atcaggtttg gaaattcttc attctatttt agtttgtaac cattggcatt 2820

tttgttttt 2829

<210> 477
<211> 577
<212> DNA
<213> Diabrotica virgifera

<220>
<221> misc_feature
<222> (1)..(577)
<223> RNAi target sequence

<400> 477
ctgactctgt tcattgggga gtggttgaaa ggggtgtttt ttggagttat tccattattt 60

gaaggagtga taaagaagat cttggaagcc accatagact taattcacat agaaccagca 120

tactttctga tcaccaagaa gaaatgactg agtattggtt gatatctgcc ccaggggata 180

agacctgtca acagacatgg gacacgatga ataatatgac aagtaaacag aataatttgt 240

caaccaacta caagtttcaa atcccggact tgaaagttgg tactctggat cagttagtag 300

gactttctga tgatctgggc aagcttgatg ggttcgtgga gcaggttacc aggaaggtag 360

ctcagtacct tggagaagta cttgaagaac agagggacaa gtggtcagaa aacctgcagg 420

ccaataacag cgatttgccc acttatttaa cccgatttac ttgggacatc gccaaatatc 480

ccatcaagca atcgctccgt aacatcgccg atataatcag caaacaagtt ggccaaattg 540

acgctgatct gaagaccaaa tcgtctgcgt acaataa 577

<210> 478
<211> 22
<212> DNA
<213> Artificial Sequence

<220>
<223> primer

<220>
<221> misc_feature
<222> (1)..(22)
<223> forward primer

<400> 478
ctgactctgt tcattgggga gt 22

<210> 479
<211> 22
<212> DNA
<213> Artificial Sequence

<220>
<223> primer

<220>
<221> misc_feature
<222> (1)..(22)
<223> reverse primer

<400> 479
ttattgtacg cagacgattt gg 22

<210> 480
<211> 2409
<212> DNA
<213> Diabrotica virgifera

<220>
<221> misc_feature
<222> (1)..(2409)
<223> WCRW transcript ta01215.004_diavv

<400>  480
ggtgaaactt caataacgat cagctgggct gactctgttc attggggagt ggttgaaagg      60

ggtgtttttt ggagttattc cattatttga aggagtgata aagaagatct tggaagccac     120

catagactta attcacatag aaccagcata ctttctgatc accaagaaga aatgactgag     180

tattggttga tatctgcccc aggggataag acctgtcaac agacatggga cacgatgaat     240

aatatgacaa gtaaacagaa taatttgtca accaactaca agtttcaaat cccggacttg     300

aaagttggta ctctggatca gttagtagga ctttctgatg atctgggcaa gcttgatggg     360

ttcgtggagc aggttaccag gaaggtagct cagtaccttg gagaagtact tgaagaacag     420

agggacaagt ggtcagaaaa cctgcaggcc aataacagcg atttgcccac ttatttaacc     480

cgatttactt gggacatcgc caaatatccc atcaagcaat cgctccgtaa catcgccgat     540

ataatcagca aacaagttgg ccaaattgac gctgatctga agaccaaatc gtctgcgtac     600

aataacttga aaggaagtct tcagaatttg gagaagaaac agacaggaag tttactaaca     660

agaaacttgg cagatttggt gaaaaaggaa cattttattt tggattctga atatttgcag     720

acattgttgg ttattgttcc aaaagcccaa ttcaacgaat ggaatgcgac atacgaaaag     780

attaccgaca tgatagtccc tcgttcttca caactgatta aacaggataa cgaatacggt     840

ctatatactg tatccctatt caaaaaggtc gtcgaagaat tcaagttaca cgctagagaa     900

aagaagttca tcgttcgtga tttcatatat aacgaagagg aactggcagc tggaaagaac     960

gagataacca aactcgtcac cgataaaaag aagcaatttg gtccattagt tagatggctt    1020

aaagtcaact tcagcgagtg tttctgcgcc tggattcacg ttaaagcgtt gagggtattt    1080

gttgaatctg ttttaagata tggcctcccc gtcaatttcc aagctatttt aatccacccc    1140

aataagaaac aattaaaacg tttaagagat gtcttgaacc aactttacgg ccacctcgat    1200

agtagtgctg ccctttcagg acctaacgtt gatagcgttg acattccagg cctcggattc    1260

ggccaatcag agtattaccc atacgtttac tacaagctga acgtcgacat gttagaatcg    1320

aagatctaaa ctcacgcatc aatccaagag tttgttatta ataggttcca aacaatttta    1380

aaaaaacgta ctacagatac aaatagtata tttttgatta ctcactcttt cgaggggctg    1440

ttttaatgtc ggtattttta agttttgtcc ttactcaatg tccatttagt accccgtcat    1500

tgtatgtaaa ttaattaaaa atgcgcaaca aaaaatgtag aacttttttt tattttaaaa    1560

tgcatttaaa attcgtttaa cgatgatata attgctttaa tgtatcatgt aatactccga    1620

ttgaacatat aaatcgttat taatattatt tcttgttgat aatacaaaag agaccatatt    1680

attagaaact gcattcgaaa aattaaaaaa atgatagata ttatgacgcg tagatgagct    1740

gacaagtgac gtgttaagat gtattgatat ctaaacatga aatttcaaca aaccatctat    1800

gttatagatg tacatatgtt tcgtaaaatt gacgaaaagc tggaaataga ttacatataa    1860

tttgtcataa aatatttgcg tcagagactt gaggttgtga tacaatatat atttactcct 1920

acagaatatt tgttgaatat taccgacacc attaaaatac cttatccgtt atgattgtag 1980

ataaataatt tactctgaat ttaatacaaa acgatgtcat aatttcaatt gtacttgttc 2040

taataatata actacttatc gaaaaagaat agaaatttat tataaaattg tgtgaaatga 2100

ctgggtatta taggattgcc ccaacaaaat attttagatg tagtatgatg cttcatcagt 2160

gacaatatat ttgacgtgtt ttattatggg ccactcttta atgtaaacca gtattgattt 2220

attgtaaata aatattgtat atatactaca aaccaataaa atattttgcc ttttgaatct 2280

atttataaaa gatgattcca tattgtaata tagttcaatt agttgactac tttttttgaa 2340

tttatttcag aaataatttt aagtggttat ttgttcaatg taattgttag aaaataaaaa 2400

ctattccaa 2409

<210> 481
<211> 689
<212> DNA
<213> Diabrotica virgifera

<220>
<221> misc_feature
<222> (1)..(689)
<223> RNAi target sequence

<220>
<221> misc_feature
<222> (338)..(338)
<223> n can be any nucleotide

<220>
<221> misc_feature
<222> (551)..(551)
<223> n can be any nucleotide

<400> 481
gggcttcttt ctgtggttaa gtccaaaatg gcaccgacag ctgacaaaac taaaacggtt 60

aaagggctcc ccgcagtccc agaatcagtc ttaaaacacc gtaaaagacg tgaagcatcc 120

cgtgctaaac aactccagtc tgtcatcaaa aagaaggcag aacagatcaa aaagaggaaa 180

gatattttca aaagagctga acagtatgtt aaggaatacc gtttaaaaga aagagacgaa 240

gttaggctag ttagacaagc caaaaccaga ggaaatttct atgttcctgc tgaggctaaa 300

ttggcttttg ttgttcgtat taagggtatc aataaagnag cacctaaagt acgcaaagta 360

ctccaactat tccggttact tcaaatcaac aatggagttt tcgttaagtt gaacaaggct 420

actattaaca tgcttaggat atgcgaacct tacattacat ggggatatcc taacttaaag 480

tcagtaagag aattaatcta caaaagagga tttgccaaag ttaatggaca gcgtgtagca 540

attaccagca ntcaaatcgt tgaagacagg ttggggaaaa tctggaatca tttgtgttga 600

agatttaatc cacgaaattt tcactgttgg acctaagttc aaatatgcct ccaacttctt 660

gtggcccttc aagctaaaca cacccactg 689

<210> 482
<211> 20
<212> DNA
<213> Artificial Sequence

<220>
<223> primer

<220>
<221> misc_feature
<222> (1)..(20)
<223> forward primer

<400> 482
gcttctttct gtggttaagt 20

<210> 483
<211> 22
<212> DNA
<213> Artificial Sequence

<220>
<223> primer

<220>
<221> misc_feature
<222> (1)..(22)
<223> reverse primer

<400> 483
cagtgggtgt gtttagcttg aa 22

<210> 484
<211> 855
<212> DNA
<213> Diabrotica virgifera

<220>
<221> misc_feature
<222> (1)..(855)
<223> WCRW transcript ta05864.001_diavv

<400> 484
ggggggcgtg tggggggtct ttctgtggtt aagtccaaaa tggcaccgac agctgacaaa 60

actaaaacgg ttaaagggct ccccgcagtc ccagaatcag tcttaaaaca ccgtaaaaga 120

cgtgaagcat cccgtgctaa acaactccag tctgtcatca aaaagaaggc agaacagatc 180

aaaaagagga aagatatttt caaaagagct gaacagtatg ttaaggaata ccgtttaaaa 240

gaaagagacg aagttaggct agttagacaa gccaaaacca gaggaaattt ctatgttcct 300

gctgaggcta aattggcttt tgttgttcgt attaagggta tcaataaagt agcacctaaa 360

gtacgcaaag tactccaact attccggtta cttcaaatca acaatggagt tttcgttaag 420

ttgaacaagg ctactattaa catgcttagg atatgcgaac cttacattac atggggatat 480

cctaacttaa agtcagtaag agaattaatc tacaaaagag gatttgccaa agttaatgga 540

cagcgtgtag caattaccag caatcaaatc gttgaagaca ggttgggaaa atctggaatc 600

atttgtgttg aagatttaat ccacgaaatt ttcactgttg gacctaagtt caaatatgcc 660

tccaacttct tgtggccctt caagctaaac acacccactg gtggatggcg taagaagact 720

aaccactatg ttgaaggagg tgactttggt aatagagaag atagaattaa tgaacttctc 780

agaaggatgg tttaaaccaa tttgtttata aaataaatta tgtataaaac acaaaaaaaa 840

aaaaaaaaaa tgcga 855


<210> 485
<211> 327
<212> DNA
<213> Diabrotica virgifera


<220>
<221> misc_feature
<222> (1)..(327)
<223> RNAi target sequence

<400> 485
acacttacac ccttcaaaaa ctcatttata aactaaaaat gggtgcatct gtaattcctt 60

tcatagtttt cactgtatta tggggaggag ttggagctgt tctacccatt tttgtaccta 120

aaggacccaa tagaggaatt atccaagtta ttcttatgat aacaggtgtg tcgtgctggt 180

tgttctggct gtgttgttac gtagctcaaa tgaatcctct gatcggtcct aaactagaac 240

aaaacatcgt gctgattatg gcaaaacaat ggaaatatgc attaccatcg tcagcttgaa 300

tgaaggtgtg agtgaccatt ccatctg 327


<210> 486
<211> 24
<212> DNA
<213> Artificial Sequence

<220>
<223> primer


<220>
<221> misc_feature
<222> (1)..(24)
<223> forward primer

<400> 486
acacttacac ccttcaaaaa ctca 24

<210>  487
<211>  22
<212>  DNA
<213>  Artificial Sequence

<220>
<223>  primer


<220>
<221>  misc_feature
<222>  (1)..(22)
<223>  reverse primer

<400>  487
cagatggaat ggtcactcac ac                                                22


<210>  488
<211>  656
<212>  DNA
<213>  Diabrotica virgifera


<220>
<221>  misc_feature
<222>  (1)..(656)
<223>  WCRW transcript ta06727.001_diavv

<400>  488
ttcaaggagt ggttagattg tatgcactga atttctgaag gcagatgatt ttgacagatt        60

ttagtttact ctacttgtaa agtgaaaatc tattcacgtg aattatttta cacttacacc       120

cttcaaaaac tcatttataa actaaaaatg ggtgcatctg taattccttt catagttttc       180

actgtattat ggggaggagt tggagctgtt ctacccattt ttgtacctaa aggacccaat       240

agaggaatta tccaagttat tcttatgata acaggtgtgt cgtgctggtt gttctggctg       300

tgttgttacg tagctcaaat gaatcctctg atcggtccta aactagaaca aaacatcgtg       360

ctgattatgg caaaacaatg gaaatatgca ttaccatcgt cagcttgaat gaaggtgtga       420

gtgaccattc catctggata gtgtgtagaa atttcaaatt gcacagctat tgcattgtat       480

tatatttatt attaatttcc tttttgtgat catccaatat ctgtatgtag tatgttttaa       540

agtgatgtgt ataatattga ttattgttta tatactatca tcaaaaccaa ccattaattg       600

taaaaaaaat attgttgaat taaattaatt tgtcagttaa aaaaaaaaaa aaaaaa           656


<210>  489
<211>  526
<212>  DNA
<213>  Diabrotica virgifera


<220>
<221>  misc_feature

<222>  (1)..(526)
<223>  RNAi target sequence

<400>  489
tgtatttgtg actggaaaag gaaattagag ccctatactt acagttaaac gcttataaat      60

ttgtagcaag tattttatct ttacaaaaga aagtttattt cttaacattg tagttttata     120

tttttattac aaatggcaac tggaactact ttgcaaaaag ccattgagtt agtcaccaaa     180

gccaccgaag aagacagaaa caagaattat gaagaagccc ttcgtctata tgaacatgga     240

gtagagtact ttctacatgc cattaaatat gaagctcagg gtgacaaagc taaggagagc     300

attagggcca aatgtgtaca gtatctagaa agggcagaaa aaattaaaga atctgtaagg     360

aaagggaaga agaagccagt tttagatgga ggttcttcaa aggaagacaa aaagagtgat     420

agtgatgaag gtgacgaaga tgatcctgac aaaaagaagt tacaaagcaa actggaaggg     480

gccatagttg tagaaaaacc acatgtcaaa tggagtgatg tggcag                    526


<210>  490
<211>  24
<212>  DNA
<213>  Artificial Sequence

<220>
<223>  primer


<220>
<221>  misc_feature
<222>  (1)..(24)
<223>  forward primer

<400>  490
tgtatttgtg actggaaaag gaaa                                             24


<210>  491
<211>  20
<212>  DNA
<213>  Artificial Sequence

<220>
<223>  primer


<220>
<221>  misc_feature
<222>  (1)..(20)
<223>  reverse primer

<400>  491
ctgccacatc actccatttg                                                  20


<210>  492
<211>  2068
<212>  DNA
<213>  Diabrotica virgifera

<220>
<221>  misc_feature
<222>  (1)..(2068)
<223>  WCRW transcript ta02217.001_diavv

<400>  492
tgtgaagtgt caaaactgtc aaaagaatta tgccaatgtc aaaagtgtaa tttggagggg      60

tgattataaa attattctta gacattacaa tatttcatat aaagtattta tttaaaagat     120

gtatttgtga ctggaaaagg aaattagagc cctatactta cagttaaacg cttataaatt     180

tgtagcaagt attttatctt tacaaaagaa agtttatttc ttaacattgt agttttatat     240

ttttattaca aatggcaact ggaactactt tgcaaaaagc cattgagtta gtcaccaaag     300

ccaccgaaga agacagaaac aagaattatg aagaagccct tcgtctatat gaacatggag     360

tagagtactt tctacatgcc attaaatatg aagctcaggg tgacaaagct aaggagagca     420

ttagggccaa atgtgtacag tatctagaaa gggcagaaaa aattaaagaa tctgtaagga     480

aagggaagaa gaagccagtt ttagatggag gttcttcaaa ggaagacaaa aagagtgata     540

gtgatgaagg tgacgaagat gatcctgaca aaaagaagtt acaaagcaaa ctggaagggg     600

ccatagttgt agaaaaacca catgtcaaat ggagtgatgt ggcagggctg gaagctgcca     660

aggaggcttt gaaagaagct gttatattac ctattaggtt tccccatttg ttcagtggaa     720

agagagtacc ttggaaaggg attttgttat ttgggcctcc cggtactggt aaatcgtact     780

tagccaaggc tgtagctaca gaagcaaaca attcaacatt tttttctgtt tcgtcgtccg     840

atctcgtttc caaatggttg ggagagtcag aaaaattagt caagaatctt ttcgaattgg     900

ctagagccca caaacctagt attatattca ttgatgaaat cgattcttta tgctcatccc     960

gttccgacaa cgaatcagag tcagccagga gaatcaaaac cgagtttttg gttcagatgc    1020

aaggtgtggg ccacgatact gaaggtatcc ttgtcctcgg tgctacgaat attccgtggg    1080

tattggattc tgctattcga agaagatttg agaagagaat ttacatacct ctaccagaag    1140

aacctgccag agccgttatg tttaaattgc atttaggaaa tactcataca gagctaacag    1200

atgaagatat taaagagttg gcatctaaaa ccgaagggta ttccggagct gatataagta    1260

tcgtagttcg tgacgctctt atgcaacccg tccgaaaagt ccaaactgca actcacttca    1320

agaaggtcag aggtccgtcg ccgcttgatc ctaataccat cgtagatgat ttgttgcttc    1380

catgttctcc cggagatccg ggagctgtgg aaatgtcttg gatggatatc tccagtgata    1440

aattagctga gcctcctgtt acagtgaatg acatgctgag atcaatcgca acctccaaac    1500

caaccgtcaa cgacgaagat ctcacgaaac tcaaaaagtt tatggaagac ttcggccaag    1560

aaggataaaa tttaggtata caccagattt actatttaaa agagagattc ttgtataata    1620

tttgtattct tttaatgcat atatagatcg taaaaaccac acaaaacttt caacattttg    1680

cattaagtgt tattagaaaa caaaagatta atgttgttat actgttgctt taatattatt 1740

tttttaataa attgaatttt tcttttttat attacggcct tattatacag taagatagta 1800

tatatttgta ttcttaatga taaatgaagt taatgaaaca tgtttttaga atattcactt 1860

gttctttgta tataaaataa taaaacaccg attggtttaa aatacttgta ttggcttaaa 1920

gcaagttatc tataagcttc aaagacatac tcagtgtctt atccgatttc tgttactata 1980

aacgatctga ttcatttatt tatctgttgt aattaaaatc tgaaataaaa tgtcacaata 2040

tgtagattgt tttccacctt aggattgg 2068

<210> 493
<211> 621
<212> DNA
<213> Diabrotica virgifera

<220>
<221> misc_feature
<222> (1)..(621)
<223> RNAi target sequence

<400> 493
gcgattcggt aaaggagaaa gtaaccagaa ttattttagc tgttttcagg aatttgatcg 60

aaaaacccga agatcaacaa gtttcaaaag aacattgtat agctatggtg caatgcaaag 120

tattgaaaca attggccatt ttagaacagc gcaagttcga tgatgaagat gtgacagctg 180

atgtcgaatt tctaactgaa aaactacaga gttctgttca ggatctgagt tccttcgatg 240

aatattcaac tgaagtcaaa tctggacgtt tggaatggtc tccagtacac aagagcaagt 300

tctggcggga aaatgcgcag cgcctcaacg agaaaaacta cgaattactc cgtattctca 360

ttcatttgtt agaaaccagc aaggacccct tggtattgag cgtagctagt ttcgatattg 420

gagagtatgt tcgccattat cctcgcggca aacacataat cgagcagttg ggaggaaagc 480

aattagttat gcaactctta gcccacgaag atcccaatgt acgatatgaa gctctgttgg 540

cagtccaaaa attgatggtc cacaattggg aataccttgg tcgtcaactt gaaaaagagc 600

aaagtacaga taaaaccgcg c 621

<210> 494
<211> 21
<212> DNA
<213> Artificial Sequence

<220>
<223> primer

<220>
<221> misc_feature
<222> (1)..(21)

<223>  forward primer

<400>  494
gcgattcggt aaaggagaaa g                                                21


<210>  495
<211>  22
<212>  DNA
<213>  Artificial Sequence

<220>
<223>  primer


<220>
<221>  misc_feature
<222>  (1)..(22)
<223>  reverse primer

<400>  495
gcgcggtttt atctgtactt tg                                               22


<210>  496
<211>  1830
<212>  DNA
<213>  Diabrotica virgifera


<220>
<221>  misc_feature
<222>  (1)..(1830)
<223>  WCRW transcript ta02168.002_diavv

<400>  496
tacttctgtc agtgtcaaaa tttatatcac aagacttcca gttgggagtt gggtcagagt      60

taatttttta ggtcttctat taaaagggtt gtaattttaa tttaaaaaaa tggcatccaa     120

tttagttgcg ggagcaaatc ctaaaataca ggatataatg tcgtctttgg acgatgacaa     180

aacagatatg ttggctgcta caagtgtatt gcagcaaaga gctacagata taagaaatca     240

acatgtaaac tggcaatcct atttccagtc acaaatgatt tcgcaggaag actacaactt     300

catagtagct tttgatgtga ctgatagtgc aaaaagagaa gctctcctga aaagcgacag     360

aaaccaatgt gctcaaacat tgttgaacct tttggggcat gtatccaagg accaaactct     420

gcagtacatt ttagttttgg ttgatgatat gttacaggag aacagaagtc gtgtagagat     480

atttcacgaa tatgccaacc aaaagaaaga atctgtttgg ggtccatttt tgaaccttct     540

taaccgccag gatggattta ttaccaacat gacttctagg atcatcgcta aaattgcttg     600

ttggtcgcag acacttatgg atcgttctga ccttcatttt tacttgacct ggttaaaaga     660

ccaactaaaa actcagaaca acgaatacat ccaatcggtt gcaagatgcc ttcaaatgat     720

gctccgaatt gacgactacc gttttgcctt cgtgtccgtc gatggaatat ccactctgtt     780

gtcggtactc tctggaagag taaatttcca agttcagtac cagctgatat tctgcttgtg     840

ggtactcact ttcaacccgt tgttagccga aaagatgaac aaattcaacg ttattcctat 900

attggcagat attttgagcg attcggtaaa ggagaaagta accagaatta ttttagctgt 960

tttcaggaat ttgatcgaaa aacccgaaga tcaacaagtt tcaaaagaac attgtatagc 1020

tatggtgcaa tgcaaagtat tgaaacaatt ggccatttta gaacagcgca agttcgatga 1080

tgaagatgtg acagctgatg tcgaatttct aactgaaaaa ctacagagtt ctgttcagga 1140

tctgagttcc ttcgatgaat attcaactga agtcaaatct ggacgtttgg aatggtctcc 1200

agtacacaag agcaagttct ggcgggaaaa tgcgcagcgc ctcaacgaga aaaactacga 1260

attactccgt attctcattc atttgttaga aaccagcaag gaccccttgg tattgagcgt 1320

agctagtttc gatattggag agtatgttcg ccattatcct cgcggcaaac acataatcga 1380

gcagttggga ggaaagcaat tagttatgca actcttagcc cacgaagatc ccaatgtacg 1440

atatgaagct ctgttggcag tccaaaaatt gatggtccac aattgggaat accttggtcg 1500

tcaacttgaa aaagagcaaa gtacagataa aaacgcgcca aagggtggag ctcctgtcgc 1560

aggcaaggcg taaatactac catgaaaagt atttaaagat atttactgca ataattgtag 1620

aaactttaaa aataattgta aaaattatat cgatttctgt acatagctgt ataggtatac 1680

attcctgtca tattttatta ttgttacgat aaccaataat caaccatgca catttaaaac 1740

atttaaaaat attgctttta aagtaacttt tttctgattg ttagtattat aataaagtat 1800

atgttgtcgt ttaaaaaaaa aaaaaaaaaa 1830

<210> 497
<211> 642
<212> DNA
<213> Diabrotica virgifera

<220>
<221> misc_feature
<222> (1)..(642)
<223> RNAi target sequence

<400> 497
gggaaccttt ttctggaggt taggaaaagt acgtgacatt ttcactttat tttccataca 60

gtttaaacaa tattgtattc ttgtagttat tgcctgaaaa taatcacttt ttaagattag 120

caatgccagc agctgacgaa ataaagtcca gctgggctga cgaggtagag ttagaaggag 180

gtgccttacc accacccagt gaagtcttcg aaaatggttt caagattgta actgaatata 240

aatacaatga agacgacaag aaagttaaga ttgtcaggac gtacaaaatt gagaaacgtg 300

ctgtgtccaa gtcaattgct ctccgtaaaa catggaaaaa gtttggtgat gttgccaatg 360

ataaacctgg cccgaatccc gcgactacca tagtagctga agacgtttat atgcagtaca 420

tcactagtaa ggaagaagat aataagcagg atgatgactc tttggacaaa ctgaaaggaa 480

tgggtgataa gaaattcttt aaatgtcgta catgtaatgg tgaacattgg actacaaaat 540

gtccatggaa ggacactatg tttgttggtg gaaagccacc agatgataag aaaatatcag 600

ctgttggagg ggcagctgtt ggtgaagctg gtaaacaagg at 642

<210> 498
<211> 20
<212> DNA
<213> Artificial Sequence

<220>
<223> primer

<220>
<221> misc_feature
<222> (1)..(20)
<223> forward primer

<400> 498
gaaccttttt ctggaggtta 20

<210> 499
<211> 22
<212> DNA
<213> Artificial Sequence

<220>
<223> primer

<220>
<221> misc_feature
<222> (1)..(22)
<223> reverse primer

<400> 499
atccttgttt accagcttca cc 22

<210> 500
<211> 1014
<212> DNA
<213> Diabrotica virgifera

<220>
<221> misc_feature
<222> (1)..(1014)
<223> WCRW transcript ta06291.001_diavv

<400> 500
cggcaacctt tttcttgagg ttaggaaaag tacgtgacat tttcacttta ttttccatac 60

agtttaaaca atattgtatt cttgtagtta ttgcctggaa ataatcactt tttaagatta 120

gcaatgccag cagctgacga aataaagtcc agctgggctg acgaggtaga gttagaagga 180

ggtgccttac caccacccag tgaagtcttc gaaaatggtt tcaagattgt aactgaatat 240

aaatacaatg aagacgacaa gaaagttaag attgtcagga cgtacaaaat tgagaaacgt 300

gttgtgtcca agtcaattgc tctccgtaaa acatggaaaa agtttggtga tgttgccaat 360

gataaacctg gcccgaatcc cgcgactacc atagtagctg aagacgttta tatgcagtac 420

atcactagta aggaagaaga taataagcag gatgatgact ctttggacaa actgaaagga 480

atgggtgata agaaattctt taaatgtcgt acatgtaatg gtgaacattg gactacaaaa 540

tgtccatgga aggacactat gtttgttggt ggaaagccac cagatgataa gaaaatatca 600

gctgttggag gtgcagctgt tggtgaagct ggtaaacaag gatccaaata tgtaccacct 660

aatttgagag atggaggtgt gaagagaact gatggatctg gtgtagctaa acgagatgat 720

agttcctgtg ctatcagaat tgccaatttg agtgaatcca caactgaaac tgatttggaa 780

gatctggtta aacaatttgg accaattcaa aaactgtatt tggcgaaaga caagataact 840

ggtggatgta aaggatttgc ttatatacat ttcaaattcc gttctgatgc agctaaggca 900

atcgcaatgc ttcatggaca tggttatgat catcttattc ttaatgttga ttggtccaag 960

ccatcaccaa attctaacca ataattgtat caccagattt ttgaataata aaat 1014

<210> 501
<211> 524
<212> DNA
<213> Diabrotica virgifera

<220>
<221> misc_feature
<222> (1)..(524)
<223> RNAi target sequence

<400> 501
tcaacgtcat ctcagtttgg tttattgttg ggtgtaatgt ttaaataaaa taattattat 60

tttgtaaacc tgtgccatta aacaaataaa gcgatggtgc taattgcagc agcagtctgc 120

acgaaagcag gcaaaacaat tgtgtctcga caatttgttg aaatgaccaa agctagaata 180

gaaggtttgt tggctgcctt tcctaaatta attcctacag gaacccagca tacatttgtg 240

gaaacagatt cagtacggta tgtttatcaa cccctagaga aactgtatat ggttcttatt 300

acagctagag ctagcaacat attagaagat cttgaaaccc tccgtctatt cgcaagagtg 360

attcctgagt actgcaaatc tttggatgaa aatgaaattg cagagaatgc attttcactt 420

atatttgctt ttgatgaaat agtggcatta ggttataggg aaagtgtcaa tctgtctcaa 480

attcgcacat ttgtggaaat ggactcgcac gaagaaaaag ttta 524

<210> 502
<211> 22
<212> DNA
<213> Artificial Sequence

<220>
<223>  primer


<220>
<221>  misc_feature
<222>  (1)..(22)
<223>  forward primer

<400>  502
tcaacgtcat ctcagtttgg tt                                                22


<210>  503
<211>  23
<212>  DNA
<213>  Artificial Sequence

<220>
<223>  primer


<220>
<221>  misc_feature
<222>  (1)..(23)
<223>  reverse primer

<400>  503
taaacttttt cttcgtgcga gtc                                               23


<210>  504
<211>  1812
<212>  DNA
<213>  Diabrotica virgifera


<220>
<221>  misc_feature
<222>  (1)..(1812)
<223>  WCRW transcript ta03896.001_diavv

<400>  504
cgaagatcct tgtcacgtca ctgctttttc gtgaaggggg ggtacacgtc aacgtcatct      60

cagtttggtt tattgttggg tgtaatgttt aaataaaata attattattt tgtaaacctg     120

tgccattaaa caaataaagc gatggtgcta attgcagcag cagtctgcac gaaagcaggc     180

aaaacaattg tgtctcgaca atttgttgaa atgaccaaag ctagaataga aggtttgttg     240

gctgcctttc ctaaattaat tcctacagga acccagcata catttgtgga aacagattca     300

gtacggtatg tttatcaacc cctagagaaa ctgtatatgg ttcttattac aactagagct     360

agcaacatat tagaagatct tgaaaccctc cgtctattcg caagagtgat tcctgagtac     420

tgcaaatctt tggatgaaaa tgaaattgca gagaatgcat tttcacttat atttgctttt     480

gatgaaatag tggcattagg ttatagggaa agtgtcaatc tgtctcaaat tcgcacattt     540

gtggaaatgg actcgcacga agaaaaagtt tatcaggccg tgagacagac tcaagagcgc     600

gaggccaaga atatgatgag agaaaaggca aaagaacttc agagacagaa gatcgaagcg 660

gccaaaaaag gagggaagac ctcgtttggt agtagcggtg gctttggcag ctcgacaggt 720

tatactccta cgccatctgt tggtgatgta gctaaccaga caaatgatgt taaaacttct 780

tcatacacac cagcccctgc gcaaaaacct cggggtatga aattaggtgg aaaaggtaga 840

gatgtagaat cattcgtaga tcagcttaaa tcggaaggag aaaacgtcat tactccaaac 900

aaaaatagta tttcacagcc aggaactaaa gctccagcta tcaaaactga catcgatgat 960

gttcatttaa gattggaaga aaaattaata gtgagaatag gtcgtgatgg tggcgtacaa 1020

caattcgaat tattgggact tgctacttta cacattggag atgagagatg gggtaggata 1080

cgtgtgcaat tggaaaatca gaatacccac ggtgttcaac ttcaaacgca tcctaatgta 1140

gataaagaat tattcaagct acgctcacag attggattga aacaaccggc taaacctttt 1200

cctctaaata cagatgttgg tgtactgaaa tggagattac aaagtactga ggaagctcta 1260

attccactct taataaattg ctggccttca gaagcgggag atggtagttg cgatgttaat 1320

atagaatatg agcttgccca cactaatttg gaactagttg atgtcaatat tgttattccc 1380

ttgccaattg gatgttcacc aatagttggt gaatgtgatg gtatgtacac acacgaagcc 1440

aagcgtaatc aactggtatg gaatttgcct ctgattgatg ccagtaataa aactggttct 1500

ttagaattta acgctcctag ggccataccc gctgatttct tcccgctttc cgttggattc 1560

aattcgaagt catcctatgc gagtattaag attaccgaag ttgtcctagt tgatgatgat 1620

tctcctataa aatattcagt ggagaccgca ctatatccag ataaatatga agtagtataa 1680

tggggcagaa gtaaagaatc acttgtaaaa attatataac taatattata tcttatatgc 1740

aacattgctt tttgtacaaa agttaagtaa cttgtcttta ttttatctat attaataaaa 1800

taagtaagaa ag 1812

<210> 505
<211> 612
<212> DNA
<213> Diabrotica virgifera


<220>
<221> misc_feature
<222> (1)..(612)
<223> RNAi target sequence

<400> 505
attctgatcc cagcacacaa attttgtata accggacaat ggcgcatttg ggattgtgtg 60

catttagaca cgctaacatt aaggatgccc ataactgttt agttgatttg atgatgactg 120

gaaaaccaaa ggagttactg gcacaaggtt tgcttccaca aagacaacac gaacgcagca 180

aagaacagga gaaaatcgag aaacagcgtc aaatgccctt ccacatgcac atcaatctcg 240

aactcttaga atgtgtgtat ttagtctccg ccatgttgat cgaaatcccg tatatggctg 300

cgcatgaatt cgacgccaga cgcaggatga tttctaagac cttctatcag cagttgcgtt 360

cttccgaacg tcaaagctta gtgggacctc cggaaagcat gcgagagcac gtcgtcgcgg 420

cggctaaagc aatgatgaac ggcaattggg cggcatgcaa cacttttatc attaacgaca 480

agatgaatgc caaagtgtgg gatctgtttt accaagccga ccaagttaga tccatgttga 540

cgaaattgat aaaagaagag tctttgagga cgtatttgtt tacctactcc cacgtttacg 600

attcaatttc ga 612

<210> 506
<211> 22
<212> DNA
<213> Artificial Sequence

<220>
<223> primer

<220>
<221> misc_feature
<222> (1)..(22)
<223> forward primer

<400> 506
attctgatcc cagcacacaa at 22

<210> 507
<211> 22
<212> DNA
<213> Artificial Sequence

<220>
<223> primer

<220>
<221> misc_feature
<222> (1)..(22)
<223> reverse primer

<400> 507
tcgaaattga atcgtaaacg tg 22

<210> 508
<211> 2785
<212> DNA
<213> Diabrotica virgifera

<220>
<221> misc_feature
<222> (1)..(2785)
<223> WCRW transcript ta05048.001_diavv

<400> 508
caatgactaa tgtcacctcg ctcttttaac tcacgttgca tatgtgaggt attttccaaa 60

attcgagaaa agtttattta gtgttattat aataagggaa aacagtaaaa atgagtagat 120

ttttcgctgg atccgattca gattcggata gctcatccga agaagagatt atccaacgtc 180

cccaagtacc agcttatact ttcagtgacg atgaggagga ggtaaaacgt gtagtaagat 240

cagcgaaaga aaagcgctat gaagaactta caaatactat taaacaaatc agaaatttta 300

agaaaattaa ggatatgtct agcatgttga atagttttga ggaattacaa aaggcatacc 360

aaaaagctgc tcctgttatt gtcaaagaag aaaatggcca gactccaaga ttttttatta 420

ggtgtttggc cgaaatggaa gactttatta atgaaatgtg ggaagataga gatgggagga 480

aaaacatgag caaaaacaat agcaaatcac tcacatctat gagacagaag ttgagaaaat 540

atttgaaaga ttttgaagat gacttggcta aatttaagga aaacccagaa cagcctgatg 600

atgaggaaga ggaggaaaag cctgaagtag ctgaggattc cgagaacgaa gctccatcca 660

aaaacgcgtt tctcaaagct ccacccaaac aagttgtaga cgacaaagat gaatcgtcag 720

actcggatga ctggccatct gactctgaat ctagtctgtc atcatctgat gacgacgaag 780

gcaaatacca gagcataagg gagcgtttct tgaagagaac tgggcccgct cacgaagaag 840

atgaagaaag ggaaaggaga aaggaggaga aaaggaagga gagaaaggag aaaggtagga 900

agttgaaaaa ggatgaggag gaggatggtg aaggcaagtg ggagactgtt aatcgaggtt 960

tcgccatccc tagtgaaaaa cctaaaatgt tcgccaagga cgccgaaatc gatctaaacc 1020

ttgtaatcaa gaaactcagc gaaatcatgg cagctagagg caaaaagcgc acagatagac 1080

gcgaacaaat agaactcctg cacgaactcc aaaatgtttc tgagcaacac caattgggag 1140

cggctatttt cattaaaatt aaactcgcca tcatatctgc cattttcgat tacaacccca 1200

aagtttctga cgcaatgaag ccggagtatt ggctgaaatt gctggagagg atgagcgaaa 1260

tgttgggcat gttgttgaat actaccaatc tagtagtttc tgatggaatt actgaagaaa 1320

tggaaaatct tgaagaaccc ccgtatcgca ttaggggctg tatactaaca tcagtggaaa 1380

gactggatga cgagtttact aaactactca aagagtgtga tcctcacagt aacgagtatg 1440

tagaacggtt aaaggatgaa tgtaaggttt ctagtatcat cgataaaaca atgaaatact 1500

tggaaaggag taacgtatct tccgaactat gtaggatata tttacgaaaa atcgagcatt 1560

tgtattacaa attcgatcct agagtattac agcaaaaagc tgatcctctc gtagtaccca 1620

aagacgagat gaactccgtg caagaaatgg agaaactatg caaatttatc tacgcaaaag 1680

atagtactga cagattgaga actagagcta ttttgtccca tatatatcat cacgctctgc 1740

atgacaactg gttccaagca agggatttgg tattgatgtc acatctacaa gaaacgattc 1800

agcattctga tcccagcaca caaattttgt ataaccggac aatggcgcat ttgggattgt 1860

gtgcatttag acacgctaac attaaggatg cccataactg tttagttgat ttgatgatga 1920

ctggaaaacc aaaggagtta ctggcacaag gtttgcttcc acaaagacaa cacgaacgca 1980

gcaaagaaca ggagaaaatc gagaaacagc gtcaaatgcc cttccacatg cacatcaatc 2040

tcgaactctt agaatgtgtg tatttagtct ccgccatgtt gatcgaaatc ccgtatatgg 2100

ctgcgcatga attcgacgcc agacgcagga tgatttctaa gaccttctat cagcagttgc 2160

gttcttccga acgtcaaagc ttagtgggac ctccggaaag catgcgagag cacgtcgtcg 2220

cggcggctaa agcaatgagg aacggcaatt gggcggcatg caacactttt atcattaacg 2280

acaagatgaa tgccaaagtg tgggatctgt tttaccaagc cgaccaagtt agatccatgt 2340

tgacgaaatt gataaaagaa gagtctttga ggacgtattt gtttacctac tcccacgttt 2400

acgattcaat ttcgatggcc actttggcgg aaatgttcca gttgggcaaa cccgtcgtgc 2460

attctattat ttctaagatg atcattaacg aagaattgat ggcatcttta gacgacccga 2520

ctcaaactgt tgtaatgcac agaagcgaac catcccgtct acaatctttg gctttgcaac 2580

tggcggataa aatcaacaat ttcgtcgatt ccaacgaaag aatattggaa tccaaacaag 2640

gaaacttctt cctaagggga gcaaaccagg gcaatttccg aggagatagg cagaactacc 2700

gcggaaataa ccaaaactgg gataggcaac gtcgagacag ggatagaaag gaaaaagata 2760

attattaata aacatagatc ttttg 2785

<210> 509
<211> 503
<212> DNA
<213> Diabrotica virgifera

<220>
<221> misc_feature
<222> (1)..(503)
<223> RNAi target sequence

<400> 509
ggggctcttt agttgtcatc tgtcatttaa gtagtaattt cattccaaaa tgaagatcgg 60

actttgtgct tacagcgggt acaaaattta ccccggacat ggcaaaacaa tggttaaagt 120

tgatggaaag accttcattt tcctcaactc aaaatgtgaa agggcgcact tgatgaaaag 180

gaacccccgt aaagtaacat ggactgtctt gtacagacgt aaacataaga agggtcagga 240

ggaagaagcc accaaaaaac gtaccagaag gacacagaaa ttccaaaggg ctattgtagg 300

tgcttcttta aatgatatca tggccaagag aaaccaaagg cctgaagtaa ggaaggccca 360

aagggagcaa gccattcgtg ccgctaagga acagaaaaag agcacaaaga cagccaggaa 420

ggctacagcc cccgccaagg ccaaacctgc tcctaaacag aaagtaacca agaatcaacc 480

caaagctgca cctagagtag gag 503

<210>  510
<211>  19
<212>  DNA
<213>  Artificial Sequence

<220>
<223>  primer


<220>
<221>  misc_feature
<222>  (1)..(19)
<223>  forward primer

<400>  510
gctctttagt tgtcatctg                                                    19


<210>  511
<211>  23
<212>  DNA
<213>  Artificial Sequence

<220>
<223>  primer


<220>
<221>  misc_feature
<222>  (1)..(23)
<223>  reverse primer

<400>  511
ctcctactct aggtgcagct ttg                                               23


<210>  512
<211>  2131
<212>  DNA
<213>  Diabrotica virgifera


<220>
<221>  misc_feature
<222>  (1)..(2131)
<223>  WCRW transcript ta01391.001_diavv

<400>  512
aaagatggaa ggaaatgagg ccaatactta gcccatcgtt cacaagcagc aaaatgaaat      60

cgatgtttat gctgatgtca gaatgtggcg aaaagttggt caaatttttt atggaaaaag     120

accagaatgc tatagaaatt gaaatgaaag acacattaac gcgttatact aatgatgtta     180

ttgcctctgc agctttttgg agtacagatt gattctttaa acaacactga aaatgaattc     240

tatctcatgg gaaaaaatgc aaccaatttc cgtggtttct ggaggactat gaagttgttt     300

ggatatatga ttataccaaa aatatttgag ttctttaaag tgacattgtt tgatatgaac     360

gttcgacgat tttttactga attgattagt ggaacaataa agatcaggga agagaaaggc     420

attattcgtc ccgatatgat tcacctactc atgcaagcaa gaaaaggaat ccatcaaaaa 480

gaagaaaagg ttgttgatgc tggatttgct gtagtacaag aatcgaattt agaatcaaaa 540

gtagttgcga cagaaatgac cgacttagac atctgcgctc aagcattgat tttcttcttt 600

gctggatttg agacggtctc gtcaaccatg tgctttgtgg ctcatgaatt ggcctgtaat 660

cctgatgtcc aatcgagatt aagagatgaa atacgtgaaa cactcgatca atgtaatgga 720

aaagtaacct acgaagctat gttgaaaatg aaatatctgg atatggtaat atcagaatcc 780

atgaggaaat ggccagcagt tatagctgtt gatcgaattt gcaccaaacc atacacactc 840

gaaccagcaa cactcgatga gaagcccctc cacttagaca aaggcaccat aatctggttc 900

cccatttacg gtctccaccg cgatcctaaa cactacccag acccagaacg gtttgatcct 960

gaaagattca gcgatgaaaa caaaggaaac atcaatccat acgcttacct tccatttgga 1020

gtcggcccta gaaactgcat cggttccaga tttgctttat tagagatgaa agtattgttt 1080

ttccatttgt tagccaattt tgagattgtg ccaggtccaa agacggaaat tcctttgcgg 1140

atttgtaaga aacaattcgg tttgactgca gaaaatggtt tttggttcaa cttgaagaaa 1200

ctgaagaatt agtctttgat atgtttgata aaagtcttga taaaatattg tttaaccata 1260

tctacttatt tggtgtaatg tattttctag tcataattct tttaaacttt ttcttaacgt 1320

ttcagcgggc gcaccctata atttcacatg agaagacgcg tgtcgtagat tatacgtctt 1380

tgcgtaaata atgtcttctt caaataaatg tttggaaatt gttacaatga tattttcttc 1440

ttcttcctgc ttccttatag gctcgcatct gttctatctt cggatgcctc ttcatcagat 1500

atccgcagca cgtccacaca ggtaaaccta aacataaaaa cgggggctct ttagttgtca 1560

tctgtcattt aagtagtaat ttcattccaa aatgaagatc ggactttgtg cttacagcgg 1620

gtacaaaatt taccccggac atggcaaaac aatggttaaa gttgatggaa agaccttcac 1680

tttcctcaac tcaaaatgtg aaagggcgca cttgatgaaa aggaaccccc gtaaagtaac 1740

atggactgtc ttgtacagac gtaaacataa gaagggtcag gaggaagaag ccaccaaaaa 1800

acgtaccaga aggacacaga aattccaaag ggctattgta ggtgcttctt taaatgatat 1860

catggccaag agaaaccaaa ggcctgaagt aaggaaggcc caaagggagc aagccattcg 1920

tgccgctaag gaacagaaaa agagcacaaa gacagccaag aaggctacag cccccgccaa 1980

ggccaaacct gctcctaaac agaaagtaac caagaatcaa cccaaagctg cacctagagt 2040

aggaggaaaa cgttaatatt gtataaacgt tatttaataa aataagtttt tttccataaa 2100

aaaaaaaaaa aaatgcgaaa aaaaaacatg t 2131

<210> 513
<211> 502
<212> DNA
<213> Diabrotica virgifera

<220>
<221>  misc_feature
<222>  (1)..(502)
<223>  RNAi target sequence

<400>  513
aaagtttttg agccgtcaac atgggtaagc cccgtggatt gagaaccgct aggaaacatg      60

tgaatcaccg tcgtgaacaa agatgggctg ataaagatta caaaaaggca catttaggaa     120

ctagatggaa ggctaatcct tttggaggtg cttctcatgc taaaggaatt gtattggaaa     180

aagtgggagt tgaagcaaaa caacctaact ctgctattcg caagtgcgtt agggtacagt     240

taattaaaaa tggtaaaaaa atcacagccc tcgtacctag agatggttgc ttaaaccata     300

tagaagaaaa cgatgaagtt ctagttgcag gttttggacg taaaggtcat gctgtaggag     360

atattcccgg agttagattt aaggttgtta aggtcgctaa tgtgtcccta ttagctttat     420

acaaagaaaa gaaggaaagg ccaagatcat aattaaaatg ttattttttt tatttatttg     480

cttggcactt ttaattttta aa                                              502


<210>  514
<211>  22
<212>  DNA
<213>  Artificial Sequence

<220>
<223>  primer


<220>
<221>  misc_feature
<222>  (1)..(22)
<223>  forward primer

<400>  514
aaagtttttg agccgtcaac at                                               22


<210>  515
<211>  24
<212>  DNA
<213>  Artificial Sequence

<220>
<223>  primer


<220>
<221>  misc_feature
<222>  (1)..(24)
<223>  reverse primer

<400>  515
tttaaaaatt aaaagtgcca agca                                             24

<210>  516
<211>  616
<212>  DNA
<213>  Diabrotica virgifera

<220>
<221>  misc_feature
<222>  (1)..(616)
<223>  WCRW transcript ta00545.001_diavv

<400>  516
ggggattttc ggttgggggg ttgggagcag ttatgggggc tttccggtca aagtttttga      60

gccgtcaaca tgggtaagcc ccgtggattg agaaccgcta ggaaacatgt gaatcaccgt     120

cgtgaacaaa gatgggctga taaagattac aaaaaggcac atttaggaac tagatggaag     180

gctaatcctt ttggaggtgc ttctcatgct aaaggaattg tattggaaaa agtgggagtt     240

gaagcaaaac aacctaactc tgctattcgc aagtgcgtta gggtacagtt aattaaaaat     300

ggtaaaaaaa tcacagcctt cgtacctaga gatggttgct taaaccatat agaagaaaac     360

gatgaagttc tagttgcagg ttttggacgt aaaggtcatg ctgtaggaga tattcccgga     420

gttagattta aggttgttaa ggtcgctaat gtgtccctat tagctttata caaagaaaag     480

aaggaaaggc caagatcata attaaaatgt tatttttttt atttatttgc ttggcacttt     540

taatttttaa ataaagtgtt aagcatataa ttttgtacag tgttttattt caataaacaa     600

ttttatacct gaaata                                                     616

<210>  517
<211>  719
<212>  DNA
<213>  Diabrotica virgifera

<220>
<221>  misc_feature
<222>  (1)..(719)
<223>  RNAi target sequence

<400>  517
gggtgatcct tcagttttcg tccacgcgtg tcaaaatggc gaaaaagaaa ccagaggata      60

aaaataatca aagcgatgat gatacaaaaa acgagagtga ttactcgtcg aacgaagaag     120

aaccaaactt cagtgatcct gaagactacg aagacgatgt ctctgatgaa gaactcttag     180

gtgatgtttt aaaagaaaag cctaaggaaa ctgatggagt agaaagtgtt atagtagtag     240

atggagttcc tcaagtaggt cctaatcgta ttgaaaagtt gaagtcagtc attgtcaaaa     300

tatttgtgaa gtttggaaca atagtaaatg aattctaccc tgtcaatagt gagggccaca     360

ccaaaggtta tatctttata gaatattcct caccatctga agctgctgag gctgttaagc     420

tcactaacaa ttttaaattg gataagcacc atacctttca agttaacctc tttactgatt     480

tttctaagta tgaccaaatt cctaataagt gggaccctcc agagccacaa ccttaccaag 540

ggcaagcaga cttgcattct tacttattgg aaccggatgc atatgatcag tttgctgtag 600

ttgttgcaca aacacaagct gtgcaaattt ggcagaatac acagcctgat cctagtctca 660

ttgaagctag aaacagctgg agtcaaacgt acataaaatg gtcacctctg ggtacattc 719

<210> 518
<211> 19
<212> DNA
<213> Artificial Sequence

<220>
<223> primer

<220>
<221> misc_feature
<222> (1)..(19)
<223> forward primer

<400> 518
gtgatccttc agttttcgt 19

<210> 519
<211> 22
<212> DNA
<213> Artificial Sequence

<220>
<223> primer

<220>
<221> misc_feature
<222> (1)..(22)
<223> reverse primer

<400> 519
gaatgtaccc agaggtgacc at 22

<210> 520
<211> 2397
<212> DNA
<213> Diabrotica virgifera

<220>
<221> misc_feature
<222> (1)..(2397)
<223> WCRW transcript ta01172.001_diavv

<400> 520
gggggtgatc cttcagtttt cgtccacgcg tgtcaaaatg gcgaaaaaga aaccagagga 60

taaaaataat caaagcgatg atgatacaaa aaacgagagt gattactcgt cgaacgaaga 120

agaaccaaac ttcagtgatc ctgaagacta cgaagacgat gtctctgatg aagaactctt 180

aggtgatgtt ttaaaagaaa agcctaagga aactgatgga gtagaaagtg ttatagtagt 240

agatggagtt cctcaagtag gtcctaatcg tattgaaaag ttgaagtcag tcattgtcaa 300

aatatttgtg aagtttggaa caatagtaaa tgaattctac cctgtcaata gtgagggcca 360

caccaaaggt tatatcttta tagaatattc ctcaccatct gaagctgctg aggctgttaa 420

gctcactaac aattttaaat tggataagca ccataccttt caagttaacc tctttactga 480

tttttctaag tatgaccaaa ttcctaataa gtgggaccct ccagagccac aaccttacca 540

agggcaagca gacttgcatt cttacttatt ggaaccggat gcatatgatc agtttgctgt 600

agttgttgca caaacacaag ctgtgcaaat ttggcagaat acacagcctg atcctagtct 660

cattgaagct agaaacagct ggagtcaaac gtacataaaa tggtcacctc tgggtacatt 720

cttatgtact tttcacaggt tgggtgttgc attgtgggca ggacctggat ttttgcaata 780

caaaaaattt acccattcaa atgttcaatt tattgatttc tcgccatgtg aaaagtattt 840

agtgacttat tccccacaag gagacccaca taatccagaa cagaaaagga tcatcatttg 900

ggatattagg acaggtcttg aaaagcgttc attcaatcca gaaggtccaa cagcatggcc 960

aatcttcagg tggtctcatg acgacaaata ttttgctcgt attggtcaaa acaatgatgc 1020

ccttcaagta tacgaaacac catcttttgg tcttctagat aagaaatcaa tcaaaattaa 1080

cggcataaga gatttcaatt ggtcgcccac agataatgtt gtagcttatt gggtagccga 1140

agacaaagat gtgcctgcca gtgtaactct gttagaatta ccaaacagga atgaaattag 1200

aaagaagaat ttgtttaatg ttgctgactg taagatacac tggcagaagt ctggagatta 1260

cctgtgtgtc aaggtagaca ggtattcaaa ggttcgcaag gagaagaatg aaaccaagta 1320

ttcaggtatg tactgcaact ttgaaatatt ccacatgaag gagaagcaaa tacctgtaga 1380

cagtgtagag tgcaaggaac ccattcaggc gtttgcatgg gagccggttg gttctaaatt 1440

tgcaatgatc catggcgaat cccccagcat cagtgccagt ttctacgagg ttaggatagg 1500

acaggctcct atccttctca agaaatttga gaaaagagct tgcaaccatc tgttttggtc 1560

tcctagtggg cagttcatag ttttagcagg cttgggttcc aacggaggtg gatcgctgga 1620

atttgttgat actcacgaat ttttaatcat gaacactaca gaccatttcc aattgtcaga 1680

tgtagagtgg gatccaactg gaagatatgt aattactgga gtctcatttt ggaagaccaa 1740

ggttgataca ggttattgga tttggtcctt ccaaggaaaa atcctcaagc gcatcaattt 1800

ggaaaagttc tcccagattc tctggagacc tagaccacca tcattactca gcgatgaaaa 1860

gcaaaaggag gtcaagaaga gcctaaagaa atactacccg cagttcgaga gcaaagacag 1920

gatgcgacaa tccaaggctt ccaaggaact tatcgagaag agagctgctc ttatggagaa 1980

attccaagcc tacagagagg agaggatcga ggagtacttg gaccaaaaat caaggcgttt 2040

gcagcttaga aataacattg acactgatga attaaatgca gagaacgttg aggaggaaat 2100

cgtagaattc tttattaaag aagaagtaat tccagtcgac tagcaccatc ttgaaagtcc 2160

ggtcctttcc ctgcgtgtgc tttgttacaa cacattcagg gagatttaaa gtgaaaacgt 2220

taaatattat aatataaaaa atatttgtat tttggacatt gacataaata tttgttaatc 2280

aagtgtctcg ttcaattaat agtgttttat atttttgaca cgcgtaccat ctttccaaaa 2340

actaatgaac attgttatta aacgccttgt ggttactaaa ataaaaaatt caataag 2397

<210> 521
<211> 639
<212> DNA
<213> Diabrotica virgifera

<220>
<221> misc_feature
<222> (1)..(639)
<223> RNAi target sequence

<400> 521
ccgtgatatg ggttacaacg tatctatgat ggctgactcg acatcacgtt gggccgaagc 60

tttgagagaa atttcaggtc gtttggctga aatgcctgcc gattccggtt atccggctta 120

cttaggtgcc cgtttggctt ccttctacga acgtgctggt cgcgttaaat gtttaggtaa 180

tccagacaga gaaggatccg tttcaattgt aggagccgta tcacctcctg gtggtgattt 240

ctcagatcct gttaccactg ctactcttgg tattgtacag gtgttctggg gtttggacaa 300

gaaacttgcc caacgtaagc acttcccttc agtagactgg cttggatcat attccaaata 360

tttaagagca ttggacgact tttatgacaa aaacttccaa gagtttattc ctcttagaac 420

caaagttaag gaaattcttc aggaagaaga tgatctagcc gaaattgtgc agctggtagg 480

taaagcatct ctggcagaaa cggacaaaat caccttggaa attgccaggc ttcttaaaga 540

agatttcttg caacaaaact catactcttc ttatgacaga ttctgtccat tctataaaac 600

tgtcggtatg ttgagaaaca tgatcggttt gtacgacat 639

<210> 522
<211> 22
<212> DNA
<213> Artificial Sequence

<220>
<223> primer

<220>
<221> misc_feature
<222> (1)..(22)
<223> forward primer

<400> 522
ccgtgatatg ggttacaacg ta 22

<210>  523
<211>  22
<212>  DNA
<213>  Artificial Sequence

<220>
<223>  primer


<220>
<221>  misc_feature
<222>  (1)..(22)
<223>  reverse primer

<400>  523
atgtcgtaca aaccgatcat gt                                                22


<210>  524
<211>  2533
<212>  DNA
<213>  Diabrotica virgifera


<220>
<221>  misc_feature
<222>  (1)..(2533)
<223>  WCRW transcript ta01291.009_diavv

<400>  524
cggagatgaa gagaagggtc agtcagctga cttatcttat gacaagttgt tcagtttggg      60

aaaactcgtc cataatgggg atagttgagt gggtgaggtt ccaagagaaa cataacatcc     120

atccacaaat atgtcgaaag taaggatcgg agatgaagag aaggaagggc agtatggtta     180

tgtccatgct gtctcaggtc cagtcgttac tgctgagaaa atgtctggtt ctgctatgta     240

cgaactggta cgtgtcggat actatgagct ggtaggagaa atcattagat tggaaggtga     300

catggctact attcaggtat acgaagaaac atcaggtgta actgttggtg atccagtatt     360

aagaactggt aaaccacttt cagtagaact tggacctggt attatgggtt ccatttttga     420

tggtatccaa cgtccattga aagacatttg tgacgctact gatagtattt acatccccaa     480

gggtattaat gtaccttctt tatcgagaac agcaaaatgg gacttcaacc caatcaacat     540

caagttggga tctcacttaa ctggaggtga tatatatggt ctagttcatg aaaacaccct     600

tgtcaaacac aaaatgattc tgcctcctag agctaagggt actgtaacct acattgcaga     660

accaggaaac tacactgttg atgaagtagt attggaaact gaatttgatg gtgatcgtac     720

caaatatact atgttgcaag tatggcctgt acgtcaagca aggccagtca gtgaaaaatt     780

acctgccaac catcctctgc ttacaggaca gcgtgtactt gatgctcttt tcccatgtgt     840

acagggtggt actactgcca ttcccggagc tttcggttgt ggaaaaactg taatttcaca     900

atctctttcc aaatattcca actctgatgt cattatctac gtcggttgcg gagaaagagg     960

taacgaaatg tctgaagtat tgagagattt ccctgaattg actgttgaaa ttgacgggca 1020

cactgaatct attatgaaac gtaccgcatt ggtcgccaac acatctaaca tgcctgtagc 1080

tgctcgtgaa gcttctatct atactggtat tactctttct gaatacttcc gtgatatggg 1140

ttacaacgta tctatgatgg ctgactcgac atcacgttgg gccgaagctt tgagagaaat 1200

ttcaggtcgt ttggctgaaa tgcctgccga ttccggttat ccggcttact taggtgcccg 1260

tttggcttcc ttctacgaac gtgctggtcg cgttaaatgt ttaggtaatc cagacagaga 1320

aggatccgtt tcaattgtag gagccgtatc acctcctggt ggtgatttct cagatcctgt 1380

taccactgct actcttggta ttgtacaggt gttctggggt ttggacaaga aacttgccca 1440

acgtaagcac ttcccttcag tagactggct tggatcatat tccaaatatt taagagcatt 1500

ggacgacttt tatgacaaaa acttccaaga gtttattcct cttagaacca aagttaagga 1560

aattcttcag gaagaagatg atctagccga aattgtgcag ctggtaggta aagcatctct 1620

ggcagaaacg gacaaaatca ccttggaaat tgccaggctt cttaaagaag atttcttgca 1680

acaaaactca tactcttctt atgacagatt ctgtccattc tataaaactg tcggtatgtt 1740

gagaaacatg atcggtttgt acgacatggc gagacacgct gtagaatcaa ccgcacaatc 1800

agaaaataag atcacttgga acgtaataag agattcaatg agtggaattt tatatcaact 1860

tagcagtatg aaatttaagg atcccgtaaa agatggtgaa gctaaaatca aggcagattt 1920

tgatcaatta tatgaagata ttcagcaggc cttcagaaac ttagaagatt aaatcttttt 1980

aaggaaattt tcctattttg ttcatcagtg taagtttaaa aatatagcga tatttatcaa 2040

aaagaataat aaggcctcta tccctcactt ctgtgaatat taatatggcc gtactaatga 2100

tagtaactaa agataggttt tctctttttt gatattatcc tgtacaaaat aaattatgta 2160

aattgttgaa tatgtgtata gtttttttgg gtgagggtac agtgcttatt aaatactttt 2220

taaacatttt tcccgccatt ccaattacta ttaagttttt tcgttttaat acttttttaa 2280

atatacaggt gcttaatatc gtttatattt tcagtattac ttggttttct tcatgtaaat 2340

tgttttaaat ttttctttta cccttttaat cttgtatatt acattaccca attaaagtta 2400

attgtacaga ttaagataaa cgagtatctt ataacatcta ttagattgtt agaatcaata 2460

aatgtagtgt aattgttctg ttttgaacaa ataaatgcat cattattgtt gttaaaaaaa 2520

aaaaaaaaaa aaa 2533

<210> 525
<211> 507
<212> DNA
<213> Diabrotica virgifera

<220>

<221>  misc_feature
<222>  (1)..(507)
<223>  RNAi target sequence

<400>  525
ggggtccttt tactgtgttt cttaggattt ggaaggtaca aaatgaccaa ctctaaaggt      60

taccgccgag gaaccaggga tctatttgcc cgcaagttta aaaaacgtgg tgtaattcca     120

ctttccacat atttgagagt ctacaaagtt ggagatattg tagatatcaa gggtaatggt     180

gcagttcaaa agggtatgcc ccacaaagtg taccatggta agaccggacg tgttttcaat     240

gttactgcac atgcattagg tgtaattgta aacaaaaggg ttcgaggaag aatcatcccc     300

aaaagaatca atctccgtat tgaacatgta aaccactcca agtgtcgtca agacttcttg     360

caaagagtaa aatccaatga aaagctacgt aaagaagcta aagaaaagaa cattaaagta     420

gaacttagga gacaacctgc ccaacctagg ccagcacata ttgttagcgg aaaggttcca     480

gcacaggtgc ttgctcctat cccatat                                         507


<210>  526
<211>  20
<212>  DNA
<213>  Artificial Sequence

<220>
<223>  primer


<220>
<221>  misc_feature
<222>  (1)..(20)
<223>  forward primer

<400>  526
gtccttttac tgtgtttctt                                                  20


<210>  527
<211>  21
<212>  DNA
<213>  Artificial Sequence

<220>
<223>  primer


<220>
<221>  misc_feature
<222>  (1)..(21)
<223>  reverse primer

<400>  527
atatgggata ggagcaagca c                                                21


<210>  528
<211>  696
<212>  DNA

<213>  Diabrotica virgifera

<220>
<221>  misc_feature
<222>  (1)..(696)
<223>  WCRW transcript ta01222.003_diavv

<400>  528
cgataactga gtttccttca atgataagtt catccttttg tttggtggag tttactactg      60

gaacacctgg ggggagaggg ggacagcgac atttcctccg ccgggtcctt ttactgtgtt     120

tcttaggatt tggaaggtac aaaatgacca actctaaagg ttaccgccga ggaaccaggg     180

atctatttgc ccgcaagttt aaaaaacgtg gtgtaattcc actttccaca tatttgagag     240

tctacaaagt tggagatatt gtagatatca agggtaatgg tgcagttcaa aagggtatgc     300

cccacaaagt gtaccatggt aagacaggac gtgttttcaa tgttactgca catgcattag     360

gtgtaattgt aaacaaaagg gttcgaggaa gaatcatccc caaaagaatc aatctccgta     420

ttgaacatgt aaaccactcc aagtgtcgtc aagacttctt gcaaagagta aaatccaacg     480

aaaagctacg taaagaagct aaagaaaaga acgttaaagt agaacttagg agacaacctg     540

cccaacctag gccagcacat attgttagcg gaaaggttcc agcacaggtg cttgctccta     600

tcccatatga attcattgct taggtttgtt tatcttaaaa taaaatcctt tatataataa     660

aaaaaaaaag aaaaaatcgg cagatccctt aaaaaa                               696

<210>  529
<211>  519
<212>  DNA
<213>  Diabrotica virgifera

<220>
<221>  misc_feature
<222>  (1)..(519)
<223>  RNAi target sequence

<400>  529
ggtcagtgaa gccatcttgg caaacgttag taacgtgaaa taaaacttaa attttttcaa      60

aatgggtcgt atgcacgcac caggaaaagg tattgcccag tcggcattgc catacagaag     120

gagtgtacca acatggttga aagtcacacc agaagaagta aaagaccata tttttaaact     180

tggcaagaaa ggcttgactc catcacaaat tggtgttatc ctcagggatt catatggtgt     240

tgcccaagta aggtttgttt ctggaaacaa aatcttgcgt atcatgaaag ctatgggtct     300

tgcccctgat ctaccagaag atttgtacta ccttatcaag aaggcagtag ctatccgcaa     360

acatttagaa cgtaacagaa aagacaagga cagcaaattc cgtttgattt tggtagaatc     420

acgtatccac cgtttggcta ggtactacaa aaccaagagc gtattggcac ccaactggaa     480

gtacgaatca agcacagcat ctgctttggt cgcttaaat                            519

<210>  530
<211>  19
<212>  DNA
<213>  Artificial Sequence

<220>
<223>  primer


<220>
<221>  misc_feature
<222>  (1)..(19)
<223>  forward primer

<400>  530
gtcagtgaag ccatcttgg                                                    19


<210>  531
<211>  22
<212>  DNA
<213>  Artificial Sequence

<220>
<223>  primer


<220>
<221>  misc_feature
<222>  (1)..(22)
<223>  reverse primer

<400>  531
atttaagcga ccaaagcaga tg                                                22


<210>  532
<211>  596
<212>  DNA
<213>  Diabrotica virgifera


<220>
<221>  misc_feature
<222>  (1)..(596)
<223>  WCRW transcript ta05572.002_diavv

<400>  532
ggggctgggc tcagtgaagc catcttggca aacgttagta acgtgaaata aaacttaaat      60

tttttcaaaa tgggtcgtat gcacgcacca ggaaaaggta ttgcccagtc ggcattgcca     120

tacagaagga gtgtaccaac atggttgaaa gtcacaccag aagaagtaaa agaccatatt     180

tttaaacttg gcaagaaagg cttgactcca tcacaaattg gtgttatcct cagggattca     240

tatggtgttg cccaagtaag gtttgtttct ggaaacaaaa tcttgcgtat catgaaagct     300

atgggtcttg cccctgatct accagaagat ttgtactacc ttatcaagaa ggcagtagct     360

atccgcaaac atttagaacg taacagaaaa gacaaggaca gcaaattccg tttgattttg     420

gtagaatcac gtatccaccg tttggctagg tactacaaaa ccaagagcgt attggcaccc 480

aactggaagt acgaatcaag cacagcatct gctttggtcg cttaaattgt gcttttatgt 540

taagtttata aaataaaaat ttctactaaa aaaaaaaaaa aaaaatcgga aaaaaa 596

<210> 533
<211> 600
<212> DNA
<213> Diabrotica virgifera

<220>
<221> misc_feature
<222> (1)..(600)
<223> RNAi target sequence

<400> 533
ggacagcttt tcactgtcat tcgtcattct gatacatttt tttgggtact ttgggttgta 60

cttgggataa aagacctttt tgtatttcca aatacattgt catataaatt aaaaaagaaa 120

tggctgccat cagatctatg tctcgccttc ttattaatag atcacttcaa accactgcaa 180

agaggaccta tgccgaccaa atgaacttca catttgcagc tggaaatcag gtttactata 240

ctgcaacttc agttaaacaa gtagatgtgc catctttctc tggtagcttt ggtatcctcc 300

ccgcccatgt acccacactg gctgtgctaa aacctggagt agtcactgtt tacgaagatg 360

gtggtaatgt taagaaaatc tttgtttcca gtggtactgt caccatcaat gatgattcat 420

cagttcagat tcttgctgaa gaagcccacc cagtagaaaa cattgatgtc aatgcagcaa 480

gagacatcct taccaaagcc cagagccagt tatcatctgc aagtacagat gaggcgaaag 540

ctgaagcctc cattgccgtt gaagtgggag aagcgttagt caaagcggct gaataataat 600

<210> 534
<211> 22
<212> DNA
<213> Artificial Sequence

<220>
<223> primer

<220>
<221> misc_feature
<222> (1)..(22)
<223> forward primer

<400> 534
ggacagcttt tcactgtcat tc 22

<210> 535
<211> 22
<212> DNA
<213> Artificial Sequence

<220>
<223>  primer


<220>
<221>  misc_feature
<222>  (1)..(22)
<223>  reverse primer

<400>  535
attattattc agccgctttg ac                                                22


<210>  536
<211>  797
<212>  DNA
<213>  Diabrotica virgifera


<220>
<221>  misc_feature
<222>  (1)..(797)
<223>  WCRW transcript ta03508.001_diavv

<400>  536
ggtaggggag tgacagacag tcaggtggac agcttttcac ggggattcgt cattctgata      60

catttttttt gggtactttg ggttgtactt gggataaaag acctttttgt atttccaaat      120

acattgtcat ataaattaaa aaagaaatgg ctgccatcag atctatgtct cgccttctta     180

ttaatagatc acttcaaacc actgcaaaga ggacctatgc cgaccaaatg aacttcacat     240

ttgcagctgg aaatcaggtt tactatactg caacttcagt taaacaagta gatgtgccat     300

ctttctctgg tagctttggt atcctccccg cccatgtacc cacactggct gtgctaaaac     360

ctggagtagt cactgtttac gaagatggtg gtaatgttaa gaaaatcttt gtttccagtg     420

gtactgtcac catcaatgat gattcatcag ttcagattct tgctgaagaa gcccacccag     480

tagaaaacat tgatgtcaat gcagcaagag acatccttac caaagcccag agccagttat     540

catctgcaag tacagatgag gcgaaagctg aagcctccat tgccgttgaa gtgggagaag     600

cgttagtcaa agcggctgaa taataattta atttttgtaa ttttatgtaa atttaataaa     660

ttagtgtctc aatggtactg ttcattttaa aacaacaaca tattagatct atacctctat     720

gagttacaat ttttacaaaa acgagaatca atccaaatac ataatttttg tttaccgtta      780

aaaaaaaaaa aaaaaaa                                                    797


<210>  537
<211>  612
<212>  DNA
<213>  Diabrotica virgifera


<220>
<221>  misc_feature

<222>   (1)..(612)
<223>   RNAi target sequence

<400>   537
gggggtttgt aaatctagtc attacagaaa cagtaataat aaattacctt gttctgatta        60

ttttgtagaa attgtaaaat ggaaaagtgg tgtttttgtt tgtttcttct ttttatatgg       120

tttgtttccg cacgtggtgc caacaactcg gagtatcctg acctacggcc agaatggtcc       180

tattgttatg agttcacttg gtttgggcca gattataata acgtgtccac gtacaataat       240

acgtgtgccg attatctgga cgaaacaaga gccgaaggta taccctgcgc tgctcccatc       300

gtaatatcgt atgatggaac gttaccggat atgcagtatc tctgggacaa ccacaaagaa       360

agcgtactct gcaaacgcag cataaaccag atgtgtgtca agtacactta cctctttaac       420

cagaaaattg aaaatattac ctacatgtgt tctaaagttc agcatgaagg tgggggaaat       480

attgacagtg gatgctataa acagaaattg gaaagaggat atgaaacaga agtgtgtgta       540

tgtcaatcct ccaatggaat ttatcctccc tgcaacagaa gtgcctccat atttggttcg       600

atcaatgttt tc                                                           612


<210>   538
<211>   19
<212>   DNA
<213>   Artificial Sequence

<220>
<223>   primer


<220>
<221>   misc_feature
<222>   (1)..(19)
<223>   forward primer

<400>   538
gtttgtaaat ctagtcatt                                                     19


<210>   539
<211>   22
<212>   DNA
<213>   Artificial Sequence

<220>
<223>   primer


<220>
<221>   misc_feature
<222>   (1)..(22)
<223>   reverse primer

<400>   539
gaaaacattg atcgaaccaa at                                                 22

<210>  540
<211>  893
<212>  DNA
<213>  Diabrotica virgifera

<220>
<221>  misc_feature
<222>  (1)..(893)
<223>  WCRW transcript ta03675.001_diavv

<400>  540
ggtttgtaaa tctagtcatt acagaaacag taataataaa ttaccttgtt ctgattattt      60

tgtagaaatt gtaaaatgga aaagtggtgt ttttgtttgt ttcttctttt tatatggttt     120

gtttccgcac gtggtgccaa caactcggag tatcctgacc tacggccaga atggtcctat     180

tgttatgagt tcacttggtt tgggccagat tataataacg tgtccacgta caataatacg     240

tgtgccgatt atctggacga aacaagagcc gaaggtatac cctgcgctgc tcccatcgta     300

atatcgtatg atggaacgtt accggatatg cagtatctct gggacaacca caaagaaagc     360

gtactctgca aacgcagcat aaaccagatg tgtgtcaagt acacttacct ctttaaccag     420

aaaattgaaa atattaccta catgtgttct aaagttcagc atgaaggtgg gggaaatatt     480

gacagtggat gctataaaca gaaattggaa agaggatatg aaacagaagt gtgtgtatgt     540

caatcctcca atggaattta tcctccctgc aacagaagtg tctccatatt tggttcgatc     600

aatgttttcc tagtatttgg aatactatgg ttatttgttt ataaattatt ttgactaaaa     660

ttgttgttat tcatatctct gtataaaact ttaaaaatct caatacttac taaatttcaa     720

taaggataat aaattatttt aaatatgcag tattctatta ttttactgac tattgcaaat     780

gccactatca tattttgcaa cttacacgac ctatttgaaa taccaccttc tatacctttt     840

tgtcttttag aaattgtcgg tcagatagtc aagagaggca gatgtcatta ata            893

<210>  541
<211>  558
<212>  DNA
<213>  Diabrotica virgifera

<220>
<221>  misc_feature
<222>  (1)..(558)
<223>  RNAi target sequence

<400>  541
cgggggagtg attatctttg taaaatggca cctacgaagc caaaagcagc ggataaatcc      60

gcccccaaga aggaagaaaa gaagaagcct gcggccgcac cagcagccgg agccgctgga     120

gcttcttcct caagttcaaa aacacccgcc ccagctccaa aagcagcagc caaagctgca     180

agcaaaccag ctgacaagaa agcatcttcc tcaaaaaccc ctgcacccgc acctaaagct     240

gcctcagcca aaagctccgc gaaagcgcaa gctgctgccc ccaaaccaaa agccgctgct 300

gctaaagctg gaaaggctgc aggcaaaggt gctgccaaac ccgcagccaa ggctgcagga 360

aagaaaccag ctgctgctgg aaaggccaag gctcccgcaa ctcttattgc taagcccaag 420

aagaatgttc ctgttaaaca acaacagaaa ggtgttggca aaaagccagc tgccagtgca 480

gtgcaagtag cgagagcact taaaatccaa aagaaagtaa ttaaaggccc caatggaaca 540

cactcaagga aaatccgt 558

<210> 542
<211> 22
<212> DNA
<213> Artificial Sequence

<220>
<223> primer

<220>
<221> misc_feature
<222> (1)..(22)
<223> forward primer

<400> 542
cgggggagtg attatctttg ta 22

<210> 543
<211> 22
<212> DNA
<213> Artificial Sequence

<220>
<223> primer

<220>
<221> misc_feature
<222> (1)..(22)
<223> reverse primer

<400> 543
acggattttc cttgagtgtg tt 22

<210> 544
<211> 1017
<212> DNA
<213> Diabrotica virgifera

<220>
<221> misc_feature
<222> (1)..(1017)
<223> WCRW transcript ta00525.004_diavv

<400> 544
gacgcttagg ggaggggcct tttagtgatt atctttgtaa aatggcacct acgaagccaa 60

aagcagcgga taaatccgcc cccaagaagg aagaaaagaa gaagcctgcg gccgcaccag 120

cagccggagc cgctggagct tcttcctcaa gttcaaaaac acccgcccca gctccaaaag 180

cagcagccaa agctgcaagc aaaccagctg acaagaaagc atcttcctca aaaacccctg 240

cacccgcacc taaagctgcc tcagccaaaa gctccgcgaa agcgcaagct gctgccccca 300

aaccaaaagc cgctgctgct aaagctggaa aggctgcagg caaaggtgct gccaaacccg 360

cagccaaggc tgcaggaaag aaaccagctg ctgctggaaa ggccaaggct cccgcaactc 420

ttattgctaa gcccaagaag aatgttcctg ttaaacaaca acagaaaggt gttggcaaaa 480

agccagctgc cagtgcagtg caagtagcga gagcacttaa aatccaaaag aaagtaatta 540

aaggccccaa tggaacacac tcaaggaaaa tccgtaactc agtccacttc cgtcgtccta 600

agaccctcag accacccagg agtcccaaat accccaagag gagtgttccc cacagatccc 660

gcatggatgc ttacaatatc atcaaattcc cattgaccac cgaagcagcc atgaagaaga 720

ttgaagataa caacacgttg gtatttttag tacacactag ggcaaacaag caccacatta 780

aagctgcagt taagaaattg tatgatatca atgtagccaa ggtcaacacc ttaattaggc 840

ccgatggcaa aaagaaggca tatgtaaggt tagctagaga ttatgatgct ctagatgttg 900

ccaacaagat tggcatcata taaattaata tcttaccttg ttttattttt tttgacatct 960

aataaataag ttgagaataa agttttttag tttgagttaa aaaaaaaaaa aatgcga 1017

<210> 545
<211> 544
<212> DNA
<213> Diabrotica virgifera

<220>
<221> misc_feature
<222> (1)..(544)
<223> RNAi target sequence

<400> 545
atgcgtgttg aggtcttttc ttttcccaaa acttgacaaa aatgggtatc gacatcaacc 60

ataaatacga ccgtaaggtt aggagaacag aacctagaag tcaagatgta tatttacgtc 120

ttttagtaaa actgtaccgt tatttagccc gtcgtacagg ggccaaattc aataaaataa 180

tcctgaaaag attgtttatg agcaaaatca acagaccacc actatcttta tccaagttag 240

tgcgtttgat gaagaagcct ggccgtgatg gacttattgc tgtcgtagta ggaacaatta 300

ctgatgatgc taggattttt gaaattccca aattgtctgt atgtgccctt cgcgtgacac 360

aaactgctag ggccaggatc ttgaaagctg gaggtgaagt catcaccttc gaccaattgg 420

cactcagggc cccaactggc aataaaactg tattgctaca aggcagacgt aatgcccgtg 480

aagctgtgaa acactttggt cctgctcctg gtgtaccaca cagtcatgct aagccattgg 540

taca                                                                 544

<210>  546
<211>  22
<212>  DNA
<213>  Artificial Sequence

<220>
<223>  primer

<220>
<221>  misc_feature
<222>  (1)..(22)
<223>  forward primer

<400>  546
atgcgtgttg aggtcttttc tt                                              22

<210>  547
<211>  22
<212>  DNA
<213>  Artificial Sequence

<220>
<223>  primer

<220>
<221>  misc_feature
<222>  (1)..(22)
<223>  reverse primer

<400>  547
tgtaccaatg gcttagcatg ac                                              22

<210>  548
<211>  793
<212>  DNA
<213>  Diabrotica virgifera

<220>
<221>  misc_feature
<222>  (1)..(793)
<223>  WCRW transcript ta05162.001_diavv

<400>  548
ggctgtcggg ggctgggggg ctaaccaaaa ttatacacat gcgtgttgag gtcttttctt      60

ttcccaaaac ttgacaaaaa tgggtatcga catcaaccat aaatacgacc gtaaggttag     120

gagaacagaa cctagaagtc aagatgtata tttacgtctt ttagtaaaac tgtaccgtta     180

tttagcccgt cgtacagggg ccaaattcaa taaaataatc ctgaaaagat tgtttatgag     240

caaaatcaac agaccaccac tatctttatc caagttagtg cgtttgatga agaagcctgg     300

ccgtgatgga cttattgctg tcgtagtagg aacaattact gatgatgcta ggatttttga      360

aattcccaaa ttgtctgtat gtgcccttcg cgtgacacaa actgctaggg ccaggatctt 420

gaaagctgga ggtgaagtca tcaccttcga ccaattggca ctcagggccc caactggcaa 480

taaaactgta ttgctacaag gcagacgtaa tgcccgtgaa gctgtgaaac actttggtcc 540

tgctcctggt gtaccacaca gtcatgctaa gccattggta cattccaagg gacgcaaatt 600

tgagagagca cgaggtcgta ggcgctcatg tggttacaag aaatagactt aagttaatca 660

ttttgtttct gctgtattca tatttgttgt tcaataaata ggaattgcag tacattggaa 720

ttttgttttg tattttatag aatataacaa aataaaggaa tttaatataa aaaaaaaaaa 780

aaaaaaaaaa aaa 793

<210> 549
<211> 401
<212> DNA
<213> Diabrotica virgifera

<220>
<221> misc_feature
<222> (1)..(401)
<223> RNAi target sequence

<400> 549
aaagaaaccg aaggcagaga aaaaggcacc aaaggagggt gcaactccaa gtgataaacc 60

ctccgttaaa agacgtcctt tcaaaagaca tggacgttta tatgctaaag ccatcttcac 120

aggttacaaa agaggtcttc gtaatcaaca cgaaaataca gccctattat ctgtagaagg 180

aaccagatca aaagatgaga gttggtttta cgtaggaaag aaatgcgtct atgtatacaa 240

agcaaaaaac aggacatgtg ttcccggtag gcccaaatct gtcaaatcca aagttagggc 300

tatttgggga aaagtcacca gaccacatgg cacaaggggt gcagtgagag ccaaattcaa 360

taggaattta ccagccaagg ctatgggaca tcgtattcgt a 401

<210> 550
<211> 21
<212> DNA
<213> Artificial Sequence

<220>
<223> primer

<220>
<221> misc_feature
<222> (1)..(21)
<223> forward primer

<400> 550
aaagaaaccg aaggcagaga a 21

<210> 551

<211>   22
<212>   DNA
<213>   Artificial Sequence

<220>
<223>   primer


<220>
<221>   misc_feature
<222>   (1)..(22)
<223>   reverse primer

<400>   551
tacgaatacg atgtcccata gc                                                22


<210>   552
<211>   1008
<212>   DNA
<213>   Diabrotica virgifera


<220>
<221>   misc_feature
<222>   (1)..(1008)
<223>   WCRW transcript ta00344.005_diavv

<400>   552
ccgatttttt tttttttttt ttgtgttgga aaacgtattt tttatttctt atttatattt       60

ataaaaacaa ctatatctta gatgggtaca gcataatacg aatacgatgt cccatagcct      120

tggctggtaa attcctattg aatttggctc tcactgcacc ccttgtgcca tgtggtctgg      180

tgacttttcc ccaaatagcc ctaactttgg atttgacaga tttgggccta ccgggaacac      240

atgtcctgtt ttttgctttg tatacataga cgcatttctt tcctacgtaa aaccaactct      300

catcttttga tctggttcct tctacagata atagggctgt attttcgtgt tgattacgaa      360

gacctctttt gtaacctgtg aagatggctt tagcatataa acgtccatgt cttttgaaag      420

gacgtctttt aacggagggt ttatcacttg gagttgcacc ctcctttggt gcctttttct      480

ctgccttcgg tttctttgcc gctgcgtctg ccattccgaa gatctcgttt agaattaaaa      540

agaagttatt tcatttttct cctccaagtg ataaaccctc cgttaaaaga cgtcctttca      600

aaagacatgg acgtttatat gctaaagcca tcttcacagg ttacaaaaga ggtcttcgta      660

atcaacacga aaatacagcc ctattatctg tagaaggaac cagatcaaaa gatgagagtt      720

ggttttacgt aggaaagaaa tgcgtctatg tatacaaagc aaaaaacagg acatgtgttc      780

ccggtaggcc caaatctgtc aaatccaaag ttagggctat ttggggaaaa gtcaccagac      840

cacatggcac aaggggtgca gtgagagcca aattcaatag gaatttacca gccaaggcta      900

tgggacatcg tattcgtatt atgctgtacc catctaagat atagttgttt ttataaatat      960

aaataagaaa taaaaaatac gttttccaac aaaaaaaaaa aaaaaaaa                  1008

<210>  553
<211>  542
<212>  DNA
<213>  Diabrotica virgifera

<220>
<221>  misc_feature
<222>  (1)..(542)
<223>  RNAi target sequence

<400>  553
aatctgtaaa catcggcgta gaatcgccat taatatataa gtattttata tagaattaat      60

tcaaaatgcc acccaagttc gatcccaccg aaattaaata tgtttacatg agatgtgtcg     120

gaggagaagt tggagctacc tcttctttgg ctcctaaaat tggtccacta ggtttgtctc     180

ccaaaaaagt tggtgacgat attgccaaag gcactgctga ctggaaaggt cttaaaatca     240

cagttcagtt gaaaatccaa aatcgtcaag ctaccatttc tgtagtccca tctgcctcct     300

ccctgatcat tagggctctt aaagaaccac cacgcgatag gaagaagcaa aagaacatta     360

aacacactgg taacattact ttcgatgaaa tcatcgctat tgccagacag atgaggccca     420

ggtcaatggc caaacagtta tccggaactg taaaagaggt tttaggaaca gcccaatcag     480

ttggatgtac aattgatggt aaagcagcac atgatgttat tgacgaggtt aatgaaggaa     540

cc                                                                    542

<210>  554
<211>  23
<212>  DNA
<213>  Artificial Sequence

<220>
<223>  primer

<220>
<221>  misc_feature
<222>  (1)..(23)
<223>  forward primer

<400>  554
aatctgtaaa catcggcgta gaa                                              23

<210>  555
<211>  21
<212>  DNA
<213>  Artificial Sequence

<220>
<223>  primer

<220>
<221>  misc_feature

<222>   (1)..(21)
<223>   reverse primer

<400>   555
gttccttcat taacctcgtc a                                                 21


<210>   556
<211>   768
<212>   DNA
<213>   Diabrotica virgifera


<220>
<221>   misc_feature
<222>   (1)..(768)
<223>   WCRW transcript ta01789.001_diavv

<400>   556
ggaagatgat tgaccatgtc aattgtcacg tcatgtgtca ggggtccttt tcgaatttac        60

cattgtgaaa atattttcac tattgtaaaa atctgtaaac atcggcgtag aatcgccatt       120

aatatataag tattttatat agaattaatt caaaatgcca cccaagttcg atcccaccga       180

aattaaatat gtttacatga gatgtgtcgg aggagaagtt ggagctacct cttctttggc       240

tcctaaaatt ggtcctctag gtttgtctcc caaaaaagtt ggtgacgata ttgccaaagg       300

tactgctgat tggaaaggtc ttaaaatcac agttcaattg aaaatccaaa atcgtcaagc       360

taccatttct gtagtcccat ctgcttcctc cctgatcatt agggctctta aagaaccacc       420

acgcgatagg aagaagcaaa agaacattaa acacactggt aacattactt tcgatgaaat       480

catcgctatt gccagacaga tgaggcccag gtcgatggcc aaacagttat ccggaactgt       540

aaaagaggtt ttaggaacag cccaatcagt tggatgtaca attgatggta aagcagcaca       600

tgatgttatt gacgaggtta atgaaggaac catagaagta ccagatgaat aaactattat       660

tagaatattt tttctaagtt atcagttgtt ttatttaacc tttatccaaa tgtggcattt       720

tttaatttgt taagaatgta attgatggat tcgaccgtta cttgatgg                    768


<210>   557
<211>   450
<212>   DNA
<213>   Diabrotica virgifera


<220>
<221>   misc_feature
<222>   (1)..(450)
<223>   RNAi target sequence

<220>
<221>   misc_feature
<222>   (341)..(341)
<223>   n can be any nucleotide

<220>

<221>  misc_feature
<222>  (413)..(413)
<223>  n can be any nucleotide

<220>
<221>  misc_feature
<222>  (426)..(426)
<223>  n can be any nucleotide

<400>  557
aggcatttca aaaacaacca accgtttttc tcaaccgcaa aagtggagga aagaaaaatt      60

taagacattc tcgcagtgta gggctcggtt ttaaaactcc cagagaggca attgaaggtt      120

cttgcattga caagaaatgt cccttcactg gaaatgtatc aatccgtggc cgtattctca      180

ctggagttgt ccaaaaaatg aagatgcaaa ggactattgt catccgtaga gattatttgc      240

attatatcag gaaatacaat cgttttgaaa agaggcatag gaatatgtct gtacatttat      300

caccctgctt cagggatgtg gaaattggag atgtagttac nattggtgaa tgcagaccac      360

tttcaaaaac agttagattc aacgtattga aagtaactaa aggtagcagt tcnaagaaga      420

gcttcnggaa gttttgagtg atgaaacatt                                        450


<210>  558
<211>  22
<212>  DNA
<213>  Artificial Sequence

<220>
<223>  primer


<220>
<221>  misc_feature
<222>  (1)..(22)
<223>  forward primer

<400>  558
aggcatttca aaaacaacca ac                                                22


<210>  559
<211>  24
<212>  DNA
<213>  Artificial Sequence

<220>
<223>  primer


<220>
<221>  misc_feature
<222>  (1)..(24)
<223>  reverse primer

<400>  559
aatgtttcat cactcaaaac ttcc                                              24

<210>  560
<211>  615
<212>  DNA
<213>  Diabrotica virgifera

<220>
<221>  misc_feature
<222>  (1)..(615)
<223>  WCRW transcript ta02086.002_diavv

<400>  560
cttgtcaatg taagaacctt caattgcctc tctgggagtt ttaaaaccga gccctacact      60

gcgagaaggt cataaagggg aatcctttct tttactagtg aaaatggcgg atcagaccga      120

aaaggcattt caaaaacaac caaccgtttt tctcaaccgc aaaagtggag gaaagaaaaa      180

tttaagacat tctcgcagtg tagggctcgg ttttaaaact cccagagagg caattgaagg      240

ttcttacatt gacaagaaat gtcccttcac tggaaatgta tcaatccgtg gccgtattct      300

cactggagtt gtccaaaaaa tgaagatgca aaggactatt gtcatccgta gagattattt      360

gcattatatc aggaaataca atcgttttga aaagaggcat aggaatatgt ctgtacattt      420

atcaccctgc ttcagggatg tggaaattgg agatgtagtt acaattggtg aatgcagacc      480

actttcaaaa acagttagat tcaacgtatt gaaagtaact aaaggtagca gttcaaagaa      540

gagcttcagg aagttttgag tgatgaaaca tttttaaaat aaaaatctta taaaatgaaa      600

aaaaaaaaaa aaaaa                                                       615

<210>  561
<211>  225
<212>  DNA
<213>  Diabrotica virgifera

<220>
<221>  misc_feature
<222>  (1)..(225)
<223>  RNAi target sequence DV-HP2-FRAG1

<400>  561
gaaacgacaa ggaggaaagt tgtgtcttgc ggagtactag aaacaccgag attcggacga      60

aagagtactt tcctgttcgt gcccggtacg aaggttacct atgaatgcat ccaagagttc      120

gtgttggtag gtgatcagcg gagagagtgt caagcggatg gaacatggaa tattcctgaa      180

tacggataca catattgctt acgtcagcag gaatactctc aacga                      225

<210>  562
<211>  212
<212>  DNA
<213>  Diabrotica virgifera

<220>

<221>  misc_feature
<222>  (1)..(212)
<223>  RNAi target sequence DV-HP2-FRAG2

<400>  562
gagcattgtt ccacagggaa ttcggaagga cttcagcgac ttacaacaac aaaacgttca      60

agcctcaatt cctaatgaat ccggaagatt tcctaccaac caacagatct acagatctta     120

agcgaatatc cgaaatatgt cctctaaagt tgtacgaatg ttactacgat tacgctatga     180

cggttgacag ggatttggcg cattacacca ag                                   212


<210>  563
<211>  210
<212>  DNA
<213>  Diabrotica virgifera


<220>
<221>  misc_feature
<222>  (1)..(210)
<223>  RNAi target sequence DV-HP2-FRAG3

<400>  563
cttgggagaa gcagaatttg accacaaacg acaacgctaa catccgaatt tctctctggg      60

gttacagaga aacgactatg aaaccagtgt ttgtttatat caccgatatc gctgataatg     120

ttcaaaacat tggagaacac acgattgttc cttctcagtt tagaactaga gtcaatcaat     180

atctgactga tattaagttt ggattcctcc                                      210


<210>  564
<211>  229
<212>  DNA
<213>  Diabrotica virgifera


<220>
<221>  misc_feature
<222>  (1)..(229)
<223>  RNAi target sequence DV-HP2-FRAG4

<400>  564
aatggaaagg gagaattcgc attggtgaaa tcagttacgc aaacggacaa tctggaaatc      60

caaggaagat ttgagcaaat ggaccctaac acttatggtg aggttagagc tactcagctg     120

acttctgtag tagctaaagg taataacaca atagcaattg aagtcagaag gagacctctg     180

gattctagat ggagatatag gttggatgtt atagcagata atagaagac                 229


<210>  565
<211>  170
<212>  DNA
<213>  Diabrotica virgifera


<220>

<221>  misc_feature
<222>  (1)..(170)
<223>  RNAi target sequence DV-HP2-FRAG5

<400>  565
gaaacgacaa ggaggaaagt tgtgtcttgc ggagtactag aaacaccgag attcggacga      60

aagagtactt tcctgttcgt gcccggtacg aaggttacct atgaatgcat ccaagagttc     120

gtgttggtag gtgatcagcg gagagagtgt caagcggatg gaacatggaa                170


<210>  566
<211>  233
<212>  DNA
<213>  Diabrotica virgifera


<220>
<221>  misc_feature
<222>  (1)..(233)
<223>  RNAi target sequence DV-HP2-FRAG6

<400>  566
gaaacgacaa ggaggaaagt tgtgtcttgc ggagtactag aaacaccgag attcggacga      60

aagagtactt tcctgttcgt gcccggtacg aaggttacct atgaatgcat ccaagagttc     120

gtgttggtag gtgatcagcg gagagagtgt caagcggatg gaacatggaa tattcctgaa     180

tacggataca catattgctt acgtcagcag gaatactctc aacgacaagc tgg            233


<210>  567
<211>  162
<212>  DNA
<213>  Diabrotica virgifera


<220>
<221>  misc_feature
<222>  (1)..(162)
<223>  RNAi target sequence DV-HP2-FRAG7

<400>  567
tttgttattt ttgttttgtt tagtgttagc gttaaaggac tagatggtga tcttccacca      60

gatgtcgaaa ctatcgatgt ggagactacg aatgtaagta acgcggatcc agacgcaggg     120

aaacctattc cggtggaaga tacgcctaat ggagtccctc ca                        162


<210>  568
<211>  473
<212>  DNA
<213>  Diabrotica virgifera


<220>
<221>  misc_feature
<222>  (1)..(473)
<223>  RNAi target sequence DV-HP2-FRAG8

<400>  568
gtgaatacaa actatgaagt agataaatcc ttagttaatg taaccaatag tttggtaaat      60

gtgactaaaa atcccagacg gacttatcag acagttaata aaatgggcaa atattcgaca     120

ttttatgctg cagattacga tcctatgacc tcagacacag ctccaccaga cactgaccaa     180

cgaggaggag gaggaactcc atacactata tcagaaccga gactggcaga aataaggaaa     240

cattttatgt acccattttt cgataaagga ggcagtgata ataacttggg agatttccaa     300

aaggacattc aatcttccat tcctcaggtg cacaagaatt taaacttcca gttgcctttc     360

tttggattca ggtataatta cacaagagtt tcagtgaatg ggtatctgga attcagcgat     420

ccacctccaa attatgacta tccattagta tttccaatta aagaatggcc caa            473


<210>  569
<211>  430
<212>  DNA
<213>  Diabrotica virgifera


<220>
<221>  misc_feature
<222>  (1)..(430)
<223>  RNAi target sequence DV-HP2-FRAG9

<400>  569
aaagaatgat cctgccttta ttggcatttt cttcagcaga tgcagaatcg gtaatctacg      60

tgatggagac attgatcaaa gaacacccgg ggtctatttt agaatggaaa gagatttaag     120

aacgaggcaa gacagaatgg gtgtagaaat tagagaaaga cttaagtggg atatcagaac     180

aggagtcatt ggatcagaaa cattcgatcc taagcacgcc gttattgtca cttggaaaaa     240

tgttactttc actggaggat tcgctaatgc taaatacaag acaaacacct tccaaatggt     300

tcttgcaact gacgaagtct tcacttacgc gatgttcaac tacctcaact tggactggac     360

ctcccacact gaagcgggtg gtgattccca aaacggggaa ggtggtgtca gtgcctatgt     420

tggttttaac                                                            430


<210>  570
<211>  486
<212>  DNA
<213>  Diabrotica virgifera


<220>
<221>  misc_feature
<222>  (1)..(486)
<223>  RNAi target sequence DV-HP2-FRAG10

<400>  570
ccagaagcta cgaatacaat ccttacagtc aagcttccgt cattcgagat ctcacatcag      60

ttggatttgg aaatggtttt aagggaagac atattttccg tatagacgaa gacattttgc     120

ttggatcgtg caacaaagat atagatggtg ccaaccttcc cctgaaattc gctcctgaaa 180

gtggtaacat gttgggtgga acagtggtaa atataactgg tccatgcttc aatttgaatg 240

ataggattcg atgcaagttt gacgtatcaa atgaagtatt tgggtatgtt gttgataaaa 300

acagagctat ctgtgtacaa cctcaacttt atgccgaagg atgggtgaat cttcaaattg 360

ctgttaattc cgaagcattc aagtggaagg ggaagtatta tgtagaatca cctgctagtg 420

cgactcaaaa aatcttcttt aaggacatga aattacacga aaaatctccg agcgaattaa 480

aaatca 486

<210> 571
<211> 500
<212> DNA
<213> Diabrotica virgifera

<220>
<221> misc_feature
<222> (1)..(500)
<223> RNAi target sequence DV-HP2-FRAG11

<400> 571
aaattaattt aactgagtct atcacagtta atacatatac tacaagccag agcagcatag 60

aattaacacc ggtaatatgg agccgaccca ttccactcgg ttggtacttc cagttccaat 120

gggaaaacca gtacggtaaa aattggccca aatatctatg tgacgattgg ctgagaacgg 180

acagatatct aaaaaacttc gcccacgaac ttgctcaatg tccctgcacc gtcgaacagg 240

ctttagctga caagggcagg tttatgccag acttcgattg cgacaaagat tcgaacccga 300

tttgttacta taacaaccaa gcacttcatt gtgtgaaaac tggatctccc actttagaag 360

gctccgaaca acaatgttgc tatgataaaa acggcttttt gatgctctct tacgatcagc 420

aatggggttc tagtccgaga cgttgccaca acttgggtaa aatgccttac aatgaagcga 480

caaaagtgcc tactctctca 500

<210> 572
<211> 310
<212> DNA
<213> Diabrotica virgifera

<220>
<221> misc_feature
<222> (1)..(310)
<223> RNAi target sequence DV-HP2-FRAG12

<400> 572
ggtgttacta tttacactcc aagctacatc cttaatcaat cagaagtcat cattatgttt 60

gacaatggag ctggaatgga ggttgtcgac aatcaaggat tcatgagtgc aagagtattt 120

ttgccttggt cgtttataaa taaaaccata ggcctttttg gaaattggag ttttaacaaa 180

gaagatgact tcacacttcc agatggttct agagcagcaa tagtgaataa cataaatgac 240

atggaaaggg tttacaatga ttttggttcc aaatggatgt tggaggatgt gttagatcct 300

caaaagggta 310

<210> 573
<211> 1327
<212> DNA
<213> Diabrotica virgifera

<220>
<221> misc_feature
<222> (1)..(1327)
<223> RNAi target sequence DV-RYANR-FIS

<400> 573
   ggggataata agttcgattt tttacgaaaa tgacaagtat cgagactgtg gggaccattg 60
   tcctgaaatt gctgaagttg gtgatcaatt tgatatgtct catcttgtac cgaaccggat 120
   atcaaggcta cttcttggga gtaggaggaa cctggaatct aaacgaagaa aaaaatcccg 180
   atgcagaaat tgtggcttcc ggcgtattcg taggatttat gatttacaca ttcgtctcgc 240
   tgatcagcct ttgcttcgct agtggagatc acaaaacgac attcactgat attctgatga 300
   atatagtagg gatttttatg tggatagctg ctggagctac agctcttcat tattggcttg 360
   ggtacttgtc cgaatacaaa tacacgacaa tagattctga acgacaagtt ggtttggcgt 420
   taggagcgat gtgtataata aatggagcgg tctatcttgt agacggagta ctttccgcaa 480
   tctttatcct caaagccaaa atgcaataac tttcatcgta atataaatat atttatttag 540
   gttatatact ttactttaag cagctcaagt ataccgtgac atcccactca tacatcaatg 600
   tctataattg tttcatgaca aatcatttaa tagtatttta aagcattcat tcgttcaaca 660
   ccttttttac atttttcaag tattatatca atctttttaa agaattgttt ggctattaac 720
   cggattcaat ccatatagtt ccgtaaaata attatgcgtt ttaataatta atgtaattaa 780
   aaatagttag tatatttttg aaataaaaca actgccgcaa caaattaggg agaatacttt 840
   tgtctcctgt agttttatct gttatccaaa gttcgattaa attgtttcaa tttaatgtat 900
   ttttccatat aacgcaccaa atagtcttaa aaaaatagtg cctgttacta cctaatactt 960
   tctacgtatg ccttatggtt agtagtggct ccaataggtg cattttacat ttttcatgaa 1020
   aattcaattc acattatttt cctcccgttc tcttcatatt atatttattt attctttaat 1080
   atagttttgg gcgtgaagcc cagtaaatac ctatatgaag tatattgatg gaactgccat 1140
   gcttgatggg ttttgtctgc aattaaaata aaaccaaaac aaacatttgt acagtattgt 1200
   aaaattcaca atatttaggt agatgtttaa aatttaatat tttttaagag tttgcattta 1260
   tgttaatcgt agttaagttt ttataaaaat ttagtagttg tgagaaataa aataagtaaa 1320
   agagaaa                                                              1327

<210>  574
<211>  193
<212>  DNA
<213>  Diabrotica virgifera

<220>
<221>  misc_feature
<222>  (1)..(193)
<223>  RNAi target sequence DV-RYANR-FRAG1

<400>  574
tttacgaaaa tgacaagtat cgagactgtg gggaccattg tcctgaaatt gctgaagttg      60

gtgatcaatt tgatatgtct catcttgtac cgaaccggat atcaaggcta cttcttggga     120

gtaggaggaa cctggaatct aaacgaagaa aaaaatcccg atgcagaaat tgtggcttcc     180

ggcgtattcg tag                                                        193

<210>  575
<211>  145
<212>  DNA
<213>  Diabrotica virgifera

<220>
<221>  misc_feature
<222>  (1)..(145)
<223>  RNAi target sequence DV-RYANR-FRAG2

<400>  575
acgaaaatga caagtatcga gactgtgggg accattgtcc tgaaattgct gaagttggtg      60

atcaatttga tatgtctcat cttgtaccga accggatatc aaggctactt cttgggagta     120

ggaggaacct ggaatctaaa cgaag                                           145

<210>  576
<211>  83
<212>  DNA
<213>  Diabrotica virgifera

<220>
<221>  misc_feature
<222>  (1)..(83)
<223>  RNAi target sequence DV-RYANR-FRAG3

<400>  576
accattgtcc tgaaattgct gaagttggtg atcaatttga tatgtctcat cttgtaccga      60

accggatatc aaggctactt ctt                                              83

<210>  577
<211>  292

<212>  DNA
<213>  Diabrotica virgifera

<220>
<221>  misc_feature
<222>  (1)..(292)
<223>  RNAi target sequence DV-RYANR-FRAG4

<400>  577
atttatgatt tacacattcg tctcgctgat cagcctttgc ttcgctagtg gagatcacaa      60

aacgacattc actgatattc tgatgaatat agtagggatt tttatgtgga tagctgctgg     120

agctacagct cttcattatt ggcttgggta cttgtccgaa tacaaataca cgacaataga     180

ttctgaacga caagttggtt tggcgttagg agcgatgtgt ataataaatg gagcggtcta     240

tcttgtagac ggagtacttt ccgcaatctt tatcctcaaa gccaaaatgc aa             292

<210>  578
<211>  502
<212>  DNA
<213>  Diabrotica virgifera

<220>
<221>  misc_feature
<222>  (1)..(502)
<223>  RNAi target sequence DV-RYANR-FRAG5

<400>  578
ataataagtt cgatttttta cgaaaatgac aagtatcgag actgtgggga ccattgtcct      60

gaaattgctg aagttggtga tcaatttgat atgtctcatc ttgtaccgaa ccggatatca     120

aggctacttc ttgggagtag gaggaacctg gaatctaaac gaagaaaaaa atcccgatgc     180

agaaattgtg gcttccggcg tattcgtagg atttatgatt tacacattcg tctcgctgat     240

cagcctttgc ttcgctagtg gagatcacaa aacgacattc actgatattc tgatgaatat     300

agtagggatt tttatgtgga tagctgctgg agctacagct cttcattatt ggcttgggta     360

cttgtccgaa tacaaataca cgacaataga ttctgaacga caagttggtt tggcgttagg     420

agcgatgtgt ataataaatg gagcggtcta tcttgtagac ggagtacttt ccgcaatctt     480

tatcctcaaa gccaaaatgc aa                                              502

<210>  579
<211>  199
<212>  DNA
<213>  Diabrotica virgifera

<220>
<221>  misc_feature
<222>  (1)..(199)
<223>  RNAi target sequence DV-RYANR-FRAG6

<400>  579
gcagctcaag tataccgtga catcccactc atacatcaat gtctataatt gtttcatgac      60

aaatcattta atagtatttt aaagcattca ttcgttcaac acctttttta catttttcaa      120

gtattatatc aatcttttta aagaattgtt tggctattaa ccggattcaa tccatatagt      180

tccgtaaaat aattatgcg                                                  199


<210>  580
<211>  197
<212>  DNA
<213>  Diabrotica virgifera


<220>
<221>  misc_feature
<222>  (1)..(197)
<223>  RNAi target sequence DV-RYANR-FRAG7

<400>  580
ctgccgcaac aaattaggga gaatactttt gtctcctgta gttttatctg ttatccaaag      60

ttcgattaaa ttgtttcaat ttaatgtatt tttccatata acgcaccaaa tagtcttaaa      120

aaaatagtgc ctgttactac ctaatacttt ctacgtatgc cttatggtta gtagtggctc      180

caataggtgc attttac                                                    197


<210>  581
<211>  142
<212>  DNA
<213>  Diabrotica virgifera


<220>
<221>  misc_feature
<222>  (1)..(142)
<223>  RNAi target sequence DV-RYANR-FRAG8

<400>  581
gatttatgat ttacacattc gtctcgctga tcagcctttg cttcgctagt ggagatcaca      60

aaacgacatt cactgatatt ctgatgaata tagtagggat ttttatgtgg atagctgctg      120

gagctacagc tcttcattat tg                                              142


<210>  582
<211>  129
<212>  DNA
<213>  Diabrotica virgifera


<220>
<221>  misc_feature
<222>  (1)..(129)
<223>  RNAi target sequence DV-RYANR-FRAG9

<400>  582
cacattcgtc tcgctgatca gcctttgctt cgctagtgga gatcacaaaa cgacattcac      60

tgatattctg atgaatatag tagggatttt tatgtggata gctgctggag ctacagctct 120

tcattattg 129

<210> 583
<211> 152
<212> DNA
<213> Diabrotica virgifera

<220>
<221> misc_feature
<222> (1)..(152)
<223> RNAi target sequence DV-RYANR-FRAG10

<400> 583
ggcttgggta cttgtccgaa tacaaataca cgacaataga ttctgaacga caagttggtt 60

tggcgttagg agcgatgtgt ataataaatg gagcggtcta tcttgtagac ggagtacttt 120

ccgcaatctt tatcctcaaa gccaaaatgc aa 152

<210> 584
<211> 587
<212> DNA
<213> Diabrotica virgifera

<220>
<221> misc_feature
<222> (1)..(587)
<223> RNAi target sequence DV-RYANR-RASRHO-FIS

<400> 584
aactttgtgt gttttggcag gtccgatggc tgcaataaga aaaaaattgg tgattgttgg 60

tgacggagca tgtggaaaaa catgtctttt aatagtattt agtaaagatc agttcccaga 120

agtatacgtg cccactgtat tcgaaaacta tgtggcggat attgaagtag atggaaagca 180

ggtagaactc gccttatggg ataccgcagg tcaagaagat tatgatcgtc tgcggcccct 240

ttcatatcca gacaccgatg tgattttaat gtgcttctca gtagactccc ccgactcatt 300

agaaaacatc ccagaaaaat ggaccccaga ggtaaaacac ttttgtccaa atgtgccaat 360

tattctggta ggcaataaga aggatctgcg taacgaccct actaccatta acgaacttaa 420

aaagatgaaa caagaaccag taaaacctca agacggaaga tccatggcag agaaaatcaa 480

tgcttttgca tatctagaat gttctgccaa aagtaaggag ggtgtaagag aggtttttga 540

aaatgctacc cgagctgcac tgcaagtcaa aaagaagaag aagcccc 587

<210> 585
<211> 163
<212> DNA
<213> Diabrotica virgifera

<220>
<221>  misc_feature
<222>  (1)..(163)
<223>  RNAi target sequence DV-RYANR-RASRHO-FRAG1

<400>  585
aagacggaag atccatggca gagaaaatca atgcttttgc atatctagaa tgttctgcca      60

aaagtaagga gggtgtaaga gaggtttttg aaaatgctac ccgagctgca ctgcaagtca     120

aaaagaagaa gaagccccgt tgtgtaatgt tttgagccac tac                       163


<210>  586
<211>  178
<212>  DNA
<213>  Diabrotica virgifera


<220>
<221>  misc_feature
<222>  (1)..(178)
<223>  RNAi target sequence DV-DEAD-FRAG1

<400>  586
gaggattcta gtagccacaa acttatttgg acgtggaatg gatatcgaaa gagtgaacat      60

cgtcttcaac tatgacatgc ccgaagattc tgacacttac ctgcacagag tagcccgagc     120

tggacgtttc ggtaccaaag gtctagccat tactttcgta tcagaagagg gagatgcc       178


<210>  587
<211>  152
<212>  DNA
<213>  Diabrotica virgifera


<220>
<221>  misc_feature
<222>  (1)..(152)
<223>  RNAi target sequence DV-HPP15-FRAG1

<400>  587
ggacaactga ccttcatcac tcaagtgact ggcagtgtaa aataccaaga gaaaacacca      60

aaaacctttc agcgaaactt cataattaca gcacagggcg ataaatggaa aattgtcagt     120

gattgtatga ggctgcaaga accattaaac gc                                   152


<210>  588
<211>  402
<212>  DNA
<213>  Diabrotica virgifera


<220>
<221>  misc_feature
<222>  (1)..(402)
<223>  RNAi target sequence DV-HPP15-BPSAR2-FRAG1

<400>  588
ttccattggt aacatgaggt ttacgacgtt cgatttgggg ggccacgagc aggctaggag      60

agtgtggagg gactactttc cagcagtcga tgccatagtg ttccttgttg atgccaacga     120

cagctcaaga tttgtagaaa gccaggaaca gctaaatgcc ctcctctcag acgaaactct     180

atcaaactgt ccaatactta tcttaggtaa taaaattgat ctcccaggtg ctgcttcgga     240

agatgaatta cgaactagat tcggcttgtt tggccaaacc acaggcaaag gcaaagtagc     300

cagaaatgat ctacccggta ggcctctaga actatttatg tgctctatac tcaaaagaca     360

aggttatgga gaaggtttcc gttggttggc acaatatatc ga                        402


<210>  589
<211>  171
<212>  DNA
<213>  Diabrotica virgifera


<220>
<221>  misc_feature
<222>  (1)..(171)
<223>  RNAi target sequence DV-HPP15-BPSAR2-FRAG2

<400>  589
ccattggtaa catgaggttt acgacgttcg atttgggggg ccacgagcag gctaggagag      60

tgtggaggga ctactttcca gcagtcgatg ccatagtgtt ccttgttgat gccaacgaca     120

gctcaagatt tgtagaaagc caggaacagc taaatgccct cctctcagac g              171


<210>  590
<211>  137
<212>  DNA
<213>  Diabrotica virgifera


<220>
<221>  misc_feature
<222>  (1)..(137)
<223>  RNAi target sequence DV-HPP15-BPSAR2-FRAG3

<400>  590
ttgatctccc aggtgctgct tcggaagatg aattacgaac tagattcggc ttgtttggcc      60

aaaccacagg caaaggcaaa gtagccagaa atgatctacc cggtaggcct ctagaactat     120

ttatgtgctc tatactc                                                    137


<210>  591
<211>  135
<212>  DNA
<213>  Diabrotica virgifera


<220>
<221>  misc_feature
<222>  (1)..(135)

<223>  RNAi target sequence DV-HPP15-BPSAR2-FRAG4

<400>  591
ggccacgagc aggctaggag agtgtggagg gactactttc cagcagtcga tgccatagtg      60

ttccttgttg atgccaacga cagctcaaga tttgtagaaa gccaggaaca gctaaatgcc     120

ctcctctcag acgaa                                                      135


<210>  592
<211>  294
<212>  DNA
<213>  Diabrotica virgifera


<220>
<221>  misc_feature
<222>  (1)..(294)
<223>  RNAi target sequence DV-HPP15-BPSAR2-FRAG5

<400>  592
ctttataatt tgtcgttgaa aaagataggg ctccaaagtc ctattgccaa caaaaaaaca      60

acgcaagaca ttaaacaact tttttcaagc ccatctgtat ttaaaaaaac agtgcaaaat     120

gtttctgtgg gactggttta cgggaatgct gggatatcta ggattgtgga agaaacctgg     180

aaaactatta ttcttaggac tggataacgc aggcaaaact acccttctac atatgctcaa     240

ggatgacaga ctggcccagc atcttcccac gttacatccc acatcagagg agct           294


<210>  593
<211>  407
<212>  DNA
<213>  Diabrotica virgifera


<220>
<221>  misc_feature
<222>  (1)..(407)
<223>  RNAi target sequence DV-HPP15-BPSAR2-FRAG6

<400>  593
ttaattatgt atttttccat ttcgttctgt cattgagtta ggatattaat gtttgaggaa      60

ctattggcaa cactgcaact acctgattca tttcagatct taggtacttt acataaatat     120

ctaaatatag atgttggcaa tgtaattttg aacaacagta tatacattca atgtaaatta     180

tatattttta gttaagttac cttcttaaat ggtgtttgag tggctatggt actgaaatag     240

tttgactttt tgtgttcttc gataactaaa aatgatttct tgtggaaagt tacactcaga     300

attacatagt taacttcttt atcagcagtt gttgtcaaga tttccatttt gagacgattg     360

ttttgtaaat taggtgggaa tttttaaaat gttggactgt tttttaa                   407


<210>  594
<211>  168
<212>  DNA

<213>  Diabrotica virgifera

<220>
<221>  misc_feature
<222>  (1)..(168)
<223>  RNAi target sequence DV-HP1-FRAG1

<400>  594
ggggaaggta aatcagtatt cacttgatct tcaaggtaga ttaacgcaag tagaaatcta      60

aaacatgtct ggacgtggta agggaggcaa agttaaggga aaagcaaagt cccgatcaaa     120

tcgtgctggt ttacaatttc ctgtaggtcg tattcatcgt ttattgag                  168

<210>  595
<211>  190
<212>  DNA
<213>  Diabrotica virgifera

<220>
<221>  misc_feature
<222>  (1)..(190)
<223>  RNAi target sequence DV-HP1-FRAG2

<400>  595
ggggaaggta aatcagtatt cacttgatct tcaaggtaga ttaacgcaag tagaaatcta      60

aaacatgtct ggacgtggta agggaggcaa agttaaggga aaagcaaagt cccgatcaaa     120

tcgtgctggt ttacaatttc ctgtaggtcg tattcatcgt ttattgagaa aaggaaatta     180

tgccgaaaga                                                            190

<210>  596
<211>  159
<212>  DNA
<213>  Diabrotica virgifera

<220>
<221>  misc_feature
<222>  (1)..(159)
<223>  RNAi target sequence DV-HP1-FRAG3

<400>  596
ttggaattgg caggaaatgc agctagagat aacaaaaaga cccgtataat tcctagacat      60

ttacaattgg ccataagaaa tgacgaggaa ttgaacaaat tactgtcagg agttaccatc     120

gcccaaggtg gagtattgcc taatatacaa gcagtactt                            159

<210>  597
<211>  196
<212>  DNA
<213>  Diabrotica virgifera

<220>

<221>  misc_feature
<222>  (1)..(196)
<223>  RNAi target sequence DV-RNAPOL-FRAG1

<400>  597
ctgttttaaa gccatggctg atgaagaatt tgacgaaaat gatgtagcag atgatttcga      60

tgacgacgta gaggatgata atatcgaaga actcgaacaa cccgaggaag atggagataa     120

catcgatatc cttgctccag gacaagcagg aggtggtgta ccaaaaaaca agaggataac     180

aactaaatat atgacc                                                     196


<210>  598
<211>  163
<212>  DNA
<213>  Diabrotica virgifera


<220>
<221>  misc_feature
<222>  (1)..(163)
<223>  RNAi target sequence DV-CPNN-FRAG1

<400>  598
ctgttggtgc tacttgtctt ccacgcctaa cagctcccac atctcaagaa cttggttacg      60

cagatattgt atgtgtagaa gaattgggtg atacccctat agtttctttc agattggagg     120

gcaaagaatc aagaatttcc acaattgtca tcagaggtgc tac                       163


<210>  599
<211>  236
<212>  DNA
<213>  Diabrotica virgifera


<220>
<221>  misc_feature
<222>  (1)..(236)
<223>  RNAi target sequence DV-CPNN-FRAG2

<400>  599
cagccaaact catgatacta ggaagtcaaa tgcaagattc tgaggtgggt gatggaacta      60

actttgtgat tatacttgct ggagcacttt tggagcatgc tgaagaacta attcgtcttg     120

gagtaactcc cacagaaata gcagaaggtt atgagaaagc actagacaaa tgtctagaaa     180

tccttcctaa tcttatttgc tacacaatca aggatttcag aaacatcgaa gaagtc         236


<210>  600
<211>  149
<212>  DNA
<213>  Diabrotica virgifera


<220>
<221>  misc_feature
<222>  (1)..(149)

<223>  RNAi target sequence DV-CPNN-FRAG3

<400>  600
ttggagcatg ctgaagaact aattcgtctt ggagtaactc ccacagaaat agcagaaggt      60

tatgagaaag cactagacaa atgtctagaa atccttccta atcttatttg ctacacaatc     120

aaggatttca gaaacatcga agaagtcaa                                       149


<210>  601
<211>  194
<212>  DNA
<213>  Diabrotica virgifera


<220>
<221>  misc_feature
<222>  (1)..(194)
<223>  RNAi target sequence DV-CPNN-FRAG4

<400>  601
ctgcagatga acttatgaac tttagtaggg gagaagaaaa tcttcttgaa ttacaaatca      60

aatgtatagc agatgctgga gctaaagttg tagttgcagg agccaaattt ggagatatgg     120

ctctacatta cttacataaa tatggcatga tggttgtcag gttgaactcc aaatttgatt     180

tgagaagatt atgc                                                       194


<210>  602
<211>  785
<212>  DNA
<213>  Diabrotica virgifera


<220>
<221>  misc_feature
<222>  (1)..(785)
<223>  RNAi target sequence DV-TEF2-FRAG1

<400>  602
tctggtaaca tctgtggttt ggttggtgtt gatcaattct tggttaagac tggtaccatt      60

accactttca aggatgccca caacttgaag gtcatgaaat tcagtgtatc tcctgtagta     120

cgtgttgctg ttgaacctaa gaaccctgct gatttgccaa aacttgtaga aggtttgaaa     180

cgtttgacca aatccgatcc tatggtccaa tgtatcattg aagaatctgg tgagcacatc     240

attgctggtg ccggagaatt gcatcttgaa atttgcctta aggatcttga agaagatcat     300

gcctgcattc ccattaagaa atctgaccct gttgtatcct accgtgagac agtaagtgaa     360

gagtctgatc agatgtgcct ctcaaaatca cccaacaaac acaacagatt gttcatgaaa     420

gcttgtccta tgcctgatgg acttgctgaa gatattgatg atggtaaagt taacccacgt     480

gatgacttca aagtccgtgc ccgttatctt actgagaaat atgactatga tataactgaa     540

gcccgtaaga tctggtgctt tggacctgat ggtactggac ccaacatcct tgttgattgt     600

actaagggag tgcaatatct taatgaaatc aaagactctg tggtagctgg tttccaatgg 660

gcaaccaaag aaggtgtctt gtctgaagaa aacttgagag gtgttagatt caacatttat 720

gatgttacct tacacgctga tgctatccat cgtggaggtg gtcaaatcat tccaacaacc 780

cgtag 785

<210> 603
<211> 144
<212> DNA
<213> Diabrotica virgifera

<220>
<221> misc_feature
<222> (1)..(144)
<223> RNAi target sequence DV-SRP54-FRAG1

<400> 603
tgggaggcat caaaggtctc ttcaaaggtg gcgatatggc taaaaacgtc aaccacaacc 60

agatggcgaa acttaatcaa caaatggcca aaatgatgga ccctagagtc ctccaacaaa 120

tgggcggcat ggcaggtttg caaa 144

<210> 604
<211> 364
<212> DNA
<213> Diabrotica virgifera

<220>
<221> misc_feature
<222> (1)..(364)
<223> RNAi target sequence DV-SRP54-FRAG2

<400> 604
   acgagttgga caacagggat ggcgcaaaat tgtttactaa acaagttggt agagtggtca 60
   gggtagcaca aggatctggt gtaacagaaa gagaagtaaa agatctcatt acgcagtaca 120
   caaaattcgc agcggtggta aagaaaatgg gaggcatcaa aggtctcttc aaaggtggcg 180
   atatggctaa aaacgtcaac cacaaccaga tggcgaaact taatcaacaa atggccaaaa 240
   tgatggaccc tagagtcctc caacaaatgg gcggcatggc aggtttgcaa aatatgatga 300
   gacaactcca agcaggcgca gctggtggtt taggggcctt aggtaatctt atggggggct 360
   ttgg 364

<210> 605
<211> 226
<212> DNA
<213> Diabrotica virgifera

<220>
<221> misc_feature

<222>  (1)..(226)
<223>  RNAi target sequence DV-SRP54-FRAG3

<400>  605
gtagcacaag gatctggtgt aacagaaaga gaagtaaaag atctcattac gcagtacaca      60

aaattcgcag cggtggtaaa gaaaatggga ggcatcaaag gtctcttcaa aggtggcgat     120

atggctaaaa acgtcaacca caaccagatg gcgaaactta atcaacaaat ggccaaaatg     180

atggacccta gagtcctcca acaaatgggc ggcatggcag gtttgc                    226


<210>  606
<211>  163
<212>  DNA
<213>  Diabrotica virgifera


<220>
<221>  misc_feature
<222>  (1)..(163)
<223>  RNAi target sequence DV-SRP54-FRAG4

<400>  606
ttatcagaaa aagaactgga aatcatgttt ggtatgtgca gatacattca gagctggtgc      60

ctacgatcag gtaaaacaga actgtacaaa agctaggata cccttttatg gaagttacac     120

agaagttgat cctgtagtca tagcccagga tggtgttgat atg                       163


<210>  607
<211>  171
<212>  DNA
<213>  Diabrotica virgifera


<220>
<221>  misc_feature
<222>  (1)..(171)
<223>  RNAi target sequence DV-TIF3-FRAG1

<400>  607
gttgagtgct gtttctggag aagatgccca agaccgttca gatcgtacaa ttcttactcc      60

gtgggttaag ttcctttggg aatcttactg tcaatgtttg gaacttttgc gtacaaatgc     120

acatgttgag aacttgtatc atgatatagc caggatggct ttccagtttt g              171


<210>  608
<211>  232
<212>  DNA
<213>  Diabrotica virgifera


<220>
<221>  misc_feature
<222>  (1)..(232)
<223>  RNAi target sequence DV-TIF3-FRAG2

<400>  608

atcgtgggga tcgcagggac gatagggaga ggagagacga cagggataac agggatagga 60

gagacgacag agatcgtaga gatgataggg ataggagaga cgacagggag cgtagagatg 120

atagagagag aagagatgac cgtggcggaa gagatgataa ccgggaccgc cgagacgatc 180

gtgatcgtag agacgacagg gaccgaagag gcggggacga aggtacttgg cg 232

<210> 609
<211> 166
<212> DNA
<213> Diabrotica virgifera

<220>
<221> misc_feature
<222> (1)..(166)
<223> RNAi target sequence DV-TIF3-FRAG3

<400> 609
ggcaaccgcg acagcggaag atttggcgac agagacagag aaggtggtgt cagattcacg 60

agagataatc gggataatcg tggggatcgc agggacgata gggagaggag agacgacagg 120

gataacaggg ataggagaga cgacagagat cgtagagatg ataggg 166

<210> 610
<211> 239
<212> DNA
<213> Diabrotica virgifera

<220>
<221> misc_feature
<222> (1)..(239)
<223> RNAi target sequence DV-TIF3-FRAG4

<400> 610
gcaagtccac aactacaaga tttgtacaat tggctggaag tggagttcca tcctttacta 60

ttgtgtcaga gagtgcatga ggtaatcaaa tctctcgaat ctgaagaaaa tgcagccctc 120

caacaatatg tgccagcttt acaagatgta actctggtgc gtctagtgag acagatagca 180

caagtctatc agaccataga atttgctagg ttgatccaga ttgccaaatt tactacacc 239

<210> 611
<211> 227
<212> DNA
<213> Diabrotica virgifera

<220>
<221> misc_feature
<222> (1)..(227)
<223> RNAi target sequence DV-TIF3-FRAG5

<400> 611
ggttgattgt gtgagacaca atgatatgca aattcgtatt gatcatggca aagactgtat 60

ccactttgga atggacctga gtgaaagcca aagagaggac aaaccagagg gacccacttt 120

gcaagttatg cctagtgagc aagtcagaaa tcaacttatt aacatgtcta ctgtattgaa 180

tcaagccata caagtcatta atcctaataa gaagaagctt gaacgcg 227

<210> 612
<211> 224
<212> DNA
<213> Diabrotica virgifera

<220>
<221> misc_feature
<222> (1)..(224)
<223> RNAi target sequence DV-TIF3-FRAG6

<400> 612
tgttataaga gggtatttgc ggatggcaga agaaagaaca gagaatgcta gagaacagtc 60

agcacaagca gttattgata tcgatgattt agataatttg gctacacctg aaagtatatt 120

gttgagtgct gtttctggag aagatgccca agaccgttca gatcgtacaa ttcttactcc 180

gtgggttaag ttcctttggg aatcttactg tcaatgtttg gaac 224

<210> 613
<211> 154
<212> DNA
<213> Diabrotica virgifera

<220>
<221> misc_feature
<222> (1)..(154)
<223> RNAi target sequence DV-TIF3-FRAG7

<400> 613
aagactgtat ccactttgga atggacctga gtgaaagcca aagagaggac aaaccagagg 60

gacccacttt gcaagttatg cctagtgagc aagtcagaaa tcaacttatt aacatgtcta 120

ctgtattgaa tcaagccata caagtcatta atcc 154

<210> 614
<211> 742
<212> DNA
<213> Diabrotica virgifera

<220>
<221> misc_feature
<222> (1)..(742)
<223> RNAi target sequence DV-TIF3-FRAG8

<400> 614
ctggcaagtc cacaactaca agatttgtac aattggctgg aagtggagtt ccatccttta 60

ctattgtgtc agagagtgca tgaggtaatc aaatctctcg aatctgaaga aaatgcagcc 120

ctccaacaat atgtgccagc tttacaagat gtaactctgg tgcgtctagt gagacagata 180

gcacaagtct atcagaccat agaatttgct aggttgatcc agattgccaa atttactaca 240

ccttttcatc tggagagact tttggttgat tgtgtgagac acaatgatat gcaaattcgt 300

attgatcatg gcaaagactg tatccacttt ggaatggacc tgagtgaaag ccaaagagag 360

gacaaaccag agggacccac tttgcaagtt atgcctagtg agcaagtcag aaatcaactt 420

attaacatgt ctactgtatt gaatcaagcc atacaagtca ttaatcctaa taagaagaag 480

cttgaacgcg aaaaacttag agctgttatg gttcagaatt atcacgaaac aaaggttaga 540

gaacaccaga aaattttaca aagacacaag attattgagg acagaaagga atatattgag 600

cgtttgaata cagtcaggga agaagaggag cagaaaagat tggaagaaat gcaacggcag 660

cacttactgg ccgaacaaaa acgattggac caagaaagag aagaaagaga gcggaaacgt 720

gctctaaatg aaatccagca gg 742

<210> 615
<211> 619
<212> DNA
<213> Diabrotica virgifera

<220>
<221> misc_feature
<222> (1)..(619)
<223> RNAi target sequence DV-TIF3-FRAG9

<400> 615
ccaacaatat gtgccagctt tacaagatgt aactctggtg cgtctagtga gacagatagc 60

acaagtctat cagaccatag aatttgctag gttgatccag attgccaaat ttactacacc 120

ttttcatctg gagagacttt tggttgattg tgtgagacac aatgatatgc aaattcgtat 180

tgatcatggc aaagactgta tccactttgg aatggacctg agtgaaagcc aaagagagga 240

caaaccagag ggacccactt tgcaagttat gcctagtgag caagtcagaa atcaacttat 300

taacatgtct actgtattga atcaagccat acaagtcatt aatcctaata agaagaagct 360

tgaacgcgaa aaacttagag ctgttatggt tcagaattat cacgaaacaa aggttagaga 420

acaccagaaa attttacaaa gacacaagat tattgaggac agaaaggaat atattgagcg 480

tttgaataca gtcagggaag aagaggagca gaaaagattg gaagaaatgc aacggcagca 540

cttactggcc gaacaaaaac gattggacca agaaagagaa gaaagagagc ggaaacgtgc 600

tctaaatgaa atccagcag 619

<210> 616
<211> 713
<212> DNA
<213> Diabrotica virgifera

<220>
<221> misc_feature
<222> (1)..(713)
<223> RNAi target sequence DV-EIF-4A-FRAG2

<400> 616
cgtctagcac atcgtttggc atggtagccg aaagtaaaat tacctgtacg tcagtgttca 60

acattttgaa cacgtcatga atttgatctt taaaacccct tgacaacatt tcatcggctt 120

catccaaaac aaacattttt atatgagtcg gccttaaggc tctccttgtt atcatatcat 180

aaactctacc aggtgtacca actacaacat gtacgccagt ttcaagtttt cttatgtctt 240

ctcgtacatt ggtaccacca atacatgcat gacactgcgc tgacatgaaa tctcctaggg 300

caattactac tttttggatc tgttgagcca attcacgagt tggtgccaat ataagagctt 360

ggcactccct cactgacgtg tcaatttgtt gtaaaataga gatggagaat gttgcagtct 420

taccagtacc tgactgagcc tgagcaataa catcgtgtcc ctttacacaa ggaatgatgg 480

ctctctgttg gatagcagaa ggtttttcaa aaccataggc ataaattcct cttaaaagtt 540

cctcttttag gttcatgtcg tcgaaattat cgaccacctc ttcccagttg gattcaataa 600

tgccatcagg ctccatacct ggtgggccgt catagccttg gggctggctg tcttcattag 660

ctgggccatt cttgtcccac tcttcacgac gatctggcat agacatgtct act 713

<210> 617
<211> 200
<212> DNA
<213> Diabrotica virgifera

<220>
<221> misc_feature
<222> (1)..(200)
<223> RNAi target sequence DV-EIF-4A-FRAG3

<400> 617
tctatgccag atcgtcgtga agagtgggac aagaatggcc cagctaatga agacagccag 60

ccccaaggct atgacggccc accaggtatg gagcctgatg gcattattga atccaactgg 120

gaagaggtgg tcgataattt cgacgacatg aacctaaaag aggaactttt aagaggaatt 180

tatgcctatg gttttgaaaa 200

<210> 618
<211> 270
<212> DNA
<213> Diabrotica virgifera

<220>
<221> misc_feature
<222> (1)..(270)
<223> RNAi target sequence DV-PAT3-SS1

<400>  618
tcaacattga taaacacata ggaatggcat tttcaggctt aatagctgat gcaaggcaaa      60

tcgttgagat tgctagaaaa gaagcatcaa attatagaca tcaatatggt tcaaatattc     120

ctcttaaata cctaaatgat agagtaagca tgtacatgca tgcatacact ttatacagtg     180

ctgttagacc atttggttgc agtgtcatct tggccagtta tgaagatagt gacccatcta     240

tgtatctgat tgatccatct ggagttagct                                      270


<210>  619
<211>  322
<212>  DNA
<213>  Diabrotica virgifera


<220>
<221>  misc_feature
<222>  (1)..(322)
<223>  RNAi target sequence DV-PAT3-FRAG5

<400>  619
ggggagtcgt caacatcaat ttcaagtttc aagaaaaagc aaatcactac gacttgccgg      60

attttgtagt agtgttaatt ttgtattaaa aaatcaaaat gagttctatt ggaactgggt     120

acgatttatc agcttcccaa ttctctcctg atggaagagt atttcaagtt gaatatgcaa     180

tgaaagcagt tgaaaatagt ggcaccgtaa taggcctccg aggtacagat ggcattgtat     240

tggctgctga aaagctcatt atgtcaaaat tgcatgaacc aagtacaaat aaacgaattt     300

tcaacattga taaacacata gg                                              322


<210>  620
<211>  322
<212>  DNA
<213>  Diabrotica virgifera


<220>
<221>  misc_feature
<222>  (1)..(322)
<223>  RNAi target sequence DV-PAT3-FRAG6

<400>  620
aatggcattt tcaggcttaa tagctgatgc aaggcaaatc gttgagattg ctagaaaaga      60

agcatcaaat tatagacatc aatatggttc aaatattcct cttaaatacc taaatgatag     120

agtaagcatg tacatgcatg catacacttt atacagtgct gttagaccat ttggttgcag     180

tgtcatcttg gccagttatg aagatagtga cccatctatg tatctgattg atccatctgg     240

agttagctat ggatactttg gatgtgctac aggtaaagca aaacagtctg caaagactga     300

aatagaaaaa ttgaagatgg gg                                              322

<210>  621
<211>  161
<212>  DNA
<213>  Diabrotica virgifera

<220>
<221>  misc_feature
<222>  (1)..(161)
<223>  RNAi target sequence DV-PAT3-FRAG7

<400>  621
ggggagtcgt caacatcaat ttcaagtttc aagaaaaagc aaatcactac gacttgccgg      60

attttgtagt agtgttaatt ttgtattaaa aaatcaaaat gagttctatt ggaactgggt      120

acgatttatc agcttcccaa ttctctcctg atggaagagt a                          161

<210>  622
<211>  161
<212>  DNA
<213>  Diabrotica virgifera

<220>
<221>  misc_feature
<222>  (1)..(161)
<223>  RNAi target sequence DV-PAT3-FRAG8

<400>  622
tttcaagttg aatatgcaat gaaagcagtt gaaaatagtg gcaccgtaat aggcctccga      60

ggtacagatg gcattgtatt ggctgctgaa aagctcatta tgtcaaaatt gcatgaacca      120

agtacaaata aacgaatttt caacattgat aaacacatag g                          161

<210>  623
<211>  161
<212>  DNA
<213>  Diabrotica virgifera

<220>
<221>  misc_feature
<222>  (1)..(161)
<223>  RNAi target sequence DV-PAT3-FRAG9

<400>  623
aatggcattt tcaggcttaa tagctgatgc aaggcaaatc gttgagattg ctagaaaaga      60

agcatcaaat tatagacatc aatatggttc aaatattcct cttaaatacc taaatgatag      120

agtaagcatg tacatgcatg catacacttt atacagtgct g                          161

<210>  624
<211>  161
<212>  DNA
<213>  Diabrotica virgifera

<220>
<221>  misc_feature
<222>  (1)..(161)
<223>  RNAi target sequence DV-PAT3-FRAG10

<400>  624
ttagaccatt tggttgcagt gtcatcttgg ccagttatga agatagtgac ccatctatgt      60

atctgattga tccatctgga gttagctatg gatactttgg atgtgctaca ggtaaagcaa     120

aacagtctgc aaagactgaa atagaaaaat tgaagatggg g                         161


<210>  625
<211>  162
<212>  DNA
<213>  Diabrotica virgifera


<220>
<221>  misc_feature
<222>  (1)..(162)
<223>  RNAi target sequence DV-PAT3-FRAG11

<400>  625
ttgtattaaa aaatcaaaat gagttctatt ggaactgggt acgatttatc agcttcccaa      60

ttctctcctg atggaagagt atttcaagtt gaatatgcaa tgaaagcagt tgaaaatagt     120

ggcaccgtaa taggcctccg aggtacagat ggcattgtat tg                        162


<210>  626
<211>  160
<212>  DNA
<213>  Diabrotica virgifera


<220>
<221>  misc_feature
<222>  (1)..(160)
<223>  RNAi target sequence DV-PAT3-FRAG12

<400>  626
gctgctgaaa agctcattat gtcaaaattg catgaaccaa gtacaaataa acgaattttc      60

aacattgata aacacatagg aatggcattt tcaggcttaa tagctgatgc aaggcaaatc     120

gttgagattg ctagaaaaga agcatcaaat tatagacatc                           160


<210>  627
<211>  161
<212>  DNA
<213>  Diabrotica virgifera


<220>
<221>  misc_feature
<222>  (1)..(161)
<223>  RNAi target sequence DV-PAT3-FRAG13

<400>  627

aatatggttc aaatattcct cttaaatacc taaatgatag agtaagcatg tacatgcatg 60

catacacttt atacagtgct gttagaccat ttggttgcag tgtcatcttg gccagttatg 120

aagatagtga cccatctatg tatctgattg atccatctgg a 161

<210> 628
<211> 80
<212> DNA
<213> Diabrotica virgifera

<220>
<221> misc_feature
<222> (1)..(80)
<223> RNAi target sequence DV-PAT3-FRAG14

<400> 628
ggggagtcgt caacatcaat ttcaagtttc aagaaaaagc aaatcactac gacttgccgg 60

attttgtagt agtgttaatt 80

<210> 629
<211> 81
<212> DNA
<213> Diabrotica virgifera

<220>
<221> misc_feature
<222> (1)..(81)
<223> RNAi target sequence DV-PAT3-FRAG15

<400> 629
ttgtattaaa aaatcaaaat gagttctatt ggaactgggt acgatttatc agcttcccaa 60

ttctctcctg atggaagagt a 81

<210> 630
<211> 80
<212> DNA
<213> Diabrotica virgifera

<220>
<221> misc_feature
<222> (1)..(80)
<223> RNAi target sequence DV-PAT3-FRAG16

<400> 630
tttcaagttg aatatgcaat gaaagcagtt gaaaatagtg gcaccgtaat aggcctccga 60

ggtacagatg gcattgtatt 80

<210> 631
<211> 81
<212> DNA
<213> Diabrotica virgifera

<220>
<221>  misc_feature
<222>  (1)..(81)
<223>  RNAi target sequence DV-PAT3-FRAG17

<400>  631
ggctgctgaa aagctcatta tgtcaaaatt gcatgaacca agtacaaata aacgaatttt      60

caacattgat aaacacatag g                                                81


<210>  632
<211>  80
<212>  DNA
<213>  Diabrotica virgifera


<220>
<221>  misc_feature
<222>  (1)..(80)
<223>  RNAi target sequence DV-PAT3-FRAG18

<400>  632
aatggcattt tcaggcttaa tagctgatgc aaggcaaatc gttgagattg ctagaaaaga      60

agcatcaaat tatagacatc                                                  80


<210>  633
<211>  81
<212>  DNA
<213>  Diabrotica virgifera


<220>
<221>  misc_feature
<222>  (1)..(81)
<223>  RNAi target sequence DV-PAT3-FRAG19

<400>  633
aatatggttc aaatattcct cttaaatacc taaatgatag agtaagcatg tacatgcatg      60

catacacttt atacagtgct g                                                81


<210>  634
<211>  80
<212>  DNA
<213>  Diabrotica virgifera


<220>
<221>  misc_feature
<222>  (1)..(80)
<223>  RNAi target sequence DV-PAT3-FRAG20

<400>  634
ttagaccatt tggttgcagt gtcatcttgg ccagttatga agatagtgac ccatctatgt      60

atctgattga tccatctgga                                                  80

<210>  635
<211>  81
<212>  DNA
<213>  Diabrotica virgifera

<220>
<221>  misc_feature
<222>  (1)..(81)
<223>  RNAi target sequence DV-PAT3-FRAG21

<400>  635
gttagctatg gatactttgg atgtgctaca ggtaaagcaa aacagtctgc aaagactgaa      60

atagaaaaat tgaagatggg g                                                81

<210>  636
<211>  644
<212>  DNA
<213>  Diabrotica virgifera

<220>
<221>  misc_feature
<222>  (1)..(644)
<223>  RNAi target sequence DV-PAT3-FIS (TR)

<400>  636
ggggagtcgt caacatcaat ttcaagtttc aagaaaaagc aaatcactac gacttgccgg      60

attttgtagt agtgttaatt ttgtattaaa aaatcaaaat gagttctatt ggaactgggt     120

acgatttatc agcttcccaa ttctctcctg atggaagagt atttcaagtt gaatatgcaa     180

tgaaagcagt tgaaaatagt ggcaccgtaa taggcctccg aggtacagat ggcattgtat     240

tggctgctga aaagctcatt atgtcaaaat tgcatgaacc aagtacaaat aaacgaattt     300

tcaacattga taaacacata ggaatggcat tttcaggctt aatagctgat gcaaggcaaa     360

tcgttgagat tgctagaaaa gaagcatcaa attatagaca tcaatatggt tcaaatattc     420

ctcttaaata cctaaatgat agagtaagca tgtacatgca tgcatacact ttatacagtg     480

ctgttagacc atttggttgc agtgtcatct tggccagtta tgaagatagt gacccatcta     540

tgtatctgat tgatccatct ggagttagct atggatactt tggatgtgct acaggtaaag     600

caaaacagtc tgcaaagact gaaatagaaa aattgaagat gggg                      644

<210>  637
<211>  367
<212>  DNA
<213>  Diabrotica virgifera

<220>
<221>  misc_feature
<222>  (1)..(367)
<223>  RNAi target sequence DV-PAT3-FRAG22

<400>  637
   aatctaacat gcaaagaact tgttaaagaa gcagccaaaa tcatttattt ggtccatgat      60
   gagctgaagg ataagaattt tgaactggaa ctttcatggg tatgcaaaga tacgaatggt     120
   ttacatacca aagtgcctga atcagtgttt gctgatgcag aaaaagctgc caaacaaatg     180
   gaagcagatt cagaatcaga tacagaagat atgtaataac tacatttagt ttttaatatt     240
   tcgctgatgg tggctgttct tacaatattt cgtgtgttat gttcatatat tatgtaatac     300
   tgtgagaatt tccatttcaa ggataggttt ataacttttt tttctaataa atacataact     360
   ttatgtc                                                               367

<210>  638
<211>  182
<212>  DNA
<213>  Diabrotica virgifera

<220>
<221>  misc_feature
<222>  (1)..(182)
<223>  RNAi target sequence DV-PAT3-FRAG23

<400>  638
aatctaacat gcaaagaact tgttaaagaa gcagccaaaa tcatttattt ggtccatgat      60

gagctgaagg ataagaattt tgaactggaa ctttcatggg tatgcaaaga tacgaatggt     120

ttacatacca aagtgcctga atcagtgttt gctgatgcag aaaaagctgc caaacaaatg     180

ga                                                                    182

<210>  639
<211>  185
<212>  DNA
<213>  Diabrotica virgifera

<220>
<221>  misc_feature
<222>  (1)..(185)
<223>  RNAi target sequence DV-PAT3-FRAG24

<400>  639
   agcagattca gaatcagata cagaagatat gtaataacta catttagttt ttaatatttc      60
   gctgatggtg gctgttctta caatatttcg tgtgttatgt tcatatatta tgtaatactg     120
   tgagaatttc catttcaagg ataggtttat aacttttttt tctaataaat acataacttt     180
   atgtc                                                                  185

<210>  640
<211>  256
<212>  DNA
<213>  Diabrotica virgifera

<220>
<221>  misc_feature
<222>  (1)..(256)
<223>  RNAi target sequence DV-PAT5-FRAG1

<400>  640
atcaagaaca tttgttcagt ttgacgcaaa ggcgatagga tcaggatccg agggtgcaca      60

gcaaagttta caggaggcat atcatcgcag tatgaccctg gaagaagcta ccacaactgc      120

tttaacaatt ctaaaacaag ttatggaaga aaagttgaat tcttcaaatg ttgaggttat      180

gactatgact ccaaaagata tgttccacat gttcacaaag gaacaagtgg aagaggtaat      240

tgctaaattg tcttaa                                                      256


<210>  641
<211>  200
<212>  DNA
<213>  Diabrotica virgifera


<220>
<221>  misc_feature
<222>  (1)..(200)
<223>  RNAi target sequence DV-PAT6-FRAG1

<400>  641
aatcatgtct agaggaagca gtgcaggttt tgaccgacac ataacaattt tctcgcccga      60

aggccgactc tatcaagtag agtatgcttt taaagccatt aaccaagccg gccccacttc      120

ggtagcagtc cgaggagtag atgctgcggc gtgtgtgacc cagagaaaga tcccggataa      180

gctgattgat cccaacacaa                                                  200


<210>  642
<211>  216
<212>  DNA
<213>  Diabrotica virgifera


<220>
<221>  misc_feature
<222>  (1)..(216)
<223>  RNAi target sequence DV-PAT6-FRAG2

<400>  642
cagaacacac tggatgtgtg atgactggca tgattgctga cagcaagtcc caggtgcaga      60

gagctagata tgaggctgcc gagttcaaat ataagtttgg atatgagatg ccaatcgatg      120

ccttgtgtag gagagtatcg gatatttccc aggtttatac gcagaatgct gagatgagac      180

ctttgggttg ctccatgctt ctgataggat atgacc                                216


<210>  643
<211>  200
<212>  DNA

<213>  Diabrotica virgifera

<220>
<221>  misc_feature
<222>  (1)..(200)
<223>  RNAi target sequence DV-PAT6-FRAG3

<400>  643
cacatctgtt ccagttaaca gaacacactg gatgtgtgat gactggcatg attgctgaca      60

gcaagtccca ggtgcagaga gctagatatg aggctgccga gttcaaatat aagtttggat      120

atgagatgcc aatcgatgcc ttgtgtagga gagtatcgga tatttcccag gtttatacgc      180

agaatgctga gatgagacct                                                  200

<210>  644
<211>  195
<212>  DNA
<213>  Diabrotica virgifera

<220>
<221>  misc_feature
<222>  (1)..(195)
<223>  RNAi target sequence DV-PAT6-FRAG4

<400>  644
aggagagtat cggatatttc ccaggtttat acgcagaatg ctgagatgag acctttgggt      60

tgctccatgc ttctgatagg atatgaccaa gaaatgggac catgtgtcca caaagctgac      120

cctgctggct attactgtgg ctacagagca gtaagtgtag gatccaaaca aactgaagcc      180

aacagctacc tagag                                                       195

<210>  645
<211>  199
<212>  DNA
<213>  Diabrotica virgifera

<220>
<221>  misc_feature
<222>  (1)..(199)
<223>  RNAi target sequence DV-PAT6-FRAG5

<400>  645
aagaagctaa agaagaaaac tgatctgtcc aaagatgaag tcatccaatt ggcaatcagt      60

tgtctttcaa gtgttttatc tgtagatttt aaaccaacag aagtcgaagt tggggttgtt      120

actaaggatg agcccaagtt taggaaattg acggaacaag aaatagattt ccatctgaca      180

gctattgctg agagggatt                                                   199

<210>  646
<211>  165
<212>  DNA

<213>  Diabrotica virgifera

<220>
<221>  misc_feature
<222>  (1)..(165)
<223>  RNAi target sequence DV-PAT6-FRAG6

<400>  646
aggagagtat cggatatttc ccaggtttat acgcagaatg ctgagatgag acctttgggt      60

tgctccatgc ttctgatagg atatgaccaa gaaatgggac catgtgtcca caaagctgac     120

cctgctggct attactgtgg ctacagagca gtaagtgtag gatcc                     165

<210>  647
<211>  157
<212>  DNA
<213>  Diabrotica virgifera

<220>
<221>  misc_feature
<222>  (1)..(157)
<223>  RNAi target sequence DV-PAT6-FRAG7

<400>  647
gagaagaagc taaagaagaa aactgatctg tccaaagatg aagtcatcca attggcaatc      60

agttgtcttt caagtgtttt atctgtagat tttaaaccaa cagaagtcga agttggggtt     120

gttactaagg atgagcccaa gtttaggaaa ttgacgg                              157

<210>  648
<211>  196
<212>  DNA
<213>  Diabrotica virgifera

<220>
<221>  misc_feature
<222>  (1)..(196)
<223>  RNAi target sequence DV-BETA2-FRAG1

<400>  648
gcactcagaa gcagaagtgc ataccatgta aaccttcttc tagctggata tgatgacaaa      60

gaaggtcctc agctatatta catggactac ctagcatcca tagcaagcgt tgattatgct     120

gctcatggtt atggaggatt cttttcactg tcaattatgg atcgtaatta tcggcagaac     180

cttagtaaag aagaag                                                     196

<210>  649
<211>  187
<212>  DNA
<213>  Diabrotica virgifera

<220>

<221>  misc_feature
<222>  (1)..(187)
<223>  RNAi target sequence DV-BETA2-FRAG2

<400>  649
tttcactgtc aattatggat cgtaattatc ggcagaacct tagtaaagaa gaaggttacg      60

aacttttgaa aaaatgtgtg aaagaagttc agaaaagatt gatcatcaat ttgcctaact     120

ttaaggtgca gtgtgttgat aaaaatggca tccaggatat gcctaatatc actgctaagg     180

atttggt                                                               187


<210>  650
<211>  155
<212>  DNA
<213>  Diabrotica virgifera


<220>
<221>  misc_feature
<222>  (1)..(155)
<223>  RNAi target sequence DV-BETA2-FRAG3

<400>  650
cagatgcact cagaagcaga agtgcatacc atgtaaacct tcttctagct ggatatgatg      60

acaaagaagg tcctcagcta tattacatgg actacctagc atccatagca agcgttgatt     120

atgctgctca tggttatgga ggattctttt cactg                                155


<210>  651
<211>  174
<212>  DNA
<213>  Diabrotica virgifera


<220>
<221>  misc_feature
<222>  (1)..(174)
<223>  RNAi target sequence DV-BETA3-FRAG1

<400>  651
tgtgaagcac tttgggagcc taacctagga cctgaggatc tttttgaaac catctctcaa      60

gctctcatca atgcctttga cagagatgcc atttctggtt ggggagctac agtgtacatc     120

atagaaaaaa ataaggtgac tgtaaaaaat ttgaagacac gtatggacta gtta           174


<210>  652
<211>  203
<212>  DNA
<213>  Diabrotica virgifera


<220>
<221>  misc_feature
<222>  (1)..(203)
<223>  RNAi target sequence DV-PROTB-FRAG1

<400>  652
taccagagaa caaaacaaac ttttcccact atcaggcact actgttttgg gttgtgcagg      60

atgttggtgt gacactctaa cattaaccag aatccttaaa tctcgcatgc agatgtacca     120

acaagagcat aacaaaacaa tgtctacaac tgcatgtgcc cagatgttgt caaccatgct     180

ctactacaag agattctttc ctt                                             203


<210>  653
<211>  291
<212>  DNA
<213>  Diabrotica virgifera


<220>
<221>  misc_feature
<222>  (1)..(291)
<223>  RNAi target sequence DV-PBT6.2-FRAG1

<400>  653
tatagtgtaa aactgatttt tttattttaa tatataccaa aacataccaa agcttaaaaa      60

ataatcaatt aaccttcata aaatttgggc aattcatttc ctaaaacaac tcttcgttca     120

ataccagctt cagtgatgat ccccagtctg actacacctc ctgaagaacc atctcttgac     180

atggcaagtg ccaatgtatt tgtgacaaac ttcacacatt cttccttgct catattgggc     240

ttgaagttgg catctacgta accataaaca taactggaac ctgatcctcc g              291


<210>  654
<211>  277
<212>  DNA
<213>  Diabrotica virgifera


<220>
<221>  misc_feature
<222>  (1)..(277)
<223>  RNAi target sequence DV-PBT6.2-FRAG2

<400>  654
attgacactt cttgtctaac acacatccca ccaattggta tggaatatac ttgtccgcct      60

ttctttttat cccaacctgc taccagtata ccagccatta gcgaatctct ataattgtag     120

caaagttctt ggaaaatggc ggcacctact tgtactttgg gttcttcacc aagttccata     180

ccatgaaaat taagatgata agcaacaatg tctgcaattg cttgtgtatc tgctgcagat     240

cctgaacgac aacagtatat atggtcagtg actttgg                              277


<210>  655
<211>  257
<212>  DNA
<213>  Diabrotica virgifera


<220>

<221>  misc_feature
<222>  (1)..(257)
<223>  RNAi target sequence DV-PBT6.2-FRAG3

<400>  655
tgagtttgtc tgctacccgg tttgcaatgt aggccccagt agttgtgcga gaatctgctc      60

ctataacaac gcctccatca aactccgcgg ccataataga ggttcctgta ctgtgagcgg     120

catctctcca atcattagga ccagtcattg caccatactc agtcataaga ggcatttttt     180

acaagtttaa tgaaaagaat acaagcttag aaaaattaca cttgtaatcc tgcaatgcaa     240

tattttgcca gtccccc                                                    257


<210>  656
<211>  273
<212>  DNA
<213>  Diabrotica virgifera


<220>
<221>  misc_feature
<222>  (1)..(273)
<223>  RNAi target sequence DV-PBT6.2-FRAG4

<400>  656
caggaacctc tattatggcc gcggagtttg atggaggcgt tgttatagga gcagattctc      60

gcacaactac tggggcctac attgcaaacc gggtagcaga caaactcacc aaagtcactg     120

accatatata ctgttgtcgt tcaggatctg cagcagatac acaagcaatt gcagacattg     180

ttgcttatca tcttaatttt catggtatgg aacttggtga agaacccaaa gtacaagtag     240

gtgccgccat tttccaagaa ctttgctaca att                                  273


<210>  657
<211>  219
<212>  DNA
<213>  Diabrotica virgifera


<220>
<221>  misc_feature
<222>  (1)..(219)
<223>  RNAi target sequence DV-PBT6.2-FRAG5

<400>  657
ggctggtata ctggtagcag gttgggataa aaagaaaggc ggacaagtat attccatacc      60

aattggtggg atgtgtgtta gacaagaagt gtcaatcgga ggatcaggtt ccagttatgt     120

ttatggttac gtagatgcca acttcaagcc caatatgagc aaggaagaat gtgtgaagtt     180

tgtcacaaat acattggcac ttgccatgtc aagagatgg                            219


<210>  658
<211>  151
<212>  DNA

<213>  Diabrotica virgifera

<220>
<221>  misc_feature
<222>  (1)..(151)
<223>  RNAi target sequence DV-PBT6.2-FRAG6

<400>  658
ttagacaaga agtgtcaatc ggaggatcag gttccagtta tgtttatggt tacgtagatg      60

ccaacttcaa gcccaatatg agcaaggaag aatgtgtgaa gtttgtcaca aatacattgg     120

cacttgccat gtcaagagat ggttcttcag g                                    151

<210>  659
<211>  229
<212>  DNA
<213>  Diabrotica virgifera

<220>
<221>  misc_feature
<222>  (1)..(229)
<223>  RNAi target sequence DV-BETA7-FRAG1

<400>  659
ggctacagtt ttgtgtccag aaataccaac gcctggtttt tcattcgaaa attgtaaaag      60

aaatgcactt ctggaaaata agggccttgc tcttccaaaa gccactaaaa caggtactac     120

gatcgtagga atagtctaca aagatggaat tattcttgga gcagatacta gagctacaga     180

agacaccaca gttgcagaca agaactgtga aaagattcac ttccttgca                 229

<210>  660
<211>  285
<212>  DNA
<213>  Diabrotica virgifera

<220>
<221>  misc_feature
<222>  (1)..(285)
<223>  RNAi target sequence DV-BETA7-FRAG2

<400>  660
gctaaattag aactccacag attatacaca aaccgagtta ctagagtgtg tacagcaaac      60

caattgttaa aacaatatct atttcgctac caggatatat ttggggctgc tttaatcctg     120

ggtggtgttg atattgaagg accacatcta tatatgattt atccacatgg atccagtgtt     180

gatcttcctt atgctacaat gggttctggt tccttagcag ccatatctgt ctttgaatcc     240

cgctggaagc ctcaaatgga agaggaagat ggtgtccaac tagta                     285

<210>  661
<211>  188
<212>  DNA

<213>  Diabrotica virgifera

<220>
<221>  misc_feature
<222>  (1)..(188)
<223>  RNAi target sequence DV-BETA7-FRAG3

<400>  661
agacatctgt gtaataagaa agggttcagt agactacaaa cgaacctatg acgaggctaa      60

caaaaaggga gttaaacaag gatcttatag atatcaaaga ggaaccactg cagtgcttag     120

tacccaggtg gtggatgttg aggaagtctc agtgattccc attgaagctg aagaaatgga     180

tgttgctt                                                              188

<210>  662
<211>  150
<212>  DNA
<213>  Diabrotica virgifera

<220>
<221>  misc_feature
<222>  (1)..(150)
<223>  RNAi target sequence DV-BETA7-FRAG4

<400>  662
aaggaccaca tctatatatg atttatccac atggatccag tgttgatctt ccttatgcta      60

caatgggttc tggttcctta gcagccatat ctgtctttga atcccgctgg aagcctcaaa     120

tggaagagga agatggtgtc caactagtaa                                      150

<210>  663
<211>  168
<212>  DNA
<213>  Diabrotica virgifera

<220>
<221>  misc_feature
<222>  (1)..(168)
<223>  RNAi target sequence DV-BETA7-FRAG5

<400>  663
cattcgaaaa ttgtaaaaga aatgcacttc tggaaaataa gggccttgct cttccaaaag      60

ccactaaaac aggtactacg atcgtaggaa tagtctacaa agatggaatt attcttggag     120

cagatactag agctacagaa gacaccacag ttgcagacaa gaactgtg                  168

<210>  664
<211>  156
<212>  DNA
<213>  Diabrotica virgifera

<220>

<221>  misc_feature
<222>  (1)..(156)
<223>  RNAi target sequence DV-BETA7-FRAG6

<400>  664
gctaaattag aactccacag attatacaca aaccgagtta ctagagtgtg tacagcaaac      60

caattgttaa aacaatatct atttcgctac cagggatata ttggggctgc tttaatcctg     120

ggtggtgttg atattgaagg accacatcta tatatg                               156


<210>  665
<211>  220
<212>  DNA
<213>  Diabrotica virgifera


<220>
<221>  misc_feature
<222>  (1)..(220)
<223>  RNAi target sequence DV-NATP1-FRAG1

<400>  665
tgaatatcac atctgcagcg ggaattattt ccctcttgga tgaaccaaga ccagaattaa      60

aagtatttgc actgaagaaa ctagattcca tagtgggtga gttttggccg gagatctcag     120

aagctatcga aaaaatcgaa attttgcatg aagacaaagt tttccagcaa caccagttag     180

ctgcacttgt ggcaagtaag gtatattacc atttaggcgc                           220


<210>  666
<211>  222
<212>  DNA
<213>  Diabrotica virgifera


<220>
<221>  misc_feature
<222>  (1)..(222)
<223>  RNAi target sequence DV-NATP1-FRAG2

<400>  666
ttttgaagac tctttaactt atgctcttgg tgcgggtgat ctctttgatg tcaatgcccg      60

gagtgagtat gtagaaacta ttctagctaa atgtatcgac tattacactc agcaacgtat     120

tgctgtagct gaaggattgg cagatgccaa acccatagac tctcgccttg aagctattgt     180

taatcgaatg tttcagaggt gtcttgatga tggtcaatat cg                        222


<210>  667
<211>  204
<212>  DNA
<213>  Diabrotica virgifera


<220>
<221>  misc_feature
<222>  (1)..(204)

<223>  RNAi target sequence DV-NATP1-FRAG3

<400>  667
tcaagctatg ggcctggctc tagaaaccag acgaatggat atctttgagt catctattac      60

tcagtctgat gatattatca gcatgttgac ttatgcattt caagtagcga tgagtttgat     120

tcaaaatcga gcatttagaa acactgtttt gagatctctt gttggacttt atagaggact     180

ggttacacct gattacgtta atat                                            204


<210>  668
<211>  155
<212>  DNA
<213>  Diabrotica virgifera


<220>
<221>  misc_feature
<222>  (1)..(155)
<223>  RNAi target sequence DV-NATP1-FRAG4

<400>  668
tgtatcgact attacactca gcaacgtatt gctgtagctg aaggattggc agatgccaaa      60

cccatagact ctcgccttga agctattgtt aatcgaatgt ttcagaggtg tcttgatgat     120

ggtcaatatc gtcaagctat gggcctggct ctaga                                155


<210>  669
<211>  226
<212>  DNA
<213>  Diabrotica virgifera


<220>
<221>  misc_feature
<222>  (1)..(226)
<223>  RNAi target sequence DV-NATP1-FRAG5

<400>  669
attaacacta atgcaaagtt acttgccgaa agaagttgga cccagctcag gttattccga      60

agggggcggc ctttatgctc ttggtcttat acatgccaac catggtacaa atatcattga     120

ttaccttctt ggacaactta aggatgcaca gaatgagatg gtgagacatg gaggttgtct     180

tggtctaggt ttggctgcta tgggcactca tagacaggat gtttat                    226


<210>  670
<211>  205
<212>  DNA
<213>  Diabrotica virgifera


<220>
<221>  misc_feature
<222>  (1)..(205)
<223>  RNAi target sequence DV-NATP1-FRAG6

<400>  670
gaacaactca aattcaattt ataccaagat gatgccaata ctggtgaagc agctggtatt      60

gcaatgggaa tggtaatgtt gggttcaagt aattctacca caattatgga tatggttgca     120

tacgcacaag agagtcaaca tgagaaaatt cttagaggct tagctgttgg tattagtttt     180

tccatgtatg gtagattaga agaag                                           205


<210>  671
<211>  236
<212>  DNA
<213>  Diabrotica virgifera


<220>
<221>  misc_feature
<222>  (1)..(236)
<223>  RNAi target sequence DV-NATP1-FRAG7

<400>  671
cagatccact gattcaacaa cttacctctg ataaagatcc tatcctcaga agatcaggaa      60

tgtatacttt gggtatggca tattgtggta ctggacacaa ccaagcaatt agaaaactac     120

ttcatgtagc tgtgtcagat gtcaacgatg atgttagaag agctgcagtt acagctttag     180

gttttctgtt attcagaact cctgaacaat gtcctagtgt agtatccctt ctagct         236


<210>  672
<211>  200
<212>  DNA
<213>  Diabrotica virgifera


<220>
<221>  misc_feature
<222>  (1)..(200)
<223>  RNAi target sequence DV-NATP1-FRAG8

<400>  672
gtatggtaga ttagaagaag cagatccact gattcaacaa cttacctctg ataaagatcc      60

tatcctcaga agatcaggaa tgtatacttt gggtatggca tattgtggta ctggacacaa     120

ccaagcaatt agaaaactac ttcatgtagc tgtgtcagat gtcaacgatg atgttagaag     180

agctgcagtt acagctttag                                                 200


<210>  673
<211>  199
<212>  DNA
<213>  Diabrotica virgifera


<220>
<221>  misc_feature
<222>  (1)..(199)
<223>  RNAi target sequence DV-NATP2-FRAG1

<400>  673
tgggcgatac aaaggtagaa cctacaaagg tggataaaac aaaggggact gaggacaaaa      60

acaaaaatga actgtcggaa gaagacaaac agcttcaaga tgaattaaat ttgtgtgttg     120

agaaactcca ggataacgat gaaaagagat acctacttgc tttgcaagct ttatcaaatc     180

atattcaagc ctcaactac                                                  199


<210>  674
<211>  198
<212>  DNA
<213>  Diabrotica virgifera


<220>
<221>  misc_feature
<222>  (1)..(198)
<223>  RNAi target sequence DV-NATP2-FRAG2

<400>  674
gcctcaacta cttcgatgac ttctgttcct aagccgttaa aatttatgag gccccattac      60

gaaaccatga agacagtaca tgaaaaaatt actgatcctg atactaaaaa agtctgtgca     120

gatattatat ctgttttagc tatgacaatg ggagaaggca gagaatgttt gaaataccgt     180

cttctttcta atttggat                                                   198


<210>  675
<211>  190
<212>  DNA
<213>  Diabrotica virgifera


<220>
<221>  misc_feature
<222>  (1)..(190)
<223>  RNAi target sequence DV-NATP2-FRAG3

<400>  675
acatctacca tagagtgtgc ttatatctca ccagctgtgt accctatgtg gcagatcctg      60

agaatactac tctactgcag actgctcttt tactgtttag aaagtttgat cagtatcccc     120

aggcactgag attagcaatg cagttaaatg actttggatt aattgaagaa atttttgtca     180

actgcccaga                                                            190


<210>  676
<211>  223
<212>  DNA
<213>  Diabrotica virgifera


<220>
<221>  misc_feature
<222>  (1)..(223)
<223>  RNAi target sequence DV-NATP2-FRAG4

<400>  676
cggacgtaca aaaacaactg gcatttatgc taggacgaca acagatatat gttgaaattc      60

ctgagcatac gcctgaatac gaagatctaa tcgagataat ggcaaactgt catcttaata     120

accacttctt gaacctcgct cgcgagttgg acattatgga tcctaaaacc cccgaagacg     180

tttacaaatc ccatttagaa aatagtcgtc ctccatttgg tgg                       223


<210>  677
<211>  165
<212>  DNA
<213>  Diabrotica virgifera


<220>
<221>  misc_feature
<222>  (1)..(165)
<223>  RNAi target sequence DV-NATP2-FRAG5

<400>  677
tgctaggacg acaacagata tatgttgaaa ttcctgagca tacgcctgaa tacgaagatc      60

taatcgagat aatggcaaac tgtcatctta ataaccactt cttgaacctc gctcgcgagt     120

tggacattat ggatcctaaa acccccgaag acgtttacaa atccc                     165


<210>  678
<211>  156
<212>  DNA
<213>  Diabrotica virgifera


<220>
<221>  misc_feature
<222>  (1)..(156)
<223>  RNAi target sequence DV-NATP2-FRAG6

<400>  678
catctaccat agagtgtgct tatatctcac cagctgtgta ccctatgtgg cagatcctga      60

gaatactact ctactgcaga ctgctctttt actgtttaga aagtttgatc agtatcccca     120

ggcactgaga ttagcaatgc agttaaatga ctttgg                               156


<210>  679
<211>  222
<212>  DNA
<213>  Diabrotica virgifera


<220>
<221>  misc_feature
<222>  (1)..(222)
<223>  RNAi target sequence DV-NATP3-FRAG1

<400>  679
cttgtgcagg ccatgagaaa agcaccccag aatgctgcca ttggtttcag acaggttgtt      60

caaaagctta ctgttgtagt tgaactactg ctaggagata taccagaacg acagattttt     120

agacaatcta gtatgaggca ctcccttgcc ccatactttc aactaacaca agcagttaga 180

atgggcaatt tgcaaagatt tggtgaggtg ttagagaact tc 222

<210> 680
<211> 222
<212> DNA
<213> Diabrotica virgifera

<220>
<221> misc_feature
<222> (1)..(222)
<223> RNAi target sequence DV-NATP3-FRAG2

<400> 680
ggcccacaat ttagacaaga tcatacattt acactaattt tgagactgcg ccacaatgtt 60

attaaaacag ctataagatc cataggatta tcatactcca gaatatctcc ccaagacata 120

gctaagaaac tggggctgga ttcagctgaa gatgcagaat ttatagtagc aaaagcaatc 180

agagatgggg tgatagaagc tacacttgat ccacagggag gt 222

<210> 681
<211> 159
<212> DNA
<213> Diabrotica virgifera

<220>
<221> misc_feature
<222> (1)..(159)
<223> RNAi target sequence DV-NATP3-FRAG3

<400> 681
caatctagta tgaggcactc ccttgcccca tactttcaac taacacaagc agttagaatg 60

ggcaatttgc aaagatttgg tgaggtgtta gagaacttcg gcccacaatt tagacaagat 120

catacattta cactaatttt gagactgcgc cacaatgtt 159

<210> 682
<211> 161
<212> DNA
<213> Diabrotica virgifera

<220>
<221> misc_feature
<222> (1)..(161)
<223> RNAi target sequence DV-NATP7-FRAG1

<400> 682
ctttagttag atctattgtc gctctacaca atcttattaa caacaaacta acaaatagag 60

acgcggaaga aggaaaaaag gaggatagta agaaagaaag caaagataag gacaaggata 120

aagagaagga aaaggataaa gatgctaaga aggacgagaa g 161

<210>  683
<211>  151
<212>  DNA
<213>  Diabrotica virgifera

<220>
<221>  misc_feature
<222>  (1)..(151)
<223>  RNAi target sequence DV-NATP8-FRAG1

<400>  683
ttgccaaaaa ggctttccag ctcaaaccgt ggaagctcaa caatgacttt ttccactttc      60

ttcctgaaga gaagacccac gaacccatac cctcacagga actggcacag caggctatag     120

agtatgccaa ggaacttgaa atgattgtct a                                    151

<210>  684
<211>  163
<212>  DNA
<213>  Diabrotica virgifera

<220>
<221>  misc_feature
<222>  (1)..(163)
<223>  RNAi target sequence DV-NATP8-FRAG2

<400>  684
gctggacacc ataagagaag aaattgcaga ctgtttatca gtagcctatg aaaagatctc      60

agtaaaagac gtagccagaa tgctgtacct gccaaacgaa gaagctgcta aaacatttgc     120

caaaaaggct ttccagctca aaccgtggaa gctcaacaat gac                       163

<210>  685
<211>  172
<212>  DNA
<213>  Diabrotica virgifera

<220>
<221>  misc_feature
<222>  (1)..(172)
<223>  RNAi target sequence DV-NATP8-FRAG3

<400>  685
tgtacaagca gttaaaatca gaatggaata ccaacaaccc aaatttaagt aaatgtgaaa      60

aactactctc aactcttaag cttgagctca ccaatctaat gtttctgccg attaccacat     120

ctactgcgtc caaacaagaa ctacttctag ccagagatgt tctagagata gg             172

<210>  686
<211>  673
<212>  DNA
<213>  Diabrotica virgifera

<220>
<221>  misc_feature
<222>  (1)..(673)
<223>  RNAi target sequence DV-NATP14

<400>  686
actcacggtt gtttttgtaa aacttgtagg taacgaattt tcgtttttag tgaattaaca      60

aaacaacaga gtgattttcc aacaagaata acttatttat tagtttcatt aatttgataa     120

acaataaaat ggatagactt cttcgactag gaggtggaat gccaggttta actcaggcac     180

cgccgccttc tgatgctcct gttgtggata ctgccgaaca agtatacata tcctccttag     240

cactattaaa aatgttgaag cacggccgag ctggagtacc catggaagta atgggtctta     300

tgttaggaga attcgtagat gactataccg ttcgtgtaat cgatgtattt gctatgcctc     360

agactggtac cggtgtcagt gtagaagctg ctgatccagt gtttcaaaca aaaatgcttg     420

acgtgttaag acagactggt aggccagaaa tggttgtggg gtggtatcat tcccatccag     480

gtttcggatg ttggttgtct ggagtggata tcaacaccca gcaaagtttt gaggctctca     540

cggaaagagc tgtagcagta gtagttgatc caatccagtc agtgaaaggc aaagtcgtta     600

ttgatgcctt taggttaata aatccgaata tgatggtatt gggccaagaa cctcgccaaa     660

ctacatccaa ctt                                                        673


<210>  687
<211>  246
<212>  DNA
<213>  Diabrotica virgifera


<220>
<221>  misc_feature
<222>  (1)..(246)
<223>  RNAi target sequence DV-NATP14-FRAG1

<400>  687
aactttagca gattactctg aaaattgtag tgtgaatgaa aagactgtag ctgatatgtt      60

agaactagcc aagaattaca acaaggcatt agaagaggag gagaaaatga ctccagaaca     120

gctcgctatt aaaaatgttg gcaaacaaga tcctaagagg catttagaag aaaaggttga     180

tattttgatg accaataata tcgtccagtg cttgggagct atgctagata cagttgtttt     240

taagta                                                                246


<210>  688
<211>  205
<212>  DNA
<213>  Diabrotica virgifera


<220>

<221>  misc_feature
<222>  (1)..(205)
<223>  RNAi target sequence DV-ATP5-FRAG1

<400>  688
tgacggtaat taacaagatg gaggtggacg gaggtccgaa gggagaaggt tttcggtcat      60

attatattac aaagattgaa gaattgcagc ttatagtggc ygaaaagagt caaaatctta     120

gacgtttgca agctcagaga aatgaactca atgccaaagt tcgtatgttg agagaagaac     180

tgatgttact tcaagagcaa ggaag                                           205


<210>  689
<211>  219
<212>  DNA
<213>  Diabrotica virgifera


<220>
<221>  misc_feature
<222>  (1)..(219)
<223>  RNAi target sequence DV-ATP5-FRAG2

<400>  689
ctatgtcgga gaagttgtaa agcccatgga taagaaaaaa gtccttgtga aagttcatcc      60

agaaggaaaa tttgtagttg acattgacaa aaacatagac ataaatgatg ttactccaaa     120

ttgtcgagta gccctcagaa atgaatctta cacattgcac aaaatacttc caaacaaggt     180

tgatccttta gtttccttga tgatggtgga gaaagtccc                            219


<210>  690
<211>  244
<212>  DNA
<213>  Diabrotica virgifera


<220>
<221>  misc_feature
<222>  (1)..(244)
<223>  RNAi target sequence DV-ATP5-FRAG3

<400>  690
tagattggac atcttaaaga ttcactccag aaaaatgaac ctcacaagag gaataaatct      60

gagaaaaata gctgagttga tgccaggtgc tagtggagct gaagttaagg gtgtgtgtac     120

ggaagctggt atgtatgcac taagagaacg aagggtgcat gttactcaag aagattttga     180

aatggccgtc gccaaagtta tgcagaagga ttctgagaag aatatgtcca tcaagaagtt     240

gtgg                                                                  244


<210>  691
<211>  163
<212>  DNA
<213>  Diabrotica virgifera

<220>
<221>  misc_feature
<222>  (1)..(163)
<223>  RNAi target sequence DV-ATP5-FRAG4

<400>  691
gattgaagaa ttgcagctta tagtggcyga aaagagtcaa aatcttagac gtttgcaagc      60

tcagagaaat gaactcaatg ccaaagttcg tatgttgaga gaagaactga tgttacttca     120

agagcaagga agctatgtcg gagaagttgt aaagcccatg gat                       163


<210>  692
<211>  176
<212>  DNA
<213>  Diabrotica virgifera


<220>
<221>  misc_feature
<222>  (1)..(176)
<223>  RNAi target sequence DV-ATP5-FRAG5

<400>  692
tagctgagtt gatgccaggt gctagtggag ctgaagttaa gggtgtgtgt acggaagctg      60

gtatgtatgc actaagagaa cgaagggtgc atgttactca agaagatttt gaaatggccg     120

tcgccaaagt tatgcagaag gattctgaga agaatatgtc catcaagaag ttgtgg         176


<210>  693
<211>  1432
<212>  DNA
<213>  Diabrotica virgifera


<220>
<221>  misc_feature
<222>  (1)..(1432)
<223>  RNAi target sequence ta00611.03_diavv

<400>  693
cagaatctta tctgtaaaga taaactaatt atctctattg actcacctca ttttatacgt      60

agcaggtgaa aatacaccat tgtagtgaaa attattatta gttcgattta taataagttc     120

gattttttac gaaaatgaca agtatcgaga ctgtggggac cattgtcctg aaattgctga     180

agttggtgat caatttgata tgtctcatct tgtaccgaac cggatatcaa ggctacttct     240

tgggagtagg aggaacctgg aatctaaacg aagaaaaaaa tcccgatgca gaaattgtgg     300

cttccggcgt attcgtagga tttatgattt acacattcgt ctcgctgatc agcctttgct     360

tcgctagtgg agatcacaaa acgacattca ctgatattct gatgaatata gtagggattt     420

ttatgtggat agctgttgga gctacagctc ttcattattg gcttgggtac ttgtccgaat     480

acaaatacac gacaatagat tctgaacgac aagttggttt ggcgttagga gcgatgtgta     540

taataaatgg agcggtctat cttgtagacg gagtactttc cgcaatcttt atcctcaaag 600

ccaaaatgca ataactttca tcgtaatata aatatattta tttaggttat atactttact 660

ttaagcagct caagtatacc gtgacatccc actcatacat caatgtctat aattgtttca 720

tgacaaatca tttaatagta ttttaaagca ttcattcgtt caacaccttt tttacatttt 780

tcaagtatta tatcaatctt tttaaagaat tgtttggcta ttaaccggat tcaatccata 840

tagttccgta aaataattat gcgttttaat aattaatgta attaaaaata gttagtatat 900

ttttgaaata aaacaactgc cgcaacaaat tagggagaat acttttgtct cctgtagttt 960

tatctgttat ccaaagttcg attaaattgt ttcaatttaa tgtatttttc catataacgc 1020

accaaatagt cttaaaaaaa tagtgcctgt tactacctaa tactttctac gtatgcctta 1080

tggttagtag tggctccaat aggtgcattt tacatttttc atgaaaattc aattcacatt 1140

attttcctcc cgttctcttc atattatatt tatttattct ttaatatagt tttgggcgtg 1200

aagcccagta aatacctata tgaagtatat tgatggaact gccatgcttg atgggttttg 1260

tctgcaatta aaataaaacc aaaacaaaca tttgtacagt attgtaaaat tcacaatatt 1320

taggtagatg tttaaaattt aatatttttt aagagtttgc atttatgtta atcgtagtta 1380

agtttttata aaaatttagt agttgtgaga aataaaataa gtaaaagaga at 1432

<210> 694
<211> 730
<212> DNA
<213> Diabrotica undecimpunctata

<220>
<221> misc_feature
<222> (1)..(730)
<223> RNAi target sequence ta01434.01_diaun

<400> 694
tttatacgta gcaagtgaaa atacaccatc gtagtgaaaa ttaatattag ttaaaacgaa 60

aagttcgatt ttttacgaaa atgacaagta tcgagactgt ggggacaatt gtcctgaaat 120

tgctcaaact ggtgatcaat ttgatatgtc tcatcttgta ccgaaccggt tatcagggct 180

actttttagg agtaggagga acctggaatc taaacgaaga aaaaaatccc gatgcagaaa 240

tcgtcgcctc cggagtattc gtaggattca tgatttatac attcgtctcg ctgatcagcc 300

tttgcttcgc tagtggagat cacaaaacga cattcactga tattctgatg aatatagttg 360

ggatttttat gtggatagct gttggagcta cagctcttca ttattggctt gggtacttgt 420

ccgaatacaa atacacaaca gtagattctg aacgacaggt cggtttggcg ttaggatcga 480

tgtgtatagt aaatggagcg gtctatcttg tagacggagt actttccgca atcttcatcc 540

tcaaagccaa aatgcaataa atttcatcgt aatataaata tatttattta gtttctatat 600

tttactttaa gcagctcaag tgtaccgtga catccaacac aaatcaatat ccataattgt 660

ttcatgacaa atcatttaat aatattttaa agcattcatt cattcaacac cttttttata 720

tttccaagta 730

<210> 695
<211> 1629
<212> DNA
<213> Diabrotica barberi

<220>
<221> misc_feature
<222> (1)..(1629)
<223> RNAi target sequence ta01092.02_diab

<400> 695
tattgactca cctcatttta tacgtagcag gtgaaaatac accattgtag tgaaaattat 60

tattagttcg atttataaaa agttcgattt tttacgaaaa tgacaagtat cgagactgtg 120

gggaccattg tcctgaaact actcaaattg gtgatcaatt tgatatgtct catcttgtac 180

cgaaccggat atcaaggcca cttcttggga gtaggaggaa cctggaatct aaacgaagaa 240

aaaaatcccg atgcagaaat tgtggcttcc ggcgtattcg taggatttat gatttacaca 300

ttcgtctcgc tgatcagcct ttgcttcgct agtggagatc acaaaacgac attcactgat 360

attctgatga atatagtagg gatttttatg tggatagctg ttggagctac agctcttcat 420

tattggcttg ggtacttgtc cgaatacaaa tacacaacaa tagattctga acgacaggtt 480

ggtttggcgt taggatcgat gtgtataata aatggagctg tctatcttgt agacggagta 540

ctttccgcaa tctttatcct caaagccaaa atgcaataac tttcatcgta atataaatat 600

atttatttag gttatatact ttactttaag cagctcaagt ataccgtgac gtcccataca 660

tgcatcaatg tctataattg tttcatgaca aatcatttaa tagtatttta aagcattcat 720

tcattcaaca ccttttttta catttttcaa gtataatata ttatacctac ctatagttcg 780

ttcgctaaac tcagacacaa ctggctagtg atttcggtag ataatttttt tgattttgga 840

caattttacc aaaattacta actacatatt tagtaattat tttttgccaa ttttacgaaa 900

ttgtcaaaat tacttactaa aatcaatagc cagttgtgtc tgagtttagc gaactgacta 960

tatcaatctt tttaaagaat tgtttggctg ttaaccggat tcaatccgta tagttccgta 1020

aaataattat gcattttaat aattaatgta attaaaagta gttagtatat ttctgaaata 1080

aaacaactgc cgcaacaaat taggaagaat acttttgtcc cctgtatttt tatgttatcc 1140

aaagttcgat tcaattgttt caatttaatg tatttttcta tataacgcac caaatagcct 1200

taaaaaaata gtacctacta ataccttcta ctacctaata ctttctacgt atgccttatg 1260

gttagtcgtg gctacaatag gtgcatttta cattttttcc tgaaaattca attcacattc 1320

ttttcctccc gtgctcttca tattatattt atttattctt taatatattt ttgggcgtga 1380

agctctgaag cccagtaaat atatatgaag tatattgatg gaactgccct gcttgatggg 1440

ttttttctgc aattaaaata aaaccaaaac aaaaatttgt acagtattgt aaaattcaca 1500

atatttaggt agatgtttaa aatttaatat tttttaagag tttgcattta tgttaatcgt 1560

agtttaaagt ttttataaaa atgtagtagt tgtgagaaat aaaataaaac caaaacaaaa 1620

atttgtaca 1629

<210> 696
<211> 720
<212> DNA
<213> Epilachna varivestis

<220>
<221> misc_feature
<222> (1)..(720)
<223> RNAi target sequence ta26722.002_epiva

<400> 696
gtaaaaactg acttaattag gtttcagtat ttttcttgtg gttaatttgg aaagtgtacg 60

aaaatgacca gtatagagac agttggtgct atagctattc gtgttctgaa actggtaata 120

aatattattg tcattatcct ctaccgaacg ggcaaccatg gggcttttct tggtgtcggt 180

ggaacatgga acttaaacga ggaaaaaacg gctgacgtgg aaattatagc ttctggagta 240

ttcgttgggt atttgatata cacagcggtc tccttgttga gcttattatt tggaacaaga 300

gacaacaaag tatatttcac tgatgtcata atggggcttg ttggaatact gatgtggctt 360

acagttggtg cagctgccct acattactgg attgggttta tcgacgaaca caaatacaca 420

gctgtatctt cggaacgatc ggttggtcta gcactgggcg ccatgtgcat cctaaattcg 480

gtcctctatt tgatagacac agtggtttca acgttattcg tagttagaga gaaattaaat 540

ggctcatttg cttgatgtgt agtaaattct tacaattagg tttactttta tacttttttc 600

tatttgtttt actgaagatt ttctctatgg agaattcaat cgacccgtga attgtgttcg 660

cagccatact gttagaatga agcgaaaaac tgaagatttt ctctatggag attcaactga 720

<210> 697
<211> 1043
<212> DNA
<213> Leptinotarsa decemlineata

<220>
<221> misc_feature
<222> (1)..(1043)
<223> RNAi target sequence ta37400.0001_lepde

<400> 697
acccactgtt tccatactag tcattttggc acaatcttat cagtcgagat aacgtgatta 60

tctgtaccaa cttactcacc tcattttgtt ccatagcagg tgctttgtgc ctaacagaaa 120

tatttgtgac ttttcaaagt tgattgaaga taaacttgtg ccaaaatgac tagtatggaa 180

acagtgggta cccttttcat aaaactgctg aaattggtga tcaatataat cgttttaatt 240

ttatatcgaa caggattcca gggggaattt ttgggagtag gtggaacatg gaatttgaac 300

gaagagaaga atccggatgc agaaatcgtg gcctccggtg tttttgttgg ttatataata 360

tataacattg tcagtatgat cagcttttgc ttcgccactt tggatcacaa atacaacttc 420

acagatatcc tgatgaatat aattggtctt ttgctgtggg tagcagtagg agcaactgct 480

atccactatt ggcacggtta tttggccgaa cataaatata cccacgtcga ctcggagaga 540

tcggttggat tggccatggg gtccctttgc gtcctgaatg gtgcagcata tttgatagat 600

tcagtgctat cgataatatt cttggttaaa acaaaaatga attgagatca ttcactaacc 660

tgatatgcat ataggataat acttttctta agttttttat atagaataaa attacgacat 720

gacatgttct gatgatgata cattaaatcc tttaagagat agatatcacc agataccaag 780

cacagcatgc tgaaatacat tctttcattc atatagaatt ctttaatcat ttttgagtag 840

ttgggaaaaa ccgaatttgt attagtattc attgtaactt atattttagt gtatgtgtcc 900

gtttgtaata aaatgaatta ttgaaaatgt atcgatttca attcaatcat gagtataatt 960

gaacacaata aagaggaacc tcagggaagt tactgaaata atcgttttat actttaaaat 1020

aaatattctt taggataaaa aaa 1043

<210> 698
<211> 648
<212> DNA
<213> Orius insidiosus

<220>
<221> misc_feature
<222> (1)..(648)
<223> RNAi target sequence ta01487.01_oriin

<400> 698
acgacgctct tccgatctgt gaattaaaaa aaaaaggggg acatcaaaaa atcaaagaaa 60

aatggaaatg gaaactataa ttacgatttt tattaaagtc ataaaatggg tgataaatct 120

cattgtaatc ttcttatacc gatacggtta ttctgggcaa ttcctcggta ttggaggaac 180

gtggaatttg aatgaggaga aagatgcgac agcagaagtt gtcgcttcgg gcgtcctcgt 240

cggctacttt atctacactt cggtcattct aatggcattc tgttttggga cgacaaaaca 300

taaagcgagc gttgtcgaca taataatgaa cataactggg acgatacttt ttgtcgcagt 360

cggcggagtg gcacttcact actgggccgg atatcaaaac atgaatcatt tccagccgat 420

agcgactgaa aaacaagtcg gtttcgccgt cgggagcctc tgcgtcatcg aaggtgccat 480

ttatttagtc gacaccgtcc taacttttgt tcacatcgtt aaaaagaacg acctctttta 540

atcaaaagta caaaaaacaa tattttactt taaagaaaaa ttttaatttt tataaatttt 600

gtattacaac tgaagtatta taataatatt aatctttttt aaaaaaaa 648

<210> 699
<211> 820
<212> DNA
<213> Coleomegilla maculata

<220>
<221> misc_feature
<222> (1)..(820)
<223> RNAi target sequence ta21166.0001_vibdu

<400> 699
aggatcagct taactatcct aatagctata tctccctgtg ttccactctg atcctatatt 60

cacttgtttt tgtgtgtggt gtttttcatc ataatttaat tggtaaaaaa aataattttc 120

gttttttttg tggaaaagtg agtgaaaatg acgagtatcg aaaccattgg agctatagct 180

attaggatag ttaagctgat cctaaatctc atcattttga taatgtatcg aacaggaaat 240

cacggaggtt ttcttggtgt tggtggtact tggaacttga acgaagaaaa aacagcagat 300

gttgaaatca ttgcttcagg agtcttcgtt ggctacttca tctataccgc agtctcactt 360

atgagtatcc tcttcggtac cagagataat aaagtatatt tcactgacgt cataatggga 420

gtcattggcg tttttatgtg gatcacagtt ggtgcagcag ctctgcacta ttggcacggg 480

tacttggatg aacacaaata caaatcagtt tcatcagaaa gaacggtggg attaattatg 540

ggctcgttgt gcatcctgaa tgcagcttta tatctattag actcagtggt tgccgttctg 600

ttcgtcgtaa gagaaaaatt aaatggccaa ttttgaaaaa taatttcata attcataagt 660

gtttttttgt attttttcaa atatgaaatc gacaacgaga gtttaagtcc aaccagaact 720

gatttccaga atgacaaaag ccccaaaggc tatcctcact gcagttattt cgaaatagtg 780

gatacttata atttttaatg aagagcaaat atgaaatcga 820

<210> 700
<211> 5238
<212> DNA
<213> Diabrotica undecimpunctata

<220>
<221> misc_feature
<222> (1)..(5238)
<223> RNAi target sequence ta00809.07_diaun

<400> 700
cgaactttac taaccgtagc tacttttttt gatgtgatct ttatgttgcg ctcatcagta 60

gtgcgccagt ttgtagtgcc aattaaccaa aatgtggtta aagtactttg ttatttttgt 120

tttgtttagt gttagcatta aaggacaaga cgatggactt ccaccagatg tagaaactat 180

cgacaccaat gtaagtaacg cggagccaga tgtagggaaa cctattccgg tggaagatac 240

gcctaatgga gtgtctccag tgaatccaaa ctatgaagta gataaatccc tagttaatgt 300

aagcaatagt ttggtaaatg tgactaaaaa tcccagacgg acttatcaga cagttaacaa 360

actgggcaaa tattcgacat tttatgctgc agattacgat cctatgacct cagacaccgc 420

tccaccagac actgaccaac gaggaggagg gactccatac actataaccg aaacgagatt 480

ggcagaaata aggaaacatt ttatgtaccc atttttcgat aaaggaggta gtgataataa 540

cttgggagat ttccaaaagg acattcaatc ttccattcct caggtgcaca agaatttaaa 600

cttccaattg cctttctttg gattcaggta taactacaca agagtgtcag tgaatggata 660

tctggaattt agcgacccac ctccaaatta tgactatcct ttagtatttc ctattaaaga 720

atggcccaaa aagaatgatc ctgcctttat tggtatattc ttcagcagat gcagaatcgg 780

taatcttcgt gatggagacg tcgatcaaag aacaccaggg gtctatttta gaatggaaag 840

agatttaaga accaggcaag acagaatggg tgtagaaatt agagaaagac ttaaatggga 900

catcagagaa ggcgtcattg gatctgacac cttcgatcct aagcacgccg tgattgtcac 960

ttggaaaaat gtttccttca ctggaggatt cgctaatgct aagtacaaga caaacacctt 1020

ccaaatggtt cttgcaactg acgaagtatt cacttacgcg atgttcaact acctaaactt 1080

ggactggacc tcccacactg aagccggtgg tgattcgcaa aacggagaag gtggtgtcag 1140

tgcctacgtt ggttttaacg cgggaaatgg tactagaagc tacgaataca atccttacag 1200

tcaagcttcc gtcattcgag atcttacttc agttggattt ggaaatggtt ttaagggaag 1260

acatattttc cgtattgatg aggaaatttt gcttggatcg tgcaacaaag atatagatgg 1320

tgccaacctt cccctgaaat tcgctcctga aagtggtaat atgttgggag gaaccgtggt 1380

aaatataaca ggtccatgct tcaatttgaa tgataggatt cgatgcaagt ttgacgtttc 1440

aaatgaagta ttcggttatg ttgttgataa gaacagagct atatgcgtac agccccaact 1500

gtttgccgaa ggatgggtta atcttcaaat tgctataaat tctgaatcat tcaagtggaa 1560

gggaaagtat tatgtagaat ccccttctag tgcaacccaa aaaatcttct tcaaggacat 1620

gaaataccac gaaaaatctc cgagtgaatt aaaaataact tgggagaagc aaaatttgac 1680

cacaaatgaa aacgctaaca tccgaatttc cctctggggt tacagagaaa caactatgaa 1740

accagtgttt gtttatatca ccgatatcgc agatagtgtt caaaacattg gagaatacac 1800

gattgttcct tctcagtata gaactagagt aaaccaatat ttgactgata ttaagtttgg 1860

attcctccaa attaatttaa ctgagtctat caaagttaat acatacacta caacccaaac 1920

aaccatagaa ttaacaccgg tagtatggag ccggcccatt ccactcggtt ggtattttca 1980

attccaatgg gagaaccagt acggcaaaaa ttggcccaaa actctatgtg acgattggct 2040

gagaacggac agatatctaa agaacttcgc tcacgaactt gctcagtgtc cctgcaccgt 2100

cgaacaggct ttagctgaca agggcaggtt tatgccagat ttcgactgtg acaaagattc 2160

gaatccgatt tgttactata acaaccaagc acttcattgt gtgaaaactg gatctcccac 2220

tttagaaggt tctgaacaac aatgttgcta tgataaaaac ggctttttga tgctctctta 2280

cgatcaacaa tggggttcta gtccgagacg ttgccacaac ttaggtaaaa tgccttacaa 2340

tgaagcgaca aaagttccta ctctctcaca atggtttaat gatatggtac ccaaatattt 2400

atgttgcctt tggcaagacg agcaagctgt tggatgtgaa acactgagat ttgaacgtag 2460

accaacacaa gattgtgtag cttaccaagc accaggcgtc gcaggagtct atggtgatcc 2520

acactttgtt actttcgacg atgtagaata tacgttcaat ggaaaagggg aattcgcatt 2580

ggtgaaatcg gttacacaaa cggacaatct ggaaattcaa ggaagattcg agcaaatgga 2640

ccctaacgct tatggtgagg ttagagctac tcagctgaca tctgtagtag ctaaaggtaa 2700

taacacaata gcaattgaag tcagaaggcg accactagat tctagatgga gatataggtt 2760

ggatgttata gcagataata gaagactgta tttcgataga ccctcactaa aattccagca 2820

cttccaagga gttactattt atactccaac ctacatcctt aatcaatcag aagtcatcat 2880

tatgtttgac aatggagctg gaatggaggt tgtcgataat caaggataca tgagtgcaag 2940

agtatttttg ccttggtcgt ttataaataa aaccataggc ctttttggaa attggagttt 3000

caacaaagaa gacgacttta cacttccaga tggttccaga gcagcaatag tctctaacat 3060

aaatgacatg gaaagggttt acactgattt tggttccaaa tggatgttag atgatgtgtt 3120

agatcctcaa aagggtagag cgttattcca tagggaattt ggaagaactt cagccactta 3180

caacaacaag acgttcaagc ctcaattcct aatgaccccg gaagatttca taccagccaa 3240

cagaactgcg gatattcagc gaacatacga aatatgtcct ctaaggatgt acgaatgtta 3300

ctacgattac ggtatgacgc ttgataggga tttggcacat tataccaaga attataaagc 3360

cacaatatat caatataaag aaactacaag gaccaaagtt gtgtcttgtg gtgtactgga 3420

aacaccgagg tttggacgaa agagtacttt cctgttcgtg cctggaacta aggttaccta 3480

cgaatgcatt caggagttcg tgctggtagg tgatcagcgc agggagtgtc aagcggatgg 3540

aacgtggaat attcctgaat acggatacac ttattgctta cgtcagcaag aatactccca 3600

acgacaggct ggtctggctt ccgggataat tatggcaatt cttataccgc tcatcttgct 3660

tttcgtctat ctagcgtatc gtttcttaca aaaaaagcag aaagagaaag aagctgaaaa 3720

attagaagag cagcaattta tggatcaaca aagaagggca cacgaagcag ctactaagaa 3780

gttaacagaa gccgaatatg cttcagaaga tgaagacagt aatgttacaa gcacgggcgc 3840

aaaagaaaca actgtatatt aactcctata aaatacaatg attcaaccta tgagttgcaa 3900

cattttaaag tttaatattt ctaaaatctt tatatagaat aagtactaga tgtagttaat 3960

gtataaatag gactttttta ttgtaggatt aaaaatccac tgatgactaa attcaaacac 4020

tatgttttat tcaataaagt gtagactttt ttatgtttat gttccatgta gagtgtgatg 4080

tgtcaaatat ggcgttttta accaccacgt aatgtcttct tcctatagta aatttaatat 4140

tcttcaaagg tacttgtcca ttgaattgta tgcctatcta atcctgtaat gcccatcttt 4200

ttttttcgaa ttaaatgaat aatacaaaat ttgaaaatca aaataaatta atcaacttca 4260

tataacccgg tagaaagcga tgtcctgcaa ctattatcta aattaatata ttattcagta 4320

accatatcaa gctctattag tggtgttata ctcctgtatt ttataaacaa cggtttgtta 4380

atccaccaac ttgcttctat ttgagaagat gaatcaatac gctcagctgc tcacataaaa 4440

tttcacatag aatcgtttca agcaagccag agtcatactc acaatttgtc aaaatttttg 4500

ccgacaaact gtatttagca tagaactatc ttatactagg aagatcagaa aatatctatg 4560

ccatgaactt aagcacaaaa ctagccaaat gaaacggctt attggaaggg atatgaagtg 4620

gaaaatggat gtacctaatt atgctaaaag tttttcacat accaaagcat ttcacaaatt 4680

tcggccgaat attgttatca cagtaattgt atcttgcatc gttagtctga aatatcatac 4740

atcgacaaaa ttattagatc taaaatcatc ctaagctaaa catacagttc aaaaccattg 4800

ttgcgtggtt acaattctag ttttgtgtat aaacttcgtc cacataagtc gaatccggtt 4860

tgaatctact cacgagtttg cgataatcgg acttttagat ttatatatgt atctaatcca 4920

ggattccaaa tataataatg tctaaaatgt aattgttcaa accgaagtag gaatctctaa 4980

gaccacttaa ttataaaagt agttatttga aagagaaggt aatagttacc tgcgttcaca 5040

tatacaaatt aattagaact tgaaagaaat tcgttgttaa aacacctatg aaaagaaaaa 5100

ttgatttagt caacgaagga ttccttctag aatgttctat gttatttatt aagtacaaca 5160

tattttccta ccttagtaat actttacttt attagaaaat ctagtacagt tcgtgttaaa 5220

tctcaaattt actattat 5238

<210> 701
<211> 4313
<212> DNA
<213> Diabrotica barberi

<220>
<221> misc_feature
<222> (1)..(4313)
<223> RNAi target sequence ta06241.06_diab

<400> 701
aaaacataac tttttgactg aagaatgttt ctaggtacag cggcacaaat actttggtct 60

taagatagta aaataatgaa aatttataaa ttaaatgata ctctataaac tattgaagtt 120

cacttctact ctaattatat tctaaacgtc tgtcactata ctactctgac tagcgctaat 180

cctttcttta ggtttcttta ttggttctgc atacatgtcg ttatagttca aattctttga 240

gctgttccct ctaataatgt ctggtcgact ataaggcgtt gtatattgaa tactagaagt 300

aggactagtt ggacttcccg taggcgttct agaaacccta tcgtcttcga aggtttgatc 360

gtaagggtcc aatggcttct cctcgaactc agaattcggt ctattgggca aaggttcgtg 420

agttctgtag gacttgtcgt aacttcttcg tttcttgcta agcgagtcag tgtcgctttg 480

aggatacttt tcgtcctcgt cttcttctct gtatggactt aaagttcgac tgaattgttg 540

gagattttga gtcttttgta cggtgtcgta gtcatagttt tgattgttac ttgttagtct 600

ttgatatact ttgtagccac cgtaacctat tagaagaacc aatggtatga taattgcgag 660

gattatacct gaagtgatgg cagcttgacg cgaagaatat tcttcttgac gtaagcaata 720

tgtgtatccg tattcaggaa tattccatgt tccatccgct tgacactctc tccgctgatc 780

acctaccaac acgaactctt ggatgcattc ataggtaacc ttcgtaccgg gcacgaacag 840

gaaagtactc tttcgtccga atctcggtgt ttctagtact ccgcaagaca caactttcct 900

ccttgtcgtt tctttatatt gatagatcgt ggctttataa ttcttggtgt aatgcgccaa 960

atccctgtca accgtcatag cgtaatcgta gtaacattcg tacaacttta gaggacatat 1020

ttcggatatt cgcttaagat ctgtagatct gttggttggt aggaaatctt ccggattcat 1080

taggaattga ggcttgaacg ttttgttgtt gtaagtcgct gaagttcttc cgaattccct 1140

gtgaaacagc gctctaccct tttgaggatc taacacatcc tccaacatcc atttggaacc 1200

aaaatcattg taaacccttt ccatgtcatt tatgttattg actattgctg ctctagaacc 1260

atctggaagt gtgaagtcat cttctttgtt gaaactccaa tttccaaaaa ggcctatggt 1320

tttatttata aacgaccaag gcaaaaatac tcttgcactc atgaatcctt gattgtcgac 1380

aacctccatt ccagctccat tgtcaaacat aatgatgact tctgattgat taagaatgta 1440

ggttggagtg taaatagtaa caccttggaa gtgctggaat tttagtgagg gtctatcaaa 1500

ataaagtctt ctattatctg ctataacatc caacctatat ctccatctag aatctagagg 1560

tctccttctg acttcaattg ctattgtgtt attaccttta gctactacag aagtcagctg 1620

agtagctcta acctcaccat aagtgttagg gtccatttgc tcaaatcttc cttggatttc 1680

cagattgtcc gtttgcgtaa ctgatttcac caatgcgaat tctccttttc cattaaacgt 1740

atattctaca tcgtcgaaag taacaaagtg tggatcaccg tagactcctg ctacacctgg 1800

tgcttggtaa gctacacaat cctgtgtagg tctacgttca aatctcagtg tttcacatcc 1860

cacagcttgc tcgtcttgcc aaagacaaca caaatatttg ggtaccatgt cattaaacca 1920

ttgtgagaga gtaggcactt ttgtcgcttc gttgtaaggc attttaccca agttgtggca 1980

acgtctggga ctagaacccc attgttgatc gtaagagagc atcaaaaagc cgtttttatc 2040

atagcaacat tgttgttcgg agccttctaa agtgggagat ccagttttca cacaatgaag 2100

tgcttggttg ttatagtaac aaatcgggtt cgaatctttg tcgcaatcga agtctggcat 2160

aaacctgccc ttgtcagcta gagcttgttc gacggtgcag ggacattgag caagttcgtg 2220

agcgaagttc tttagatatc tgtccgttct cagccaatcg tcacataaat atttgggcca 2280

atttttaccg tactggtttt cccattggaa ctggaagtac caaccgagtg gaatgggtcg 2340

gctccatact accggtgtta attctatgct gctctggctt gtagtatatg tattaacttt 2400

gatagactca gttaaattaa tttggaggaa tccaaactta atatcagtca aatattggtt 2460

cactttagtt ctaaactgag aaggaacaat cgtgtattct ccaatgtttt gaacattatc 2520

tgcgatatcg gtgatataaa caaacactgg tttcatagtt gtttctctgt aaccccagag 2580

agaaattcga atattagcgt tgtcgtttgt ggtcaaattc tgcttctccc aagtgatttt 2640

taattcgctc ggagattttt cgtgtaattt catgtccttg aagaagattt tttgagtcgc 2700

actagcagga gattctacat aatacttccc cttccacttg aatgcttcgg aattaacagc 2760

aatttgaaga ttcacccatc cttcggcata aagttgaggt tgtacacaga tcgctctgtt 2820

tttatcaaca acatacccaa atacttcatt tgatacgtca aacttgcatc gaatcctatc 2880

attcaaattg aagcatggac cagttatatt taccactgtt ccacccaaca tgttaccact 2940

ttcaggagcg aatttcaggg gaaggttggc accatctata tctttgttgc atgatccaag 3000

caaaatgtct tcgtctatac ggaaaatatg tcttccctta aaaccatttc caaatccaac 3060

tgcagtgaga tctcgaatga cggaagcttg actgtaagga ttgtattcgt agcttctggt 3120

accgtttcca gcgttaaaac caacataggc actgacacca ccttctccgt tttgggaatc 3180

accacccgct tcagtgtggg aggtccagtc caagttgagg tagttgaaca tcgcgtaagt 3240

gaagacttcg tcagtggcaa gaaccatttg gaaggtgttg gtcttgtatt tagcattagc 3300

gaatcctcca gtgaaagaaa cgtttttcca agtgacaata acggcgtgct taggatcgaa 3360

tgtttctgat ccaatgactc ctgttctgat gtcccactta agtctttctc taatttctac 3420

acccattctg tcttgcctcg ttcttaaatc tctttccatt ctaaaataga ccccgggtgt 3480

tctttggtca atgtctccat cacgcagatt accgattctg catctgctga agaaaatacc 3540

aataaaggca ggatcattct tttgggccaa ttctttaatt ggaaatacta atggatagtc 3600

ataatttgga ggtggatcgc tgaattccag atacccattc actgaaactc tcgtgtaatt 3660

atatctgaat ccaaagaaag gcaactggaa gtttaaattc ttgtgcacct gaggaatgga 3720

agattgaatg tccttttgga aatctcccaa gttattatca ctgcctcctt tatcgaaaaa 3780

tgggtacata aaatgtttcc ttatttctgc cagtcttgtt tctgttatag tgtatggagt 3840

tcctcctcct cgttggtcag tgtctggtgg agctgtgtct gaggtcatag gatcgtaatc 3900

tgcagcataa aatgtcgaat atttgcccag tttgttaact gtctgataag tccgtctggg 3960

atttttagtc acatttacca aactattggt tacattaact aaggatttat ctacttcata 4020

gtttggatta actggagcga ctccattagg tgtatcttcc accgggatag gtttccctgc 4080

atctggattc gcgttactta cattggtagt gtccacatcg atagtttcaa catctggtgg 4140

aagaccacca tctagtcctt taacgctaac actaaacaaa acaaaaataa caaagtactt 4200

taaccacatt ttggttattt ggcactacaa tctggcgcac atcacatcaa aaaaagttgc 4260

tacggttagt aaagttcgta ctcgagtgtt tgcaagaatc gctagaacga gat 4313

<210> 702
<211> 1564
<212> DNA
<213> Epilachna varivestis

<220>
<221> misc_feature
<222> (1)..(1564)
<223> RNAi target sequence ta29117.001_epiva

<400> 702
cttcaatgga aaaggtgaat tcgtgctgat gaagactaac gtggcttcgc agaggttaga 60

agttcaagct cgtttcgatc aaatgccacc taatgaatac ggtcaagtta gagccaccca 120

gctctcctcc atagctgtca gaggcaattc ttcgactatt attgaagtta gaataagacc 180

aaaggaagcc aggtggagat ataaaatgga tgttattgcg gataagcaaa gaagactttt 240

cttcgaccga ccttcgctta aattccaaca ttttccaggt ctaaccgttt acactccaac 300

atatatcctg aaccaatctg aagttattat aatgttcgac tcaggagctg gtgtagaaat 360

tgtagaaagt ttgggattca tgtcagcaag agtttactta ccatggactt tcataaataa 420

aacaatggga cttctaggaa attggagttt tgacatgacc gatgatttca ctttacctga 480

tggtacaaaa cgacctgttt ctggtaatat caacgacatg caagacattt acgaaaactt 540

cggcaagaaa tggatgattg atgactcaga cgatcctcaa aaaggaaaat cgctgttctt 600

tatcgaaaac ggacggactg cagccttcta caacgaccta aatttcgtac ctgaatacag 660

catgaatata aactacatac tggcaaacaa aacaggtcgt attgaaatgg caaatcaatg 720

gtgttccaat aaagactatc agtgtatgta cgactatgcg atgacattag ataaagatat 780

ggctttttat accacaattt ttaaatcggg cttgcaaaac ctaagagaag cggcaagcaa 840

aagggtaacg tcttgcggtg tgttggaaac gcccaggttt ggttggaaaa cgaatttctt 900

cttcacgcct ggaactaaag tcagtttcga ttgtgctcaa gacttcgtat tgagcggtga 960

tcctagaagg gaatgcctga agaatggaga gtggaatatc ccagaatttg gttatactga 1020

atgtttacgt gaagaagaat attctctgag acaggcagga actacatttg gaataatact 1080

tgcagtgatg attcctctta tacttttatt tgtattcttt ggttacaaat atcttcagaa 1140

gctgacagcg caaaataatg agcaatatga agagaaaatg acattggaac aacaaaaaag 1200

gttagcacaa gaaagggcag ctaaaaaatt aaccgccgtt gatgaggacc atgatgatga 1260

tgtgctttcg gaaacaaata cttcatcgcc aagagaaaca acagtgtatt aattcgtaaa 1320

tttcgattag tcatgagtta ggaaaacgta gtttgccatc attcataata ttttacttca 1380

aaatttgaat gtgaaaattt tttcaaaaaa tttttaaata tggatatata aaattatagg 1440

agtttttgtt tgatgattgt taagtagaat ccaatcgctc tctataaatc aaaaaattca 1500

gagacagaac agctctttta gatttagagg tattttaaat gttcataatc gcattttaca 1560

tttc 1564

<210> 703
<211> 5020
<212> DNA
<213> Leptinotarsa decemlineata

<220>
<221> misc_feature
<222> (1)..(5020)
<223> RNAi target sequence ta36833.0009_lepde

<400> 703
ataacaaatt ttatcttgtt acagagcaca cttgtgagaa ggcaatcgag taacgaacct 60

catacataaa agtacacgta tggtgataga aaacaataaa ttatcaaact gaggctctgt 120

gccaagtttt ggaaaatgta cgtcaagtgg aaattggttt tgactctagt actgtgtgtt 180

ggtgtcgtga ttggggaaga tatttcaaca gatataatcc ccttgccaca agacaataca 240

gcagatgtgg aaatagtggc tacagaaact aggtcaaatt caggatctga agctgctgta 300

gaagagccca ccaataacac tgggccttca gatactacaa acccaaatta tgctgtagtg 360

ccccctgtag tttctgatag caaacctgca ataacaaata gtgaaacaaa tgacactaac 420

aatgatgttg taatgttgag tcctacgaaa tttctcatga aaaagggacg atctggacgt 480

ttattggaat atccgactga ttacgatcct atgacttccc atattgctcc accggatagt 540

gatcagagag gatattcagg agtaccatat gtcttgacgg aaaccagact gcaacagatc 600

cgccaaaatt tcatgtatcc ctactacaac agaggcggta atgcagatga cgaaggagac 660

taccagaaag aaattcagtc atctattccg caagtgtaca agaacctcaa cttccaactc 720

cctttcttcg gatttcgatt caattacacg agggtctcct taaacggcta tttggaattc 780

agcgatcctc ctccaaatta cgactatcct ttggtctttc cagtcaagga atggcctaaa 840

aagaacgacc cttctttcat cggtatcttt ttcagtaaat gtagaatcgg taacctgagg 900

gacggagata ttgatcaaag agaccctgga gtgtacttta ggatggaaag ggatctcaga 960

aataggcagg acaggatggg agtggagatc agagaacgac tgaaatggga tataagggaa 1020

ggggtgatag ggtcagaaac attcaatccc aaacatgcca ttatagtcac atggaaaaat 1080

atctctttca atggaggttt tggcaatgct ctctaccaga ctaacacttt ccaaatgatc 1140

ctcgccactg atgaagtttt cacctacgcc atgttcaact acttgaatct tgactggacc 1200

acccacactg aagcgggagg cgacacaaga aaaggagaag gaggagttcc cgcttttgtg 1260

ggattcaacg ctggaaacgg tactagaagt tttgaataca aaccatacag tcaagaatct 1320

gttattcgag atctcacaca aactggtttc gctaatggtt tcaaaggaag gcacattttc 1380

cgaatcgacg aaaatatcct aactggaaca tgcaataaag atatagatgg tgctaatcta 1440

ccgctaatga tatctccaga aagtggaaat atgctgggtg gaacaatagt gaatataaca 1500

ggaccttgtt tcggcctaga cgaccaagtt aaatgcaaat ttgatgtagc aaatgaaata 1560

aatggcgtcg ttatagataa aaacagggct atatgcatcc aacctagact gtatgccgaa 1620

ggatgggtga atttacaaat agccataggg gctggggtat acaaatggaa gggaaaatat 1680

tatgtcgaat cccccgcagc ggcatctcaa aaatctactt caaggacatt gaaggttcat 1740

gaaaaatcgc ctagtgaaat aagaataact tgggaaaaat acaacttgac cactaacgaa 1800

aacgctaaca ttcgtatctc cttatggggt tacagagaaa caacaataag gccaacgttc 1860

gtttacatca ctgatatcgc agacagtctc caaaatactg gagagtatac catcgtaccg 1920

tcccaatata gaacaaaagt taatgagttt ctcacggata tcaaatttgg tttcttgcaa 1980

attaacttga ctgaatctat caaagtgaac acttatacat ctgtacaaag atcggtggaa 2040

atagttcctg ttgtatggag tcgacccatt cccctaggat ggtactttca gttccaatgg 2100

gaaaatatgt atggacgaag ctggcccaaa gcactctgcg atgactggct aagaacagac 2160

agatacctga aaaactttgc tcatgagtta cctcaatgcc cttgcactgt agaacaggct 2220

ttggcagaca aagggaggta tatgcccgac tttgattgcg acaaggactc aaatcccgta 2280

tgctactaca ataaccaagc tctgcactgt gtgaaaacag gatcaccaac gttggaggga 2340

tcagaacagc agtgctgcta tgacaaaaac gggtatctca tgttatcata cgatcagcag 2400

tggggttcaa gtccacggcg ttgccacaat ctgggaaaaa tgccctacaa cgaagcaaca 2460

aaagttccaa ccttatcgca atggttcaac gatatggtac cgaagtatct ttgctgtttg 2520

tggcaggaag aacaggcggt gggttgcgaa acgctgagat tcgaaagaag accaactcag 2580

gactgtgtcg cgtaccaagc tccaggattt gctgggattt acggagatcc ccacgtcatc 2640

actttcgatg acgtcgagta caccttcaac gggaaaggag agtttgctct tgtgaaatct 2700

gtgacacaaa ctgacaactt ggaggtgcaa ggcagatttg agcaaatgga ccctaacgcc 2760

tacggagaag tacgtgcaac acaattgact tcaattgtgg caaggggaaa caacaccata 2820

gcagtggagg tcagaaggag gcccttggat gctaggtgga ggtataggct ggatgtcata 2880

gctgataata ggaagttgtt cttcgacaga ccctctttga aatttcaaca tttccaagga 2940

gtgactattt atacacctac ttatatcctc aatcagtctg aagtcatcat tatgtttgat 3000

aacggagcag gagttcaagt aatggataac cagggattca tgaccgcgag ggtgtatctt 3060

ccttggtcat tcatcaacaa aactgttggt ctctttggca actggagttt caataaggaa 3120

gatgacttca ctcttcctga tgagtcgaag gctgccgtcg tgagtaatat caatgatatg 3180

gaaagggtct acaatgactt tggttccaaa tggatggtgg acgacgtact agatccgaaa 3240

agaggtagat ccttattttt cagagaattc ggcagatcat cggcaacgta caacaacaaa 3300

actttcaaac cgcagttcct tatgttacct gaggacataa tacccgcaaa caggtcgata 3360

cagatacaga gaacttacga catttgtagc acaaaaatgt acgaatgctt ctacgattat 3420

gccatgacgc tcaacagaga tcttgcccat tttactcaga attataaagc aaccatatat 3480

caactcaaag aaacgacgag gcagaaggtt gtttcttgcg gagttctgga aacaccgcga 3540

ttcggtagga agagtacttt tctttttata ccaggaacca aagtcactta cgagtgcaat 3600

caagacttcg tattggtggg agatcccaga agagaatgtt tggcagatgg cacatggaat 3660

gctcctgaat atggctacac cgaatgttta cgtcaacaag aatattctca gcgccaatca 3720

gccattgcct ctggagccgt tctcgcaata attattccac tagttctatt atttgtatat 3780

ctggcttata tgttcctcaa gaagaaacag aaagaacgag acgaagaaaa tttacaaacg 3840

caagcgtacg agcaacagaa aagacaagct caggaagctg ctgctagaaa attaactgct 3900

gccgaagagt acaactcaga tgaagacgat aataacagca acgttacgag cacagcaaaa 3960

gaaacaacag tgtattagta ttagatatgg taacactttt acgatattat tattttggtt 4020

ggcatacttc tcagacaaac agtccaaatt attatttgca acaagaacta tttgaatttc 4080

gaagtttcct ttgaagatcc caagttgctt attatcctaa atttttgcaa aatgtcaaaa 4140

tcgtactatt ccatcagttg tatattttat ttttctgaga tagttaggcc aacaggtatc 4200

taaaggagtg aataacagat ttttcatatt ttccagtttt atctgattca ataatgcact 4260

atgggttttt tctgttaatg gtttttataa cacctctgtt gcaaaataca tattaggtaa 4320

tataaatata cgtaattaag tcgtaattaa gtattataac aaaatggcgt tctctattat 4380

ttaaattgta attagatata tattttttat attcataatc acttttgcat gtaaagtagg 4440

agtccaattt tctgtacttc aaccatcact atcgctactt accccgatca ctttttggat 4500

gcacatttca tgcacatttt tgcacattgt ttgcacagta agttcacttt gcaaacaatg 4560

tgcgaagact atgcgggagg acatgtttat gcagtaattt tgtatatatg tgagtaggta 4620

tatacctact attttcatca tactctggtc ctacttttct tatttacagt gataagatga 4680

tgtttgaaat cttgaattag gtacaatgaa ttcatttttg tcagttattt gaaacatttt 4740

cggatatttt aataagactg tgttttatca aagttcagtt tgattgtcca aatactagta 4800

gatcattaga gtcctactgt agttttagca ttttaataaa gtaattcaat attaatatac 4860

aagtaatctt gcacttctgc taactcaaaa ttttgcaaaa ttattcactc gaatcagtat 4920

tcacacgttt ctctttcaga aataaccaaa atatttcacc ccatacacaa atttcattag 4980

gtttagtgaa atttgtgtat ggggtgaaat attttggtta 5020

<210> 704
<211> 3724
<212> DNA
<213> Orius insidiosus

<220>
<221> misc_feature
<222> (1)..(3724)
<223> RNAi target sequence ta02139.06_oriin

<400> 704
tttttaacgt taataaaaca gttttttaat ataaaaaaag tgtacaaaag tttagtatat 60

gtcggtttct acgctatcgg cgcgacgcat ttgagatttg ggggagtcga acgagctgtc 120

ggttccgtct tcgaagtcga tgtcggggcg gttcggaagg ggctcgtgtg tattgtaaac 180

ggcgtcgaaa ttgctcggat tcctaacgag tttatcctct tctttacccc ggccagataa 240

ggaagaacgc ctcctgctgt acttccagct ttggaccccg ttctgaagac gggctgagtg 300

acggcgataa aaccacgtcg cgatacagac cagtgtgagg accaacggca aaagaatgcc 360

caaaatcata agcgacatat tcgccagttg ttgcctgtcg tattcgacat acctgagaca 420

ttcggtgaac ccttcatccc cttcgatcca tttcccttga gcggagcact tcctttgttg 480

atctccaaca aaatgaaacc ttgatcgcat tcgaaaataa ctttggtatc aggaacgaag 540

aagaagttac ttttccttcc aaatcttggc gtttcaagaa ctccacagct caatgttctc 600

attttgttgg tctgcttgat ggatgaatat tcgctgaagt agtccaaagt ctgtttcgcc 660

atttcacgat tgaggctcac cgcgtaatcg tatttacatt gatacgagtc gccgcagagg 720

tcttcagcct tagagacgtc ctgtgtccga ttcggtggta tgatttcctg aggagttttt 780

ttccattctg gtacaaaagt acgattcgca tagtacgatg ctgaacggcc atactccctt 840

atgaagaggg gctccccttt tttcggatct gctctatctt ccagcaacca tttgagagca 900

aaatctttgt gtattcgttc gaaatcttca aggtctccga cttggacgat ttgtccgtca 960

ggagtggtga agtcatcgga aatgtcgaag ctccaatttc ccatcaaacc tcgcgtttga 1020

ttgatcatca tccagggcaa gtagacacga caggccatgt atcctttgtt ctcaactacc 1080

tcaactccta ctccggtttc gaacataatc acgatctgcg attggtccaa aacctgagat 1140

ggcaaataga cggtgacgcc tcggaagtgc tgaacacgaa gtgcaggacg gtcgaaaaag 1200

actcgacgtt tgttggcgaa aacgtccaat ctgtatcgcc attgggcgta tcttggtcgc 1260

aagcggactt cgatgatcgc cgaattattt cctctagctg caacagatgt cagtaaagtg 1320

gcagcagtgt ctccgtagat attcggcccg acctgttcga atctcccttg tacgtcaagt 1380

ttgtatttat ctgttgaaga atggagcaaa acgaattcgc ctttaccgtt gaaagtgtat 1440

tcaagctcgt cgaatgtgac aatgtgagga tcgccgaaga cggtagcgac gtaaggcgct 1500

tggtatccta cgcagtcttg actgggtcgc cgttcgaaac gtaatgtctc gcatccgaca 1560

gcttgctcag actgccataa acaacagtgg tagaatgggc ttatatcatg ccaccactgc 1620

gataatgttg gaactttgtt cggctcagtc cacggtaaaa gacccaaatt tgatgatctc 1680

ttcggcgaag acccccattt ctggtcgtac gacaacatca aatattcgtt tttgtcataa 1740

cagcattgtt gttctgctcc ttccaaattc ggagcaccag ttttgacgca atgacgtgca 1800

cctttgtggt aaaaacagtt cggattcgcg accatatcgc aatctggatc gggcaaaaaa 1860

cgccctttgt cgtttaacgc ttgctgtaac gtgcacgggc attgggccaa ctcgttagcg 1920

aaattcttca aaaatctatc gttcataagc cactggtcgc atttcgcttc tgcccacttt 1980

ttcccgtaga gtctctccca ttgcggagcg aagtaccatc cgagaggtat cggcttactc 2040

catattacgg gggttatgct aagtccttga tattgggtcg ggtttgtcaa gttgatttga 2100

atgaagccga aagtgagctc ggacaattca tagttgttcc ttgtccggaa attggccggt 2160

tgtatgacgt agctgcctgt gtttgccaca cctgattcta acgtgtcaac gtaaagaagt 2220

ttcggtgcta tattcgtctc tttgtacgcc caaatcgaaa tctgcaaagg agccgcagaa 2280

ttggaagtta aattgtattt gttccacgtt attttgattt ctccaggata agcatcattc 2340

acacttccat aatgatcgtt ctcagcaaaa gagatccttt gcgtcgccgt cgctggagtt 2400

tcgacaaagt atagtccttt ccaaatatat ttgtcgtcgt ttaacgagat ttcgaagggt 2460

atgtaacctt cagccatgat gaaaggctga acgcatatcg cccggttggt gtcgaccact 2520

gttcctaaag tcggcgtcgt atcaaacctg catattatct tcatttcttt ggtaaaacaa 2580

ggtccggtga tgttcacaat agtcccgccc aacatgtttc cagattcagg tgcgaaatta 2640

aggtccaaac ttgctgcggc tatatccttg ttgcaagatc ccggaagaat gttttcgtca 2700

atacggaata tgtgacgtcc aggaaatccg ttagcccaac cacgccccgt taaatctcga 2760

atcgtacttg cctgcgaata aggtttatat tgataagtcc gagtcccgtt tccagcattg 2820

aatccaacaa acgcaggagt acctccttca cctccagtgg tatcaccatt tgcttcagtg 2880

tgcgttgtcc attgtatgtc cgcatagttg aacatcgcat atgtgatgac ttcatcagta 2940

gccaatacaa gttggaatgt attcgttttg tagagactga tgtcgattcc tccggcaaaa 3000

gatacgtttt tccaggtgat gatgactgcg tgttttggaa cgaatgagtc agaaccgatg 3060

acgccctgac ggatgtccca cataagacgt tctctaactt caaccccgaa acggtcagtc 3120

cttccttgta aatctctttc cattctgaag tagacaccag gaggtctttg atccacatct 3180

gtatctttga tgagaccgat acggcatttg gaatagaaaa taccgataaa actcggatcg 3240

ttctttttgg gccactcgac cgtcgggaaa tgaagggggt aactgtactg ttctggaggg 3300

tccgaaaact cgagataacc gtttaagctt acgcgtgtgt agttgaagcg gaaaccgaaa 3360

aagggaagtt ggaaattgaa gtttttatgg atttgggtag aggaaggatg aatgtctttt 3420

tgtaggtcac cggaatcatt tggaccacct ttatcgaaga accaataaag gaagttggaa 3480

cggattttcc gtaaacggtc ttcagttatc acgtacggga gttccccagg ccctgcacgt 3540

tgcgttggag gtgccgtcct ttcatcatca aaatcgtatg gatttctcgg gttgtagggg 3600

ttgtaaggag tcaaaaccct cgaaggctgc ctgtagttgt tgctcctcac gttgataact 3660

tcattgaacc ccgggcgcac actttgtcct ctttggcctt ccgaactttt caaaaagtaa 3720

aaaa 3724

<210> 705
<211> 3845
<212> DNA
<213> Coleomegilla maculata

<220>
<221> misc_feature
<222> (1)..(3845)
<223> RNAi target sequence ta22533.0010_vibdu

<400> 705
caaatttcaa aatagaaatg aatctaaaag attagcaaat ccgatcatta gtgatgtaca 60

atactgatga acggaacatt ttaacatggt gaaaattcga atattagtta ctttttgtat 120

attcaaattt gttctgggtg aaggaagtgt cagttctaat gatgtggaaa gcattgttgg 180

tgtgcctatg aaaaaagaag tacttggagc tataggagag gataatactg atacattccc 240

caatgtaaat ggagccggtc ctgataataa ttacgcatcc gccccgattc ctggagcttc 300

tgttactttc cataggcctt cagggaaata tataaccaac tatgataaca attacgatcc 360

aatgcgctcg aatgttgcgc ccccatccgt agatcagagg ggtggttccg gcttacctta 420

ctacataact gaagataggc tgaaagaaat acgttctctg tttatgtatc catactataa 480

taagggtgga ggtggtgaca acgatggaga ttatcagaaa caaatccaag cctctacttc 540

aatcgtacac aagaacctga attttcagct tcccttcttc gggttcaggt acaattacac 600

tagagtttca ctcaacgggt atttggaatt cagcgatcca ccaccaacct atggcaaata 660

tccactcatc tttcctagtc ctggatggcc ggaggtgaat gatccagctt tcatcggaat 720

tttttatagc aaatgtcgta tgggtcttct aagggctgaa gatatagatc agaggaatcc 780

tggagtgtat ttcaggcttg agcgggattt gaggagcaga accgaccaga tgggtgtcga 840

aataagagaa aggaccaaat gggacatcag ggaaggtgtg ataggaactg acagtttcga 900

tcctaagcat gcaattatta taacttggaa aaatgtttct ttcgctgggg gtatatctga 960

cagtttgatg aggaccaaca ctttccaaat ggtgctcgtg acagatgaag ttttcactta 1020

tgccattttc aactacctcc acctaggctg gaccactcat acggaagcaa gaggtgatac 1080

gactacagga gaaggaggta ctccagcatt tgttggattc aatggaggaa atggcactag 1140

aagttttgaa tacaaaccat acagtcaaga ttcagtaata cgagatctta catcaactgg 1200

tttcgccaat ggttttccag gaaggcacat attcagaata gatgaaaaca ttttactagg 1260

aacttgtaat aaggatatag atgccggtcg gttagatctc atgtttgctc cagaaagtgg 1320

aaacatgtta ggaggaactg ttgttaacat aactggtcct tgcttcaaac cgaatcaaaa 1380

aatcacctgc atctttgata ttgctccgcc agttattggc gttattgttg atagaaatag 1440

agccatttgt atacaacctc cgctaatggc tgaaggttgg gttaggttcg agattgcagt 1500

gggaaacgag aggtttaaat ggaaaggaag atattttgtc gaaacaccca caatggcagc 1560

gcaaaagata ttcttttcag acatgacgta tcatgacaaa tctcctgcgg aaattcggat 1620

atcatgggaa aataagaact taacatctaa cgtaaatgcg aatataagaa tttcactatg 1680

gggttataga gaaacactcg tatctcccca attcgtttat ataacccaat tggcgacgaa 1740

catacaaaac accggagaat acgtgattgt cccatctcaa tacagattga atgaaaacaa 1800

gtttcaacaa gatataactg tcggtttcat ccagatcaac ctgactgatt cgatacctgt 1860

tggtaacagt agcgtgaaaa taacaccgct catatggagc agacccattc ctttggcgtg 1920

gtactttggt ccacaatggc agaggttata tggaaacagc tggccttcag ttctttgtga 1980

taattggatc aaggatgatc gttatctgag taatttcgct catgaattac ctcaatgtcc 2040

ttgtacttta caacaagctt tagttgataa aggaaggtac atgcctgggt tcgattgtga 2100

cagagactcg aacccattat gctactacca agacaatgcc atacattgtg ttcgatcagg 2160

tttaccaacg aatgaaggag ggaacagcaa atgctgctac gataaaaata atttccttat 2220

gctgtcttat gaccaaatag gaggctcaaa tccgagacgt tctcacaatt taggtttcaa 2280

cccttacaca gaatctacaa aagtacccgg tttgtcacag tggtaccacg acatgatccc 2340

gaagtttgtc tgttgtcgct ggcaagacaa ccaagcagtc ggttgtgaaa ccttcaggtt 2400

tgaaagacga ccaactcaag attgtacagc ttaccaagga cctgccgtgg ctggggtata 2460

cggagatccc catgtaatca cgtttgatgg attggagtac acattcaacg gaaaggccca 2520

gttcgttctt gtgaaaacca acgttgcctc gcaaaggtta gaggttcaag caaggttcga 2580

gcagatgcaa ccaaatcaat tcaatgaggt tagagccact cagttgacgt ctatagccat 2640

tagagggaat gcttcaacta tcattgaagt tagaataaga ccaaaacatg caaggtggag 2700

gtatcggatg gatgttatag ccaacaagga gaagagacta ttttttgaca gaccttcatt 2760

gaagttccaa catttctcag gattgacggt ttacacccca acatacatac tgaaccagtc 2820

ggaagtgatt gtaatgcttg attctggagc aggtgtggaa atagtcgaaa gtttagggta 2880

catgagtgca agagttttcc ttccatggtc gtttattaat aaaactgtcg gtctctttgg 2940

aaactggagt tttgatataa cagatgattt cactcttcca gatggaacga aaaaacctat 3000

catcaccaac ataaatgact gcaaagtgtt tatgacaact ttggaaaact gtggatgata 3060

gacgactccg aagattcgca aacaggacaa tctcttttcg ttaacgaatt tggacgtact 3120

gctgcatact ataacgacct cgactttgta ccagaattcc gaatgaacat tagagatctg 3180

ctaataccaa ccaacgcatc ggattattat atacagcagg catacaaagc atgcaccaac 3240

aaagactacc aatgcttgta tgattacgca atgacattgg acaaagactg cgcattctat 3300

acaacaatat tcaaatcagg tttgaaaaat ataagagaag ctagcggtga acagactgta 3360

tcatgcggtg ttctggaaac cccaaggttc ggttggaaat cgaatttttc ttcactcctg 3420

gaagtaaagt tagtttcgaa tgcgctcaag atttcgttct tgacggggat cctagaagag 3480

aatgcatgga aaatggacaa tggaacctac ccgaattcgg attcacggaa tgtttacgtc 3540

aagaagaata ttcgttgaga caagcaggaa ctacatatgg aataatcctg gcagtgatga 3600

ttcctcttat tcttctacta gttttctttg gattcagatt tatgcgtaaa ctatctgaac 3660

aaaatgacca gaccaatcaa gaaaaactga ctatggagca acagaaaagg ttggcacagg 3720

agagggcagc gaaaaaatta acagatgtcg atgatgagca taatgatcta ttttcagaac 3780

attctcaatg ttatcagcaa tatcaatggt tttctacatg aatatatctt caaatgtgtg 3840

aaaaa 3845

<210> 706
<211> 982
<212> DNA
<213> Diabrotica undecimpunctata

<220>
<221> misc_feature
<222> (1)..(982)
<223> RNAi target sequence ta01950.01_diaun

<400> 706
tgaaaaagca aatcactacg acttgccgga ttttgtagta gtgttaattt tgtattagaa 60

aatcaaaatg agttctattg gaactgggta cgatttgtca gcttcccaat tctctcctga 120

tggaagagtt tttcaagttg aatatgcaat gaaagcagtt gaaaatagtg gcactgtaat 180

aggtctccga ggtacagatg gaattgtatt agctgctgaa aagcttatta tgtcaaaatt 240

acatgaaccc agtacaaaca aacgaatttt caacattgat aaacatatag gaatggcatt 300

ttcaggttta atagctgatg caaggcaaat agttgagatt gctagaaaag aagcatcaaa 360

ttataggcac caatatggat caaatattcc tcttaaatac ctaaatgata gagtaagcat 420

gtacatgcat gcatacactt tatacagtgc tgtcagacca tttggttgca gtgtcatatt 480

gtccagttat gaagataatg acccatctat gtttttgatt gatccatctg gagttagcta 540

tggatacttt ggatgtgcta caggaaaagc taaacagtct gcaaagactg aaatagaaaa 600

attgaagatg gggaatttaa catgcaaaga acttgttaaa gaagcagcca aaataattta 660

tttggtccat gatgaattga aggataagaa ttttgaactg gaactttcat gggtatgcaa 720

agatacaaat ggtttacata ccaaagtgcc tgagtcagtg tttgctgatg cagaaaaggc 780

tgctaagcaa gcaatggaag cagattcaga atcagataca gaagatatgt aataactaca 840

tttagttttt aatatttcac tgatggtggc tgttcttaca atattcgtgt gttatgtcta 900

taaattatgt aatactgtga gaatttccat ttcaatgata ggtttataac tttttttcta 960

ataaatacat aattttaaaa aa 982

<210> 707
<211> 795
<212> DNA
<213> Diabrotica barberi

<220>
<221> misc_feature
<222> (1)..(795)
<223> RNAi target sequence ta01738.01_diab

<400> 707
tgacactgca accaaatggt ctaacagcac cgtaataggc cttcgaggta cagacggcat 60

tgtattggct gctgaaaagc tcattatgtc aaaattgcat gaaccaagta caaataaacg 120

aattttcaac attgataaac acataggaat ggcattttca ggcttaatag ctgatgcaag 180

gcaaattgtt gagattgcta gaaaagaagc atcaaattat agacaccaat atggatcaaa 240

tattcctctt aaatacctaa atgatagagt aagcatgtac atgcatgcat acactttata 300

cagtgctgtt agaccatttg gttgcagtgt catcttggcc agttatgaag atagtgaccc 360

atctatgtat ctgattgatc cgtctggagt tagctatgga tactttggat gtgctacagg 420

taaagcaaaa cagtctgcaa agactgaaat agaaaaattg aagatgggga atctaacttg 480

caaagaactt gttaaagaag cagccaaaat catttatttg gtccatgatg agctaaagga 540

taagaatttt gaactggaac tttcatgggt atgcaaagat acgaatggtt tacataccaa 600

agtgcctgaa tcagtgtttg ctgatgcaga aaaagctgcc aaacaagcaa tggaagcaga 660

ttcagaatca gatacagaag atatgtaata actagataca tttagttttt aatatttcgc 720

tgatgtggct gttcttacaa tatttcatgt gttatgtcca tatattatgt aatactgtga 780

gaatttccat ttcaa 795

<210> 708
<211> 993
<212> DNA
<213> Epilachna varivestis

<220>
<221> misc_feature
<222> (1)..(993)
<223> RNAi target sequence ta26097.001_epiva

<400> 708
atttgtatat ttataatcat agataatata taatatattt aggttttcaa tcaagtaata 60

ccaaattatt aatataaaat gagttctatc ggcactggct atgatctttc ggcttctcaa 120

ttttctcctg atggtagagt ttttcaagtc gaatatgcta tgaaagctgt ggaaaatagt 180

ggaacagtta taggtcttaa aggaactgat ggggtagtac ttgcagcaga aaaattaatc 240

atgtctaaac tacatgaatc tggcaccaac aaaagaattt ataatataga tacacatatt 300

ggaatgtgtt tttctggtct tattgctgat gctaggcaat tagttgatat agcgagaaag 360

gaggcatcaa attatagatc acagtatggt gtaagcattc cactcaaata tcttaatgac 420

agagtcagca tgtatatgca cgcatataca ctttacagtg ctgtaagacc ctacggctgt 480

agcattattc ttgctagtta tgaggaagat cagcctatga tgtatttaat tgacccctca 540

ggtgtaaact atggatttaa tggttgtgct actggaaaag caaagcaatc tgctaaaaca 600

gagattgaaa aactgaaatt gggtgagctg agttgcaaag agttggttag agaagctgcc 660

aaaattattt acttagtcca tgatgagttg aaggataaaa attttgagct tgagttatca 720

tgggtgggaa aagatagcaa tggtagacat gaaaaagttc ccagccacgt tttagctgat 780

gctgagaaag cagcaaaaca agcaatggag gcagattccg attctgatac tgaggatttg 840

tagatataca tttgattatt ttataagtgc agttctaata aagcgtaatt tctttcaatg 900

tgtcatctta aataacgccc tttttcttgt aattaacgtc aaagaaaaaa ctcattacaa 960

acaatttgtt ctgtaccata atgtgttcat tgg 993

<210> 709
<211> 936
<212> DNA
<213> Leptinotarsa decemlineata

<220>
<221> misc_feature
<222> (1)..(936)
<223> RNAi target sequence ta37784.0003_lepde

<400>  709
caactatttg agtatttgac agatgagttg tattttaaga aagaagcaaa tcacgctagt      60

tcaaaactcc aaaactaaat ttttaaaatc gaatttaaac agtcaatatg agttcaatag     120

gaactgggta tgacttgtcg gcttcgcaat tctcacctga cggaagagtc ttccaagtcg     180

aatacgccat gaaagccgta gaaaacagtg ggaccatcat tggcctcaag ggcactgacg     240

gaatcgtcct ggcagccgag aagctcatca tgtctaaact gcatgaaccg ggaacgaaca     300

ggagaatctt caatatcgat agacatattg gaatggcgtt ctctggcctg atagcggatg     360

ctcggcagat cgtcgaaatc gctaggaaag aggcagctaa ttataggctc cagtacggca     420

ttaacatacc tctgaagtac ttgaacgaca gagtgagcat gtacatgcat gcatatacct     480

tgtacagtgc cgttcggcct tacggatgca gcgttatatt agctagttac gatgaggatg     540

agcctcagat gtacctaatt gatccatcag gagttagcta tgggtttcac ggctgcgcaa     600

cagggaaagc gaaacagtcg gccaaaaccg aaatagagaa gctcaaacta ggcaatttga     660

catgtaaaga attggtgaaa gaagcagcaa aaataatata catggtccat gacgaactca     720

aagacaaaaa ctttgaaatg gagctttctt ggatatgtaa agaaagcaat gggttacacg     780

tgaaagttcc tgaatccgta tatttggagg cagaacgggc cgccaagcaa gccatggaag     840

ctgattcgga atcggataac gaggacatgt gaggctttgt cagttagtat taagattatg     900

gcttagttgt aagatgtctt ggctaaatct tttttt                               936


<210>  710
<211>  927
<212>  DNA
<213>  Orius insidiosus


<220>
<221>  misc_feature
<222>  (1)..(927)
<223>  RNAi target sequence ta09672.01_oriin

<400>  710
   aggtaacatt acatttgagg ccgcatattt tgctttatca ttactttaaa tttaatttta      60
   cttttatttc ttgttaaaac gtttgtttga catcaaaaat gagttccatc ggtaccgggt     120
   atgatttgtc ggcgtcacaa ttttcgcccg acggacgagt ctttcaagta gaatacgctc     180
   agaaagccat tgaaaacagt gggacggtaa tcggcctgag aggtaaagat ggtgttgtct     240
   tggctgtcga aaagcttgtc acgtcgaaac tgtatgaacc aggagccaat aaaaggatat     300
   tcactattga caaacatatc ggaatggctg ttgctggctt gatatctgat gcgaggcagg     360
   tggcggaagt cgctaggacc gaagctgcta attatcgctc ccaatacgga agtaacatcc     420
   cgttgaagta tttgaacgag cgagtctccg gttatatgca tgcatatact ttatacagcg     480
   ctgtccgacc tttcggctgc agcgtcatcc tcagcactta caacgagcga gatggacccg     540
   aaatgtacat gattgaccca tcaggtgtct catacggtta tttcggatgc gctgtcggca 600
   aagcgaaaca agccgccaaa accgaaatcg aaaaattaaa attgaaagat ttaacatgca 660
   aagaattagt caaagaagct gccagaataa tttatttggt ccatgatgag cttaaagata 720
   aacagtttga acttgaaatg agctggatcg gcgctcatac aaacggtgta aaccaagttg 780
   tcccgccgac cgtccacgcc gaagctgaaa aagatgcgaa agctgctatg gaagaagatt 840
   cagattctga aactgaagat atttaactta attatactat gttctaatta acttttttcc 900
   attaataact ttttttttct attcaaa 927

<210> 711
<211> 1115
<212> DNA
<213> Coleomegilla maculata

<220>
<221> misc_feature
<222> (1)..(1115)
<223> RNAi target sequence ta36121.0001_vibdu

<400> 711
   caatgagttg tggatttcag atttgaagga tcatataaaa ttgtttgctt gaaacataaa 60
   ttacaatttc attctaattt aataaaggaa aaatgagttc aataggcact ggttatgatc 120
   tttctgcgtc acaattctcg ccggatggta gagtttttca agtggaatac gctatgaaag 180
   ctgttgaaaa cagcggaaca gtaataggtc ttaaaggtac agatggaatt gtcctagcag 240
   cagagaaatt aattacctct aaattacatg aaccaggaac aaataagagg atattcaata 300
   tagatacgca tattggaatg tgtttttcgg gtttgatagc tgatgctagg caactggtgg 360
   atatagctag aaaagaagcg gctaactata gatcacaata tggtgtgagc attccactga 420
   aataccttaa cgatagagtg agcatgtaca tgcatgctta cactctatat agtgctatca 480
   gaccttatgg ttgtagtgta attctagcaa gctatgaaga aaatcaacca tttatgtatt 540
   tgattgatcc ttctggagtt agctatggtt acaatgggtg tgctacaggt aaagcgaaac 600
   agtcagcaaa gaccgaaatt gaaaagttaa agttgggcga attgagctgc aaggaattag 660
   ttagagaagc ggctaaaatt atttaccaag tacatgatga attgaaagat aagaattttg 720
   aacttgaatt atcatgggta ggaaaggata gtaatggtag acatgaaaga gtaccaagtc 780
   atatttatgc tgatgccgaa aaagctgcta aacaagcaat ggaagcagat tcagattctg 840
   atacagagga tttataagtt ttaactttat tatttaagtt cactcagaaa ttaataaatg 900
   ttttgactta catttttttt aagacattat gttatagcca cattttctct atccatttat 960
   atcatttata tcaacaattc tttaaaactg aaggaccgaa atttagttaa acaatactcg 1020
   ataaaatatg aaataatttc aaagcaattg aaagattggt taatataggt tgaaatgttt 1080
   tagaggctgc aacagtgatt tgtagaattt tgcaa                                1115

<210>  712
<211>  853
<212>  DNA
<213>  Diabrotica undecimpunctata

<220>
<221>  misc_feature
<222>  (1)..(853)
<223>  RNAi target sequence ta07256.01_diaun

<400>  712
cattattgta ccgtgttgtc tagcggatat tttagattta acaatcttat tttataaaat      60

aattagttaa aatgatgcaa accgccaata atgtatatta tcccgattat tccactgctc     120

caatgcaacg tcaaattaat ccttatgccg acaatggagg gagtgtagta gcaatagcag     180

gtgaagactt tgtaataatt ggtgcagata cacgtttgag tgctggattt tccatttaca     240

ctagagaaca aaacaaacta ttcccgctat caggcactac agttttaggc tgtgcaggat     300

gttggtgtga tactctaaca ttaaccagaa tccttaaatc tcgcatgcag atgtaccaac     360

aagagcataa caaaacaatg tctacaactg catgtgccca aatgttgtca accatgctct     420

actacaagag attctttcct tattatatat caaacattct tgttggttta gataatgaag     480

gaaagggctg tgtttacagc tatgatccaa tcggacattg tgaaaaagct acttatagag     540

caggtggttc ggctggagct ctgctccaac cccttttgga caatcagatt ggacaaaaga     600

acatgcttaa aacatctgga gaacctctta gtcaggagaa agctttggct acactgaaag     660

atgtatttat ttctgctgct gaaagagata tctacactgg agatagtgta cttataaata     720

ttattactaa agatggagta aaggaagagt ccttccagtt gagaagggat tagaagcaag     780

tgtttttgtt tatattattt tcttatgtgt aattcaaata tactttctaa ataaacaatt     840

ttaagataaa aaa                                                        853

<210>  713
<211>  856
<212>  DNA
<213>  Diabrotica barberi

<220>
<221>  misc_feature
<222>  (1)..(856)
<223>  RNAi target sequence ta07284.01_diab

<400>  713
gcataggggt taatttgacg ttgcattgga ccagcggatt ttagattcaa caagcttatt      60

ttataaaata attagttaaa atgatgcaaa cagctaataa tgcatattat cccgattatt     120

ccactgctcc aatgcaacgt caaattaacc cctatgcaga taatggaggg agtgtagtag 180

caatcgcagg tgaagacttt gtaataattg gtgcagatac acgtttgagt actggatttt 240

ccatctatac cagagaacaa aacaaacttt tcccactatc aggcactaca gttttgggtt 300

gcgcaggatg ttggtgtgac actctaacat taaccagaat ccttaaatct cgcatgcaga 360

tgtaccaaca agagcataac aaaacaatgt ctacaactgc atgtgcccag atgttgtcta 420

ccatgctcta ctacaagaga ttctttcctt attatatatc aaacattcta gtaggtttag 480

ataatgaagg aaagggctgt gtttacagct atgatcctat tggacattgt gaaaaagcta 540

cgtatagagc aggtggttca gctggagctc ttcttcagcc tctgttggac aatcaaattg 600

gacagaagaa catgcttaaa acatctggag aacctcttag tcaggagaaa gctttgtcta 660

cccttaaaga tgtatttatt tctgctgctg aaagagacat ctacactgga gatagcgtac 720

ttataaatat tattactaaa gatggagtaa aggaagagtc cttccagttg agacgggatt 780

agaagcaagt ggttttgttt atattttctt atgtgtaatt caaattaata tactttctaa 840

ataaacaatt ttaagt 856

<210> 714
<211> 796
<212> DNA
<213> Epilachna varivestis

<220>
<221> misc_feature
<222> (1)..(796)
<223> RNAi target sequence ta27792.001_epiva

<400> 714
agtgagtttg ttgttctagt agagtaaata ccgaataaaa atgcattcaa taatgaatga 60

ttctcgtcca tttttcgaag attattctgt taagccaatg cagcgtcaat tcagccccta 120

cgctgataat ggagggagca ttattgcaat ttctggtaac gacttcgtgg tgattggtgc 180

agatactcgt ttgagttctg gattttcgat atacactcgg gatcaaaata agttattcaa 240

actctcagat aatacagttt tgggatgttc tggatgttgg tgtgatactc tttctttaac 300

tcgtattttg aacgcacgta tgcaaatgta tgaacaagaa cacaataaga aaatgtccac 360

tccagcttgt gctcaaatgt tatcgaccat gttatattac aagcgattct ttccgtatta 420

catttctaac atattagttg gtttggattc tgagggaaaa ggatgtgttt atagttacga 480

tcctattggt catactgaaa gatctagtta tagagctgga ggatcagcag gtgccttact 540

acaacccctc ttagataatc aagttggtat gaaaaatatc cagaatgcac ataatgaacc 600

actaactatt gaaagagctg ttggaatttt aaaggataca tttatatctg cagctgaaag 660

agatatttat acaggtgata gcattaagat caatgtcatc actaaagatg gcatcaaaga 720

agatactttt gagttgagga aagattaaat tgtatacttt ttaatatctt caataaaaat 780

ttaattataa aaaaaa 796

<210> 715
<211> 842
<212> DNA
<213> Leptinotarsa decemlineata

<220>
<221> misc_feature
<222> (1)..(842)
<223> RNAi target sequence ta37916.0001_lepde

<400> 715
tttttttata ttttcggctt gtcattctag ttgacttgtt taaaagtaga aagtatttgt 60

gtttcctcca ctataaaatg caaacagcaa tgaacgctta ttatcctgat tattcaacgg 120

taccggttca gcagtctttc agcccctatg ctgataatgg agggagcgta gtggccatag 180

ctggagatga tttcgtggta gtgggagcag atacacgtct aagcgccggc ttctccatct 240

acactagaga ccaaaataag ttgtttcctc tttcaaaaac aacggttctc ggatgctcag 300

gttgctggtg tgacacgcta acgttaactc gtattttgaa gtctcgcatg cagatgtacg 360

agcaggagca taataaagta atgtcgacca ctgcttgcgc gcagatgctc tccaccatgc 420

tctactacaa acgctttttt ccttattaca tctctaatgt tctcgtgggt ctcgacaacg 480

aaggaaaagg ttgtgtttac agctacgacc ctattggaca ttgtgaaaag actacgtaca 540

gagctggtgg ttctgctggg gctctgctcc agcctctgtt ggataatcaa atcggacaga 600

agaatatgga gatagtgtct accgagccct tgtcgaaaga gagagctctg gctatattga 660

aggatgtctt catctctgca gccgagagag acatatacac cggagatagt gtttctatca 720

atatcataac taaggatggt gtgaaagagg aatcatttga attgaggaaa gattagagga 780

attttatatt gaattttcct tcatgaattt ttgattatat gatttaagta aaaaaaaaag 840

tc 842

<210> 716
<211> 755
<212> DNA
<213> Orius insidiosus

<220>
<221> misc_feature
<222> (1)..(755)
<223> RNAi target sequence ta09310.01_oriin

<400> 716
cgcttccgat actcgtttga gccaaagcga aaaatttttg tttggtcatc tgctgtacgg 60

tattagctga agaaatgtct ttttcaccgt attctgataa tggagggagc atcgttgctg 120

tcgccggtga cgattattcg atcatcgctt ccgatactcg tttgagcgct gggtattcga 180

tttatacgcg tgagcagtcg aaattgttcc cgctgtcgga aaaagcgatt ttgggaagcg 240

ctggatgttg gtgtgacgtt ctttctcttg ctagattgct ccagacgcgc attaaaatgt 300

acttgtatga gcataataag gcgatgtcaa ctccagctat agcgcagatg ctatctatca 360

taatgtacta taaacgattc tttccgtatt acacgtcgac aattttggcc ggattagatt 420

ccgagggtaa aggaatcgtc tacagctacg acccaatcgg tcatatggaa agctctaaat 480

atcgtgctgc aggttctgca ggagcacttc tccagcctct tcttgacagc cagctgggca 540

agaaaaacca ggagaatgtc gtgaataaat cgtacagctt ggaagaagct ttggcgatcg 600

tgaaggacac ttttatttcg gctgctgaaa gagatattta tactggagac agcatcgctt 660

tagccatcat cactcaagat ggcgtcaaaa acgagacttt ccaactgagg aaagattaaa 720

aacatattat ttgtactatt tttgctcttt ttgaa 755

<210> 717
<211> 813
<212> DNA
<213> Coleomegilla maculata

<220>
<221> misc_feature
<222> (1)..(813)
<223> RNAi target sequence ta23917.0001_vibdu

<400> 717
gaaaatgtct ataatgaatg attctcgtcc tattttcact gattattctc aaaaacctat 60

gcagcgacaa tttagtccct atgccgataa cggagggagc atcattgcta tagctggaga 120

cgattttgtt gttatcggag cagatactcg cttgagtgct ggttattcca tatacacccg 180

cgatcaaaat aaactcttta aattgtctaa cacaacagta ttgggttgtt ctggttgttg 240

gtgtgatact cttactctaa caaggattct gaatgcccgt atgcaaatgt atgaacaaga 300

gcataataag attatgtcta ctcctgcttg tgctcaaatg ctttcaacca tgctatatta 360

taaacgattc tttccttact atatatccaa tatattggca ggcttggact ctgacggaaa 420

aggctgcgta tacagttatg atccaatagg tcatacagaa cgctctgctt atagagctgg 480

tggatccgca ggtgttctac tgcaacctct tttagacaac caaattggcc ttaaaaatat 540

ggttaatact ccgaatttgc ctctgaccat tgaaagagct gttggaattt taaaagacac 600

attcatctct gctgcagaaa gagatataca cactggtgat agcatcaaaa ttaacataat 660

aactaaagat ggaattaagg aagacacttt tgaattgaga aaagattaaa catttttatt 720

ttttgtcaac aatgggtaaa aattatctaa ttttgaggat gttattgtga gtgtctctca 780

agtatttaat tataactccg aaatataaaa aaa 813

<210>  718
<211>  785
<212>  DNA
<213>  Diabrotica undecimpunctata

<220>
<221>  misc_feature
<222>  (1)..(785)
<223>  RNAi target sequence ta08127.01_diaun

<400>  718
tttttttagt gcaaaactta ttttttttat tttaatatat accaaaacat accaaagctt      60

aaaaaataat taattaacct tcataaaatt tgggcaattc atttcctaaa ataactcttc     120

gttcaatacc agcttcattg ataattccca gtctgactac acctcctgaa gaaccatctc     180

tggacatagc aagtgccaat gtatttgtga caaacttcac acattcttct ttgctcatat     240

tgggtttgaa attggcatct acataaccat aaacataact ggaacctgat cctcctattg     300

acacttcttg cctcacacac atcccaccaa ttggtatgga atagacttga ccgcctttct     360

tcttatccca acctgctacc agtattccag ccattagtga atctctataa ttgtagcaaa     420

gttcttggaa aatggcagca cctacttcta ctttgggttc ttcaccaagt tccataccat     480

gaaaattaag atgataagca acaatgtctg caattgcttg tgtatctgct gcagatccgg     540

aacgacaaca gtagatatgg tcagtgactt tggtgagttt gtctgctaca cggtttgcta     600

tgtaagcccc agtagttgtg cgagaatctg ctcctataac aacgccgccg tcaaactctg     660

cggccataat agaggtgcct gtactgtgag cagcatctct ccaatcatca ggaccagtca     720

ttgcaccaaa ctcattcata agaggcattt ttataagttt aatttcaaga atacaagctg     780

agaaa                                                                 785

<210>  719
<211>  842
<212>  DNA
<213>  Diabrotica barberi

<220>
<221>  misc_feature
<222>  (1)..(842)
<223>  RNAi target sequence ta05581.01_diab

<400>  719
tttttggtgt aaacctgatt tttttatttt aatatatacc aaaacatacc aaagcttaaa      60

aaataattaa ttaaccttca taaaatttgg gcaattcatt tcctaaaaca actcttcgtt     120

caataccagc ttcagtgatg atccccagtc tgactacacc tcctgaagaa ccatctcttg     180

acatggcaag tgccaatgta tttgtgacaa acttcacaca ttcctctttg ctcatgttgg     240

gcttgaagtt ggcatctacg taaccataaa cataactgga gcctgatcct ccaattgaca 300

cttcttgtct cacacacatc ccaccaattg gtatggaata gacttgtccg cctttctttt 360

tatcccaacc tgctaccagt ataccagcca ttagtgaatc tctataattg tagcaaagtt 420

cttggaaaat ggcggcacct acttgtactt tgggttcttc accaagttcc ataccatgaa 480

aattaagatg ataagcaaca atgtctgcaa ttgcttgtgt atctgctgca gatcctgaac 540

gacaacagta tatatggtca gtgactttgg tgagtttgtc tgctacccgg tttgcaatgt 600

aagccccggt agttgtgcga gaatctgctc ctataacaac gcctccatca aactctgcgg 660

ccataataga ggttcctgta ctgtgagcgg catctctcca atcattagga gcagtcattg 720

caccatactc agtcataaga ggcatttttt acaagtttaa tgaaaagaat acaagcttag 780

aaaaattact tgtaatccta caatccaata ttttgccagt caccagctcc tataacaacg 840

cc 842

<210> 720
<211> 935
<212> DNA
<213> Epilachna varivestis

<220>
<221> misc_feature
<222> (1)..(935)
<223> RNAi target sequence ta23856.001_epiva

<400> 720
cgatcttaaa aatcggattt atatcgtgat tattgtaata attattctca cagctagccg 60

aataatttcg tttcgcgtta ctggtaacac tgaaaatgca tttttcattt tgtggttatg 120

attatttgga gaaaaataat ttgtcatagt cattgagtac aaattgtgtt cgatatattt 180

gcaatattag attcattaaa cttgatttta tactgaaaat cacaaactac catcgaaaac 240

atggactccg attggatgaa tgcaccacac agtacaggga cttctattat ggctgctgag 300

tttaatggtg gagttgttat tggtgccgac actagaacaa ctacaggggc atacatagcc 360

aaccgagtaa cggataaact aacaaaagta acagatcaca tttattgttg tcgttctggt 420

tcagcagcag atactcaagc tattgctgat atagtttctt atcatctagg atttcatgga 480

attgagttgg gagaagagcc acttgtagaa acaagtgcag ctgtgtttca agaactttgt 540

tacagctatc gtgattcttt aatggctgga attctagtag caggttggga taaacgtaaa 600

ggtggtcagg tttattctat tcctataggt ggaatgtgtg taagacagag cgtatccata 660

ggtggttcag gatcaagcta tgtttatggt tatgttgatg ccaattataa acctggaatg 720

tcaaaagaag cttgtgtaga ctttgtttca aaaactttgg cacttgcaat gtcaagagat 780

ggttcttctg gtggttgtat tcgtattgga gtcattacaa aggatggcat tgaacgcaag 840

gttattctgg gttctgaggt ccctaaattc tatgaagatt gattttattt gtattcaatt 900

aaataataaa taaaaatgtt aacatctaaa aaaaa 935

<210> 721
<211> 1264
<212> DNA
<213> Leptinotarsa decemlineata

<220>
<221> misc_feature
<222> (1)..(1264)
<223> RNAi target sequence ta33564.0001_lepde

<400> 721
tttacataat tacggaatac tgtgccatag attacagaaa tttaaatttt agtgttctgt 60

ggttctaacc tagcaaataa tttgtagagt cattttttga cttatttttt gttagtgatt 120

ttgtattcag attgtctccg ttcgtaaaaa tgctactgaa tcaaatatcc atagcaggtg 180

ccgatgactg gagaaatgcc gctcatagca ctgggacttc tattatggca gcagaattcg 240

atggaggagt aattattggt gctgattccc gtacaactac aggggcgtac attgcaaatc 300

gtgtaactga caaactaact aaagtaactg accatatata ttgttgtcga tctggatcag 360

cagcagatac tcaagccatt gctgatattg tatcgtacca tctgaacttc catggtatgg 420

aactaggaga agagcccctt gtagaggtgg gggctgctat cttcagggag ctttgctaca 480

actataggga ttctctcatg gcgggaatcc tagttgctgg ctgggacaaa aaaaaggggg 540

gacaaattta ttctattcct atcggaggga tgtgtgttag gcaagcggtt tctattggag 600

gttcaggttc cagttatgtt tatggttatg tagatgctaa tttcaaaccc aagatgtcaa 660

aggaggaatg tgcgaaattt gtcacaaata ctctggctct tgcaatgtct agagatggat 720

catctggagg tgttatccgt ctaggtataa ttacggagtc gggaattgaa cgtcgtgtca 780

tcttaggcga tgatctaccc aagttctatg aaggatagag catataataa tcataatttt 840

tcattttgta attggtgtca ataaatcaaa taaaataagg ttttctttaa aaaacaatta 900

tatgattata aaatacatgt taatgaacaa tgaatttgaa agagaaaaaa gatggttgtg 960

taccatgaag gcactaattc gtatctttat taatgtagtc tgtattttta gagagaatcg 1020

tgacaaaatt agtcgcatgg cgccatttgg caccaacata ttttgaattc ctttattttt 1080

tctggaactc tggtaataaa attatttctg ttatatctta gtattcaaaa tgccactgaa 1140

aaaattgaat cattattttc ttaccaattc tttttcttaa gaagatatcg ttacagcact 1200

accatcacca caaacagatt taccattttt tttgtcaacc ttctaatttt tacagtaaat 1260

gtat 1264

<210> 722

<211>  912
<212>  DNA
<213>  Orius insidiosus

<220>
<221>  misc_feature
<222>  (1)..(912)
<223>  RNAi target sequence ta09100.01_oriin

<400>  722
ttcagaatta gctatttaac cgaaggcgac tcagcgaatt tactaagtga aggtgaacta      60

ttctgtggag aaaaatggcc aaagtcgctg ttcatcatga tcgtttcggt gcccctgcaa     120

tgactgagga accagtttct actggaacta caattatggc ttgtactttt gacggaggcg     180

tcatcatcgg tgcggattct aggacgagta gcggacctta cgtcgccaat cgcgtttccg     240

acaaactgac aaaaatctcc gatcacattt actgttgtag gtcaggatcg gccgctgaca     300

ctcaagctat cgctgatatc gtcaaatatc acctttcttt ttttaagctt gaaactggtg     360

aagaaccgct tgttgcaact gctgcagcga ttttccgtga gctttgttat aactacagaa     420

atcgcttgat ggctggcatc atcgtcgctg gatgggacag agttaaaggc ggacaggttt     480

atacgatacc tttgggtggt atgataatcg agcagaaatt cgcaatgggc ggttctggaa     540

gttcttatgt ttatggcttc actgatgcca actatagaga aaacatgagt tcagaagaat     600

gcaaacagtt cattaaacaa gcaatcgctc ttgccattcg tcgtgacggc tcttccggag     660

gtgtcataag aatgggtctc atcacaaaag atggtattaa acgagaaatt ttttcgggta     720

aagattaccc ttcatattta actccagcta aagattagaa tatctttttg tattccttga     780

gctgtaaaat ttaagtacat tcatttataa ctttttgaaa tgattttctt tcatttatta     840

acaaaataat ccagtactaa taatactcca gcaagtacta tttcaataca catctttatc     900

acaaaggcaa aa                                                         912

<210>  723
<211>  686
<212>  DNA
<213>  Coleomegilla maculata

<400>  723
cctcacagta caggaacctc tatcatggct gctgaatttg agggtggtgt agtaattggt      60

gctgattcta gaaccaccac aggaacatat attgccaatc gagtaactga taagcttaca     120

aaaataactg atcgtatata ttgttgtcga tctggttcag ctgctgatac ccaagccatt     180

gcagatattg tatcttatca tttaggtttc catgggatgg aattgggaga agaaccactt     240

gtagaaacta gtgctgcagt attccaagaa ctatgctata attatcgaga ttctttgatg     300

gctggtatac ttgtagcagg atgggataag cgaaaggagg gtcaaattta ctcaattcct     360

attgggggaa tgtgtgtgag gcaaaaagtc tctattggag gttctggttc tggttttgta     420

tacggctatg tagatgctaa ctataagtct ggaatgacta aagaagcttg cgttgatttc 480

gtaattaaaa ctttggcact tgctatgtcg agagatggct catcaggtgg ttgtgttagg 540

attggagtta taactaaaga tggtatcgaa cgtaaagtaa ttttgggtac agaattaccc 600

aaattttatg aagattaata tttcataata tattttgtct taattcaata cttcattcaa 660

taaaattatg aaattttcga aaaaaa 686

<210> 724
<211> 250
<212> DNA
<213> Diabrotica virgifera

<400> 724
tcaacgtcat ctcagtttgg tttattgttg ggtgtaatgt ttaaataaaa taattattat 60

tttgtaaacc tgtgccatta aacaaataaa gcgatggtgc taattgcagc agcagtctgc 120

acgaaagcag gcaaaacaat tgtgtctcga caatttgttg aaatgaccaa agctagaata 180

gaaggtttgt tggctgcctt tcctaaatta attcctacag gaacccagca tacatttgtg 240

gaaacagatt 250

<210> 725
<211> 251
<212> DNA
<213> Diabrotica virgifera

<400> 725
acttcattcc agtcttgtga tatttagtgc ttacttggta cagcagtttc agtgctgtgc 60

tttagaataa tttatttttt aacatttata tagaaatcaa atactaacca atcaacatgt 120

gtgaagaaga agttgccgct ttagtcgtag acaatggatc cggtatgtgc aaagctggtt 180

ttgctgggga tgatgcacct cgtgctgtat tcccttcaat tgttggacgc ccaagacatc 240

agggtgtgat g 251

<210> 726
<211> 249
<212> DNA
<213> Diabrotica virgifera

<400> 726
gtaggaatgg gacaaaaaga ttcctatgta ggtgatgaag ctcaaagtaa aagaggtatc 60

cttaccttaa aataccccat cgagcacgga atagtcacaa actgggatga tatggagaaa 120

atttggcatc atacattcta caatgaactc agagcagccc cagaagaaca ccctgttctg 180

ttgacagaag ctcctctcaa ccccaaggcc aacagggaaa agatgacaca aataatgttt 240

gaaactttc 249

<210>  727
<211>  250
<212>  DNA
<213>  Diabrotica virgifera

<400>  727
acacttacac ccttcaaaaa ctcatttata aactaaaaat gggtgcatct gtaattcctt      60

tcatagtttt cactgtatta tggggaggag ttggagctgt tctacccatt tttgtaccta     120

aaggacccaa tagaggaatt atccaagtta ttcttatgat aacaggtgtg tcgtgctggt     180

tgttctggct gtgttgttac gtagctcaaa tgaatcctct gatcggtcct aaactagaac     240

aaaacatcgt                                                            250


<210>  728
<211>  211
<212>  DNA
<213>  Diabrotica virgifera

<400>  728
gataataagt tcgatttttt acgaaaatga caagtatcga gactgtgggg accattgtcc      60

tgaaattgct gaagttggtg atcaatttga tatgtctcat cttgtaccga accggatatc     120

aaggctactt cttgggagta ggaggaacct ggaatctaaa cgaagaaaaa aatcccgatg     180

cagaaattgt ggcttccggc gtattcgtag g                                    211


<210>  729
<211>  3114
<212>  DNA
<213>  Diabrotica virgifera

<400>  729
ggccagaatg gcccggaccg gcggccgccc tgcagctcta gagaaacttc gaaacgcgtg      60

gaccgaagct tgcatgcctg cagtgcagcg tgacccggtc gtgcccctct ctagagataa     120

tgagcattgc atgtctaagt tataaaaaat taccacatat tttttttgtc acacttgttt      180

gaagtgcagt ttatctatct ttatacatat atttaaactt tactctacga ataatataat     240

ctatagtact acaataatat cagtgtttta gagaatcata taaatgaaca gttagacatg     300

gtctaaagga caattgagta ttttgacaac aggactctac agttttatct ttttagtgtg     360

catgtgttct cctttttttt tgcaaatagc ttcacctata taatacttca tccattttat     420

tagtacatcc atttagggtt tagggttaat ggtttttata gactaatttt tttagtacat     480

ctattttatt ctattttagc ctctaaatta agaaaactaa aactctattt tagttttttt     540

atttaataat ttagatataa aatagaataa aataaagtga ctaaaaatta aacaaatacc     600

ctttaagaaa ttaaaaaaac taaggaaaca tttttcttgt ttcgagtaga taatgccagc     660

ctgttaaacg ccgtcgacga gtctaacgga caccaaccag cgaaccagca gcgtcgcgtc     720

gggccaagcg aagcagacgg cacggcatct ctgtcgctgc ctctggaccc ctctcgagag     780

ttccgctcca ccgttggact tgctccgctg tcggcatcca gaaattgcgt ggcggagcgg 840

cagacgtgag ccggcacggc aggcggcctc ctcctcctct cacggcaccg gcagctacgg 900

gggattcctt tcccaccgct ccttcgcttt cccttcctcg cccgccgtaa taaatagaca 960

ccccctccac accctctttc cccaacctcg tgttgttcgg agcgcacaca cacacaacca 1020

gatctccccc aaatccaccc gtcggcacct ccgcttcaag gtacgccgct cgtcctcccc 1080

cccccccctc tctaccttct ctagatcggc gttccggtcc atgcatggtt agggcccggt 1140

agttctactt ctgttcatgt ttgtgttaga tccgtgtttg tgttagatcc gtgctgctag 1200

cgttcgtaca cggatgcgac ctgtacgtca gacacgttct gattgctaac ttgccagtgt 1260

ttctctttgg ggaatcctgg gatggctcta gccgttccgc agacgggatc gatttcatga 1320

ttttttttgt ttcgttgcat agggtttggt ttgccctttt cctttatttc aatatatgcc 1380

gtgcacttgt ttgtcgggtc atcttttcat gctttttttt gtcttggttg tgatgatgtg 1440

gtctggttgg gcggtcgttc tagatcggag tagaattctg tttcaaacta cctggtggat 1500

ttattaattt tggatctgta tgtgtgtgcc atacatattc atagttacga attgaagatg 1560

atggatggaa atatcgatct aggataggta tacatgttga tgcgggtttt actgatgcat 1620

atacagagat gctttttgtt cgcttggttg tgatgatgtg gtgtggttgg gcggtcgttc 1680

attcgttcta gatcggagta gaatactgtt tcaaactacc tggtgtattt attaattttg 1740

gaactgtatg tgtgtgtcat acatcttcat agttacgagt ttaagatgga tggaaatatc 1800

gatctaggat aggtatacat gttgatgtgg gttttactga tgcatataca tgatggcata 1860

tgcagcatct attcatatgc tctaaccttg agtacctatc tattataata aacaagtatg 1920

ttttataatt attttgatct tgatatactt ggatgatggc atatgcagca gctatatgtg 1980

gattttttta gccctgcctt catacgctat ttatttgctt ggtactgttt cttttgtcga 2040

tgctcaccct gttgtttggt gttacttctg caggtcgact ttaacttagc ctaggatcct 2100

aactaactag gataataagt tcgatttttt acgaaaatga caagtatcga gactgtgggg 2160

accattgtcc tgaaattgct gaagttggtg atcaatttga tatgtctcat cttgtaccga 2220

accggatatc aaggctactt cttgggagta ggaggaacct ggaatctaaa cgaagaaaaa 2280

aatcccgatg cagaaattgt ggcttccggc gtattcgtag gtaattgaga attcgatatc 2340

aggtccgcct tgtttctcct ctgtctcttg atctgactaa tcttggttta tgatcttgat 2400

gatttagctt gactatgcga ttgctttcct ggacccgtgc agctgcccat cgaccctcaa 2460

ttacctacga atacgccgga agccacaatt tctgcatcgg gatttttttc ttcgtttaga 2520

ttccaggttc ctcctactcc caagaagtag ccttgatatc cggttcggta caagatgaga 2580

catatcaaat tgatcaccaa cttcagcaat ttcaggacaa tggtccccac agtctcgata 2640

cttgtcattt tcgtaaaaaa tcgaacttat tatcctagtt agttaggtta acctagactt 2700

gtccatcttc tggattggcc aacttaatta atgtatgaaa taaaaggatg cacacatagt 2760

gacatgctaa tcactataat gtgggcatca aagttgtgtg ttatgtgtaa ttactagtta 2820

tctgaataaa agagaaagag atcatccata tttcttatcc taaatgaatg tcacgtgtct 2880

ttataattct ttgatgaacc agatgcattt cattaaccaa atccatatac atataaatat 2940

taatcatata taattaatat caattgggtt agcaaaacaa atctagtcta ggtgtgtttt 3000

gcgaattatc gatgggcccc ggccgaagct ggccgcgggc atgtggtacc taagggccca 3060

taggcgcgcc cggtgcatgc aagcttgctt caagggcccg tttgtacaac ttta 3114

<210> 730
<211> 159
<212> PRT
<213> Diabrotica virgifera

<400> 730

Met Thr Ser Ile Glu Thr Val Gly Thr Ile Val Leu Lys Leu Leu Lys
1               5                   10                  15

Leu Val Ile Asn Leu Ile Cys Leu Ile Leu Tyr Arg Thr Gly Tyr Gln
            20                  25                  30

Gly Tyr Phe Leu Gly Val Gly Gly Thr Trp Asn Leu Asn Glu Glu Lys
        35                  40                  45

Asn Pro Asp Ala Glu Ile Val Ala Ser Gly Val Phe Val Gly Phe Met
    50                  55                  60

Ile Tyr Thr Phe Val Ser Leu Ile Ser Leu Cys Phe Ala Ser Gly Asp
65                  70                  75                  80

His Lys Thr Thr Phe Thr Asp Ile Leu Met Asn Ile Val Gly Ile Phe
                85                  90                  95

Met Trp Ile Ala Ala Gly Ala Thr Ala Leu His Tyr Trp Leu Gly Tyr
            100                 105                 110

Leu Ser Glu Tyr Lys Tyr Thr Thr Ile Asp Ser Glu Arg Gln Val Gly
        115                 120                 125

Leu Ala Leu Gly Ala Met Cys Ile Ile Asn Gly Ala Val Tyr Leu Val
    130                 135                 140

Asp Gly Val Leu Ser Ala Ile Phe Ile Leu Lys Ala Lys Met Gln
145                 150                 155

<210>  731
<211>  162
<212>  PRT
<213>  Drosophila melanogaster

<400>  731

Met Val Ser Val Glu Thr Val Gly Ser Ile Phe Ile Lys Ala Leu Lys
1               5                   10                  15

Leu Ile Ile Asn Leu Val Ile Ile Phe Leu Tyr Arg Trp Gly Asp Gly
            20                  25                  30

Gly Glu Phe Leu Gly Ile Gly Gly Thr Trp Asn Leu Asn Glu Glu Lys
        35                  40                  45

Ser Ala Asp Ala Glu Ile Val Ala Ser Gly Val Met Val Gly Phe Leu
    50                  55                  60

Ile Tyr Thr Gly Cys His Thr Ile Ala Phe Ala Phe Gly Thr Thr Lys
65                  70                  75                  80

His Lys Gly Glu Leu Cys Asp Thr Ile Met Asn Val Val Gly Cys Ile
                85                  90                  95

Met Trp Ile Ala Val Gly Gly Val Ala Leu His Tyr Trp Lys Gly Tyr
            100                 105                 110

Met Ser Asp Glu Gly Phe Leu Tyr Val Asn Ser Glu Arg Gln Val Gly
        115                 120                 125

Ile Ala Met Gly Ser Leu Cys Val Ile Glu Gly Ala Leu Tyr Leu Leu
    130                 135                 140

Asp Thr Val Leu Ala Cys Ile His Tyr Ser Lys Gly Asp Thr Asp Tyr
145                 150                 155                 160

Thr Gln